(19) Europäisches Patentamt
European Patent Office
Office européen des brevets

(11) EP 3 193 919 B1

(12) **EUROPEAN PATENT SPECIFICATION**

(45) Date of publication and mention of the grant of the patent:
**08.04.2020 Bulletin 2020/15**

(21) Application number: **15842660.1**

(22) Date of filing: **15.09.2015**

(51) Int Cl.:
***A61K 39/00*** *(2006.01)*

(86) International application number:
**PCT/US2015/050270**

(87) International publication number:
**WO 2016/044326 (24.03.2016 Gazette 2016/12)**

(54) **IMMUNOGENIC/THERAPEUTIC GLYCOCONJUGATE COMPOSITIONS AND USES THEREOF**

IMMUNOGENE/THERAPEUTISCHE GLYCOKONJUGATZUSAMMENSETZUNGEN UND VERWENDUNGEN DAVON

COMPOSITIONS DE GLYCOCONJUGUÉS IMMUNOGÈNES/THÉRAPEUTIQUES ET UTILISATIONS DESDITES COMPOSITIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2014 US 201462050567 P**

(43) Date of publication of application:
**26.07.2017 Bulletin 2017/30**

(73) Proprietor: **OBI Pharma, Inc.**
**Tapei City, 11503 (TW)**

(72) Inventors:
- **YU, Cheng-der Tony**
  **Taipei City 11503 (TW)**
- **HSIEH, Yih Huang**
  **Taipei City 11503 (TW)**
- **CHEN, I Ju**
  **Taipei City 11503 (TW)**
- **LEE, Wei-han**
  **Taipei City 11503 (TW)**
- **WANG, Nan-hsuan**
  **Taipei City 11503 (TW)**

(74) Representative: **HGF Limited**
**4th Floor**
**Merchant Exchange**
**17-19 Whitworth Street West**
**Manchester M1 5WG (GB)**

(56) References cited:

| | |
|---|---|
| **WO-A1-03/015796** | **WO-A1-03/015796** |
| **WO-A2-2015/159118** | **US-A1- 2004 208 884** |
| **US-A1- 2009 317 411** | **US-A1- 2010 136 042** |
| **US-A1- 2010 136 042** | **US-A1- 2012 237 532** |

- **GILEWSKI T ET AL: "Immunization of metastatic breast cancer patients with a fully synthetic globo H conjugate: a phase I trial", PROCEEDINGS NATIONAL ACADEMY OF SCIENCES PNAS, NATIONAL ACADEMY OF SCIENCES, US, vol. 98, no. 6, 13 March 2001 (2001-03-13) , pages 3270-3275, XP002964246, ISSN: 0027-8424, DOI: 10.1073/PNAS.051626298**
- **RAGUPATHI GOVINDASWAMI ET AL: "Constructing an adenocarcinoma vaccine: Immunization of mice with synthetic KH-1 nonasaccharide stimulates anti-KH-1 and anti-Ley antibodies", INTERNATIONAL JOURNAL OF CANCER, vol. 99, no. 2, 10 May 2002 (2002-05-10), pages 207-212, XP002769439, ISSN: 0020-7136**
- **SLOVIN ET AL.: 'Carbohydrate vaccines in cancer: Immunogenicity of a fully synthetic globo H hexasaccharide conjugate in man' PROC. NATL. ACAD. SCI. USA vol. 96, 01 May 1999, pages 5710 - 5715, XP002155262**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 193 919 B1

SEQUENCE LISTING

<110> OBI PHARMA, INC.

<120> IMMUNOGENIC/THERAPEUTIC GLYCOCONJUGATE COMPOSITIONS AND USES THEREOF

<130> G3004-00401

<140> TBD
<141> 2015-09-15

<150> US 62/050,567
<151> 2014-09-14

<160> 2

<170> PatentIn version 3.5

<210> 1
<211> 3125
<212> PRT
<213> Megathura crenulata

<400> 1

```
Met Thr Pro Glu Glu Leu Lys Thr Tyr Leu Asp Glu Arg Ser Ser Arg
1               5                   10                  15

Ala Arg Ala Phe Ala Ser Phe Arg Leu Lys Gly Phe Gly Gly Ser Ala
            20                  25                  30

Asn Val Phe Val Tyr Val Cys Ile Pro Asp Asp Asn Asp Arg Asn Asp
        35                  40                  45

Asp His Cys Glu Lys Ala Gly Asp Phe Phe Val Leu Gly Gly Pro Ser
    50                  55                  60

Glu Met Lys Trp Gln Phe Tyr Arg Pro Tyr Leu Phe Asp Leu Ser Asp
65                  70                  75                  80

Thr Val His Lys Met Gly Met Lys Leu Asp Gly His Tyr Thr Val Lys
                85                  90                  95

Ala Glu Leu Phe Ser Val Asn Gly Thr Ala Leu Pro Asp Asp Leu Leu
            100                 105                 110

Pro His Pro Val Val Val His His Pro Glu Lys Gly Phe Thr Asp Pro
        115                 120                 125

Pro Val Lys His His Gln Ser Ala Asn Leu Leu Val Arg Lys Asn Ile
    130                 135                 140

Asn Asp Leu Thr Arg Glu Glu Val Leu Asn Leu Arg Glu Ala Phe His
```

145 150 155 160

Lys Phe Gln Glu Asp Arg Ser Val Asp Gly Tyr Gln Ala Thr Ala Glu
165 170 175

Tyr His Gly Leu Pro Ala Arg Cys Pro Arg Pro Asp Ala Lys Asp Arg
180 185 190

Tyr Ala Cys Cys Val His Gly Met Pro Ile Phe Pro His Trp His Arg
195 200 205

Leu Phe Val Thr Gln Val Glu Asp Ala Leu Val Gly Arg Gly Ala Thr
210 215 220

Ile Gly Ile Pro Tyr Trp Gly Leu Pro Tyr Trp Asp Trp Thr Glu Pro
225 230 235 240

Met Thr His Ile Pro Gly Leu Ala Gly Asn Lys Thr Tyr Val Asp Ser
245 250 255

His Gly Ala Ser His Thr Asn Pro Phe His Ser Ser Val Ile Ala Phe
260 265 270

Glu Glu Asn Ala Pro His Thr Lys Arg Gln Ile Asp Gln Arg Leu Phe
275 280 285

Lys Pro Ala Thr Phe Gly His His Thr Asp Leu Phe Asn Gln Ile Leu
290 295 300

Tyr Ala Phe Glu Gln Glu Asp Tyr Cys Asp Phe Glu Val Gln Phe Glu
305 310 315 320

Ile Thr His Asn Thr Ile His Ala Trp Thr Gly Gly Ser Glu His Phe
325 330 335

Ser Met Ser Ser Leu His Tyr Thr Ala Phe Asp Pro Leu Phe Tyr Phe
340 345 350

His His Ser Asn Val Asp Arg Leu Trp Ala Val Trp Gln Ala Leu Gln
355 360 365

Met Arg Arg His Lys Pro Tyr Arg Ala His Cys Ala Ile Ser Leu Glu
370 375 380

His Met His Leu Lys Pro Phe Ala Phe Ser Ser Pro Leu Asn Asn Asn
385 390 395 400

```
Glu Lys Thr His Ala Asn Ala Met Pro Asn Lys Ile Tyr Asp Tyr Glu
                405                 410                 415

Asn Val Leu His Tyr Thr Tyr Glu Asp Leu Thr Phe Gly Gly Ile Ser
            420                 425                 430

Leu Glu Asn Ile Glu Lys Met Ile His Glu Asn Gln Gln Glu Asp Arg
        435                 440                 445

Ile Tyr Ala Gly Phe Leu Leu Ala Gly Ile Arg Thr Ser Ala Asn Val
    450                 455                 460

Asp Ile Phe Ile Lys Thr Thr Asp Ser Val Gln His Lys Ala Gly Thr
465                 470                 475                 480

Phe Ala Val Leu Gly Gly Ser Lys Glu Met Lys Trp Gly Phe Asp Arg
                485                 490                 495

Val Phe Lys Phe Asp Ile Thr His Val Leu Lys Asp Leu Asp Leu Thr
            500                 505                 510

Ala Asp Gly Asp Phe Glu Val Thr Val Asp Ile Thr Glu Val Asp Gly
        515                 520                 525

Thr Lys Leu Ala Ser Ser Leu Ile Pro His Ala Ser Val Ile Arg Glu
    530                 535                 540

His Ala Arg Gly Lys Leu Asn Arg Val Lys Phe Asp Lys Val Pro Arg
545                 550                 555                 560

Ser Arg Leu Ile Arg Lys Asn Val Asp Arg Leu Ser Pro Glu Glu Met
                565                 570                 575

Asn Glu Leu Arg Lys Ala Leu Ala Leu Leu Lys Glu Asp Lys Ser Ala
            580                 585                 590

Gly Gly Phe Gln Gln Leu Gly Ala Phe His Gly Glu Pro Lys Trp Cys
        595                 600                 605

Pro Ser Pro Glu Ala Ser Lys Lys Phe Ala Cys Cys Val His Gly Met
    610                 615                 620

Ser Val Phe Pro His Trp His Arg Leu Leu Thr Val Gln Ser Glu Asn
625                 630                 635                 640

Ala Leu Arg Arg His Gly Tyr Asp Gly Ala Leu Pro Tyr Trp Asp Trp
                645                 650                 655
```

```
Thr Ser Pro Leu Asn His Leu Pro Glu Leu Ala Asp His Glu Lys Tyr
            660                 665                 670

Val Asp Pro Glu Asp Gly Val Glu Lys His Asn Pro Trp Phe Asp Gly
        675                 680                 685

His Ile Asp Thr Val Asp Lys Thr Thr Thr Arg Ser Val Gln Asn Lys
    690                 695                 700

Leu Phe Glu Gln Pro Glu Phe Gly His Tyr Thr Ser Ile Ala Lys Gln
705                 710                 715                 720

Val Leu Leu Ala Leu Glu Gln Asp Asn Phe Cys Asp Phe Glu Ile Gln
                725                 730                 735

Tyr Glu Ile Ala His Asn Tyr Ile His Ala Leu Val Gly Gly Ala Gln
            740                 745                 750

Pro Tyr Gly Met Ala Ser Leu Arg Tyr Thr Ala Phe Asp Pro Leu Phe
        755                 760                 765

Tyr Leu His His Ser Asn Thr Asp Arg Ile Trp Ala Ile Trp Gln Ala
    770                 775                 780

Leu Gln Lys Tyr Arg Gly Lys Pro Tyr Asn Val Ala Asn Cys Ala Val
785                 790                 795                 800

Thr Ser Met Arg Glu Pro Leu Gln Pro Phe Gly Leu Ser Ala Asn Ile
                805                 810                 815

Asn Thr Asp His Val Thr Lys Glu His Ser Val Pro Phe Asn Val Phe
            820                 825                 830

Asp Tyr Lys Thr Asn Phe Asn Tyr Glu Tyr Asp Thr Leu Glu Phe Asn
        835                 840                 845

Gly Leu Ser Ile Ser Gln Leu Asn Lys Lys Leu Glu Ala Ile Lys Ser
    850                 855                 860

Gln Asp Arg Phe Phe Ala Gly Phe Leu Leu Ser Gly Phe Lys Lys Ser
865                 870                 875                 880

Ser Leu Val Lys Phe Asn Ile Cys Thr Asp Ser Ser Asn Cys His Pro
                885                 890                 895

Ala Gly Glu Phe Tyr Leu Leu Gly Asp Glu Asn Glu Met Pro Trp Ala
            900                 905                 910
```

```
Tyr Asp Arg Val Phe Lys Tyr Asp Ile Thr Glu Lys Leu His Asp Leu
        915                 920                 925

Lys Leu His Ala Glu Asp His Phe Tyr Ile Asp Tyr Glu Val Phe Asp
    930                 935                 940

Leu Lys Pro Ala Ser Leu Gly Lys Asp Leu Phe Lys Gln Pro Ser Val
945                 950                 955                 960

Ile His Glu Pro Arg Ile Gly His His Glu Gly Glu Val Tyr Gln Ala
                965                 970                 975

Glu Val Thr Ser Ala Asn Arg Ile Arg Lys Asn Ile Glu Asn Leu Ser
            980                 985                 990

Leu Gly Glu Leu Glu Ser Leu Arg  Ala Ala Phe Leu Glu  Ile Glu Asn
        995                 1000                 1005

Asp Gly  Thr Tyr Glu Ser Ile  Ala Lys Phe His Gly  Ser Pro Gly
    1010                 1015                 1020

Leu Cys  Gln Leu Asn Gly Asn  Pro Ile Ser Cys Cys  Val His Gly
    1025                 1030                 1035

Met Pro  Thr Phe Pro His Trp  His Arg Leu Tyr Val  Val Val Val
    1040                 1045                 1050

Glu Asn  Ala Leu Leu Lys Lys  Gly Ser Ser Val Ala  Val Pro Tyr
    1055                 1060                 1065

Trp Asp  Trp Thr Lys Arg Ile  Glu His Leu Pro His  Leu Ile Ser
    1070                 1075                 1080

Asp Ala  Thr Tyr Tyr Asn Ser  Arg Gln His His Tyr  Glu Thr Asn
    1085                 1090                 1095

Pro Phe  His His Gly Lys Ile  Thr His Glu Asn Glu  Ile Thr Thr
    1100                 1105                 1110

Arg Asp  Pro Lys Asp Ser Leu  Phe His Ser Asp Tyr  Phe Tyr Glu
    1115                 1120                 1125

Gln Val  Leu Tyr Ala Leu Glu  Gln Asp Asn Phe Cys  Asp Phe Glu
    1130                 1135                 1140

Ile Gln  Leu Glu Ile Leu His  Asn Ala Leu His Ser  Leu Leu Gly
```

1145 1150 1155

Gly Lys Gly Lys Tyr Ser Met Ser Asn Leu Asp Tyr Ala Ala Phe
1160 1165 1170

Asp Pro Val Phe Phe Leu His His Ala Thr Thr Asp Arg Ile Trp
1175 1180 1185

Ala Ile Trp Gln Asp Leu Gln Arg Phe Arg Lys Arg Pro Tyr Arg
1190 1195 1200

Glu Ala Asn Cys Ala Ile Gln Leu Met His Thr Pro Leu Gln Pro
1205 1210 1215

Phe Asp Lys Ser Asp Asn Asn Asp Glu Ala Thr Lys Thr His Ala
1220 1225 1230

Thr Pro His Asp Gly Phe Glu Tyr Gln Asn Ser Phe Gly Tyr Ala
1235 1240 1245

Tyr Asp Asn Leu Glu Leu Asn His Tyr Ser Ile Pro Gln Leu Asp
1250 1255 1260

His Met Leu Gln Glu Arg Lys Arg His Asp Arg Val Phe Ala Gly
1265 1270 1275

Phe Leu Leu His Asn Ile Gly Thr Ser Ala Asp Gly His Val Phe
1280 1285 1290

Val Cys Leu Pro Thr Gly Glu His Thr Lys Asp Cys Ser His Glu
1295 1300 1305

Ala Gly Met Phe Ser Ile Leu Gly Gly Gln Thr Glu Met Ser Phe
1310 1315 1320

Val Phe Asp Arg Leu Tyr Lys Leu Asp Ile Thr Lys Ala Leu Lys
1325 1330 1335

Lys Asn Gly Val His Leu Gln Gly Asp Phe Asp Leu Glu Ile Glu
1340 1345 1350

Ile Thr Ala Val Asn Gly Ser His Leu Asp Ser His Val Ile His
1355 1360 1365

Ser Pro Thr Ile Leu Phe Glu Ala Gly Thr Asp Ser Ala His Thr
1370 1375 1380

```
Asp Asp  Gly His Thr Glu Pro  Val Met Ile Arg Lys  Asp Ile Thr
    1385                 1390                 1395

Gln Leu  Asp Lys Arg Gln Gln  Leu Ser Leu Val Lys  Ala Leu Glu
    1400                 1405                 1410

Ser Met  Lys Ala Asp His Ser  Ser Asp Gly Phe Gln  Ala Ile Ala
    1415                 1420                 1425

Ser Phe  His Ala Leu Pro Pro  Leu Cys Pro Ser Pro  Ala Ala Ser
    1430                 1435                 1440

Lys Arg  Phe Ala Cys Cys Val  His Gly Met Ala Thr  Phe Pro Gln
    1445                 1450                 1455

Trp His  Arg Leu Tyr Thr Val  Gln Phe Gln Asp Ser  Leu Arg Lys
    1460                 1465                 1470

His Gly  Ala Val Val Gly Leu  Pro Tyr Trp Asp Trp  Thr Leu Pro
    1475                 1480                 1485

Arg Ser  Glu Leu Pro Glu Leu  Leu Thr Val Ser Thr  Ile His Asp
    1490                 1495                 1500

Pro Glu  Thr Gly Arg Asp Ile  Pro Asn Pro Phe Ile  Gly Ser Lys
    1505                 1510                 1515

Ile Glu  Phe Glu Gly Glu Asn  Val His Thr Lys Arg  Asp Ile Asn
    1520                 1525                 1530

Arg Asp  Arg Leu Phe Gln Gly  Ser Thr Lys Thr His  His Asn Trp
    1535                 1540                 1545

Phe Ile  Glu Gln Ala Leu Leu  Ala Leu Glu Gln Thr  Asn Tyr Cys
    1550                 1555                 1560

Asp Phe  Glu Val Gln Phe Glu  Ile Met His Asn Gly  Val His Thr
    1565                 1570                 1575

Trp Val  Gly Gly Lys Glu Pro  Tyr Gly Ile Gly His  Leu His Tyr
    1580                 1585                 1590

Ala Ser  Tyr Asp Pro Leu Phe  Tyr Ile His His Ser  Gln Thr Asp
    1595                 1600                 1605

Arg Ile  Trp Ala Ile Trp Gln  Ser Leu Gln Arg Phe  Arg Gly Leu
    1610                 1615                 1620
```

Ser Gly Ser Glu Ala Asn Cys Ala Val Asn Leu Met Lys Thr Pro
1625 1630 1635

Leu Lys Pro Phe Ser Phe Gly Ala Pro Tyr Asn Leu Asn Asp His
1640 1645 1650

Thr His Asp Phe Ser Lys Pro Glu Asp Thr Phe Asp Tyr Gln Lys
1655 1660 1665

Phe Gly Tyr Ile Tyr Asp Thr Leu Glu Phe Ala Gly Trp Ser Ile
1670 1675 1680

Arg Gly Ile Asp His Ile Val Arg Asn Arg Gln Glu His Ser Arg
1685 1690 1695

Val Phe Ala Gly Phe Leu Leu Glu Gly Phe Gly Thr Ser Ala Thr
1700 1705 1710

Val Asp Phe Gln Val Cys Arg Thr Ala Gly Asp Cys Glu Asp Ala
1715 1720 1725

Gly Tyr Phe Thr Val Leu Gly Gly Glu Lys Glu Met Pro Trp Ala
1730 1735 1740

Phe Asp Arg Leu Tyr Lys Tyr Asp Ile Thr Glu Thr Leu Asp Lys
1745 1750 1755

Met Asn Leu Arg His Asp Glu Ile Phe Gln Ile Glu Val Thr Ile
1760 1765 1770

Thr Ser Tyr Asp Gly Thr Val Leu Asp Ser Gly Leu Ile Pro Thr
1775 1780 1785

Pro Ser Ile Ile Tyr Asp Pro Ala His His Asp Ile Ser Ser His
1790 1795 1800

His Leu Ser Leu Asn Lys Val Arg His Asp Leu Ser Thr Leu Ser
1805 1810 1815

Glu Arg Asp Ile Gly Ser Leu Lys Tyr Ala Leu Ser Ser Leu Gln
1820 1825 1830

Ala Asp Thr Ser Ala Asp Gly Phe Ala Ala Ile Ala Ser Phe His
1835 1840 1845

Gly Leu Pro Ala Lys Cys Asn Asp Ser His Asn Asn Glu Val Ala
1850 1855 1860

Cys Cys Ile His Gly Met Pro Thr Phe Pro His Trp His Arg Leu
1865 1870 1875

Tyr Thr Leu Gln Phe Glu Gln Ala Leu Arg Arg His Gly Ser Ser
1880 1885 1890

Val Ala Val Pro Tyr Trp Asp Trp Thr Lys Pro Ile His Asn Ile
1895 1900 1905

Pro His Leu Phe Thr Asp Lys Glu Tyr Tyr Asp Val Trp Arg Asn
1910 1915 1920

Lys Val Met Pro Asn Pro Phe Ala Arg Gly Tyr Val Pro Ser His
1925 1930 1935

Asp Thr Tyr Thr Val Arg Asp Val Gln Glu Gly Leu Phe His Leu
1940 1945 1950

Thr Ser Thr Gly Glu His Ser Ala Leu Leu Asn Gln Ala Leu Leu
1955 1960 1965

Ala Leu Glu Gln His Asp Tyr Cys Asp Phe Ala Val Gln Phe Glu
1970 1975 1980

Val Met His Asn Thr Ile His Tyr Leu Val Gly Gly Pro Gln Val
1985 1990 1995

Tyr Ser Leu Ser Ser Leu His Tyr Ala Ser Tyr Asp Pro Ile Phe
2000 2005 2010

Phe Ile His His Ser Phe Val Asp Lys Val Trp Ala Val Trp Gln
2015 2020 2025

Ala Leu Gln Glu Lys Arg Gly Leu Pro Ser Asp Arg Ala Asp Cys
2030 2035 2040

Ala Val Ser Leu Met Thr Gln Asn Met Arg Pro Phe His Tyr Glu
2045 2050 2055

Ile Asn His Asn Gln Phe Thr Lys Lys His Ala Val Pro Asn Asp
2060 2065 2070

Val Phe Lys Tyr Glu Leu Leu Gly Tyr Arg Tyr Asp Asn Leu Glu
2075 2080 2085

Ile Gly Gly Met Asn Leu His Glu Ile Glu Lys Glu Ile Lys Asp

2090 2095 2100

Lys Gln His His Val Arg Val Phe Ala Gly Phe Leu Leu His Gly
2105 2110 2115

Ile Arg Thr Ser Ala Asp Val Gln Phe Gln Ile Cys Lys Thr Ser
2120 2125 2130

Glu Asp Cys His His Gly Gly Gln Ile Phe Val Leu Gly Gly Thr
2135 2140 2145

Lys Glu Met Ala Trp Ala Tyr Asn Arg Leu Phe Lys Tyr Asp Ile
2150 2155 2160

Thr His Ala Leu His Asp Ala His Ile Thr Pro Glu Asp Val Phe
2165 2170 2175

His Pro Ser Glu Pro Phe Phe Ile Lys Val Ser Val Thr Ala Val
2180 2185 2190

Asn Gly Thr Val Leu Pro Ala Ser Ile Leu His Ala Pro Thr Ile
2195 2200 2205

Ile Tyr Glu Pro Gly Leu Gly Asp His His Glu Asp His His Ser
2210 2215 2220

Ser Ser Met Ala Gly His Gly Val Arg Lys Glu Ile Asn Thr Leu
2225 2230 2235

Thr Thr Ala Glu Val Asp Asn Leu Lys Asp Ala Met Arg Ala Val
2240 2245 2250

Met Ala Asp His Gly Pro Asn Gly Tyr Gln Ala Ile Ala Ala Phe
2255 2260 2265

His Gly Asn Pro Pro Met Cys Pro Met Pro Asp Gly Lys Asn Tyr
2270 2275 2280

Ser Cys Cys Thr His Gly Met Ala Thr Phe Pro His Trp His Arg
2285 2290 2295

Leu Tyr Thr Lys Gln Met Glu Asp Ala Leu Thr Ala His Gly Ala
2300 2305 2310

Arg Val Gly Leu Pro Tyr Trp Asp Gly Thr Thr Ala Phe Thr Ala
2315 2320 2325

```
Leu Pro  Thr Phe Val Thr Asp  Glu Glu Asp Asn Pro  Phe His His
    2330                 2335                 2340

Gly His  Ile Asp Tyr Leu Gly  Val Asp Thr Thr Arg  Ser Pro Arg
    2345                 2350                 2355

Asp Lys  Leu Phe Asn Asp Pro  Glu Arg Gly Ser Glu  Ser Phe Phe
    2360                 2365                 2370

Tyr Arg  Gln Val Leu Leu Ala  Leu Glu Gln Thr Asp  Phe Cys Gln
    2375                 2380                 2385

Phe Glu  Val Gln Phe Glu Ile  Thr His Asn Ala Ile  His Ser Trp
    2390                 2395                 2400

Thr Gly  Gly Leu Thr Pro Tyr  Gly Met Ser Thr Leu  Glu Tyr Thr
    2405                 2410                 2415

Thr Tyr  Asp Pro Leu Phe Trp  Leu His His Ala Asn  Thr Asp Arg
    2420                 2425                 2430

Ile Trp  Ala Ile Trp Gln Ala  Leu Gln Glu Tyr Arg  Gly Leu Pro
    2435                 2440                 2445

Tyr Asp  His Ala Asn Cys Glu  Ile Gln Ala Met Lys  Arg Pro Leu
    2450                 2455                 2460

Arg Pro  Phe Ser Asp Pro Ile  Asn His Asn Ala Phe  Thr His Ser
    2465                 2470                 2475

Asn Ala  Lys Pro Thr Asp Val  Phe Glu Tyr Ser Arg  Phe Asn Phe
    2480                 2485                 2490

Gln Tyr  Asp Asn Leu Arg Phe  His Gly Met Thr Ile  Lys Lys Leu
    2495                 2500                 2505

Glu His  Glu Leu Glu Lys Gln  Lys Glu Glu Asp Arg  Thr Phe Ala
    2510                 2515                 2520

Ala Phe  Leu Leu His Gly Ile  Lys Lys Ser Ala Asp  Val Ser Phe
    2525                 2530                 2535

Asp Val  Cys Asn His Asp Gly  Glu Cys His Phe Ala  Gly Thr Phe
    2540                 2545                 2550

Ala Ile  Leu Gly Gly Glu His  Glu Met Pro Trp Ser  Phe Asp Arg
    2555                 2560                 2565
```

```
Leu Phe  Arg Tyr Asp Ile Thr  Gln Val Leu Lys Gln  Met His Leu
    2570                 2575                 2580

Glu Tyr  Asp Ser Asp Phe Thr  Phe His Met Arg Ile  Ile Asp Thr
    2585                 2590                 2595

Ser Gly  Lys Gln Leu Pro Ser  Asp Leu Ile Lys Met  Pro Thr Val
    2600                 2605                 2610

Glu His  Ser Pro Gly Gly Lys  His His Glu Lys His  His Glu Asp
    2615                 2620                 2625

His His  Glu Asp Ile Leu Val  Arg Lys Asn Ile His  Ser Leu Ser
    2630                 2635                 2640

His His  Glu Ala Glu Glu Leu  Arg Asp Ala Leu Tyr  Lys Leu Gln
    2645                 2650                 2655

Asn Asp  Glu Ser His Gly Gly  Tyr Glu His Ile Ala  Gly Phe His
    2660                 2665                 2670

Gly Tyr  Pro Asn Leu Cys Pro  Glu Lys Gly Asp Glu  Lys Tyr Pro
    2675                 2680                 2685

Cys Cys  Val His Gly Met Ser  Ile Phe Pro His Trp  His Arg Leu
    2690                 2695                 2700

His Thr  Ile Gln Phe Glu Arg  Ala Leu Lys Lys His  Gly Ser His
    2705                 2710                 2715

Leu Gly  Ile Pro Tyr Trp Asp  Trp Thr Gln Thr Ile  Ser Ser Leu
    2720                 2725                 2730

Pro Thr  Phe Phe Ala Asp Ser  Gly Asn Asn Asn Pro  Phe Phe Lys
    2735                 2740                 2745

Tyr His  Ile Arg Ser Ile Asn  Gln Asp Thr Val Arg  Asp Val Asn
    2750                 2755                 2760

Glu Ala  Ile Phe Gln Gln Thr  Lys Phe Gly Glu Phe  Ser Ser Ile
    2765                 2770                 2775

Phe Tyr  Leu Ala Leu Gln Ala  Leu Glu Glu Asp Asn  Tyr Cys Asp
    2780                 2785                 2790

Phe Glu  Val Gln Tyr Glu Ile  Leu His Asn Glu Val  His Ala Leu
    2795                 2800                 2805
```

Ile Gly Gly Ala Glu Lys Tyr Ser Met Ser Thr Leu Glu Tyr Ser
2810 2815 2820

Ala Phe Asp Pro Tyr Phe Met Ile His His Ala Ser Leu Asp Lys
2825 2830 2835

Ile Trp Ile Ile Trp Gln Glu Leu Gln Lys Arg Arg Val Lys Pro
2840 2845 2850

Ala His Ala Gly Ser Cys Ala Gly Asp Ile Met His Val Pro Leu
2855 2860 2865

His Pro Phe Asn Tyr Glu Ser Val Asn Asn Asp Asp Phe Thr Arg
2870 2875 2880

Glu Asn Ser Leu Pro Asn Ala Val Val Asp Ser His Arg Phe Asn
2885 2890 2895

Tyr Lys Tyr Asp Asn Leu Asn Leu His Gly His Asn Ile Glu Glu
2900 2905 2910

Leu Glu Glu Val Leu Arg Ser Leu Arg Leu Lys Ser Arg Val Phe
2915 2920 2925

Ala Gly Phe Val Leu Ser Gly Ile Arg Thr Thr Ala Val Val Lys
2930 2935 2940

Val Tyr Ile Lys Ser Gly Thr Asp Ser Asp Asp Glu Tyr Ala Gly
2945 2950 2955

Ser Phe Val Ile Leu Gly Gly Ala Lys Glu Met Pro Trp Ala Tyr
2960 2965 2970

Glu Arg Leu Tyr Arg Phe Asp Ile Thr Glu Thr Val His Asn Leu
2975 2980 2985

Asn Leu Thr Asp Asp His Val Lys Phe Arg Phe Asp Leu Lys Lys
2990 2995 3000

Tyr Asp His Thr Glu Leu Asp Ala Ser Val Leu Pro Ala Pro Ile
3005 3010 3015

Ile Val Arg Arg Pro Asn Asn Ala Val Phe Asp Ile Ile Glu Ile
3020 3025 3030

Pro Ile Gly Lys Asp Val Asn Leu Pro Pro Lys Val Val Val Lys

```
    3035                3040                3045

Arg Gly Thr Lys Ile Met Phe Met Ser Val Asp Glu Ala Val Thr
    3050                3055                3060

Thr Pro Met Leu Asn Leu Gly Ser Tyr Thr Ala Met Phe Lys Cys
    3065                3070                3075

Lys Val Pro Pro Phe Ser Phe His Ala Phe Glu Leu Gly Lys Met
    3080                3085                3090

Tyr Ser Val Glu Ser Gly Asp Tyr Phe Met Thr Ala Ser Thr Thr
    3095                3100                3105

Glu Leu Cys Asn Asp Asn Asn Leu Arg Ile His Val His Val Asp
    3110                3115                3120

Asp Glu
    3125
```

<210> 2
<211> 3421
<212> PRT
<213> Megathura crenulata

<400> 2

```
Met Trp Thr Ile Leu Ala Leu Leu Thr Ala Thr Leu Leu Phe Glu Gly
1               5                   10                  15

Ala Phe Ser Val Asp Thr Val Val Arg Lys Asn Val Asp Ser Leu Ser
            20                  25                  30

Ser Asp Glu Val Leu Ala Leu Glu Lys Ala Leu Asp Asp Leu Gln Gln
        35                  40                  45

Asp Asp Ser Asn Gln Gly Tyr Gln Ala Ile Ala Gly Tyr His Gly Val
    50                  55                  60

Pro Thr Met Cys Val Asp Lys His Glu Lys Asn Val Ala Cys Cys Leu
65                  70                  75                  80

His Gly Met Pro Ser Phe Pro Leu Trp His Arg Leu Tyr Val Val Gln
                85                  90                  95

Leu Glu Arg Ala Leu Ile Arg Lys Lys Ala Thr Ile Ser Ile Pro Tyr
            100                 105                 110

Trp Asp Trp Thr Ser Glu Leu Thr His Leu Pro Glu Leu Val Ser His
```

```
        115                 120                 125

Pro Leu Phe Val Gly Thr Glu Gly Gly Lys Ala His Asp Asn Ser Trp
    130                 135                 140

Tyr Arg Ala Asp Ile Thr Phe Leu Asn Lys Lys Thr Ser Arg Ala Val
145                 150                 155                 160

Asp Asp Arg Leu Phe Glu Lys Val Gln Pro Gly His His Thr Arg Leu
                165                 170                 175

Met Glu Gly Ile Leu Asp Ala Leu Glu Gln Asp Glu Phe Cys Lys Phe
            180                 185                 190

Glu Ile Gln Phe Glu Leu Ala His Asn Ala Ile His Tyr Leu Val Gly
        195                 200                 205

Gly Arg His Thr Tyr Ser Met Ser His Leu Glu Tyr Thr Ser Tyr Asp
    210                 215                 220

Pro Leu Phe Phe Leu His His Ser Asn Thr Asp Arg Ile Phe Ala Ile
225                 230                 235                 240

Trp Gln Arg Leu Gln Gln Leu Arg Gly Lys Asp Pro Asn Ser Ala Asp
                245                 250                 255

Cys Ala His Asn Leu Ile His Thr Pro Met Glu Pro Phe Asp Arg Asp
            260                 265                 270

Thr Asn Pro Leu Asp Leu Thr Arg Glu His Ala Lys Pro Ala Asp Ser
        275                 280                 285

Phe Asp Tyr Gly Arg Leu Gly Tyr Gln Tyr Asp Asp Leu Ser Leu Asn
    290                 295                 300

Gly Met Ser Pro Glu Glu Leu Asn Val Tyr Leu Gly Glu Arg Ala Ala
305                 310                 315                 320

Lys Glu Arg Thr Phe Ala Ser Phe Ile Leu Ser Gly Phe Gly Gly Ser
                325                 330                 335

Ala Asn Val Val Val Tyr Val Cys Arg Pro Ala His Asp Glu Ile Ser
            340                 345                 350

Asp Asp Gln Cys Ile Lys Ala Gly Asp Phe Phe Leu Leu Gly Gly Pro
        355                 360                 365
```

Thr Glu Met Lys Trp Gly Phe Tyr Arg Ala Tyr His Phe Asp Val Thr
370 375 380

Asp Ser Val Ala Ser Ile Asp Asp Asp Gly His Gly His Tyr Tyr Val
385 390 395 400

Lys Ser Glu Leu Phe Ser Val Asn Gly Ser Ala Leu Ser Asn Asp Ile
405 410 415

Leu Arg Gln Pro Thr Leu Val His Arg Pro Ala Lys Gly His Phe Asp
420 425 430

Lys Pro Pro Val Pro Val Ala Gln Ala Asn Leu Ala Val Arg Lys Asn
435 440 445

Ile Asn Asp Leu Thr Ala Glu Glu Thr Tyr Ser Leu Arg Lys Ala Met
450 455 460

Glu Arg Phe Gln Asn Asp Lys Ser Val Asp Gly Tyr Gln Ala Thr Val
465 470 475 480

Glu Phe His Ala Leu Pro Ala Arg Cys Pro Arg Pro Asp Ala Lys Asp
485 490 495

Arg Phe Ala Cys Cys Val His Gly Met Ala Thr Phe Pro His Trp His
500 505 510

Arg Leu Phe Val Thr Gln Val Glu Asp Ala Leu Leu Arg Arg Gly Ser
515 520 525

Thr Ile Gly Leu Pro Asn Trp Asp Trp Thr Met Pro Met Asp His Leu
530 535 540

Pro Glu Leu Ala Thr Ser Glu Thr Tyr Leu Asp Pro Val Thr Gly Glu
545 550 555 560

Thr Lys Asn Asn Pro Phe His His Ala Gln Val Ala Phe Glu Asn Gly
565 570 575

Val Thr Ser Arg Asn Pro Asp Ala Lys Leu Phe Met Lys Pro Thr Tyr
580 585 590

Gly Asp His Thr Tyr Leu Phe Asp Ser Met Ile Tyr Ala Phe Glu Gln
595 600 605

Glu Asp Phe Cys Asp Phe Glu Val Gln Tyr Glu Leu Thr His Asn Ala
610 615 620

```
Ile His Ala Trp Val Gly Gly Ser Glu Lys Tyr Ser Met Ser Ser Leu
625                 630                 635                 640

His Tyr Thr Ala Phe Asp Pro Ile Phe Tyr Leu His His Ser Asn Val
                645                 650                 655

Asp Arg Leu Trp Ala Ile Trp Gln Ala Leu Gln Ile Arg Arg Gly Lys
            660                 665                 670

Ser Tyr Lys Ala His Cys Ala Ser Ser Gln Glu Arg Glu Pro Leu Lys
        675                 680                 685

Pro Phe Ala Phe Ser Ser Pro Leu Asn Asn Asn Glu Lys Thr Tyr His
    690                 695                 700

Asn Ser Val Pro Thr Asn Val Tyr Asp Tyr Val Gly Val Leu His Tyr
705                 710                 715                 720

Arg Tyr Asp Asp Leu Gln Phe Gly Gly Met Thr Met Ser Glu Leu Glu
                725                 730                 735

Glu Tyr Ile His Lys Gln Thr Gln His Asp Arg Thr Phe Ala Gly Phe
            740                 745                 750

Phe Leu Ser Tyr Ile Gly Thr Ser Ala Ser Val Asp Ile Phe Ile Asn
        755                 760                 765

Arg Glu Gly His Asp Lys Tyr Lys Val Gly Ser Phe Val Val Leu Gly
    770                 775                 780

Gly Ser Lys Glu Met Lys Trp Gly Phe Asp Arg Met Tyr Lys Tyr Glu
785                 790                 795                 800

Ile Thr Glu Ala Leu Lys Thr Leu Asn Val Ala Val Asp Asp Gly Phe
                805                 810                 815

Ser Ile Thr Val Glu Ile Thr Asp Val Asp Gly Ser Pro Pro Ser Ala
            820                 825                 830

Asp Leu Ile Pro Pro Pro Ala Ile Ile Phe Glu Arg Ala Asp Ala Lys
        835                 840                 845

Asp Phe Gly His Ser Arg Lys Ile Arg Lys Ala Val Asp Ser Leu Thr
    850                 855                 860

Val Glu Glu Gln Thr Ser Leu Arg Arg Ala Met Ala Asp Leu Gln Asp
865                 870                 875                 880
```

```
Asp Lys Thr Ser Gly Gly Phe Gln Gln Ile Ala Ala Phe His Gly Glu
                885                 890                 895

Pro Lys Trp Cys Pro Ser Pro Glu Ala Glu Lys Lys Phe Ala Cys Cys
            900                 905                 910

Val His Gly Met Ala Val Phe Pro His Trp His Arg Leu Leu Thr Val
        915                 920                 925

Gln Gly Glu Asn Ala Leu Arg Lys His Gly Phe Thr Gly Gly Leu Pro
    930                 935                 940

Tyr Trp Asp Trp Thr Arg Ser Met Ser Ala Leu Pro His Phe Val Ala
945                 950                 955                 960

Asp Pro Thr Tyr Asn Asp Ala Ile Ser Ser Gln Glu Glu Asp Asn Pro
                965                 970                 975

Trp His His Gly His Ile Asp Ser Val Gly His Asp Thr Thr Arg Asp
            980                 985                 990

Val Arg Asp Asp Leu Tyr Gln Ser  Pro Gly Phe Gly His  Tyr Thr Asp
        995                 1000                 1005

Ile Ala  Lys Gln Val Leu Leu  Ala Phe Glu Gln Asp  Asp Phe Cys
    1010                 1015                 1020

Asp Phe  Glu Val Gln Phe Glu  Ile Ala His Asn Phe  Ile His Ala
    1025                 1030                 1035

Leu Val  Gly Gly Asn Glu Pro  Tyr Ser Met Ser Ser  Leu Arg Tyr
    1040                 1045                 1050

Thr Thr  Tyr Asp Pro Ile Phe  Phe Leu His Arg Ser  Asn Thr Asp
    1055                 1060                 1065

Arg Leu  Trp Ala Ile Trp Gln  Ala Leu Gln Lys Tyr  Arg Gly Lys
    1070                 1075                 1080

Pro Tyr  Asn Thr Ala Asn Cys  Ala Ile Ala Ser Met  Arg Lys Pro
    1085                 1090                 1095

Leu Gln  Pro Phe Gly Leu Asp  Ser Val Ile Asn Pro  Asp Asp Glu
    1100                 1105                 1110

Thr Arg  Glu His Ser Val Pro  Phe Arg Val Phe Asp  Tyr Lys Asn
```

1115 1120 1125

Asn Phe Asp Tyr Glu Tyr Glu Ser Leu Ala Phe Asn Gly Leu Ser
1130 1135 1140

Ile Ala Gln Leu Asp Arg Glu Leu Gln Arg Arg Lys Ser His Asp
1145 1150 1155

Arg Val Phe Ala Gly Phe Leu Leu His Glu Ile Gly Gln Ser Ala
1160 1165 1170

Leu Val Lys Phe Tyr Val Cys Lys His Asn Val Ser Asp Cys Asp
1175 1180 1185

His Tyr Ala Gly Glu Phe Tyr Ile Leu Gly Asp Glu Ala Glu Met
1190 1195 1200

Pro Trp Arg Tyr Asp Arg Val Tyr Lys Tyr Glu Ile Thr Gln Gln
1205 1210 1215

Leu His Asp Leu Asp Leu His Val Gly Asp Asn Phe Phe Leu Lys
1220 1225 1230

Tyr Glu Ala Phe Asp Leu Asn Gly Gly Ser Leu Gly Gly Ser Ile
1235 1240 1245

Phe Ser Gln Pro Ser Val Ile Phe Glu Pro Ala Ala Gly Ser His
1250 1255 1260

Gln Ala Asp Glu Tyr Arg Glu Ala Val Thr Ser Ala Ser His Ile
1265 1270 1275

Arg Lys Asn Ile Arg Asp Leu Ser Glu Gly Glu Ile Glu Ser Ile
1280 1285 1290

Arg Ser Ala Phe Leu Gln Ile Gln Lys Glu Gly Ile Tyr Glu Asn
1295 1300 1305

Ile Ala Lys Phe His Gly Lys Pro Gly Leu Cys Glu His Asp Gly
1310 1315 1320

His Pro Val Ala Cys Cys Val His Gly Met Pro Thr Phe Pro His
1325 1330 1335

Trp His Arg Leu Tyr Val Leu Gln Val Glu Asn Ala Leu Leu Glu
1340 1345 1350

Arg Gly Ser Ala Val Ala Val Pro Tyr Trp Asp Trp Thr Glu Lys
1355 1360 1365

Ala Asp Ser Leu Pro Ser Leu Ile Asn Asp Ala Thr Tyr Phe Asn
1370 1375 1380

Ser Arg Ser Gln Thr Phe Asp Pro Asn Pro Phe Phe Arg Gly His
1385 1390 1395

Ile Ala Phe Glu Asn Ala Val Thr Ser Arg Asp Pro Gln Pro Glu
1400 1405 1410

Leu Trp Asp Asn Lys Asp Phe Tyr Glu Asn Val Met Leu Ala Leu
1415 1420 1425

Glu Gln Asp Asn Phe Cys Asp Phe Glu Ile Gln Leu Glu Leu Ile
1430 1435 1440

His Asn Ala Leu His Ser Arg Leu Gly Gly Arg Ala Lys Tyr Ser
1445 1450 1455

Leu Ser Ser Leu Asp Tyr Thr Ala Phe Asp Pro Val Phe Phe Leu
1460 1465 1470

His His Ala Asn Val Asp Arg Ile Trp Ala Ile Trp Gln Asp Leu
1475 1480 1485

Gln Arg Tyr Arg Lys Lys Pro Tyr Asn Glu Ala Asp Cys Ala Val
1490 1495 1500

Asn Glu Met Arg Lys Pro Leu Gln Pro Phe Asn Asn Pro Glu Leu
1505 1510 1515

Asn Ser Asp Ser Met Thr Leu Lys His Asn Leu Pro Gln Asp Ser
1520 1525 1530

Phe Asp Tyr Gln Asn Arg Phe Arg Tyr Gln Tyr Asp Asn Leu Gln
1535 1540 1545

Phe Asn His Phe Ser Ile Gln Lys Leu Asp Gln Thr Ile Gln Ala
1550 1555 1560

Arg Lys Gln His Asp Arg Val Phe Ala Gly Phe Ile Leu His Asn
1565 1570 1575

Ile Gly Thr Ser Ala Val Val Asp Ile Tyr Ile Cys Val Glu Gln
1580 1585 1590

```
Gly Gly  Glu Gln Asn Cys Lys  Thr Lys Ala Gly Ser  Phe Thr Ile
    1595                 1600                 1605

Leu Gly  Gly Glu Thr Glu Met  Pro Phe His Phe Asp  Arg Leu Tyr
    1610                 1615                 1620

Lys Phe  Asp Ile Thr Ser Ala  Leu His Lys Leu Gly  Val Pro Leu
    1625                 1630                 1635

Asp Gly  His Gly Phe Asp Ile  Lys Val Asp Val Arg  Ala Val Asn
    1640                 1645                 1650

Gly Ser  His Leu Asp Gln His  Ile Leu Asn Glu Pro  Ser Leu Leu
    1655                 1660                 1665

Phe Val  Pro Gly Glu Arg Lys  Asn Ile Tyr Tyr Asp  Gly Leu Ser
    1670                 1675                 1680

Gln His  Asn Leu Val Arg Lys  Glu Val Ser Ser Leu  Thr Thr Leu
    1685                 1690                 1695

Glu Lys  His Phe Leu Arg Lys  Ala Leu Lys Asn Met  Gln Ala Asp
    1700                 1705                 1710

Asp Ser  Pro Asp Gly Tyr Gln  Ala Ile Ala Ser Phe  His Ala Leu
    1715                 1720                 1725

Pro Pro  Leu Cys Pro Ser Pro  Ser Ala Ala His Arg  His Ala Cys
    1730                 1735                 1740

Cys Leu  His Gly Met Ala Thr  Phe Pro Gln Trp His  Arg Leu Tyr
    1745                 1750                 1755

Thr Val  Gln Phe Glu Asp Ser  Leu Lys Arg His Gly  Ser Ile Val
    1760                 1765                 1770

Gly Leu  Pro Tyr Trp Asp Trp  Leu Lys Pro Gln Ser  Ala Leu Pro
    1775                 1780                 1785

Asp Leu  Val Thr Gln Glu Thr  Tyr Glu His Leu Phe  Ser His Lys
    1790                 1795                 1800

Thr Phe  Pro Asn Pro Phe Leu  Lys Ala Asn Ile Glu  Phe Glu Gly
    1805                 1810                 1815

Glu Gly  Val Thr Thr Glu Arg  Asp Val Asp Ala Glu  His Leu Phe
    1820                 1825                 1830
```

Ala Lys Gly Asn Leu Val Tyr Asn Asn Trp Phe Cys Asn Gln Ala
1835 1840 1845

Leu Tyr Ala Leu Glu Gln Glu Asn Tyr Cys Asp Phe Glu Ile Gln
1850 1855 1860

Phe Glu Ile Leu His Asn Gly Ile His Ser Trp Val Gly Gly Ser
1865 1870 1875

Lys Thr His Ser Ile Gly His Leu His Tyr Ala Ser Tyr Asp Pro
1880 1885 1890

Leu Phe Tyr Ile His His Ser Gln Thr Asp Arg Ile Trp Ala Ile
1895 1900 1905

Trp Gln Ala Leu Gln Glu His Arg Gly Leu Ser Gly Lys Glu Ala
1910 1915 1920

His Cys Ala Leu Glu Gln Met Lys Asp Pro Leu Lys Pro Phe Ser
1925 1930 1935

Phe Gly Ser Pro Tyr Asn Leu Asn Lys Arg Thr Gln Glu Phe Ser
1940 1945 1950

Lys Pro Glu Asp Thr Phe Asp Tyr His Arg Phe Gly Tyr Glu Tyr
1955 1960 1965

Asp Ser Leu Glu Phe Val Gly Met Ser Val Ser Ser Leu His Asn
1970 1975 1980

Tyr Ile Lys Gln Gln Gln Glu Ala Asp Arg Val Phe Ala Gly Phe
1985 1990 1995

Leu Leu Lys Gly Phe Gly Gln Ser Ala Ser Val Ser Phe Asp Ile
2000 2005 2010

Cys Arg Pro Asp Gln Ser Cys Gln Glu Ala Gly Tyr Phe Ser Val
2015 2020 2025

Leu Gly Gly Ser Ser Glu Met Pro Trp Gln Phe Asp Arg Leu Tyr
2030 2035 2040

Lys Tyr Asp Ile Thr Lys Thr Leu Lys Asp Met Lys Leu Arg Tyr
2045 2050 2055

Asp Asp Thr Phe Thr Ile Lys Val His Ile Lys Asp Ile Ala Gly

2060 2065 2070

Ala Glu Leu Asp Ser Asp Leu Ile Pro Thr Pro Ser Val Leu Leu
2075 2080 2085

Glu Glu Gly Lys His Gly Ile Asn Val Arg His Val Gly Arg Asn
2090 2095 2100

Arg Ile Arg Met Glu Leu Ser Glu Leu Thr Glu Arg Asp Leu Ala
2105 2110 2115

Ser Leu Lys Ser Ala Met Arg Ser Leu Gln Ala Asp Asp Gly Val
2120 2125 2130

Asn Gly Tyr Gln Ala Ile Ala Ser Phe His Gly Leu Pro Ala Ser
2135 2140 2145

Cys His Asp Asp Glu Gly His Glu Ile Ala Cys Cys Ile His Gly
2150 2155 2160

Met Pro Val Phe Pro His Trp His Arg Leu Tyr Thr Leu Gln Met
2165 2170 2175

Asp Met Ala Leu Leu Ser His Gly Ser Ala Val Ala Ile Pro Tyr
2180 2185 2190

Trp Asp Trp Thr Lys Pro Ile Ser Lys Leu Pro Asp Leu Phe Thr
2195 2200 2205

Ser Pro Glu Tyr Tyr Asp Pro Trp Arg Asp Ala Val Val Asn Asn
2210 2215 2220

Pro Phe Ala Lys Gly Tyr Ile Lys Ser Glu Asp Ala Tyr Thr Val
2225 2230 2235

Arg Asp Pro Gln Asp Ile Leu Tyr His Leu Gln Asp Glu Thr Gly
2240 2245 2250

Thr Ser Val Leu Leu Asp Gln Thr Leu Leu Ala Leu Glu Gln Thr
2255 2260 2265

Asp Phe Cys Asp Phe Glu Val Gln Phe Glu Val Val His Asn Ala
2270 2275 2280

Ile His Tyr Leu Val Gly Gly Arg Gln Val Tyr Ala Leu Ser Ser
2285 2290 2295

```
Gln His  Tyr Ala Ser Tyr Asp  Pro Ala Phe Phe Ile  His His Ser
    2300                 2305                 2310

Phe Val  Asp Lys Ile Trp Ala  Val Trp Gln Ala Leu  Gln Lys Lys
    2315                 2320                 2325

Arg Lys  Arg Pro Tyr His Lys  Ala Asp Cys Ala Leu  Asn Met Met
    2330                 2335                 2340

Thr Lys  Pro Met Arg Pro Phe  Ala His Asp Phe Asn  His Asn Gly
    2345                 2350                 2355

Phe Thr  Lys Met His Ala Val  Pro Asn Thr Leu Phe  Asp Phe Gln
    2360                 2365                 2370

Asp Leu  Phe Tyr Thr Tyr Asp  Asn Leu Glu Ile Ala  Gly Met Asn
    2375                 2380                 2385

Val Asn  Gln Leu Glu Ala Glu  Ile Asn Arg Arg Lys  Ser Gln Thr
    2390                 2395                 2400

Arg Val  Phe Ala Gly Phe Leu  Leu His Gly Ile Gly  Arg Ser Ala
    2405                 2410                 2415

Asp Val  Arg Phe Trp Ile Cys  Lys Thr Ala Asp Asp  Cys His Ala
    2420                 2425                 2430

Ser Gly  Met Ile Phe Ile Leu  Gly Gly Ser Lys Glu  Met His Trp
    2435                 2440                 2445

Ala Tyr  Asp Arg Asn Phe Lys  Tyr Asp Ile Thr Gln  Ala Leu Lys
    2450                 2455                 2460

Ala Gln  Ser Ile His Pro Glu  Asp Val Phe Asp Thr  Asp Ala Pro
    2465                 2470                 2475

Phe Phe  Ile Lys Val Glu Val  His Gly Val Asn Lys  Thr Ala Leu
    2480                 2485                 2490

Pro Ser  Ser Ala Ile Pro Ala  Pro Thr Ile Ile Tyr  Ser Ala Gly
    2495                 2500                 2505

Glu Gly  His Thr Asp Asp His  Gly Ser Asp His Ile  Ala Gly Ser
    2510                 2515                 2520

Gly Val  Arg Lys Asp Val Thr  Ser Leu Thr Ala Ser  Glu Ile Glu
    2525                 2530                 2535
```

```
Asn Leu  Arg His Ala Leu Gln  Ser Val Met Asp Asp  Asp Gly Pro
    2540                 2545                 2550

Asn Gly  Phe Gln Ala Ile Ala  Ala Tyr His Gly Ser  Pro Pro Met
    2555                 2560                 2565

Cys His  Met His Asp Gly Arg  Asp Val Ala Cys Cys  Thr His Gly
    2570                 2575                 2580

Met Ala  Ser Phe Pro His Trp  His Arg Leu Phe Val  Lys Gln Met
    2585                 2590                 2595

Glu Asp  Ala Leu Ala Ala His  Gly Ala His Ile Gly  Ile Pro Tyr
    2600                 2605                 2610

Trp Asp  Trp Thr Ser Ala Phe  Ser His Leu Pro Ala  Leu Val Thr
    2615                 2620                 2625

Asp His  Glu His Asn Pro Phe  His His Gly His Ile  Ala His Arg
    2630                 2635                 2640

Asn Val  Asp Thr Ser Arg Ser  Pro Arg Asp Met Leu  Phe Asn Asp
    2645                 2650                 2655

Pro Glu  His Gly Ser Glu Ser  Phe Phe Tyr Arg Gln  Val Leu Leu
    2660                 2665                 2670

Ala Leu  Glu Gln Thr Asp Phe  Cys Gln Phe Glu Val  Gln Phe Glu
    2675                 2680                 2685

Ile Thr  His Asn Ala Ile His  Ser Trp Thr Gly Gly  His Thr Pro
    2690                 2695                 2700

Tyr Gly  Met Ser Ser Leu Glu  Tyr Thr Ala Tyr Asp  Pro Leu Phe
    2705                 2710                 2715

Tyr Leu  His His Ser Asn Thr  Asp Arg Ile Trp Ala  Ile Trp Gln
    2720                 2725                 2730

Ala Leu  Gln Lys Tyr Arg Gly  Phe Gln Tyr Asn Ala  Ala His Cys
    2735                 2740                 2745

Asp Ile  Gln Val Leu Lys Gln  Pro Leu Lys Pro Phe  Ser Glu Ser
    2750                 2755                 2760

Arg Asn  Pro Asn Pro Val Thr  Arg Ala Asn Ser Arg  Ala Val Asp
    2765                 2770                 2775
```

```
Ser Phe Asp Tyr Glu Arg Leu Asn Tyr Gln Tyr Asp Thr Leu Thr
    2780                2785                2790

Phe His Gly His Ser Ile Ser Glu Leu Asp Ala Met Leu Gln Glu
    2795                2800                2805

Arg Lys Lys Glu Glu Arg Thr Phe Ala Ala Phe Leu Leu His Gly
    2810                2815                2820

Phe Gly Ala Ser Ala Asp Val Ser Phe Asp Val Cys Thr Pro Asp
    2825                2830                2835

Gly His Cys Ala Phe Ala Gly Thr Phe Ala Val Leu Gly Gly Glu
    2840                2845                2850

Leu Glu Met Pro Trp Ser Phe Glu Arg Leu Phe Arg Tyr Asp Ile
    2855                2860                2865

Thr Lys Val Leu Lys Gln Met Asn Leu His Tyr Asp Ser Glu Phe
    2870                2875                2880

His Phe Glu Leu Lys Ile Val Gly Thr Asp Gly Thr Glu Leu Pro
    2885                2890                2895

Ser Asp Arg Ile Lys Ser Pro Thr Ile Glu His His Gly Gly Asp
    2900                2905                2910

His His Gly Gly Asp Thr Ser Gly His Asp His Ser Glu Arg His
    2915                2920                2925

Asp Gly Phe Phe Arg Lys Glu Val Gly Ser Leu Ser Leu Asp Glu
    2930                2935                2940

Ala Asn Asp Leu Lys Asn Ala Leu Tyr Lys Leu Gln Asn Asp Gln
    2945                2950                2955

Gly Pro Asn Gly Tyr Glu Ser Ile Ala Gly Tyr His Gly Tyr Pro
    2960                2965                2970

Phe Leu Cys Pro Glu His Gly Glu Asp Gln Tyr Ala Cys Cys Val
    2975                2980                2985

His Gly Met Pro Val Phe Pro His Trp His Arg Leu His Thr Ile
    2990                2995                3000

Gln Phe Glu Arg Ala Leu Lys Glu His Gly Ser His Leu Gly Ile
```

3005 3010 3015

Pro Tyr Trp Asp Trp Thr Lys Ser Met Ile Ala Leu Pro Ala Phe
3020 3025 3030

Phe Ala Asp Ser Ser Asn Ser Asn Pro Phe Tyr Lys Tyr His Ile
3035 3040 3045

Met Lys Ala Gly His Asp Thr Ala Arg Ser Pro Ser Asp Leu Leu
3050 3055 3060

Phe Asn Gln Pro Gln Leu His Gly Tyr Asp Tyr Leu Tyr Tyr Leu
3065 3070 3075

Ala Leu Ser Thr Leu Glu Glu Asp Asn Tyr Cys Asp Phe Glu Val
3080 3085 3090

His Tyr Glu Ile Leu His Asn Ala Val His Leu Trp Leu Gly Gly
3095 3100 3105

Thr Glu Thr Tyr Ser Met Ser Ser Leu Ala Phe Ser Ala Tyr Asp
3110 3115 3120

Pro Val Phe Met Ile Leu His Ser Gly Leu Asp Arg Leu Trp Ile
3125 3130 3135

Ile Trp Gln Glu Leu Gln Lys Leu Arg Lys Lys Pro Tyr Asn Ala
3140 3145 3150

Ala Lys Cys Ala Gly His Met Met Asp Glu Pro Leu His Pro Phe
3155 3160 3165

Asn Tyr Glu Ser Ala Asn His Asp Ser Phe Thr Arg Ala Asn Ala
3170 3175 3180

Lys Pro Ser Thr Val Phe Asp Ser His Lys Phe Asn Tyr His Tyr
3185 3190 3195

Asp Asn Pro Asp Val Arg Gly Asn Ser Ile Gln Glu Ile Ser Ala
3200 3205 3210

Ile Ile His Asp Leu Arg Asn Gln Pro Arg Val Phe Ala Gly Phe
3215 3220 3225

Val Leu Ser Gly Ile Tyr Thr Ser Ala Asn Val Lys Ile Tyr Leu
3230 3235 3240

```
Val Arg Glu Gly His Asp Asp Glu Asn Val Gly Ser Phe Val Val
    3245                3250                3255

Leu Gly Gly Pro Lys Glu Met Pro Trp Ala Tyr Glu Arg Ile Phe
    3260                3265                3270

Lys Tyr Asp Ile Thr Glu Val Ala Asn Arg Leu Asn Met His His
    3275                3280                3285

Asp Asp Thr Phe Asn Phe Arg Leu Glu Val Gln Ser Tyr Thr Gly
    3290                3295                3300

Glu Met Val Thr His His Leu Pro Glu Pro Leu Ile Ile Tyr Arg
    3305                3310                3315

Pro Ala Lys Gln Glu Tyr Asp Val Leu Val Ile Pro Leu Gly Ser
    3320                3325                3330

Gly His Lys Leu Pro Pro Lys Val Ile Val Lys Arg Gly Thr Arg
    3335                3340                3345

Ile Met Phe His Pro Val Asp Asp Thr Val Asn Arg Pro Val Val
    3350                3355                3360

Asp Leu Gly Ser His Thr Ala Leu Tyr Asn Cys Val Val Pro Pro
    3365                3370                3375

Phe Thr Tyr Asn Gly Tyr Glu Leu Asp His Ala Tyr Ser Leu Arg
    3380                3385                3390

Asp Gly His Tyr Tyr Ile Ala Gly Pro Thr Lys Asp Leu Cys Thr
    3395                3400                3405

Ser Gly Asn Val Arg Ile His Ile His Ile Glu Asp Glu
    3410                3415                3420
```

## Description

### FIELD OF THE INVENTION

The present disclosure is directed to compositions and methods for cancer immunotherapy and immunogenic/therapeutic glycoconjugates able to elicit anti-cancer immune responses in particular.

BACKGROUND The carbohydrate antigen Globo H (Fuc $\alpha$ 1→2 Gal $\beta$ 1→3 GalNAc $\beta$ 1→3 Gal $\alpha$ 1 →4 Gal $\beta$ 1→4 Glc) was first isolated as a ceramide-linked Glycolipid and identified in 1984 by Hakomori et al. from breast cancer MCF-7 cells. (Bremer E G, et al. (1984) J Biol Chem 259:14773-14777). Further studies with anti-Globo H monoclonal antibodies showed that Globo H was present on many other cancers, including prostate, gastric, pancreatic, lung, ovarian and colon cancers and only minimal expression on luminal surface of normal secretory tissue which is not readily accessible to immune system. (Ragupathi G, et al. (1997) Angew Chem Int Ed 36:125-128). In addition, it has been established that the serum of breast cancer patient contains high level of anti-Globo H antibody. (Gilewski T et al. (2001) Proc Natl Acad Sci USA 98:3270-3275; Huang C- Y, et al. (2006) Proc Natl Acad Sci USA 103:15-20; Wang C-C, et al. (2008) Proc Natl Acad Sci USA 105(33):11661-11666). Patients with Globo H-positive tumors showed a shorter survival in comparison to patients with Globo H-negative tumors. (Chang, Y-J, et al. (2007) Proc Natl Acad Sci USA 104(25):10299-10304). These findings render Globo H, a hexasaccharide epitope, an attractive tumor marker and a feasible target for cancer vaccine development. Wong et al. (US 2010/0136042) refer to immunogenic compositions comprising Globo H or an immunogenic fragment thereof linked to a carrier protein by a p-nitrophenyl linker.

### SUMMARY OF THE INVENTION

While vaccines have been developed to elicit antibody responses against Globo H, their anti-cancer efficacies are unsatisfactory due to low antigenicity of Globo H. There is a need for a new vaccine capable of eliciting high levels of immune responses targeting Globo H.

KLH contains glycosylated polypeptide subunits that can assemble to form decameric (10-mer), didecameric (20-mer), and larger particles. These multimeric structures have been characterized by ultracentrifugation techniques that yield sedimentation coefficients of 11-19S for the dissociated subunits and 92-107S for the didecameric multimers. A variety of factors may affect the size distribution of molluscam hemocyanins, including KLH. These factors include ionic strength, pH, temperature, pO2, and the availability of certain divalent cations, notably calcium and magnesium.

The current inventors have developed a composition with surprising increased efficacy that is primarily comprised of dimers, trimers as well as other multimers of KLH linked to a plurality of Globo H moieties.

Accordingly, the present disclosure generally encompasses therapeutic and/or prophylactic compositions including Globo H, as well as, immunotherapeutics, vaccines, dosage forms, kits, and methods of manufacture, and treatment thereof.

In one embodiment, the invention encompasses an isolated therapeutic conjugate comprising a Globo H moiety linked to a keyhole limpet hemocyanin (KLH) moiety subunit. In certain embodiments, the linkage is a covalent bond.

In another embodiment, the invention encompasses an isolated therapeutic conjugate comprising a Globo H moiety covalently linked to a keyhole limpet hemocyanin (KLH) moiety subunit, wherein the KLH is a derivatized KLH. As used herein the term "covalently linked" when referring to Globo-H and KLH means: Globo- H is directly covalently linked to KLH, or Globo-H is covalently linked to derivatized KLH (as set forth herein), or Globo-H is covalently linked to KLH through a linker group (as set forth herein), or Globo-H is covalently linked to KLH through both a linker group and a derivatized KLH.

In certain illustrative embodiments, the derivatized KLH of the invention has the following structure:

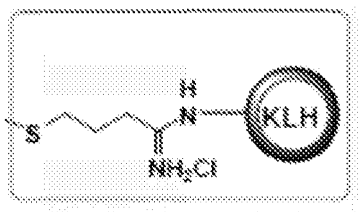

Derivatized KLH

In one embodiment, the invention encompasses an isolated therapeutic conjugate comprising a Globo H moiety covalently linked to a keyhole limpet hemocyanin (KLH) moiety subunit through a linker molecule.

In one embodiment, the Globo H moieties are bound to a lysine residue of a KLH moiety subunit.

In one embodiment, there are total of exactly or about 145, 146, 147, 148, 149,150, 151, 152, 153, 154, 155, 156, 157, 158, 159, 160 total lysine residues per KLH moiety subunit which are available for or actually directly or indirectly bind a Globo H moiety.

In another embodiment, the invention encompasses an isolated therapeutic conjugate comprising a Globo H moiety covalently linked to a keyhole limpet hemocyanin (KLH) moiety subunit via a 4-(4-N-maleimidomethyl) cyclohexane-1-carboxyl hydrazide (MMCCH) linker group. The MMCCH linker of the invention has the following structure:

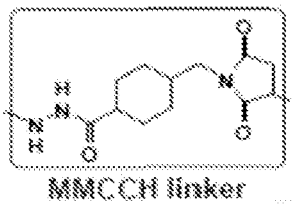

MMCCH linker .

In another illustrative embodiment, the invention encompasses an isolated therapeutic conjugate having the following general structure:

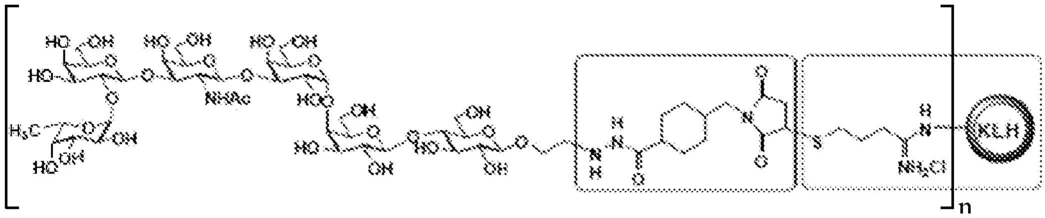

wherein n is an integer from about 1 to about 160. In certain embodiments, a monomeric KLH moiety can include from about 1 to about 160 Globo H moieties. One of ordinary skill in the art will recognize that the structures are illustrated as the iminium hydrochloride salts but can also exist or co-exist as the imine form. Accordingly, the invention encompasses both the imine as well as salts thereof including, for example, the iminium hydrochloride salt. In certain embodiments, a monomeric KLH moiety can include from about 1 to about 125 Globo H moieties. In certain embodiments, a monomeric KLH moiety can include about 1 to about 100 Globo H moieties. In certain embodiments, a monomeric KLH moiety can include from 1 to about 75 Globo H moieties. In certain embodiments, a monomeric KLH moiety can include from about 1 to about 50 Globo H moieties. In certain embodiments, a monomeric KLH moiety can include from about 1 to about 25 Globo H moieties. In certain embodiments, a monomeric KLH moiety can include from about 1 to about 10 Globo H moieties.

In certain embodiments, the Globo H moieties are conjugated to the KLH moieties covalently to certain amino acid residues. In certain embodiments, the amino acid residues can include or exclude arginine, lysine, histidine, asparagine, proline, glutamine or a combination thereof.

In another embodiment the Globo H moieties are bound to lysine conjugation sites on a monomeric KLH moiety subunit.

In another embodiment, there are exactly or about 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91,92, 93, 94, 95, 96, 97, 98, 99, 100, 101, 102, 103, 104, 105, 106, 107, 108, 109 or 110 lysine conjugation sites on each monomeric KLH moiety subunit available for binding to or actually bound to a Globo H moiety. In another embodiment, there are 62, 66, 67, 68, 70, 72, 76, 86, 87, 88, 90, 92, 93, 100 such lysine conjugation sites on each KLH moiety subunit.

In certain therapeutic composition embodiments containing a mixture of moiety subunits (e.g., KLH1 and KLH2 or variants thereof), total available lysine (for both subunits) as are counted together across the different subunit types the and may be or are exactly about 290, 291, 292, 293, 294, 295, 296, 297, 298, 299, 300, 301, 302, 303, 304, 305, 306, 307, 308, 309 or 310 in number. In such embodiments, there are exactly or about 130, 131, 132, 133, 134, 135, 136, 137, 138, 139, 140, 141, 142, 143, 144, 145, 146, 147, 148, 149, 150, 151, 152, 153, 154, 155, 156, 157, 158, 159 or 160 lysine conjugation sites together across the different subunits (e.g., KLH1 and KLH2 or variants thereof). In other such embodiments, there are 136, 137, 141, 140, 143, 147 or 155 lysine conjugation sites.

In another illustrative embodiment, the invention encompasses an isolated immunogenic/therapeutic conjugate having the following general structure:

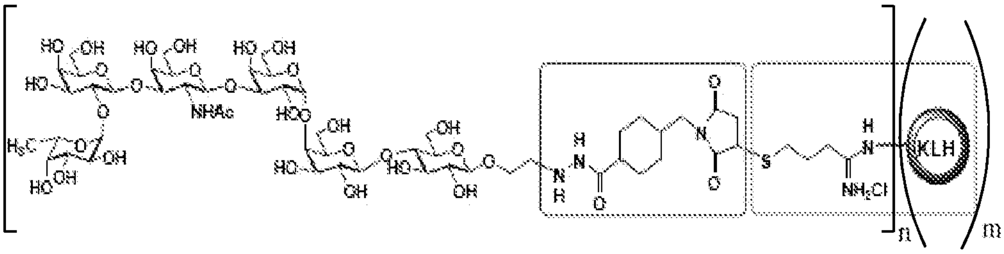

wherein n is independently an integer from about 1 to about 3000 and m is independently an integer from about 1 to about 20. In certain embodiments, when m is greater than 1, KLH moieties can aggregate to form multimeric structures. In certain embodiments, the aggregation is a covalent bond. In certain other embodiments, the aggregation is not a covalent bond (e.g., the aggregation is formed by H-bonding or hydrophobic interactions). In certain embodiments, a monomeric KLH moiety (*i.e.*, where m=1) can include from about 1 to about 160 Globo H moieties. In certain embodiments, a dimeric KLH moiety (*i.e.*, where m=2) can include from about 1 to about 300 Globo H moieties. In certain embodiments, a trimeric KLH moiety (*i.e.*, where m=3) can include from about 1 to about 450 Globo H moieties. In certain embodiments, a tetrameric KLH moiety (*i.e.*, where m=4) can include from about 1 to about 600 Globo H moieties. In certain embodiments, a pentameric KLH moiety (*i.e.*, where m=5) can include from about 1 to about 750 Globo H moieties. In certain embodiments, a hexameric KLH moiety *(i.e.,* where m=6) can include from about 1 to about 900 Globo H moieties. In certain embodiments, a didecameric KLH moiety *(i.e.,* where m=20) can include from about 1 to about 3000 Globo H moieties.

In another illustrative embodiment, the invention encompasses an isolated immunogenic/therapeutic conjugate having the following general structure:

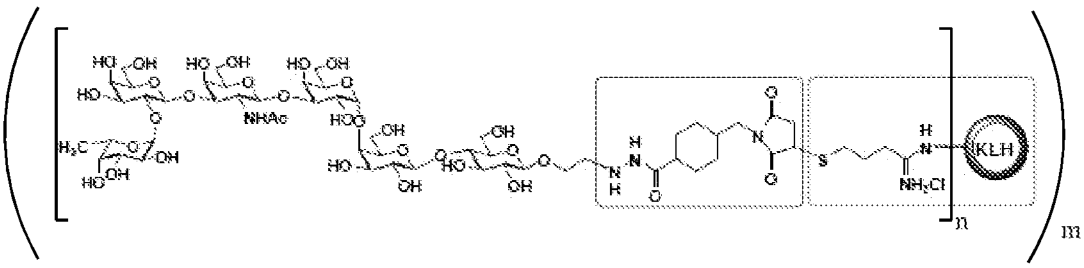

wherein n is independently an integer from about 1 to about 150 and m is independently an integer from about 1 to about 20.

In another embodiments, the invention encompasses an isolated therapeutic conjugate having the following general structure:

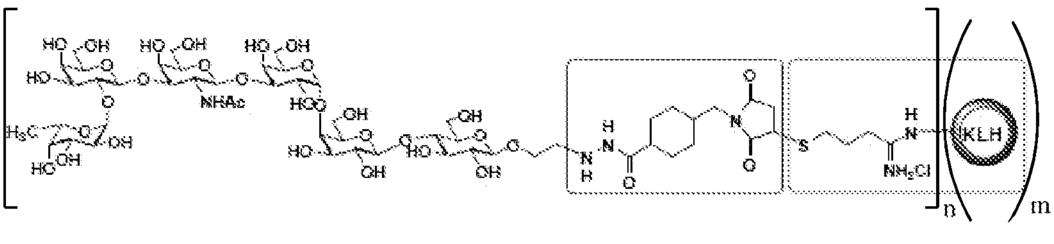

wherein n independently is an integer from about 1 to about 160, and wherein m is independently an integer from about 1 to about 20. In certain embodiments, m is an integer from about 1 to about 5. In certain embodiments, m is an integer from about 1 to about 3. In certain embodiments, m is 1. In certain embodiments, m is 2. In certain embodiments, m is 3. In certain embodiments, m is 4. In certain embodiments, m is 5. In certain embodiments, m is 6. In certain embodiments, m is 7. In certain embodiments, m is 8. In certain embodiments, m is 9. In certain embodiments, m is 10. In certain embodiments, m is 11. In certain embodiments, m is 12. In certain embodiments, m is 13. In certain embodiments, m is 14. In certain embodiments, m is 15. In certain embodiments, m is 16. In certain embodiments, m is 17. In certain embodiments, m is 18. In certain embodiments, m is 19. In certain embodiments, m is 20. In certain embodiments, for any of the above embodiments, when m is 1 to 20, each n is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80,

81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, or 160, respectively.

In certain embodiments, there is more than one Globo H moiety attached to each monomeric KLH moiety. In certain illustrative embodiments, the more than one Globo H moiety attached to each KLH moiety is attached via a linker. In other illustrative embodiments, the more than one Globo H moieties attached to each KLH moiety are attached via a linker and attached to a derivatized KLH moiety.

In another embodiment, the ratio of Globo H moieties to KLH moiety subunits is at least 1. In another embodiment, the ratio of Globo H moieties to KLH moiety subunits is at least 10. In another embodiment, the ratio of Globo H moieties to KLH moiety is at least 25. In another embodiment, the ratio of Globo H moieties to KLH moiety subunits is at least 50. In a further embodiment, the ratio of Globo H moieties to KLH moiety subunits is at least 100. In a further embodiment, the ratio of Globo H moieties to KLH moiety subunits is at least 150. In yet another embodiment, the ratio of Globo H moieties to KLH moiety subunits is at least 500. In yet a further embodiment, the ratio of Globo H moieties to KLH moiety subunits is at least 750. In still another embodiment, the ratio of Globo H moieties to KLH moiety subunits is at least 1000. In still another embodiment, the ratio of Globo H moieties to KLH moiety subunits is at least 1500. In still another embodiment, the ratio of Globo H moieties to KLH moiety subunits is at least 2000.

In various embodiments, the invention encompasses a single monomer of KLH to multiple KLH subunits (e.g., 2, 3, 4, 5, 6, 7, 8, 9, 10, 11, 12, 13, 14, 15, 16, 17, 18, 19, 20) each having attached multiple Globo H moieties. In certain embodiments, the ratio of Globo H moieties to KLH moiety is the same. In other embodiments, the ratio of Globo H moieties to KLH moiety is different.

Another embodiment of the invention encompasses a composition comprising at least two KLH moieties. For example, a derivatized KLH moiety in the form of a dimer. In another embodiment, the at least two KLH moieties are the same. In another embodiment, the at least two KLH moieties are different. In a further embodiment, the at least two KLH moieties have the same Globo H moiety to KLH moiety subunit ratio. In still a further embodiment, the at least two KLH moieties have a different Globo H moiety to KLH moiety subunit ratio.

Another embodiment of the invention encompasses a therapeutic composition comprising at least three KLH moieties, for example, a derivatized KLH moiety in the form of a trimer. In certain embodiments, the at least three KLH moieties are the same. In another embodiment, the at least three KLH moieties are not the same. In a further embodiment, the at least three KLH moieties have the same Globo H moiety to KLH moiety subunit ratio. In still a further embodiment, the at least three KLH moieties have a different Globo H moiety to KLH moiety subunit ratio.

Another embodiment of the invention encompasses a therapeutic composition comprising at least four KLH moieties, for example, a derivatized KLH moiety in the form of a tetramer. In certain embodiments, the at least four KLH moieties are the same. In another embodiment, the at least four KLH moieties are not the same. In a further embodiment, the at least four KLH moieties have the same Globo H moiety to KLH moiety subunit ratio. In still a further embodiment, the at least four KLH moieties have a different Globo H moiety to KLH moiety subunit ratio.

Another embodiment of the invention encompasses a therapeutic composition comprising at least five KLH moieties, for example, a derivatized KLH moiety in the form of a pentamer. In certain embodiments, the at least five KLH moieties are the same. In another embodiment, the at least five KLH moieties are not the same. In a further embodiment, the at least five KLH moieties have the same Globo H moiety to KLH moiety subunit ratio. In still a further embodiment, the at least five KLH moieties have a different Globo H moiety to KLH moiety subunit ratio.

Another embodiment of the invention encompasses a therapeutic composition comprising at least six KLH moieties, for example, a derivatized KLH moiety in the form of a hexamer. In certain embodiments, the at least six KLH moieties are the same. In another embodiment, the at least six KLH moieties are not the same. In a further embodiment, the at least six KLH moieties have the same Globo H moiety to KLH moiety subunit ratio. In still a further embodiment, the at least six KLH moieties have a different Globo H moiety to KLH moiety subunit ratio.

Another embodiment of the invention encompasses a therapeutic composition comprising at least twenty KLH moieties, for example, a derivatized KLH moiety in the form of a didecamer. In certain embodiments, the at least twenty KLH moieties are the same. In another embodiment, the at least twenty KLH moieties are not the same. In a further embodiment, the at least twenty KLH moieties have the same Globo H moiety to KLH moiety subunit ratio. In still a further embodiment, the at least twenty KLH moieties have a different Globo H moiety to KLH moiety subunit ratio.

In one embodiment, the Globo H moiety comprises (Fuc $\alpha$ 1$\rightarrow$2 Gal $\beta$ 1$\rightarrow$3 GalNAc $\beta$ 1$\rightarrow$- 3 Gal $\alpha$ 1$\rightarrow$4 Gal $\beta$ 1$\rightarrow$4 Glc). In a further embodiment, the KLH moiety subunit is a KLH-1 or KLH-2 moiety or a combination thereof. As used herein, the term "KLH" refers to KLH-1, KLH-2, and/or combinations thereof.

In another embodiment, the KLH moiety subunit is at least 99% identical to a corresponding naturally occurring KLH moiety subunit.

In another embodiment, the KLH moiety subunit is at least 95% identical to a corresponding naturally occurring KLH moiety subunit.

In another embodiment, the KLH moiety subunit is at least 90% identical to a corresponding naturally occurring KLH moiety subunit.

In another embodiment, the KLH moiety subunit is at least 80% identical to a corresponding naturally occurring KLH moiety subunit.

In another embodiment, the KLH moiety subunit is at least 70% identical to a corresponding naturally occurring KLH moiety subunit.

In another embodiment, the KLH moiety subunit is at least 60% identical to a corresponding naturally occurring KLH moiety subunit.

In another embodiment, the Globo H moiety is covalently linked to a keyhole limpet hemocyanin (KLH) moiety subunit via a linker. In yet a further embodiment, the Globo H moiety is covalently linked to a keyhole limpet hemocyanin (KLH) moiety subunit by a 4-(4-N-maleimidomethyl) cyclohexane-1-carboxyl hydrazide (MMCCH) linkage. In another further embodiment, the Globo H moiety is covalently linked to a derivatized keyhole limpet hemocyanin (KLH) moiety subunit and is linked by a 4-(4-N-maleimidomethyl) cyclohexane-1-carboxyl hydrazide (MMCCH) linkage.

In another embodiment, the isolated therapeutic conjugate has an epitope/carrier ratio based on a KLH monomer having molecular weight of about 350KDa to about 400KDa of at least or about 150. In another embodiment, the isolated therapeutic conjugate has an epitope/carrier ratio of at least or about 100. In a further embodiment, the isolated therapeutic conjugate has an epitope/carrier ratio of at least or about 75. In still a further embodiment, the isolated therapeutic conjugate has an epitope/carrier ratio of at least or about 50. In yet a further embodiment, the isolated therapeutic conjugate has an epitope/carrier ratio of at least or about 25. In still another embodiment, the isolated therapeutic conjugate has an epitope/carrier ratio of at least or about 15. In still another embodiment, the isolated therapeutic conjugate has an epitope/carrier ratio of at least or about 5. In still another embodiment, the isolated therapeutic conjugate has an epitope/carrier ratio of at least or about 1.

Another embodiment of the invention encompasses a pharmaceutical composition comprising KLH moiety subunits, wherein each KLH moiety subunit comprises one or more Globo H moieties covalently linked to a keyhole limpet hemocyanin (KLH) moiety subunit. In certain embodiments, the pharmaceutical composition comprises dimers of at least two KLH moiety subunits, wherein each KLH moiety subunits comprises one or more Globo H moieties covalently linked to a KLH moiety subunit. In certain embodiments, the pharmaceutical composition comprises trimers of at least three KLH moiety subunits, wherein each KLH moiety subunits comprises one or more Globo H moieties covalently linked to a KLH moiety subunit. In certain embodiments, the pharmaceutical composition comprises at least four KLH moiety subunits, wherein each KLH moiety subunit comprises one or more Globo H moieties covalently linked to a KLH moiety subunit. In certain embodiments, the pharmaceutical composition comprises a mixture of KLH moiety subunits (e.g., monomers, dimers, trimers, tetramers, pentamers, hexamers etc.), wherein each KLH moiety subunits comprises multiple Globo H moieties covalently linked to a KLH moiety subunit.

Another aspect of the invention relates to a pharmaceutical composition comprising monomers, dimers, trimers, tetramers, pentamers, hexamers or combinations thereof of KLH moieties, wherein each KLH comprises one or more Globo H moiety covalently linked to a keyhole limpet hemocyanin (KLH) moiety subunit.

In one embodiment the invention, the epitope/carrier ratios of the therapeutic conjugates in the composition ranges from about 1 to 3000. In a further embodiment, the epitope/carrierratios of the therapeutic conjugates in the composition range from about 75 to 2000. In still another embodiment, the epitope/carrier ratios of the therapeutic conjugates in the composition range from about 100 to 1000. In yet a further embodiment the average epitope/carrier ratio of the therapeutic conjugates in the composition ranges from about 150 to 500.

In another embodiment, about 1% to 99% of the therapeutic conjugates in the composition are KLH monomers. In a further embodiment, about 0% to 99% of the therapeutic conjugates in the composition are KLH dimers. In still another embodiment, about 0% to 99% of the therapeutic conjugates in the composition are KLH trimers. In yet another embodiment, about 0% to 99% of the therapeutic conjugates in the composition are KLH tetramers. In a further embodiment, about 1% to 99% of the therapeutic conjugates in the composition are KLH pentamers. In yet another embodiment, about 0% to 99% of the therapeutic conjugates in the composition include 6 KLH subunits. In yet another embodiment, about 0% to 99% of the therapeutic conjugates in the composition include 7 KLH subunits. In yet another embodiment, about 0% to 99% of the therapeutic conjugates in the composition include 8 KLH subunits. In yet another embodiment, about 0% to 99% of the therapeutic conjugates in the composition include 9 KLH subunits. In yet another embodiment, about 0% to 99% of the therapeutic conjugates in the composition include 10 KLH subunits. In yet another embodiment, about 0% to 99% of the therapeutic conjugates in the composition include 11 KLH subunits. In yet another embodiment, about 0% to 99% of the therapeutic conjugates in the composition include 12 KLH subunits. In yet another embodiment, about 0% to 99% of the therapeutic conjugates in the composition include 13 KLH subunits. In yet another embodiment, about 0% to 99% of the therapeutic conjugates in the composition include 14 KLH subunits. In yet another embodiment, about 0% to 99% of the therapeutic conjugates in the composition include 15 KLH subunits. In yet another embodiment, about 0% to 99% of the therapeutic conjugates in the composition include 16 KLH subunits. In yet another embodiment, about 0% to 99% of the therapeutic conjugates in the composition include 17 KLH subunits. In yet another embodiment,

about 0% to 99% of the therapeutic conjugates in the composition include 18 KLH subunits. In yet another embodiment, about 0% to 99% of the therapeutic conjugates in the composition include 19 KLH subunits. In yet another embodiment, about 0% to 99% of the therapeutic conjugates in the composition include 20 KLH subunits. In still another embodiment, about 1% to 99% of the therapeutic conjugates in the composition are monomers, dimers, trimers, tetramers or combinations thereof. In still another embodiment, about 99% of the therapeutic conjugates in the composition are monomers, dimers, trimers, tetramers or combinations thereof.

In certain embodiments, certain exemplary composition embodiments and methods of use thereof can include or exclude (e.g. proviso out) any one or more of the other representative compound and/or composition embodiments described herein.

In another embodiment, the pharmaceutical composition comprises an adjuvant. As used herein, the terms "immunologic adjuvant" refers to a substance used in conjunction with an immunogen which enhances or modifies the immune response to the immunogen. Specifically, the terms "adjuvant" and "immunoadjuvant" are used interchangeably in the present invention and refer to a compound or mixture that may be non-immunogenic when administered to a host alone, but that augments the host's immune response to another antigen when administered conjointly with that antigen. Adjuvant-mediated enhancement and/or extension of the duration of the immune response can be assessed by any method known in the art including without limitation one or more of the following: (i) an increase in the number of antibodies produced in response to immunization with the adjuvant/antigen combination versus those produced in response to immunization with the antigen alone; (ii) an increase in the number of T cells recognizing the antigen or the adjuvant; and (iii) an increase in the level of one or more Type I cytokines.

The adjuvant of can be administered as part of a pharmaceutical or vaccine composition comprising an antigen or as a separate formulation, which is administered conjointly with a second composition containing an antigen. In any of these compositions glycosphingolipids (GSLs) can be combined with other adjuvants and/or excipients/carriers. These other adjuvants include, but are not limited to, oil-emulsion and emulsifier-based adjuvants such as complete Freund's adjuvant, incomplete Freund's adjuvant, MF59, or SAF; mineral gels such as aluminum hydroxide (alum), aluminum phosphate or calcium phosphate; microbially-derived adjuvants such as cholera toxin (CT), pertussis toxin, *Escherichia coli* heat-labile toxin (LT), mutant toxins (e.g., LTK63 or LTR72), Bacille Calmette-Guerin (BCG), *Corynebacterium parvum*, DNA CpG motifs, muramyl dipeptide, or monophosphoryl lipid A; particulate adjuvants such as immunostimulatory complexes (ISCOMs), liposomes, biodegradable microspheres, or saponins (e.g., QS-21); cytokines such as IFN-γ, IL-2, IL-12 or GM-CSF; synthetic adjuvants such as nonionic block copolymers, muramyl peptide analogues (e.g., N-acetyl-muramyl-L-threonyl-D-isoglutamine [thr-MDP], N-acetyl-nor-muramyl-L-alanyl-D-isoglutamine, N-acetyl-muramyl-L-alanyl-D-isoglutaminyl-L-alanine-2-[1'-2'-dipalmitoyl-sn-glycero-3-hydroxyphosphoryloxy]-ethylamine), polyphosphazenes, or synthetic polynucleotides, and surface active substances such as lysolecithin, pluronic polyols, polyanions, peptides, hydrocarbon emulsions, or keyhole limpet hemocyanins (KLH), Toll-Like Receptor molecules, LPS, lipoproteins, lipopeptides, flagellin, doublestranded RNA, viral DNA, unmethylated CpG islands, levamisole, bacillus Calmette-Guerin, Isoprinosine, Zadaxin, PD-1 antagonists, PD-1 antibodies, CTLA antagonists, CTLA antibodies, interleukin, cytokines, GM-CSF, glycolipid, aluminum salt based, aluminum phosphate, alum, aluminum hydroxide, liposomes, TLR2 agonists, lipopeptide, nanoparticles, monophosphoryl lipid A, OBI-821 saponin, saponin, OBI-834 adjuvant, C34 adjuvant, oil in water nano-emulsions, and bacteria-like particle. Preferably, these additional adjuvants are also pharmaceutically acceptable for use in humans.

In another embodiment, the pharmaceutical composition comprises a cytokine selected from the group consisting of IL-2, IL-12, IL-18, IL-2, IFN-γ, TNF, IL-4, IL-10, IL-13, IL-21, GM-CSF and TGF-β. In a further embodiment, the pharmaceutical composition comprises a chemokine.

In a further embodiment, the immunogenic/therapeutic agent is administered as a pharmaceutical composition.

In still another embodiment, the pharmaceutical composition comprises monoclonal antibodies, chemotherapeutics, hormonal therapeutic agents, retinoid receptor modulators, cytotoxic/cytostatic agents, antineoplastic agents, antiproliferative agents, anti-mTOR agents, anti-Her2 agents, anti-EGFR agents, prenyl-protein transferase inhibitors, HMG-CoA reductase inhibitors, nitrogen mustards, nitroso ureas, angiogenesis inhibitors, bevacizumab, inhibitors of cell proliferation and survival signaling pathway, apoptosis inducing agents, agents that interfere with cell cycle checkpoints, agents that interfere with receptor tyrosine kinases (RTKs), integrin blockers, NSAIDs, PPAR agonists, inhibitors of inherent multidrug resistance (MDR), anti-emetic agents, agents useful in the treatment of anemia, agents useful in the treatment of neutropenia, immunologic-enhancing drugs, biphosphonates, aromatase inhibitors, agents inducing terminal differentiation of neoplastic cells, γ-secretase inhibitors, cancer vaccines (*e.g.*, active immunotherapy), monoclonal antibody therapeutics (*e.g.*, passive immunotherapy), and any combination thereof.

In another embodiment, the therapeutic compositions of the invention can further include PD-1/PD-L1 inhibitors (cytotoxic T cell lymphocyte (CTLs) immunotherapy), CTLA-4 immunotherapy, CDK4/6 inhibitors (target therapy), PI3K inhibitors (target therapy), mTOR inhibitors (target therapy), AKT inhibitors (target therapy), Pan-Her inhibitors (target therapy). These inhibitors can be modified to generate the respective monoclonal antibody as well. Such antibodies can be included in therapeutic compositions of the invention.

In another embodiment, the pharmaceutical composition comprises a pharmaceutically acceptable carrier. In a further embodiment, the pharmaceutical composition is a cancer vaccine. In still another embodiment, the pharmaceutical composition is formulated for subcutaneous administration. In still another embodiment, the pharmaceutical composition is formulated for intramuscular administration. In still another embodiment, the pharmaceutical composition is formulated for intra-arterial administration. In still another embodiment, the pharmaceutical composition is formulated for intravenous administration.

Another embodiment of the invention encompasses a method of treating a patient in need thereof comprising administering to the patient a therapeutically effective amount of the therapeutic composition comprising Globo H and KLH. In one embodiment, the patient has been diagnosed with or is suspected of having cancer. In another embodiment, the cancer is an epithelial cancer. In a further embodiment, the cancer is breast cancer. In still another embodiment, the therapeutically effective amount of a Globo-H moiety in the pharmaceutical/therapeutic composition may range from about 0.001 μg/kg to about 250 mg/kg. In yet a further embodiment, the therapeutically effective amount Globo-H moiety in the pharmaceutical/therapeutic composition comprises about 10 μg/kg to about 50 μg/kg of one therapeutic conjugate per dose. In yet a further embodiment, the therapeutically effective amount Globo-H moiety in the pharmaceutical/therapeutic composition comprises about 0.10 μg/kg to about 0.75 μg/kg of one therapeutic conjugate per dose.

In still another embodiment, the therapeutically effective amount of the Globo-H- KLH complex in the therapeutic composition may range from about 0.001μg/kg to about 250 mg/kg. In yet a further embodiment, the therapeutically effective amount of the Globo-H-KLH complex in the therapeutic composition comprises about 10 μg/kg to about 50 μg/kg of one therapeutic conjugate per dose. In yet a further embodiment, the therapeutically effective amount of the Globo-H-KLH complex in the therapeutic composition comprises about 0.60 μg/kg to about 4.50 μg/kg of one therapeutic conjugate per dose.

In still another embodiment, the method is capable of extending progression free survival over a control placebo by about or at least 1 week. In still another embodiment, the method is capable of extending progression free survival over a control placebo by about or at least 2 weeks. In still another embodiment, the method is capable of extending progression free survival over a control placebo by about or at least 1 month. In still another embodiment, the method is capable of extending progression free survival over a control placebo by about or at least 3 months. In still another embodiment, the method is capable of extending progression free survival over a control placebo by about or at least 6 months. In yet another embodiment, the method is capable of extending or overall survival over a control placebo by about or at least 12 months.

## BRIEF DESCRIPTION OF THE FIGURES

A more complete understanding of the invention may be obtained by reference to the accompanying drawings, when considered in conjunction with the subsequent detailed description. The embodiments illustrated in the drawings are intended only to exemplify the invention and should not be construed as limiting the invention to the illustrated embodiments.

Figure 1A shows the chemical structure Globo H and as well as several exemplary Globo H analogs. Glc stands for glucose, Gal stands for galactose, GalNAc stands for N-acetylgalactosamine, and Fuc stands for fucose. Figure 1B shows an exemplary Globo H-KLH subunit conjugated by way of an MMCCH linker.

Figure 2A shows an exemplary Globo H-KLH subunit conjugation synthesis pathway. Figure 2B shows Globo H-KLH dimers and trimers of the invention compared to Globo H conjugates disclosed in Slovin et al (1999), Proc Natl Acad Sci USA 96:5710-5 and Gilewski et al (2001), Proc Natl Acad Sci USA 98: 3270-5.

Figure 3 shows the result of multi-angle laser scattering spectrometry (MALS) of native KLH (8.3 MDa).

Figure 4A shows the result of size exclusion chromatography of KLH using multi-angle laser scattering spectrometry (MALS) as detector. Figure 4B shows the mass distribution analysis of KLH. Figure 4C shows the result of size exclusion chromatography of Globo H-KLH glycoconjugate (OBI-822 Lot no. 14001) using multi-angle laser scattering spectrometry (MALS) as detector. Figure 4D shows the mass distribution analysis of Globo H-KLH glycoconjugate.

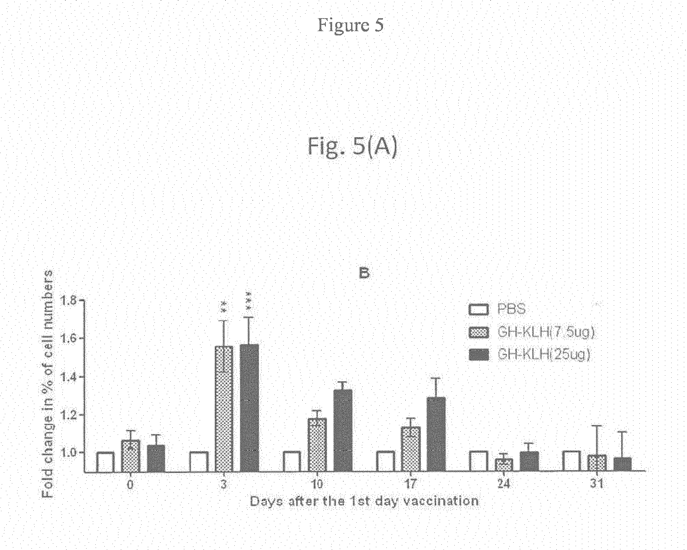

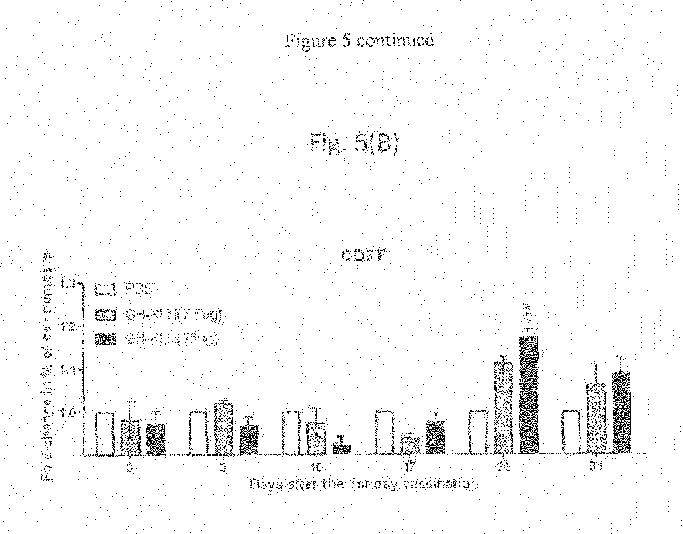

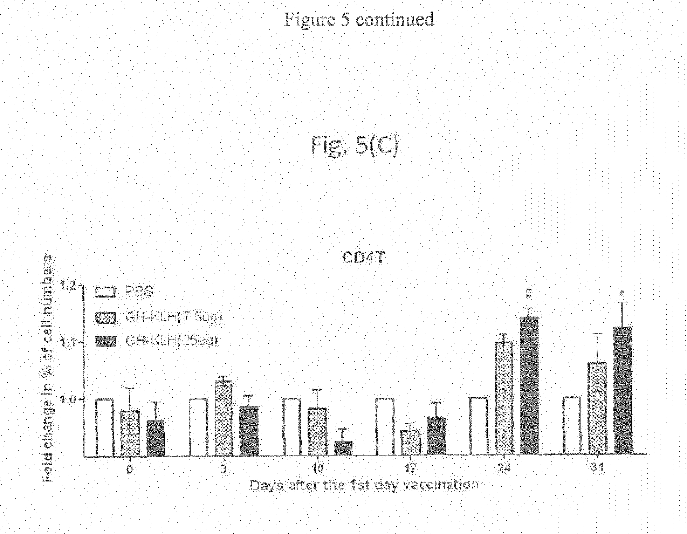

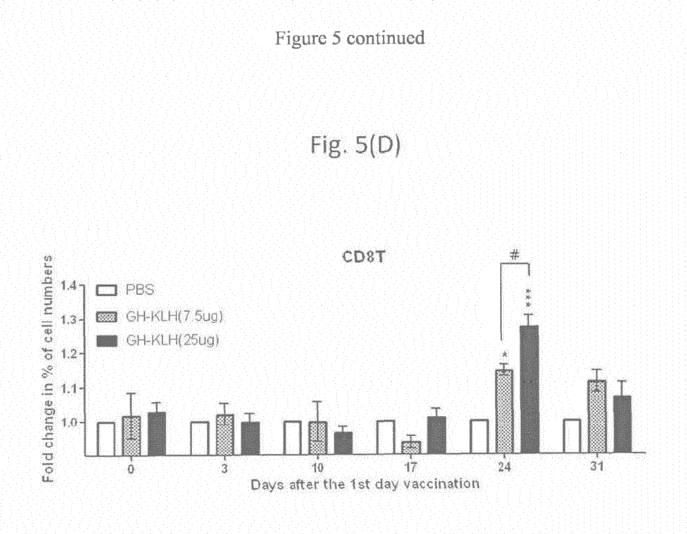

Figure 5A shows the chronological expansion of B cell populations in Lewis rats immunized with an exemplary Globo H-KLH glycoconjugate (7.5 μg and 25 μg). Figure 5B shows the chronological expansion of CD3 T cell populations in Lewis rats immunized with an exemplary Globo H-KLH glycoconjugate (7.5 μg and 25 μg). Figure 5C shows the chronological expansion of CD4 T cell populations in Lewis rats immunized with an exemplary Globo H-KLH glycoconjugate (7.5 μg and 25 μg). Figure 5D shows the chronological expansion of CD8 T cell populations in Lewis rats immunized with an exemplary Globo H-KLH glycoconjugate (7.5 μg and 25 μg). Data were presented as percentage

of cell numbers in indicated group normalized to the percentage of cell numbers of PBS group. Multiple comparisons were analyzed using two-way ANOVA, followed by Bonferroni's post hoc tests. *, $p<0.05$, **, $p<0.01$, and ***, $p<0.001$ compared with PBS.

Figure 6A shows the chronological changes in reciprocal titers of IgM antibodies in the blood from Lewis rats immunized with an exemplary Globo H-KLH glycoconjugate (7.5 μg and 25 μg). Figure 6B shows the chronological changes in reciprocal titers of IgG antibodies in the blood from Lewis rats immunized with an exemplary Globo H-KLH glycoconjugate (7.5 μg and 25 μg).

Figure 7 shows the IgM antibody titers in mice in response to the conjugation ratio between Globo H and KLH (0.17:1 and 0.07:1).

Figure 8 illustrates the immunogenicity of C57BL/6 mice that were immunized with PBS, Globo H-KLH glycoconjugate + saponin adjuvant (OBI-822 + OBI-821) and Globo H-KLH glycoconjugate + C34 adjuvant (OBI-822 + OBI-834). The sera were collected on day 42 for ELISA analysis to determine the anti-Globo H IgM and IgG antibodies production. Figure 8A shows the IgM production. Figure 8B shows the IgG production. The subcutaneous (SC) administration of Globo H-KLH glycoconjugate was 2 μg and adjuvant was 20 μg.

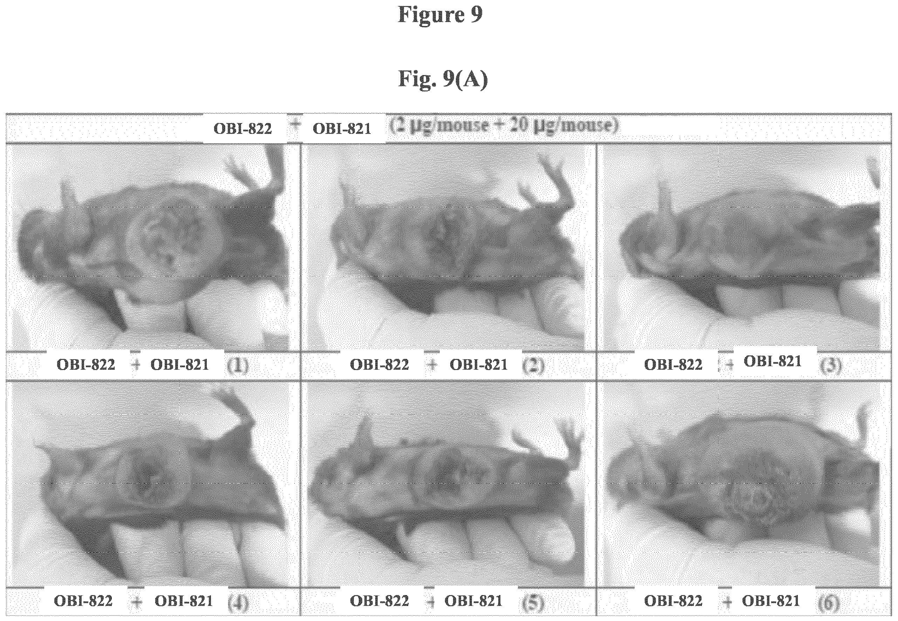

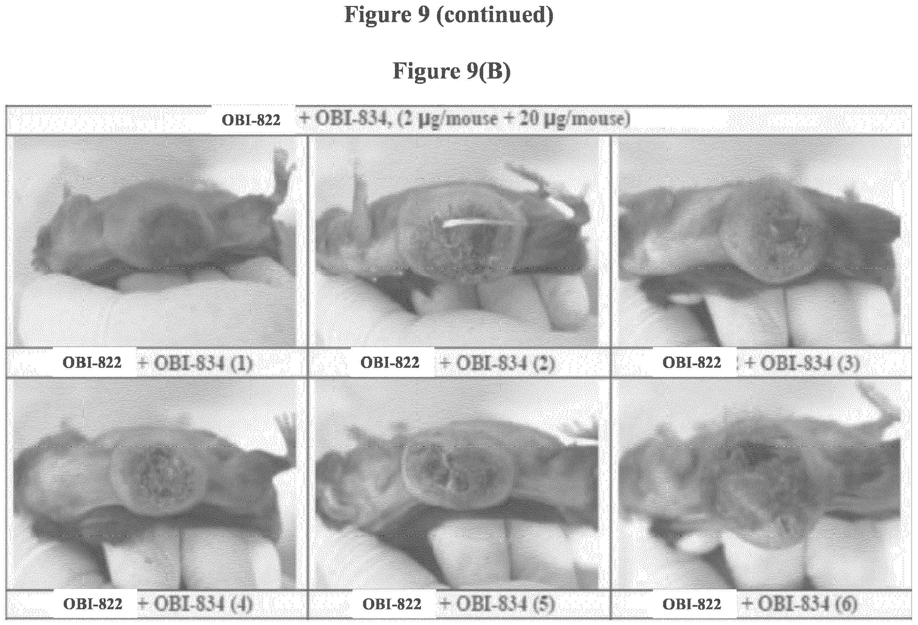

Figure 9 illustrates six groups of immunocompetent (6-8 weeks old), pathogen-free (SPF) C57BL/6 female mice were injected with $5.0\times10^6$ in 0.1 mL Lewis Lung carcinoma (LL/2, ATCC CRL-1642) cells, syngeneic for C57BL/6 mice, were injected subcutaneously into the abdominal region toward the lateral side of experimental mice on day 16. Three testing conditions (PBS only, OBI-822 + OBI-821 adjuvant and OBI-822 + OBI-834 adjuvant) were administered subcutaneously in 0.2 mL/mouse (at both left and right abdominal sties, 0.1 mL/site) or intraperitoneally in the dosing volume of 10 mL/kg on days 0, 5, 11, 19, 29, 34 and 39. The pictures including tumor with whole body were taken after sacrifice on day 42

Figure 10 illustrates LL/2 (Lewis lung carcinoma cell line) tumor growth on Globo H-KLH glycoconjugate immunized C57BL/6 mice that were subcutaneously vaccinated with PBS, OBI-822 + OBI-821 adjuvant and OBI-822 + OBI-834 adjuvant on day 0, 5, 11, 19, 29, 34 and 39. LL/2 cells ($5.0\times10^6$ in 0.1 mL) were subcutaneously injected into each mouse on day 16. Tumor sizes were monitored on day 16, 19, 23, 26, 30, 34, 37, 40 and 42.

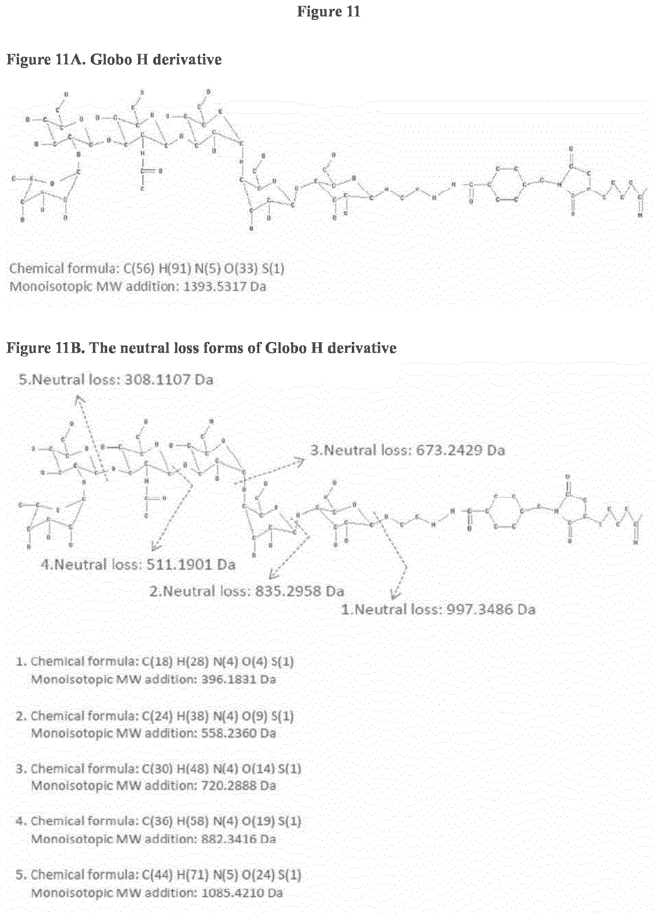

Figure 11 illustrates the chemical structure of Globo H derivative. Figure 11A illustrates the Globo H derivative [Chemical formula: C(56) H(91) N(5) O(33) S(1), Monoisotopic MW addition: 1393.5317 Da]. Figure 11B illustrates the neutral loss forms of Globo H derivative. Chemical formula 1: C(18) H(28) N(4) O(4) S(1), Monoisotopic MW addition: 396.1831 Da; Chemical formula 2: C(24) H(38) N(4) O(9) S(1), Monoisotopic MW addition: 558.2360 Da; Chemical formula 3: C(30) H(48) N(4) 0(14) S(1), Monoisotopic MW addition: 720.2888 Da; Chemical formula 4: C(36) H(58) N(4) 0(19) S(1), Monoisotopic MW addition: 882.3416 Da; Chemical formula 5: C(44) H(71) N(5) O(24) S(1), Monoisotopic MW addition: 1085.4210 Da.

Figure 12 illustrates the chemical structure of MMCCH derivative. Figure 12A illustrates the chemical structure of MMCCH derivative [Chemical formula: C(16) H(24) N(4) O(3) S(1), Monoisotopic MW addition: 352.1569 Da]. Figure 12B illustrates the deamidated MMCCH derivative [Chemical formula: C(16) H(22) N(2) O(4) S(1), Monoisotopic MW addition: 338.1300 Da].

Figure 13 shows a summary of Globo H and MMCCH derivative peptide identification on lysine residue

Figure 14 illustrates the identification details of Globo-H conjugated peptides on lysine residue for sample 1 ($1^{st}$LC-MS/MS) for KLH1 (A) and KLH2 (B).

Figure 15 illustrates the identification details of Globo-H conjugated peptides on lysine residue for sample 2 ($1^{st}$LC-MS/MS) for KLH1 (A) and KLH2 (B).

Figure 16 illustrates the identification details of Globo-H conjugated peptides on lysine residue for sample 3 ($1^{st}$LC-MS/MS) for KLH1 (A) and KLH2 (B).

Figure 17 illustrates the identification details of Globo-H conjugated peptides on lysine residue for sample 4 ($1^{st}$LC-MS/MS) for KLH1 (A) and KLH2 (B).

Figure 18 illustrates the identification details of Globo-H conjugated peptides on lysine residue for sample 1 ($2^{nd}$ LC-MS/MS) for KLH1 (A) and KLH2 (B).

Figure 19 illustrates the identification details of Globo-H conjugated peptides on lysine residue for sample 2 ($2^{nd}$ LC-MS/MS) for KLH1 (A) and KLH2 (B).

Figure 20 illustrates the identification details of Globo-H conjugated peptides on lysine residue for sample 3 ($2^{nd}$ LC-MS/MS) for KLH1 (A) and KLH2 (B).

Figure 21 illustrates the identification details of Globo-H conjugated peptides on lysine residue for sample 4 ($2^{nd}$ LC-MS/MS) for KLH1 (A) and KLH2 (B).

Figure 22 illustrates the identification details of MMCCH-conjugated peptides on lysine residue for sample 1 ($1^{st}$ LC-MS/MS) for KLH1 (A) and KLH2 (B).

Figure 23 illustrates the identification details of MMCCH-conjugated peptides on lysine residue for sample 2 ($1^{st}$ LC-MS/MS) for KLH1 (A) and KLH2 (B).

Figure 24 illustrates the identification details of MMCCH-conjugated peptides on lysine residue for sample 3 ($1^{st}$ LC-MS/MS) for KLH1 (A) and KLH2 (B).

Figure 25 illustrates the identification details of MMCCH-conjugated peptides on lysine residue for sample 4 ($1^{st}$ LC-MS/MS) for KLH1 (A) and KLH2 (B).

Figure 26 illustrates the identification details of MMCCH-conjugated peptides on lysine residue for sample 1 ($2^{nd}$ LC-MS/MS) for KLH1 (A) and KLH2 (B).

Figure 27 illustrates the identification details of MMCCH-conjugated peptides on lysine residue for sample 2 ($2^{nd}$ LC-MS/MS) for KLH1 (A) and KLH2 (B).

Figure 28 illustrates the identification details of MMCCH-conjugated peptides on lysine residue for sample 3 ($2^{nd}$ LC-MS/MS) for KLH1 (A) and KLH2 (B).

Figure 29 illustrates the identification details of MMCCH-conjugated peptides on lysine residue for sample 4 ($2^{nd}$ LC-MS/MS) for KLH1 (A) and KLH2 (B).Figure 30 illustrates the summary of Globo-H conjugated lysine sites identification for ($1^{st}$ LC-MS/MS) for KLH1 (A) and KLH2 (B); ($2^{nd}$ LC-MS/MS) for KLH1 (C) and KLH2 (D).

Figure 31 illustrates the summary of MMCCH-conjugated lysine sites identification for ($1^{st}$ LC-MS/MS) for KLH1 (A) and KLH2 (B) and ($2^{nd}$ LC-MS/MS) for KLH1 (C) and KLH2 (D).

Figure 32 illustrates a summary of the Globo-H conjugation analysis in the first (A) and second (B) LC-MS/MS runs.

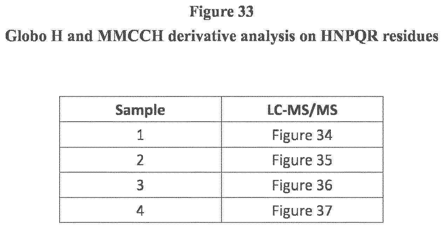

Figure 33 shows a summary of an exemplary Globo H and MMCCH derivative peptide identification on Histidine (H), Asparagine (N), Proline (P), Glutamine (Q) and Arginine (R) residues.

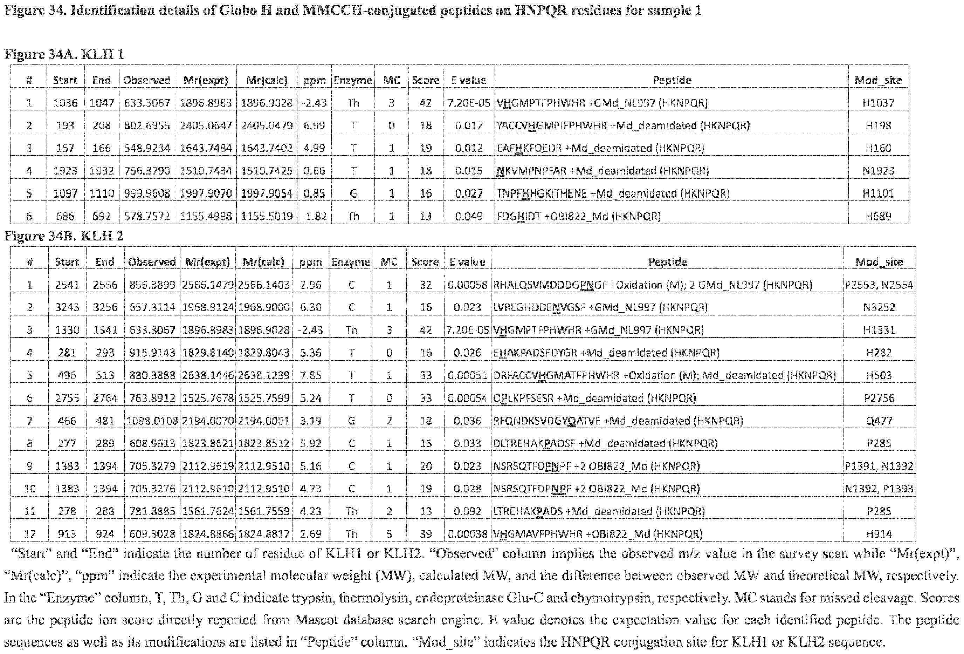

Figure 34 illustrates the identification details of Globo H and MMCCH-conjugated peptides on HNPQR residues for sample 1 (LC-MS/MS) for KLH1 (A) and KLH2 (B).

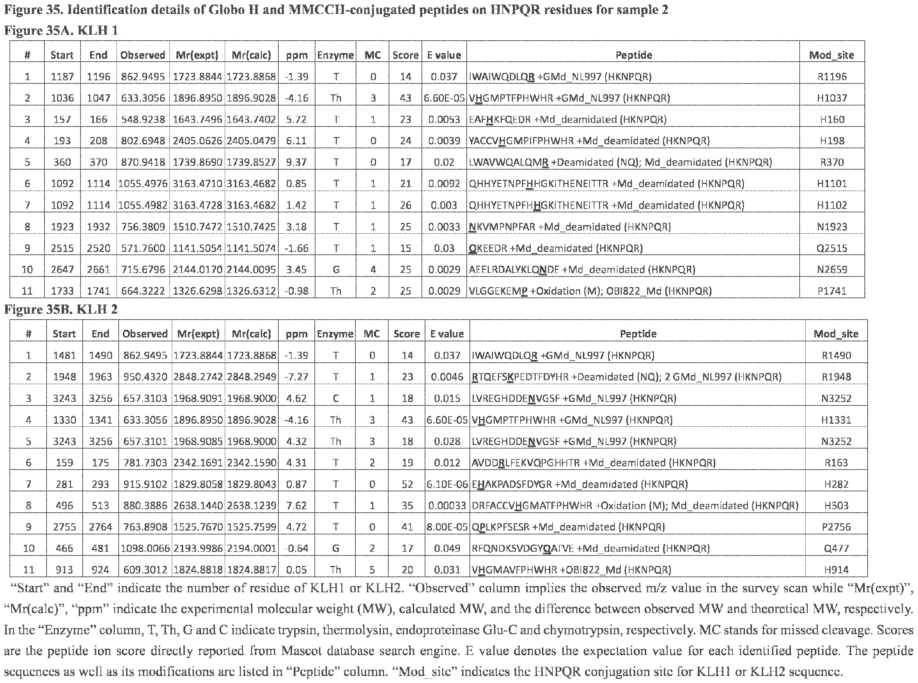

Figure 35 illustrates the identification details of Globo H and MMCCH-conjugated peptides on HNPQR residues for sample 2 (LC-MS/MS) for KLH1 (A) and KLH2 (B).

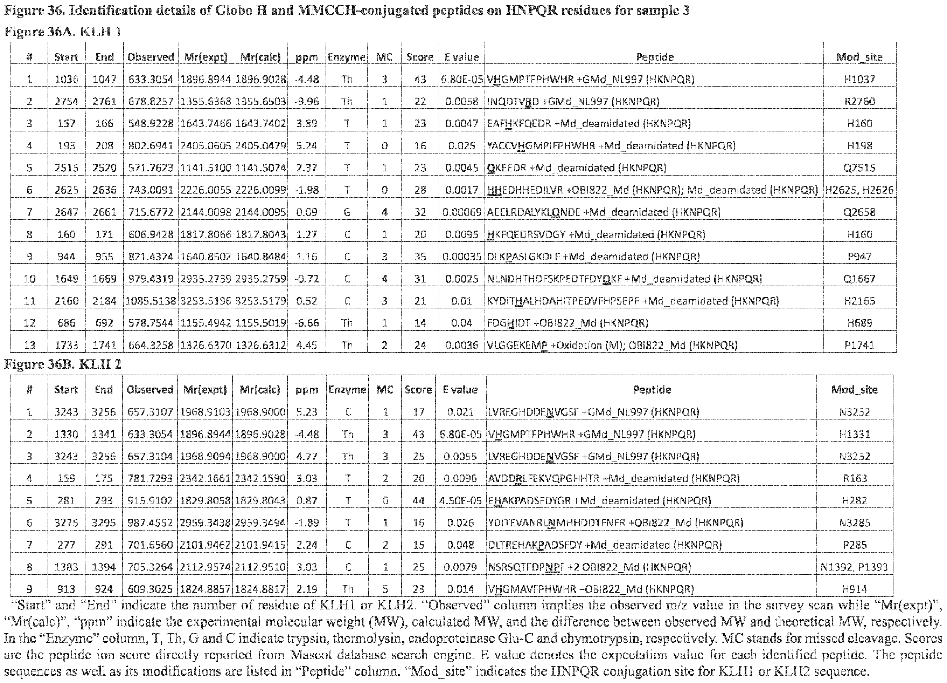

Figure 36 illustrates the identification details of Globo H and MMCCH-conjugated peptides on HNPQR residues for sample 3 (LC-MS/MS) for KLH1 (A) and KLH2 (B).

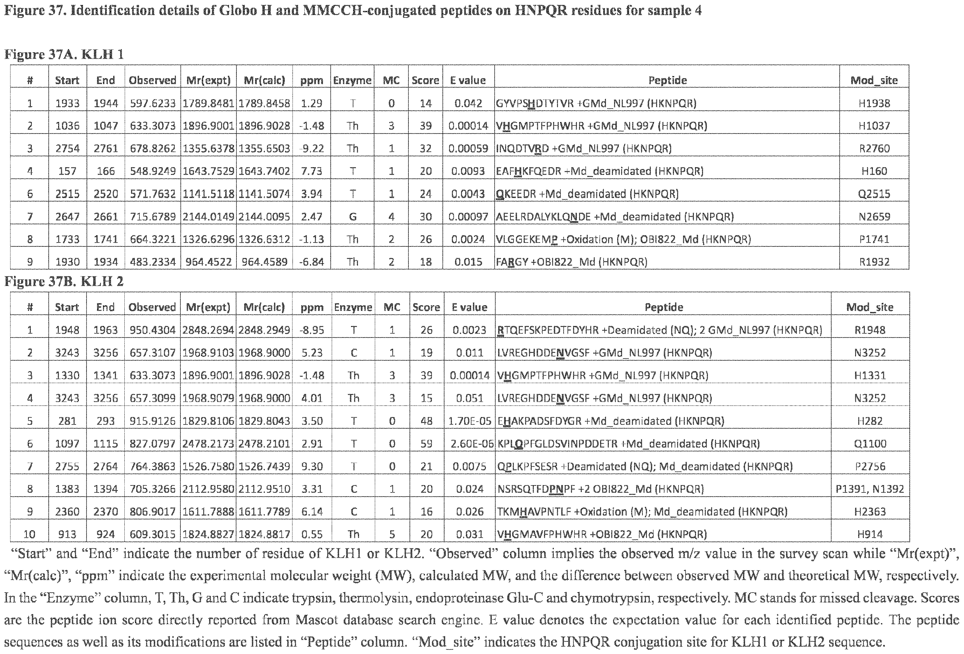

Figure 37 illustrates the identification details of Globo H and MMCCH-conjugated peptides on HNPQR residues for sample 4 (LC-MS/MS) for KLH1 (A) and KLH2 (B).

Figure 38 illustrates a summary of exemplary Globo H and MMCCH derivative analysis on Histidine (H), Asparagine

(N), Proline (P), Glutamine (Q) and Arginine (R) residues.

## DETAILED DESCRIPTION OF THE INVENTION

The practice of the present invention will employ, unless otherwise indicated, conventional techniques of molecular biology, microbiology, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press, 1989); DNA Cloning, Volumes I and II (D. N. Glover ed., 1985); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Antibodies: A Laboratory Manual, by Harlow and Lanes (Cold Spring Harbor Laboratory Press, 1988); and Handbook Of Experimental Immunology, Volumes I-IV (D. M. Weir and C. C. Blackwell, eds., 1986).

The use of synthetic carbohydrate conjugates to elicit antibodies was first demonstrated by Goebel and Avery in 1929. (Goebel, W. F., and Avery, O. T., J. Exp. Med., 1929, 50, 521; Avery, O. T., and Goebel, W. F., J. Exp. Med., 1929, 50, 533.) Carbohydrates were linked to carrier proteins via the benzenediazonium glycosides. Immunization of rabbits with the synthetic antigens generated polyclonal antibodies. Other workers (Allen, P. Z., and Goldstein, I. J., Biochemistry, 1967, 6, 3029; Rude, E., and Delius, M. M., Carbohydr. Res., 1968, 8, 219; Himmelspach, K., et al., Eur. J. Immunol., 1971, 1, 106; Fielder, R. J., et al., J. Immunol., 1970, 105, 265) developed similar techniques for conjugation of carbohydrates to protein carriers.

Glycoconjugates may be used in active immunotherapy generated from vaccinations to specifically target known target agents on tumor cells. The response to carbohydrate antigens normally does not enlist the use of T-cells, which would aid in the body's rejection of the tumor. While the probability of complete tumor rejection as a result of vaccination with a conjugate is thought to be unlikely, such treatments will boost immune surveillance and recurrence of new tumor colonies can be reduced. (Dennis, J., Oxford Glycosystems Glyconews Second, 1992; Lloyd, K. O., in Specific Immunotherapy of Cancer with Vaccines, 1993, New York Academy of Sciences, 50- 58). Toyokuni and Singhal have described a synthetic glycoconjugate (Toyokuni, T., et al., J. Am. Chem. Soc., 1994, 116, 395) that stimulated a measurable IgG titer, a result which is significant since an IgG response is generally associated with enlistment of helper T cells.

A synthetic Globo H vaccine in combination with an immunological adjuvant was shown to induce mainly IgM and to a lesser extent IgG antibodies in both prostate and metastatic breast cancer patients. In a phase I clinical trial, the vaccine also showed minimal toxicity with transient local skin reactions at the vaccination site. (Gilewski T et al. (2001) Proc Natl Acad Sci USA 98:3270-3275; Ragupathi G, et al. (1997) Angew Chem Int Ed 36:125-128; Slovin S F et al (1997) Proc Natl Acad Sci USA 96:5710- 5715). Mild flu-like symptoms which have been observed in some of the patients were probably associated with the side effect of QS-21. A pentavalent vaccine containing five prostate and breast cancer associated carbohydrate antigens-Globo-H, GM2, STn, TF and Tn-conjugated to maleimide-modified carrier protein KLH has been reported to produce anti-Globo H sera with higher titers of IgG than IgM in ELISA assays. (Zhu J. et al. (2009) J. Am. Chem. Soc. 131(26):9298-9303).

Accordingly, the present disclosure is directed to immunogenic/therapeutic compounds, compositions, and/or pharmaceutical formulation compositions targeted to/mediated by Globo H, as well as, immunotherapeutics, vaccines, dosage forms, kits, and methods of manufacture, and treatment thereof.

The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes for example, the inherent variation of error for a measuring device, the method being employed to determine the value, or the variation that exists among the study subjects. Typically the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

As used herein, the term "alkyl" refers to a straight or branched monovalent hydrocarbon containing, unless otherwise stated, 1-20 carbon atoms, e.g., C1-C8 or C1-C4, which can be substituted or unsubstituted. Examples of alkyl include, but are not limited to, methyl, ethyl, *n*-propyl, *i*-propyl, *n*-butyl, *i*-butyl, and *t*-butyl.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain")

are inclusive or open-ended and do not exclude additional, unrequired elements or method steps. It is contemplated that any embodiment discussed in this specification can be implemented with respect to any method or composition of the invention, and vice versa. Furthermore, compositions of the invention can be used to achieve methods of the invention.

"Treating" or "treating" is referred to herein as administration of a therapeutic composition to a subject with the purpose to cure, alleviate, relieve, remedy, prevent, or ameliorate a disorder, symptoms of the disorder, a disease state secondary to the disorder, or predisposition toward the disorder.

An "effective amount" is an amount of a therapeutic composition that is capable of producing a medically desirable result as delineated herein in a treated subject. The medically desirable result may be objective (*i.e.,* measurable by some test or marker) or subjective (*i.e.,* subject gives an indication of or feels an effect).

"Disease amenable to treatment with a therapeutic composition" as referred to herein means any procedures, conditions, disorders, ailments and/or illnesses which can be treated by the administration of the therapeutic compositions disclosed herein.

A "proliferative disorder" is one in which too many of some type of cell are produced resulting in deterioration of health. A proliferative disorder can be benign or malignant. Proliferative disorders can include for example, cancer.

As used herein, "cancer" that can be treated by the therapeutic compositions disclosed herein, includes cells with an abnormal growth state. Cancer cells can be characterized by loss of normal control mechanisms and thus are able to expand continuously, invade adjacent tissues, migrate to distant parts of the body, and promote the growth of new blood vessels from which the cells derive nutrients. As used herein, a cancer can be malignant or benign. Cancer can develop from any tissue within the body. As cells grow and multiply, they form a mass of tissue, called a tumor. The term tumor can include an abnormal growth or mass. Tumors can be cancerous (malignant) or noncancerous (benign). Cancerous tumors can invade neighboring tissues and spread throughout the body (metastasize). Benign tumors, however, generally do not invade neighboring tissues and do not spread throughout the body. Cancer can be divided into those of the blood and blood-forming tissues (leukemia and lymphoma) and "solid" tumors. "Solid" tumors can include carcinomas or sarcomas.

Cancers that may be treated by the therapeutic compositions of the invention include those classified by site include cancer of the oral cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and leukemia (lymphocytic leukemia; myeloid leukemia; monocytic leukemia; and other leukemia).

Other cancers, classified by histological type, that may be suitable targets for the therapeutic compositions according to the present invention include, but are not limited to, neoplasm, malignant; Carcinoma, NOS; Carcinoma, undifferentiated, NOS; Giant and spindle cell carcinoma; Small cell carcinoma, NOS; Papillary carcinoma, NOS; Squamous cell carcinoma, NOS; Lymphoepithelial carcinoma; Basal cell carcinoma, NOS; Pilomatrix carcinoma; Transitional cell carcinoma, NOS; Papillary transitional cell carcinoma; Adenocarcinoma, NOS; Gastrinoma, malignant; Cholangiocarcinoma; Hepatocellular carcinoma, NOS; Combined hepatocellular carcinoma and cholangiocarcinoma; Trabecular adenocarcinoma; Adenoid cystic carcinoma; Adenocarcinoma in adenomatous polyp; Adenocarcinoma, familial polyposis coli; Solid carcinoma, NOS; Carcinoid tumor, malignant; Bronchioloalveolar adenocarcinoma; Papillary adenocarcinoma, NOS; Chromophobe carcinoma; Acidophil carcinoma; Oxyphilic adenocarcinoma; Basophil carcinoma; Clear cell adenocarcinoma, NOS; Granular cell carcinoma; Follicular adenocarcinoma, NOS; Papillary and follicular adenocarcinoma; Nonencapsulating sclerosing carcinoma; Adrenal cortical carcinoma; Endometroid carcinoma; Skin appendage carcinoma; Apocrine adenocarcinoma; Sebaceous adenocarcinoma; Ceruminous adenocarcinoma; Mucoepidermoid carcinoma; Cystadenocarcinoma, NOS; Papillary cystadenocarcinoma, NOS; Papillary serous cystadenocarcinoma; Mucinous cystadenocarcinoma, NOS; Mucinous adenocarcinoma; Signet ring cell carcinoma; Infiltrating duct carcinoma; Medullary carcinoma, NOS; Lobular carcinoma; Inflammatory carcinoma; Paget's disease, mammary; Acinar cell carcinoma; Adenosquamous carcinoma; Adenocarcinoma w/ squamous metaplasia; Thymoma, malignant; Ovarian stromal tumor, malignant; Thecoma, malignant; Granulosa cell tumor, malignant; Androblastoma, malignant; Sertoli cell carcinoma; Leydig cell tumor, malignant; Lipid cell tumor, malignant; Paraganglioma, malignant; Extra-mammary paraganglioma, malignant; Pheochromocytoma; Glomangiosarcoma; Malignant melanoma, NOS; Amelanotic melanoma; Superficial spreading melanoma; Malig melanoma in giant pigmented nevus; Epithelioid cell melanoma; Blue nevus,

malignant; Sarcoma, NOS; Fibrosarcoma, NOS; Fibrous histiocytoma, malignant; Myxosarcoma; Liposarcoma, NOS; Leiomyosarcoma, NOS; Rhabdomyosarcoma, NOS; Embryonal rhabdomyosarcoma; Alveolar rhabdomyosarcoma; Stromal sarcoma, NOS; Mixed tumor, malignant, NOS; Mullerian mixed tumor; Nephroblastoma; Hepatoblastoma; Carcinosarcoma, NOS; Mesenchymoma, malignant; Brenner tumor, malignant; Phyllodes tumor, malignant; Synovial sarcoma, NOS; Mesothelioma, malignant; Dysgerminoma; Embryonal carcinoma, NOS; Teratoma, malignant, NOS; Struma ovarii, malignant; Choriocarcinoma; Mesonephroma, malignant; Hemangiosarcoma; Hemangioendothelioma, malignant; Kaposi's sarcoma; Hemangiopericytoma, malignant; Lymphangiosarcoma; Osteosarcoma, NOS; Juxtacortical osteosarcoma; Chondrosarcoma, NOS; Chondroblastoma, malignant; Mesenchymal chondrosarcoma; Giant cell tumor of bone; Ewing's sarcoma; Odontogenic tumor, malignant; Ameloblastic odontosarcoma; Ameloblastoma, malignant; Ameloblastic fibrosarcoma; Pinealoma, malignant; Chordoma; Glioma, malignant; Ependymoma, NOS; Astrocytoma, NOS; Protoplasmic astrocytoma; Fibrillary astrocytoma; Astroblastoma; Glioblastoma, NOS; Oligodendroglioma, NOS; Oligodendroblastoma; Primitive neuroectodermal; Cerebellar sarcoma, NOS; Ganglioneuroblastoma; Neuroblastoma, NOS; Retinoblastoma, NOS; Olfactory neurogenic tumor; Meningioma, malignant; Neurofibrosarcoma; Neurilemmoma, malignant; Granular cell tumor, malignant; Malignant lymphoma, NOS; Hodgkin's disease, NOS; Hodgkin's; paragranuloma, NOS; Malignant lymphoma, small lymphocytic; Malignant lymphoma, large cell, diffuse; Malignant lymphoma, follicular, NOS; Mycosis fungoides; Other specified non-Hodgkin's lymphomas; Malignant histiocytosis; Multiple myeloma; Mast cell sarcoma; Immunoproliferative small intestinal disease; Leukemia, NOS; Lymphoid leukemia, NOS; Plasma cell leukemia; Erythroleukemia; Lymphosarcoma cell leukemia; Myeloid leukemia, NOS; Basophilic leukemia; Eosinophilic leukemia; Monocytic leukemia, NOS; Mast cell leukemia; Megakaryoblastic leukemia; Myeloid sarcoma; and Hairy cell leukemia.

"Epithelial cancers" as defined herein refers to cancer(s) that develops from epithelium or related tissues in the skin, hollow viscera, and other organs. Epithelial cancers include but are not limited to breast cancer, lung cancer, liver cancer, buccal cancer, stomach cancer, colon cancer, nasopharyngeal cancer, dermal cancer, renal cancer, brain tumor, prostate cancer, ovarian cancer, cervical cancer, endometrial cancer, intestinal cancer, pancreatic cancer, and bladder cancer.

"Patient" or "Subject" as used herein refers to a mammalian subject diagnosed with or suspected of having or developing a proliferative disease such as cancer. Exemplary patients may be humans, apes, dogs, pigs, cattle, cats, horses, goats, sheep, rodents and other mammalians that can benefit develop proliferative diseases such as cancer.

As used herein, "substantially purified" or "substantially isolated" refers to a molecule (e.g. a compound) in a state that it is separated from substantially all other molecules normally associated with it in its native state. Preferably, a substantially purified molecule is the predominant species present in a preparation. Particularly, a substantially purified molecule may be greater than 60% free, preferably 75% free, more preferably 90% free, and most preferably 95% free from the other molecules (exclusive of solvent) present in the natural mixture. The term "substantially purified" or "substantially isolated" is not intended to include molecules or substances present in their native state. In certain embodiments, the term "substantially purified" or "substantially isolated" includes purifying one KLH moiety from another KLH moiety (*e.g.*, substantially purifying or substantially isolating a KLH dimer moiety from a KLH trimer moiety). In another embodiment, the term "substantially purified" or "substantially isolated" does not include purifying one KLH moiety from another KLH moiety (e.g. KLH dimers and KLH trimmers are included in a substantially purified or substantially isolated composition) but impurities are substantially removed.

"Administering" is referred to herein as providing a therapeutic composition of the invention to a patient. By way of example and not limitation, composition administration, e.g., injection, may be performed by intravenous (i.v.) injection, subcutaneous (s.c.) injection, intradermal (i.d.) injection, intraperitoneal (i.p.) injection, or intramuscular (i.m.) injection. One or more such routes may be employed. Parenteral administration can be, for example, by bolus injection or by gradual perfusion over time. Alternatively, or concurrently, administration may be by the oral route. Additionally, administration may also be by surgical deposition of a bolus or positioning of a medical device.

"A patient in need thereof' is referred to herein as a patient diagnosed with or suspected of having a proliferative disorder. In one embodiment, the patient has or is likely to develop cancer.

As used herein, the term "antigen" is defined as any substance capable of eliciting an immune response, with or without the help of a protein carrier and/or an adjuvant. Preferably the antigen of the inventive compositions includes a carbohydrate and more preferably glycan-antigen and most preferably a Globo H moiety.

As used herein, the term "immunogenicity" refers to the ability of an immunogen, antigen, or vaccine to stimulate an immune response.

As used herein, the term "immunotherapy" refers to an array of treatment strategies based upon the concept of modulating the immune system to achieve a prophylactic and/or therapeutic goal.

As used herein, the term "epitope" is defined as the parts of an antigen molecule which contact the antigen binding site of an antibody or a T cell receptor.

The "therapeutic compositions" of the invention include "immunogenic conjugates and/or therapeutic conjugates and/or "therapeutic antibodies." The therapeutic conjugates include at least one antigen linked to a carrier. Preferably,

the linkage of the therapeutic conjugate is covalent. In one embodiment of the therapeutic conjugate, the antigen is a glycan such as Globo H moiety, and the carrier is a KLH moiety and/or a KLH moiety subunit. As such, the term therapeutic conjugate encompasses one or more KLH moiety subunits linked to one or more Globo H moieties. In one embodiment, the term therapeutic conjugate encompasses a one or more KLH moieties linked to about or at least 1, 10, $10^2$ or $10^3$ Globo H moieties. In another embodiment, the term therapeutic conjugate encompasses one or more KLH moieties linked to about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, or more Globo H moieties. Another embodiment encompasses isolated dimers, trimers, tetramers, pentamers or hexamers of such Globo H linked KLH moiety subunits, or combinations thereof.

In one embodiment, the therapeutic conjugate is: Fuca(1→2)Galp(1→3)GalNAcβ(1→3)Galα(1→4)Galβ(1→4)Gluβ(1-O-ethylhydrazyl-1-carbonyl-cyclohexyl-4-(methyl-N-maleimido)-3-(thiobutyl-imidyl)-Keyhole Limpet Hemocyanin (KLH) also referred to as OBI-822.

"Therapeutic antibodies" are defined to be as antibodies (as further defined below) that specifically bind the inventive therapeutic conjugates and preferably the Globo H moiety portion of the therapeutic conjugates.

As used herein, the term "vaccine" refers to a therapeutic composition that contains a therapeutic conjugate that is used to confer immunity against a disease associated with the antigen. Cancer vaccines are designed to boost the body's natural ability to protect itself, through the immune system, from dangers posed by damaged or abnormal cells such as cancer cells. A protective immune response is one that reduces the severity of disease, including but not limited to, prevention of disease, delay in onset of disease, decreased severity of symptoms, decreased morbidity, and delayed mortality. Preferably, a vaccine is capable of activating both humoral immune response (e.g. stimulation of the production of antibodies by B lymphocytes) and cellular immune response (e.g. an immune response that is mediated by T-lymphocytes and/or other cells, such as NK cells and macrophages). Standard assays have been developed to determine the immune response such as enzyme-linked immunosorbent assay (ELISA), flow cytometry, cell proliferation assay, CTL assays, and ADCC/CDC assays.

As used herein, the term "glycan" refers to a polysaccharide, or oligosaccharide. Glycan is also used herein to refer to the carbohydrate portion of a glycoconjugate, such as a glycoprotein, glycolipid, glycopeptide, glycoproteome, peptidoglycan, lipopolysaccharide or a proteoglycan. Glycans usually consist solely of O-glycosidic linkages between monosaccharides. For example, cellulose is a glycan (or more specifically a glucan) composed of β-1,4-linked D-glucose, and chitin is a glycan composed of β-1,4-linked N-acetyl-D-glucosamine. Glycans can be homo or heteropolymers of monosaccharide residues, and can be linear or branched. Glycans can be found attached to proteins as in glycoproteins and proteoglycans. They are generally found on the exterior surface of cells. O- and N-linked glycans are very common in eukaryotes but may also be found, although less commonly, in prokaryotes. N-Linked glycans are found attached to the R-group nitrogen (N) of asparagine in the sequon. The sequon is an Asn-X-Ser or Asn-X-Thr sequence, where X is any amino acid except praline. The preferred glycan is a Globo H moiety.

Globo H is a hexasaccharide, which is a member of a family of antigenic carbohydrates that are highly expressed on a various types of cancers, especially cancers of breast, prostate, pancreas, stomach, ovary, colon, and lung. In illustrative embodiments, certain patients exhibited no anti-Globo H antibody levels at time zero, and after immunization with the therapeutic composition of the invention high titers were detected. In other illustrative embodiments, certain patients exhibited anti-Globo H antibody levels at time zero, and after immunization with the therapeutic composition of the invention high titers were detected. In certain embodiments, the anti-Globo H antibody is expressed on the cancer cell surface as a glycolipid and possibly as a glycoprotein. In other embodiments, the serum of breast cancer patients contained high levels of antibodies against the Globo H epitope. In certain embodiments, this epitope is also targeted by the monoclonal antibodies Mbrl, VK9 and anti-SSEA-3 in immunohistochemistry studies. Although certain normal tissues also react with Mbrl, including normal breast, pancreas, small bowel, and prostate tissue, the antigen in these tissues is predominantly localized at the secretary borders where access to the immune system is restricted.

"Globo H moiety" is defined herein to be a glycan (i.e., a molecule containing a sugar moiety) that is Globo H or a fragment or analog thereof. Globo H is a glycan containing the hexasaccharide epitope (Fuc α 1→2 Gal β 1→3 GalNAc β 1→3 Gal α 1→4 Gal β 1→ 4 Glc), and optionally, a non-sugar moiety. Its fragment is a glycan containing a fragment of the hexasaccharide epitope and, if applicable, the non-sugar moiety. These oligosaccharides can be prepared by routine methods. (See Huang et al., Proc. Natl. Acad. Sci. USA 103:15-20 (2006)). If desired, they can be linked to a non-sugar moiety. U.S. patent application Ser. No. 12/485,546 relates to a method of producing antibody specific to Globo H or its fragment by administering to a non-human mammal (e.g., mouse, rabbit, goat, sheep, or horse) the immune composition described above and isolating from the mammalian antibody that binds to Globo H or its fragment.

Analogs of Globo H can be generated using glycan microarray and include those disclosed in Wang et al., Proc

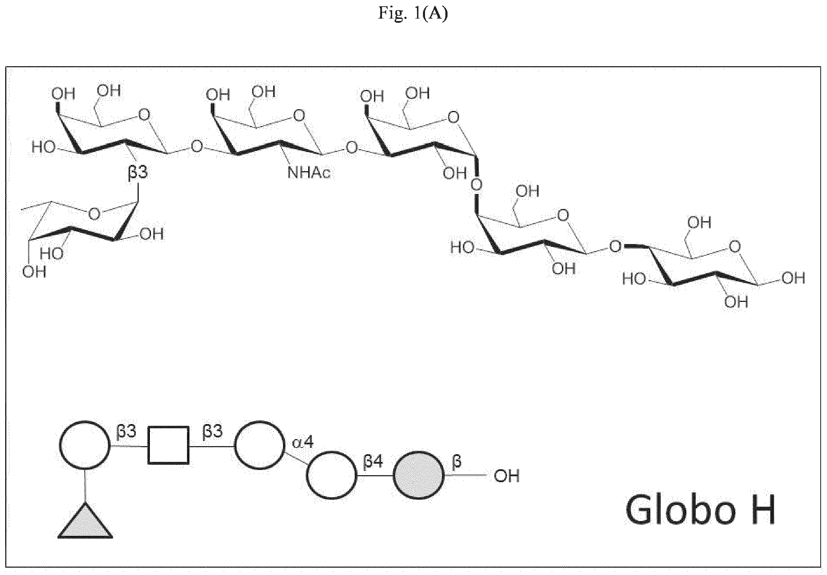

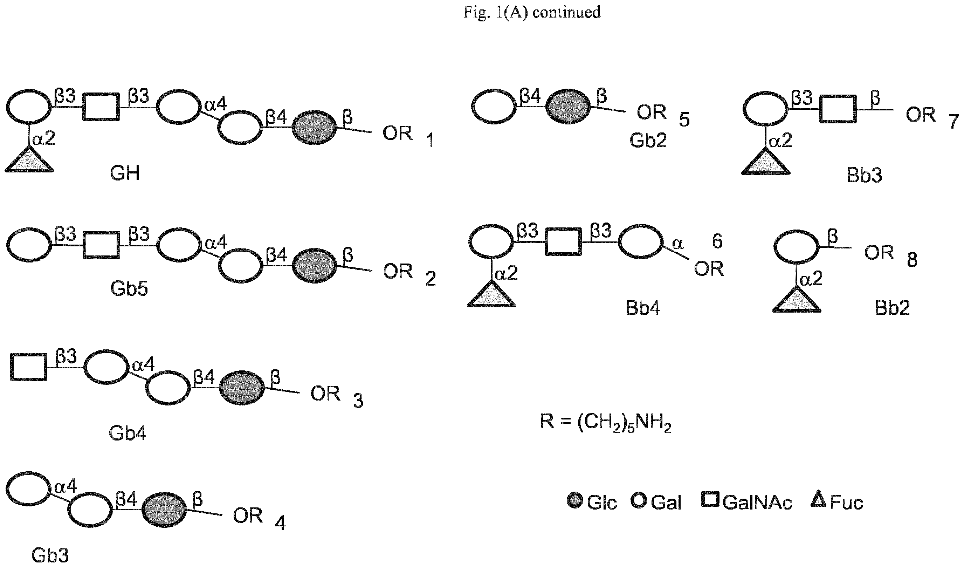

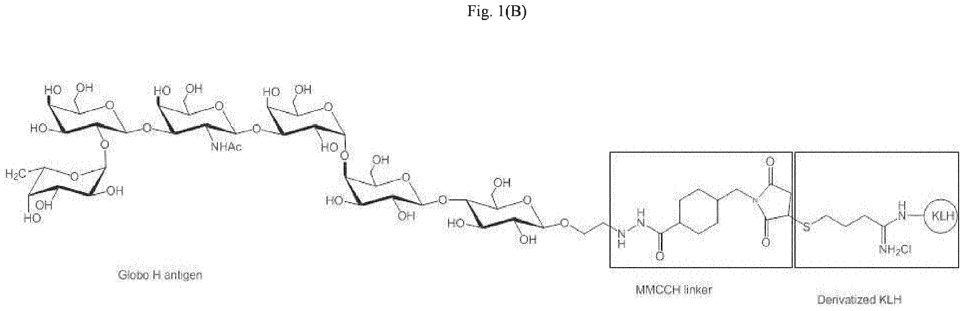

Natl Acad Sci USA. 2008 August 19; 105(33): 11661-11666 and shown in Figure 1.

Globo H analogs preferably bind antibodies VK-9, Mbrl, and anti-SSEA-3. Preferably, the Globo H Analogs bind with a particular dissociation constant (KD,surf). The Langmuir isotherm can be used for analyzing the binding curves to generate the dissociation constants on surface (KD,surf). At the equilibrium conditions during incubation, the mean fluorescence of the replicate spots ($F_{obs}$) can be described by:

$$F_{obs} = F_{max}[P]/(K_{D,surf} + [P])$$

where $F_{max}$ is the maximum fluorescence intensity, a measure of the amount of active carbohydrate on the surface, [P] is the total antibody concentration, and KD,surf is the equilibrium dissociation constant for surface carbohydrate and the antibody. As described in Wang et al. In some embodiments the preferred (KD,surf) of Globo H analogs is at least, about or exactly 0.4, 0.5., 0.6, 0.7, 0.8, 0.9, 1.0, 1.1, 1.2, 1.3, 1.4, 1.5 or 1.6 nM with respect to the VK-9, Mbrl, and anti-SSEA-3 antibodies described in Wang et al.

"Keyhole Limpet Hemocyanin" (KLH) is a large, multisubunit, oxygen-carrying, metalloprotein found in the hemolymph of the giant keyhole limpet, *Megathura crenulata.* KLH is heterogeneous glycosylated protein consisting of subunits with a molecular weight of about 350,000 to about 390,000 in aggregates with molecular weights of about 400 kDa (*e.g.*, a KLH monomer) to about 8000 kDa (*e.g.*, a KLH didecamer). Each domain of a KLH subunit contains two copper atoms that together bind a single oxygen molecule. When oxygen is bound to hemocyanin, the molecule takes on a distinctive transparent, opalescent blue color. In certain embodiments, the KLH protein is potently immunogenic yet safe in humans. In certain embodiments, KLH may be purified from the hemolymph of *Megathura crenulata* by a series of steps that typically includes ammonium sulfate precipitation and dialysis, and may involve chromatographic purification to obtain the highest purity. In certain embodiments, KLH purification may also include endotoxin removal, but this step may be unnecessary because the endotoxin can serve as an adjuvant when injected for antibody production. Preferably, a high quality KLH preparation with the clear opalescent blue color is the best indicator of KLH solubility. In certain embodiments, the KLH monomeric units assemble into a large multimer (decamer or didecamer) with a total molecular weight of about 4,000 kDa to 8,000 kDa. "Keyhole Limpet Hemocyanin moiety" or "KLH moiety" is defined herein to be a KLH1 (SEQ ID NO. 1) or KLH2 (SEQ ID NO. 2) protein or a protein substantially identical thereto or a mixture thereof. Substantially identical in this context means each KLH moiety has an amino sequence at least, about or exactly: 100, 99, 98, 97, 96, 95, 94, 93, 92, 91, 90, 89, 88, 87, 86, 85, 84, 83, 82, 81, 80, 79, 78, 77, 76 or 75 percent identical to that of native wild type KLH. In certain embodiments, the KLH of the invention has enhanced immunogenic activity, particularly enhanced anti- tumor activity. In certain embodiments, the KLH in the composition of the present invention comprises an intact, non-degraded subunit of approximately 400,000 in molecular weight. In other embodiments, the KLH of the invention comprises higher KLH multimers.

In certain embodiments, the higher KLH multimers have molecular weights of approximately 8-10 million with sedimentation coefficients of about 92-107S. The amount of higher KLH multimers present is based on sedimentation-equilibrium and/or sedimentation-velocity ultracentrifugation analyses. In other embodiments, the KLH of the invention demonstrates an enhanced immunogenic activity, particularly enhanced anti-tumor activity. The enhanced immunogenic activity is seen for example, but not limited, (a) with injection of KLH (without adjuvant), (b) with KLH used as an adjuvant, (c) with KLH used as a carrier immunogen for haptens or weakly immunogenic antigens, and (d) with KLH used as an anti-tumor agent. The KLH composition of the invention exhibits enhanced anti-tumor activity for many tumors, including, but not limited to, bladder, breast, ovarian tumors, etc. In certain embodiments, two KLH moieties can form a dimer via a covalent linkage between KLH monomers. Without being limited by theory, it is believed that the covalent linkage between KLH moieties is through a disulfide bond. In certain embodiments, two or more KLH moieties can form a dimer, trimer, tetramer, pentamer, hexamer, etc. via a covalent linkage between KLH monomers, dimers, trimers, etc. Without being limited by theory, it is believed that the covalent linkage between KLH moieties is through a disulfide bond.

There are a variety of methods for linking of a KLH moiety to an antigen, including direct conjugation and conjugation with a bifunctional linker group such as 4- (4-N-maleimidomethyl) cyclohexane-1-carboxyl hydrazide (MMCCH). Such linkage techniques are disclosed in U.S. Patent No. 6,544,952. In some embodiments, to prepare the therapeutic conjugates of the invention, for example, the Globo H allyl glycoside is converted to an aldehyde by ozonolysis and the aldehyde group is attached to the NH groups on the crosslinker MMCCH, giving Globo H-MMCCH; the carrier protein, KLH, is subjected to thiolation to produce KLH-SH; and the sulfhydryl groups on thiolated KLH are then attached to the maleimide group on the MMCCH, producing Globo H-KLH conjugates.

In one embodiment, Globo H allyl glycoside is prepared via chemical synthesis. A thiolating reagent, 2-iminothiolane and cGMP-grade KLH and 4-(4-N-maleimidomethyl)-cyclohexane-1-carboxyl hydrazide (MMCCH) linker are also used. In some embodiments the following steps are carried out: (1) Conversion of Globo H allyl glycoside to the Globo H-aldehyde; (2) Coupling of Globo H-aldehyde with MMCCH to Globo H-MMCCH, separately; (3) Chemical thiolation of KLH; (4) Conjugation of Globo H-MMCCH to the thiolated KLH; and (5) Purification of the Globo H-KLH

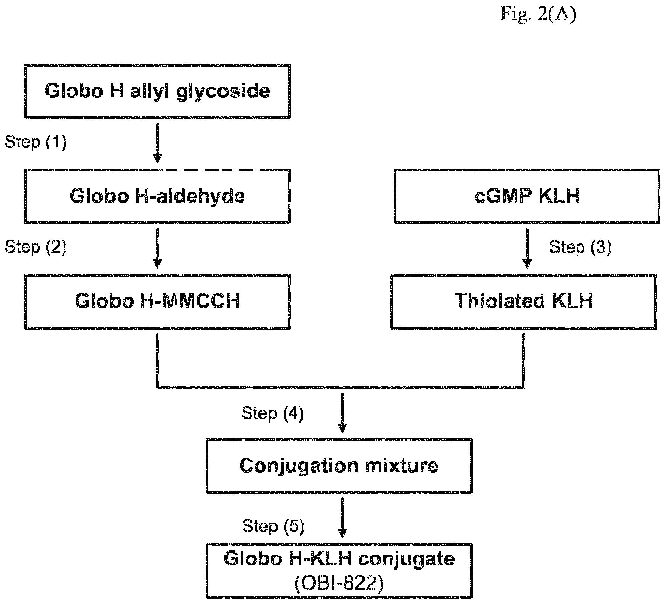

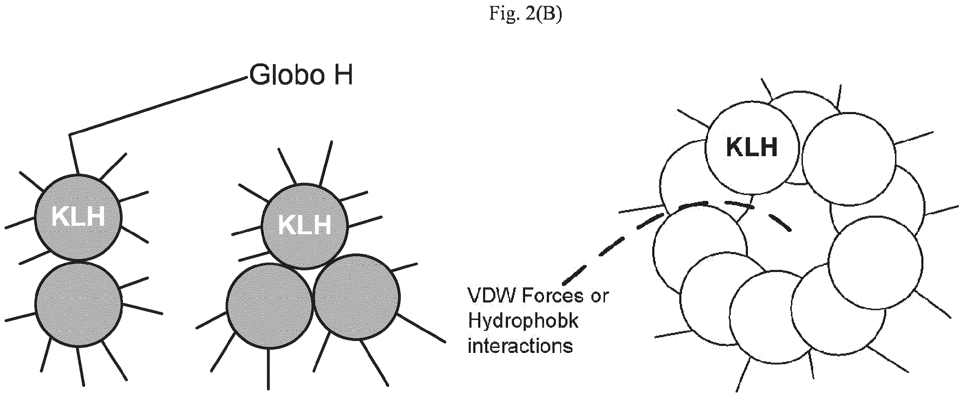

glycoconjugate (OBI-822). The Globo H-KLH subunit conjugation pathway showed in Figure 2A.

In certain embodiments, during conjugation of a Globo H moiety protein to a KLH moiety, a KLH moiety protein in certain embodiments shows a reduction in molecular weight compared to the intact molecule preferably due to Globo H moiety subunit dissociation. In other embodiments, the conjugation methods disclosed herein result in a KLH subunit dissociation not previously reported. While not wishing to be bound to any particular theory, it is envisaged that the high glycosylation level of the inventive Globo H moiety-KLH moiety subunit conjugates results in the formation hydrogen bonding between the Globo H moieties. As such, in certain embodiments, the Van Der Waals forces and hydrophobic interactions between the KLH moiety subunits are displaced by Globo H hydrogen bonding and this leads to KLH moiety subunit separation. Following conjugation, the KLH moiety subunits of a Globo H moiety-KLH moiety conjugate preferably aggregate to form novel monomers, dimers, trimers, tetramers, pentamers, hexamers or any combination thereof. The resulting exemplary therapeutic Globo H moiety-KLH moiety conjugates, with an unexpectedly large epitope/carrier ratio, have surprising and unexpected superior immunogenic attributes. In certain embodiments, the Globo H moieties are conjugated to lysines on KLH1 and KLH2. In other embodiments, the Globo H moieties are not conjugated to lysines on KLH1 and KLH2. In certain embodiments, the Globo H-conjugated lysine sites are found conserved in the peptide mapping analysis suggesting the Globo H-KLH glycoconjugate composition is unique in its structure.

In one embodiment, therapeutic compositions of the invention include one or more KLH moiety subunits wherein at least one such subunit is conjugated to at least, about or exactly 1, 10, 100 or 1000 times: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108,109, 110, 111, 112, 113, 114, 115, 116, 117, 118, 119, 120, 121, 122, 123, 124, 125, 126,127, 128, 129, 130, 131, 132, 133, 134, 135, 136, 137, 138, 139, 140, 141, 142, 143, 144,145, 146, 147, 148, 149, 150, 151, 152, 153, 154, 155, 156, 157, 158, 159 or 160 or more Globo H moieties.

The inventors found using mass spectrometric analysis that the Globo H moieties are conjugated to lysine residues of KLH. In certain embodiments, it is therefore preferred that the Globo H moieties are conjugated to lysine residues.

In one embodiment, there are total of exactly or about 145, 146, 147, 148, 149, 150, 151, 152, 153, 154, 155, 156, 157, 158, 159, 160 total lysine residues per KLH moiety subunit. In another embodiment there are exactly or about 150 or 156 lysine residues per KLH moiety subunit. In another embodiment, there are exactly or about 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109 or 110 lysine conjugation sites on each KLH moiety subunit available for binding to or actually bound to a Globo H moiety. In another embodiment, there are 62, 66, 67, 68, 70, 72, 76, 86, 87, 88, 90, 92, 93, 100 such lysine conjugation sites on each KLH moiety subunit. Lysine conjugation sites are those lysine residues in the KLH moiety which are available for binding or actually bind to a Globo H moiety and/or a linker to a Globo H moiety such as for example an MMCCH linker.

In certain therapeutic embodiments containing a mixture of moiety subunits (e.g., KLH1 and KLH2 or variants thereof), total available lysine (for both subunits) as are counted together across the different subunit types the and may be or are exactly about 290, 291, 292, 293, 294, 295, 296, 297, 298, 299, 300, 301, 302, 303, 304, 305, 306, 307, 308, 309 or 310 in number. In such embodiments, there are or may be exactly or about 130, 131, 132, 133, 134, 135, 136, 137, 138, 139, 140, 141, 142, 143, 144, 145, 146, 147, 148, 149, 150, 151, 152, 153, 154, 155, 156, 157, 158, 159 or 160 lysine conjugation sites together across the different subunits (e.g., KLH1 and KLH2 or variants thereof). In other such embodiments, there are 136, 137, 141, 140, 143, 147 or 155 lysine conjugation sites.

In a most preferred embodiment there are 136, 137, 140, 141, 143, 147 or 155 and lysine conjugation sites among the total 306 lysine residues in KLH1/KLH2.

In certain embodiments, the therapeutic compositions of the invention contain a mixture of KLH moiety subunit-Globo H moiety conjugates wherein such conjugates remain monomers or form dimers, trimers, tetramers, pentamers, hexamers or any combination thereof. In another embodiment, the therapeutic compositions of the invention include isolated KLH moiety subunit-Globo H moiety conjugate monomers, dimers, trimers or tetramers or combinations of thereof. In a further embodiment, the therapeutic compositions of the invention include only KLH moiety subunit-Globo H moiety conjugate dimers and trimers.

In another embodiment, the therapeutic compositions contain at least two KLH moiety subunits wherein each of the two KLH-moiety subunits is linked to different glycans. Other tumor-associated glycan antigens linkable to KLH moiety subunits can include but are not limited to GM2, GD2, GD3, fucosyl, GM1, sTn, sialyl-Lewis$^x$, Lewis$^x$, sialyl Lewis$^a$, Lewis$^a$, sTn, TF, polysialic acid, Lewis$^y$, mucins, T antigen, and the like. In some embodiments only, at least or about 10, 20, 30, 40, 50, 60, 70, 80, 90 or 100 percent of the KLH moiety subunits in a therapeutic composition are linked to a Globo H moiety whereas the remaining KLH moiety subunits in the therapeutic composition are linked to other tumor-associated glycan antigens.

As used herein, "epitope/carrier ratio" relating to the therapeutic conjugates disclosed herein refers to for

example, the relationship of antigen epitopes to carrier molecules in a therapeutic conjugate. Preferably, it refers to the relationship of Globo H moieties to KLH moieties. Most preferably the epitope/carrier ratio of a therapeutic conjugate is calculated using the following formula=(actual Globo H moiety weight/Globo H moiety molecular weight)/(actual KLH moiety weight/KLH moiety molecular weight)combination. Epitope/carrier ratios are readily determinable by those of skill in the art. Preferably, the weights of Globo H are determined for example by high performance anion exchange chromatography with pulsed amperometric detection (HPAEC-PAD).

Preferably, the epitope/carrier ratios of the therapeutic conjugates of the invention are about, at least or exactly: 1, 10, 25, 50, 75, 100, 125, 150, 175, 200, 225, 250, 275, 300, 325, 350, 375, 400, 425, 450, 475, 500, 525, 575, 600, 625, 650, 675, 700, 725, 750, 775, 800, 825, 850, 875, 900, 925, 950, 975, 1000, 1025, 1050, 1075, 1100, 1125, 1150, 1175, 1200, 1225, 1250, 1275, 1300, 1325, 1350, 1375, 1400, 1425, 1450, 1475, 1500, 1525, 1550, 1575, 1600, 1625, 1650, 1675, 1700, 1725, 1750, 1775, 1800, 1825, 1850, 1875, 1900, 1925, 1950, 1975, 2000, 2025, 2050, 2075, 2100, 2125, 2150, 2175, 2200, 2225, 2250, 2275, 2300, 2325, 2350, 2375, 2400, 2425, 2450, 2475, 2500, 2525, 2550, 2575, 2600, 2625, 2650, 2675, 2700, 2725, 2750, 2775, 2800, 2825, 2850, 2875, 2900, 2925, 2950, 2975 or 3000.

In one embodiment, the therapeutic compositions of the invention include a mixture of therapeutic conjugates having a range of epitope/carrier ratios. In one embodiment, the range, the mean or the median epitope/carrier ratios of the therapeutic conjugates in the therapeutic composition is about 10 to about 3200, about 800 to about 2500, about 1000 to about 2000, about 1250 to about 1750 or about 1400 to about 1600. In another embodiment, the range, the mean or the median epitope/carrier ratios of the therapeutic conjugates in the therapeutic composition is about 10 to about 150, about 40 to about 125, about 50 to about 100, about 62 to about 87 or about 70 to about 80. In another embodiment, the range, the mean or the median epitope/carrier ratios of the therapeutic conjugates in the therapeutic composition is about 20 to about 300, about 80 to about 250, about 100 to about 200, about 125 to about 175 or about 140 to about 160. In another embodiment, the range, the mean or the median epitope/carrier ratios of the therapeutic conjugates in the therapeutic composition is about 30 to about 450, about 120 to about 375, about 150 to about 300, about 185 to about 260 or about 210 to about 240. In some the pharmaceutical compositions at least or about 30, 40, 50, 60, 70, 80, 90, 95, 98, 99, or 100% of the therapeutic conjugates exist as monomers, or as dimers, trimers, tetramers, pentamers, hexamers or combinations thereof.

## ANTIBODIES TO THERAPEUTIC CONJUGATES

In certain illustrative embodiments, the invention also encompasses isolated therapeutic antibodies, which specifically bind the therapeutic conjugates disclosed herein with affinity, as well as their use in the treatment and/or diagnosis of proliferative disease.

As used herein, the terms "antibody" and "antibodies" (immunoglobulins) encompass monoclonal antibodies (including full-length monoclonal antibodies), polyclonal antibodies, multispecific antibodies (e.g., bispecific antibodies) formed from at least two intact antibodies, human antibodies, humanized antibodies, camelised antibodies, chimeric antibodies, single-chain Fvs (scFv), single-chain antibodies, single domain antibodies, domain antibodies, Fab fragments, F(ab')2 fragments, antibody fragments that exhibit the desired biological activity, disulfide-linked Fvs (sdFv), and anti-idiotypic (anti-Id) antibodies (including, e.g., anti-Id antibodies to antibodies of the invention), intrabodies, and epitope-binding fragments of any of the above. In particular, antibodies include immunoglobulin molecules and immunologically active fragments of immunoglobulin molecules, i.e., molecules that contain an antigen-binding site Immunoglobulin molecules can be of any type (e.g., IgG, IgE, IgM, IgD, IgA and IgY), class (e.g., IgG1, IgG2, IgG3, IgG4, IgA1 and IgA2) or subclass.

"Affinity" of an antibody for an epitope, e.g., the Globo H moiety of a therapeutic conjugate, to be used in the treatment(s) described herein is a term well understood in the art and means the extent, or strength, of binding of antibody to epitope. Affinity may be measured and/or expressed in a number of ways known in the art, including, but not limited to, equilibrium dissociation constant (KD or Kd), apparent equilibrium dissociation constant (KD' or Kd'), and IC50 (amount needed to effect 50% inhibition in a competition assay). It is understood that, for purposes of this invention, an affinity is an average affinity for a given population of antibodies which bind to an epitope. Values of KD' reported herein in terms of mg IgG per mL or mg/mL indicates mg Ig per mL of serum, although plasma can be used. When antibody affinity is used as a basis for administration of the treatment methods described herein, or selection for the treatment methods described herein, antibody affinity can be measured before and/or during treatment, and the values obtained can be used by a clinician in assessing whether a human patient is an appropriate candidate for treatment.

As used herein, the term "specifically binding," refers to the interaction between binding pairs (e.g., an antibody and an antigen). In various instances, specifically binding can be embodied by an affinity constant of at least or about 10-6 moles/liter, about 10-7 moles/liter, or about 10-8 moles/liter, or less.

Exemplary antibodies against the Globo H may be prepared by collecting body fluid from the immunized subject examined for the increase of desired antibodies such as the serum, and by separating serum from the blood by any conventional method.

Antibodies are generally raised by multiple injections of the relevant antigen and an adjuvant. It may be useful to conjugate the relevant antigen to a protein that is immunogenic in the species to be immunized, e.g., keyhole limpet hemocyanin.

Methods for immunizing animals with antigens are known in the art. Intraperitoneal injection or subcutaneous injection of antigens is a standard method for immunization of mammals. More specifically, antigens may be diluted and suspended in an appropriate amount of phosphate buffered saline (PBS), physiological saline, etc. If desired, the antigen suspension may be mixed with an appropriate amount of an adjuvant, and then administered to the subject.

In certain embodiments, subjects can be boosted until the titer plateaus by several administrations of antigen mixed with an appropriately amount of adjuvant. An appropriate carrier may also be used for immunization. After immunization as above, serum is examined by a method for an increase in the amount of desired antibodies.

## BIOLOGICAL ASSAYS

In one embodiment, when administered to a patient, the therapeutic compositions containing therapeutic conjugates of the invention are able to induce anti-Globo H antibody titers at least or about 1, 2, 3, 4, 5, 6, 7, 8, 9, 10, 11, 12, 13, 14, 15, 16, 17, 18, 19, 20, 50, 100, 250, 500, 1000, 1500, 2000, 2500, 3000, 4000, or 5000 fold greater that the same anti-Globo H antibody titer prior to the administration (i.e., a pre-treatment baseline titer) in the same experiment. In certain embodiments the anti-Globo H antibodies are IgM antibodies. In another embodiment, the anti-Globo H antibodies are IgG antibodies.

The therapeutic compositions of the invention are capable of inducing both humoral and cellular responses in a subject. In certain embodiments, the vaccine composition of the invention induces production of Globo H moiety-specific IgG and IgM antibodies and expansion of B cells and T cells (e.g. $CD3^+$T cells, $CD4^+$T cells and/or $CD8^+$T cells). Typically, these immune responses occur chronologically after administration. In a particular example, after administration, the B cell production appears at about day 3, 4, 5, 6, 7, 8, 9, 10, 20, 30, or 60 days, followed by production of IgG and IgM antibodies at about day 10, 20, 30, 60, or 90 and subsequent T cell production at about day 24, 30, 40, 50, 60, 90, 120, 150, or 180. The vaccine composition of the invention potentially provides a long term immunological protective effect which could prevent the growth of small quantities of cancer cells, thereby being ideal for minimal residual disease so as to achieve disease stabilization and survival improvement.

"Antibody-dependent cell-mediated cytotoxicity" and "ADCC" refer to a cell-mediated reaction in which non-specific cytotoxic cells (e.g., Natural Killer (NK) cells, neutrophils, and macrophages) recognize bound antibody on a target cell and subsequently cause lysis of the target cell. In one embodiment, such cells are human cells. While not wishing to be limited to any particular mechanism of action, these cytotoxic cells that mediate ADCC generally express Fc receptors (FcRs). The primary cells for mediating ADCC, NK cells, express FcγRIII, whereas monocytes express FcγRI, FcγRII, FcγRIII and/or FcγRIV. FcR expression on hematopoietic cells is summarized in Ravetch and Kinet, Annu. Rev. Immunol., 9:457-92 (1991). To assess ADCC activity of a molecule, an in vitro ADCC assay, such as that described in U.S. Pat. No. 5,500,362 or 5,821,337 may be performed. Useful effector cells for such assays include peripheral blood mononuclear cells (PBMC) and Natural Killer (NK) cells. Alternatively, or additionally, ADCC activity of the therapeutic conjugates of the invention may be assessed *in vivo*, e.g., in an animal model such as that disclosed in Clynes et al., Proc. Natl. Acad. Sci. (USA), 95:652-656 (1998).

In certain embodiments, the administration of the pharmaceutical composition can generate an immune response, including generation of antibodies that specifically bind to Globo H. In certain embodiments, the antibodies are developed to target one or more of GloboH expressed on the surface of cancer cells and trigger CDC and/or ADCC to kill these cells. In certain embodiments, the antibodies predominantly include IgG antibodies. In certain embodiments, the immunogenic compositions provided herein mainly induce IgG1, IgG2b, IgG2c and IgG3.

"Complement dependent cytotoxicity" or "CDC" refers to the ability of a therapeutic conjugate to initiate complement activation and lyse a target in the presence of complement. The complement activation pathway is initiated by the binding of the first component of the complement system (C1q) to a molecule (e.g., an antibody) complexed with a cognate antigen. To assess complement activation, a CDC assay, e.g., as described in Gazzano-Santaro et al., J. Immunol. Methods, 202:163 (1996), may be performed.

In another embodiment, when administered to a patient the therapeutic compositions containing therapeutic conjugates of the invention are able to induce the production in a patient/subject of anti-Globo H immune sera, which specifically binds to Globo H positive cancer cell lines, for example,MCF-7 cells.

## COMBINATIONS

Therapeutic compositions can include other anti-cancer/anti-proliferative drugs as well as adjuvants and other immunomodulatory molecules such as cytokines or chemokines. In certain embodiments, the combination can be a co-administration of separate agent/compositions or co-formulation. These agents can be delivered in a kit together in

separate containers or a single container. The agents may be combined at the time of administration or at least or about 1, 2, 3, 4, 5, 6, 7, 8, 9, 10, 11, 12, 13, 14, 15, 16, 17, 18, 19, 20, 21, 22, 23 or 24 minutes, hours or days prior to administration.

Adjuvants are pharmacological or immunological agents that modify the effects of other agents. They can be an inorganic or organic chemical, macromolecule or whole cancer cells or portions thereof which enhance the immune response to given antigen. Adjuvants include complete and incomplete Freund's adjuvant, Toll-Like Receptor molecules and mimetics thereof, LPS, lipoproteins, lipopeptides, flagellin, double- stranded RNA, unmethylated CpG islands, levamisole, bacillus Calmette-Guerin, octreotide, isoprinosine and Zadaxin, various forms of DNA and RNA classically released by bacteria and viruses, PD-1 antagonists and CTLA antagonists. In one embodiment, the adjuvant is a saponin adjuvant.

In certain embodiment, the saponin adjuvant is OBI-821 saponin, which is substantially pure. In other embodiments, the OBI-821 saponin is a biologically active fragments thereof. The adjuvant may also encompass impure forms of OBI-821 saponins. The purified OBI-821 saponins exhibit enhanced adjuvant effect when administered with a vaccine described herein or admixed with other substantially pure saponin or non-saponin adjuvants.

OBI-821 saponins are naturally occurring glycosides, extracted in high purify from the bark of the *Quillaja saponaria* Molina tree, by high pressure liquid chromatography (HPLC), low pressure liquid silica chromatography, and hydrophilic interactive chromatography (HILIC) as described in, for example, U.S. Patent No. 5,057,540 and U.S. Patent No. 6,524,584. High-pressure liquid chromatography analysis shows that OBI-821 are a mixture of structurally related isomeric compounds. Different purified isomeric compounds of OBI-821 saponins have been identified and disclosed herein.

In certain embodiments, OBI-821 saponin comprise at least one isolated compound of formula I as follows:

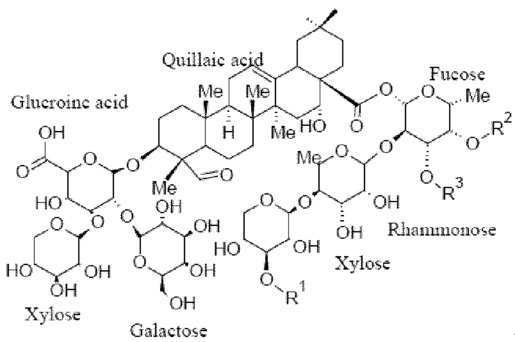

Formula (I)

Wherein

R1 is β-D-Apiose or β-D-Xylose; and

R2 and R3 are independently H, alkyl,

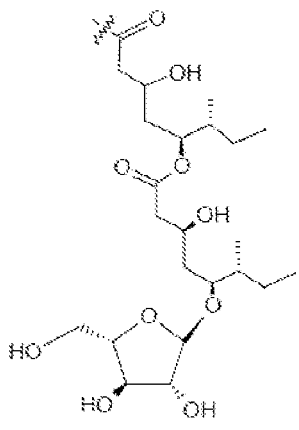

(fatty acyl moiety for Compound 1989),

or

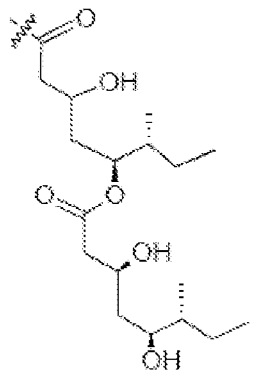

(fatty acyl moiety for Compound 1857).

OBI-821 saponin can also comprise an isolated compound of formula I, wherein

(i) $R^1$ is β-D-Apiose, $R^2$ is the fatty acyl moiety for the 1989 compound depicted above, and $R^3$ is H (1989 compound V1A);
(ii) $R^1$ is β-D-Apiose, $R^2$ is H, and $R^3$ is the fatty acyl moiety fatty acyl moiety for the 1989 compound depicted above (1989 compound V1B);
(iii) $R^1$ is β-D-Xylose, $R^2$ is the fatty acyl moiety fatty acyl moiety for the 1989 compound depicted above, and $R^3$ is H (1989 compound V2A); or
(iv) $R^1$ is β-D- Xylose, $R^2$ is H, and $R^3$ is the fatty acyl moiety fatty acyl moiety for the 1989 compound depicted above (1989 compound V2B). Collectively, 1989 compound VIA, 1989 compound V1B, 1989 compound V2A and 1989 compound V2B are called "1989 compounds mixture."

Table 1 summarizes the functional groups of 1989 compounds and the mole % of each 1857 compound in the 1857 compounds mixture.

Table 1

| Mole % | $R^1$ | $R^2$ | $R^3$ |
|---|---|---|---|
| 1989 Compound V1A 64.5% | β-D-Apiose 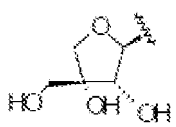 | 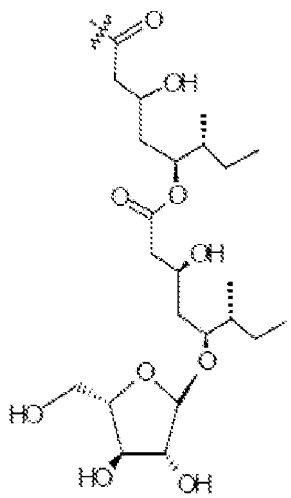 | H |

(continued)

| Mole % | $R^1$ | $R^2$ | $R^3$ |
|---|---|---|---|
| 1989 Compound V1B 1.5% | β-D-Apiose 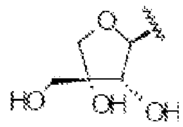 | H | 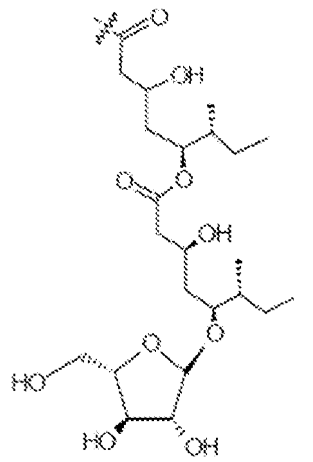 |
| 1989 Compound V2A 33.3% | β-D-Xylose 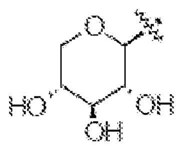 | 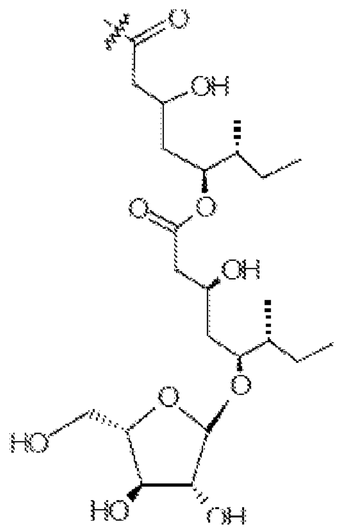 | H |
| 1989 Compound V2B 0.7% | β-D-Xylose 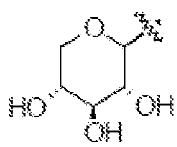 | H | 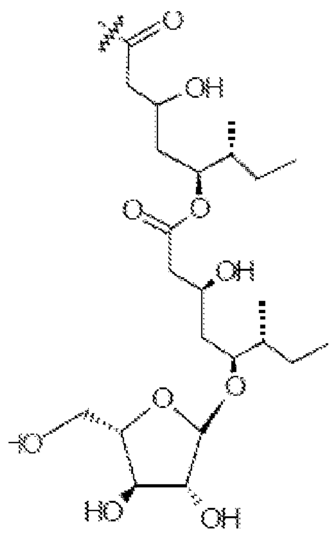 |

OBI-821 saponin can comprise an isolated compound of formula I where:

(i) $R^1$ is β-D-Apiose, $R^2$ is the fatty acyl moiety for the 1857 compound depicted above, and $R^3$ is H (1857 compound

V1A);
(ii) $R^1$ is β-D-Apiose, $R^2$ is H, and $R^3$ is the fatty acyl moiety for the 1857 compound depicted above (1857 compound V1B);
(iii) $R^1$ is β-D-Xylose, $R^2$ is the fatty acyl moiety for the 1857 compound depicted above, and $R^3$ is H (1857 compound V2A); or
(iv) $R^1$ is β-D- Xylose, $R^2$ is H, and $R^3$ is the fatty acyl moiety for the 1857 compound depicted above (1857 compound V2B).
Collectively, 1857 compound VIA, 1857 compound V1B, 1857 compound V2A and 1857 compound V2B are called "1857 compounds mixture."

Table 2 summarizes the functional groups of 1857 compounds and the mole % of each 1857 compound in the 1857 compounds mixture. HPLC.

Table 2

| Mole % | $R^1$ | $R^2$ | $R^3$ |
|---|---|---|---|
| 1857 Compound V1A 64.7% | β-D-Apiose 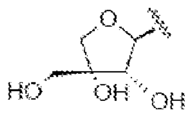 | 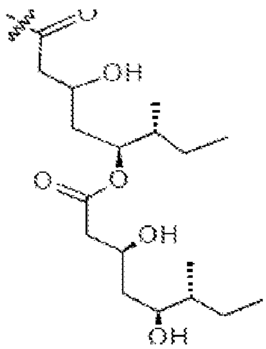 | H |
| 1857 Compound V1B 1.3% | β-D-Apiose 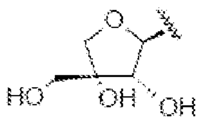 | H | 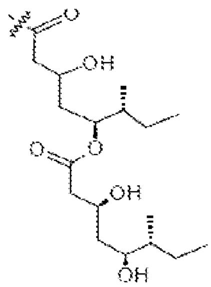 |
| | | | |
| 1857 Compound V2A 33.4% | β-D-Xylose 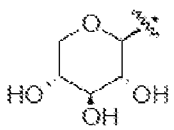 | 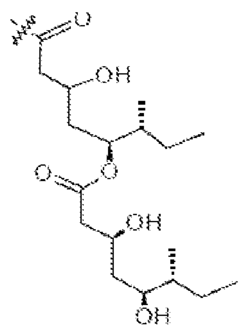 | H |

(continued)

| Mole % | R1 | R2 | R3 |
|---|---|---|---|
| 1857 Compound V2B 0.6% | β-D-Xylose 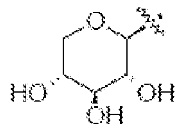 | H | 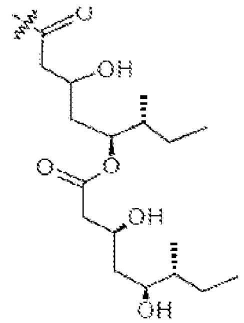 |

OBI-821 saponin comprises one or more of the following compounds:

(i) 1857 compound V1A;
(ii) 1857 compound V1B;
(iii) 1857 compound V2A;
(iv) 1857 compound V2B;
(v) 1989 compound V1A;
(vi) 1989 compound V1B;
(vii) 1989 compound V2A; or
(viii) 1989 compound V2B.

The percentages of the 1857 compounds mixture and the 1989 compound mixture in OBI-821 saponin can range as follows:

(i) about 1 mole % to about 15 mole % of OBI-821 comprising an 1857 compounds mixture; and
(ii) about 85 mole % to about 99 mole % of OBI-821 comprising an 1989 compounds mixture.

All of the mole % can be varied by 0.1% increment (e.g. about 87% to about 90%, about 90.5% to about 97%, about 3.5% to about 11%, about 10% to about 14%).

The 1989 compounds mixture may comprise about 60-70 mole % of 1989 compound V1A; about 1-5 mole % of 1989 compound V1B; about 30-40 mole % of 1989 compound V2A; and about 0.1-3 mole % of 1989 compound V2B. All of the mole % can be varied by 0.1 increment (e.g. 65%, 2.5%, 35.6%).

The 1857 compounds mixture may comprise about 60-70 mole % of 1857 compound V1A; about 1-5 mole % of 1857 compound V1B; about 30-40 mole % of 1857 compound V2A; and, about 0.1-3 mole % of 1857 compound V2B. All of the mole % can be varied by 0.1 increment (e.g., 65%, 2.5%, 35.6%).

In another embodiment, the substantially pure OBI-821 is purified from a crude Quillaja saponaria extract, wherein said OBI-821 is characterized by a single predominant peak which comprises 90% or more of the total area of all peaks of a chromatogram, excluding the solvent peak, when analyzed on reverse phase-HPLC on a Symmetry C18 column having 5 um particle size, 100 Å pore, 4.6mm IDx25cm L with a elution program comprising mobile phase of A:B 95%:5% to 75%:25% in 11 minutes , which mobile phase A is distilled water with 0.1% trifluoroacetic acid, and mobile phase B is acetonitrile with 0.1 % trifluoroacetic acid at a flow rate of 1ml/min.

In one embodiment, the pharmaceutical composition comprises the compound of formula (I)

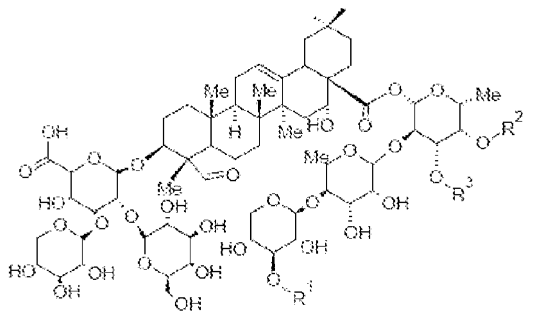

Formula (I)

wherein,

$R^1$ is β-D-Apiose or β-D-Xylose; and

$R^2$ and $R^3$ are independently H, alkyl, or

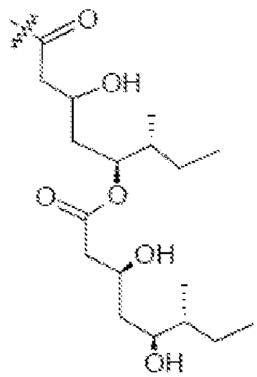

(Fatty acyl moiety for the 1857 Compound), and a pharmaceutically acceptable carrier.

The vaccine can comprise a carbohydrate antigen or its immunogenic fragment and an OBI-821 saponin. In yet another embodiment, the vaccine comprises a carbohydrate antigen or its immunogenic fragment; a carrier protein and an OBI-821 saponin. In another embodiment, the vaccine comprises a carbohydrate antigen selected from Globo H, KLH, and an OBI-821 saponin. Non limiting examples of carrier protein include KLH.

The terms "a-galactosyl-ceramide" and "a-GalCer" refer to a glycolipid that stimulates natural killer T cells to produce both T helper 1 (TH1) and TH2 cytokine, as described in US Pat. No. 8,268,969, the content of which is incorporate by reference in its entirety. In certain embodiment, OBI-834 (known as C34) adjuvant is characterized by the following exemplary structure:

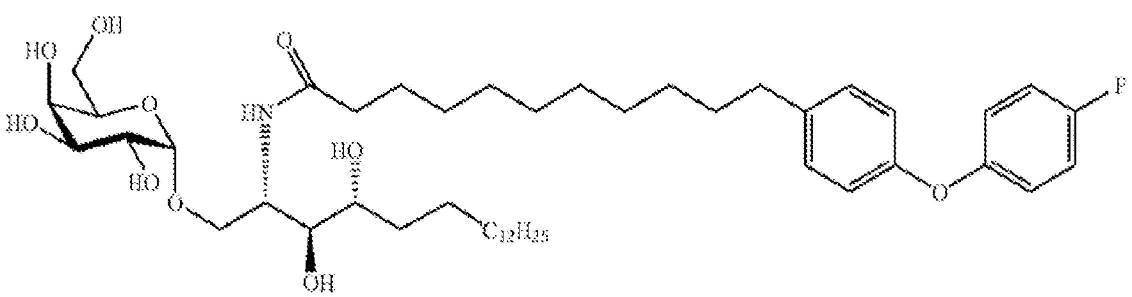

As used herein, the term "cytokine" refers to any of numerous small, secreted proteins that regulate the intensity and duration of the immune response by affecting immune cells differentiation process usually involving changes in gene expression by which a precursor cell becomes a distinct specialized cell type. Cytokines have been variously named as lymphokines, interleukins, and chemokines, based on their presumed function, cell of secretion, or target of action. For example, some common interleukins include, but are not limited to, IL-2, IL-12, IL-18, IL-2, IFN-γ, TNF, IL-4, IL-10, IL-13, IL-21, GM-CSF, and TGF-β.

As used herein, the term "chemokine" refers to any of various small chemotactic cytokines released at the site of infection that provide a means for mobilization and activation of lymphocytes. Chemokines attract leukocytes to infection sites. Chemokines have conserved cysteine residues that allow them to be assigned to four groups. The groups, with representative chemokines, are C-C chemokines (RANTES, MCP-1, MIP- 1α, and MIP-1β), C-X-C chemokines (IL-8), C chemokines (Lymphotactin), and CXXXC chemokines (Fractalkine).

The therapeutic compositions of the invention can further include PD-1/PD-L1 inhibitors (cytotoxic T cell lymphocyte (CTLs) immunotherapy), CTLA-4 immunotherapy, CDK4/6 inhibitors (target therapy), PI3K inhibitors (target therapy), mTOR inhibitors (target therapy), AKT inhibitors (target therapy), Pan-Her inhibitors (target therapy). These inhibitors can be modified to generate the respective monoclonal antibody as well. Such antibodies can be included in therapeutic compositions of the invention.

The therapeutic compositions can include other anti-cancer/anti-proliferative or chemotherapeutic agents. In some embodiments, examples of such agents are found in Cancer Principles and Practice of Oncology by V.T. Devita and S. Hellman (editors), 6th edition (February 15, 2001), Lippincott Williams & Wilkins Publishers. Such anti-cancer agents include, but are not limited to, the following: hormonal therapeutic agents (e.g., selective estrogen receptor modulators, androgen receptor modulators), monoclonal antibody therapy, chemotherapy, retinoid receptor modulators, cytotoxic/cytostatic agents, antineoplastic agents, antiproliferative agents, prenyl-protein transferase inhibitors, HMG-CoA reductase inhibitors, nitrogen mustards, nitroso ureas, angiogenesis inhibitors (e.g., bevacizumab), inhibitors of cell proliferation and survival signaling pathway, apoptosis inducing agents, agents that interfere with cell cycle checkpoints, agents that interfere with receptor tyrosine kinases (RTKs), mammalian target of rapamycin (mTOR) inhibitors, human epidermal growth factor receptor 2 (HER2) inhibitors, epidermal growth factor receptor (EGFR) inhibitors, integrin blockers, NSAIDs, PPAR agonists, inhibitors of inherent multidrug resistance (MDR), anti-emetic agents, agents useful in the treatment of anemia, agents useful in the treatment of neutropenia, immunologic-enhancing drugs, biphosphonates, aromatase inhibitors, agents inducing terminal differentiation of neoplastic cells, γ-secretase inhibitors, cancer vaccines, and any combination thereof.

## FORMULATIONS OF THE INVENTION

The therapeutic compositions (also referred to herein as pharmaceutical compositions) generally include a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions. A pharmaceutical composition is formulated to be compatible with its intended route of administration. Examples of routes of administration include parenteral, e.g., intravenous, intradermal, subcutaneous, intramuscular, intra-arterial, oral (e.g., inhalation), transdermal (topical), transmucosal, and rectal administration. Solutions or suspensions used for parenteral, intradermal, or subcutaneous application can include the following components: a sterile diluent such as water for injection, saline solution, phosphate buffered saline, tris-buffered saline, fixed oils, polyethylene glycols, glycerine, propylene glycol, or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates, or phosphates and agents for the adjustment of tonicity such as sodium chloride or dextrose. The pH value can be adjusted with acids or bases, such as hydrochloric acid or sodium hydroxide. The parenteral preparation can be enclosed in ampoules, disposable syringes, or multiple dose vials made of glass or plastic.

Pharmaceutical compositions suitable for an injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL® (BASF, Parsippany, N.J.), or phosphate buffered saline (PBS). In all cases, the composition should be sterile and should be fluid to the extent that easy syringability exists. It should be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, or sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered

sterilization. Generally, dispersions are prepared by incorporating the active compound into a sterile vehicle which contains a basic dispersion medium and the required other ingredients from those enumerated above. In the case of sterile powders for the preparation of sterile injectable solutions, methods of preparation include vacuum drying and freeze-drying, which yields a powder of the active ingredient plus any additional desired ingredient from a previously sterile- filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

Furthermore, for oral administration, the formulations of the invention can take the form of, for example, tablets or capsules prepared by conventional means with pharmaceutically acceptable excipients such as binding agents (e.g., pregelatinized maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets can be coated by methods well known in the art. The compositions of the invention can be also introduced in microspheres or microcapsules, e.g., fabricated from poly-glycolic acid/lactic acid (PGLA) (see, U.S. Pat. Nos. 5,814,344; 5,100,669 and 4,849,222; PCT Publication Nos. WO 95/11010 and WO 93/07861). Liquid preparations for oral administration can take the form of, for example, solutions, syrups, emulsions or suspensions, or they can be presented as a dry product for reconstitution with water or other suitable vehicle before use. Such liquid preparations can be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations can also contain buffer salts, flavoring, coloring and sweetening agents as appropriate. Preparations for oral administration can be suitably formulated to give controlled release of the active compound.

For administration by inhalation, the compounds are delivered in the form of an aerosol spray from pressured container or dispenser which contains a suitable propellant, e.g., a gas such as carbon dioxide, or a nebulizer.

Systemic administration can also be transmucosal or transdermal. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration may be accomplished through the use of nasal sprays or suppositories. For transdermal administration, the active compounds are formulated into ointments, salves, gels, or creams as generally known in the art. The compounds can also be prepared in the form of suppositories (e.g., with conventional suppository bases such as cocoa butter and other glycerides) or retention enemas for rectal delivery.

According to implementations, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially. Liposomal suspensions (including liposomes targeted to infected cells with monoclonal antibodies to cell-specific antigens) can also be used as pharmaceutically acceptable carriers. These can be prepared according to methods known to those skilled in the art, for example, as described in U.S. Pat. No. 4,522,811, which is incorporated by reference herein.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

The immunogenic formulations of the invention can be delivered parenterally, i.e., by intravenous (i.v.), subcutaneous (s.c.), intraperitoneal (i.p.), intramuscular (i.m.), subdermal (s.d.), or intradermal (i.d.) administration, by direct injection, via, for example, bolus injection, continuous infusion, or gene gun (e.g., to administer a vector vaccine to a subject, such as naked DNA or RNA). Formulations for injection can be presented in unit dosage form, e.g., in ampoules or in multi-dose containers, with an added preservative. The compositions can take such forms as excipients, suspensions, solutions or emulsions in oily or aqueous vehicles, and can contain formulatory agents such as suspending, stabilizing and/or dispersing agents. Alternatively, the active ingredient can be in powder form for reconstitution with a suitable vehicle, e.g., sterile pyrogen-free water, before use.

The present invention also contemplates various mucosal vaccination strategies.

## DOSAGE FORMS

Toxicity and therapeutic efficacy of such therapeutic compositions may be determined by standard pharmaceutical procedures in cell cultures or experimental animals, e.g., for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. Therapeutic compositions which exhibit high therapeutic indices are preferred. While compounds that exhibit toxic side effects can be used, care should be taken to design a delivery system that targets such compounds to the site of affected location to minimize potential damage to uninfected cells and, thereby, reduce side effects.

Data obtained from cell culture assays and animal studies can be used in formulating a range of dosage for use in humans. The dosage of such compounds lies preferably within a range of circulating concentrations that include the $ED_{50}$ with little or no toxicity. The dosage can vary within this range depending upon the dosage form employed and the route of administration utilized. For any compound used in the method of the disclosure, the therapeutically effective dose can be estimated initially from cell culture assays. A dose can be formulated in animal models to achieve a circulating plasma concentration range that includes the $IC_{50}$ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

In the disclosed compositions, both the antigen and the adjuvant are present in immunogenically effective amounts. For each specific antigen, the optimal immunogenically effective amount should be determined experimentally (taking into consideration specific characteristics of a given patient and/or type of treatment). Generally, this amount is in the range of 0.01 μg-250 mg of an antigen per kg of the body weight. For certain exemplary adjuvant of the present invention, the immunogenically effective amount can be in the range of 10-250 μg of the adjuvant per kg of the body weight.

In some embodiments, a therapeutically effective amount of a therapeutic composition (i.e., an effective dosage) may range from about 0.001μg/kg to about 250 g/kg, 0.01 μg/kg to 10 g/kg, or 0.1 μg/kg to 1g/kg or about or at least: 0.001, 0.002, 0.003, 0.004, 0.005, 0.006, 0.007, 0.008, 0.009; 0.01, 0.02, 0.03, 0.04, 0.05, 0.06, 0.07, 0.08, 0.09;0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 125, 150, 175, 200, 225, or 250 grams or micrograms per kilogram of patient body weight, or any range between any of the numbers listed herein, or other ranges that would be apparent and understood by artisans without undue experimentation. The skilled artisan will appreciate that certain factors can influence the dosage and timing required to effectively treat a subject, including but not limited to the severity of the disease or disorder, previous treatments, the general health or age of the subject, and other diseases present.

In other embodiments, a therapeutically effective amount of Globo-H moiety in the therapeutic composition (*i.e.,* an effective dosage) may range from about 0.001μg/kg to about 250 g/kg, 0.01 μg/kg to 10 g/kg, or 0.1 μg/kg to 1g/kg or about or at least: 0.001, 0.002, 0.003, 0.004, 0.005, 0.006, 0.007, 0.008, 0.009; 0.01, 0.02, 0.03, 0.04, 0.05, 0.06, 0.07, 0.08, 0.09;0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 125, 150, 175, 200, 225, or 250 grams or micrograms per kilogram of patient body weight, or any range between any of the numbers listed herein, or other ranges that would be apparent and understood by artisans without undue experimentation. The skilled artisan will appreciate that certain factors can influence the dosage and timing required to effectively treat a subject, including but not limited to the severity of the disease or disorder, previous treatments, the general health or age of the subject, and other diseases present.

In certain embodiments, the therapeutic compositions disclosed herein contain or are associated with, at least one therapeutic conjugate or therapeutic antibody whereby each at least one therapeutic conjugate or therapeutic antibody is present in single dose at a concentration of about, at least or more than: 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 each times $10^{-9}$, $10^{-8}$, $10^{-7}10^{-7}10^{-6}10^{-5}$ $10^{-4}10^{-3}10^{-2}10^{-1}$ molar per dose. Preferably, the therapeutic conjugate is present in single dose at a concentration between about: 1-100, 2- 60, 3-50, 4-40, 5-30, 6-20, 7-15, 8-10, 2-18, 3-16, 4-14, 5-12, 6-10 or 7-8 μM, or any range between any of the numbers listed herein.

In some embodiments, the therapeutic compositions disclosed herein contain or are associated with, at least one therapeutic conjugate or therapeutic antibody whereby each at least one therapeutic conjugate or therapeutic

antibody is present in single dose at a concentration of about, at least or more than: 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 each times $10^{-3}$, $10^{-2}$, $10^{-1}$, or 10 micrograms. In certain embodiments about or at least 10, 20, 30, 40, 50, 60, 70, 80, 90, 100, 110, 120, 130, 140, 150, 200, 250 or more micrograms of one therapeutic conjugate or therapeutic antibody is included per dose.

In certain embodiments, the therapeutic compositions disclosed herein are administered in a dose about or at least or more than: 1, 2, 3, 4, 5, 6, 7, 8, 9, 10, 11, 12, 13, 14, 15, 16, 17, 18, 19, 20, 21, 22, 23 or 24 times per day, week or month over a period of about or at least or more than: 1, 2, 3, 4, 5, 6, 7, 8, 9, 10, 11, 12, 13, 14, 15, 16, 17, 18, 19, 20, 21, 22, 23 or 24 days, weeks, months, or years.

Effective Dose and Safety Evaluations

According to the methods of the present invention, the pharmaceutical and vaccine compositions described herein are administered to a patient at immunogenically effective doses, preferably, with minimal toxicity. As recited in the Section entitled "Definitions", "immunogenically effective dose" or "therapeutically effective dose" of disclosed formulations refers to that amount of an antigen and/or adjuvant that is sufficient to produce an effective immune response in the treated subject and therefore sufficient to result in a healthful benefit to said subject.

## KITS

According to another aspect, one or more kits of parts can be envisioned by the person skilled in the art, the kits of parts to perform at least one of the methods herein disclosed, the kit of parts comprising one or more therapeutic conjugates, anti- cancer/antiproliferative agents, adjuvants, cytokines and/or chemokines. The therapeutic compositions comprising alone or in combination an effective amount of the therapeutic compositions disclosed herein according to the at least one of the above mentioned methods. The aforementioned agents may come in a single container or in different containers in the kit.

The compositions may, if desired, be presented in a pack or dispenser device which may contain one or more unit dosage forms containing the active ingredient (i.e., an antigen and/or a glycosphingolipids (GSLs) -containing adjuvant). The pack may, for example, comprise metal or plastic foil, such as a blister pack. The pack or dispenser device may be accompanied by instructions for administration. Compositions of the invention formulated in a compatible pharmaceutical carrier may also be prepared, placed in an appropriate container, and labeled for treatment of an indicated condition.

The kits possibly include also identifiers of a biological event, or other compounds identifiable by a person skilled upon reading of the present disclosure. The kit can also comprise at least one composition comprising an effective amount of the therapeutic compositions disclosed herein. The therapeutic compositions of the kits to perform the at least one method herein disclosed according to procedure identifiable by a person skilled in the art.

The disclosure also includes methods of treating proliferative diseases utilizing the therapeutic compositions disclosed herein. In one embodiment, the methods involve the treatment of cancer, e.g., breast cancer. The methods generally involve providing the therapeutic compositions disclosed herein to a patient in need thereof in an amount effective to treat the proliferative disorder.

In some embodiments, the therapeutic compositions of the invention are administered to a subject in need thereof (e.g., one having a cancer such as breast cancer) in a method that on average extends progression free survival or overall survival over a control placebo, e.g., a phosphate buffered saline placebo, by about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 days, weeks, months, or years.

In some embodiments, the therapeutic compositions are given subcutaneously on week 0-2, 6, 14, and 26 in the absence of unacceptable toxicity or disease progression.

In some embodiments, the therapeutic compositions of the invention are administered to a subject in need thereof (e.g., one having a cancer such as breast cancer) in a method that on average shrinks the volume of a tumor in the patient relative to a control placebo, e.g., a phosphate buffered saline placebo, by about or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51 52 53 54 55 56 57 58 59 60 61 62 63 64 65 66 67 68 69 70 71 72 73 74 74 76 77 78 79 80 81 82 83 84 85 86 87 88 89 90 91 92 93 94 95 96 97 98 99 or more percent over the course of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 days, weeks, months, or years.

In some embodiments, tumors volumes may be accurately measured in at least one dimension (longest diameter

in the plane of measurement is to be recorded) with a minimum size of 10 mm by CT scan (CT scan slice thickness recommended to be in between 2.5 mm and 5 mm).

## METHODS OF SYNTHESIZING THE COMPOSITIONS OF THE INVENTION

The Globo H hexasaccharide portion of the therapeutic compositions of the invention was chemically synthesized as the allyl glycoside and then prepared for conjugation with KLH.

In one illustrative embodiment, the chemical synthesis of Globo H involves the following general steps:

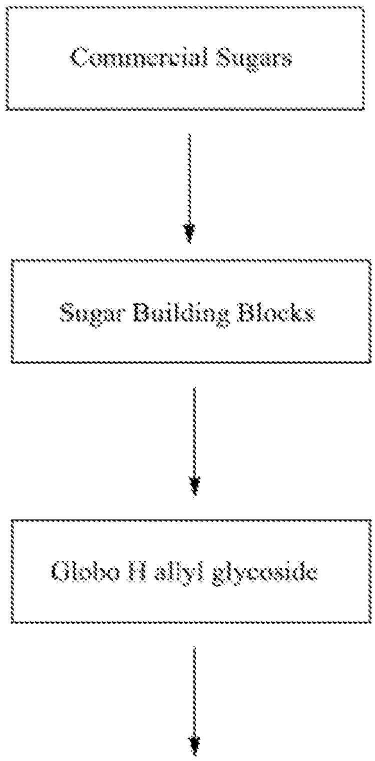

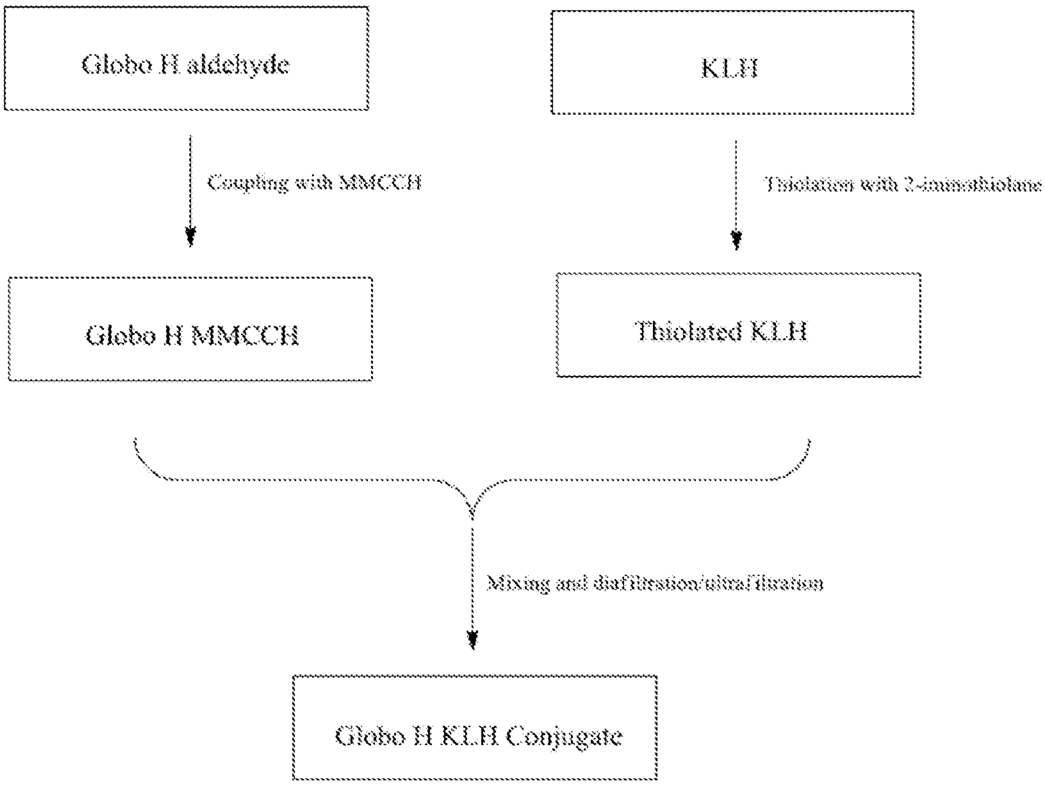


KLH was treated with 2-iminothiolane in an aqueous buffer. The thiolated KLH was then isolated from the unreacted 2-iminothiolane, via a size exclusion column of Sephadex G-15 column. The thiolated KLH was stored under inert gas (nitrogen or argon) atmosphere and used immediately for the conjugation with Globo H-MMCCH.

## EXAMPLES

Example 1: Preparation of Glycoconjugate of the Invention (Globo H-KLH)

Globo H allyl glycoside (commercially available) was converted to an aldehyde by ozonolysis. Globo H aldehyde was reacted with MMCCH linker and $NaCNBH_3$ to give Globo H-MMCCH. The mixture was purified with a column to receive Globo H-MMCCH. The fraction with Globo H-MMCCH positive was confirmed by high performance liquid chromatography (HPLC) and then pooled together. KLH was dissolved in thiolation buffer and 2-iminothiolane was added into the reaction by portion. The reaction was incubated to completion and then KLH-SH was purified by a column. Globo H-MMCCH and KLH-SH were combined. The reaction was stirred to completion. Globo H-KLH glycoconjugate was then purified to provide the final product.

Example 2: Analysis of Weight Ratio of Globo H to KLH in the Glycoconjugate

The weight ratio of Globo H and KLH in the glycoconjugate as prepared was confirmed by high performance anion exchange chromatography with pulsed amperometric detection (HPAEC-PAD). The result was shown in Table 3.

Table 3: The weight ratio of Globo H and KLH in glycoconjugate

| Glycoconjugate epitope/carrier ratio (KLH Didecamer MW: 8,600,000 Da) | Epitope/carrier ratio (KLH monomer MW: 400 kDa) | Globo H Weight to KLH moiety Weight (mg/mg) |
|---|---|---|
| 3000 | 150 | 0.368 |
| 1950 | 97.5 | 0.239 |
| 1500 | 75 | 0.184 |
| 1050 | 52.5 | 0.129 |

(continued)

| Glycoconjugate epitope/carrier ratio (KLH Didecamer MW: 8,600,000 Da) | Epitope/carrier ratio (KLH monomer MW: 400 kDa) | Globo H Weight to KLH moiety Weight (mg/mg) |
|---|---|---|
| 300 | 15 | 0.037 |
| 100 | 5 | 0.012 |
| 20 | 1 | 0.002 |

Example 3: Analysis of Epitope/carrier Ratio of Globo H to KLH in the Glycoconjugate

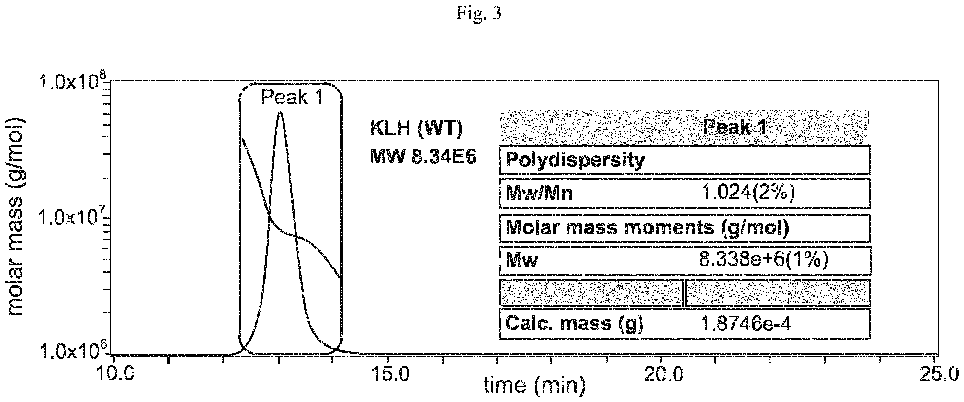

The molecular weight of a KLH didecamer (the naturally aggregated form) is around 7.5 MDa ~ 8.6 MDa, as described in literatures, such as Micron 30 (1999) 597- 623. The native KLH was confirmed by the size exclusion chromatography and multi-angle laser scattering spectrometry (MALS), having the molecular weight of around 8.6 MDa (see Figure 3).

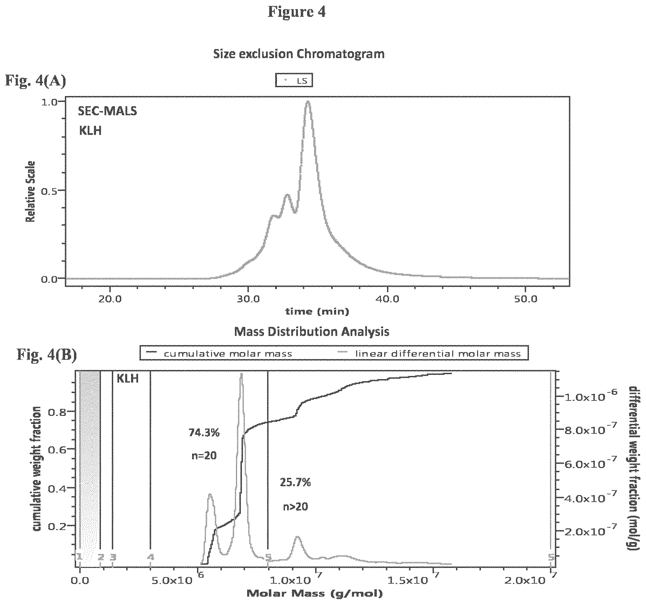

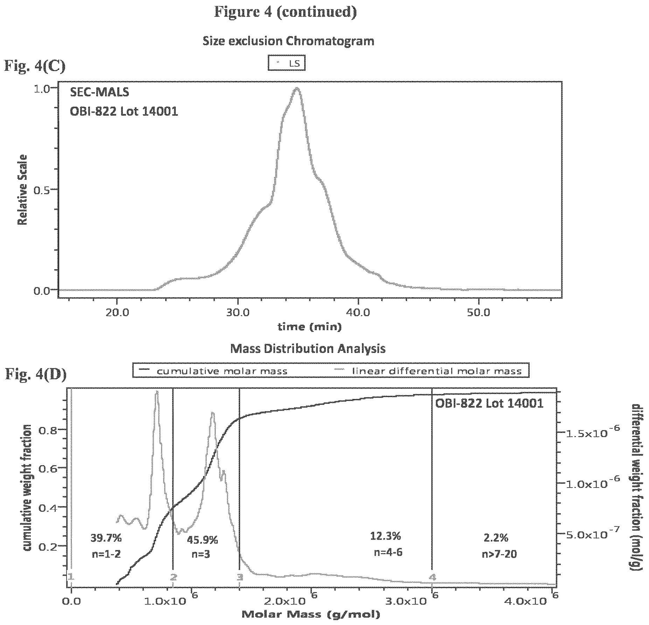

The mass distribution of KLH and Globo H-KLH glycoconjugate (OBI-822, Lot No. 14001) was estimated and derived by size exclusion chromatography using multi-angle laser scattering spectrometer (SEC-MALS). The molecular weight was calculated based on protein content (ε=1.39) (see Figure 4). In Figure 4A, didecamer (n=20) and multi-decamer (n>20) of KLH were observed. Figure 4B showed the peak area of didecamer was 74.3% and multi-decamer was 25.7%. In Figure 4C, monomer to hexamer (n=1-6) and multimer (n>7-20) of Globo H-KLH glycoconjugate (OBI-822) were observed. Figure 4D showed the peak area of monomer to hexamer (n=1-6) was the major component (>97%) and multimer (n>7-20) was only a small amount (2.2%). The summary of mass distribution analysis of KLH and OBI-822 oligomer was shown as in Table 4.

Table 4: The summary of mass distribution analysis of KLH and OBI-822 oligomer

| **Mass Distribution** | **Range 1 (%)** | **Range 2 (%)** | **Range 3 (%)** | **Range 4 (%)** | **Range 5 (%)** |
|---|---|---|---|---|---|
| MW Start (kDa)*<br>MW Stop (kDa) | 0<br>850 | 850<br>1400 | 1400<br>3000 | 3000<br>8000 | 8000<br>20000 |
| KLH Oligomer ** | | | | Didecamer n=20 | Multi-decamer n>20 |
| KLH amount (%) | 0 | 0 | 0 | 74.3% | 25.7% |
| OBI-822 Oligomer*** | Monomer to Dimer<br>n=1-2 | Trimer<br>n=3 | Tetramer to Hexamer<br>n=4-6 | Multimer<br>n>7-20 | Oligomer<br>n>20 |
| OBI-822 Lot 14001 amount (%) | 39.7% | 45.9% | 12.3% | 2.2% | 0 |
| *MW Start/Stop: Molecular Weight (MW) range in kDa<br>**For KLH, monomer is estimated about 350-400 kDa<br>***For OBI-822, monomer is estimated about 400-500 kDa | | | | | |

The results showed that the glycoconjugate of the invention exhibited a reduced mass in molecular weight as compared with native aggregated KLH didecamers. The molecular ratio was calculated as in Table 5.

Table 5: Calculation of molecular ratio of Globo H to KLH

| **Experimental Molecular Weight from Globo H-KLH (kDa)** | **Mass of 75 Globo H per KLH subunit in Globo H-KLH: 75 kDa** | **Mass of KLH subunit: 400 kDa** | **Assumption of (Globo H) - (KLH multimer) from the experimental data** |
|---|---|---|---|
| 8000 | 75×20=1500 | 400×20=8000 | 1500+8000=9500<br>KLH forms a **Didecamer** after Globo H-KLH conjugation. |

(continued)

| Experimental Molecular Weight from Globo H-KLH (kDa) | Mass of 75 Globo H per KLH subunit in Globo H-KLH: 75 kDa | Mass of KLH subunit: 400 kDa | Assumption of (Globo H) - (KLH multimer) from the experimental data |
|---|---|---|---|
| 3000 | 75×6=450 | 400×6=2400 | 450+2400=2850<br>KLH forms a **Hexamer** after Globo H-KLH conjugation. |
| 1400 | 75×3=225 | 400×3= 1200 | 225+1077=1425<br>KLH forms a **Trimer** after Globo H-KLH conjugation. |
| 850 | 75×2=150 | 400×2=800 | 150+800=950<br>KLH forms a **Dimer** after Globo H-KLH conjugation. |
| * The above molecular ratio is calculated based on the formula as below:<br>$$\text{molecular ratio} = \frac{\text{Globo H Weight}/\text{Globo H M.W.}}{\dfrac{KLH\ Weight}{KLH\ Molecular\ Weight\ **}}$$<br>** The molecular weight of KLH depends on the forms of dimer, trimer, tetramer, pentamer, hexamer to didecamer | | | |

Given the above, in certain glycoconjugate embodiments of the invention, the KLH monomeric units form monomers, dimers, trimers, tetramers, pentamers and/or hexamers after conjugation to Globo H.

Example 4: Preparation of Vaccine Compositions and Immunization in Rats

Different samples of the glycoconjugates (Globo H-KLH) as prepared in Example 1 were stored at 4 °C, and mixed with a saponin adjuvant under a laminar flow hood. The resultant vaccine compositions were placed on ice and transported to animal facility for subsequent immunization.

Three groups of Lewis rats were immunized with the various vaccine compositions as shown in Table 6.

Table 6: Groups of immunized rats

| Group | Vaccine compositions | Animal number | Route |
|---|---|---|---|
| I | Globo H-KLH* (25μg) + saponin adjuvant (25μg) | 8 | subcutaneous injection |
| II | Globo H-KLH* (7.5μg) + saponin adjuvant (25μg) | 4 | |
| III | PBS (Phosphate Buffered Saline) | 4 | |
| *Globo H : KLH=0.17 : 1 (w/w) | | | |

The rats were immunized on day 0, 7, 14, and 21. Peripheral blood mononuclear cells (PBMC) and plasma were collected on day 0, 3, 10, 17, 24, and 31. Spleen, lymph node, and peritoneal wash were harvested on day 31.

Example 5: Assays for Induction of Humoral and Cellular Immune Responses in Rats

Example 5.1: Analysis of Immune Effector Cell Subpopulations by Flow Cytometry

The peripheral blood mononuclear cells (PBMCs) were isolated from the animals and then various immune effector cell subpopulations in the PBMC were identified by flow cytometry using specific antibodies against different cell markers The PBMCs, isolated from the immunized rats on day 0, 3, 10, 17, 24, and 31, were stained with different fluorescence (FITC)-conjugated antibodies and placed on ice for 30 minutes. After incubation, cells were washed with the washing buffer (1% bovine serum albumin (Sigma) and 0.1% $NaN_3$ (Sigma) in phosphate buffered saline (UniRegion Biotech) and centrifuged at 350 g for 5 minutes. Cells were resuspended in washing buffer for determination of fluorescence by FACS Canto (BD Bioscience). The data were analyzed with BD FACSDiva (BD Bioscience) software. The results show that in the immunized rats by the glycoconjugate of the present invention, B cells and T cells were significantly expanded when compared to the PBS control group. Specifically, the B cell population first appeared at day 3 and subsequent CD3T, CD4T and CD8T cells appeared at day 24. See Figure 5A to Figure 5D.

Example 5.2: Globo H-specific antibody test by ELISA

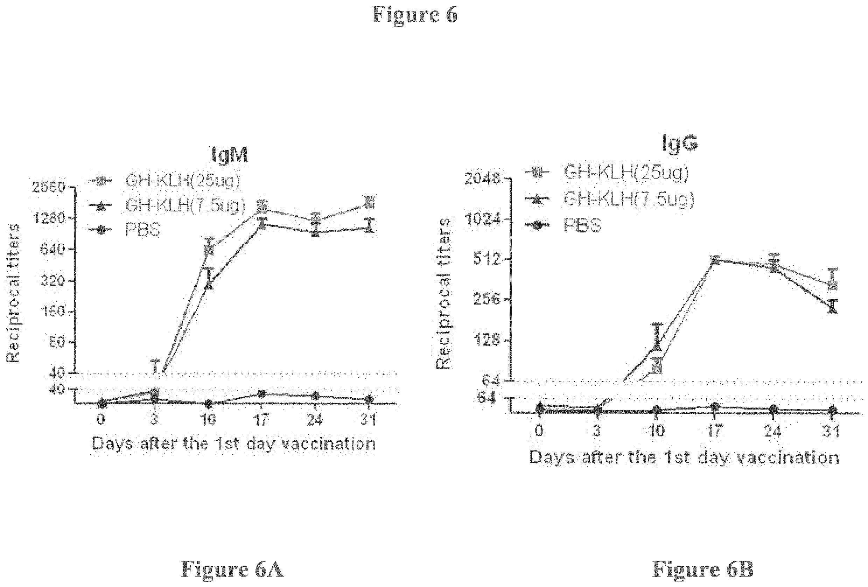

The production of Globo H-specific antibodies in the plasma from the immunized rats was determined by ELISA assay. The results showed the titers of Globo H-specific IgG began to rise at 10 days and peaked at 17 days after immunization. Similar patterns were observed in the production of Globo H-specific IgM antibody. See Figure 6A to Figure 6B. There was no response of Globo H-specific IgG and IgM antibody in rats treated with PBS only.

In summary, in the immunized rats, B cell production appears at day 3, followed by production of IgM and IgG antibody against Globo H which appears at day 10 and subsequent CD3T, CD4T, and CD8T cell which appears at day 24. The glycoconjugate (Globo H-KLH) of the invention was effective to induce both humoral and cellular responses.

Example 6: Immunization in Mice and Antibody Test by ELISA

Different samples of the glycoconjugates (Globo H-KLH) were stored at 4 °C, and mixed with a saponin adjuvant under a laminar flow hood. The resultant vaccine compositions were placed on ice and transported to animal facility for subsequent immunization.

Balb/c mice of approximately eight weeks old were given Globo H-KLH glycoconjugate with different carbohydrate-to-protein (Globo H : KLH) ratios once every week for four weeks (Day 0, 7, 14, and 21) via subcutaneous injection. Blood samples were collected through retro-orbital or facial vein without anticoagulant pre-immune or Day 0, and three days after each vaccination (Day 10, 17 and 24). Samples were then centrifuged to separate sera and red cells. Sera were collected and stored at -20 °C, which were later analyzed by ELISA. Mann-Whitney t-test was used for statistical analysis.

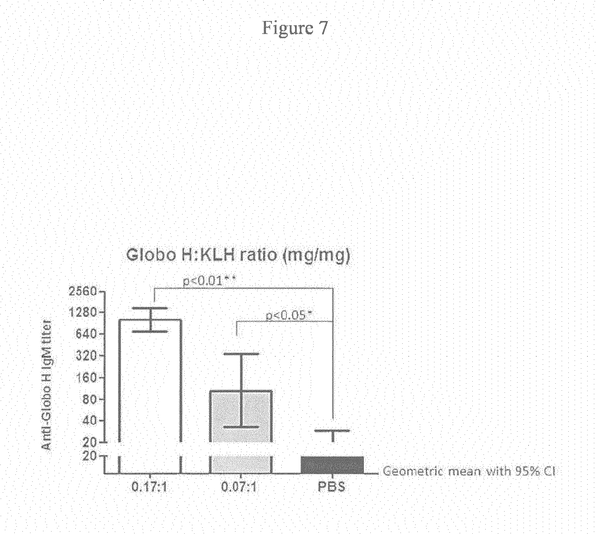

As shown in Figure 7, the glycoconjugate (Globo H-KLH), in combination with a saponin adjuvant, according to the invention, has been demonstrated to significantly induce the Globo H-specific IgM antibody responses in the animal model, as compared with the PBS control group. Specifically, the glycoconjugate with a weight ratio of 0.17: 1 (Globo H: KLH) induced a better antibody titer than the glycoconjugate with a weight ratio of 0.07: 1 (Globo H: KLH).

In summary, the Globo H-KLH glycoconjugate (OBI-822), in combination with a proper adjuvant, according to the invention, has been demonstrated to induce unexpectedly superior humoral and cellular immune response in the animal model, particularly expansion of B cells and T cells including CTLs and IgM and IgG responses, which are important in cancer immunotherapy.

Example 7: Immunogenicity Study of Globo H - KLH with Different Adjuvants in Mice

The ability of Globo H composition of the invention, when paired with different adjuvants (OBI-821/OBI-834), to elicit an immune response in mice has been performed. Viable Lewis Lung carcinoma (LL/2, ATCC CRL-1642) cells (provided from Eurofins Panlabs Taiwan Ltd., $5.0 \times 10^6$ in 0.1 mL), syngeneic for C57BL/6 mice, were injected subcutaneously into the abdominal region toward the lateral side of experimental mice on day 16. Six groups of 6-8 weeks old female C57BL/6 mice were immunized subcutaneously with Globo H- KLH (OBI-822) and different adjuvants. OBI-822 dose levels were the equivalent of amount in each group. Each injection contained a doses equivalent to 2.0 μg vaccine (OBI-822) and 20 μg adjuvants (OBI-821/OBI-834) administered subcutaneously in 0.2 mL/mouse (at both left and right abdominal sties, 0.1 mL/site). The immunizations occurred on Days 0, 5, 11, 19, 29, 34 and 39, and serum was collected on Day 0 (pre-injection) and Day 42 for comparative analysis by ELISA. Serological responses were measured by ELISA to determine the titer of IgM and IgG antibodies against Globo H.

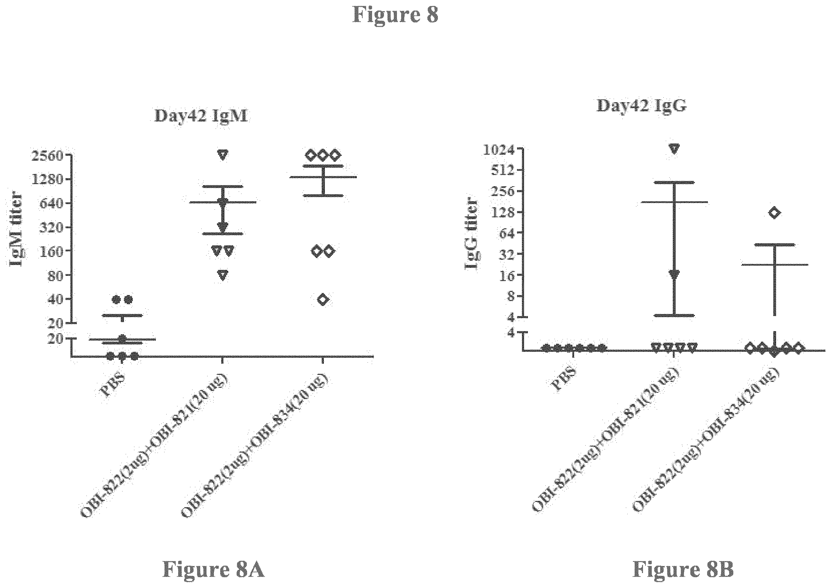

As shown in Figure 8, there was no response in mice treated with Phosphate Buffered Saline (PBS). In contrast, the mice treated with OBI-822 + OBI821 and OBI-822 + OBI-834 responded with significant IgM anti-Globo H titers (Figure 8A). However, the average IgG titers were lower than IgG (Figure 8B). It indicated that both OBI-821 and OBI-834 adjuvants could induce the immunogenicity of Globo H - KLH (OBI-822). Vaccination with Globo H- KLH and an adjuvant has demonstrated to elicit both IgG and IgM anti-Globo H responses in female C57BL/6 mice.

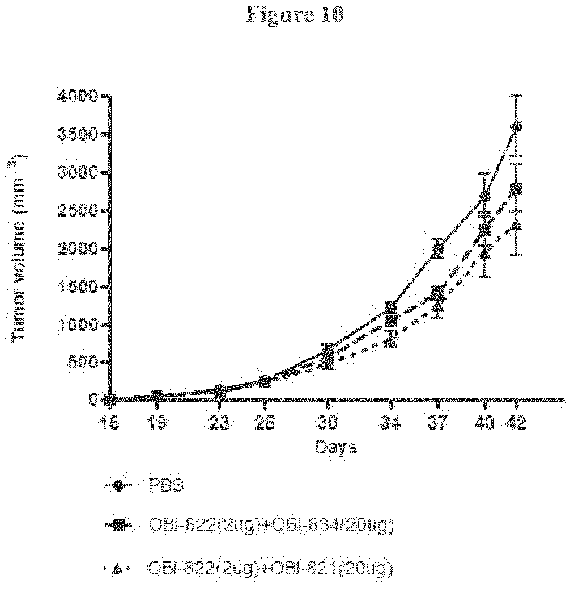

As shown in Figure 9, the six groups of C57BL/6 mice were monitored for body weight and tumor size on days 16, 19, 23, 26, 30, 34, 37, 40 and 42. The pictures including tumor with whole body were taken after sacrifice on day 42. As shown in Figure 10, tumor volume ($mm^3$) is estimated according to the formula for a prolate ellipsoid: length (mm) $\times$ [width (mm)]$^2$ x 0.5. Tumor growth in compound treated animals is calculated as T/C (Treatment/Control) x 100%; one-way ANOVA followed by Dunnetts test or unpaired Student's t-test was used to determine the significant inhibition in tumor growth demonstrating antitumor activity. The tumor inhibition rate on day 42 of OBI-822 + OBI-821 was about

35% and OBI-822 + OBI-834 was about 22%. It seemed that both OBI-821 and OBI-834 adjuvants could inhibit tumor growth with Globo H- KLH (OBI-822).

Example 8: LC-MS/MS Analysis of Globo-H Conjugation Sites (lysine) on KLH

Globo H conjugation sites in four KLH samples using multiple enzyme digestion and LC-MS/MS were identified. The four Globo H-conjugated KLH samples were first digested with four different enzymes and then analyzed by LC-MS/MS and Mascot database search. Two types of derivatives were identified: Globo H derivative (Globo H + MMCCH) and the MMCCH derivative (MMCCH alone). The Globo H derivative and its neutral loss forms were taken into account for Globo H conjugation site identification. The MMCCH form and its deamidated form were taken into account for MMCCH conjugation site identification. Only those peptides with high quality MS/MS spectra and Mascot score were taken into account. For Globo H conjugation analysis, 31 and 28 conjugated lysines were observed from the two replicate LC-MS/MS analyses of OBI-822-13001-DP (sample 1); 19 and 21 conjugated lysines were observed from the two replicate LC-MS/MS analyses of OBI-822-13002-DP (sample 2); 10 and 11 conjugated lysines were observed from the two replicate LC-MS/MS analyses of OBI-822-13003-DP (sample 3); 18 and 19 conjugated lysines were observed from the two replicate LC-MS/MS analyses of OBI-822-13004-DS (sample 4). For MMCCH conjugation analysis, 155 and 141 conjugated lysines were found from the two replicate LC-MS/MS analyses of OBI-822-13001-DP (sample 1); 143 and 137 conjugated lysines were found from the two replicate LC-MS/MS analyses of OBI-822-13002-DP (sample 2); 147 and 143 conjugated lysines were found from the two replicate LC-MS/MS analyses of OBI-822-13003-DP (sample 3); 140 and 136 conjugated lysines were found from the two replicate LC-MS/MS analyses of OBI-822-13004-DS (sample 4).

Example 8 Materials and Methods:

The abbreviations were listed as follows:

K=Lysine; LC-MS/MS=Liquid Chromatography-Tandem Mass Spectrometry;

DTT=Dithiolthreitol; IAM =Iodoacetamide; ACN=Acetonitrile; FA=Formic Acid; Glu-C=Endoproteinase Glu-C; ABC=Ammonium bicarbonate; RT=Room temperature; MW=Molecular weight.

The four KLH samples, sample 1-4, were first processed for buffer exchange into 50 mM ammonium bicarbonate buffer solution by 100 kDa cut-off Amicon Ultra Centrifugal Filters and denatured with 6 M urea. The samples were then reduced with 10 mM DTT at 37 °C for 1 hr, alkylated using 50 mM IAM for 30 mins in dark at RT and quenched with 50 mM DTT at RT for 5 mins. The resulting proteins were diluted until the urea concentration is 1 M and subjected to in-solution digestion with different enzymes as described in the following section.

In-solution digestion with different enzymes was performed with the following digestion conditions: (1) trypsin digestion at 37 °C for 24 hrs (protein: enzyme=40:1) (2) Glu-C digestion at 37 °C for 24 hrs (protein: enzyme=25:1); (3) chymotrypsin digestion at RT for 24 hrs (protein: enzyme=25:1); (4) thermolysin digestion at 37 °C for 24 hrs (protein: enzyme=25:1).

Digestion reactions were terminated by adding formic acid and all four digested samples were subjected to LC-MS/MS analysis. Samples were analyzed with Q Exactive mass spectrometer (Thermo Scientific) coupled with Ultimate 3000 RSLC system (Dionex). The LC separation was performed using the C 18 column (Acclaim PepMap RSLC, 75 μm x 150 mm, 2 μm, 100 Å) with the gradient shown in Table 7.

Table 7: The LC separation gradient

| Time (min) | Mobile phase A* % | Mobile phase B** % | Flow (μL/min) |
|---|---|---|---|
| 0 | 99 | 1 | 0.25 |
| 5 | 99 | 1 | 0.25 |
| 35 | 90 | 10 | 0.25 |
| 67 | 65 | 35 | 0.25 |
| 77 | 15 | 85 | 0.25 |
| 82 | 99 | 1 | 0.25 |

(continued)

| Time (min) | Mobile phase A* % | Mobile phase B** % | Flow (μL/min) |
|---|---|---|---|
| 90 | 99 | 1 | 0.25 |
| *Mobile phase A: 5% Acetonitrile / 0.1% Formic acid<br>**Mobile phase B: 95% Acetonitrile / 0.1% Formic acid | | | |

In-source CID was set at 45 eV. Full MS scan was performed with the range of m/z 350-2000, and the ten most intense ions from MS scan were subjected to fragmentation for MS/MS spectra. Raw data were processed into peak lists by Proteome Discoverer 1.4 for Mascot database search.

Database search was performed with Mascot version 2.4.1 and Thermo Proteome Discoverer version 1.4 against the KLH1 sequence (SEQ ID NO. 1) and KLH2 sequence (SEQ ID NO. 2). The parameters used were as follows:

Enzyme: Trypsin, Glu-C, Chymotrypsin and Thermolysin according to the digestion method; Fixed modification: Carbamidomethyl (C);

Variable modifications for MMCCH derivatives (MMCCH alone): Deamidated (NQ), Oxidation (M), dK_MMCCH-1 (K), dK_MMCCH-2 (K);

Variable modifications for Globo H derivatives (Globo H + MMCCH): Deamidated (NQ), Oxidation (M), Globo_H_MMCCH (K), dK_MMCCH_NL997 (K), dK_MMCCH_NL835 (K), dK_MMCCH_NL673 (K), dK_MMCCH_NL511 (K), dK_MMCCH_NL308 (K);

Peptide Mass Tolerance: ± 10 ppm; Fragment Mass Tolerance: ± 0.05 Da; Max Missed Cleavages: 5; Instrument type: ESI-TRAP; Ion cut-off score: 13.

"dK_MMCCH-1" in the search parameters indicates the MMCCH-conjugated lysine with the MW addition of 352.1569 Da.

"dK_MMCCH-2" in the search parameters indicates the deamidated form of MMCCH-conjugated lysine with the MW addition of 338.1300 Da.

"Globo_H_MMCCH (K)" in the search parameters indicates the Globo H-conjugated lysine with the MW addition of 1393.5317 Da.

"dK_MMCCH_NL997" in the search parameters indicates the neutral loss form of Globo H-conjugated lysine with the MW addition of 396.1831 Da.

"dK_MMCCH_NL835" in the search parameters indicates the neutral loss form of Globo H-conjugated lysine with the MW addition of 558.2360 Da.

"dK_MMCCH_NL673" in the search parameters indicates the neutral loss form of Globo H-conjugated lysine with the MW addition of 720.2888 Da.

"dK_MMCCH_NL511" in the search parameters indicates the neutral loss form of Globo H-conjugated lysine with the MW addition of 882.3416 Da.

"dK_MMCCH_NL308" in the search parameters indicates the neutral loss form of Globo H-conjugated lysine with the MW addition of 1085.4210 Da.

The "MW addition" implies the molecular weight addition compared to intact lysine residue.

Example 8 Results:

LC-MS/MS based techniques have emerged as an important tool for identification of protein and characterization of amino acid modification. Detailed information regarding peptide sequences and modification sites can be obtained by the assignment of fragment ions provided by MS/MS spectra. Mascot is a search engine and its probability based scoring algorithm has been well accepted. Mascot score was adopted in this study as a reference of confidence for protein sequencing and Globo H or MMCCH conjugation site identification. To extensively analyze the distribution of Globo H or MMCCH conjugation sites in sample 1-4, these samples were digested with multiple enzymes followed by LC-MS/MS analyses.

The expected chemical structure for Globo H derivative (Globo H + MMCCH) was shown in Figure 11A and the corresponding MW addition of 1393.53 Da on lysine-containing peptides can be observed among the results. Besides, the labile polysaccharides tend to fall off via neutral loss during the electrospray ionization in LC- MS analysis. Therefore,

the molecular weight addition of 396.18 Da, 558.24 Da, 720.29 Da, 882.34 Da and 1085.42 Da resulted from neutral loss can also be observed for glycoconjugated peptides, as shown in Figure 11B. All the derivitization forms were considered for the identification of Globo H conjugation sites.

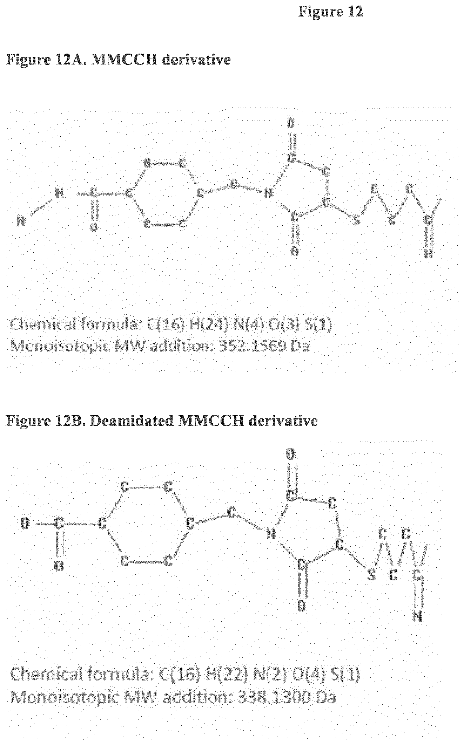

In addition, the MMCCH derivative is also observed (MMCCH alone) in these Globo H conjugated KLH samples. The expected chemical structure for MMCCH derivative and its deamidated form were shown in Figure 12A and Figure 12B and the corresponding MW addition of 352.16 Da and 338.13 Da respectively on lysine-containing peptides can be observed among the results. Both derivitization forms were considered for the identification of MMCCH conjugation sites. The conversion from Globo H derivative to MMCCH is not clear but it is supposed to happen during the sample treatment.

The mass accuracy of ± 10 ppm for precursor ion and ± 0.05 Da for fragment ion were used as the criteria for protein identification and spectra interpretation. The Globo H derivative as well as its neutral loss forms was chosen as variable modification for Globo H conjugation site identification, and MMCCH derivative as well as its deamidated form was chosen as variable modification for MMCCH conjugation site identification. Database search was performed against the sequence of KLH1 (SEQ ID NO. 1) and KLH2 (SEQ ID NO. 2). Only those peptides with high quality MS/MS spectra (ion score ≥ 13, $p < 0.05$) were listed in the report.

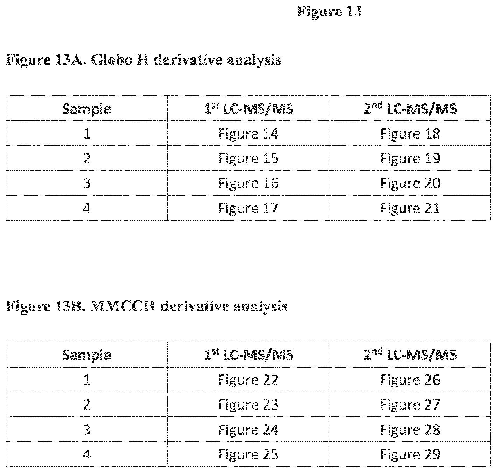

To demonstrate a repeatable result, the LC-MS/MS analysis was performed twice followed by individual Mascot database search for both Globo H conjugation site identification and MMCCH conjunction site identification, as summarized in Figure 13A and Figure 13B.

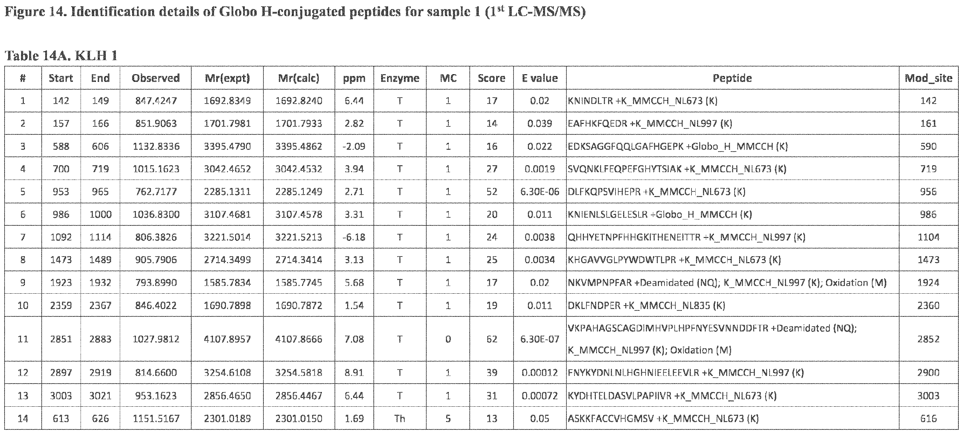

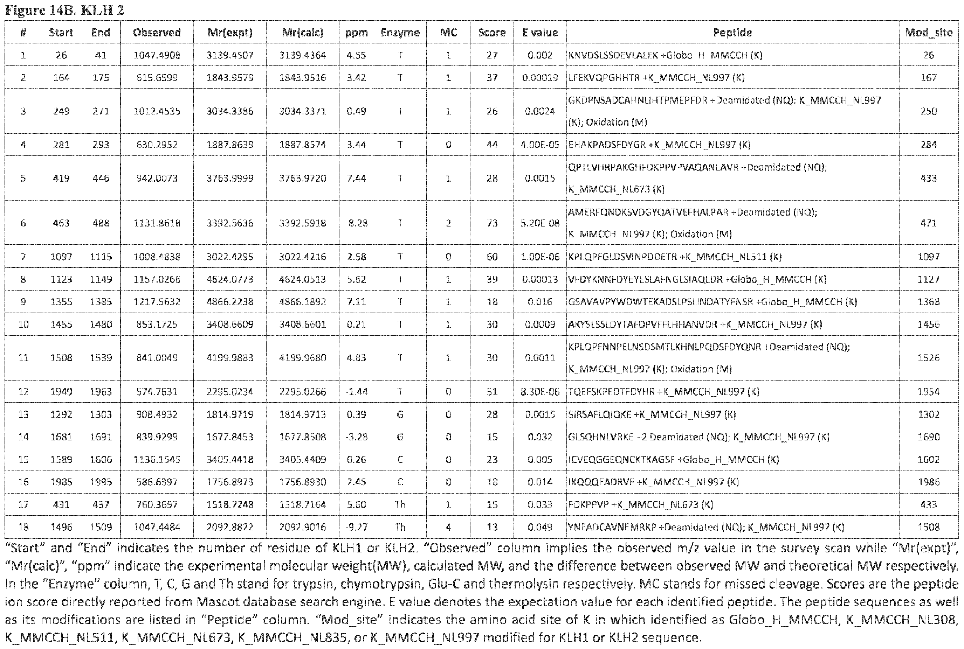

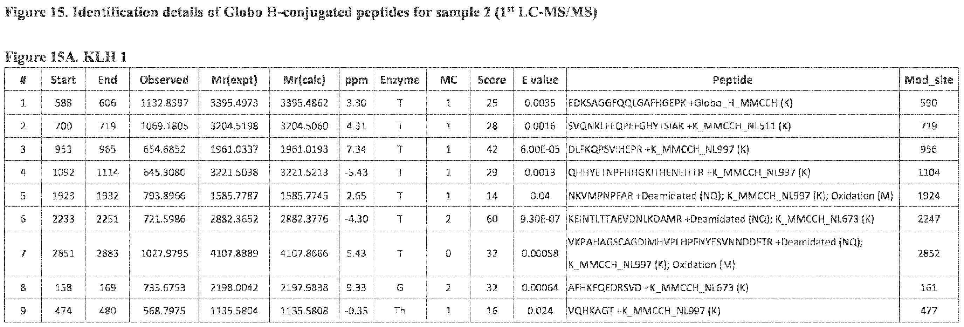

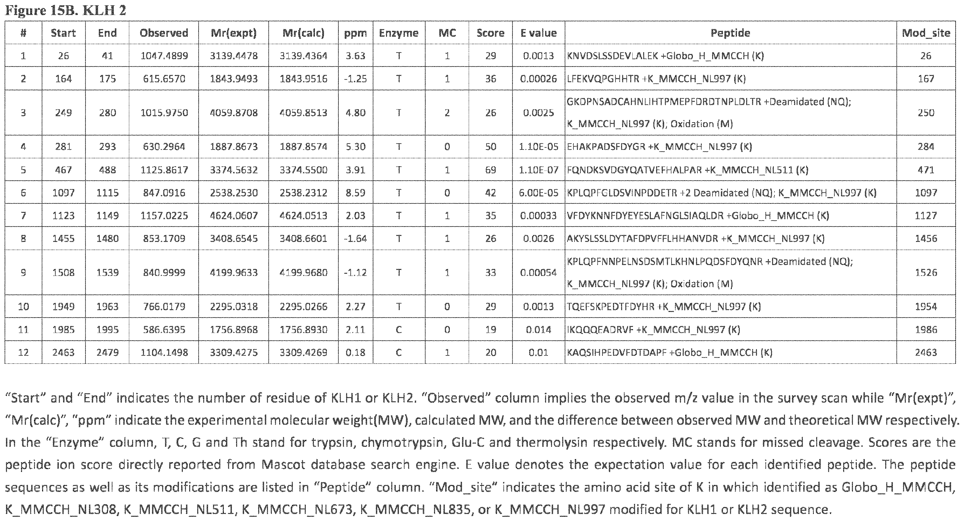

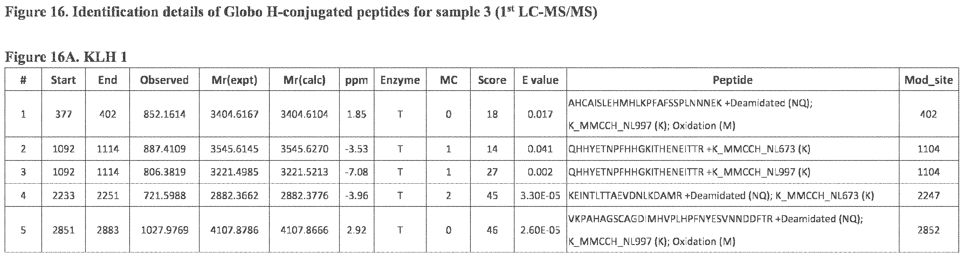

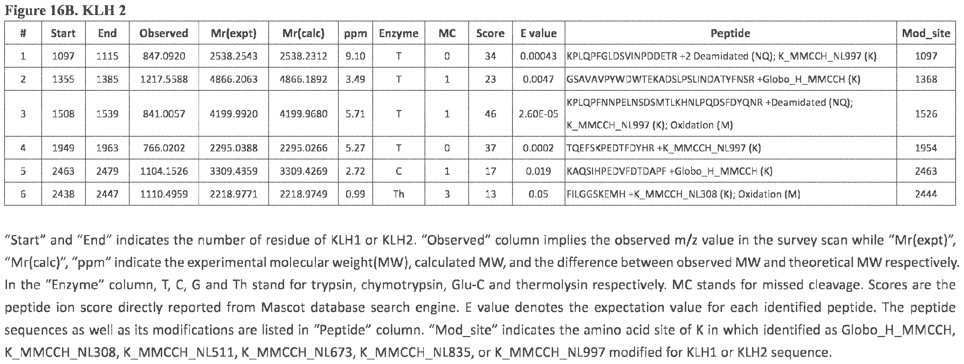

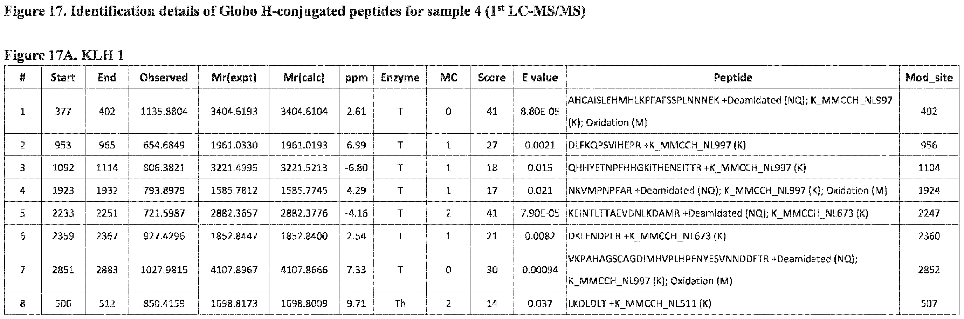

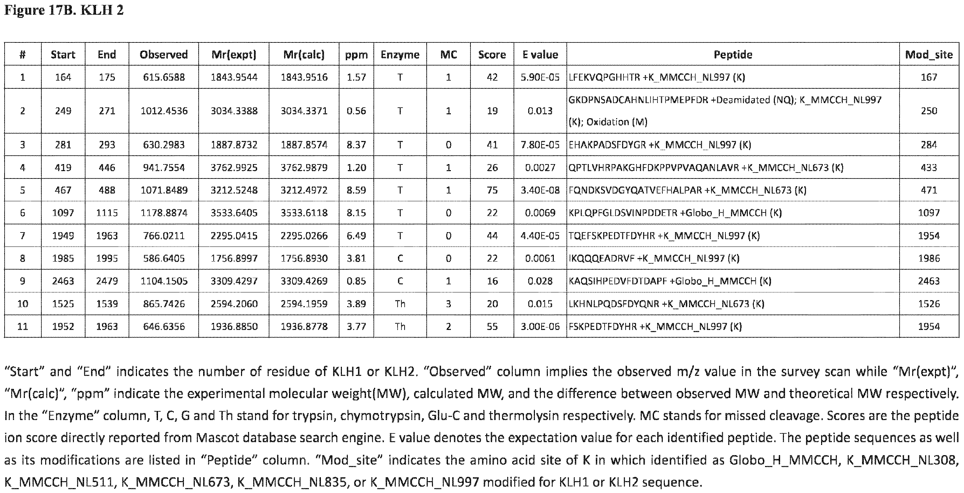

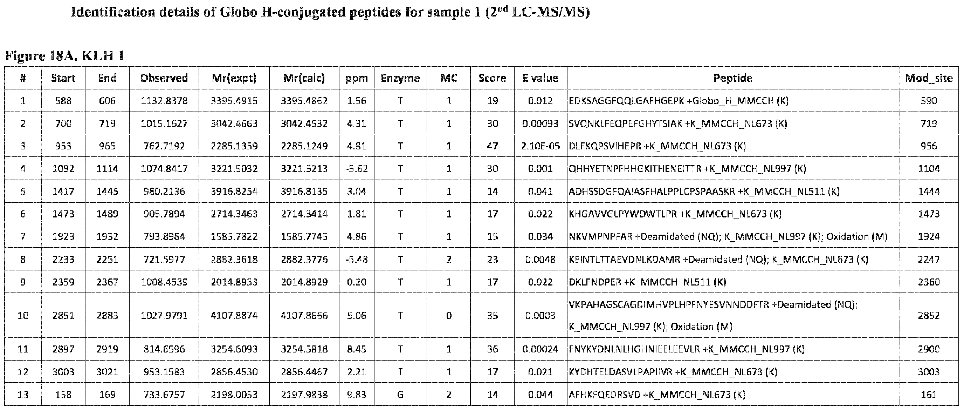

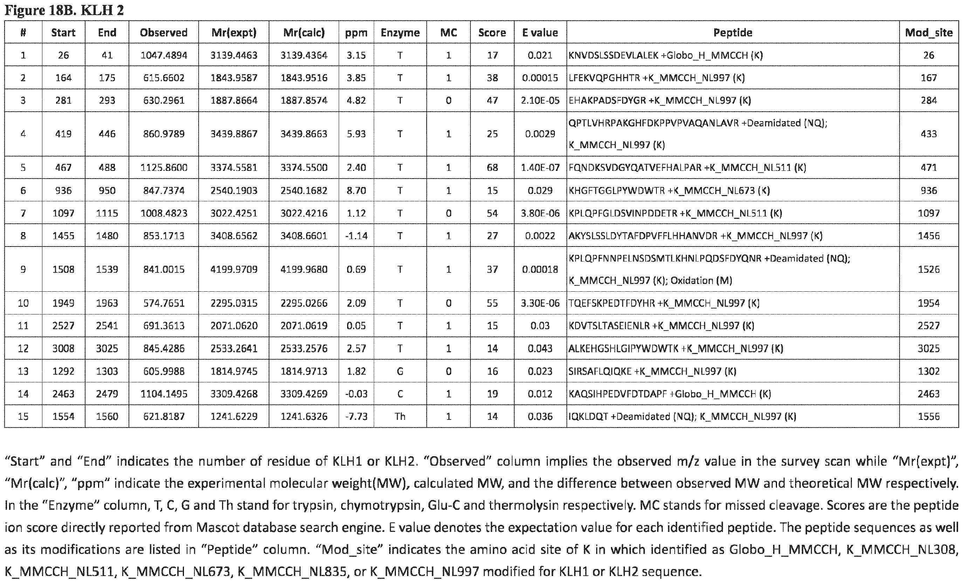

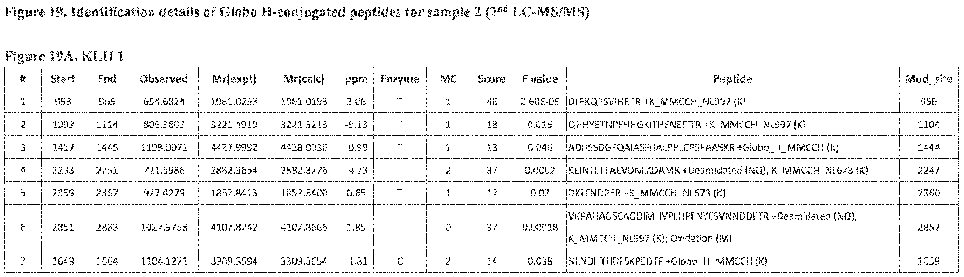

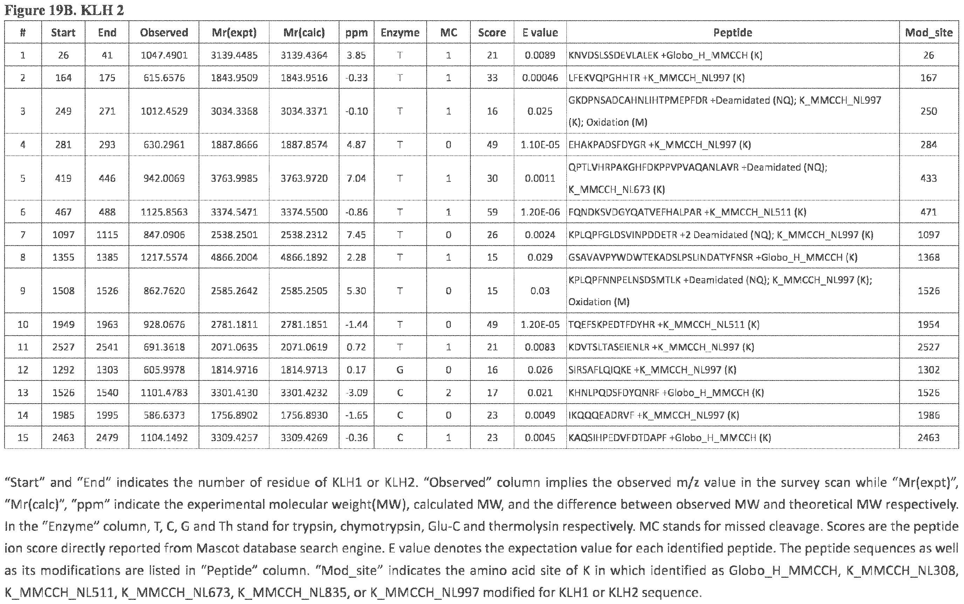

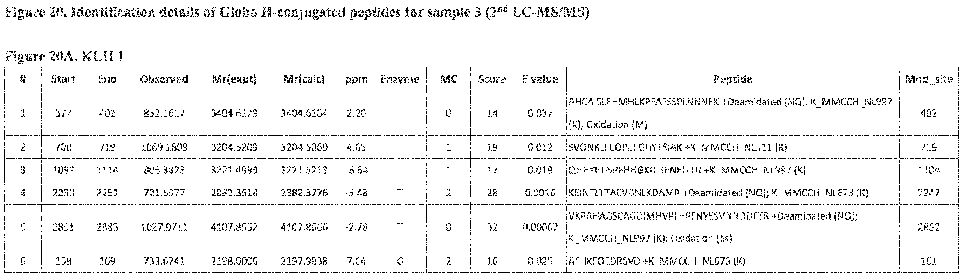

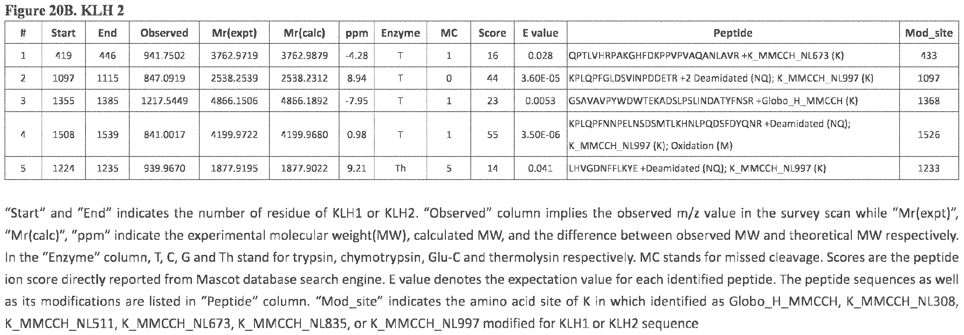

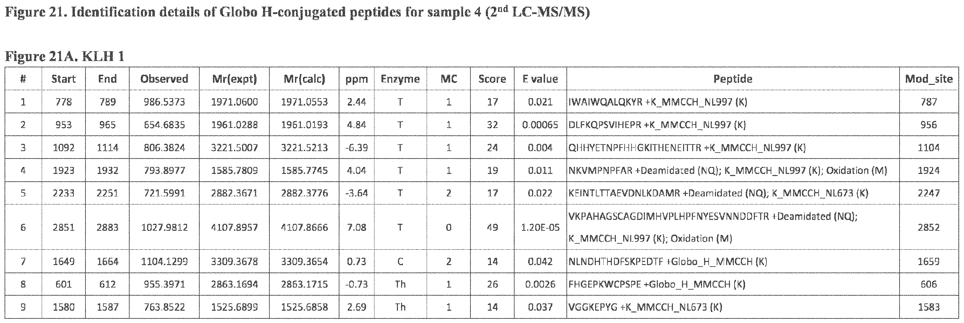

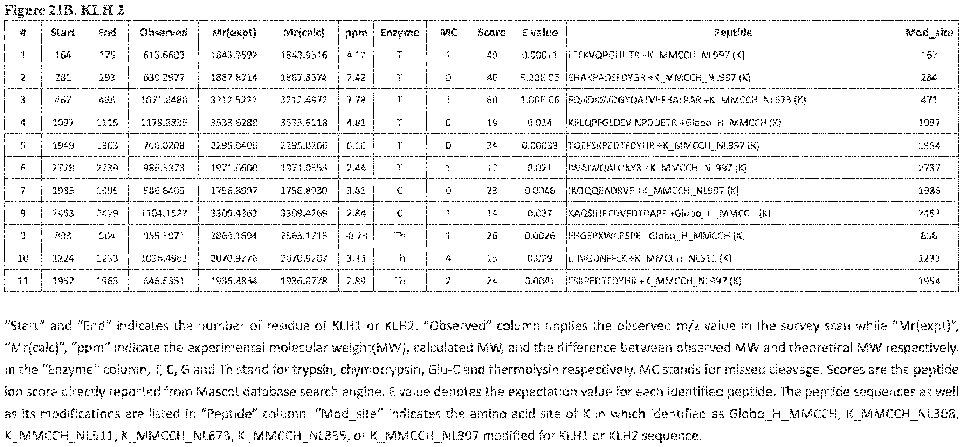

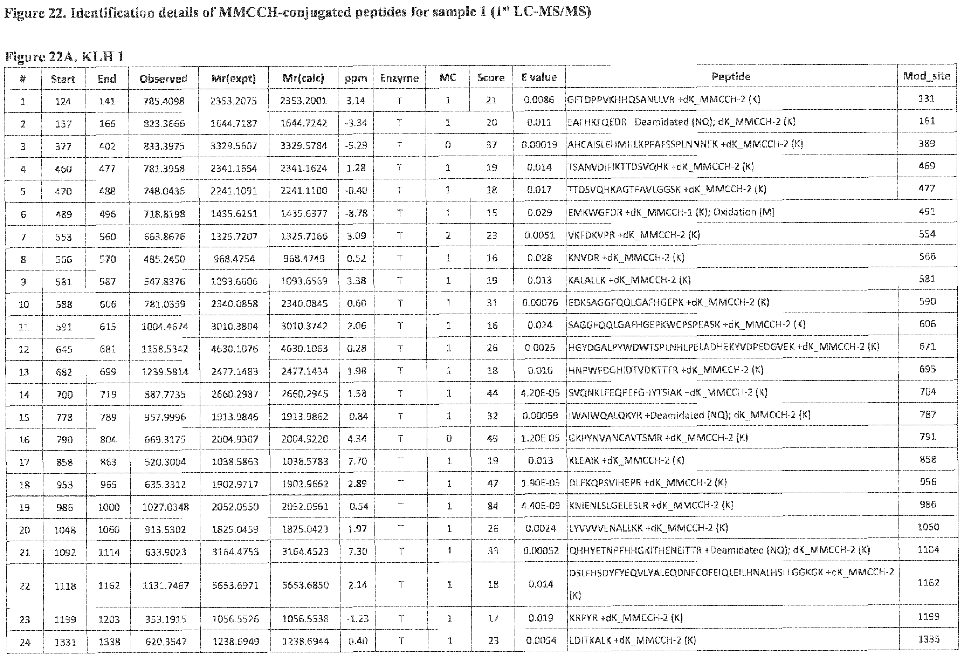

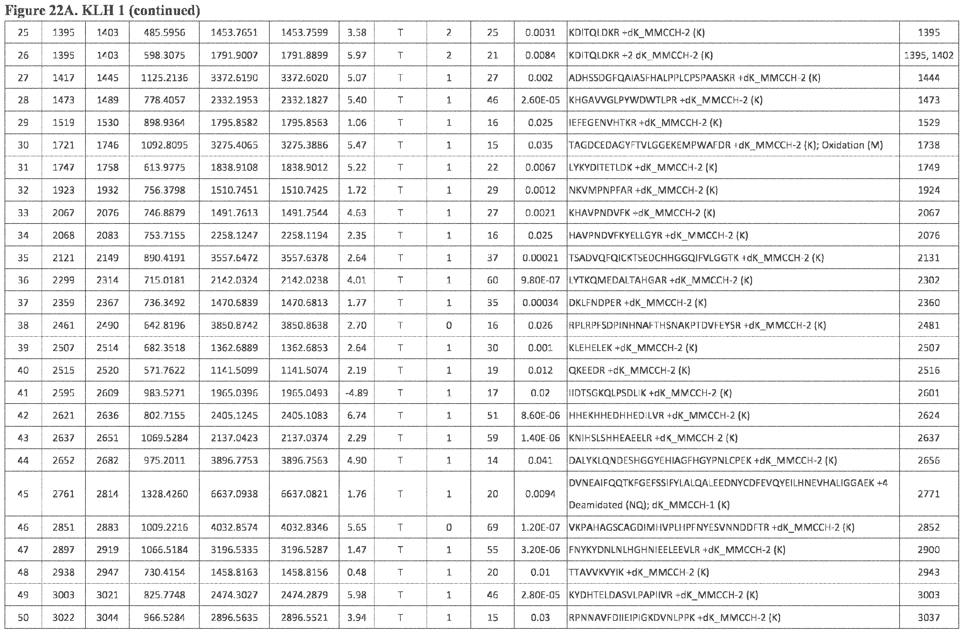

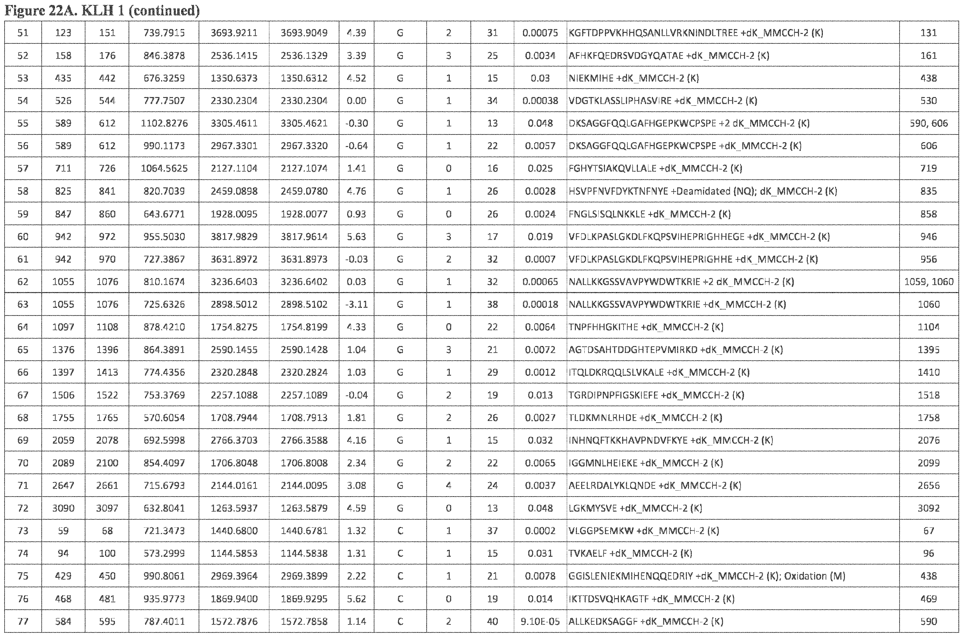

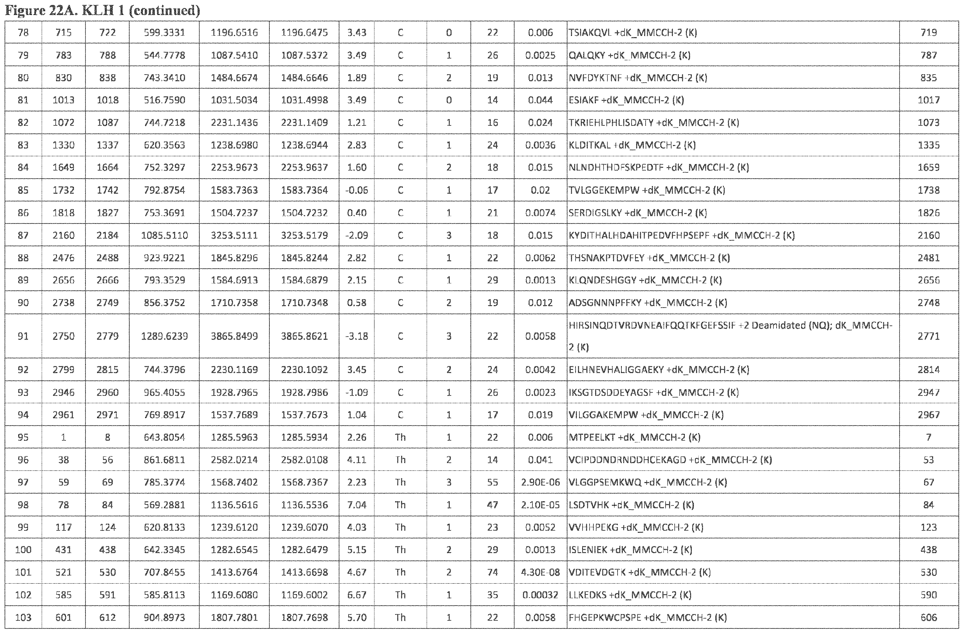

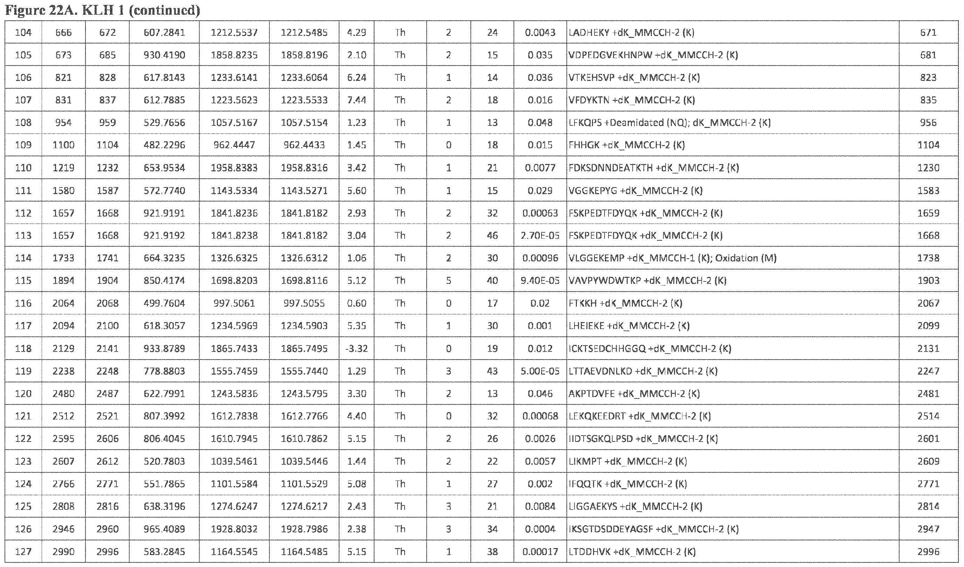

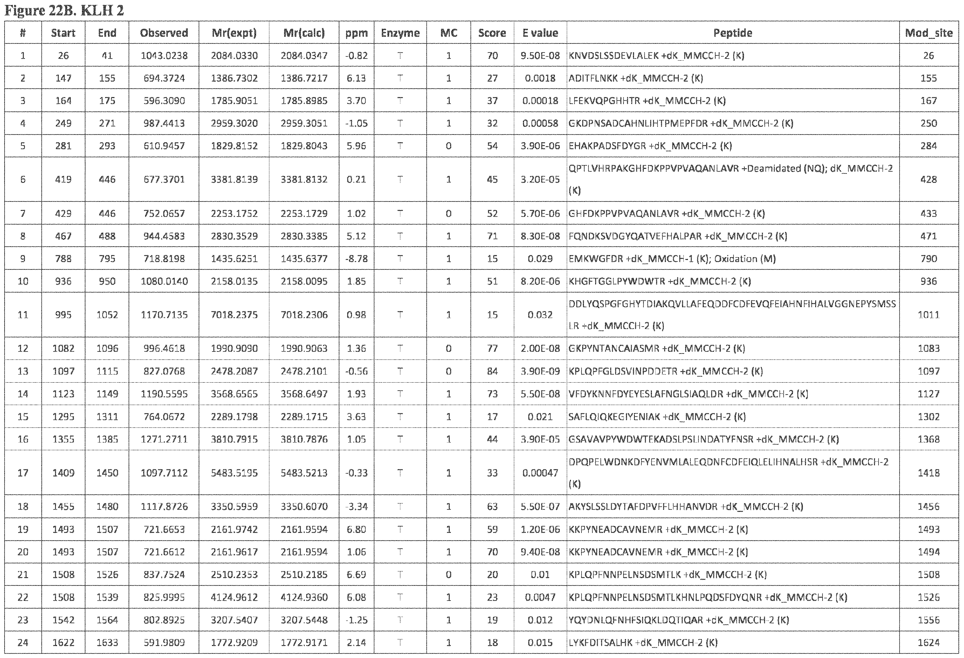

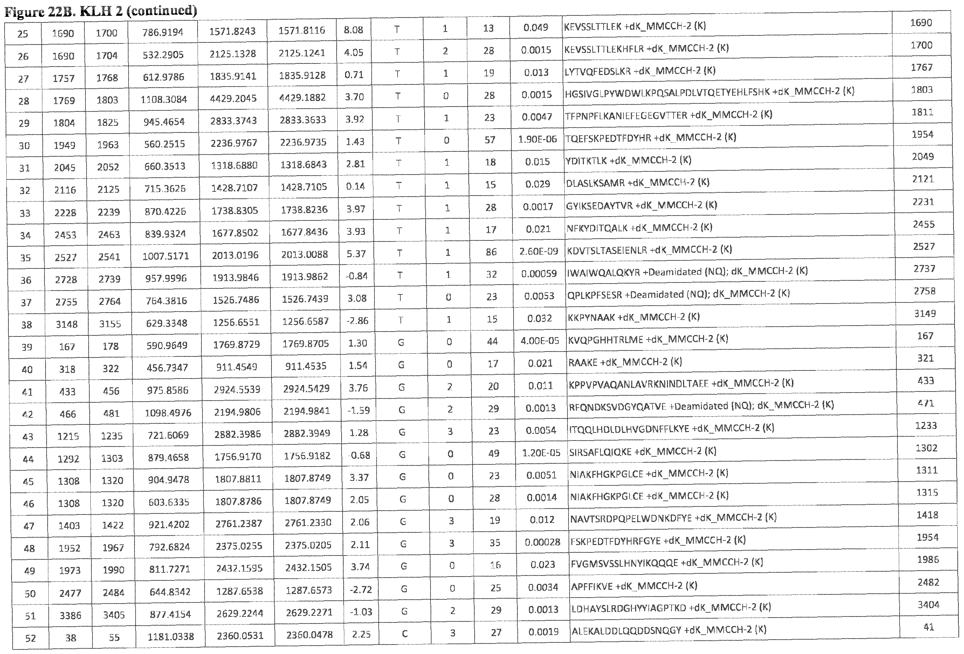

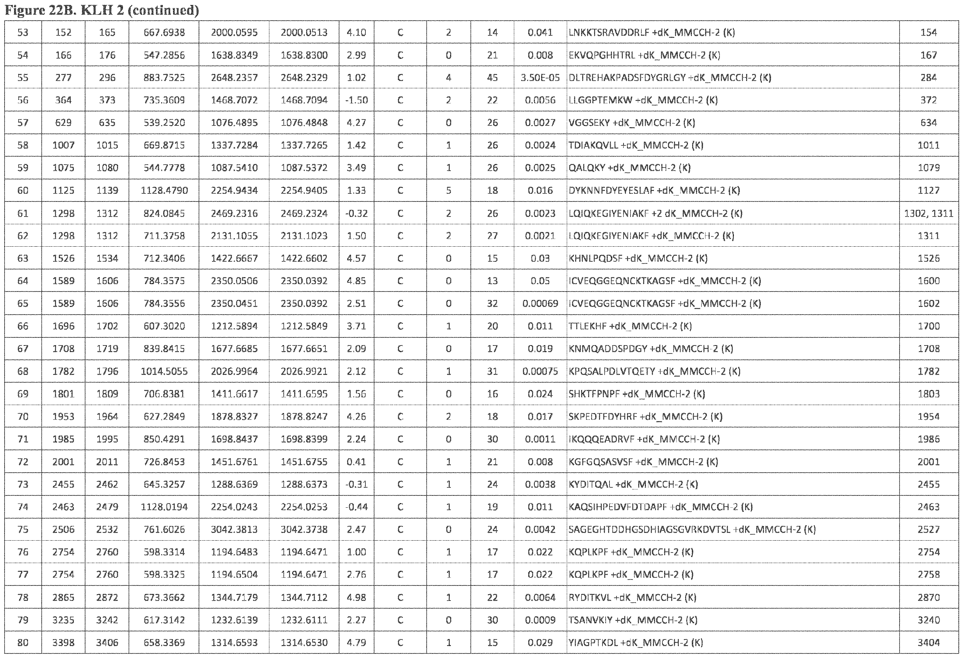

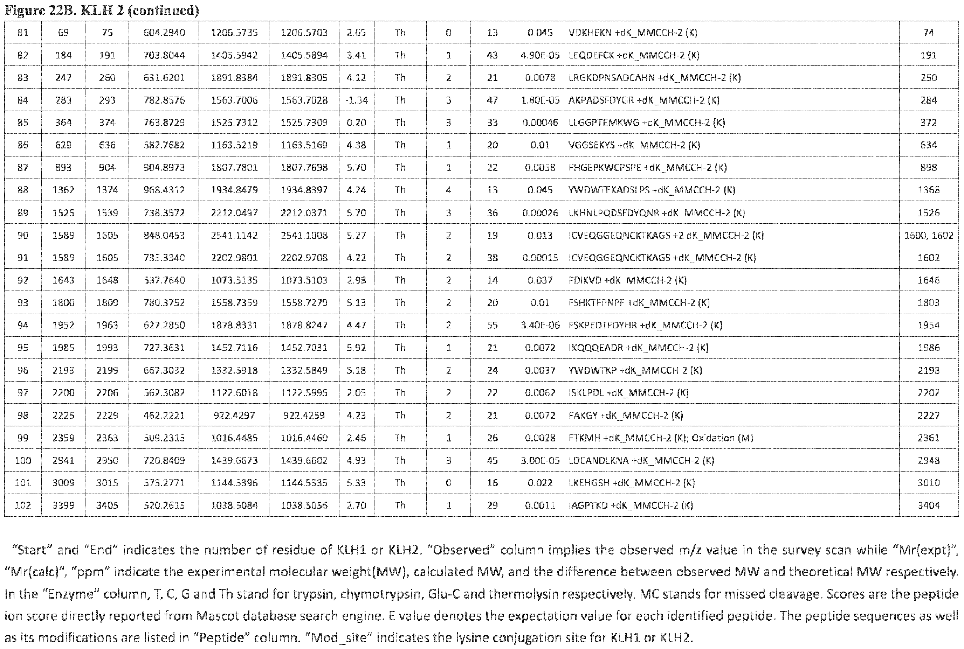

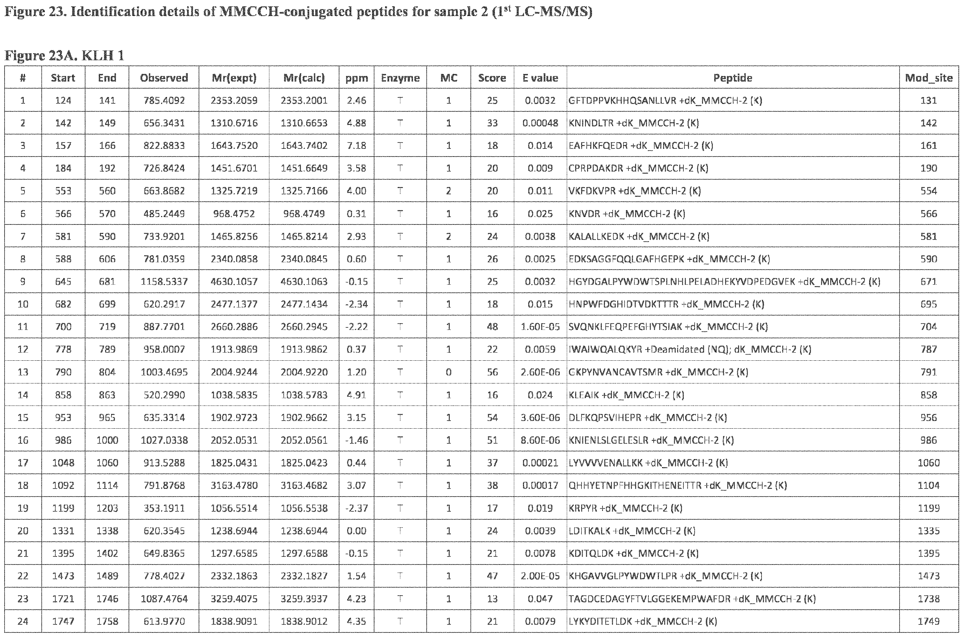

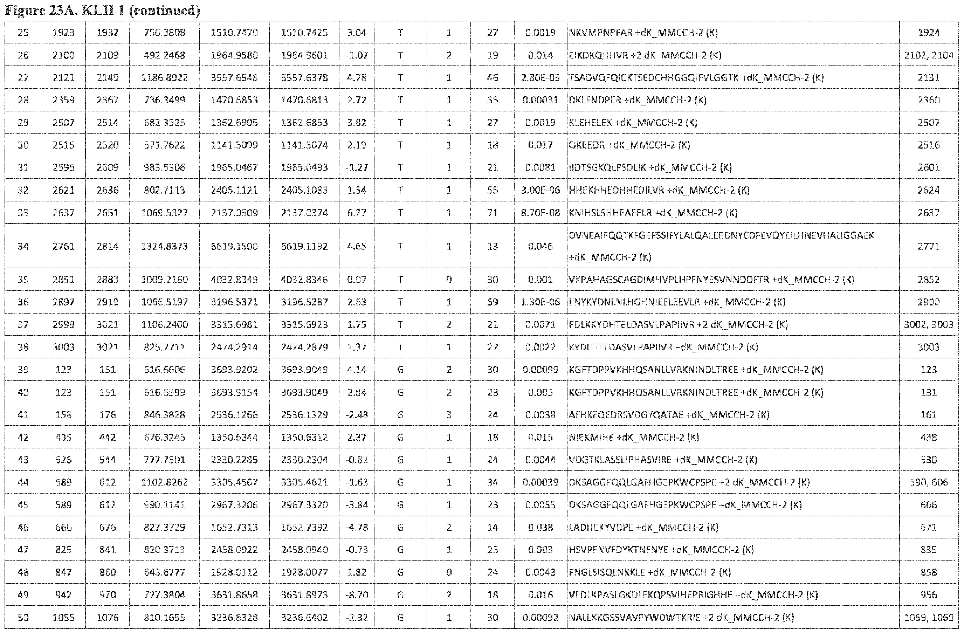

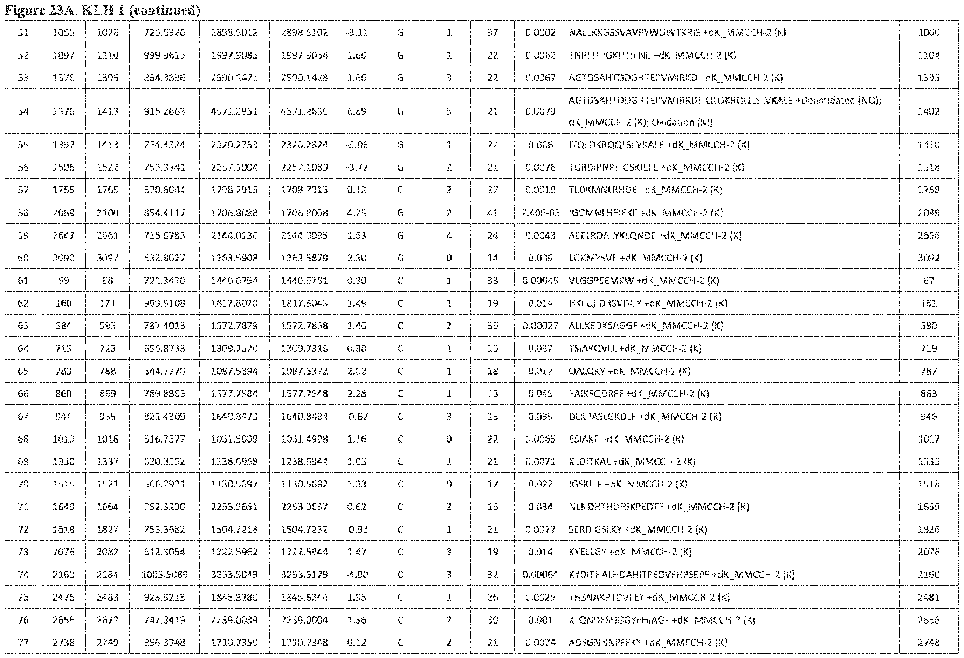

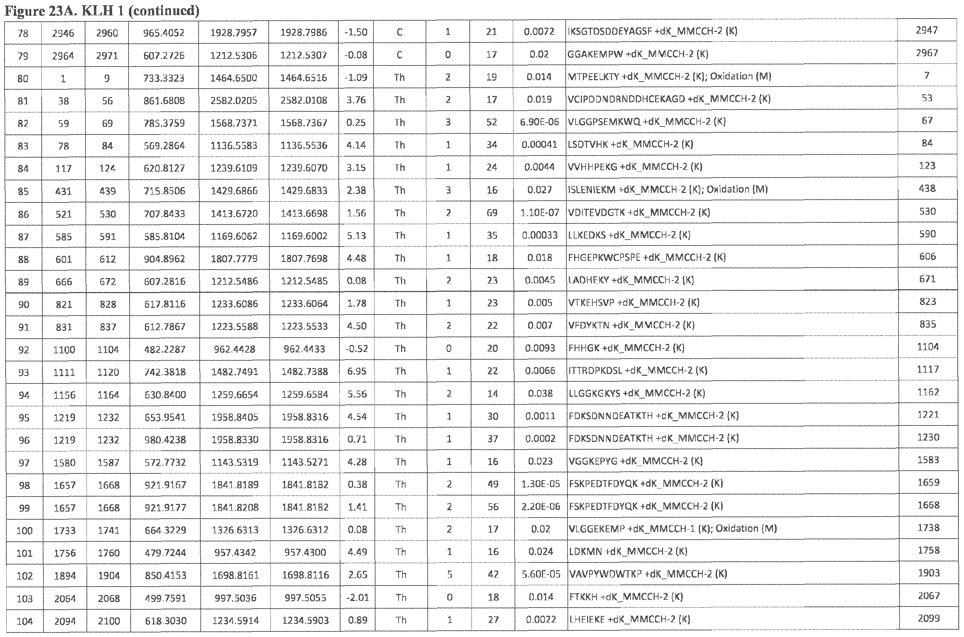

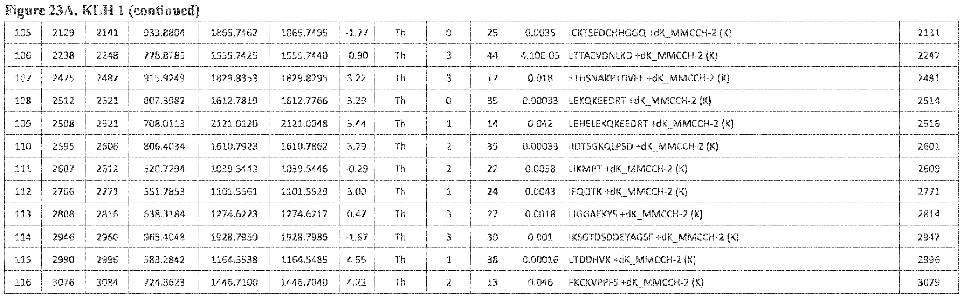

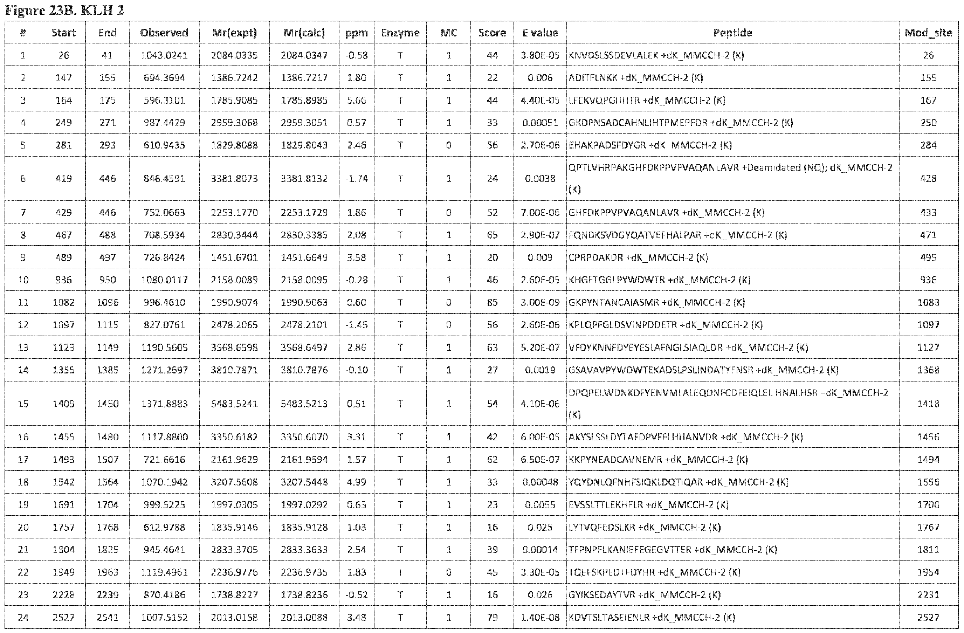

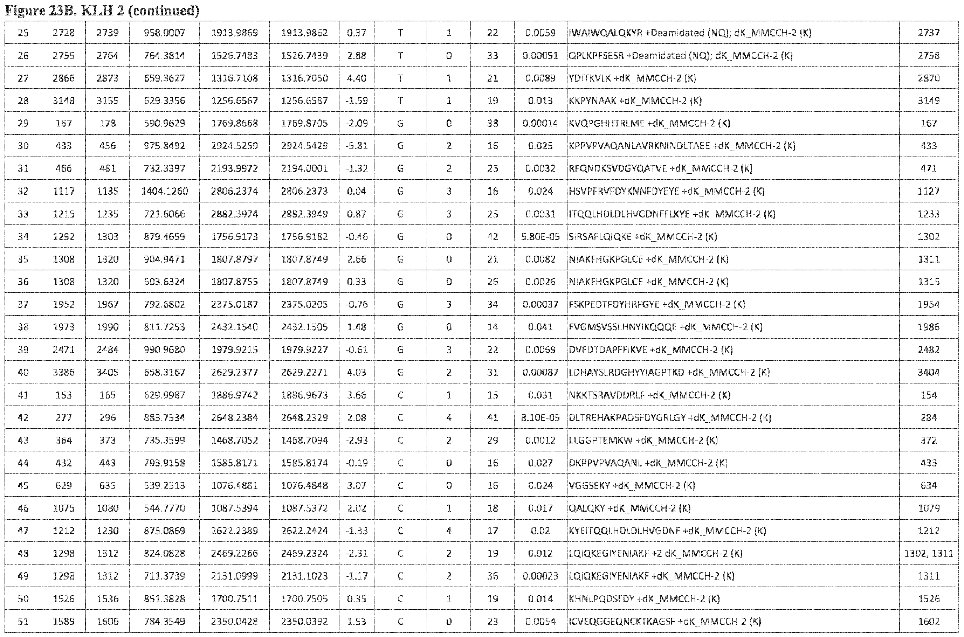

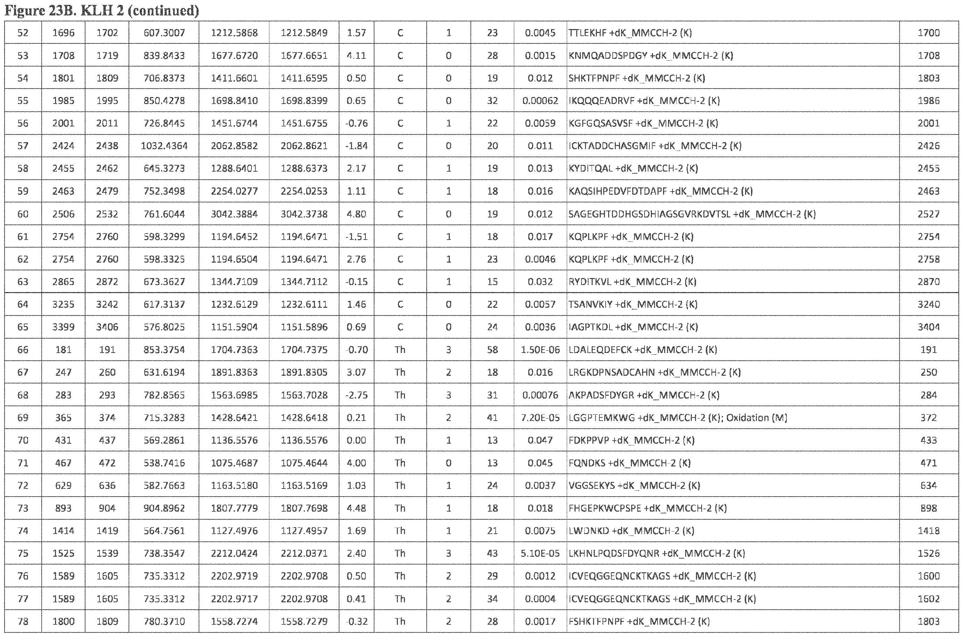

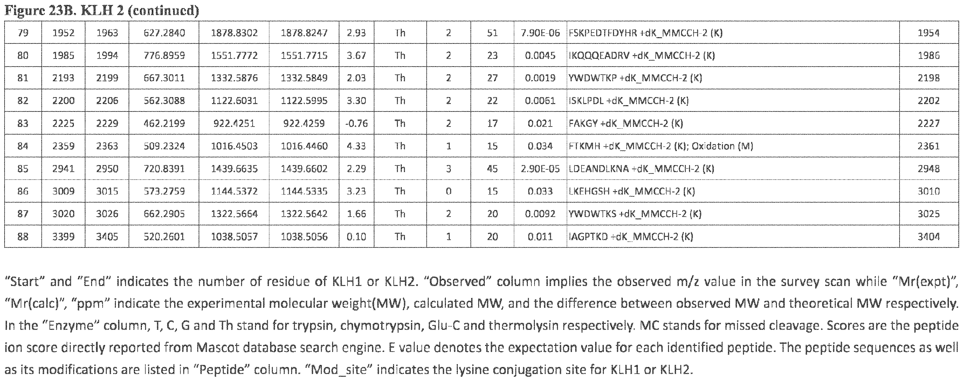

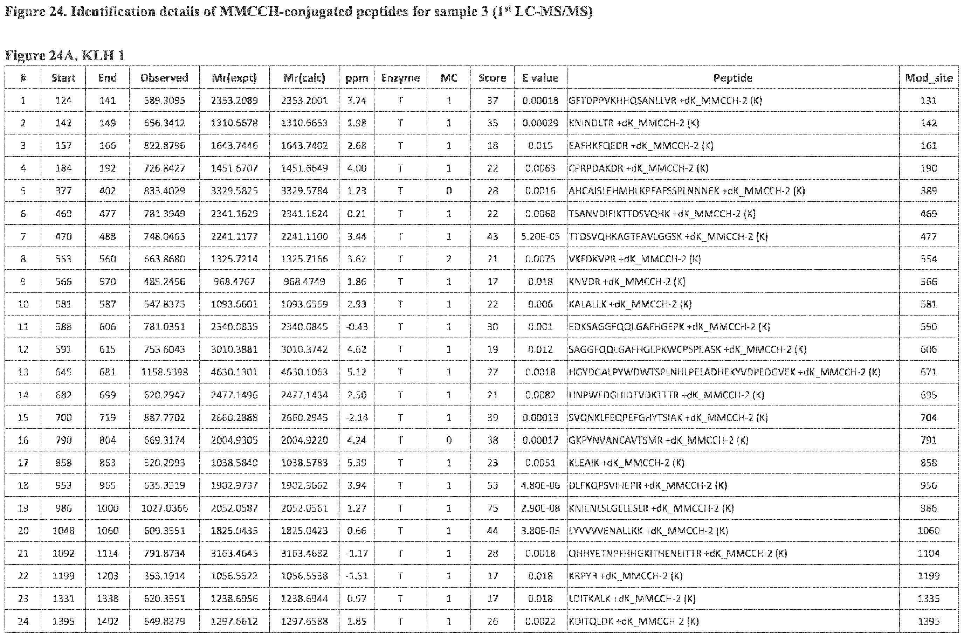

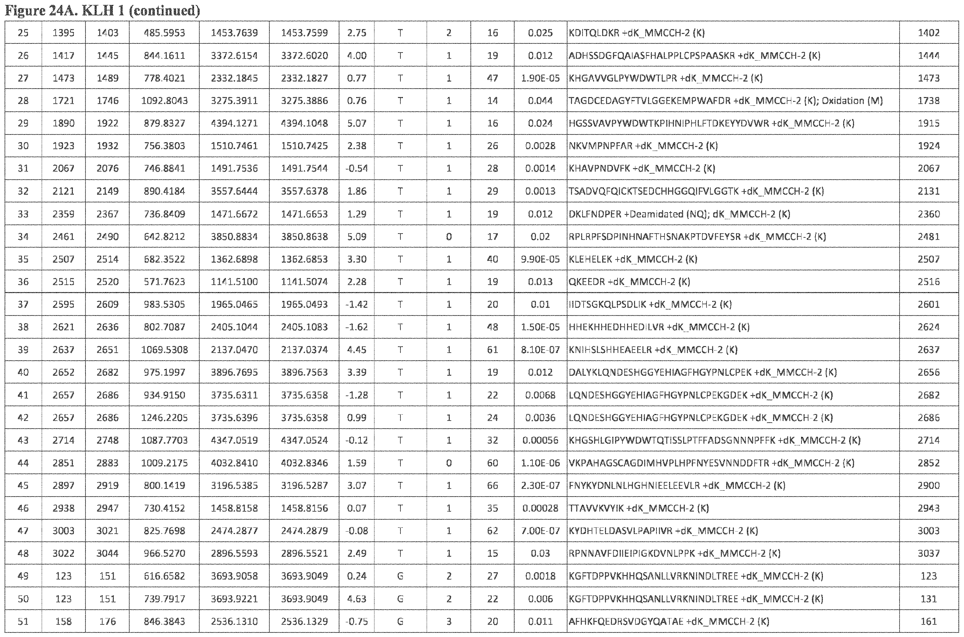

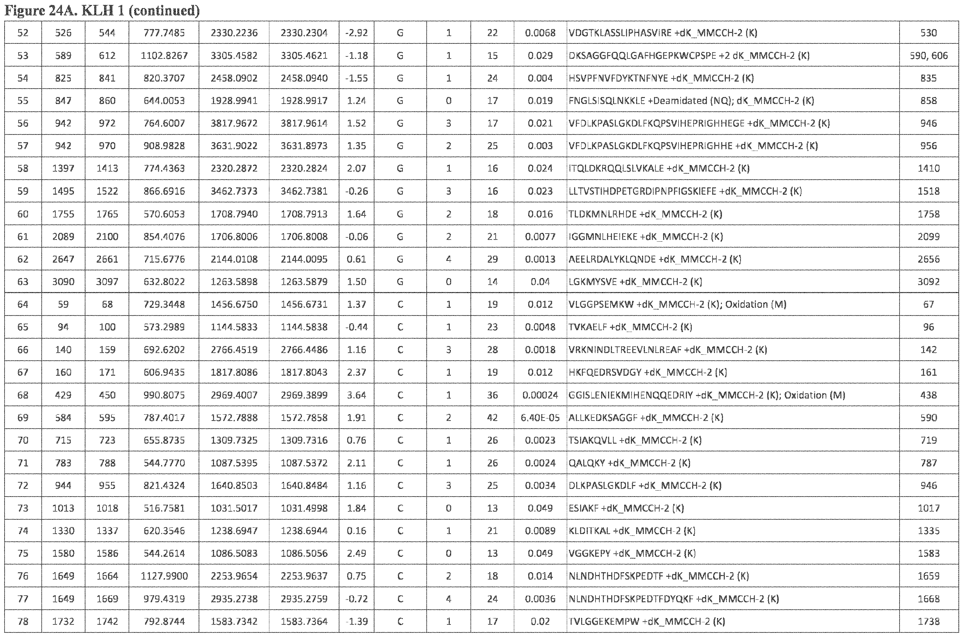

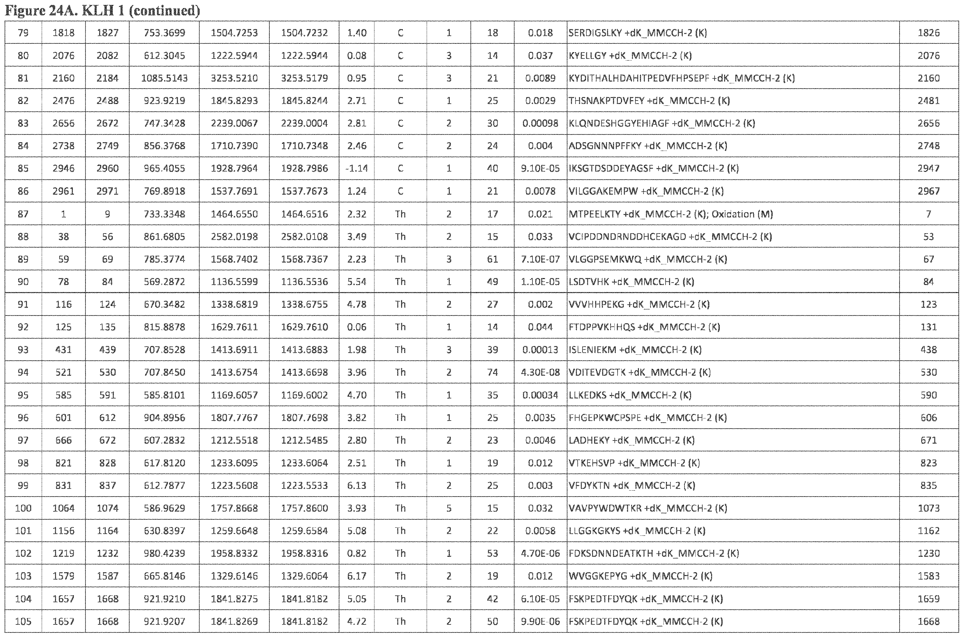

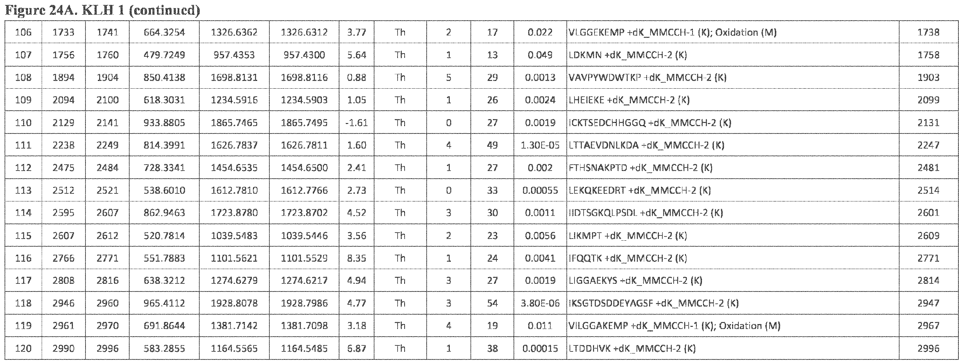

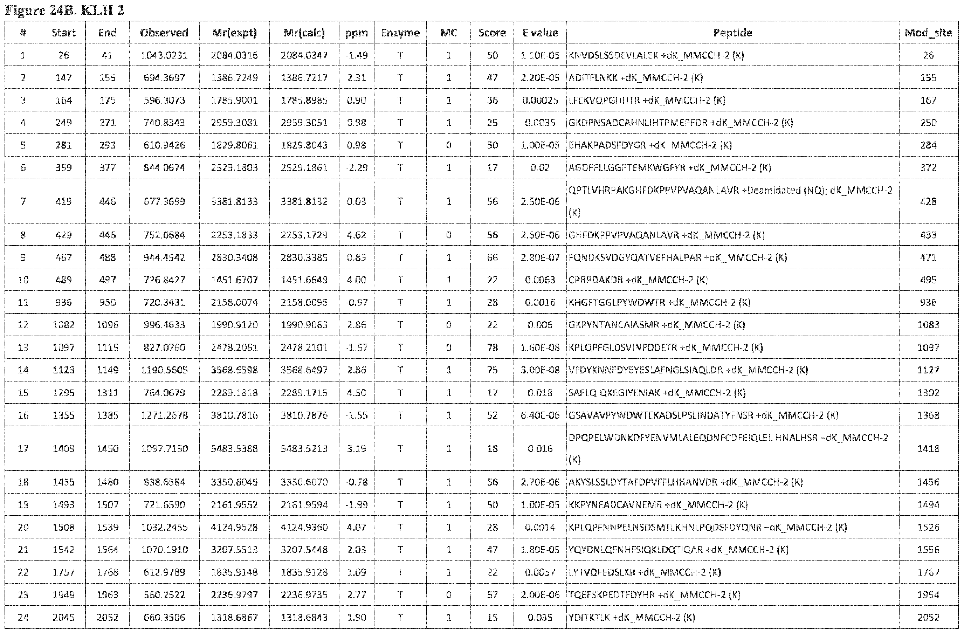

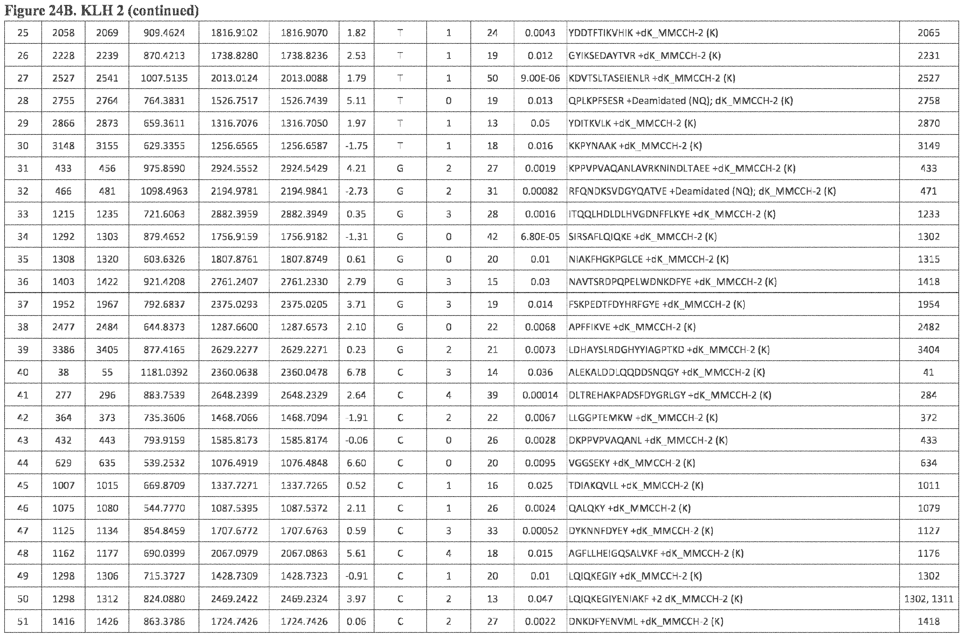

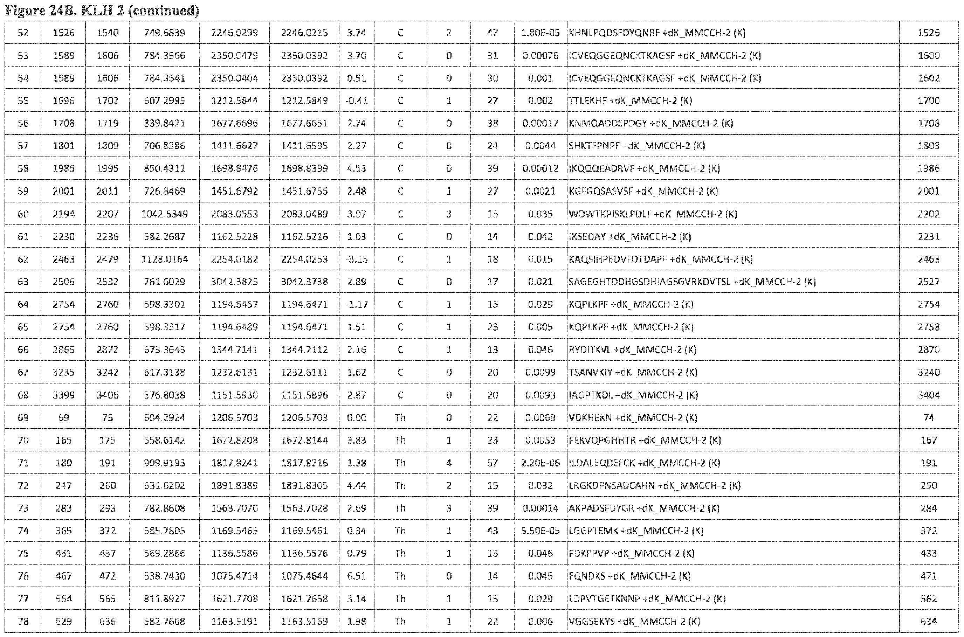

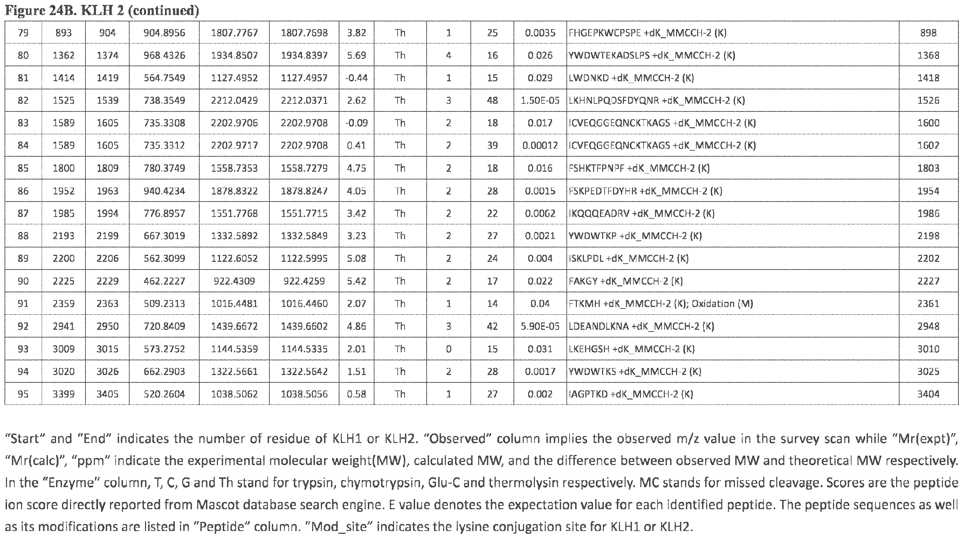

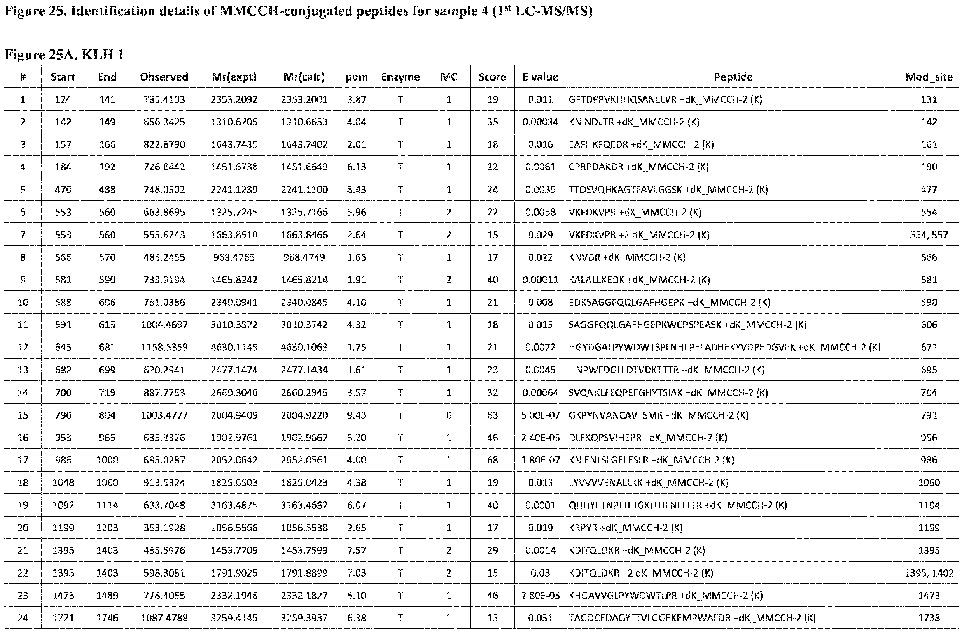

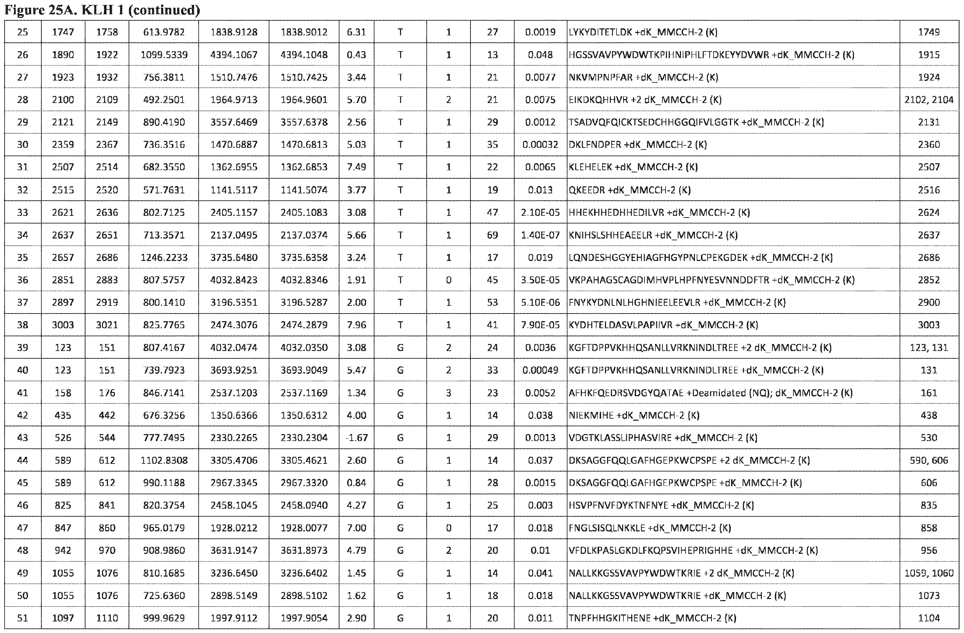

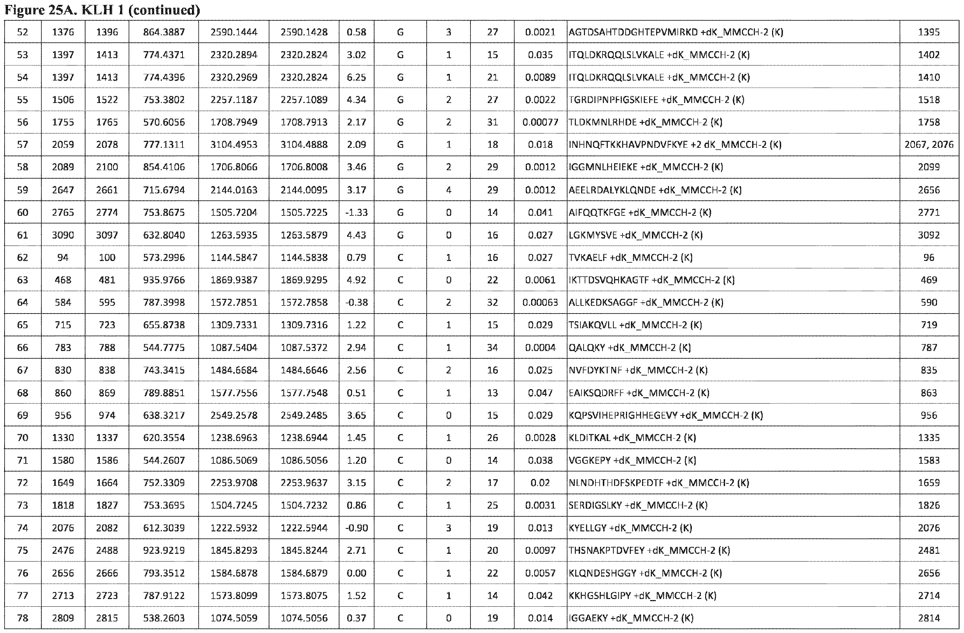

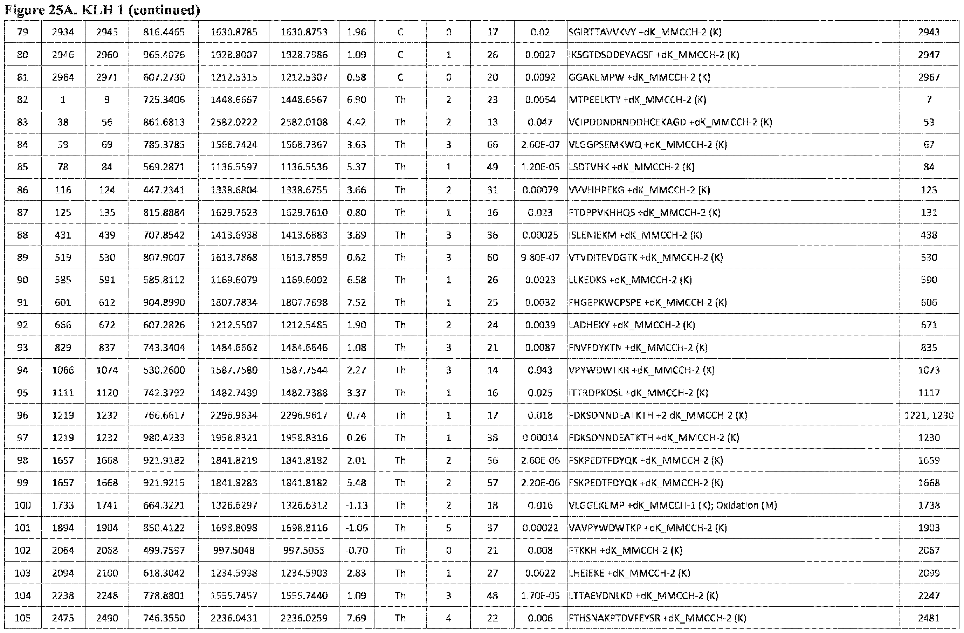

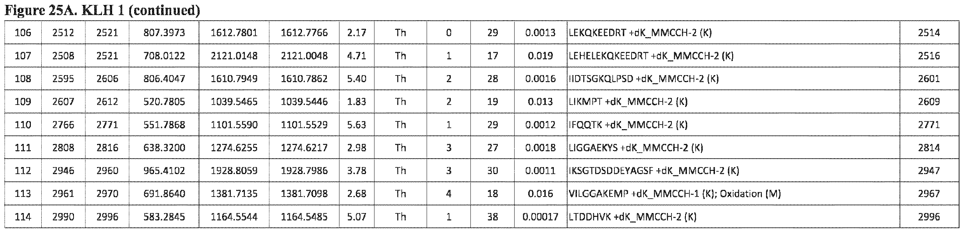

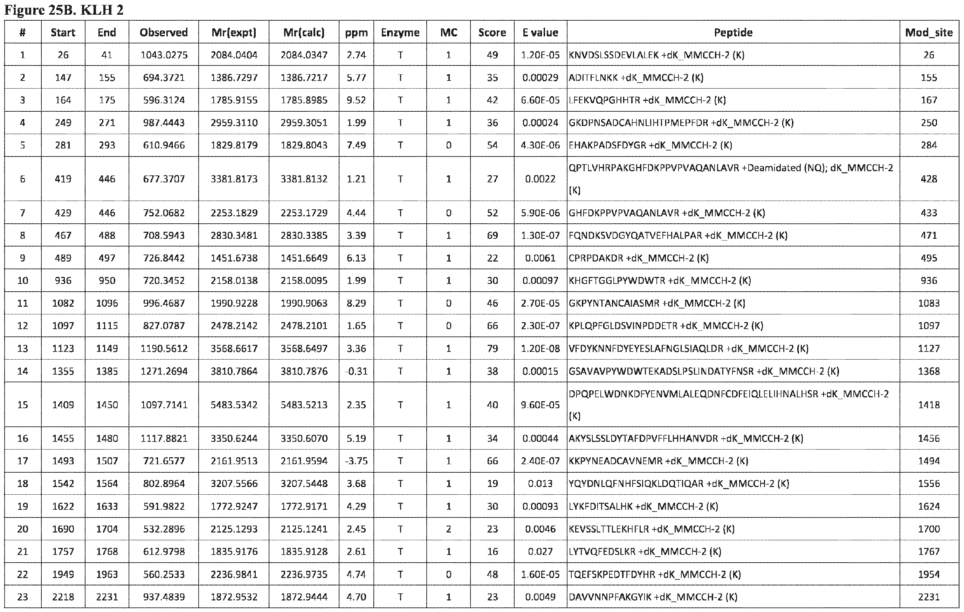

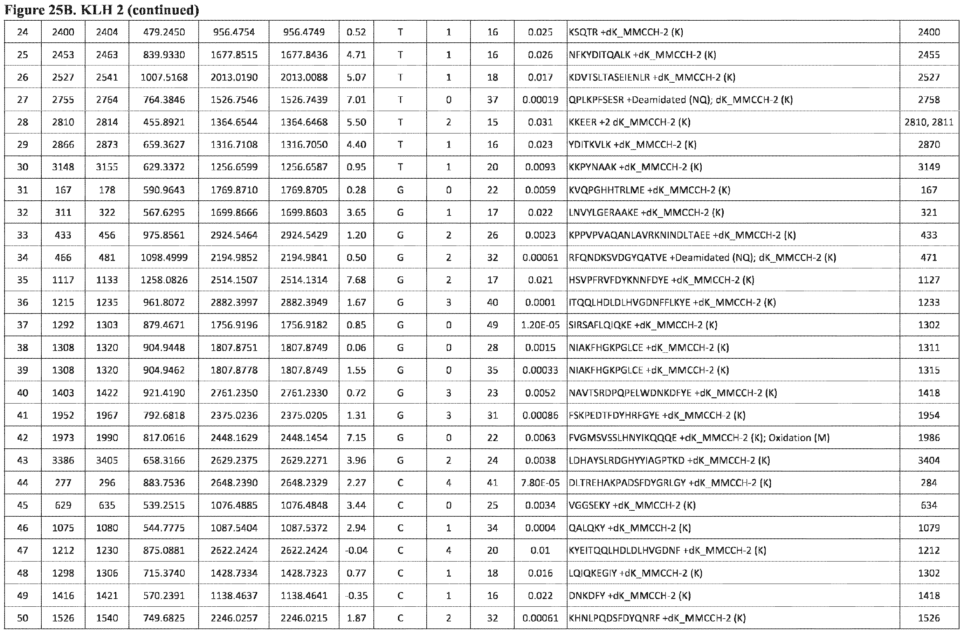

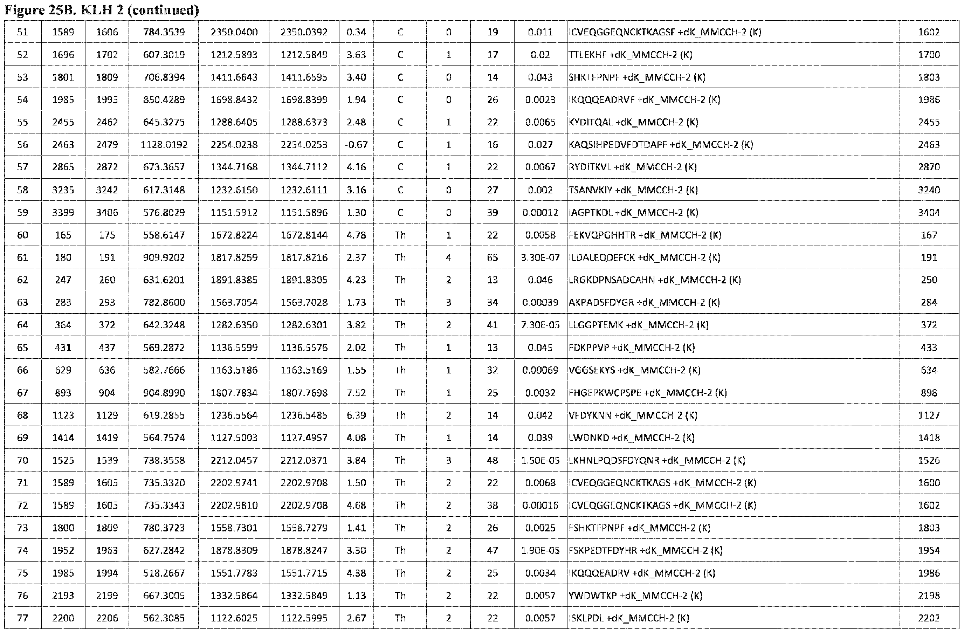

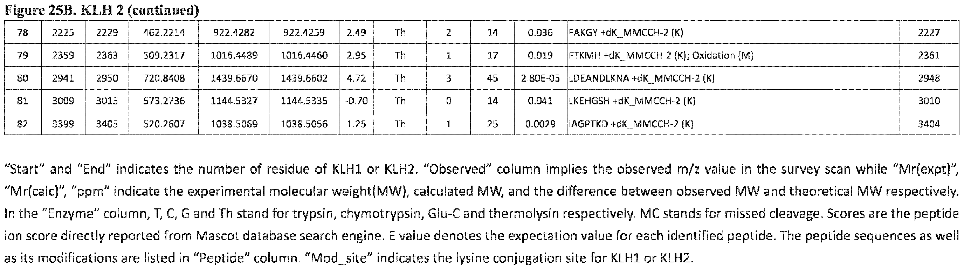

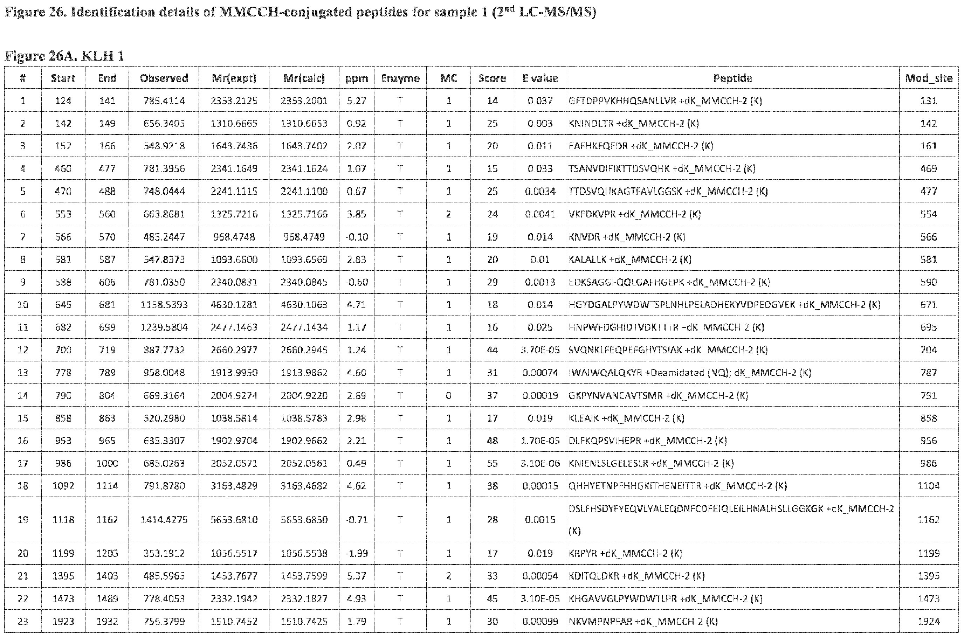

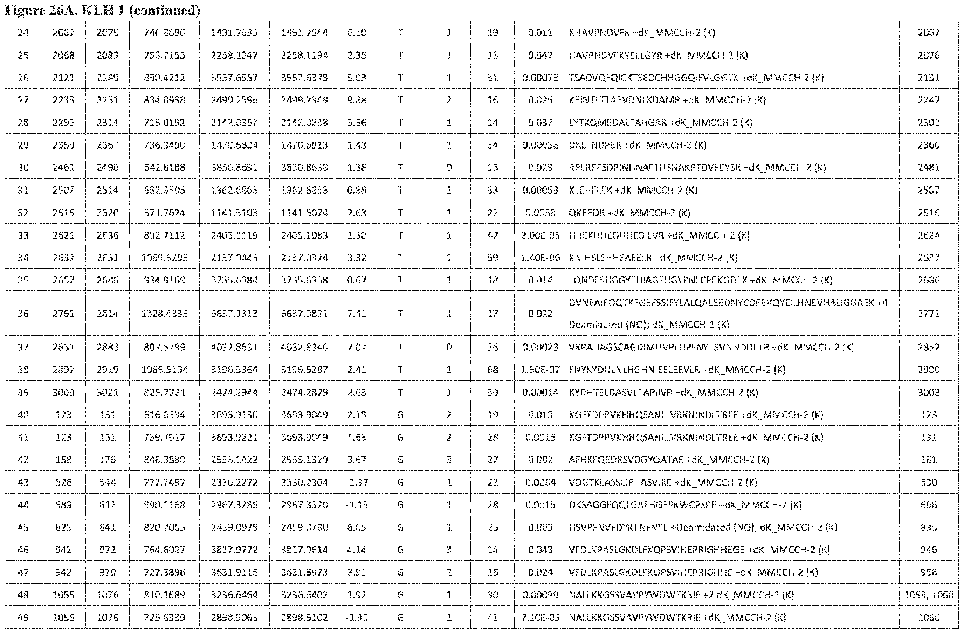

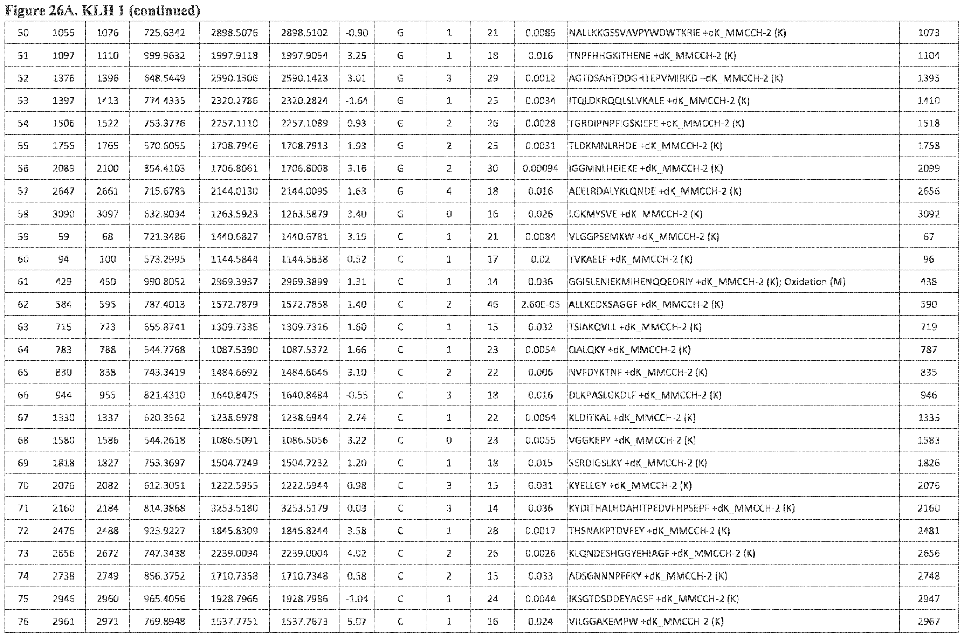

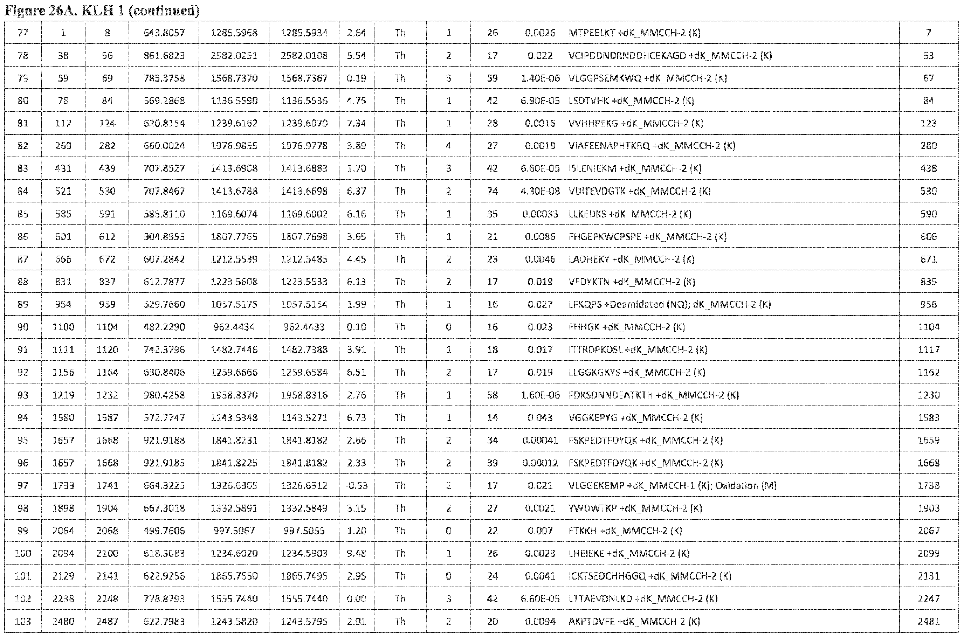

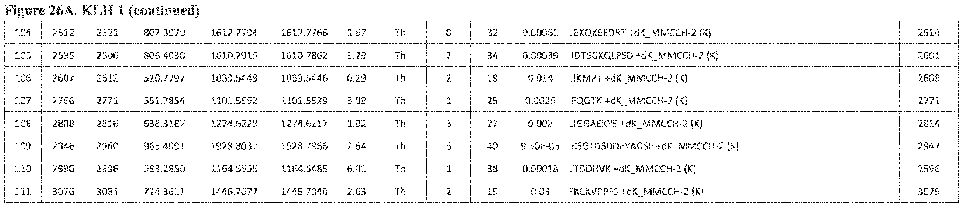

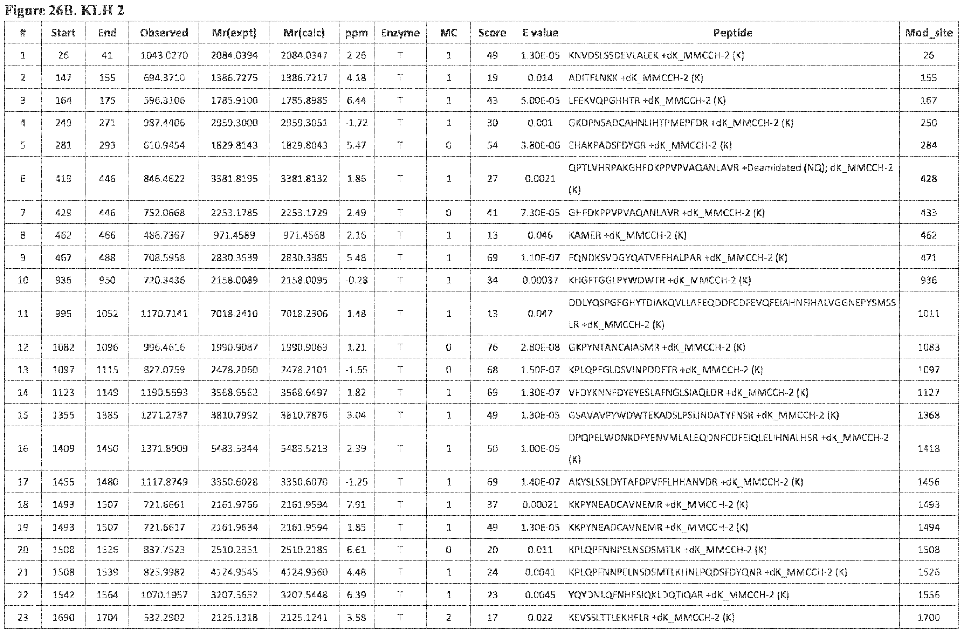

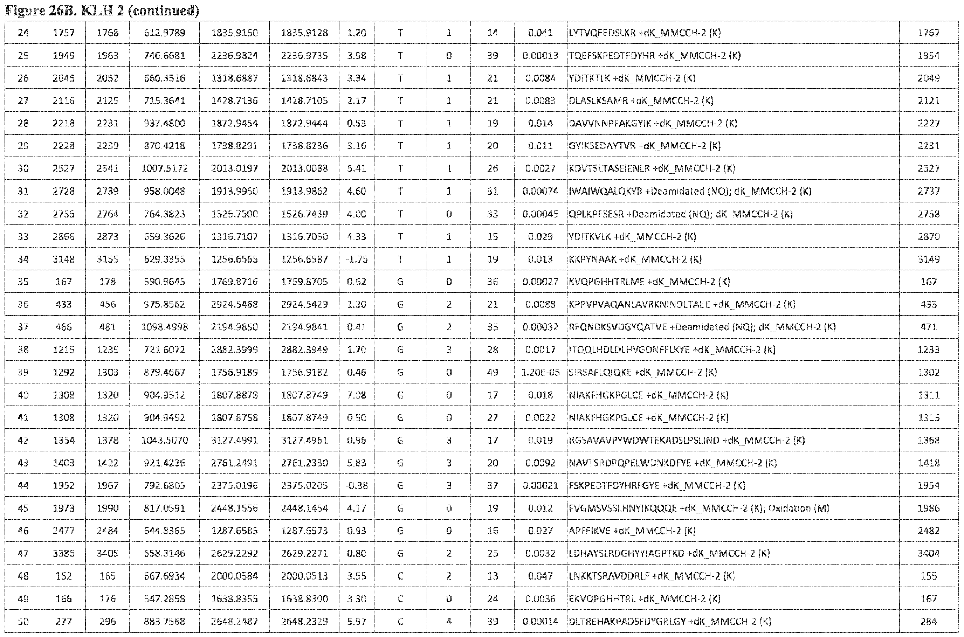

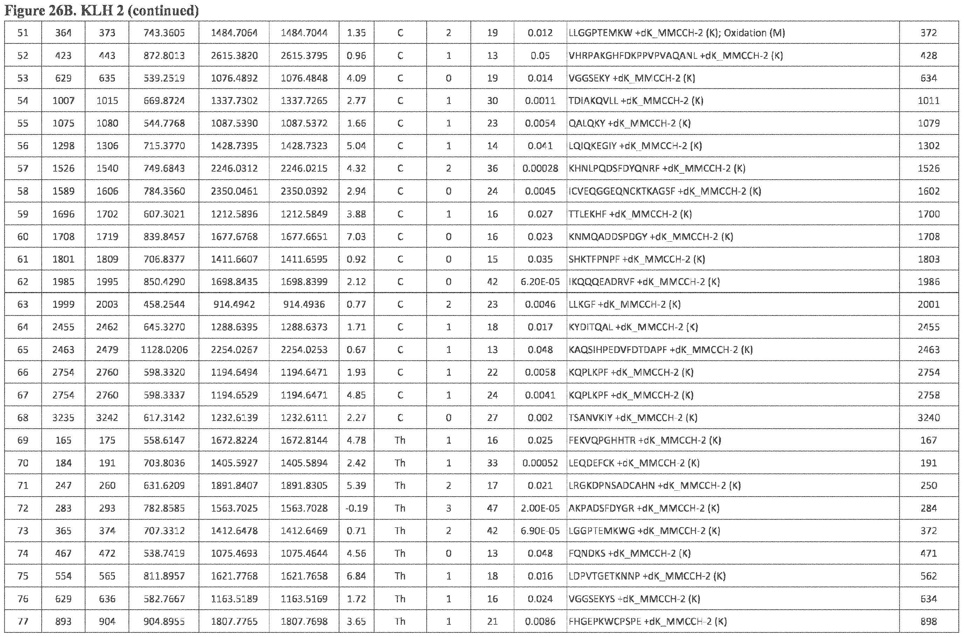

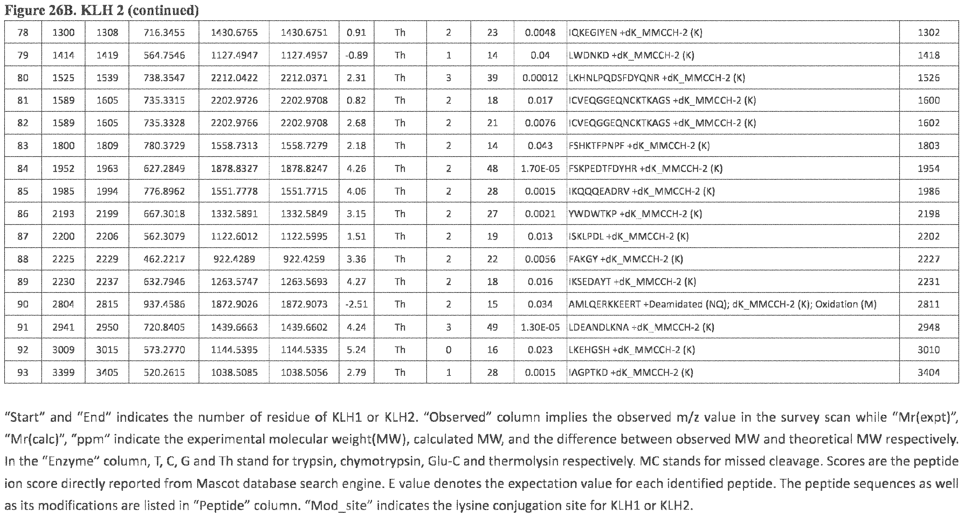

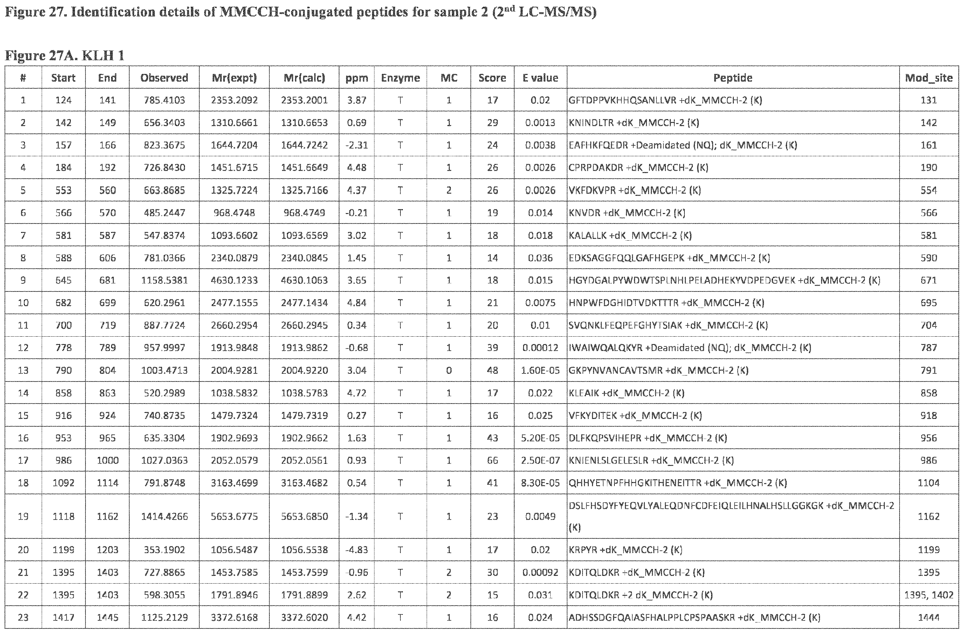

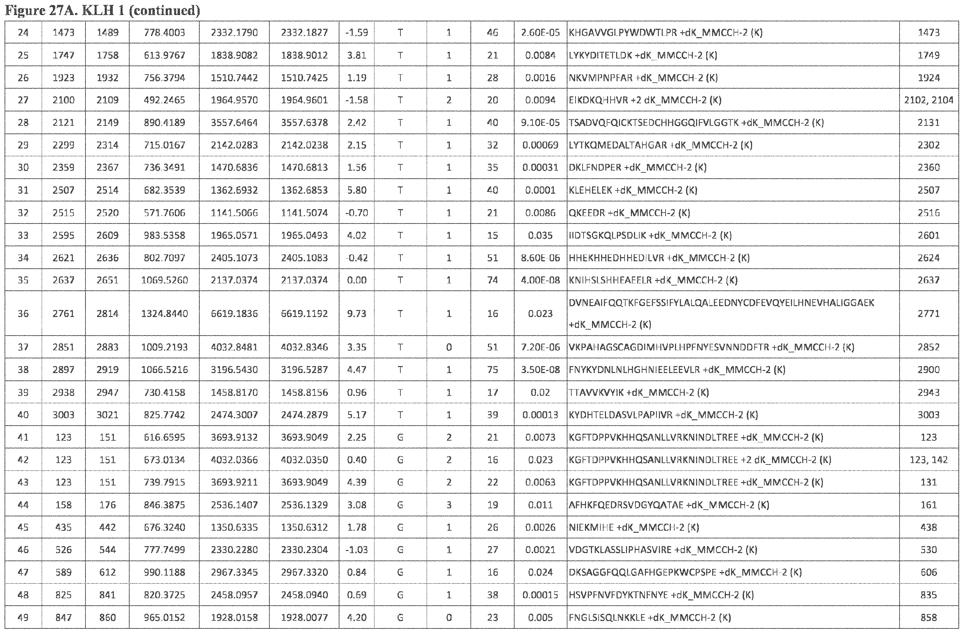

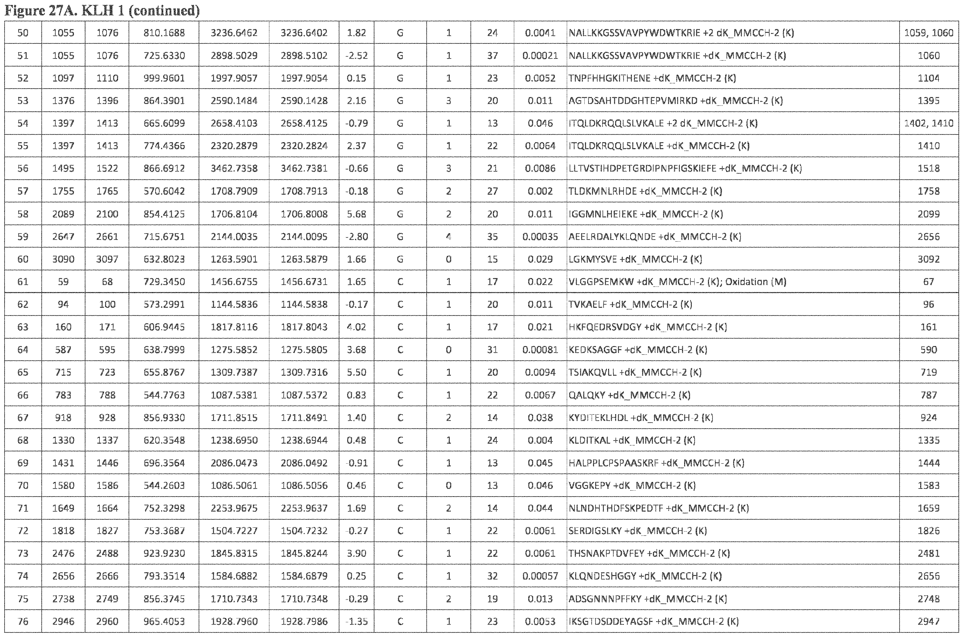

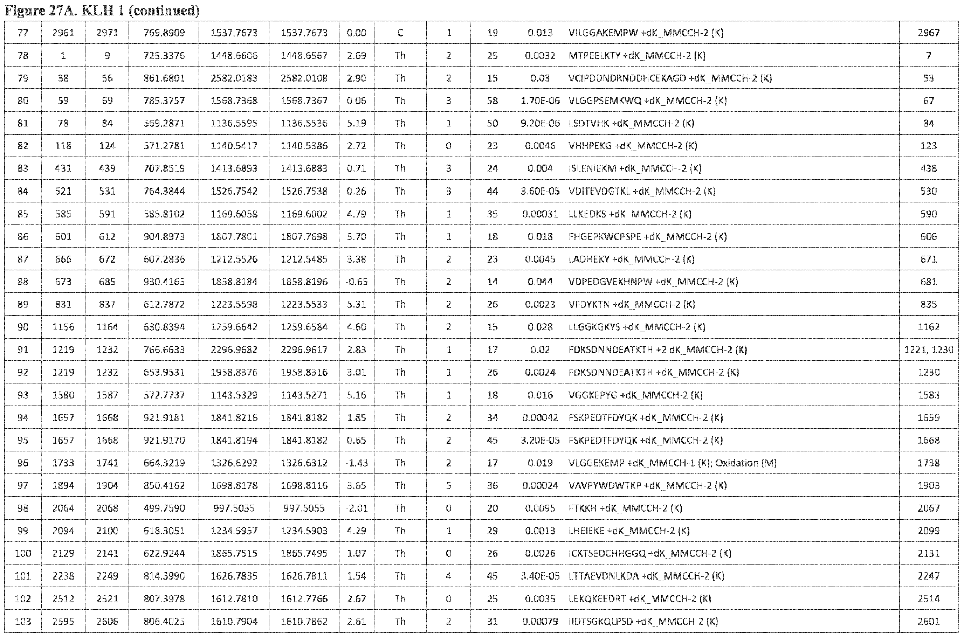

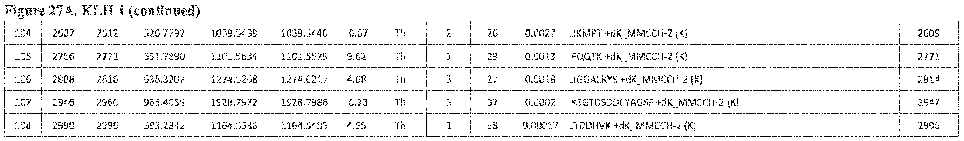

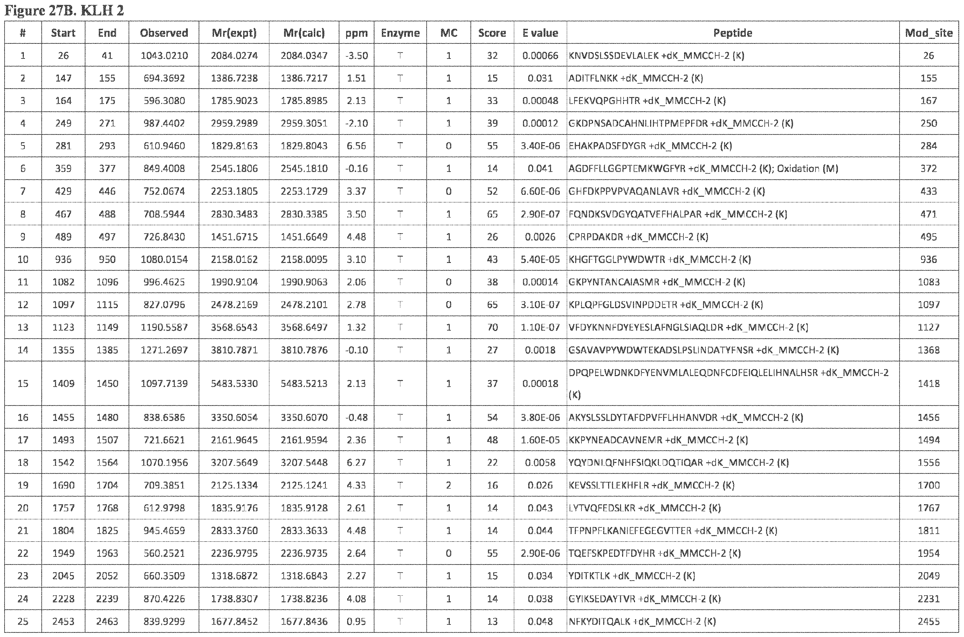

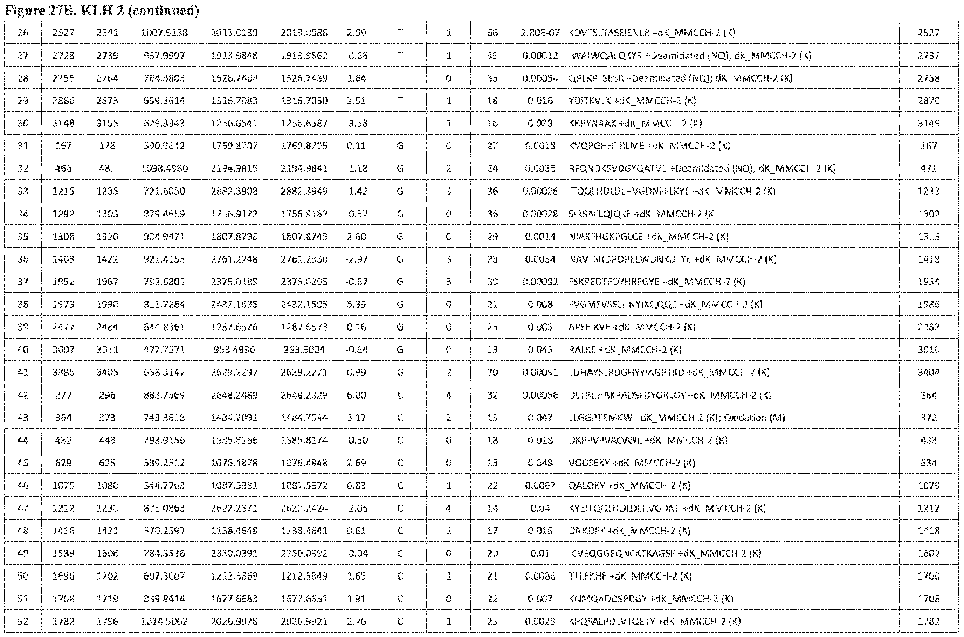

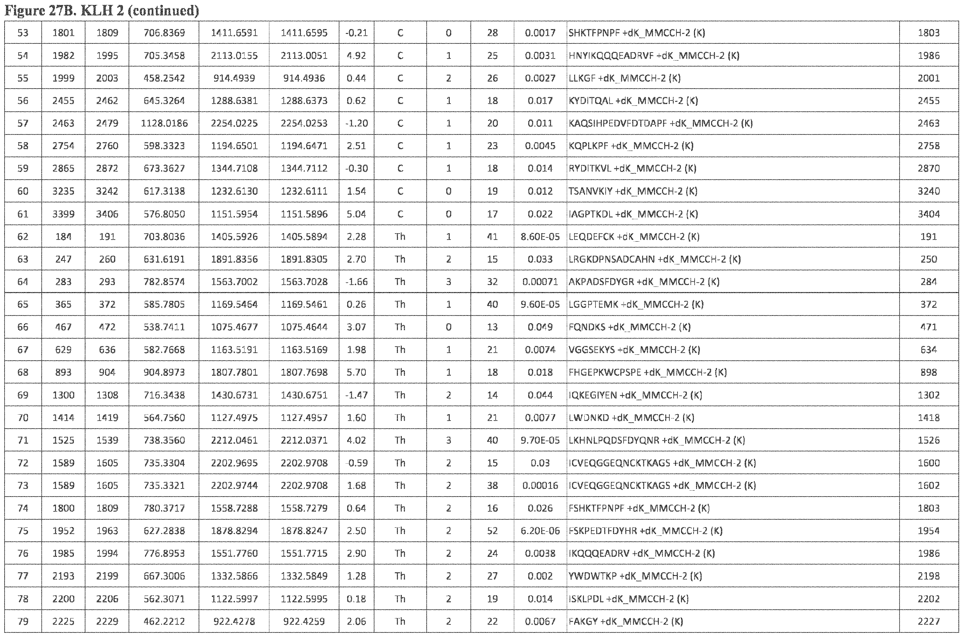

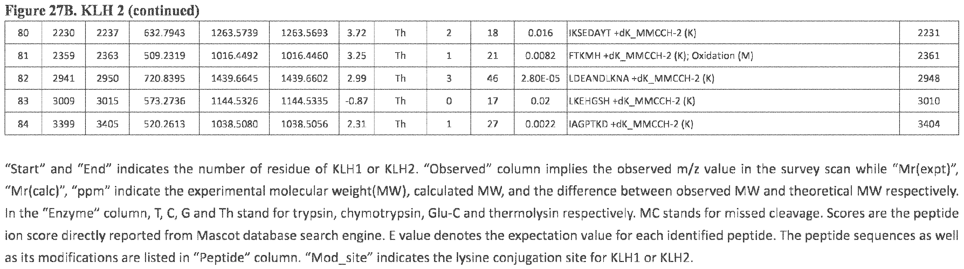

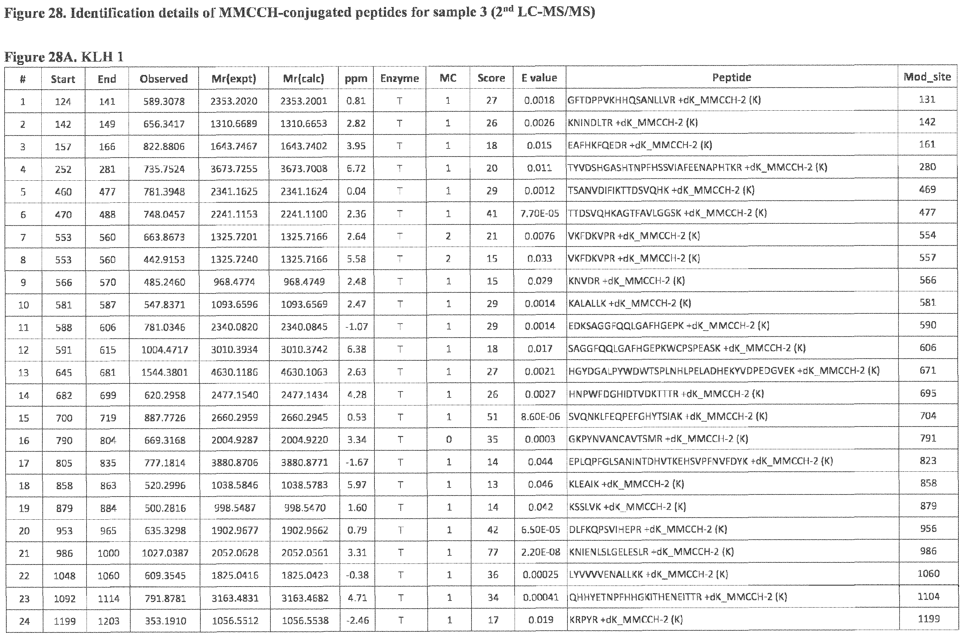

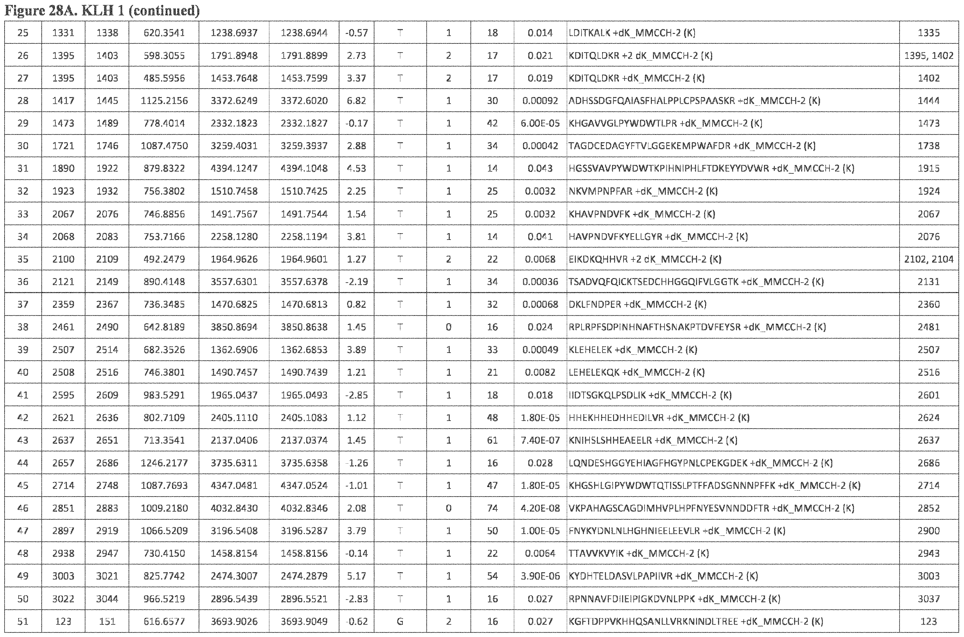

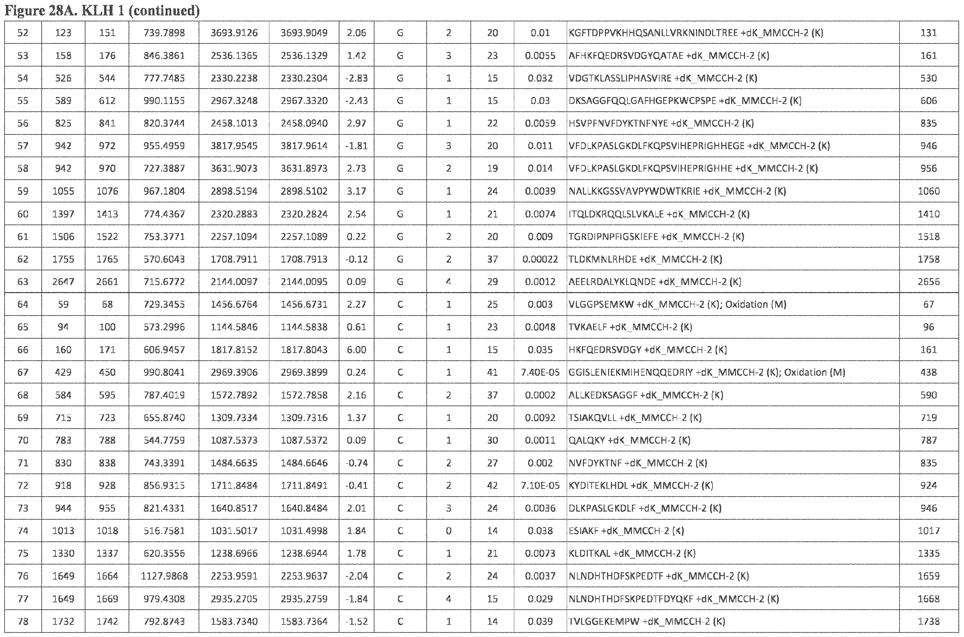

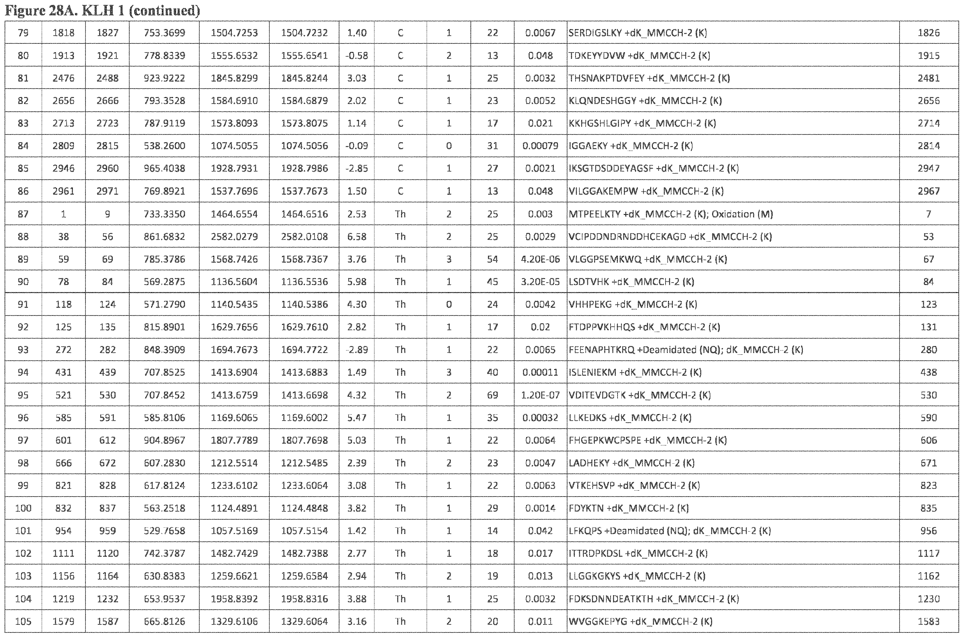

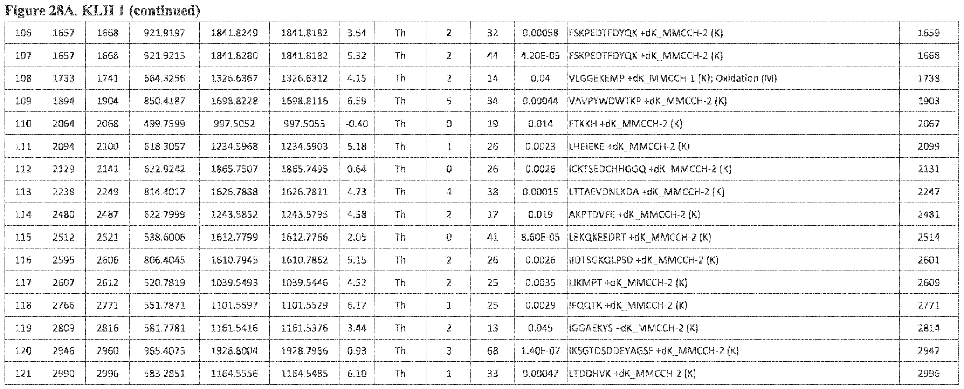

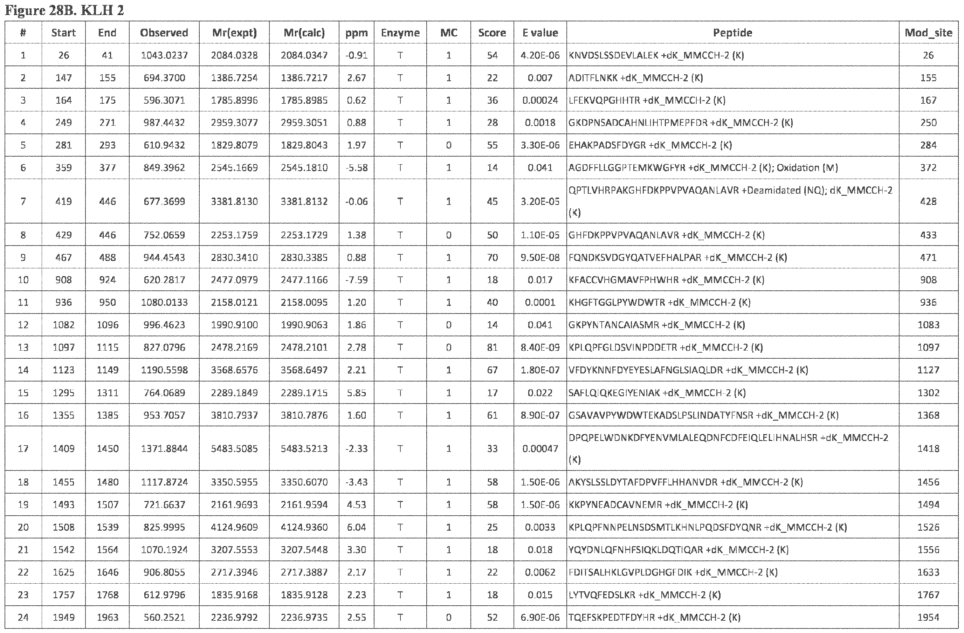

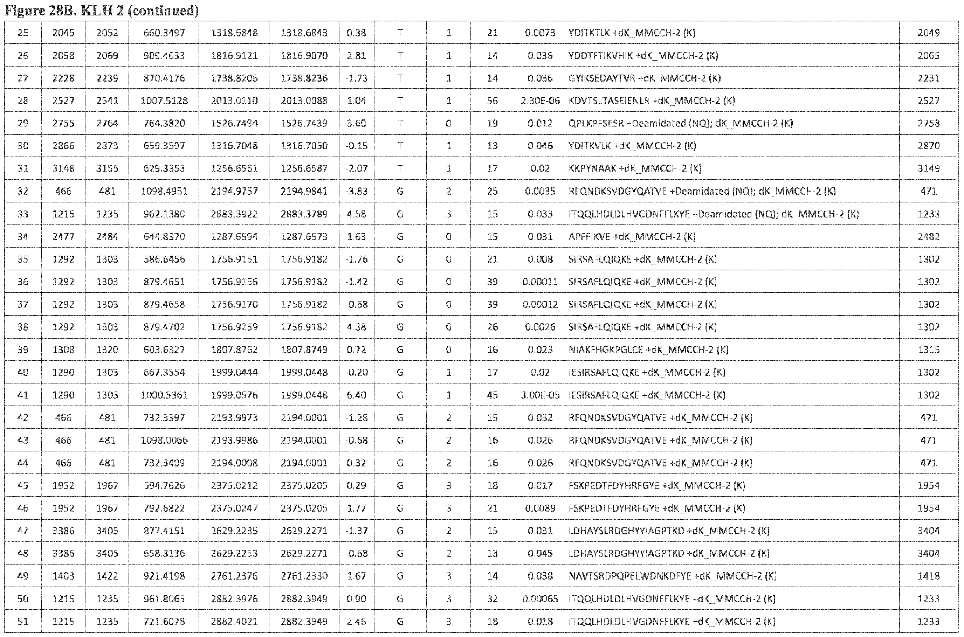

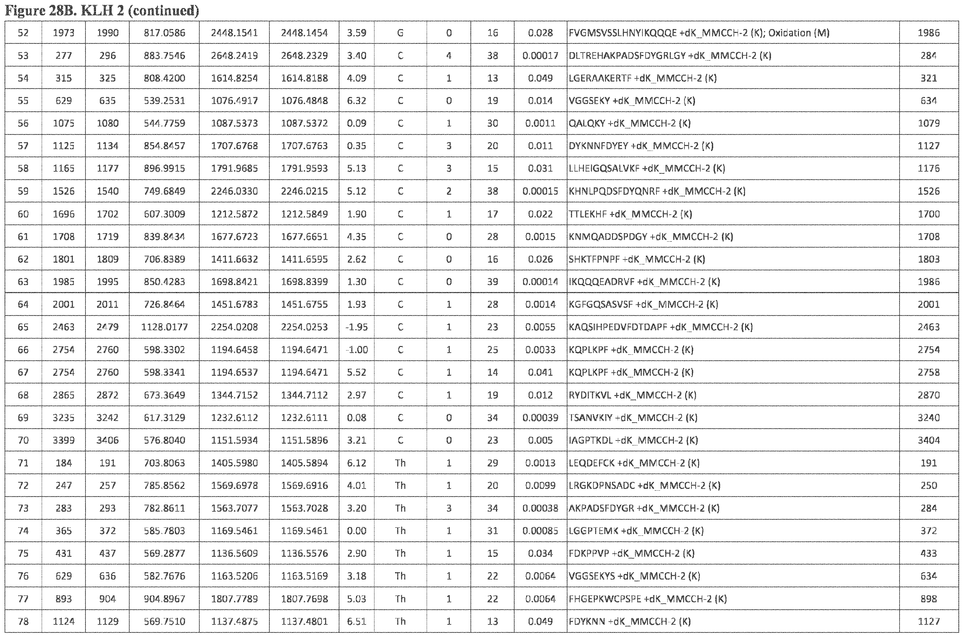

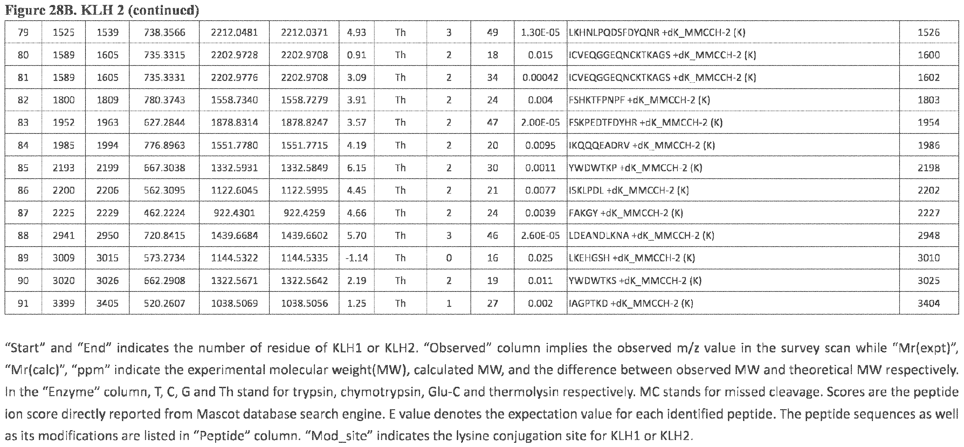

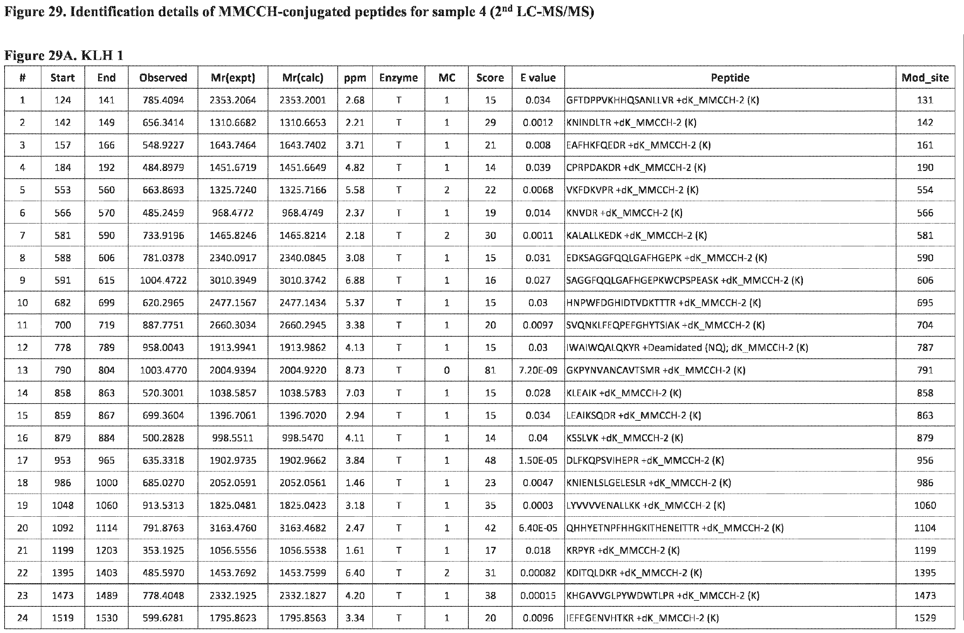

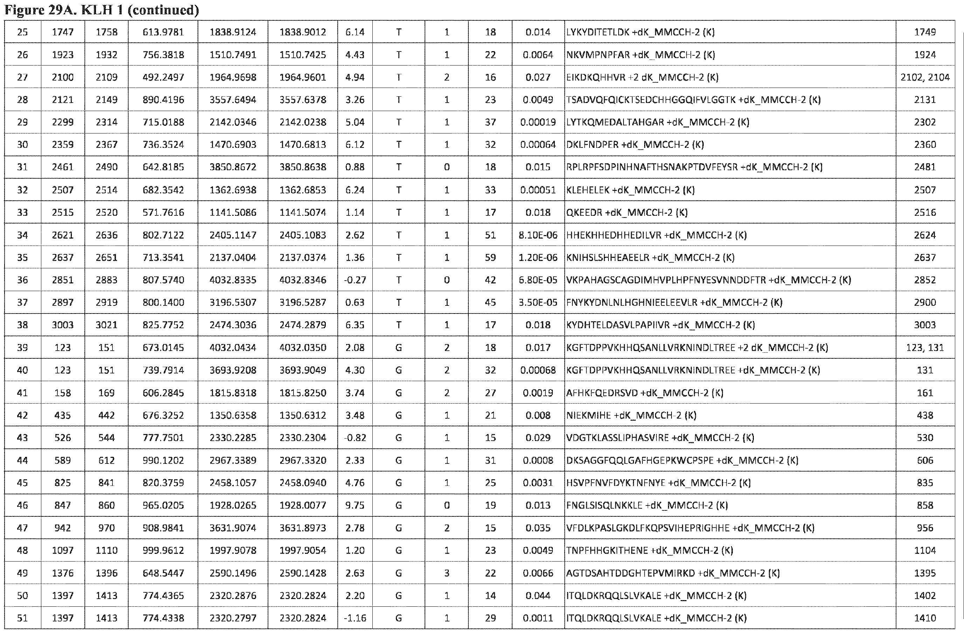

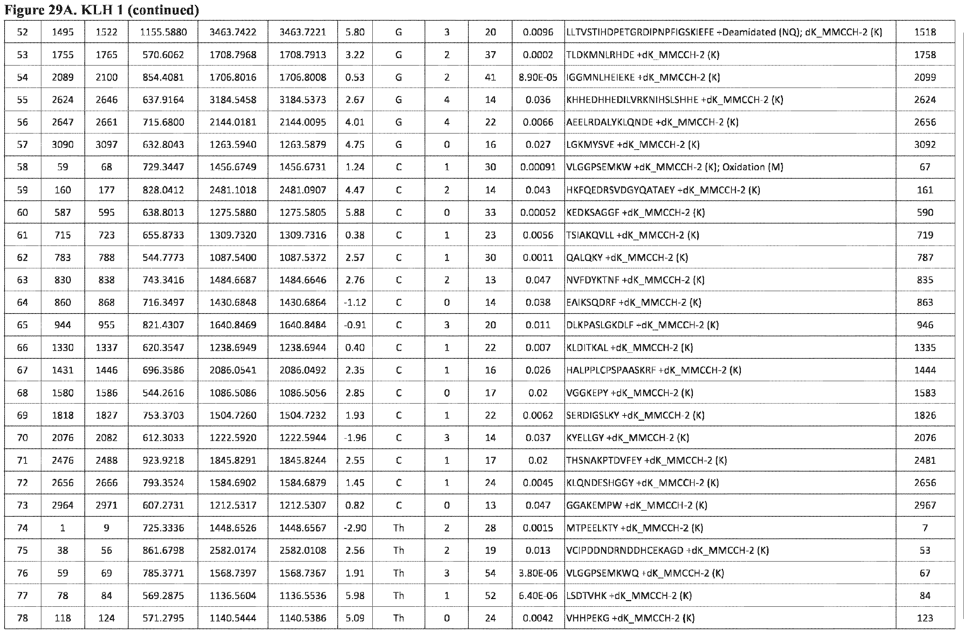

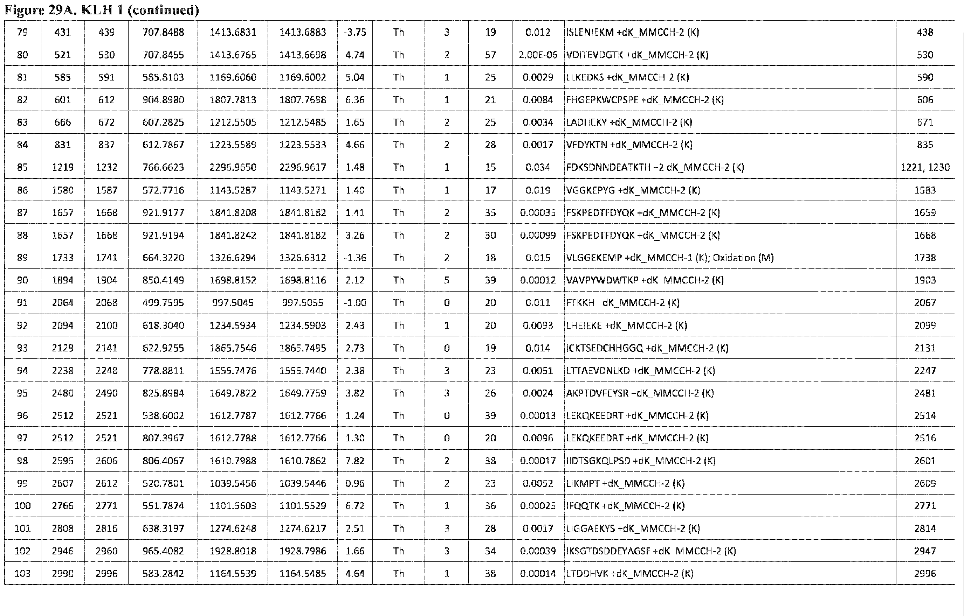

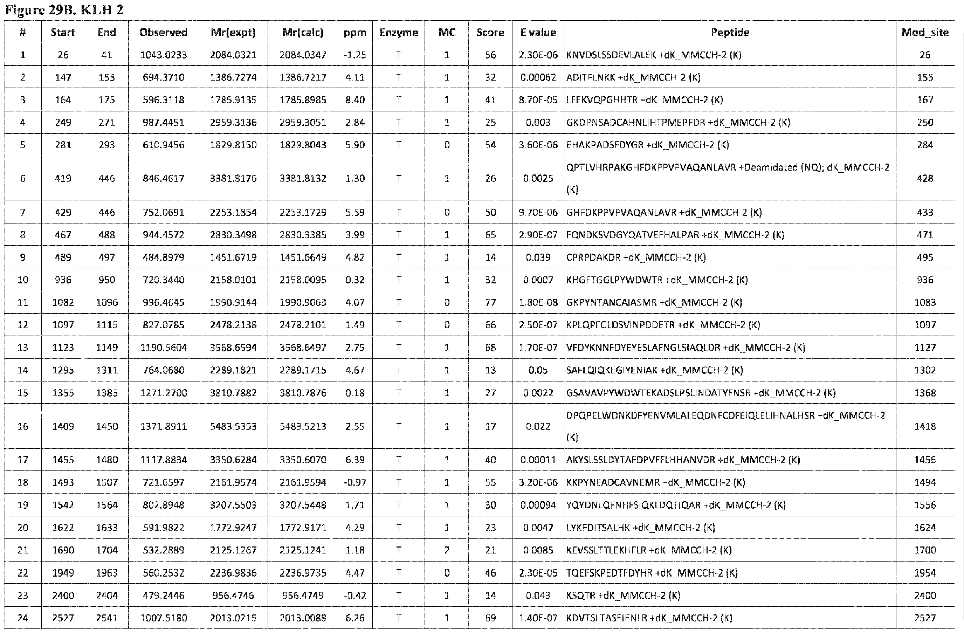

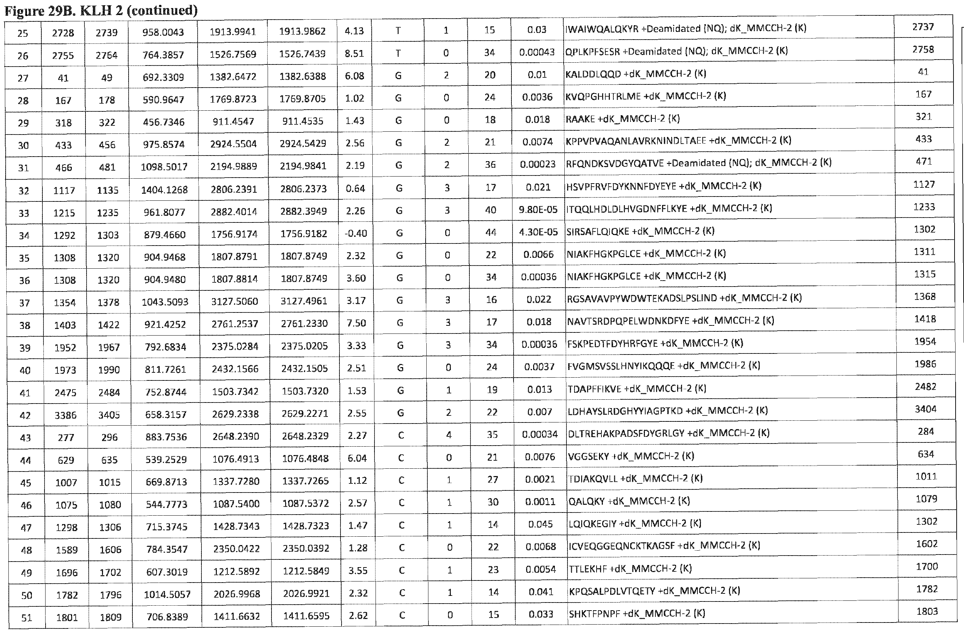

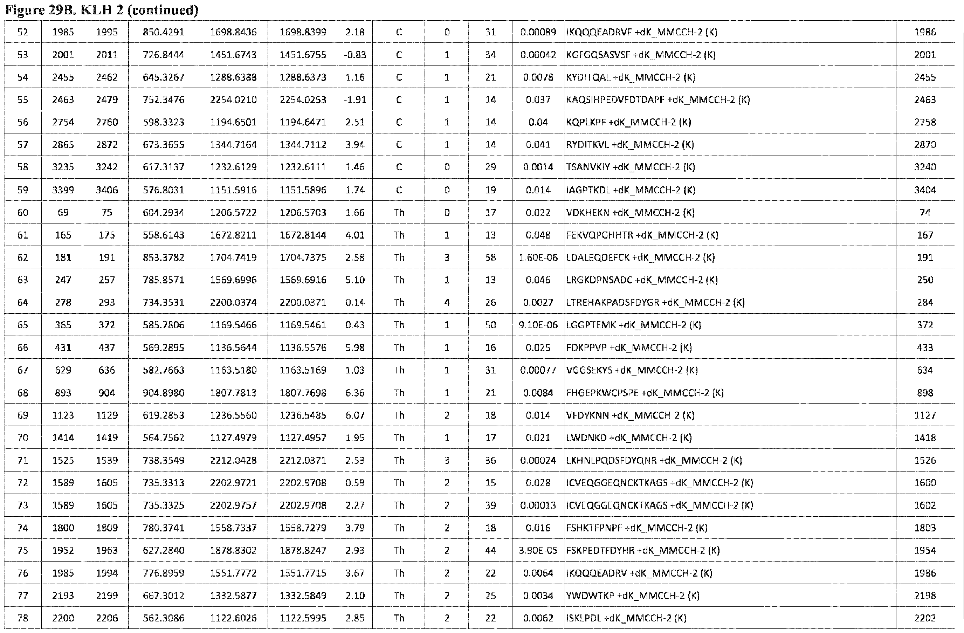

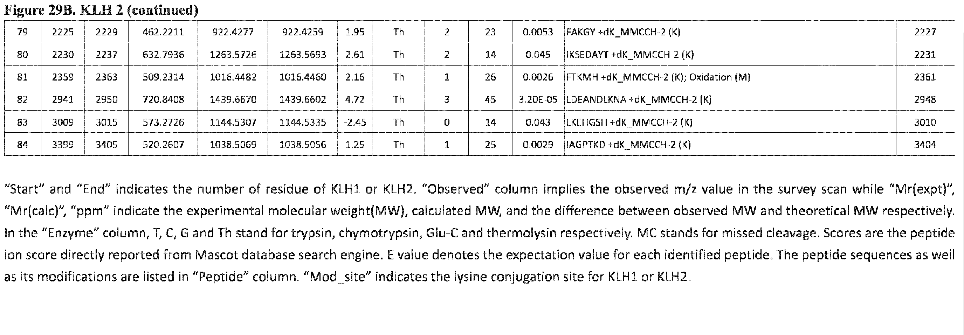

In Globo H conjugation site analysis, 31 and 28 lysines were found respectively in 1st LC-MS/MS and 2nd LC-MS/MS for sample 1; 19 and 21 lysines were found respectively in 1st LC-MS/MS and 2nd LC-MS/MS for sample 2; 10 and 11 lysines were found respectively in 1st LC-MS/MS and 2nd LC-MS/MS for sample 3; 18 and 19 lysines were found respectively in 1st LC-MS/MS and 2nd LC-MS/MS for sample 4. The identification details were listed in Figures 14 to 21. In MMCCH conjugation analysis, 155 and 141 lysines were found respectively in 1st LC-MS/MS and 2nd LC-MS/MS for sample 1; 143 and 137 lysines were found respectively in 1st LC-MS/MS and 2nd LC- MS/MS for sample 2; 147 and 143 lysines were found respectively in 1st LC-MS/MS and 2nd LC-MS/MS for sample 3; 140 and 136 lysines were found respectively in 1st LC- MS/MS and 2nd LC-MS/MS for sample 4. The identification details were listed in Figures 22 to 29.

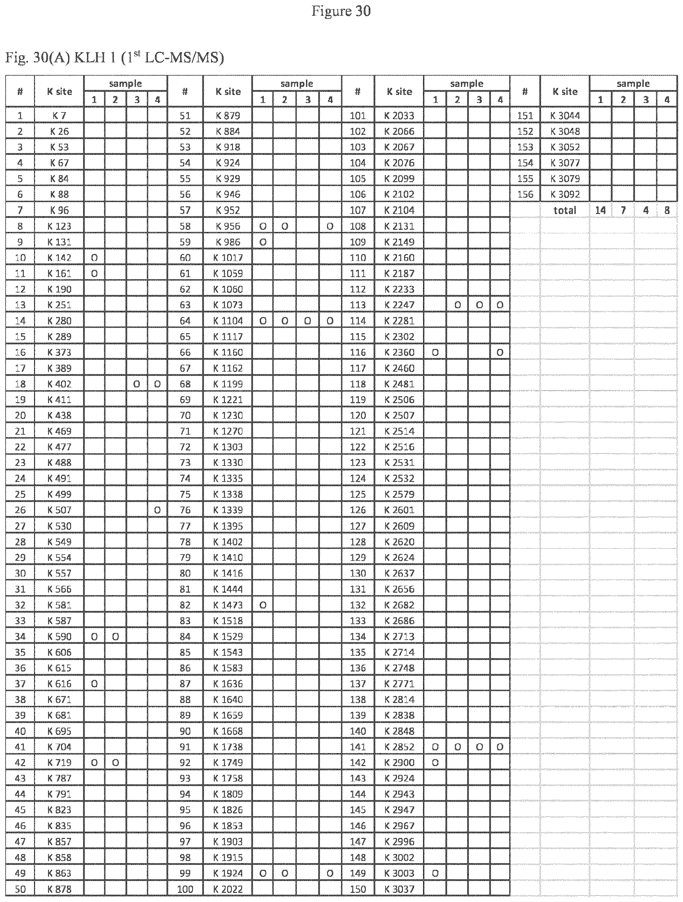

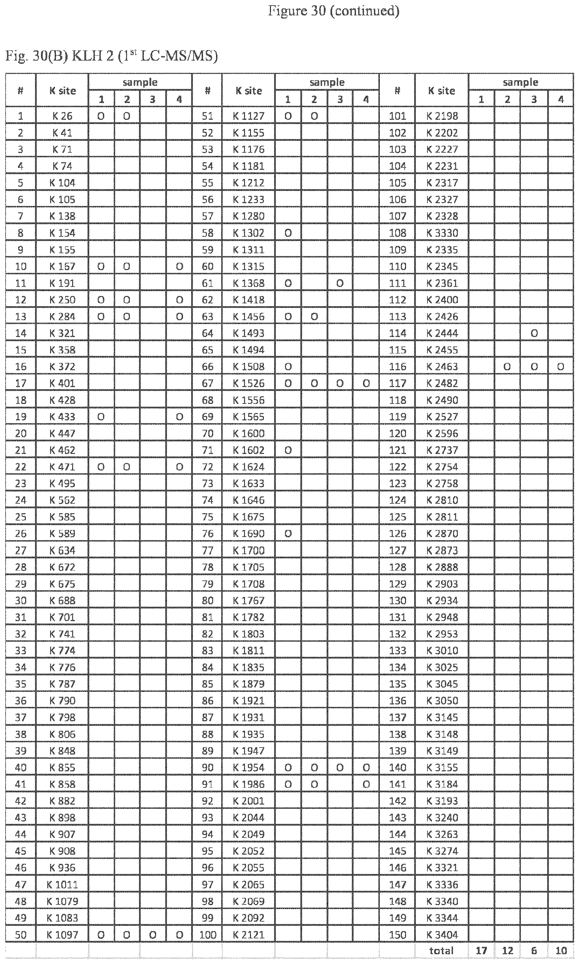

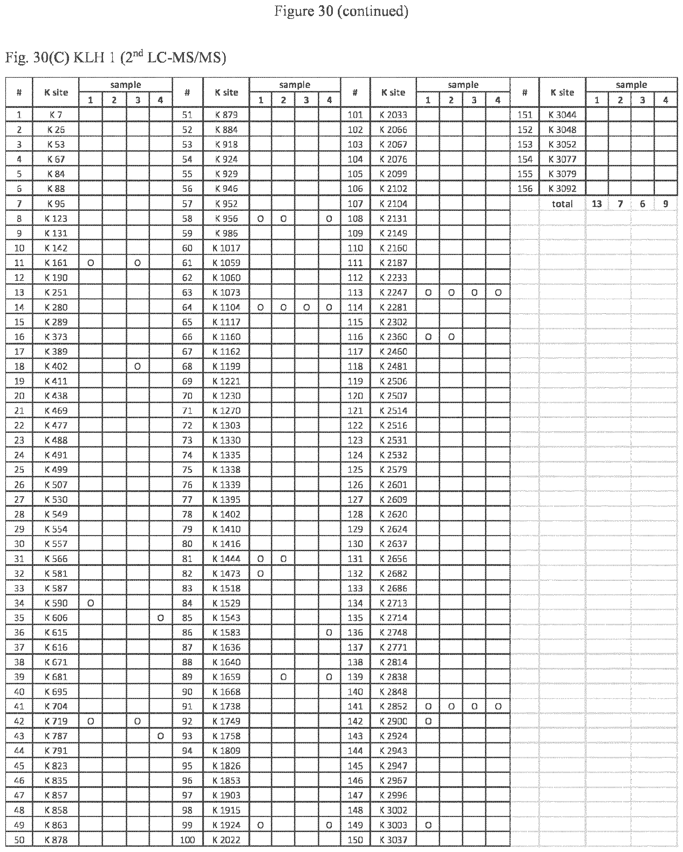

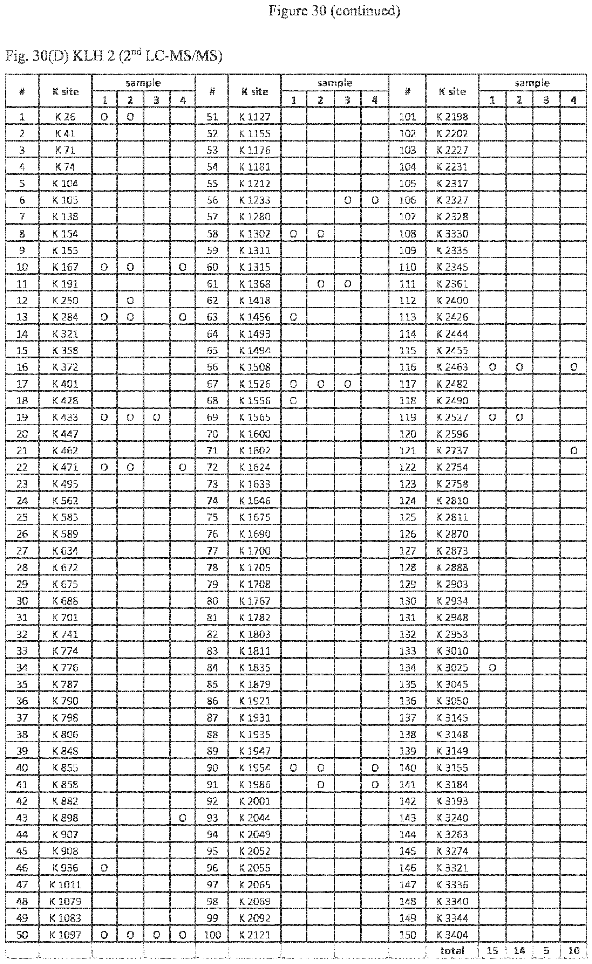

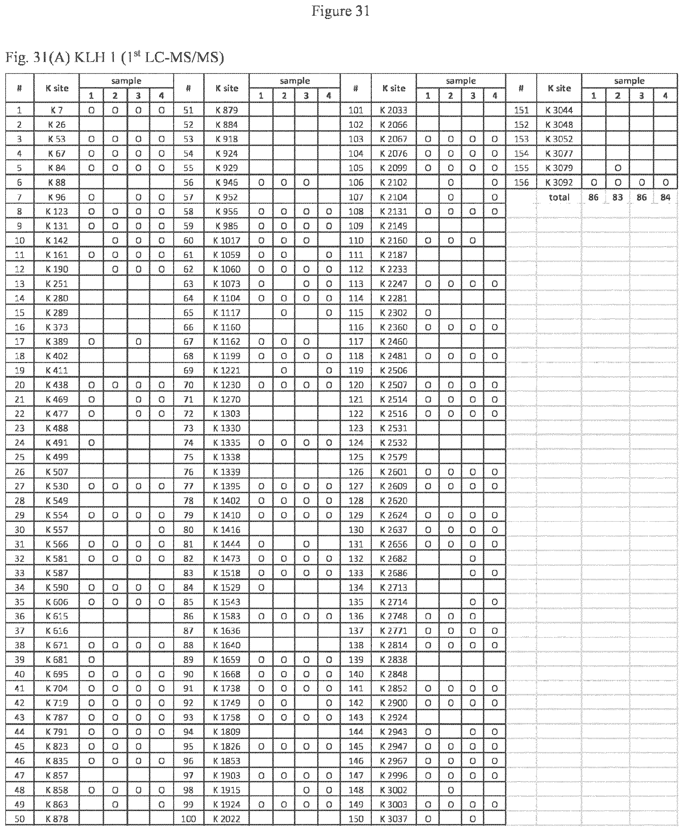

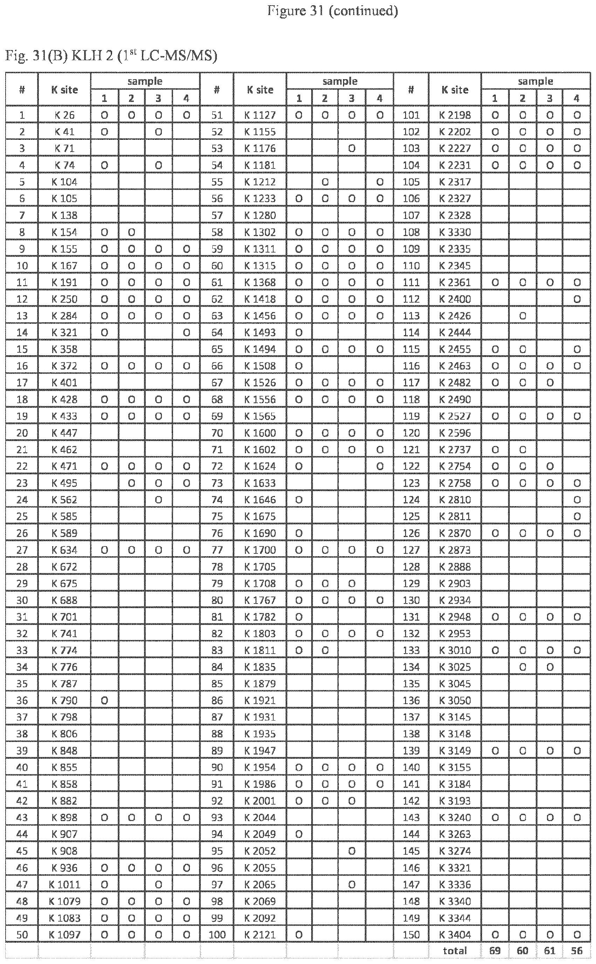

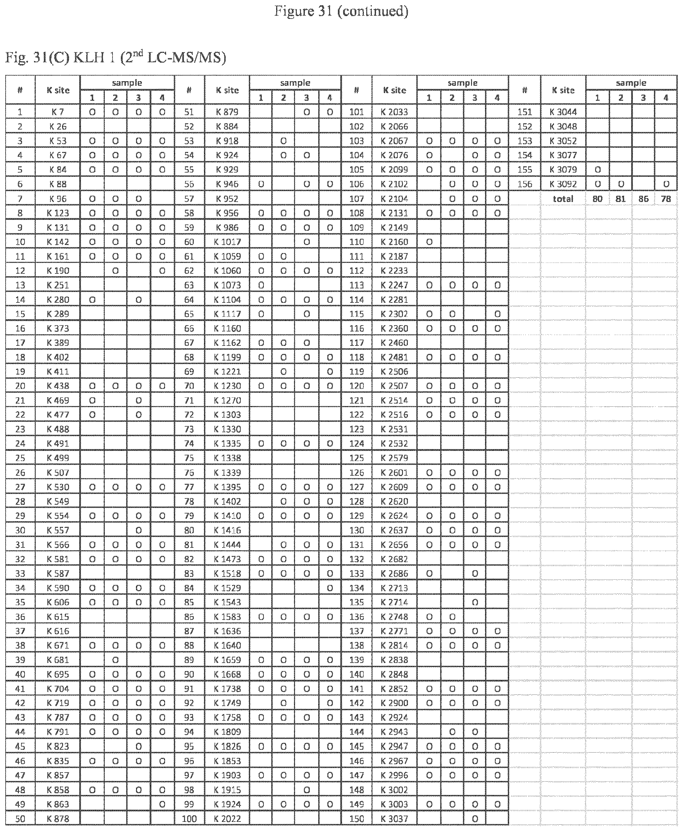

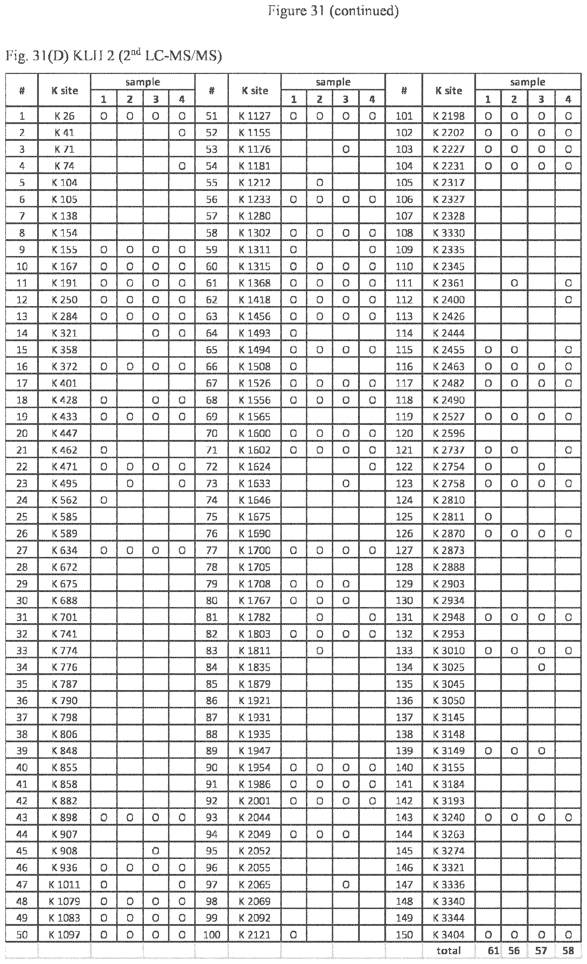

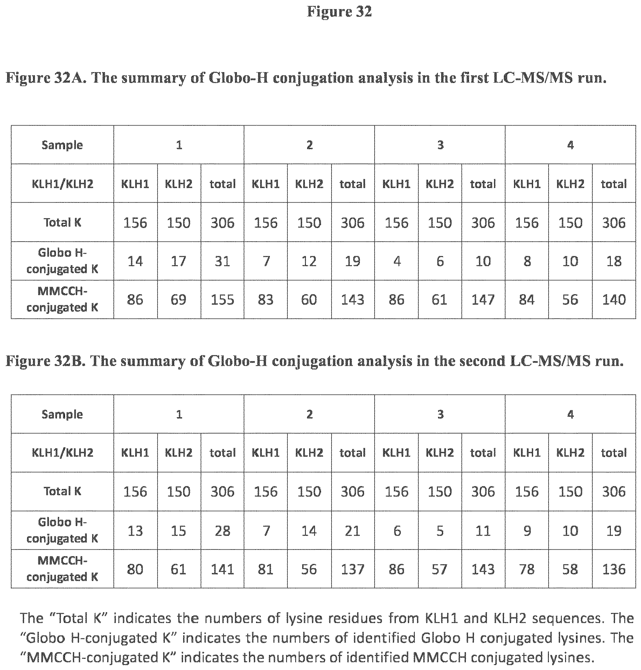

Globo H conjugation sites from multiple enzyme experiments were summarized in Figure 30 and MMCCH conjugation sites were summarized in Figure 31. The overall conjugation site analysis results for Globo H conjugated samples were summarized in Figure 32.

Example 8 Conclusion:

The mass spectrometric signals of Globo H derivative conjugated peptides are lower than those of MMCCH conjugated peptides due to the multiple neutral loss forms and lower ionization efficiency of polysaccharide, which makes the direct identification of Globo H conjugation more difficult. This is why the numbers of identified peptides are higher for MMCCH conjugation analysis. Therefore, the MMCCH results may be used to represent the Globo H conjugation.

The conjugation site analysis suggests that there are 155, 143, 147 and 140 lysine conjugation sites identified among the total 306 lysine residues in KLH1/KLH2 for sample 1-4, respectively. In the replicate analysis, 141, 137, 143 and 136 conjugation sites were identified for samples 1-4, respectively.

Example 9: LC-MS/MS Analysis of Globo-H Conjugation Sites (Histidine, Asparagine, Proline, Glutamine and Arginine) on KLH

Two types of derivatives on the five residues, H, N, P, Q, R were examined: GMd (Globo H + MMCCH linker + lysine derivative) and Md (MMCCH linker + lysine derivative).

Modification analysis on HNPQR (GMd and Md) yielded 6 and 13 conjugated HNPQR sites in KLH1 and KLH2, respectively, for OBI-822-13001-DP (sample 1); 11 and 10 conjugated HNPQR sites in KLH1 and KLH2, respectively, for OBI-822-13002-DP (sample 2); 13 and 9 conjugated HNPQR sites in KLH1 and KLH2, respectively, for OBI-822-13003-DP (sample 3); and 8 and 10 conjugated HNPQR sites in KLH1 and KLH2, respectively, for OBI-822-13004-DS (sample 4).

Example 9 Materials and Methods:

The abbreviations were listed as follows:
K=Lysine; H=Histidine; N=Asparagine; P=Proline; Q=Glutamine; R=Arginine; LC-MS/MS=Liquid Chromatography-Tandem Mass Spectrometry; MW=Molecular weight.

The database search was performed with Mascot version 2.4.1 against the KLH1 sequence (SEQ ID NO. 1) and KLH2 sequence (SEQ ID NO. 2). The parameters used were as follow:

Enzyme: Trypsin, Glu-C, Chymotrypsin and Thermolysin according to the digestion method;

Fixed modification: Carbamidomethyl (C);

Variable modifications: Oxidation(M), Deamidation (NQ);

1. OBI822_GMd (HKNPQR), GMd_NL997 (HKNPQR);

2. OBI822_Md (HKNPQR), Md_deamidated (HKNPQR);

Peptide Mass Tolerance: ± 10 ppm; Fragment Mass Tolerance: ± 0.05 Da; Max Missed Cleavages: 5; Instrument type: ESI-TRAP; Ion cut-off score: 13.

"OBI822_GMd" in the search parameters indicates the GMd-conjugated residues (HKNPQR) with the MW addition of 1393.5317 Da.

"GMd_NL997" in the search parameters indicates the neutral loss form of GMd-conjugated residues (HKNPQR) with the MW addition of 396.1831 Da.

"OBI822_Md" in the search parameters indicates the Md-conjugated residues (HKNPQR) with the MW addition of 352.1569 Da.

"Md_ deamidated" in the search parameters indicates the deamidated form of Md-conjugated residues (HKNPQR) with the MW addition of 338.1300 Da.

Example 9 Results:

LC-MS/MS-based techniques have emerged as an important tool for the identification of proteins and characterization of amino acid modifications. Detailed information regarding peptide sequences and modification sites could be obtained by the assignment of fragment ions provided by the MS/MS spectra. Mascot was currently the most popular search engine, with a well-accepted probability-based scoring algorithm. To identify different modifications on Lysine (K) and other specific residues (HNPQR) in samples 1-4, all of the search results underwent subsequent manual checked to screen for only those peptides with high quality MS/MS spectra.

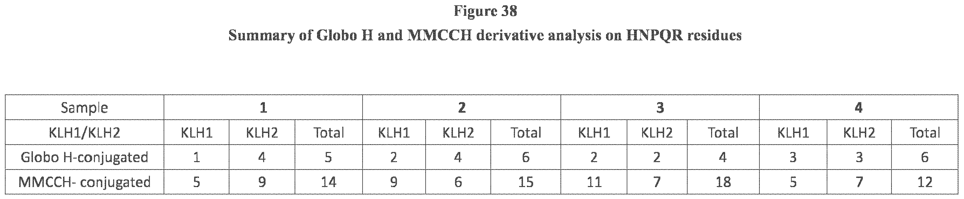

During the MASCOT search for the GMd (Globo H + MMCCH linker + lysine derivative) and Md (MMCCH linker + lysine derivative) modifications on HNPQR residues, there were false peptides identified. If the assigned modification site was close to a lysine residue, it was likely that the lysine is the actual modification site. These peptides were excluded from the identification lists. If there was no nearby lysine residue can be observed, the modification on HNPQR can be confirmed. As shown in Figure 38, GMd modification site analysis yielded 1, 2, 2 and 3 conjugated HNPQR sites in KLH1 for samples 1-4, respectively and 4, 4, 2 and 3 conjugated HNPQR sites in KLH2 for samples 1-4, respectively. Md modification site analysis yields 5, 9, 11 and 5 conjugated HNPQR sites in KLH1 for samples 1-4, respectively and 9, 6, 7 and 7 conjugated HNPQR sites in KLH2 for samples 1-4, respectively.

Example 9 Conclusion:

Among these four samples, the GMd and Md modifications analysis on specific residues (HNPQR) suggests that there were 6, 11, 13 and 8 conjugated HNPQR sites in KLH1 for samples 1-4, respectively, and 13, 10, 9 and 10 conjugated HNPQR sites in KLH2 for samples 1-4, respectively.

Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein.

The discussion of those references cited herein is intended merely to summarize the assertions made by their authors. No admission is made that any reference (or a portion of any reference) is relevant prior art. Applicants reserve the right to challenge the accuracy and pertinence of any cited reference.

## Claims

1. A composition comprising a plurality of Globo H moieties covalently linked to one to twenty keyhole limpet hemocyanin (KLH) moiety(ies), wherein the Globo H moieties are covalently bound to the KLH moieties on one or more amino acid residues; and an α-galactosyl-ceramide (α-GalCer) adjuvant; wherein Globo H moiety covalently linked to a KLH moiety subunit is linked by a 4-(4-N-maleimidomethyl) cyclohexane-1-carboxyl hydrazide (MMCCH) linkage; and wherein the composition has an epitope/carrier ratio ranging from about 800 to about 3200.

2. The composition of claim 1, wherein the amino acid residues are basic, neutral, hydrophobic amino acid residues and/or a combination thereof.

3. The composition of claim 1 or 2, wherein the amino acid residues are lysine, arginine, histidine, asparagine, proline, glutamine and/or a combination thereof.

4. The composition of any of claims 1 to 3, wherein the KLH moiety is a monomer, dimer, trimer, tetramer, pentamer, hexamer, heptamer, octamer, nonamer, decamer, undecamer, dodecamer, tridecamer, tetradecamer, pentadecamer, hexadecamer, heptadecamer, octadecamer, nonadecamer, or didecamer.

5. The composition of any of claims 1 to 4, wherein the Globo H moiety comprises (Fucα1 →2 Ga1β1→3 GalNAcβ1→3 Ga1α1→4 Galβ1→4 Glc).

6. The composition of any of claims 1 to 5, wherein the KLH moiety subunit is at least 80% identical to SEQ ID NO. 1 or SEQ ID NO. 2.

7. A composition of any of the preceding claims for use in treating cancer in a patient.

8. A composition for use according to claim 7, wherein the cancer is breast cancer, lung cancer, liver cancer, buccal cancer, stomach cancer, colon cancer, nasopharyngeal cancer, dermal cancer, renal cancer, brain tumor, prostate cancer, ovarian cancer, cervical cancer, endometrial cancer, intestinal cancer, pancreatic cancer or bladder cancer

9. The composition for use according to claim 8, wherein the cancer is a Globo H expressing cancer.

10. The composition of claim 1, wherein the α-GalCer adjuvant has the following structure:

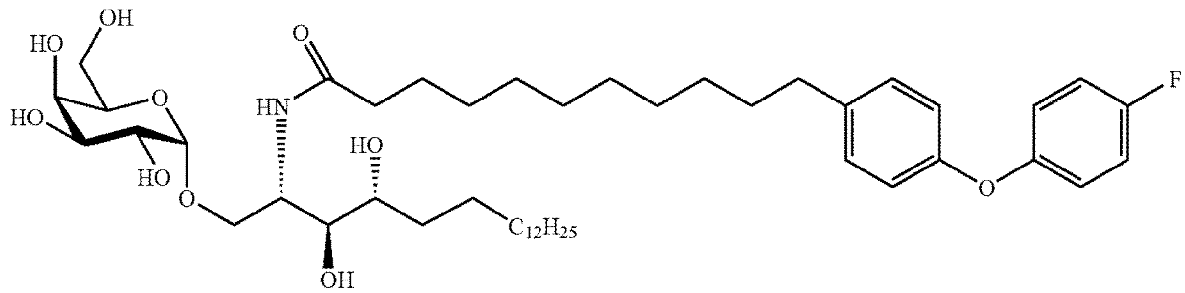

11. The composition of claim 1, wherein the KLH moieties can form a monomer, dimer, trimer, tetramer, pentamer, hexamer, heptamer, octamer, nonamer, decamer, undecamer, dodecamer, tridecamer, tetradecamer, pentadecamer, hexadecamer, heptadecamer, octadecamer, nonadecamer or didecamer.

12. The composition of any of the preceding claims for use in a method of inducing antibodies in a subject comprising administering to the subject an effective amount of the composition of any of the preceding claims.

## Patentansprüche

1. Zusammensetzungen, die eine Vielzahl von Globo-H-Anteilen, die kovalent mit einem bis zwanzig Keyhole-Limpet-Hämocyanin-(KLH-)Anteil(en) verbunden sind, wobei die Globo-H-Anteile kovalent an die KLH-Anteile an einem oder mehreren Aminosäureresten gebunden sind; und ein α-Galactosylceramid- (α-GalCer-) Adjuvans umfasst; wobei ein Globo-H-Anteil, der kovalent mit einer KLH-Anteil-Untereinheit verbunden ist, durch eine 4-(4-N-Maleimidomethyl)-cyclohexan-l-carboxylhydrazid- (MMCCH-) Verbindung verbunden ist; und wobei die Zusammensetzung ein Epitop/Träger-Verhältnis im Bereich von etwa 800 bis etwa 3200 aufweist.

2. Zusammensetzung nach Anspruch 1, wobei die Aminosäurereste basische, neutrale, hydrophobe Aminosäurereste und/oder eine Kombination davon sind.

3. Zusammensetzung nach Anspruch 1 oder 2, wobei die Aminosäurereste Lysin, Arginin, Histidin, Asparagin, Prolin, Glutamin und/oder eine Kombination davon sind.

4. Zusammensetzung nach einem der Ansprüche 1 oder 3, wobei der KLH-Anteil ein Monomer, Dimer, Trimer, Tetramer, Pentamer, Hexamer, Heptamer, Octamer, Nonamer, Decamer, Undecamer, Dodecamer, Tridecamer, Tetradecamer, Pentadecamer, Hexadecamer, Heptadecamer, Octadecamer, Nonadecamer oder Didecamer ist.

**5.** Zusammensetzung nach einem der Ansprüche 1 bis 4, wobei der Globo-H-Anteil (Fucal ->2 Galβ1-->3 GalNAcβ1->3 Galα1α->4 Galβ1->4 Glc) umfasst.

**6.** Zusammensetzung nach einem der Ansprüche 1 bis 5, wobei die KLH-Anteil-Untereinheit mindestes 80 % identisch mit SEQ ID NR 1 oder SEQ ID NR 2 ist.

**7.** Zusammensetzung nach einem der vorhergehenden Ansprüche zur Verwendung bei der Behandlung von Krebs in einem Patienten.

**8.** Zusammensetzung zur Verwendung nach Anspruch 7, wobei der Krebs Brustkrebs, Lungenkrebs, Leberkrebs, Mundkrebs, Magenkrebs, Dickdarmkrebs, Nasenrachenkrebs, Hautkrebs, Nierenkrebs, Gehirntumor, Prostatakrebs, Eierstockkrebs, Gebärmutterhalskrebs, Endometriumkrebs, Darmkrebs, Bauchspeicherdrüsenkrebs oder Blasenkrebs ist.

**9.** Zusammensetzung zur Verwendung nach Anspruch 8, wobei der Krebs ein Globo-H ausgebender Krebs ist.

**10.** Zusammensetzung nach Anspruch 1, wobei das α-GalCer-Adjuvans die folgende Struktur aufweist:

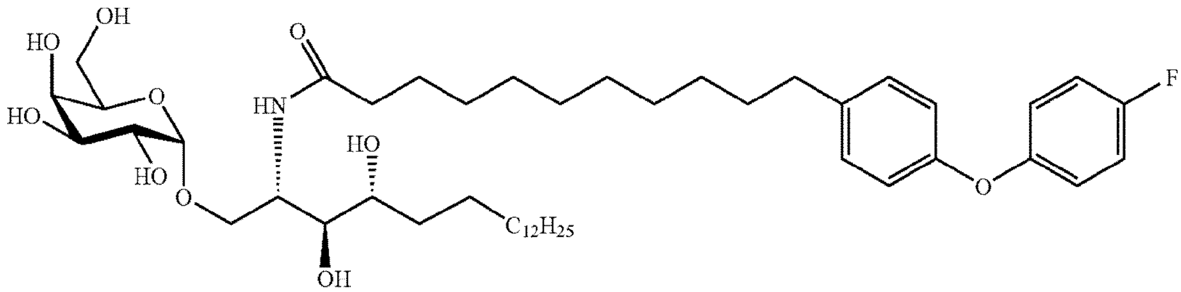

.

**11.** Zusammensetzung nach Anspruch 1, wobei die KLH-Anteile ein Monomer, Dimer, Trimer, Tetramer, Pentamer, Hexamer, Heptamer, Octamer, Nonamer, Decamer, Undecamer, Dodecamer, Tridecamer, Tetradecamer, Pentadecamer, Hexadecamer, Heptadecamer, Octadecamer, Nonadecamer oder Didecamer bilden können.

**12.** Zusammensetzung nach einem der vorhergehenden Ansprüche zur Verwendung in einem Verfahren zum Einführen von Antibiotika in ein Subjekt, das ein Verabreichen einer wirksamen Menge der Zusammensetzung nach einem der vorhergehenden Ansprüche an das Subjekt umfasst.

## Revendications

**1.** Composition comprenant une pluralité de fragments Globo H liés par covalence à un à vingt fragments d'hémocyanine de patelle (KLH), dans laquelle les fragments Globo H sont liés par covalence aux fragments de KLH sur un ou plusieurs résidus d'acides aminés ; et un adjuvant *α*-galactosyl-céramide (a-GalCer) ; dans laquelle le fragment Globo H lié par covalence à une sous-unité de fragment de KLH est lié par une liaison 4-(4-N-maléimidométhyl) cyclohexane-1-carboxyl hydrazide (MMCCH) ; et dans laquelle la composition a un rapport épitope/support allant d'environ 800 à environ 3 200.

**2.** Composition selon la revendication 1, dans laquelle les résidus d'acides aminés sont des résidus d'acides aminés basiques, neutres, hydrophobes et/ou une combinaison de ceux-ci.

**3.** Composition selon la revendication 1 ou 2, dans laquelle les résidus d'acides aminés sont la lysine, l'arginine, l'histidine, l'asparagine, la proline, la glutamine et/ou une combinaison de ceux-ci.

**4.** Composition selon l'une quelconque des revendications 1 à 3, dans laquelle le fragment KLH est un monomère, un dimère, un trimère, un tétramère, un pentamère, un hexamère, un heptamère, un octamère, un nonamère, un décamère, un undécamère, un dodécamère, un tridécamère, un tétradécamère, un pentadécamère, un hexadécamère, un heptadécamère, un octadécamère, un nonadécamère, ou un didécamère.

**5.** Composition selon l'une quelconque des revendications 1 à 4, dans laquelle le fragment Globo H comprend (Fucα1 ->2 Galβ1->3 GalNAcβ1->3 Galα1->4 Galβ1->4 Glc).

**6.** Composition selon l'une quelconque des revendications 1 à 5, dans laquelle la sous-unité de fragment KLH est identique à au moins 80 % à la SEQ ID NO. 1 ou la SEQ ID NO. 2.

**7.** Composition selon l'une quelconque des revendications précédentes destinée à être utilisée dans le traitement du cancer chez un patient.

**8.** Composition destinée à être utilisée selon la revendication 7, dans laquelle le cancer est le cancer du sein, le cancer du poumon, le cancer du foie, le cancer de la bouche, le cancer de l'estomac, le cancer du côlon, le cancer nasopharyngéal, le cancer de la peau, le cancer du rein, le cancer du cerveau, le cancer de la prostate, le cancer de l'ovaire, le cancer du col de l'utérus, le cancer de l'endomètre, le cancer de l'intestin, le cancer du pancréas ou le cancer de la vessie.

**9.** Composition destinée à être utilisée selon la revendication 8, dans laquelle le cancer est un cancer exprimant Globo H.

**10.** Composition selon la revendication 1, dans laquelle l'adjuvant α-GalCer a la structure suivante :

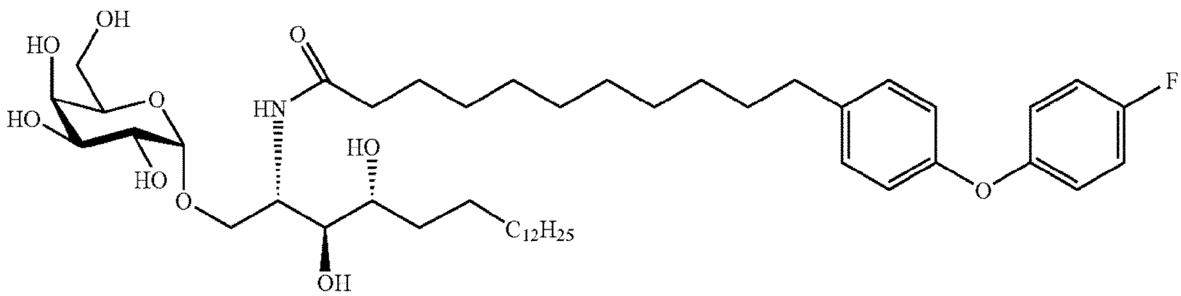

.

**11.** Composition selon la revendication 1, dans laquelle les fragments KLH peuvent former un monomère, un dimère, un trimère, un tétramère, un pentamère, un hexamère, un heptamère, un octamère, un nonamère, un décamère, un undécamère, un dodécamère, un tridécamère, un tétradécamère, un pentadécamère, un hexadécamère, un heptadécamère, un octadécamère, un nonadécamère, ou un didécamère.

**12.** Composition selon l'une quelconque des revendications précédentes destinée à être utilisée dans un procédé d'induction des anticorps chez un sujet comprenant l'administration au sujet d'une quantité efficace de la composition selon l'une quelconque des revendications précédentes.

Fig. 1(A)

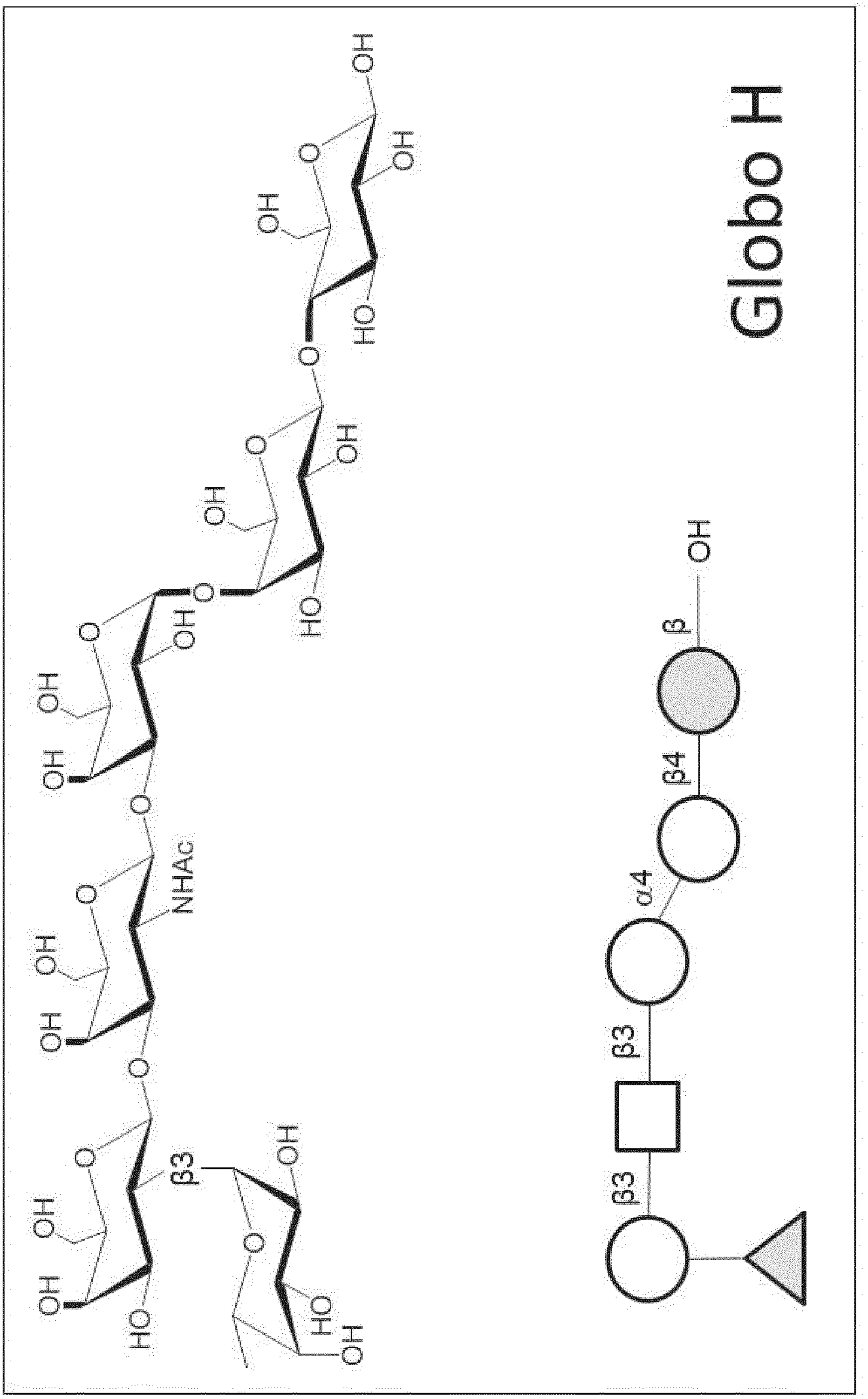

Globo H

Fig. 1(A) continued

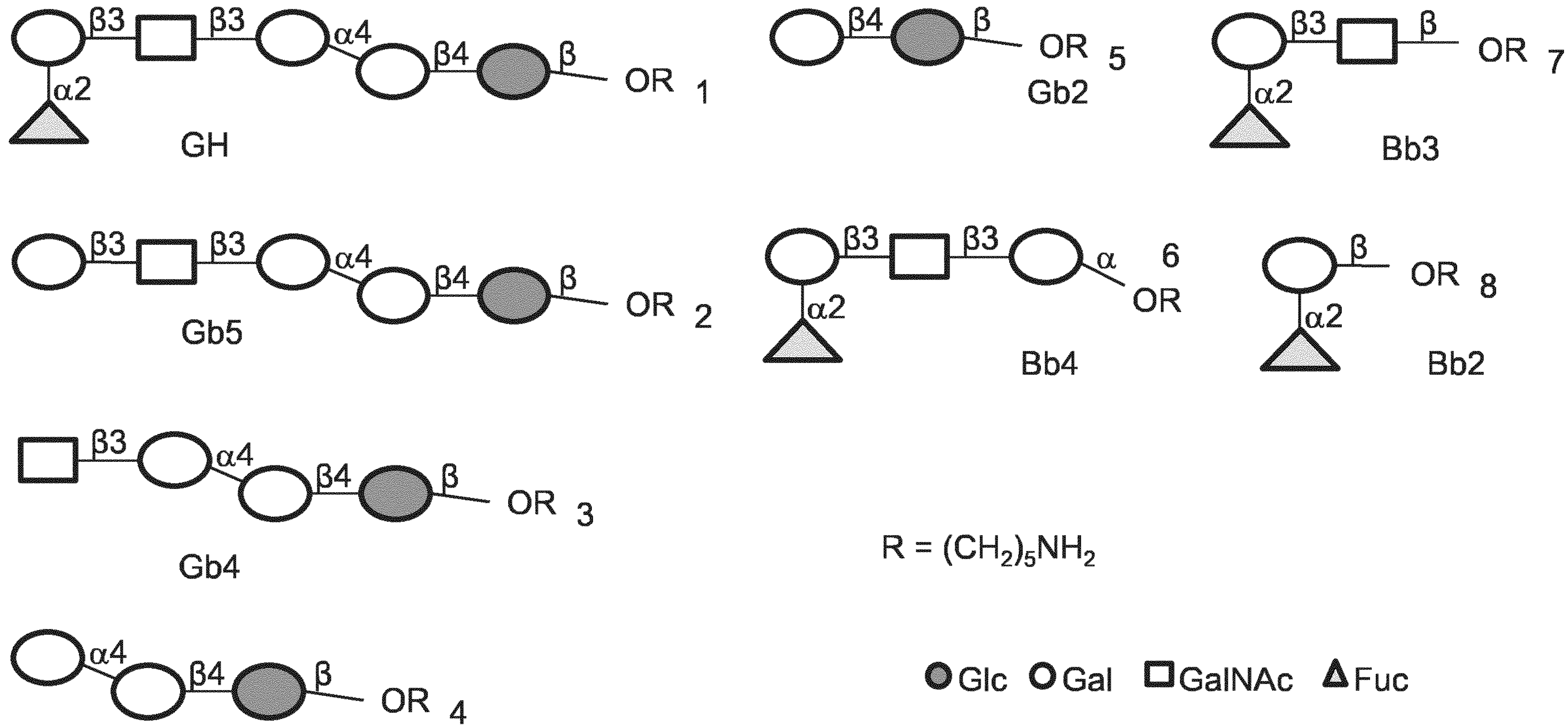


R = $(CH_2)_5NH_2$

Fig. 1(B)

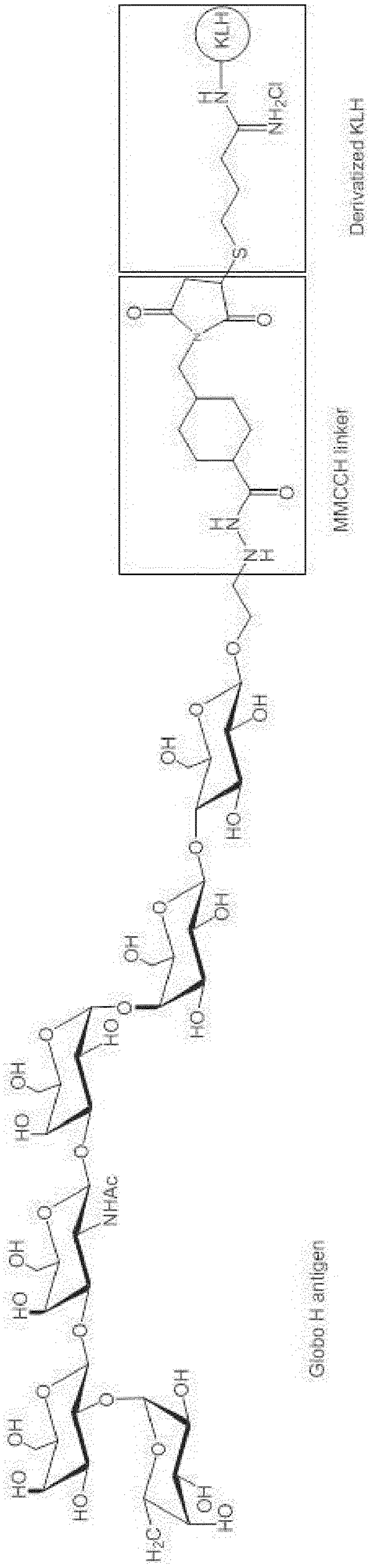

Fig. 2(A)

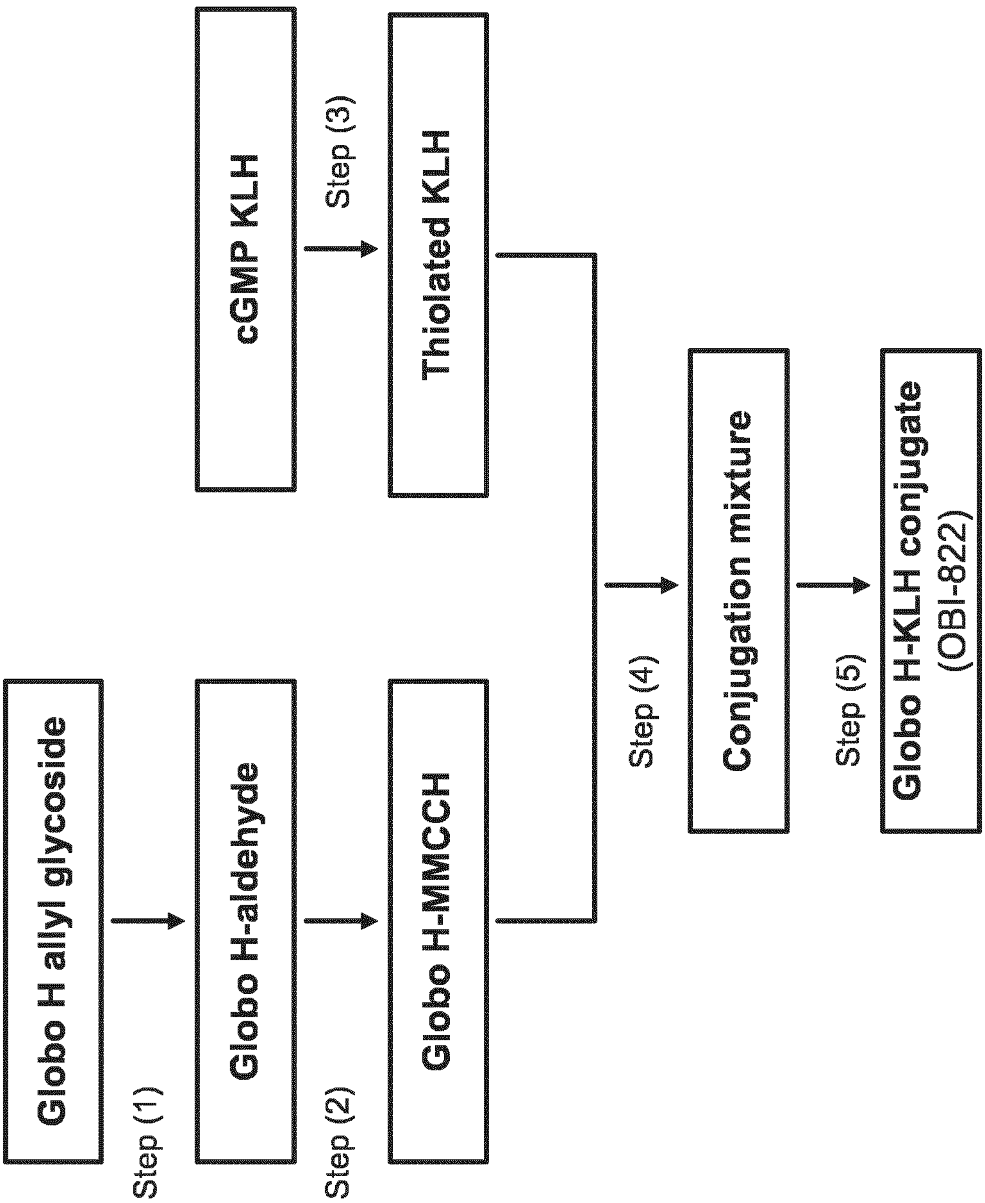
Globo H allyl glycoside
Step (1)
Globo H-aldehyde
Step (2)
Globo H-MMCCH
cGMP KLH
Step (3)
Thiolated KLH
Step (4)
Conjugation mixture
Step (5)
Globo H-KLH conjugate
(OBI-822)

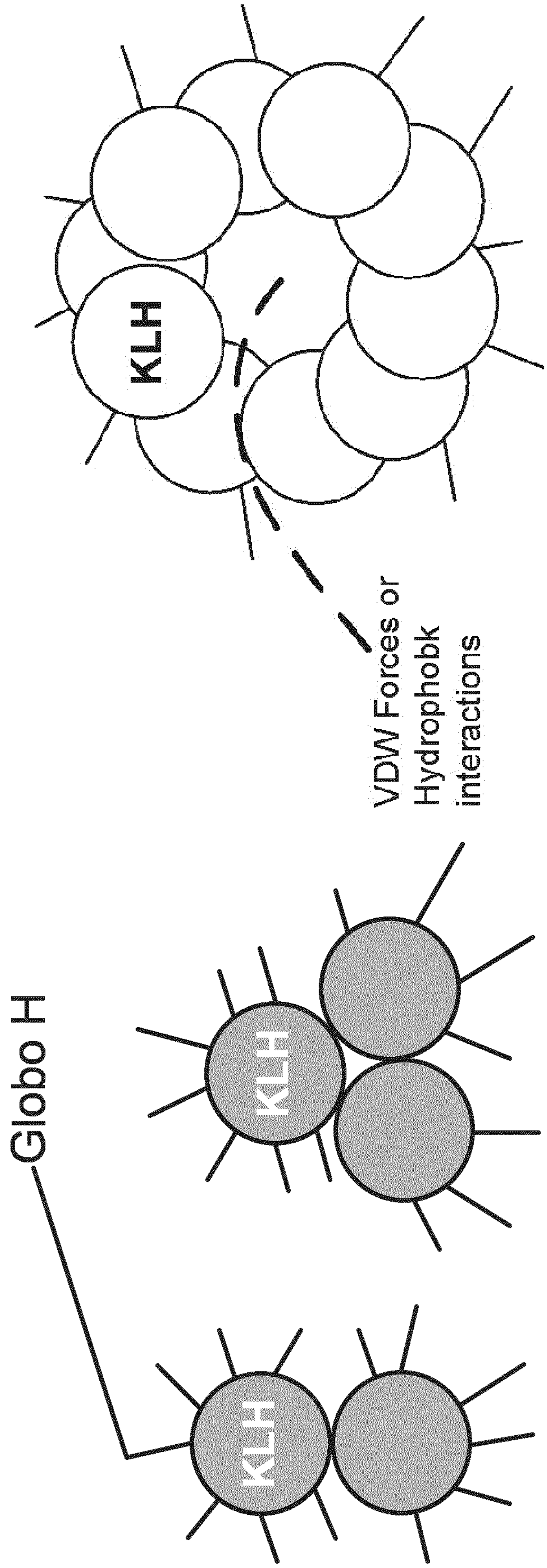


Fig. 2(B)

Fig. 3

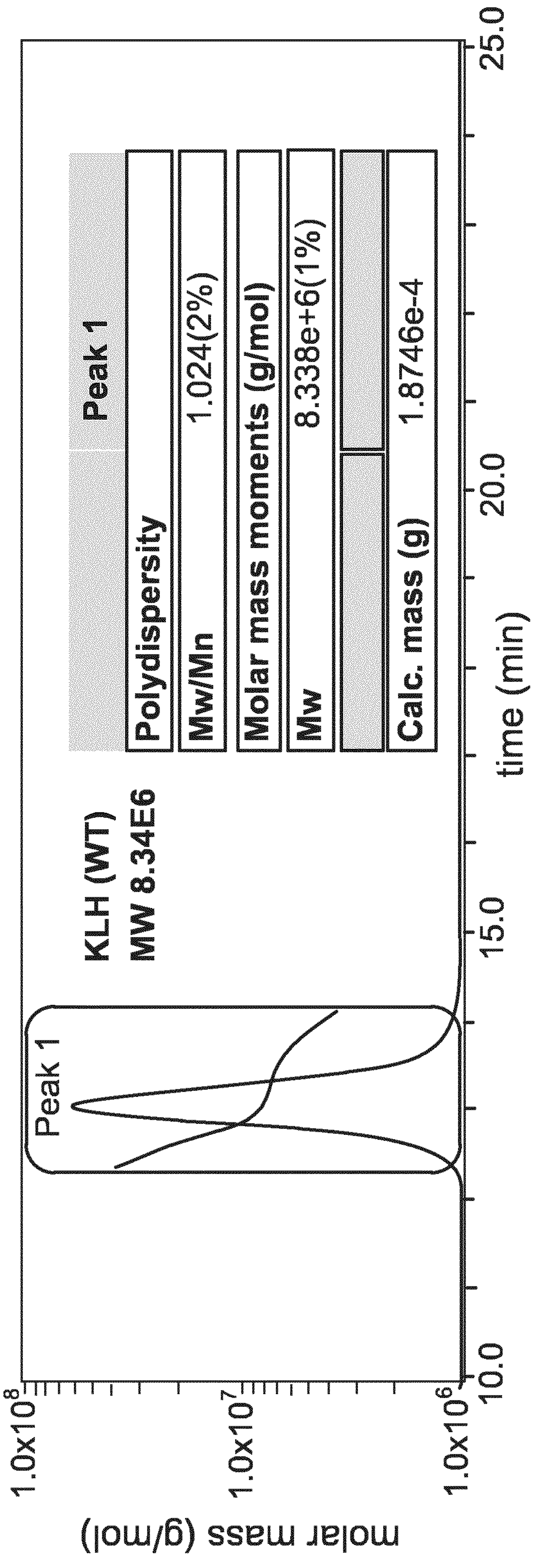
KLH (WT)
MW 8.34E6
Peak 1
Polydispersity
Mw/Mn 1.024(2%)
Molar mass moments (g/mol)
Mw 8.338e+6(1%)
Calc. mass (g) 1.8746e-4
Peak 1
molar mass (g/mol)
1.0x10^8
1.0x10^7
1.0x10^6
10.0
15.0
20.0
25.0
time (min)

Figure 4

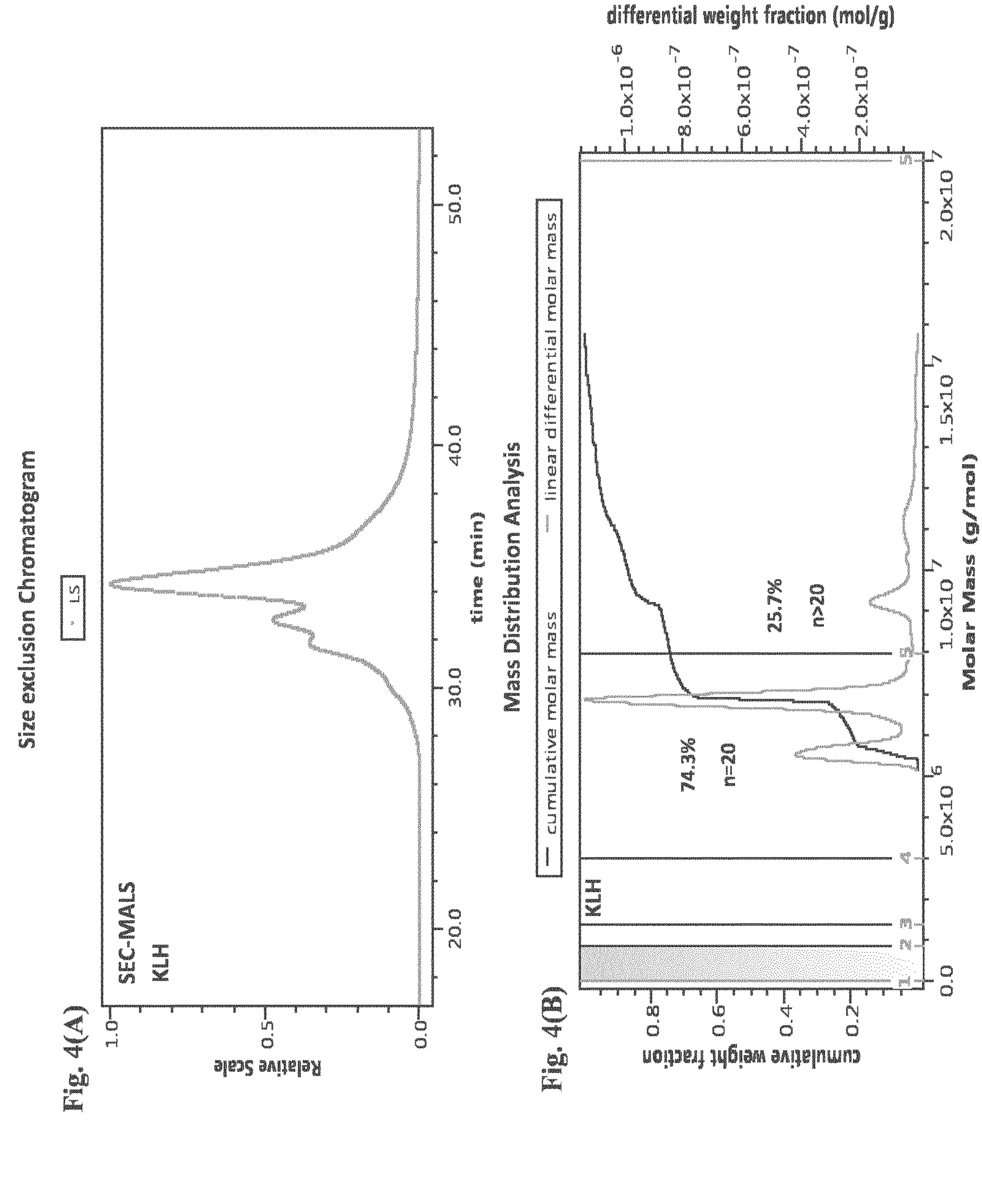


Fig. 4(A)

Fig. 4(B)

Figure 4 (continued)

Fig. 4(C)

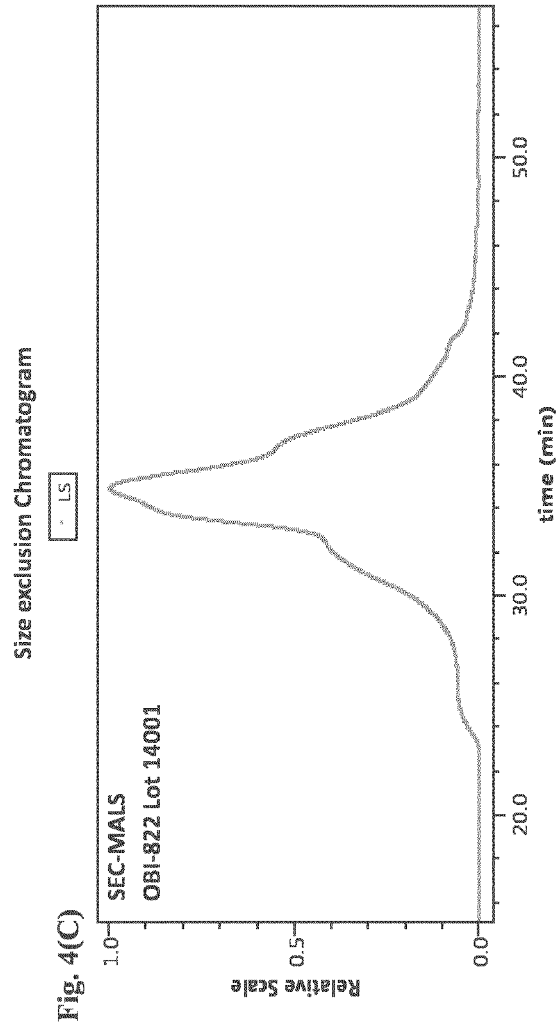


Fig. 4(D)

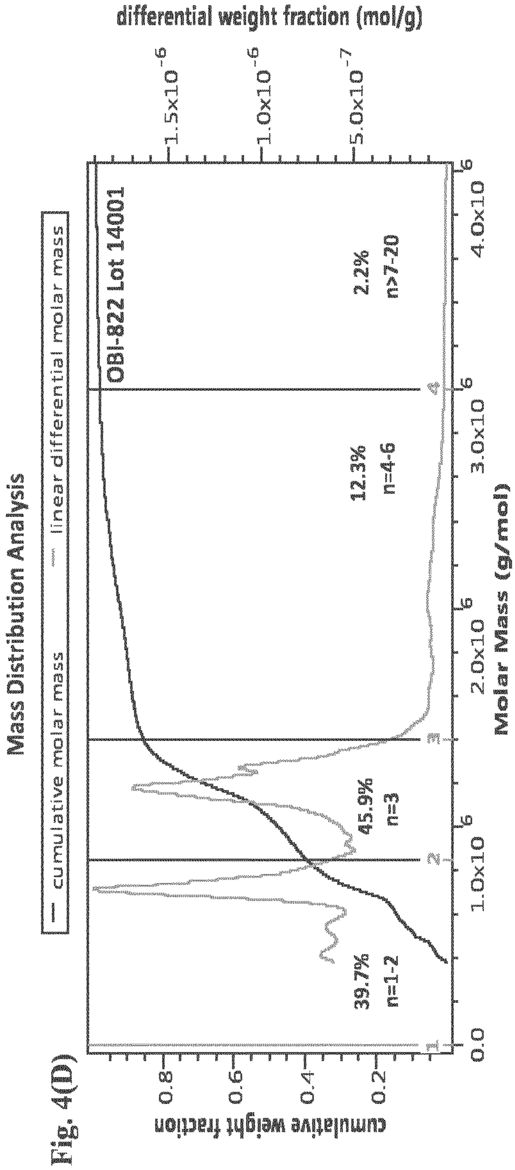

Figure 5

Fig. 5(A)

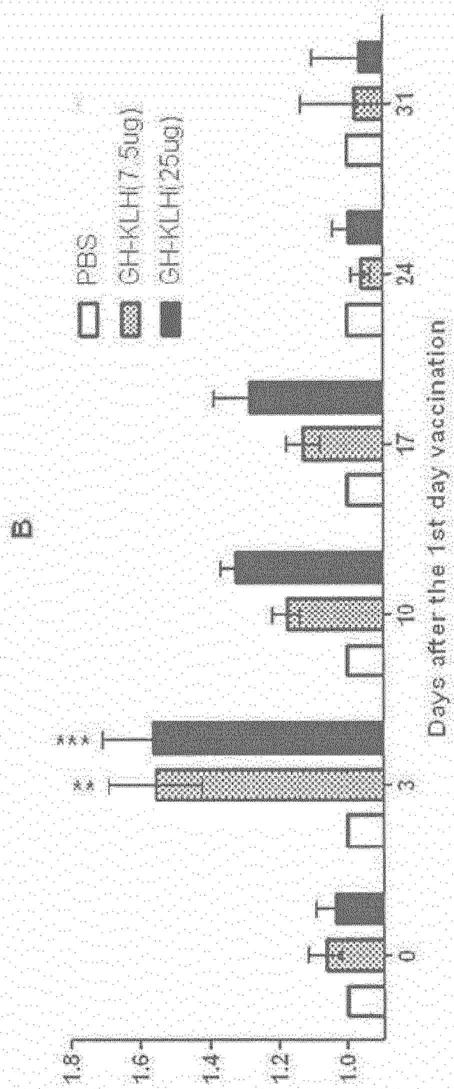

Figure 5 continued

Fig. 5(B)

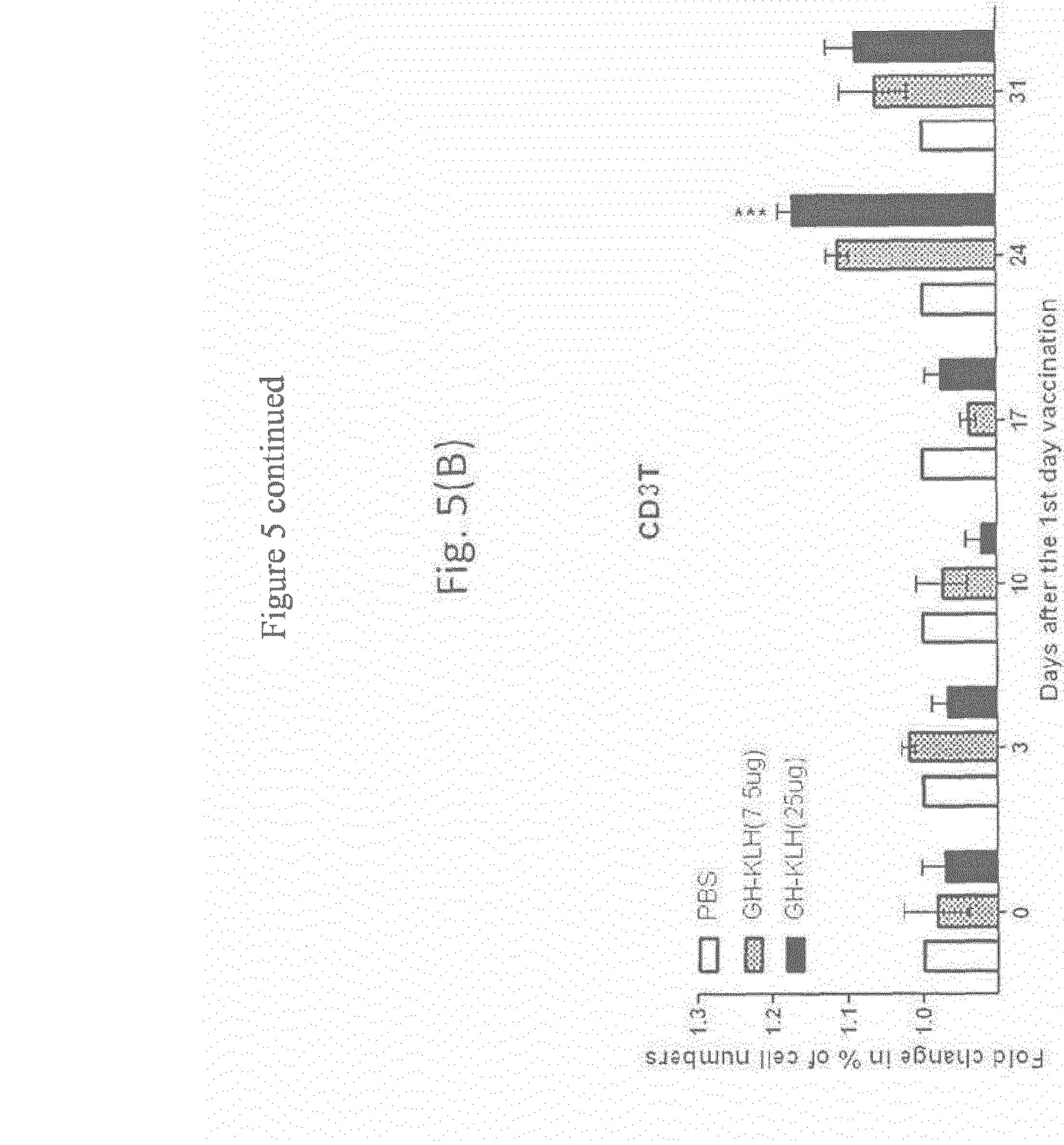

Figure 5 continued

Fig. 5(C)

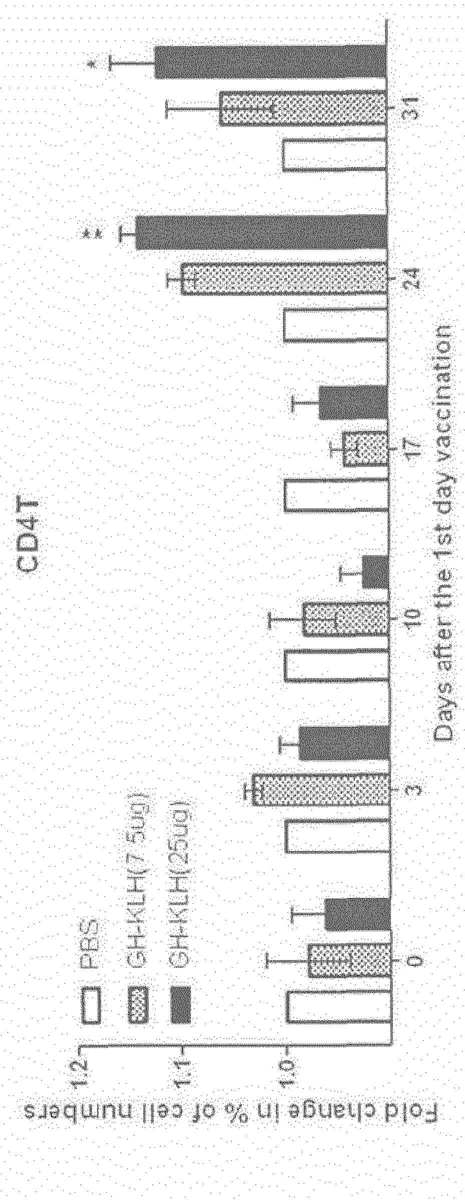

Figure 5 continued

Fig. 5(D)

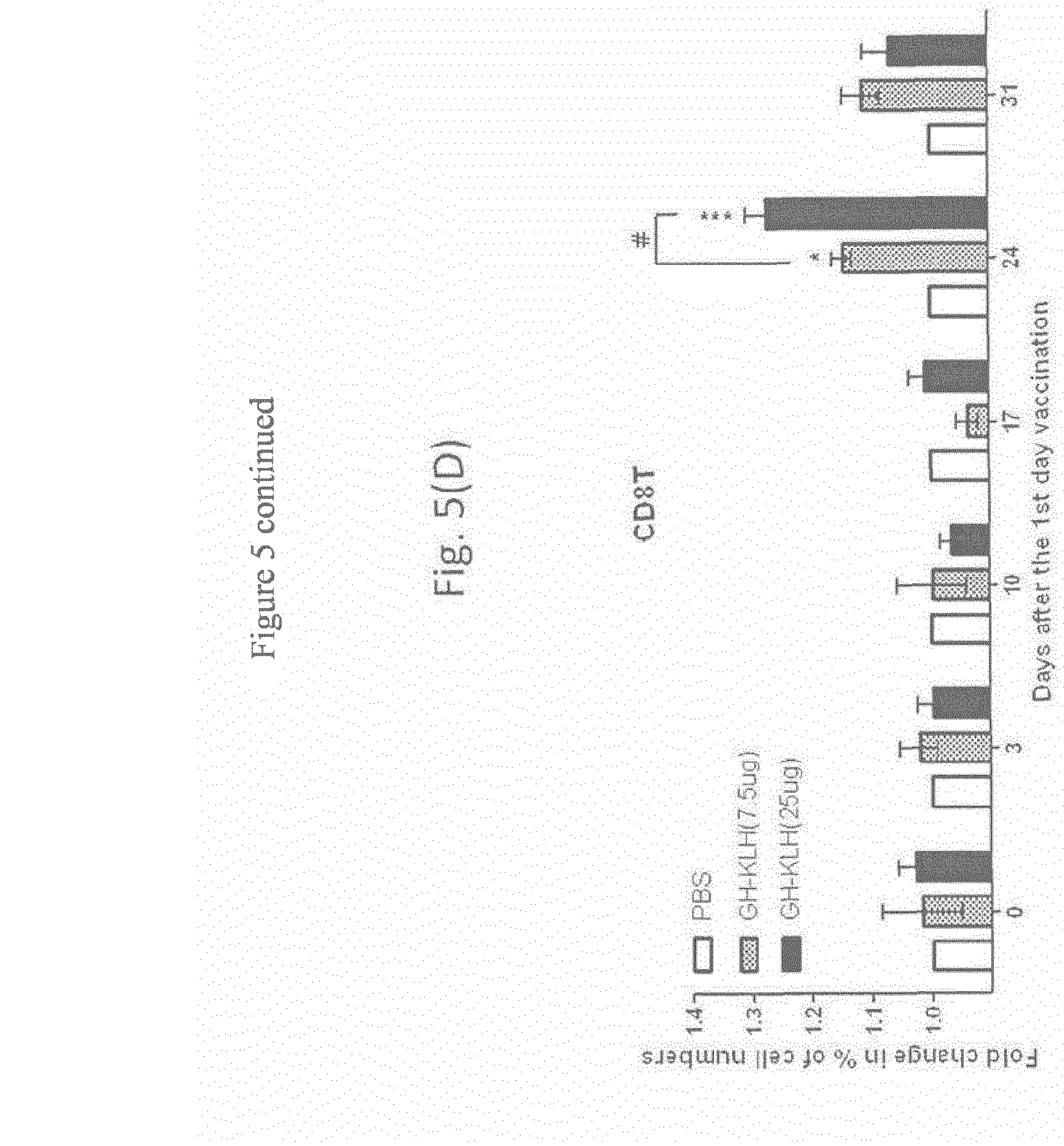

Figure 6

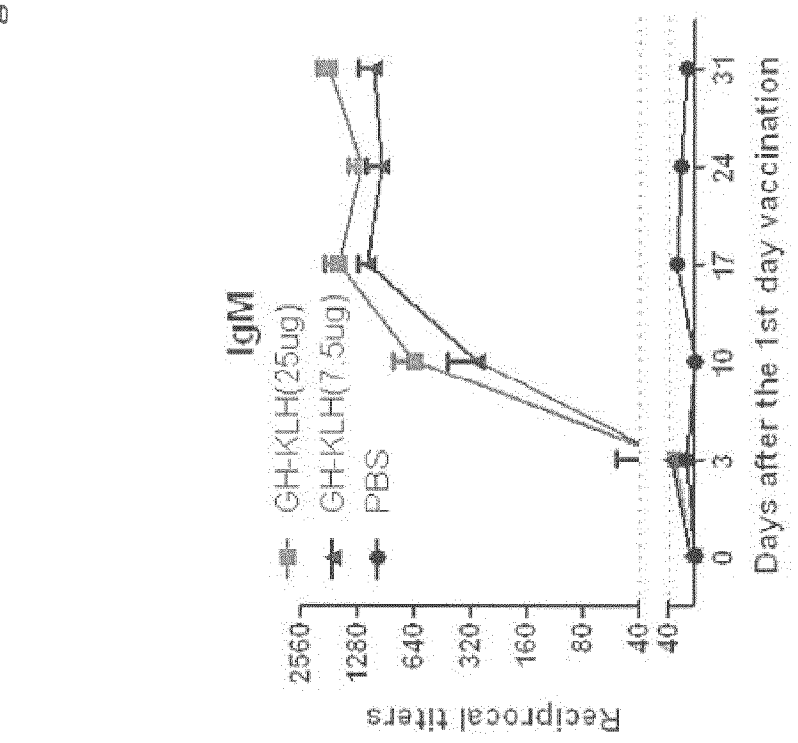


Figure 6A

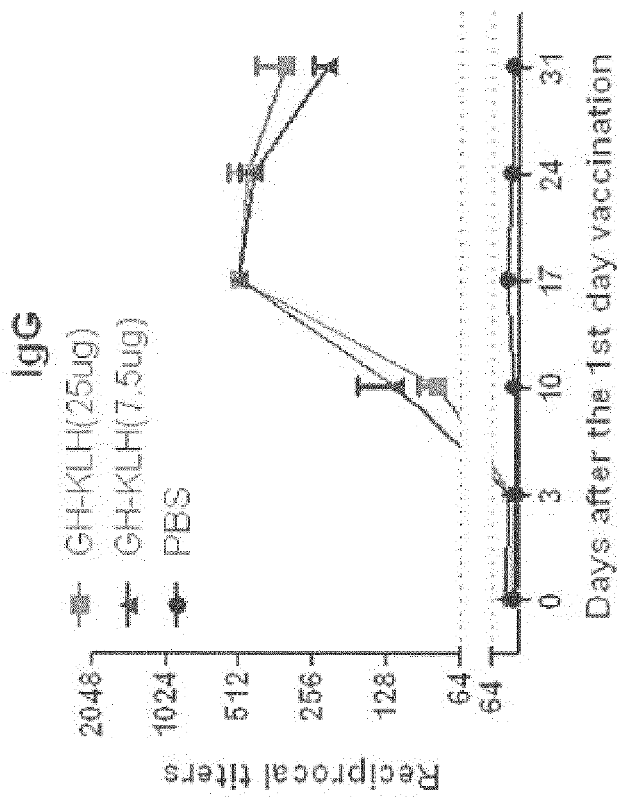


Figure 6B

Figure 7

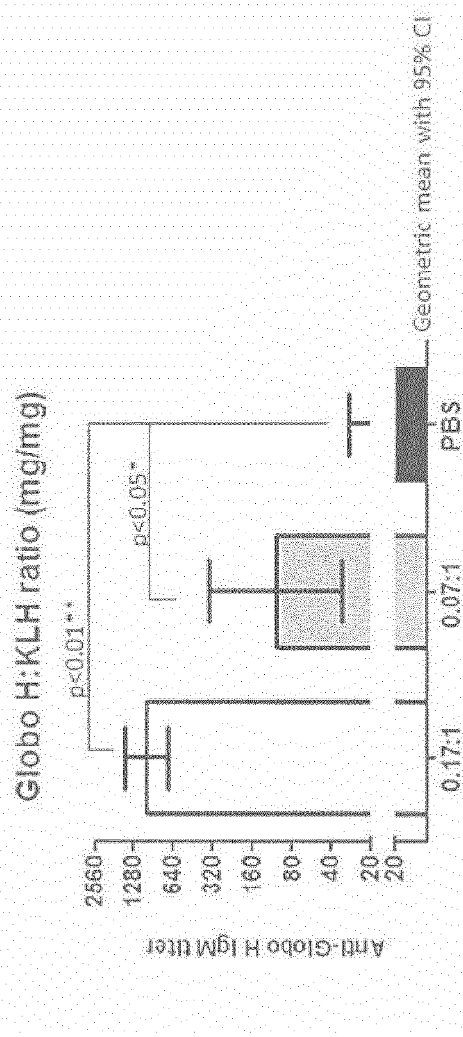
Globo H:KLH ratio (mg/mg)
Anti-Globo H IgM titer
2560
1280
640
320
160
80
40
20
20
p<0.01**
p<0.05*
0.17:1
0.07:1
PBS
Geometric mean with 95% CI

Figure 8

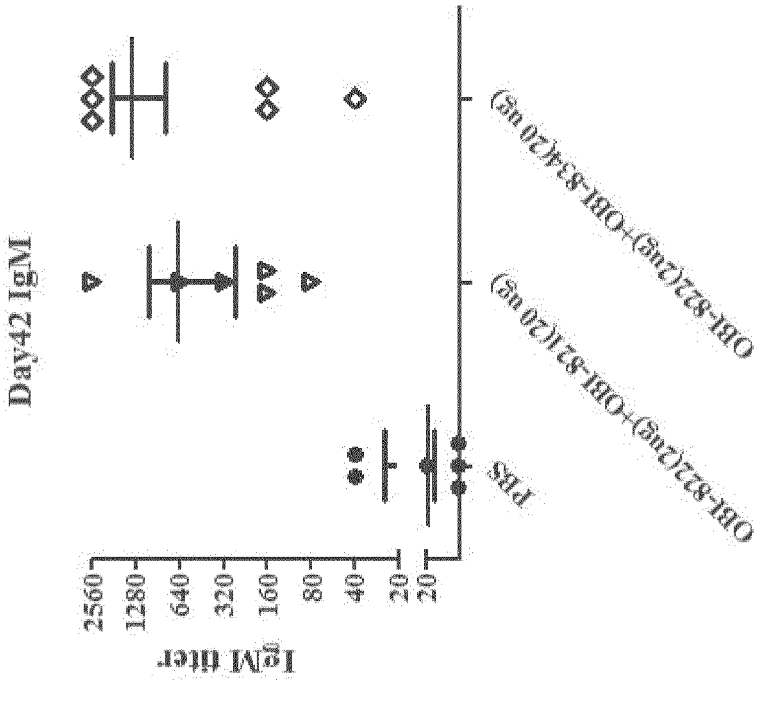


Figure 8A

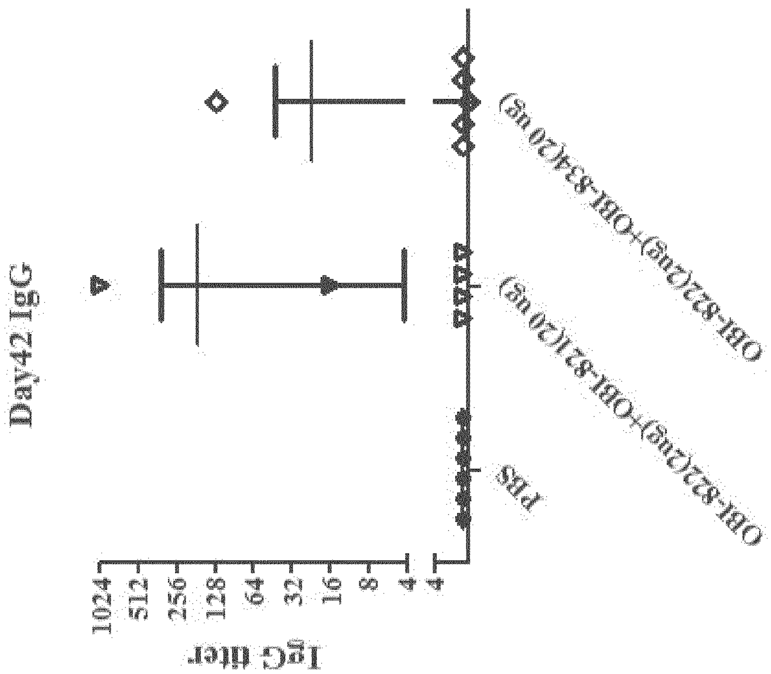


Figure 8B

# Figure 9

## Fig. 9(A)

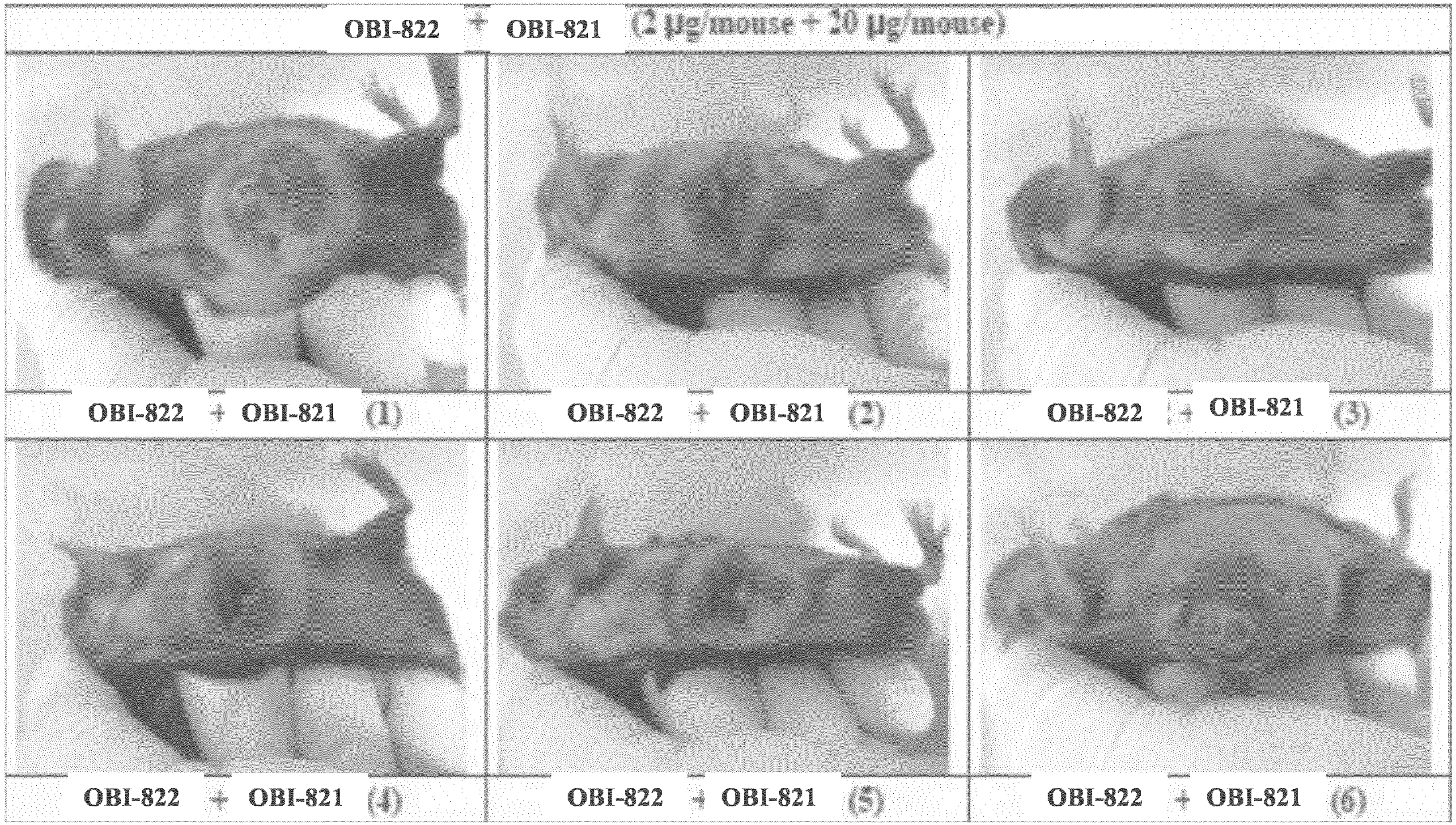

# Figure 9 (continued)

## Figure 9(B)

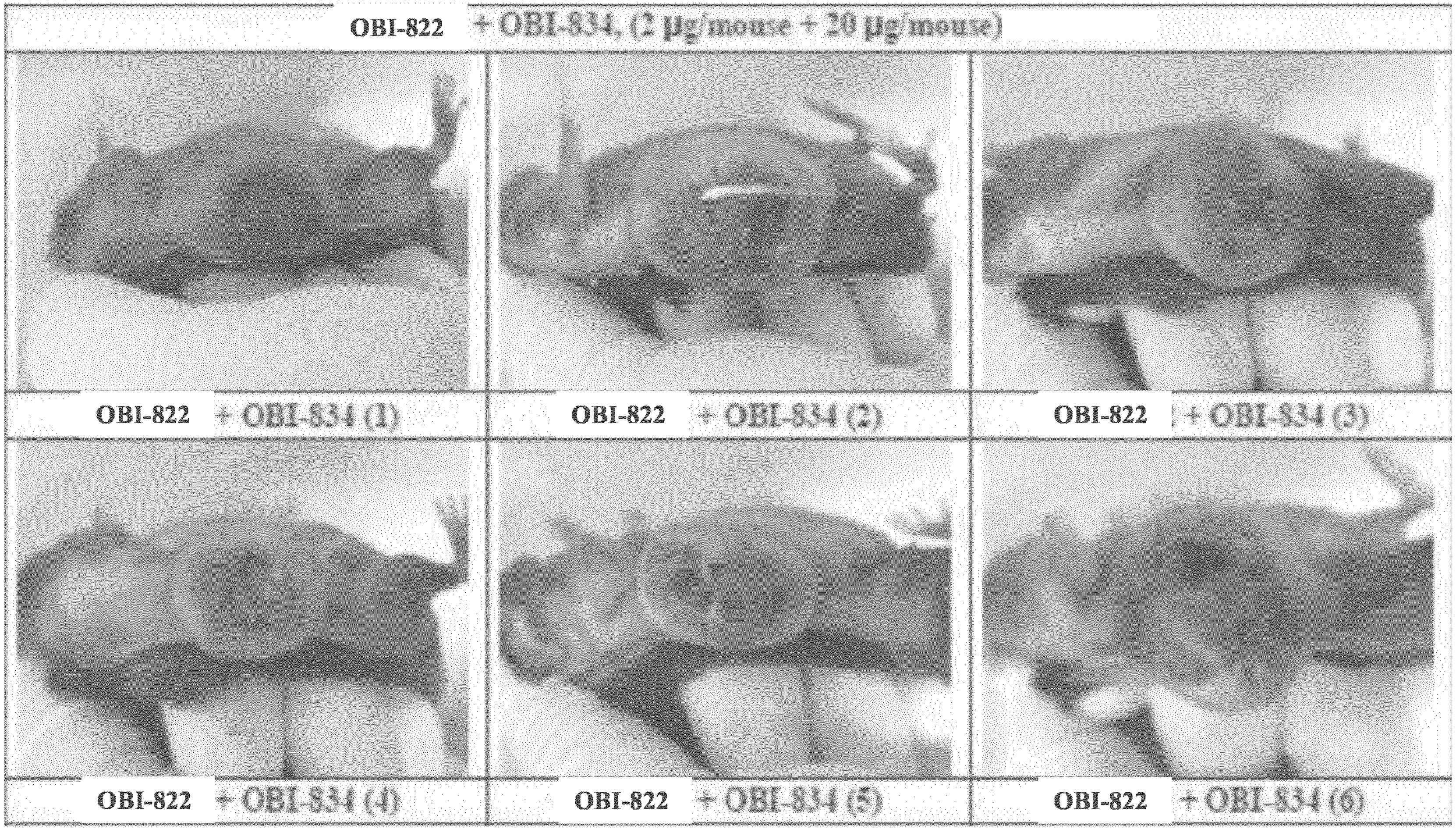

Figure 10

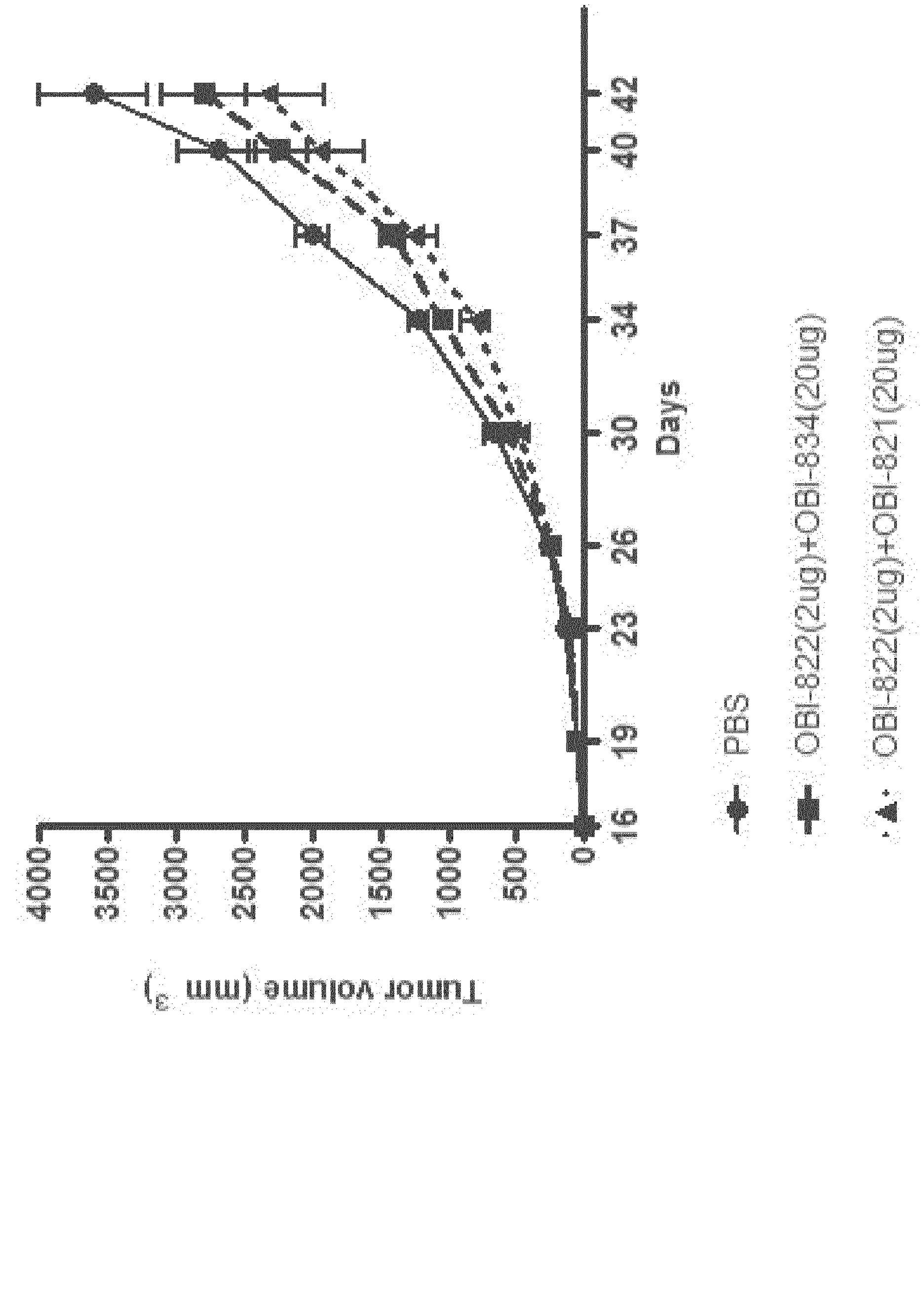
Tumor volume (mm ³)
4000
3500
3000
2500
2000
1500
1000
500
0
16
19
23
26
30
34
37
40
42
Days
PBS
OBI-822(2ug)+OBI-834(20ug)
OBI-822(2ug)+OBI-821(20ug)

# Figure 11

## Figure 11A. Globo H derivative

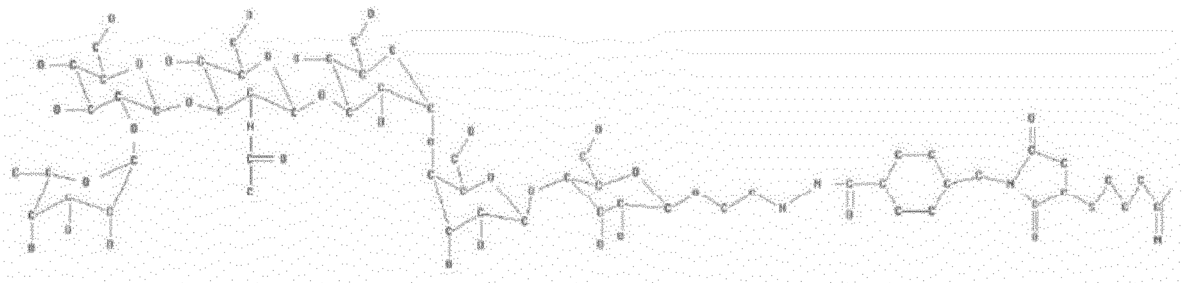

Chemical formula: C(56) H(91) N(5) O(33) S(1)
Monoisotopic MW addition: 1393.5317 Da

## Figure 11B. The neutral loss forms of Globo H derivative

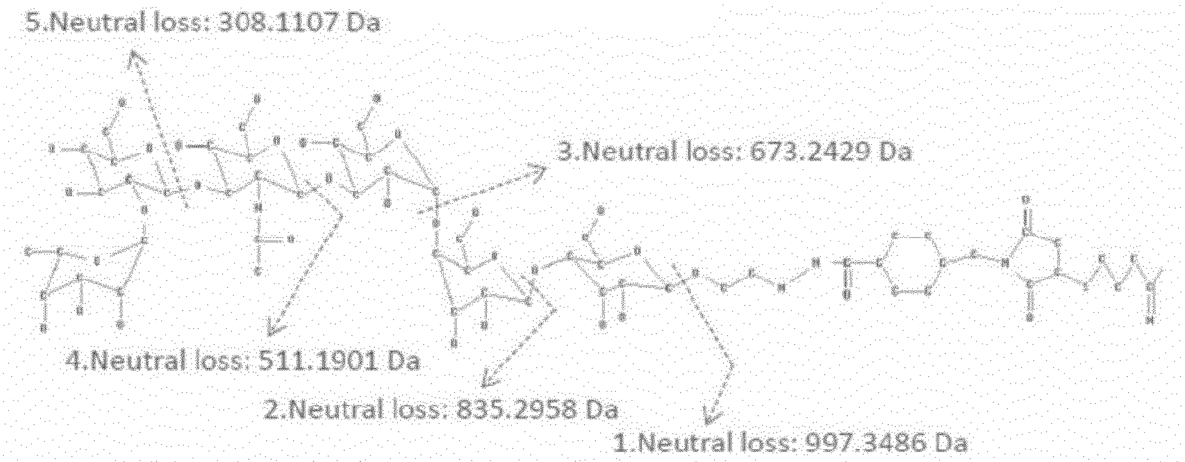


1. Chemical formula: C(18) H(28) N(4) O(4) S(1)
   Monoisotopic MW addition: 396.1831 Da

2. Chemical formula: C(24) H(38) N(4) O(9) S(1)
   Monoisotopic MW addition: 558.2360 Da

3. Chemical formula: C(30) H(48) N(4) O(14) S(1)
   Monoisotopic MW addition: 720.2888 Da

4. Chemical formula: C(36) H(58) N(4) O(19) S(1)
   Monoisotopic MW addition: 882.3416 Da

5. Chemical formula: C(44) H(71) N(5) O(24) S(1)
   Monoisotopic MW addition: 1085.4210 Da

Figure 12

**Figure 12A. MMCCH derivative**

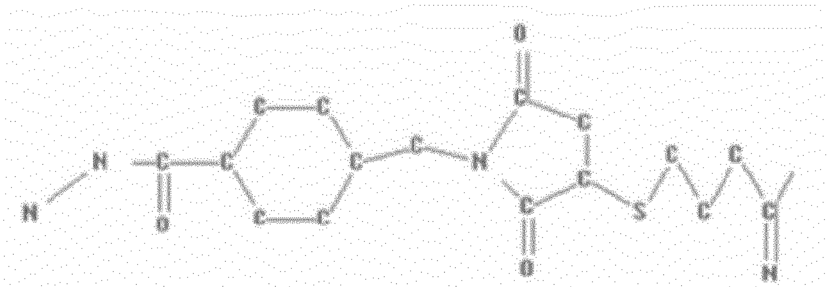

Chemical formula: C(16) H(24) N(4) O(3) S(1)
Monoisotopic MW addition: 352.1569 Da

**Figure 12B. Deamidated MMCCH derivative**

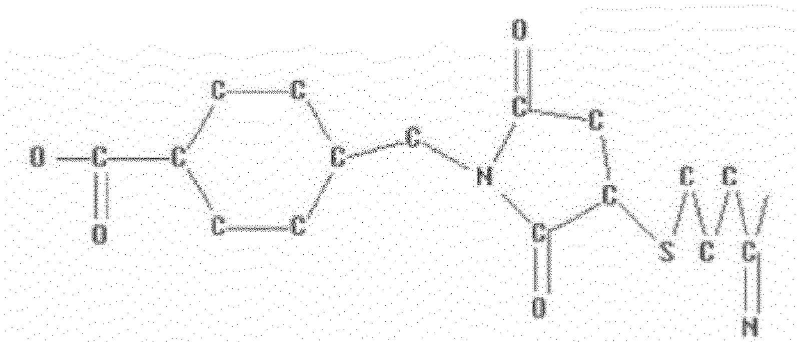

Chemical formula: C(16) H(22) N(2) O(4) S(1)
Monoisotopic MW addition: 338.1300 Da

## Figure 13

### Figure 13A. Globo H derivative analysis

| Sample | 1st LC-MS/MS | 2nd LC-MS/MS |
|---|---|---|
| 1 | Figure 14 | Figure 18 |
| 2 | Figure 15 | Figure 19 |
| 3 | Figure 16 | Figure 20 |
| 4 | Figure 17 | Figure 21 |

### Figure 13B. MMCCH derivative analysis

| Sample | 1st LC-MS/MS | 2nd LC-MS/MS |
|---|---|---|
| 1 | Figure 22 | Figure 26 |
| 2 | Figure 23 | Figure 27 |
| 3 | Figure 24 | Figure 28 |
| 4 | Figure 25 | Figure 29 |

**Figure 14. Identification details of Globo H-conjugated peptides for sample 1 (1st LC-MS/MS)**

**Table 14A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 142 | 149 | 847.4247 | 1692.8349 | 1692.8240 | 6.44 | T | 1 | 17 | 0.02 | KNINDLTR +K_MMCCH_NL673 (K) | 142 |
| 2 | 157 | 166 | 851.9063 | 1701.7981 | 1701.7933 | 2.82 | T | 1 | 14 | 0.039 | EAFHKFQEDR +K_MMCCH_NL997 (K) | 161 |
| 3 | 588 | 606 | 1132.8336 | 3395.4790 | 3395.4862 | -2.09 | T | 1 | 16 | 0.022 | EDKSAGGFQQLGAFHGEPK +Globo_H_MMCCH (K) | 590 |
| 4 | 700 | 719 | 1015.1623 | 3042.4652 | 3042.4532 | 3.94 | T | 1 | 27 | 0.0019 | SVQNKLFEQPEFGHYTSIAK +K_MMCCH_NL673 (K) | 719 |
| 5 | 953 | 965 | 762.7177 | 2285.1311 | 2285.1249 | 2.71 | T | 1 | 52 | 6.30E-06 | DLFKQPSVIHEPR +K_MMCCH_NL673 (K) | 956 |
| 6 | 986 | 1000 | 1036.8300 | 3107.4681 | 3107.4578 | 3.31 | T | 1 | 20 | 0.011 | KNIENLSLGELESLR +Globo_H_MMCCH (K) | 986 |
| 7 | 1092 | 1114 | 806.3826 | 3221.5014 | 3221.5213 | -6.18 | T | 1 | 24 | 0.0038 | QHHYETNPFHHGKITHENEITTR +K_MMCCH_NL997 (K) | 1104 |
| 8 | 1473 | 1489 | 905.7906 | 2714.3499 | 2714.3414 | 3.13 | T | 1 | 25 | 0.0034 | KHGAVVGLPYWDWTLPR +K_MMCCH_NL673 (K) | 1473 |
| 9 | 1923 | 1932 | 793.8990 | 1585.7834 | 1585.7745 | 5.68 | T | 1 | 17 | 0.02 | NKVMPNPFAR +Deamidated (NQ); K_MMCCH_NL997 (K); Oxidation (M) | 1924 |
| 10 | 2359 | 2367 | 846.4022 | 1690.7898 | 1690.7872 | 1.54 | T | 1 | 19 | 0.011 | DKLFNDPER +K_MMCCH_NL835 (K) | 2360 |
| 11 | 2851 | 2883 | 1027.9812 | 4107.8957 | 4107.8666 | 7.08 | T | 0 | 62 | 6.30E-07 | VKPAHAGSCAGDIMHVPLHPFNYESVNNDDFTR +Deamidated (NQ); K_MMCCH_NL997 (K); Oxidation (M) | 2852 |
| 12 | 2897 | 2919 | 814.6600 | 3254.6108 | 3254.5818 | 8.91 | T | 1 | 39 | 0.00012 | FNYKYDNLNLHGHNIEELEEVLR +K_MMCCH_NL997 (K) | 2900 |
| 13 | 3003 | 3021 | 953.1623 | 2856.4650 | 2856.4467 | 6.44 | T | 1 | 31 | 0.00072 | KYDHTELDASVLPAPIIVR +K_MMCCH_NL673 (K) | 3003 |
| 14 | 613 | 626 | 1151.5167 | 2301.0189 | 2301.0150 | 1.69 | Th | 5 | 13 | 0.05 | ASKKFACCVHGMSV +K_MMCCH_NL673 (K) | 616 |

**Figure 14B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1047.4908 | 3139.4507 | 3139.4364 | 4.55 | T | 1 | 27 | 0.002 | KNVDSLSSDEVLALEK +Globo_H_MMCCH (K) | 26 |
| 2 | 164 | 175 | 615.6599 | 1843.9579 | 1843.9516 | 3.42 | T | 1 | 37 | 0.00019 | LFEKVQPGHHTR +K_MMCCH_NL997 (K) | 167 |
| 3 | 249 | 271 | 1012.4535 | 3034.3386 | 3034.3371 | 0.49 | T | 1 | 26 | 0.0024 | GKDPNSADCAHNLIHTPMEPFDR +Deamidated (NQ); K_MMCCH_NL997 (K); Oxidation (M) | 250 |
| 4 | 281 | 293 | 630.2952 | 1887.8639 | 1887.8574 | 3.44 | T | 0 | 44 | 4.00E-05 | EHAKPADSFDYGR +K_MMCCH_NL997 (K) | 284 |
| 5 | 419 | 446 | 942.0073 | 3763.9999 | 3763.9720 | 7.44 | T | 1 | 28 | 0.0015 | QPTLVHRPAKGHFDKPPVPVAQANLAVR +Deamidated (NQ); K_MMCCH_NL673 (K) | 433 |
| 6 | 463 | 488 | 1131.8618 | 3392.5636 | 3392.5918 | -8.28 | T | 2 | 73 | 5.20E-08 | AMERFQNDKSVDGYQATVEFHALPAR +Deamidated (NQ); K_MMCCH_NL997 (K); Oxidation (M) | 471 |
| 7 | 1097 | 1115 | 1008.4838 | 3022.4295 | 3022.4216 | 2.58 | T | 0 | 60 | 1.00E-06 | KPLQPFGLDSVINPDDETR +K_MMCCH_NL511 (K) | 1097 |
| 8 | 1123 | 1149 | 1157.0266 | 4624.0773 | 4624.0513 | 5.62 | T | 1 | 39 | 0.00013 | VFDYKNNFDYEYESLAFNGLSIAQLDR +Globo_H_MMCCH (K) | 1127 |
| 9 | 1355 | 1385 | 1217.5632 | 4866.2238 | 4866.1892 | 7.11 | T | 1 | 18 | 0.016 | GSAVAVPYWDWTEKADSLPSLINDATYFNSR +Globo_H_MMCCH (K) | 1368 |
| 10 | 1455 | 1480 | 853.1725 | 3408.6609 | 3408.6601 | 0.21 | T | 1 | 30 | 0.0009 | AKYSLSSLDYTAFDPVFFLHHANVDR +K_MMCCH_NL997 (K) | 1456 |
| 11 | 1508 | 1539 | 841.0049 | 4199.9883 | 4199.9680 | 4.83 | T | 1 | 30 | 0.0011 | KPLQPFNNPELNSDSMTLKHNLPQDSFDYQNR +Deamidated (NQ); K_MMCCH_NL997 (K); Oxidation (M) | 1526 |
| 12 | 1949 | 1963 | 574.7631 | 2295.0234 | 2295.0266 | -1.44 | T | 0 | 51 | 8.30E-06 | TQEFSKPEDTFDYHR +K_MMCCH_NL997 (K) | 1954 |
| 13 | 1292 | 1303 | 908.4932 | 1814.9719 | 1814.9713 | 0.39 | G | 0 | 28 | 0.0015 | SIRSAFLQIQKE +K_MMCCH_NL997 (K) | 1302 |
| 14 | 1681 | 1691 | 839.9299 | 1677.8453 | 1677.8508 | -3.28 | G | 0 | 15 | 0.032 | GLSQHNLVRKE +2 Deamidated (NQ); K_MMCCH_NL997 (K) | 1690 |
| 15 | 1589 | 1606 | 1136.1545 | 3405.4418 | 3405.4409 | 0.26 | C | 0 | 23 | 0.005 | ICVEQGGEQNCKTKAGSF +Globo_H_MMCCH (K) | 1602 |
| 16 | 1985 | 1995 | 586.6397 | 1756.8973 | 1756.8930 | 2.45 | C | 0 | 18 | 0.014 | IKQQQEADRVF +K_MMCCH_NL997 (K) | 1986 |
| 17 | 431 | 437 | 760.3697 | 1518.7248 | 1518.7164 | 5.60 | Th | 1 | 15 | 0.033 | FDKPPVP +K_MMCCH_NL673 (K) | 433 |
| 18 | 1496 | 1509 | 1047.4484 | 2092.8822 | 2092.9016 | -9.27 | Th | 4 | 13 | 0.049 | YNEADCAVNEMRKP +Deamidated (NQ); K_MMCCH_NL997 (K) | 1508 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the amino acid site of K in which identified as Globo_H_MMCCH, K_MMCCH_NL308, K_MMCCH_NL511, K_MMCCH_NL673, K_MMCCH_NL835, or K_MMCCH_NL997 modified for KLH1 or KLH2 sequence.

**Figure 15. Identification details of Globo H-conjugated peptides for sample 2 (1st LC-MS/MS)**

**Figure 15A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 588 | 606 | 1132.8397 | 3395.4973 | 3395.4862 | 3.30 | T | 1 | 25 | 0.0035 | EDKSAGGFQQLGAFHGEPK +Globo_H_MMCCH (K) | 590 |
| 2 | 700 | 719 | 1069.1805 | 3204.5198 | 3204.5060 | 4.31 | T | 1 | 28 | 0.0016 | SVQNKLFEQPEFGHYTSIAK +K_MMCCH_NL511 (K) | 719 |
| 3 | 953 | 965 | 654.6852 | 1961.0337 | 1961.0193 | 7.34 | T | 1 | 42 | 6.00E-05 | DLFKQPSVIHEPR +K_MMCCH_NL997 (K) | 956 |
| 4 | 1092 | 1114 | 645.3080 | 3221.5038 | 3221.5213 | -5.43 | T | 1 | 29 | 0.0013 | QHHYETNPFHHGKITHENEITTR +K_MMCCH_NL997 (K) | 1104 |
| 5 | 1923 | 1932 | 793.8966 | 1585.7787 | 1585.7745 | 2.65 | T | 1 | 14 | 0.04 | NKVMPNPFAR +Deamidated (NQ); K_MMCCH_NL997 (K); Oxidation (M) | 1924 |
| 6 | 2233 | 2251 | 721.5986 | 2882.3652 | 2882.3776 | -4.30 | T | 2 | 60 | 9.30E-07 | KEINTLTTAEVDNLKDAMR +Deamidated (NQ); K_MMCCH_NL673 (K) | 2247 |
| 7 | 2851 | 2883 | 1027.9795 | 4107.8889 | 4107.8666 | 5.43 | T | 0 | 32 | 0.00058 | VKPAHAGSCAGDIMHVPLHPFNYESVNNDDFTR +Deamidated (NQ); K_MMCCH_NL997 (K); Oxidation (M) | 2852 |
| 8 | 158 | 169 | 733.6753 | 2198.0042 | 2197.9838 | 9.33 | G | 2 | 32 | 0.00064 | AFHKFQEDRSVD +K_MMCCH_NL673 (K) | 161 |
| 9 | 474 | 480 | 568.7975 | 1135.5804 | 1135.5808 | -0.35 | Th | 1 | 16 | 0.024 | VQHKAGT +K_MMCCH_NL997 (K) | 477 |

**Figure 15B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1047.4899 | 3139.4478 | 3139.4364 | 3.63 | T | 1 | 29 | 0.0013 | KNVDSLSSDEVLALEK +Globo_H_MMCCH (K) | 26 |
| 2 | 164 | 175 | 615.6570 | 1843.9493 | 1843.9516 | -1.25 | T | 1 | 36 | 0.00026 | LFEKVQPGHHTR +K_MMCCH_NL997 (K) | 167 |
| 3 | 249 | 280 | 1015.9750 | 4059.8708 | 4059.8513 | 4.80 | T | 2 | 26 | 0.0025 | GKDPNSADCAHNLIHTPMEPFDRDTNPLDLTR +Deamidated (NQ); K_MMCCH_NL997 (K); Oxidation (M) | 250 |
| 4 | 281 | 293 | 630.2964 | 1887.8673 | 1887.8574 | 5.30 | T | 0 | 50 | 1.10E-05 | EHAKPADSFDYGR +K_MMCCH_NL997 (K) | 284 |
| 5 | 467 | 488 | 1125.8617 | 3374.5632 | 3374.5500 | 3.91 | T | 1 | 69 | 1.10E-07 | FQNDKSVDGYQATVEFHALPAR +K_MMCCH_NL511 (K) | 471 |
| 6 | 1097 | 1115 | 847.0916 | 2538.2530 | 2538.2312 | 8.59 | T | 0 | 42 | 6.00E-05 | KPLQPFGLDSVINPDDETR +2 Deamidated (NQ); K_MMCCH_NL997 (K) | 1097 |
| 7 | 1123 | 1149 | 1157.0225 | 4624.0607 | 4624.0513 | 2.03 | T | 1 | 35 | 0.00033 | VFDYKNNFDYEYESLAFNGLSIAQLDR +Globo_H_MMCCH (K) | 1127 |
| 8 | 1455 | 1480 | 853.1709 | 3408.6545 | 3408.6601 | -1.64 | T | 1 | 26 | 0.0026 | AKYSLSSLDYTAFDPVFFLHHANVDR +K_MMCCH_NL997 (K) | 1456 |
| 9 | 1508 | 1539 | 840.9999 | 4199.9633 | 4199.9680 | -1.12 | T | 1 | 33 | 0.00054 | KPLQPFNNPELNSDSMTLKHNLPQDSFDYQNR +Deamidated (NQ); K_MMCCH_NL997 (K); Oxidation (M) | 1526 |
| 10 | 1949 | 1963 | 766.0179 | 2295.0318 | 2295.0266 | 2.27 | T | 0 | 29 | 0.0013 | TQEFSKPEDTFDYHR +K_MMCCH_NL997 (K) | 1954 |
| 11 | 1985 | 1995 | 586.6395 | 1756.8968 | 1756.8930 | 2.11 | C | 0 | 19 | 0.014 | IKQQQEADRVF +K_MMCCH_NL997 (K) | 1986 |
| 12 | 2463 | 2479 | 1104.1498 | 3309.4275 | 3309.4269 | 0.18 | C | 1 | 20 | 0.01 | KAQSIHPEDVFDTDAPF +Globo_H_MMCCH (K) | 2463 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the amino acid site of K in which identified as Globo_H_MMCCH, K_MMCCH_NL308, K_MMCCH_NL511, K_MMCCH_NL673, K_MMCCH_NL835, or K_MMCCH_NL997 modified for KLH1 or KLH2 sequence.

**Figure 16. Identification details of Globo H-conjugated peptides for sample 3 (1st LC-MS/MS)**

**Figure 16A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 377 | 402 | 852.1614 | 3404.6167 | 3404.6104 | 1.85 | T | 0 | 18 | 0.017 | AHCAISLEHMHLKPFAFSSPLNNNEK +Deamidated (NQ); K_MMCCH_NL997 (K); Oxidation (M) | 402 |
| 2 | 1092 | 1114 | 887.4109 | 3545.6145 | 3545.6270 | -3.53 | T | 1 | 14 | 0.041 | QHHYETNPFHHGKITHENEITTR +K_MMCCH_NL673 (K) | 1104 |
| 3 | 1092 | 1114 | 806.3819 | 3221.4985 | 3221.5213 | -7.08 | T | 1 | 27 | 0.002 | QHHYETNPFHHGKITHENEITTR +K_MMCCH_NL997 (K) | 1104 |
| 4 | 2233 | 2251 | 721.5988 | 2882.3662 | 2882.3776 | -3.96 | T | 2 | 45 | 3.30E-05 | KEINTLTTAEVDNLKDAMR +Deamidated (NQ); K_MMCCH_NL673 (K) | 2247 |
| 5 | 2851 | 2883 | 1027.9769 | 4107.8786 | 4107.8666 | 2.92 | T | 0 | 46 | 2.60E-05 | VKPAHAGSCAGDIMHVPLHPFNYESVNNDDFTR +Deamidated (NQ); K_MMCCH_NL997 (K); Oxidation (M) | 2852 |

**Figure 16B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 1097 | 1115 | 847.0920 | 2538.2543 | 2538.2312 | 9.10 | T | 0 | 34 | 0.00043 | KPLQPFGLDSVINPDDETR +2 Deamidated (NQ); K_MMCCH_NL997 (K) | 1097 |
| 2 | 1355 | 1385 | 1217.5588 | 4866.2063 | 4866.1892 | 3.49 | T | 1 | 23 | 0.0047 | GSAVAVPYWDWTEKADSLPSLINDATYFNSR +Globo_H_MMCCH (K) | 1368 |
| 3 | 1508 | 1539 | 841.0057 | 4199.9920 | 4199.9680 | 5.71 | T | 1 | 46 | 2.60E-05 | KPLQPFNNPELNSDSMTLKHNLPQDSFDYQNR +Deamidated (NQ); K_MMCCH_NL997 (K); Oxidation (M) | 1526 |
| 4 | 1949 | 1963 | 766.0202 | 2295.0388 | 2295.0266 | 5.27 | T | 0 | 37 | 0.0002 | TQEFSKPEDTFDYHR +K_MMCCH_NL997 (K) | 1954 |
| 5 | 2463 | 2479 | 1104.1526 | 3309.4359 | 3309.4269 | 2.72 | C | 1 | 17 | 0.019 | KAQSIHPEDVFDTDAPF +Globo_H_MMCCH (K) | 2463 |
| 6 | 2438 | 2447 | 1110.4959 | 2218.9771 | 2218.9749 | 0.99 | Th | 3 | 13 | 0.05 | FILGGSKEMH +K_MMCCH_NL308 (K); Oxidation (M) | 2444 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the amino acid site of K in which identified as Globo_H_MMCCH, K_MMCCH_NL308, K_MMCCH_NL511, K_MMCCH_NL673, K_MMCCH_NL835, or K_MMCCH_NL997 modified for KLH1 or KLH2 sequence.

**Figure 17. Identification details of Globo H-conjugated peptides for sample 4 (1st LC-MS/MS)**

**Figure 17A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 377 | 402 | 1135.8804 | 3404.6193 | 3404.6104 | 2.61 | T | 0 | 41 | 8.80E-05 | AHCAISLEHMHLKPFAFSSPLNNNEK +Deamidated (NQ); K_MMCCH_NL997 (K); Oxidation (M) | 402 |
| 2 | 953 | 965 | 654.6849 | 1961.0330 | 1961.0193 | 6.99 | T | 1 | 27 | 0.0021 | DLFKQPSVIHEPR +K_MMCCH_NL997 (K) | 956 |
| 3 | 1092 | 1114 | 806.3821 | 3221.4995 | 3221.5213 | -6.80 | T | 1 | 18 | 0.015 | QHHYETNPFHHGKITHENEITTR +K_MMCCH_NL997 (K) | 1104 |
| 4 | 1923 | 1932 | 793.8979 | 1585.7812 | 1585.7745 | 4.29 | T | 1 | 17 | 0.021 | NKVMPNPFAR +Deamidated (NQ); K_MMCCH_NL997 (K); Oxidation (M) | 1924 |
| 5 | 2233 | 2251 | 721.5987 | 2882.3657 | 2882.3776 | -4.16 | T | 2 | 41 | 7.90E-05 | KEINTLTTAEVDNLKDAMR +Deamidated (NQ); K_MMCCH_NL673 (K) | 2247 |
| 6 | 2359 | 2367 | 927.4296 | 1852.8447 | 1852.8400 | 2.54 | T | 1 | 21 | 0.0082 | DKLFNDPER +K_MMCCH_NL673 (K) | 2360 |
| 7 | 2851 | 2883 | 1027.9815 | 4107.8967 | 4107.8666 | 7.33 | T | 0 | 30 | 0.00094 | VKPAHAGSCAGDIMHVPLHPFNYESVNNDDFTR +Deamidated (NQ); K_MMCCH_NL997 (K); Oxidation (M) | 2852 |
| 8 | 506 | 512 | 850.4159 | 1698.8173 | 1698.8009 | 9.71 | Th | 2 | 14 | 0.037 | LKDLDLT +K_MMCCH_NL511 (K) | 507 |

**Figure 17B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 164 | 175 | 615.6588 | 1843.9544 | 1843.9516 | 1.57 | T | 1 | 42 | 5.90E-05 | LFEKVQPGHHTR +K_MMCCH_NL997 (K) | 167 |
| 2 | 249 | 271 | 1012.4536 | 3034.3388 | 3034.3371 | 0.56 | T | 1 | 19 | 0.013 | GKDPNSADCAHNLIHTPMEPFDR +Deamidated (NQ); K_MMCCH_NL997 (K); Oxidation (M) | 250 |
| 3 | 281 | 293 | 630.2983 | 1887.8732 | 1887.8574 | 8.37 | T | 0 | 41 | 7.80E-05 | EHAKPADSFDYGR +K_MMCCH_NL997 (K) | 284 |
| 4 | 419 | 446 | 941.7554 | 3762.9925 | 3762.9879 | 1.20 | T | 1 | 26 | 0.0027 | QPTLVHRPAKGHFDKPPVPVAQANLAVR +K_MMCCH_NL673 (K) | 433 |
| 5 | 467 | 488 | 1071.8489 | 3212.5248 | 3212.4972 | 8.59 | T | 1 | 75 | 3.40E-08 | FQNDKSVDGYQATVEFHALPAR +K_MMCCH_NL673 (K) | 471 |
| 6 | 1097 | 1115 | 1178.8874 | 3533.6405 | 3533.6118 | 8.15 | T | 0 | 22 | 0.0069 | KPLQPFGLDSVINPDDETR +Globo_H_MMCCH (K) | 1097 |
| 7 | 1949 | 1963 | 766.0211 | 2295.0415 | 2295.0266 | 6.49 | T | 0 | 44 | 4.40E-05 | TQEFSKPEDTFDYHR +K_MMCCH_NL997 (K) | 1954 |
| 8 | 1985 | 1995 | 586.6405 | 1756.8997 | 1756.8930 | 3.81 | C | 0 | 22 | 0.0061 | IKQQQEADRVF +K_MMCCH_NL997 (K) | 1986 |
| 9 | 2463 | 2479 | 1104.1505 | 3309.4297 | 3309.4269 | 0.85 | C | 1 | 16 | 0.028 | KAQSIHPEDVFDTDAPF +Globo_H_MMCCH (K) | 2463 |
| 10 | 1525 | 1539 | 865.7426 | 2594.2060 | 2594.1959 | 3.89 | Th | 3 | 20 | 0.015 | LKHNLPQDSFDYQNR +K_MMCCH_NL673 (K) | 1526 |
| 11 | 1952 | 1963 | 646.6356 | 1936.8850 | 1936.8778 | 3.77 | Th | 2 | 55 | 3.00E-06 | FSKPEDTFDYHR +K_MMCCH_NL997 (K) | 1954 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the amino acid site of K in which identified as Globo_H_MMCCH, K_MMCCH_NL308, K_MMCCH_NL511, K_MMCCH_NL673, K_MMCCH_NL835, or K_MMCCH_NL997 modified for KLH1 or KLH2 sequence.

## Identification details of Globo H-conjugated peptides for sample 1 (2nd LC-MS/MS)

**Figure 18A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 588 | 606 | 1132.8378 | 3395.4915 | 3395.4862 | 1.56 | T | 1 | 19 | 0.012 | EDKSAGGFQQLGAFHGEPK +Globo_H_MMCCH (K) | 590 |
| 2 | 700 | 719 | 1015.1627 | 3042.4663 | 3042.4532 | 4.31 | T | 1 | 30 | 0.00093 | SVQNKLFEQPEFGHYTSIAK +K_MMCCH_NL673 (K) | 719 |
| 3 | 953 | 965 | 762.7192 | 2285.1359 | 2285.1249 | 4.81 | T | 1 | 47 | 2.10E-05 | DLFKQPSVIHEPR +K_MMCCH_NL673 (K) | 956 |
| 4 | 1092 | 1114 | 1074.8417 | 3221.5032 | 3221.5213 | -5.62 | T | 1 | 30 | 0.001 | QHHYETNPFHHGKITHENEITTR +K_MMCCH_NL997 (K) | 1104 |
| 5 | 1417 | 1445 | 980.2136 | 3916.8254 | 3916.8135 | 3.04 | T | 1 | 14 | 0.041 | ADHSSDGFQAIASFHALPPLCPSPAASKR +K_MMCCH_NL511 (K) | 1444 |
| 6 | 1473 | 1489 | 905.7894 | 2714.3463 | 2714.3414 | 1.81 | T | 1 | 17 | 0.022 | KHGAVVGLPYWDWTLPR +K_MMCCH_NL673 (K) | 1473 |
| 7 | 1923 | 1932 | 793.8984 | 1585.7822 | 1585.7745 | 4.86 | T | 1 | 15 | 0.034 | NKVMPNPFAR +Deamidated (NQ); K_MMCCH_NL997 (K); Oxidation (M) | 1924 |
| 8 | 2233 | 2251 | 721.5977 | 2882.3618 | 2882.3776 | -5.48 | T | 2 | 23 | 0.0048 | KEINTLTTAEVDNLKDAMR +Deamidated (NQ); K_MMCCH_NL673 (K) | 2247 |
| 9 | 2359 | 2367 | 1008.4539 | 2014.8933 | 2014.8929 | 0.20 | T | 1 | 17 | 0.022 | DKLFNDPER +K_MMCCH_NL511 (K) | 2360 |
| 10 | 2851 | 2883 | 1027.9791 | 4107.8874 | 4107.8666 | 5.06 | T | 0 | 35 | 0.0003 | VKPAHAGSCAGDIMHVPLHPFNYESVNNDDFTR +Deamidated (NQ); K_MMCCH_NL997 (K); Oxidation (M) | 2852 |
| 11 | 2897 | 2919 | 814.6596 | 3254.6093 | 3254.5818 | 8.45 | T | 1 | 36 | 0.00024 | FNYKYDNLNLHGHNIEELEEVLR +K_MMCCH_NL997 (K) | 2900 |
| 12 | 3003 | 3021 | 953.1583 | 2856.4530 | 2856.4467 | 2.21 | T | 1 | 17 | 0.021 | KYDHTELDASVLPAPIIVR +K_MMCCH_NL673 (K) | 3003 |
| 13 | 158 | 169 | 733.6757 | 2198.0053 | 2197.9838 | 9.83 | G | 2 | 14 | 0.044 | AFHKFQEDRSVD +K_MMCCH_NL673 (K) | 161 |

**Figure 18B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1047.4894 | 3139.4463 | 3139.4364 | 3.15 | T | 1 | 17 | 0.021 | KNVDSLSSDEVLALEK +Globo_H_MMCCH (K) | 26 |
| 2 | 164 | 175 | 615.6602 | 1843.9587 | 1843.9516 | 3.85 | T | 1 | 38 | 0.00015 | LFEKVQPGHHTR +K_MMCCH_NL997 (K) | 167 |
| 3 | 281 | 293 | 630.2961 | 1887.8664 | 1887.8574 | 4.82 | T | 0 | 47 | 2.10E-05 | EHAKPADSFDYGR +K_MMCCH_NL997 (K) | 284 |
| 4 | 419 | 446 | 860.9789 | 3439.8867 | 3439.8663 | 5.93 | T | 1 | 25 | 0.0029 | QPTLVHRPAKGHFDKPPVPVAQANLAVR +Deamidated (NQ); K_MMCCH_NL997 (K) | 433 |
| 5 | 467 | 488 | 1125.8600 | 3374.5581 | 3374.5500 | 2.40 | T | 1 | 68 | 1.40E-07 | FQNDKSVDGYQATVEFHALPAR +K_MMCCH_NL511 (K) | 471 |
| 6 | 936 | 950 | 847.7374 | 2540.1903 | 2540.1682 | 8.70 | T | 1 | 15 | 0.029 | KHGFTGGLPYWDWTR +K_MMCCH_NL673 (K) | 936 |
| 7 | 1097 | 1115 | 1008.4823 | 3022.4251 | 3022.4216 | 1.12 | T | 0 | 54 | 3.80E-06 | KPLQPFGLDSVINPDDETR +K_MMCCH_NL511 (K) | 1097 |
| 8 | 1455 | 1480 | 853.1713 | 3408.6562 | 3408.6601 | -1.14 | T | 1 | 27 | 0.0022 | AKYSLSSLDYTAFDPVFFLHHANVDR +K_MMCCH_NL997 (K) | 1456 |
| 9 | 1508 | 1539 | 841.0015 | 4199.9709 | 4199.9680 | 0.69 | T | 1 | 37 | 0.00018 | KPLQPFNNPELNSDSMTLKHNLPQDSFDYQNR +Deamidated (NQ); K_MMCCH_NL997 (K); Oxidation (M) | 1526 |
| 10 | 1949 | 1963 | 574.7651 | 2295.0315 | 2295.0266 | 2.09 | T | 0 | 55 | 3.30E-06 | TQEFSKPEDTFDYHR +K_MMCCH_NL997 (K) | 1954 |
| 11 | 2527 | 2541 | 691.3613 | 2071.0620 | 2071.0619 | 0.05 | T | 1 | 15 | 0.03 | KDVTSLTASEIENLR +K_MMCCH_NL997 (K) | 2527 |
| 12 | 3008 | 3025 | 845.4286 | 2533.2641 | 2533.2576 | 2.57 | T | 1 | 14 | 0.043 | ALKEHGSHLGIPYWDWTK +K_MMCCH_NL997 (K) | 3025 |
| 13 | 1292 | 1303 | 605.9988 | 1814.9745 | 1814.9713 | 1.82 | G | 0 | 16 | 0.023 | SIRSAFLQIQKE +K_MMCCH_NL997 (K) | 1302 |
| 14 | 2463 | 2479 | 1104.1495 | 3309.4268 | 3309.4269 | -0.03 | C | 1 | 19 | 0.012 | KAQSIHPEDVFDTDAPF +Globo_H_MMCCH (K) | 2463 |
| 15 | 1554 | 1560 | 621.8187 | 1241.6229 | 1241.6326 | -7.73 | Th | 1 | 14 | 0.036 | IQKLDQT +Deamidated (NQ); K_MMCCH_NL997 (K) | 1556 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the amino acid site of K in which identified as Globo_H_MMCCH, K_MMCCH_NL308, K_MMCCH_NL511, K_MMCCH_NL673, K_MMCCH_NL835, or K_MMCCH_NL997 modified for KLH1 or KLH2 sequence.

**Figure 19. Identification details of Globo H-conjugated peptides for sample 2 (2nd LC-MS/MS)**

**Figure 19A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 953 | 965 | 654.6824 | 1961.0253 | 1961.0193 | 3.06 | T | 1 | 46 | 2.60E-05 | DLFKQPSVIHEPR +K_MMCCH_NL997 (K) | 956 |
| 2 | 1092 | 1114 | 806.3803 | 3221.4919 | 3221.5213 | -9.13 | T | 1 | 18 | 0.015 | QHHYETNPFHHGKITHENEITTR +K_MMCCH_NL997 (K) | 1104 |
| 3 | 1417 | 1445 | 1108.0071 | 4427.9992 | 4428.0036 | -0.99 | T | 1 | 13 | 0.046 | ADHSSDGFQAIASFHALPPLCPSPAASKR +Globo_H_MMCCH (K) | 1444 |
| 4 | 2233 | 2251 | 721.5986 | 2882.3654 | 2882.3776 | -4.23 | T | 2 | 37 | 0.0002 | KEINTLTTAEVDNLKDAMR +Deamidated (NQ); K_MMCCH_NL673 (K) | 2247 |
| 5 | 2359 | 2367 | 927.4279 | 1852.8413 | 1852.8400 | 0.65 | T | 1 | 17 | 0.02 | DKLFNDPER +K_MMCCH_NL673 (K) | 2360 |
| 6 | 2851 | 2883 | 1027.9758 | 4107.8742 | 4107.8666 | 1.85 | T | 0 | 37 | 0.00018 | VKPAHAGSCAGDIMHVPLHPFNYESVNNDDFTR +Deamidated (NQ); K_MMCCH_NL997 (K); Oxidation (M) | 2852 |
| 7 | 1649 | 1664 | 1104.1271 | 3309.3594 | 3309.3654 | -1.81 | C | 2 | 14 | 0.038 | NLNDHTHDFSKPEDTF +Globo_H_MMCCH (K) | 1659 |

**Figure 19B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1047.4901 | 3139.4485 | 3139.4364 | 3.85 | T | 1 | 21 | 0.0089 | KNVDSLSSDEVLALEK +Globo_H_MMCCH (K) | 26 |
| 2 | 164 | 175 | 615.6576 | 1843.9509 | 1843.9516 | -0.33 | T | 1 | 33 | 0.00046 | LFEKVQPGHHTR +K_MMCCH_NL997 (K) | 167 |
| 3 | 249 | 271 | 1012.4529 | 3034.3368 | 3034.3371 | -0.10 | T | 1 | 16 | 0.025 | GKDPNSADCAHNLIHTPMEPFDR +Deamidated (NQ); K_MMCCH_NL997 (K); Oxidation (M) | 250 |
| 4 | 281 | 293 | 630.2961 | 1887.8666 | 1887.8574 | 4.87 | T | 0 | 49 | 1.10E-05 | EHAKPADSFDYGR +K_MMCCH_NL997 (K) | 284 |
| 5 | 419 | 446 | 942.0069 | 3763.9985 | 3763.9720 | 7.04 | T | 1 | 30 | 0.0011 | QPTLVHRPAKGHFDKPPVPVAQANLAVR +Deamidated (NQ); K_MMCCH_NL673 (K) | 433 |
| 6 | 467 | 488 | 1125.8563 | 3374.5471 | 3374.5500 | -0.86 | T | 1 | 59 | 1.20E-06 | FQNDKSVDGYQATVEFHALPAR +K_MMCCH_NL511 (K) | 471 |
| 7 | 1097 | 1115 | 847.0906 | 2538.2501 | 2538.2312 | 7.45 | T | 0 | 26 | 0.0024 | KPLQPFGLDSVINPDDETR +2 Deamidated (NQ); K_MMCCH_NL997 (K) | 1097 |
| 8 | 1355 | 1385 | 1217.5574 | 4866.2004 | 4866.1892 | 2.28 | T | 1 | 15 | 0.029 | GSAVAVPYWDWTEKADSLPSLINDATYFNSR +Globo_H_MMCCH (K) | 1368 |
| 9 | 1508 | 1526 | 862.7620 | 2585.2642 | 2585.2505 | 5.30 | T | 0 | 15 | 0.03 | KPLQPFNNPELNSDSMTLK +Deamidated (NQ); K_MMCCH_NL997 (K); Oxidation (M) | 1526 |
| 10 | 1949 | 1963 | 928.0676 | 2781.1811 | 2781.1851 | -1.44 | T | 0 | 49 | 1.20E-05 | TQEFSKPEDTFDYHR +K_MMCCH_NL511 (K) | 1954 |
| 11 | 2527 | 2541 | 691.3618 | 2071.0635 | 2071.0619 | 0.72 | T | 1 | 21 | 0.0083 | KDVTSLTASEIENLR +K_MMCCH_NL997 (K) | 2527 |
| 12 | 1292 | 1303 | 605.9978 | 1814.9716 | 1814.9713 | 0.17 | G | 0 | 16 | 0.026 | SIRSAFLQIQKE +K_MMCCH_NL997 (K) | 1302 |
| 13 | 1526 | 1540 | 1101.4783 | 3301.4130 | 3301.4232 | -3.09 | C | 2 | 17 | 0.021 | KHNLPQDSFDYQNRF +Globo_H_MMCCH (K) | 1526 |
| 14 | 1985 | 1995 | 586.6373 | 1756.8902 | 1756.8930 | -1.65 | C | 0 | 23 | 0.0049 | IKQQQEADRVF +K_MMCCH_NL997 (K) | 1986 |
| 15 | 2463 | 2479 | 1104.1492 | 3309.4257 | 3309.4269 | -0.36 | C | 1 | 23 | 0.0045 | KAQSIHPEDVFDTDAPF +Globo_H_MMCCH (K) | 2463 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the amino acid site of K in which identified as Globo_H_MMCCH, K_MMCCH_NL308, K_MMCCH_NL511, K_MMCCH_NL673, K_MMCCH_NL835, or K_MMCCH_NL997 modified for KLH1 or KLH2 sequence.

Figure 20. Identification details of Globo H-conjugated peptides for sample 3 (2nd LC-MS/MS)

Figure 20A. KLH 1

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 377 | 402 | 852.1617 | 3404.6179 | 3404.6104 | 2.20 | T | 0 | 14 | 0.037 | AHCAISLEHMHLKPFAFSSPLNNNEK +Deamidated (NQ); K_MMCCH_NL997 (K); Oxidation (M) | 402 |
| 2 | 700 | 719 | 1069.1809 | 3204.5209 | 3204.5060 | 4.65 | T | 1 | 19 | 0.012 | SVQNKLFEQPEFGHYTSIAK +K_MMCCH_NL511 (K) | 719 |
| 3 | 1092 | 1114 | 806.3823 | 3221.4999 | 3221.5213 | -6.64 | T | 1 | 17 | 0.019 | QHHYETNPFHHGKITHENEITTR +K_MMCCH_NL997 (K) | 1104 |
| 4 | 2233 | 2251 | 721.5977 | 2882.3618 | 2882.3776 | -5.48 | T | 2 | 28 | 0.0016 | KEINTLTTAEVDNLKDAMR +Deamidated (NQ); K_MMCCH_NL673 (K) | 2247 |
| 5 | 2851 | 2883 | 1027.9711 | 4107.8552 | 4107.8666 | -2.78 | T | 0 | 32 | 0.00067 | VKPAHAGSCAGDIMHVPLHPFNYESVNNDDFTR +Deamidated (NQ); K_MMCCH_NL997 (K); Oxidation (M) | 2852 |
| 6 | 158 | 169 | 733.6741 | 2198.0006 | 2197.9838 | 7.64 | G | 2 | 16 | 0.025 | AFHKFQEDRSVD +K_MMCCH_NL673 (K) | 161 |

**Figure 20B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 419 | 446 | 941.7502 | 3762.9719 | 3762.9879 | -4.28 | T | 1 | 16 | 0.028 | QPTLVHRPAKGHFDKPPVPVAQANLAVR +K_MMCCH_NL673 (K) | 433 |
| 2 | 1097 | 1115 | 847.0919 | 2538.2539 | 2538.2312 | 8.94 | T | 0 | 44 | 3.60E-05 | KPLQPFGLDSVINPDDETR +2 Deamidated (NQ); K_MMCCH_NL997 (K) | 1097 |
| 3 | 1355 | 1385 | 1217.5449 | 4866.1506 | 4866.1892 | -7.95 | T | 1 | 23 | 0.0053 | GSAVAVPYWDWTEKADSLPSLINDATYFNSR +Globo_H_MMCCH (K) | 1368 |
| 4 | 1508 | 1539 | 841.0017 | 4199.9722 | 4199.9680 | 0.98 | T | 1 | 55 | 3.50E-06 | KPLQPFNNPELNSDSMTLKHNLPQDSFDYQNR +Deamidated (NQ); K_MMCCH_NL997 (K); Oxidation (M) | 1526 |
| 5 | 1224 | 1235 | 939.9670 | 1877.9195 | 1877.9022 | 9.21 | Th | 5 | 14 | 0.041 | LHVGDNFFLKYE +Deamidated (NQ); K_MMCCH_NL997 (K) | 1233 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the amino acid site of K in which identified as Globo_H_MMCCH, K_MMCCH_NL308, K_MMCCH_NL511, K_MMCCH_NL673, K_MMCCH_NL835, or K_MMCCH_NL997 modified for KLH1 or KLH2 sequence

**Figure 21. Identification details of Globo H-conjugated peptides for sample 4 (2nd LC-MS/MS)**

**Figure 21A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 778 | 789 | 986.5373 | 1971.0600 | 1971.0553 | 2.44 | T | 1 | 17 | 0.021 | IWAIWQALQKYR +K_MMCCH_NL997 (K) | 787 |
| 2 | 953 | 965 | 654.6835 | 1961.0288 | 1961.0193 | 4.84 | T | 1 | 32 | 0.00065 | DLFKQPSVIHEPR +K_MMCCH_NL997 (K) | 956 |
| 3 | 1092 | 1114 | 806.3824 | 3221.5007 | 3221.5213 | -6.39 | T | 1 | 24 | 0.004 | QHHYETNPFHHGKITHENEITTR +K_MMCCH_NL997 (K) | 1104 |
| 4 | 1923 | 1932 | 793.8977 | 1585.7809 | 1585.7745 | 4.04 | T | 1 | 19 | 0.011 | NKVMPNPFAR +Deamidated (NQ); K_MMCCH_NL997 (K); Oxidation (M) | 1924 |
| 5 | 2233 | 2251 | 721.5991 | 2882.3671 | 2882.3776 | -3.64 | T | 2 | 17 | 0.022 | KEINTLTTAEVDNLKDAMR +Deamidated (NQ); K_MMCCH_NL673 (K) | 2247 |
| 6 | 2851 | 2883 | 1027.9812 | 4107.8957 | 4107.8666 | 7.08 | T | 0 | 49 | 1.20E-05 | VKPAHAGSCAGDIMHVPLHPFNYESVNNDDFTR +Deamidated (NQ); K_MMCCH_NL997 (K); Oxidation (M) | 2852 |
| 7 | 1649 | 1664 | 1104.1299 | 3309.3678 | 3309.3654 | 0.73 | C | 2 | 14 | 0.042 | NLNDHTHDFSKPEDTF +Globo_H_MMCCH (K) | 1659 |
| 8 | 601 | 612 | 955.3971 | 2863.1694 | 2863.1715 | -0.73 | Th | 1 | 26 | 0.0026 | FHGEPKWCPSPE +Globo_H_MMCCH (K) | 606 |
| 9 | 1580 | 1587 | 763.8522 | 1525.6899 | 1525.6858 | 2.69 | Th | 1 | 14 | 0.037 | VGGKEPYG +K_MMCCH_NL673 (K) | 1583 |

**Figure 21B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 164 | 175 | 615.6603 | 1843.9592 | 1843.9516 | 4.12 | T | 1 | 40 | 0.00011 | LFEKVQPGHHTR +K_MMCCH_NL997 (K) | 167 |
| 2 | 281 | 293 | 630.2977 | 1887.8714 | 1887.8574 | 7.42 | T | 0 | 40 | 9.20E-05 | EHAKPADSFDYGR +K_MMCCH_NL997 (K) | 284 |
| 3 | 467 | 488 | 1071.8480 | 3212.5222 | 3212.4972 | 7.78 | T | 1 | 60 | 1.00E-06 | FQNDKSVDGYQATVEFHALPAR +K_MMCCH_NL673 (K) | 471 |
| 4 | 1097 | 1115 | 1178.8835 | 3533.6288 | 3533.6118 | 4.81 | T | 0 | 19 | 0.014 | KPLQPFGLDSVINPDDETR +Globo_H_MMCCH (K) | 1097 |
| 5 | 1949 | 1963 | 766.0208 | 2295.0406 | 2295.0266 | 6.10 | T | 0 | 34 | 0.00039 | TQEFSKPEDTFDYHR +K_MMCCH_NL997 (K) | 1954 |
| 6 | 2728 | 2739 | 986.5373 | 1971.0600 | 1971.0553 | 2.44 | T | 1 | 17 | 0.021 | IWAIWQALQKYR +K_MMCCH_NL997 (K) | 2737 |
| 7 | 1985 | 1995 | 586.6405 | 1756.8997 | 1756.8930 | 3.81 | C | 0 | 23 | 0.0046 | IKQQQEADRVF +K_MMCCH_NL997 (K) | 1986 |
| 8 | 2463 | 2479 | 1104.1527 | 3309.4363 | 3309.4269 | 2.84 | C | 1 | 14 | 0.037 | KAQSIHPEDVFDTDAPF +Globo_H_MMCCH (K) | 2463 |
| 9 | 893 | 904 | 955.3971 | 2863.1694 | 2863.1715 | -0.73 | Th | 1 | 26 | 0.0026 | FHGEPKWCPSPE +Globo_H_MMCCH (K) | 898 |
| 10 | 1224 | 1233 | 1036.4961 | 2070.9776 | 2070.9707 | 3.33 | Th | 4 | 15 | 0.029 | LHVGDNFFLK +K_MMCCH_NL511 (K) | 1233 |
| 11 | 1952 | 1963 | 646.6351 | 1936.8834 | 1936.8778 | 2.89 | Th | 2 | 24 | 0.0041 | FSKPEDTFDYHR +K_MMCCH_NL997 (K) | 1954 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the amino acid site of K in which identified as Globo_H_MMCCH, K_MMCCH_NL308, K_MMCCH_NL511, K_MMCCH_NL673, K_MMCCH_NL835, or K_MMCCH_NL997 modified for KLH1 or KLH2 sequence.

**Figure 22. Identification details of MMCCH-conjugated peptides for sample 1 (1st LC-MS/MS)**

**Figure 22A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 124 | 141 | 785.4098 | 2353.2075 | 2353.2001 | 3.14 | T | 1 | 21 | 0.0086 | GFTDPPVKHHQSANLLVR +dK_MMCCH-2 (K) | 131 |
| 2 | 157 | 166 | 823.3666 | 1644.7187 | 1644.7242 | -3.34 | T | 1 | 20 | 0.011 | EAFHKFQEDR +Deamidated (NQ); dK_MMCCH-2 (K) | 161 |
| 3 | 377 | 402 | 833.3975 | 3329.5607 | 3329.5784 | -5.29 | T | 0 | 37 | 0.00019 | AHCAISLEHMHLKPFAFSSPLNNNEK +dK_MMCCH-2 (K) | 389 |
| 4 | 460 | 477 | 781.3958 | 2341.1654 | 2341.1624 | 1.28 | T | 1 | 19 | 0.014 | TSANVDIFIKTTDSVQHK +dK_MMCCH-2 (K) | 469 |
| 5 | 470 | 488 | 748.0436 | 2241.1091 | 2241.1100 | -0.40 | T | 1 | 18 | 0.017 | TTDSVQHKAGTFAVLGGSK +dK_MMCCH-2 (K) | 477 |
| 6 | 489 | 496 | 718.8198 | 1435.6251 | 1435.6377 | -8.78 | T | 1 | 15 | 0.029 | EMKWGFDR +dK_MMCCH-1 (K); Oxidation (M) | 491 |
| 7 | 553 | 560 | 663.8676 | 1325.7207 | 1325.7166 | 3.09 | T | 2 | 23 | 0.0051 | VKFDKVPR +dK_MMCCH-2 (K) | 554 |
| 8 | 566 | 570 | 485.2450 | 968.4754 | 968.4749 | 0.52 | T | 1 | 16 | 0.028 | KNVDR +dK_MMCCH-2 (K) | 566 |
| 9 | 581 | 587 | 547.8376 | 1093.6606 | 1093.6569 | 3.38 | T | 1 | 19 | 0.013 | KALALLK +dK_MMCCH-2 (K) | 581 |
| 10 | 588 | 606 | 781.0359 | 2340.0858 | 2340.0845 | 0.60 | T | 1 | 31 | 0.00076 | EDKSAGGFQQLGAFHGEPK +dK_MMCCH-2 (K) | 590 |
| 11 | 591 | 615 | 1004.4674 | 3010.3804 | 3010.3742 | 2.06 | T | 1 | 16 | 0.024 | SAGGFQQLGAFHGEPKWCPSPEASK +dK_MMCCH-2 (K) | 606 |
| 12 | 645 | 681 | 1158.5342 | 4630.1076 | 4630.1063 | 0.28 | T | 1 | 26 | 0.0025 | HGYDGALPYWDWTSPLNHLPELADHEKYVDPEDGVEK +dK_MMCCH-2 (K) | 671 |
| 13 | 682 | 699 | 1239.5814 | 2477.1483 | 2477.1434 | 1.98 | T | 1 | 18 | 0.016 | HNPWFDGHIDTVDKTTTR +dK_MMCCH-2 (K) | 695 |
| 14 | 700 | 719 | 887.7735 | 2660.2987 | 2660.2945 | 1.58 | T | 1 | 44 | 4.20E-05 | SVQNKLFEQPEFGHYTSIAK +dK_MMCCH-2 (K) | 704 |
| 15 | 778 | 789 | 957.9996 | 1913.9846 | 1913.9862 | -0.84 | T | 1 | 32 | 0.00059 | IWAIWQALQKYR +Deamidated (NQ); dK_MMCCH-2 (K) | 787 |
| 16 | 790 | 804 | 669.3175 | 2004.9307 | 2004.9220 | 4.34 | T | 0 | 49 | 1.20E-05 | GKPYNVANCAVTSMR +dK_MMCCH-2 (K) | 791 |
| 17 | 858 | 863 | 520.3004 | 1038.5863 | 1038.5783 | 7.70 | T | 1 | 19 | 0.013 | KLEAIK +dK_MMCCH-2 (K) | 858 |
| 18 | 953 | 965 | 635.3312 | 1902.9717 | 1902.9662 | 2.89 | T | 1 | 47 | 1.90E-05 | DLFKQPSVIHEPR +dK_MMCCH-2 (K) | 956 |
| 19 | 986 | 1000 | 1027.0348 | 2052.0550 | 2052.0561 | -0.54 | T | 1 | 84 | 4.40E-09 | KNIENLSLGELESLR +dK_MMCCH-2 (K) | 986 |
| 20 | 1048 | 1060 | 913.5302 | 1825.0459 | 1825.0423 | 1.97 | T | 1 | 26 | 0.0024 | LYVVVVENALLKK +dK_MMCCH-2 (K) | 1060 |
| 21 | 1092 | 1114 | 633.9023 | 3164.4753 | 3164.4523 | 7.30 | T | 1 | 33 | 0.00052 | QHHYETNPFHHGKITHENEITTR +Deamidated (NQ); dK_MMCCH-2 (K) | 1104 |
| 22 | 1118 | 1162 | 1131.7467 | 5653.6971 | 5653.6850 | 2.14 | T | 1 | 18 | 0.014 | DSLFHSDYFYEQVLYALEQDNFCDFEIQLEILHNALHSLLGGKGK +dK_MMCCH-2 (K) | 1162 |
| 23 | 1199 | 1203 | 353.1915 | 1056.5526 | 1056.5538 | -1.23 | T | 1 | 17 | 0.019 | KRPYR +dK_MMCCH-2 (K) | 1199 |
| 24 | 1331 | 1338 | 620.3547 | 1238.6949 | 1238.6944 | 0.40 | T | 1 | 23 | 0.0054 | LDITKALK +dK_MMCCH-2 (K) | 1335 |

**Figure 22A. KLH 1 (continued)**

| | | | | | | | | | | | | |
|---|---|---|---|---|---|---|---|---|---|---|---|---|
| 25 | 1395 | 1403 | 485.5956 | 1453.7651 | 1453.7599 | 3.58 | T | 2 | 25 | 0.0031 | KDITQLDKR +dK_MMCCH-2 (K) | 1395 |
| 26 | 1395 | 1403 | 598.3075 | 1791.9007 | 1791.8899 | 5.97 | T | 2 | 21 | 0.0084 | KDITQLDKR +2 dK_MMCCH-2 (K) | 1395, 1402 |
| 27 | 1417 | 1445 | 1125.2136 | 3372.6190 | 3372.6020 | 5.07 | T | 1 | 27 | 0.002 | ADHSSDGFQAIASFHALPPLCPSPAASKR +dK_MMCCH-2 (K) | 1444 |
| 28 | 1473 | 1489 | 778.4057 | 2332.1953 | 2332.1827 | 5.40 | T | 1 | 46 | 2.60E-05 | KHGAVVGLPYWDWTLPR +dK_MMCCH-2 (K) | 1473 |
| 29 | 1519 | 1530 | 898.9364 | 1795.8582 | 1795.8563 | 1.06 | T | 1 | 16 | 0.025 | IEFEGENVHTKR +dK_MMCCH-2 (K) | 1529 |
| 30 | 1721 | 1746 | 1092.8095 | 3275.4065 | 3275.3886 | 5.47 | T | 1 | 15 | 0.035 | TAGDCEDAGYFTVLGGEKEMPWAFDR +dK_MMCCH-2 (K); Oxidation (M) | 1738 |
| 31 | 1747 | 1758 | 613.9775 | 1838.9108 | 1838.9012 | 5.22 | T | 1 | 22 | 0.0067 | LYKYDITETLDK +dK_MMCCH-2 (K) | 1749 |
| 32 | 1923 | 1932 | 756.3798 | 1510.7451 | 1510.7425 | 1.72 | T | 1 | 29 | 0.0012 | NKVMPNPFAR +dK_MMCCH-2 (K) | 1924 |
| 33 | 2067 | 2076 | 746.8879 | 1491.7613 | 1491.7544 | 4.63 | T | 1 | 27 | 0.0021 | KHAVPNDVFK +dK_MMCCH-2 (K) | 2067 |
| 34 | 2068 | 2083 | 753.7155 | 2258.1247 | 2258.1194 | 2.35 | T | 1 | 16 | 0.025 | HAVPNDVFKYELLGYR +dK_MMCCH-2 (K) | 2076 |
| 35 | 2121 | 2149 | 890.4191 | 3557.6472 | 3557.6378 | 2.64 | T | 1 | 37 | 0.00021 | TSADVQFQICKTSEDCHHGGQIFVLGGTK +dK_MMCCH-2 (K) | 2131 |
| 36 | 2299 | 2314 | 715.0181 | 2142.0324 | 2142.0238 | 4.01 | T | 1 | 60 | 9.80E-07 | LYTKQMEDALTAHGAR +dK_MMCCH-2 (K) | 2302 |
| 37 | 2359 | 2367 | 736.3492 | 1470.6839 | 1470.6813 | 1.77 | T | 1 | 35 | 0.00034 | DKLFNDPER +dK_MMCCH-2 (K) | 2360 |
| 38 | 2461 | 2490 | 642.8196 | 3850.8742 | 3850.8638 | 2.70 | T | 0 | 16 | 0.026 | RPLRPFSDPINHNAFTHSNAKPTDVFEYSR +dK_MMCCH-2 (K) | 2481 |
| 39 | 2507 | 2514 | 682.3518 | 1362.6889 | 1362.6853 | 2.64 | T | 1 | 30 | 0.001 | KLEHELEK +dK_MMCCH-2 (K) | 2507 |
| 40 | 2515 | 2520 | 571.7622 | 1141.5099 | 1141.5074 | 2.19 | T | 1 | 19 | 0.012 | QKEEDR +dK_MMCCH-2 (K) | 2516 |
| 41 | 2595 | 2609 | 983.5271 | 1965.0396 | 1965.0493 | -4.89 | T | 1 | 17 | 0.02 | IIDTSGKQLPSDLIK +dK_MMCCH-2 (K) | 2601 |
| 42 | 2621 | 2636 | 802.7155 | 2405.1245 | 2405.1083 | 6.74 | T | 1 | 51 | 8.60E-06 | HHEKHHEDHHEDILVR +dK_MMCCH-2 (K) | 2624 |
| 43 | 2637 | 2651 | 1069.5284 | 2137.0423 | 2137.0374 | 2.29 | T | 1 | 59 | 1.40E-06 | KNIHSLSHHEAEELR +dK_MMCCH-2 (K) | 2637 |
| 44 | 2652 | 2682 | 975.2011 | 3896.7753 | 3896.7563 | 4.90 | T | 1 | 14 | 0.041 | DALYKLQNDESHGGYEHIAGFHGYPNLCPEK +dK_MMCCH-2 (K) | 2656 |
| 45 | 2761 | 2814 | 1328.4260 | 6637.0938 | 6637.0821 | 1.76 | T | 1 | 20 | 0.0094 | DVNEAIFQQTKFGEFSSIFYLALQALEEDNYCDFEVQYEILHNEVHALIGGAEK +4 Deamidated (NQ); dK_MMCCH-1 (K) | 2771 |
| 46 | 2851 | 2883 | 1009.2216 | 4032.8574 | 4032.8346 | 5.65 | T | 0 | 69 | 1.20E-07 | VKPAHAGSCAGDIMHVPLHPFNYESVNNDDFTR +dK_MMCCH-2 (K) | 2852 |
| 47 | 2897 | 2919 | 1066.5184 | 3196.5335 | 3196.5287 | 1.47 | T | 1 | 55 | 3.20E-06 | FNYKYDNLNLHGHNIEELEEVLR +dK_MMCCH-2 (K) | 2900 |
| 48 | 2938 | 2947 | 730.4154 | 1458.8163 | 1458.8156 | 0.48 | T | 1 | 20 | 0.01 | TTAVVKVYIK +dK_MMCCH-2 (K) | 2943 |
| 49 | 3003 | 3021 | 825.7748 | 2474.3027 | 2474.2879 | 5.98 | T | 1 | 46 | 2.80E-05 | KYDHTELDASVLPAPIIVR +dK_MMCCH-2 (K) | 3003 |
| 50 | 3022 | 3044 | 966.5284 | 2896.5635 | 2896.5521 | 3.94 | T | 1 | 15 | 0.03 | RPNNAVFDIIEIPIGKDVNLPPK +dK_MMCCH-2 (K) | 3037 |

Figure 22A. KLH 1 (continued)

| | | | | | | | | | | | | |
|---|---|---|---|---|---|---|---|---|---|---|---|---|
| 51 | 123 | 151 | 739.7915 | 3693.9211 | 3693.9049 | 4.39 | G | 2 | 31 | 0.00075 | KGFTDPPVKHHQSANLLVRKNINDLTREE +dK_MMCCH-2 (K) | 131 |
| 52 | 158 | 176 | 846.3878 | 2536.1415 | 2536.1329 | 3.39 | G | 3 | 25 | 0.0034 | AFHKFQEDRSVDGYQATAE +dK_MMCCH-2 (K) | 161 |
| 53 | 435 | 442 | 676.3259 | 1350.6373 | 1350.6312 | 4.52 | G | 1 | 15 | 0.03 | NIEKMIHE +dK_MMCCH-2 (K) | 438 |
| 54 | 526 | 544 | 777.7507 | 2330.2304 | 2330.2304 | 0.00 | G | 1 | 34 | 0.00038 | VDGTKLASSLIPHASVIRE +dK_MMCCH-2 (K) | 530 |
| 55 | 589 | 612 | 1102.8276 | 3305.4611 | 3305.4621 | -0.30 | G | 1 | 13 | 0.048 | DKSAGGFQQLGAFHGEPKWCPSPE +2 dK_MMCCH-2 (K) | 590, 606 |
| 56 | 589 | 612 | 990.1173 | 2967.3301 | 2967.3320 | -0.64 | G | 1 | 22 | 0.0057 | DKSAGGFQQLGAFHGEPKWCPSPE +dK_MMCCH-2 (K) | 606 |
| 57 | 711 | 726 | 1064.5625 | 2127.1104 | 2127.1074 | 1.41 | G | 0 | 16 | 0.025 | FGHYTSIAKQVLLALE +dK_MMCCH-2 (K) | 719 |
| 58 | 825 | 841 | 820.7039 | 2459.0898 | 2459.0780 | 4.76 | G | 1 | 26 | 0.0028 | HSVPFNVFDYKTNFNYE +Deamidated (NQ); dK_MMCCH-2 (K) | 835 |
| 59 | 847 | 860 | 643.6771 | 1928.0095 | 1928.0077 | 0.93 | G | 0 | 26 | 0.0024 | FNGLSISQLNKKLE +dK_MMCCH-2 (K) | 858 |
| 60 | 942 | 972 | 955.5030 | 3817.9829 | 3817.9614 | 5.63 | G | 3 | 17 | 0.019 | VFDLKPASLGKDLFKQPSVIHEPRIGHHEGE +dK_MMCCH-2 (K) | 946 |
| 61 | 942 | 970 | 727.3867 | 3631.8972 | 3631.8973 | -0.03 | G | 2 | 32 | 0.0007 | VFDLKPASLGKDLFKQPSVIHEPRIGHHE +dK_MMCCH-2 (K) | 956 |
| 62 | 1055 | 1076 | 810.1674 | 3236.6403 | 3236.6402 | 0.03 | G | 1 | 32 | 0.00065 | NALLKKGSSVAVPYWDWTKRIE +2 dK_MMCCH-2 (K) | 1059, 1060 |
| 63 | 1055 | 1076 | 725.6326 | 2898.5012 | 2898.5102 | -3.11 | G | 1 | 38 | 0.00018 | NALLKKGSSVAVPYWDWTKRIE +dK_MMCCH-2 (K) | 1060 |
| 64 | 1097 | 1108 | 878.4210 | 1754.8275 | 1754.8199 | 4.33 | G | 0 | 22 | 0.0064 | TNPFHHGKITHE +dK_MMCCH-2 (K) | 1104 |
| 65 | 1376 | 1396 | 864.3891 | 2590.1455 | 2590.1428 | 1.04 | G | 3 | 21 | 0.0072 | AGTDSAHTDDGHTEPVMIRKD +dK_MMCCH-2 (K) | 1395 |
| 66 | 1397 | 1413 | 774.4356 | 2320.2848 | 2320.2824 | 1.03 | G | 1 | 29 | 0.0012 | ITQLDKRQQLSLVKALE +dK_MMCCH-2 (K) | 1410 |
| 67 | 1506 | 1522 | 753.3769 | 2257.1088 | 2257.1089 | -0.04 | G | 2 | 19 | 0.013 | TGRDIPNPFIGSKIEFE +dK_MMCCH-2 (K) | 1518 |
| 68 | 1755 | 1765 | 570.6054 | 1708.7944 | 1708.7913 | 1.81 | G | 2 | 26 | 0.0027 | TLDKMNLRHDE +dK_MMCCH-2 (K) | 1758 |
| 69 | 2059 | 2078 | 692.5998 | 2766.3703 | 2766.3588 | 4.16 | G | 1 | 15 | 0.032 | INHNQFTKKHAVPNDVFKYE +dK_MMCCH-2 (K) | 2076 |
| 70 | 2089 | 2100 | 854.4097 | 1706.8048 | 1706.8008 | 2.34 | G | 2 | 22 | 0.0065 | IGGMNLHEIEKE +dK_MMCCH-2 (K) | 2099 |
| 71 | 2647 | 2661 | 715.6793 | 2144.0161 | 2144.0095 | 3.08 | G | 4 | 24 | 0.0037 | AEELRDALYKLQNDE +dK_MMCCH-2 (K) | 2656 |
| 72 | 3090 | 3097 | 632.8041 | 1263.5937 | 1263.5879 | 4.59 | G | 0 | 13 | 0.048 | LGKMYSVE +dK_MMCCH-2 (K) | 3092 |
| 73 | 59 | 68 | 721.3473 | 1440.6800 | 1440.6781 | 1.32 | C | 1 | 37 | 0.0002 | VLGGPSEMKW +dK_MMCCH-2 (K) | 67 |
| 74 | 94 | 100 | 573.2999 | 1144.5853 | 1144.5838 | 1.31 | C | 1 | 15 | 0.031 | TVKAELF +dK_MMCCH-2 (K) | 96 |
| 75 | 429 | 450 | 990.8061 | 2969.3964 | 2969.3899 | 2.22 | C | 1 | 21 | 0.0078 | GGISLENIEKMIHENQQEDRIY +dK_MMCCH-2 (K); Oxidation (M) | 438 |
| 76 | 468 | 481 | 935.9773 | 1869.9400 | 1869.9295 | 5.62 | C | 0 | 19 | 0.014 | IKTTDSVQHKAGTF +dK_MMCCH-2 (K) | 469 |
| 77 | 584 | 595 | 787.4011 | 1572.7876 | 1572.7858 | 1.14 | C | 2 | 40 | 9.10E-05 | ALLKEDKSAGGF +dK_MMCCH-2 (K) | 590 |

**Figure 22A. KLH 1 (continued)**

| | | | | | | | | | | | | |
|---|---|---|---|---|---|---|---|---|---|---|---|---|
| 78 | 715 | 722 | 599.3331 | 1196.6516 | 1196.6475 | 3.43 | C | 0 | 22 | 0.006 | TSIAKQVL +dK_MMCCH-2 (K) | 719 |
| 79 | 783 | 788 | 544.7778 | 1087.5410 | 1087.5372 | 3.49 | C | 1 | 26 | 0.0025 | QALQKY +dK_MMCCH-2 (K) | 787 |
| 80 | 830 | 838 | 743.3410 | 1484.6674 | 1484.6646 | 1.89 | C | 2 | 19 | 0.013 | NVFDYKTNF +dK_MMCCH-2 (K) | 835 |
| 81 | 1013 | 1018 | 516.7590 | 1031.5034 | 1031.4998 | 3.49 | C | 0 | 14 | 0.044 | ESIAKF +dK_MMCCH-2 (K) | 1017 |
| 82 | 1072 | 1087 | 744.7218 | 2231.1436 | 2231.1409 | 1.21 | C | 1 | 16 | 0.024 | TKRIEHLPHLISDATY +dK_MMCCH-2 (K) | 1073 |
| 83 | 1330 | 1337 | 620.3563 | 1238.6980 | 1238.6944 | 2.83 | C | 1 | 24 | 0.0036 | KLDITKAL +dK_MMCCH-2 (K) | 1335 |
| 84 | 1649 | 1664 | 752.3297 | 2253.9673 | 2253.9637 | 1.60 | C | 2 | 18 | 0.015 | NLNDHTHDFSKPEDTF +dK_MMCCH-2 (K) | 1659 |
| 85 | 1732 | 1742 | 792.8754 | 1583.7363 | 1583.7364 | -0.06 | C | 1 | 17 | 0.02 | TVLGGEKEMPW +dK_MMCCH-2 (K) | 1738 |
| 86 | 1818 | 1827 | 753.3691 | 1504.7237 | 1504.7232 | 0.40 | C | 1 | 21 | 0.0074 | SERDIGSLKY +dK_MMCCH-2 (K) | 1826 |
| 87 | 2160 | 2184 | 1085.5110 | 3253.5111 | 3253.5179 | -2.09 | C | 3 | 18 | 0.015 | KYDITHALHDAHITPEDVFHPSEPF +dK_MMCCH-2 (K) | 2160 |
| 88 | 2476 | 2488 | 923.9221 | 1845.8296 | 1845.8244 | 2.82 | C | 1 | 22 | 0.0062 | THSNAKPTDVFEY +dK_MMCCH-2 (K) | 2481 |
| 89 | 2656 | 2666 | 793.3529 | 1584.6913 | 1584.6879 | 2.15 | C | 1 | 29 | 0.0013 | KLQNDESHGGY +dK_MMCCH-2 (K) | 2656 |
| 90 | 2738 | 2749 | 856.3752 | 1710.7358 | 1710.7348 | 0.58 | C | 2 | 19 | 0.012 | ADSGNNNPFFKY +dK_MMCCH-2 (K) | 2748 |
| 91 | 2750 | 2779 | 1289.6239 | 3865.8499 | 3865.8621 | -3.18 | C | 3 | 22 | 0.0058 | HIRSINQDTVRDVNEAIFQQTKFGEFSSIF +2 Deamidated (NQ); dK_MMCCH-2 (K) | 2771 |
| 92 | 2799 | 2815 | 744.3796 | 2230.1169 | 2230.1092 | 3.45 | C | 2 | 24 | 0.0042 | EILHNEVHALIGGAEKY +dK_MMCCH-2 (K) | 2814 |
| 93 | 2946 | 2960 | 965.4055 | 1928.7965 | 1928.7986 | -1.09 | C | 1 | 26 | 0.0023 | IKSGTDSDDEYAGSF +dK_MMCCH-2 (K) | 2947 |
| 94 | 2961 | 2971 | 769.8917 | 1537.7689 | 1537.7673 | 1.04 | C | 1 | 17 | 0.019 | VILGGAKEMPW +dK_MMCCH-2 (K) | 2967 |
| 95 | 1 | 8 | 643.8054 | 1285.5963 | 1285.5934 | 2.26 | Th | 1 | 22 | 0.006 | MTPEELKT +dK_MMCCH-2 (K) | 7 |
| 96 | 38 | 56 | 861.6811 | 2582.0214 | 2582.0108 | 4.11 | Th | 2 | 14 | 0.041 | VCIPDDNDRNDDHCEKAGD +dK_MMCCH-2 (K) | 53 |
| 97 | 59 | 69 | 785.3774 | 1568.7402 | 1568.7367 | 2.23 | Th | 3 | 55 | 2.90E-06 | VLGGPSEMKWQ +dK_MMCCH-2 (K) | 67 |
| 98 | 78 | 84 | 569.2881 | 1136.5616 | 1136.5536 | 7.04 | Th | 1 | 47 | 2.10E-05 | LSDTVHK +dK_MMCCH-2 (K) | 84 |
| 99 | 117 | 124 | 620.8133 | 1239.6120 | 1239.6070 | 4.03 | Th | 1 | 23 | 0.0052 | VVHHPEKG +dK_MMCCH-2 (K) | 123 |
| 100 | 431 | 438 | 642.3345 | 1282.6545 | 1282.6479 | 5.15 | Th | 2 | 29 | 0.0013 | ISLENIEK +dK_MMCCH-2 (K) | 438 |
| 101 | 521 | 530 | 707.8455 | 1413.6764 | 1413.6698 | 4.67 | Th | 2 | 74 | 4.30E-08 | VDITEVDGTK +dK_MMCCH-2 (K) | 530 |
| 102 | 585 | 591 | 585.8113 | 1169.6080 | 1169.6002 | 6.67 | Th | 1 | 35 | 0.00032 | LLKEDKS +dK_MMCCH-2 (K) | 590 |
| 103 | 601 | 612 | 904.8973 | 1807.7801 | 1807.7698 | 5.70 | Th | 1 | 22 | 0.0058 | FHGEPKWCPSPE +dK_MMCCH-2 (K) | 606 |

Figure 22A. KLH 1 (continued)

| | | | | | | | | | | | | |
|---|---|---|---|---|---|---|---|---|---|---|---|---|
| 104 | 666 | 672 | 607.2841 | 1212.5537 | 1212.5485 | 4.29 | Th | 2 | 24 | 0.0043 | LADHEKY +dK_MMCCH-2 (K) | 671 |
| 105 | 673 | 685 | 930.4190 | 1858.8235 | 1858.8196 | 2.10 | Th | 2 | 15 | 0.035 | VDPEDGVEKHNPW +dK_MMCCH-2 (K) | 681 |
| 106 | 821 | 828 | 617.8143 | 1233.6141 | 1233.6064 | 6.24 | Th | 1 | 14 | 0.036 | VTKEHSVP +dK_MMCCH-2 (K) | 823 |
| 107 | 831 | 837 | 612.7885 | 1223.5623 | 1223.5533 | 7.44 | Th | 2 | 18 | 0.016 | VFDYKTN +dK_MMCCH-2 (K) | 835 |
| 108 | 954 | 959 | 529.7656 | 1057.5167 | 1057.5154 | 1.23 | Th | 1 | 13 | 0.048 | LFKQPS +Deamidated (NQ); dK_MMCCH-2 (K) | 956 |
| 109 | 1100 | 1104 | 482.2296 | 962.4447 | 962.4433 | 1.45 | Th | 0 | 18 | 0.015 | FHHGK +dK_MMCCH-2 (K) | 1104 |
| 110 | 1219 | 1232 | 653.9534 | 1958.8383 | 1958.8316 | 3.42 | Th | 1 | 21 | 0.0077 | FDKSDNNDEATKTH +dK_MMCCH-2 (K) | 1230 |
| 111 | 1580 | 1587 | 572.7740 | 1143.5334 | 1143.5271 | 5.60 | Th | 1 | 15 | 0.029 | VGGKEPYG +dK_MMCCH-2 (K) | 1583 |
| 112 | 1657 | 1668 | 921.9191 | 1841.8236 | 1841.8182 | 2.93 | Th | 2 | 32 | 0.00063 | FSKPEDTFDYQK +dK_MMCCH-2 (K) | 1659 |
| 113 | 1657 | 1668 | 921.9192 | 1841.8238 | 1841.8182 | 3.04 | Th | 2 | 46 | 2.70E-05 | FSKPEDTFDYQK +dK_MMCCH-2 (K) | 1668 |
| 114 | 1733 | 1741 | 664.3235 | 1326.6325 | 1326.6312 | 1.06 | Th | 2 | 30 | 0.00096 | VLGGEKEMP +dK_MMCCH-1 (K); Oxidation (M) | 1738 |
| 115 | 1894 | 1904 | 850.4174 | 1698.8203 | 1698.8116 | 5.12 | Th | 5 | 40 | 9.40E-05 | VAVPYWDWTKP +dK_MMCCH-2 (K) | 1903 |
| 116 | 2064 | 2068 | 499.7604 | 997.5061 | 997.5055 | 0.60 | Th | 0 | 17 | 0.02 | FTKKH +dK_MMCCH-2 (K) | 2067 |
| 117 | 2094 | 2100 | 618.3057 | 1234.5969 | 1234.5903 | 5.35 | Th | 1 | 30 | 0.001 | LHEIEKE +dK_MMCCH-2 (K) | 2099 |
| 118 | 2129 | 2141 | 933.8789 | 1865.7433 | 1865.7495 | -3.32 | Th | 0 | 19 | 0.012 | ICKTSEDCHHGGQ +dK_MMCCH-2 (K) | 2131 |
| 119 | 2238 | 2248 | 778.8803 | 1555.7459 | 1555.7440 | 1.29 | Th | 3 | 43 | 5.00E-05 | LTTAEVDNLKD +dK_MMCCH-2 (K) | 2247 |
| 120 | 2480 | 2487 | 622.7991 | 1243.5836 | 1243.5795 | 3.30 | Th | 2 | 13 | 0.046 | AKPTDVFE +dK_MMCCH-2 (K) | 2481 |
| 121 | 2512 | 2521 | 807.3992 | 1612.7838 | 1612.7766 | 4.40 | Th | 0 | 32 | 0.00068 | LEKQKEEDRT +dK_MMCCH-2 (K) | 2514 |
| 122 | 2595 | 2606 | 806.4045 | 1610.7945 | 1610.7862 | 5.15 | Th | 2 | 26 | 0.0026 | IIDTSGKQLPSD +dK_MMCCH-2 (K) | 2601 |
| 123 | 2607 | 2612 | 520.7803 | 1039.5461 | 1039.5446 | 1.44 | Th | 2 | 22 | 0.0057 | LIKMPT +dK_MMCCH-2 (K) | 2609 |
| 124 | 2766 | 2771 | 551.7865 | 1101.5584 | 1101.5529 | 5.08 | Th | 1 | 27 | 0.002 | IFQQTK +dK_MMCCH-2 (K) | 2771 |
| 125 | 2808 | 2816 | 638.3196 | 1274.6247 | 1274.6217 | 2.43 | Th | 3 | 21 | 0.0084 | LIGGAEKYS +dK_MMCCH-2 (K) | 2814 |
| 126 | 2946 | 2960 | 965.4089 | 1928.8032 | 1928.7986 | 2.38 | Th | 3 | 34 | 0.0004 | IKSGTDSDDEYAGSF +dK_MMCCH-2 (K) | 2947 |
| 127 | 2990 | 2996 | 583.2845 | 1164.5545 | 1164.5485 | 5.15 | Th | 1 | 38 | 0.00017 | LTDDHVK +dK_MMCCH-2 (K) | 2996 |

Figure 22B. KLH 2

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1043.0238 | 2084.0330 | 2084.0347 | -0.82 | T | 1 | 70 | 9.50E-08 | KNVDSLSSDEVLALEK +dK_MMCCH-2 (K) | 26 |
| 2 | 147 | 155 | 694.3724 | 1386.7302 | 1386.7217 | 6.13 | T | 1 | 27 | 0.0018 | ADITFLNKK +dK_MMCCH-2 (K) | 155 |
| 3 | 164 | 175 | 596.3090 | 1785.9051 | 1785.8985 | 3.70 | T | 1 | 37 | 0.00018 | LFEKVQPGHHTR +dK_MMCCH-2 (K) | 167 |
| 4 | 249 | 271 | 987.4413 | 2959.3020 | 2959.3051 | -1.05 | T | 1 | 32 | 0.00058 | GKDPNSADCAHNLIHTPMEPFDR +dK_MMCCH-2 (K) | 250 |
| 5 | 281 | 293 | 610.9457 | 1829.8152 | 1829.8043 | 5.96 | T | 0 | 54 | 3.90E-06 | EHAKPADSFDYGR +dK_MMCCH-2 (K) | 284 |
| 6 | 419 | 446 | 677.3701 | 3381.8139 | 3381.8132 | 0.21 | T | 1 | 45 | 3.20E-05 | QPTLVHRPAKGHFDKPPVPVAQANLAVR +Deamidated (NQ); dK_MMCCH-2 (K) | 428 |
| 7 | 429 | 446 | 752.0657 | 2253.1752 | 2253.1729 | 1.02 | T | 0 | 52 | 5.70E-06 | GHFDKPPVPVAQANLAVR +dK_MMCCH-2 (K) | 433 |
| 8 | 467 | 488 | 944.4583 | 2830.3529 | 2830.3385 | 5.12 | T | 1 | 71 | 8.30E-08 | FQNDKSVDGYQATVEFHALPAR +dK_MMCCH-2 (K) | 471 |
| 9 | 788 | 795 | 718.8198 | 1435.6251 | 1435.6377 | -8.78 | T | 1 | 15 | 0.029 | EMKWGFDR +dK_MMCCH-1 (K); Oxidation (M) | 790 |
| 10 | 936 | 950 | 1080.0140 | 2158.0135 | 2158.0095 | 1.85 | T | 1 | 51 | 8.20E-06 | KHGFTGGLPYWDWTR +dK_MMCCH-2 (K) | 936 |
| 11 | 995 | 1052 | 1170.7135 | 7018.2375 | 7018.2306 | 0.98 | T | 1 | 15 | 0.032 | DDLYQSPGFGHYTDIAKQVLLAFEQDDFCDFEVQFEIAHNFIHALVGGNEPYSMSSLR +dK_MMCCH-2 (K) | 1011 |
| 12 | 1082 | 1096 | 996.4618 | 1990.9090 | 1990.9063 | 1.36 | T | 0 | 77 | 2.00E-08 | GKPYNTANCAIASMR +dK_MMCCH-2 (K) | 1083 |
| 13 | 1097 | 1115 | 827.0768 | 2478.2087 | 2478.2101 | -0.56 | T | 0 | 84 | 3.90E-09 | KPLQPFGLDSVINPDDETR +dK_MMCCH-2 (K) | 1097 |
| 14 | 1123 | 1149 | 1190.5595 | 3568.6565 | 3568.6497 | 1.93 | T | 1 | 73 | 5.50E-08 | VFDYKNNFDYEYESLAFNGLSIAQLDR +dK_MMCCH-2 (K) | 1127 |
| 15 | 1295 | 1311 | 764.0672 | 2289.1798 | 2289.1715 | 3.63 | T | 1 | 17 | 0.021 | SAFLQIQKEGIYENIAK +dK_MMCCH-2 (K) | 1302 |
| 16 | 1355 | 1385 | 1271.2711 | 3810.7915 | 3810.7876 | 1.05 | T | 1 | 44 | 3.90E-05 | GSAVAVPYWDWTEKADSLPSLINDATYFNSR +dK_MMCCH-2 (K) | 1368 |
| 17 | 1409 | 1450 | 1097.7112 | 5483.5195 | 5483.5213 | -0.33 | T | 1 | 33 | 0.00047 | DPQPELWDNKDFYENVMLALEQDNFCDFEIQLELIHNALHSR +dK_MMCCH-2 (K) | 1418 |
| 18 | 1455 | 1480 | 1117.8726 | 3350.5959 | 3350.6070 | -3.34 | T | 1 | 63 | 5.50E-07 | AKYSLSSLDYTAFDPVFFLHHANVDR +dK_MMCCH-2 (K) | 1456 |
| 19 | 1493 | 1507 | 721.6653 | 2161.9742 | 2161.9594 | 6.80 | T | 1 | 59 | 1.20E-06 | KKPYNEADCAVNEMR +dK_MMCCH-2 (K) | 1493 |
| 20 | 1493 | 1507 | 721.6612 | 2161.9617 | 2161.9594 | 1.06 | T | 1 | 70 | 9.40E-08 | KKPYNEADCAVNEMR +dK_MMCCH-2 (K) | 1494 |
| 21 | 1508 | 1526 | 837.7524 | 2510.2353 | 2510.2185 | 6.69 | T | 0 | 20 | 0.01 | KPLQPFNNPELNSDSMTLK +dK_MMCCH-2 (K) | 1508 |
| 22 | 1508 | 1539 | 825.9995 | 4124.9612 | 4124.9360 | 6.08 | T | 1 | 23 | 0.0047 | KPLQPFNNPELNSDSMTLKHNLPQDSFDYQNR +dK_MMCCH-2 (K) | 1526 |
| 23 | 1542 | 1564 | 802.8925 | 3207.5407 | 3207.5448 | -1.25 | T | 1 | 19 | 0.012 | YQYDNLQFNHFSIQKLDQTIQAR +dK_MMCCH-2 (K) | 1556 |
| 24 | 1622 | 1633 | 591.9809 | 1772.9209 | 1772.9171 | 2.14 | T | 1 | 18 | 0.015 | LYKFDITSALHK +dK_MMCCH-2 (K) | 1624 |

**Figure 22B. KLH 2 (continued)**

| | | | | | | | | | | | | |
|---|---|---|---|---|---|---|---|---|---|---|---|---|
| 25 | 1690 | 1700 | 786.9194 | 1571.8243 | 1571.8116 | 8.08 | T | 1 | 13 | 0.049 | KEVSSLTTLEK +dK_MMCCH-2 (K) | 1690 |
| 26 | 1690 | 1704 | 532.2905 | 2125.1328 | 2125.1241 | 4.05 | T | 2 | 28 | 0.0015 | KEVSSLTTLEKHFLR +dK_MMCCH-2 (K) | 1700 |
| 27 | 1757 | 1768 | 612.9786 | 1835.9141 | 1835.9128 | 0.71 | T | 1 | 19 | 0.013 | LYTVQFEDSLKR +dK_MMCCH-2 (K) | 1767 |
| 28 | 1769 | 1803 | 1108.3084 | 4429.2045 | 4429.1882 | 3.70 | T | 0 | 28 | 0.0015 | HGSIVGLPYWDWLKPQSALPDLVTQETYEHLFSHK +dK_MMCCH-2 (K) | 1803 |
| 29 | 1804 | 1825 | 945.4654 | 2833.3743 | 2833.3633 | 3.92 | T | 1 | 23 | 0.0047 | TFPNPFLKANIEFEGEGVTTER +dK_MMCCH-2 (K) | 1811 |
| 30 | 1949 | 1963 | 560.2515 | 2236.9767 | 2236.9735 | 1.43 | T | 0 | 57 | 1.90E-06 | TQEFSKPEDTFDYHR +dK_MMCCH-2 (K) | 1954 |
| 31 | 2045 | 2052 | 660.3513 | 1318.6880 | 1318.6843 | 2.81 | T | 1 | 18 | 0.015 | YDITKTLK +dK_MMCCH-2 (K) | 2049 |
| 32 | 2116 | 2125 | 715.3626 | 1428.7107 | 1428.7105 | 0.14 | T | 1 | 15 | 0.029 | DLASLKSAMR +dK_MMCCH-2 (K) | 2121 |
| 33 | 2228 | 2239 | 870.4226 | 1738.8305 | 1738.8236 | 3.97 | T | 1 | 28 | 0.0017 | GYIKSEDAYTVR +dK_MMCCH-2 (K) | 2231 |
| 34 | 2453 | 2463 | 839.9324 | 1677.8502 | 1677.8436 | 3.93 | T | 1 | 17 | 0.021 | NFKYDITQALK +dK_MMCCH-2 (K) | 2455 |
| 35 | 2527 | 2541 | 1007.5171 | 2013.0196 | 2013.0088 | 5.37 | T | 1 | 86 | 2.60E-09 | KDVTSLTASEIENLR +dK_MMCCH-2 (K) | 2527 |
| 36 | 2728 | 2739 | 957.9996 | 1913.9846 | 1913.9862 | -0.84 | T | 1 | 32 | 0.00059 | IWAIWQALQKYR +Deamidated (NQ); dK_MMCCH-2 (K) | 2737 |
| 37 | 2755 | 2764 | 764.3816 | 1526.7486 | 1526.7439 | 3.08 | T | 0 | 23 | 0.0053 | QPLKPFSESR +Deamidated (NQ); dK_MMCCH-2 (K) | 2758 |
| 38 | 3148 | 3155 | 629.3348 | 1256.6551 | 1256.6587 | -2.86 | T | 1 | 15 | 0.032 | KKPYNAAK +dK_MMCCH-2 (K) | 3149 |
| 39 | 167 | 178 | 590.9649 | 1769.8729 | 1769.8705 | 1.30 | G | 0 | 44 | 4.00E-05 | KVQPGHHTRLME +dK_MMCCH-2 (K) | 167 |
| 40 | 318 | 322 | 456.7347 | 911.4549 | 911.4535 | 1.54 | G | 0 | 17 | 0.021 | RAAKE +dK_MMCCH-2 (K) | 321 |
| 41 | 433 | 456 | 975.8586 | 2924.5539 | 2924.5429 | 3.76 | G | 2 | 20 | 0.011 | KPPVPVAQANLAVRKNINDLTAEE +dK_MMCCH-2 (K) | 433 |
| 42 | 466 | 481 | 1098.4976 | 2194.9806 | 2194.9841 | -1.59 | G | 2 | 29 | 0.0013 | RFQNDKSVDGYQATVE +Deamidated (NQ); dK_MMCCH-2 (K) | 471 |
| 43 | 1215 | 1235 | 721.6069 | 2882.3986 | 2882.3949 | 1.28 | G | 3 | 23 | 0.0054 | ITQQLHDLDLHVGDNFFLKYE +dK_MMCCH-2 (K) | 1233 |
| 44 | 1292 | 1303 | 879.4658 | 1756.9170 | 1756.9182 | -0.68 | G | 0 | 49 | 1.20E-05 | SIRSAFLQIQKE +dK_MMCCH-2 (K) | 1302 |
| 45 | 1308 | 1320 | 904.9478 | 1807.8811 | 1807.8749 | 3.37 | G | 0 | 23 | 0.0051 | NIAKFHGKPGLCE +dK_MMCCH-2 (K) | 1311 |
| 46 | 1308 | 1320 | 603.6335 | 1807.8786 | 1807.8749 | 2.05 | G | 0 | 28 | 0.0014 | NIAKFHGKPGLCE +dK_MMCCH-2 (K) | 1315 |
| 47 | 1403 | 1422 | 921.4202 | 2761.2387 | 2761.2330 | 2.06 | G | 3 | 19 | 0.012 | NAVTSRDPQPELWDNKDFYE +dK_MMCCH-2 (K) | 1418 |
| 48 | 1952 | 1967 | 792.6824 | 2375.0255 | 2375.0205 | 2.11 | G | 3 | 35 | 0.00028 | FSKPEDTFDYHRFGYE +dK_MMCCH-2 (K) | 1954 |
| 49 | 1973 | 1990 | 811.7271 | 2432.1595 | 2432.1505 | 3.74 | G | 0 | 16 | 0.023 | FVGMSVSSLHNYIKQQQE +dK_MMCCH-2 (K) | 1986 |
| 50 | 2477 | 2484 | 644.8342 | 1287.6538 | 1287.6573 | -2.72 | G | 0 | 25 | 0.0034 | APFFIKVE +dK_MMCCH-2 (K) | 2482 |
| 51 | 3386 | 3405 | 877.4154 | 2629.2244 | 2629.2271 | -1.03 | G | 2 | 29 | 0.0013 | LDHAYSLRDGHYYIAGPTKD +dK_MMCCH-2 (K) | 3404 |
| 52 | 38 | 55 | 1181.0338 | 2360.0531 | 2360.0478 | 2.25 | C | 3 | 27 | 0.0019 | ALEKALDDLQQDDSNQGY +dK_MMCCH-2 (K) | 41 |

**Figure 22B. KLH 2 (continued)**

| | | | | | | | | | | | | |
|---|---|---|---|---|---|---|---|---|---|---|---|---|
| 53 | 152 | 165 | 667.6938 | 2000.0595 | 2000.0513 | 4.10 | C | 2 | 14 | 0.041 | LNKKTSRAVDDRLF +dK_MMCCH-2 (K) | 154 |
| 54 | 166 | 176 | 547.2856 | 1638.8349 | 1638.8300 | 2.99 | C | 0 | 21 | 0.008 | EKVQPGHHTRL +dK_MMCCH-2 (K) | 167 |
| 55 | 277 | 296 | 883.7525 | 2648.2357 | 2648.2329 | 1.02 | C | 4 | 45 | 3.50E-05 | DLTREHAKPADSFDYGRLGY +dK_MMCCH-2 (K) | 284 |
| 56 | 364 | 373 | 735.3609 | 1468.7072 | 1468.7094 | -1.50 | C | 2 | 22 | 0.0056 | LLGGPTEMKW +dK_MMCCH-2 (K) | 372 |
| 57 | 629 | 635 | 539.2520 | 1076.4895 | 1076.4848 | 4.27 | C | 0 | 26 | 0.0027 | VGGSEKY +dK_MMCCH-2 (K) | 634 |
| 58 | 1007 | 1015 | 669.8715 | 1337.7284 | 1337.7265 | 1.42 | C | 1 | 26 | 0.0024 | TDIAKQVLL +dK_MMCCH-2 (K) | 1011 |
| 59 | 1075 | 1080 | 544.7778 | 1087.5410 | 1087.5372 | 3.49 | C | 1 | 26 | 0.0025 | QALQKY +dK_MMCCH-2 (K) | 1079 |
| 60 | 1125 | 1139 | 1128.4790 | 2254.9434 | 2254.9405 | 1.33 | C | 5 | 18 | 0.016 | DYKNNFDYEYESLAF +dK_MMCCH-2 (K) | 1127 |
| 61 | 1298 | 1312 | 824.0845 | 2469.2316 | 2469.2324 | -0.32 | C | 2 | 26 | 0.0023 | LQIQKEGIYENIAKF +2 dK_MMCCH-2 (K) | 1302, 1311 |
| 62 | 1298 | 1312 | 711.3758 | 2131.1055 | 2131.1023 | 1.50 | C | 2 | 27 | 0.0021 | LQIQKEGIYENIAKF +dK_MMCCH-2 (K) | 1311 |
| 63 | 1526 | 1534 | 712.3406 | 1422.6667 | 1422.6602 | 4.57 | C | 0 | 15 | 0.03 | KHNLPQDSF +dK_MMCCH-2 (K) | 1526 |
| 64 | 1589 | 1606 | 784.3575 | 2350.0506 | 2350.0392 | 4.85 | C | 0 | 13 | 0.05 | ICVEQGGEQNCKTKAGSF +dK_MMCCH-2 (K) | 1600 |
| 65 | 1589 | 1606 | 784.3556 | 2350.0451 | 2350.0392 | 2.51 | C | 0 | 32 | 0.00069 | ICVEQGGEQNCKTKAGSF +dK_MMCCH-2 (K) | 1602 |
| 66 | 1696 | 1702 | 607.3020 | 1212.5894 | 1212.5849 | 3.71 | C | 1 | 20 | 0.011 | TTLEKHF +dK_MMCCH-2 (K) | 1700 |
| 67 | 1708 | 1719 | 839.8415 | 1677.6685 | 1677.6651 | 2.09 | C | 0 | 17 | 0.019 | KNMQADDSPDGY +dK_MMCCH-2 (K) | 1708 |
| 68 | 1782 | 1796 | 1014.5055 | 2026.9964 | 2026.9921 | 2.12 | C | 1 | 31 | 0.00075 | KPQSALPDLVTQETY +dK_MMCCH-2 (K) | 1782 |
| 69 | 1801 | 1809 | 706.8381 | 1411.6617 | 1411.6595 | 1.56 | C | 0 | 16 | 0.024 | SHKTFPNPF +dK_MMCCH-2 (K) | 1803 |
| 70 | 1953 | 1964 | 627.2849 | 1878.8327 | 1878.8247 | 4.26 | C | 2 | 18 | 0.017 | SKPEDTFDYHRF +dK_MMCCH-2 (K) | 1954 |
| 71 | 1985 | 1995 | 850.4291 | 1698.8437 | 1698.8399 | 2.24 | C | 0 | 30 | 0.0011 | IKQQQEADRVF +dK_MMCCH-2 (K) | 1986 |
| 72 | 2001 | 2011 | 726.8453 | 1451.6761 | 1451.6755 | 0.41 | C | 1 | 21 | 0.008 | KGFGQSASVSF +dK_MMCCH-2 (K) | 2001 |
| 73 | 2455 | 2462 | 645.3257 | 1288.6369 | 1288.6373 | -0.31 | C | 1 | 24 | 0.0038 | KYDITQAL +dK_MMCCH-2 (K) | 2455 |
| 74 | 2463 | 2479 | 1128.0194 | 2254.0243 | 2254.0253 | -0.44 | C | 1 | 19 | 0.011 | KAQSIHPEDVFDTDAPF +dK_MMCCH-2 (K) | 2463 |
| 75 | 2506 | 2532 | 761.6026 | 3042.3813 | 3042.3738 | 2.47 | C | 0 | 24 | 0.0042 | SAGEGHTDDHGSDHIAGSGVRKDVTSL +dK_MMCCH-2 (K) | 2527 |
| 76 | 2754 | 2760 | 598.3314 | 1194.6483 | 1194.6471 | 1.00 | C | 1 | 17 | 0.022 | KQPLKPF +dK_MMCCH-2 (K) | 2754 |
| 77 | 2754 | 2760 | 598.3325 | 1194.6504 | 1194.6471 | 2.76 | C | 1 | 17 | 0.022 | KQPLKPF +dK_MMCCH-2 (K) | 2758 |
| 78 | 2865 | 2872 | 673.3662 | 1344.7179 | 1344.7112 | 4.98 | C | 1 | 22 | 0.0064 | RYDITKVL +dK_MMCCH-2 (K) | 2870 |
| 79 | 3235 | 3242 | 617.3142 | 1232.6139 | 1232.6111 | 2.27 | C | 0 | 30 | 0.0009 | TSANVKIY +dK_MMCCH-2 (K) | 3240 |
| 80 | 3398 | 3406 | 658.3369 | 1314.6593 | 1314.6530 | 4.79 | C | 1 | 15 | 0.029 | YIAGPTKDL +dK_MMCCH-2 (K) | 3404 |

**Figure 22B. KLH 2 (continued)**

| | | | | | | | | | | | | |
|---|---|---|---|---|---|---|---|---|---|---|---|---|
| 81 | 69 | 75 | 604.2940 | 1206.5735 | 1206.5703 | 2.65 | Th | 0 | 13 | 0.045 | VDKHEKN +dK_MMCCH-2 (K) | 74 |
| 82 | 184 | 191 | 703.8044 | 1405.5942 | 1405.5894 | 3.41 | Th | 1 | 43 | 4.90E-05 | LEQDEFCK +dK_MMCCH-2 (K) | 191 |
| 83 | 247 | 260 | 631.6201 | 1891.8384 | 1891.8305 | 4.12 | Th | 2 | 21 | 0.0078 | LRGKDPNSADCAHN +dK_MMCCH-2 (K) | 250 |
| 84 | 283 | 293 | 782.8576 | 1563.7006 | 1563.7028 | -1.34 | Th | 3 | 47 | 1.80E-05 | AKPADSFDYGR +dK_MMCCH-2 (K) | 284 |
| 85 | 364 | 374 | 763.8729 | 1525.7312 | 1525.7309 | 0.20 | Th | 3 | 33 | 0.00046 | LLGGPTEMKWG +dK_MMCCH-2 (K) | 372 |
| 86 | 629 | 636 | 582.7682 | 1163.5219 | 1163.5169 | 4.38 | Th | 1 | 20 | 0.01 | VGGSEKYS +dK_MMCCH-2 (K) | 634 |
| 87 | 893 | 904 | 904.8973 | 1807.7801 | 1807.7698 | 5.70 | Th | 1 | 22 | 0.0058 | FHGEPKWCPSPE +dK_MMCCH-2 (K) | 898 |
| 88 | 1362 | 1374 | 968.4312 | 1934.8479 | 1934.8397 | 4.24 | Th | 4 | 13 | 0.045 | YWDWTEKADSLPS +dK_MMCCH-2 (K) | 1368 |
| 89 | 1525 | 1539 | 738.3572 | 2212.0497 | 2212.0371 | 5.70 | Th | 3 | 36 | 0.00026 | LKHNLPQDSFDYQNR +dK_MMCCH-2 (K) | 1526 |
| 90 | 1589 | 1605 | 848.0453 | 2541.1142 | 2541.1008 | 5.27 | Th | 2 | 19 | 0.013 | ICVEQGGEQNCKTKAGS +2 dK_MMCCH-2 (K) | 1600, 1602 |
| 91 | 1589 | 1605 | 735.3340 | 2202.9801 | 2202.9708 | 4.22 | Th | 2 | 38 | 0.00015 | ICVEQGGEQNCKTKAGS +dK_MMCCH-2 (K) | 1602 |
| 92 | 1643 | 1648 | 537.7640 | 1073.5135 | 1073.5103 | 2.98 | Th | 2 | 14 | 0.037 | FDIKVD +dK_MMCCH-2 (K) | 1646 |
| 93 | 1800 | 1809 | 780.3752 | 1558.7359 | 1558.7279 | 5.13 | Th | 2 | 20 | 0.01 | FSHKTFPNPF +dK_MMCCH-2 (K) | 1803 |
| 94 | 1952 | 1963 | 627.2850 | 1878.8331 | 1878.8247 | 4.47 | Th | 2 | 55 | 3.40E-06 | FSKPEDTFDYHR +dK_MMCCH-2 (K) | 1954 |
| 95 | 1985 | 1993 | 727.3631 | 1452.7116 | 1452.7031 | 5.92 | Th | 1 | 21 | 0.0072 | IKQQQEADR +dK_MMCCH-2 (K) | 1986 |
| 96 | 2193 | 2199 | 667.3032 | 1332.5918 | 1332.5849 | 5.18 | Th | 2 | 24 | 0.0037 | YWDWTKP +dK_MMCCH-2 (K) | 2198 |
| 97 | 2200 | 2206 | 562.3082 | 1122.6018 | 1122.5995 | 2.05 | Th | 2 | 22 | 0.0062 | ISKLPDL +dK_MMCCH-2 (K) | 2202 |
| 98 | 2225 | 2229 | 462.2221 | 922.4297 | 922.4259 | 4.23 | Th | 2 | 21 | 0.0072 | FAKGY +dK_MMCCH-2 (K) | 2227 |
| 99 | 2359 | 2363 | 509.2315 | 1016.4485 | 1016.4460 | 2.46 | Th | 1 | 26 | 0.0028 | FTKMH +dK_MMCCH-2 (K); Oxidation (M) | 2361 |
| 100 | 2941 | 2950 | 720.8409 | 1439.6673 | 1439.6602 | 4.93 | Th | 3 | 45 | 3.00E-05 | LDEANDLKNA +dK_MMCCH-2 (K) | 2948 |
| 101 | 3009 | 3015 | 573.2771 | 1144.5396 | 1144.5335 | 5.33 | Th | 0 | 16 | 0.022 | LKEHGSH +dK_MMCCH-2 (K) | 3010 |
| 102 | 3399 | 3405 | 520.2615 | 1038.5084 | 1038.5056 | 2.70 | Th | 1 | 29 | 0.0011 | IAGPTKD +dK_MMCCH-2 (K) | 3404 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the lysine conjugation site for KLH1 or KLH2.

**Figure 23. Identification details of MMCCH-conjugated peptides for sample 2 (1st LC-MS/MS)**

**Figure 23A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 124 | 141 | 785.4092 | 2353.2059 | 2353.2001 | 2.46 | T | 1 | 25 | 0.0032 | GFTDPPVKHHQSANLLVR +dK_MMCCH-2 (K) | 131 |
| 2 | 142 | 149 | 656.3431 | 1310.6716 | 1310.6653 | 4.88 | T | 1 | 33 | 0.00048 | KNINDLTR +dK_MMCCH-2 (K) | 142 |
| 3 | 157 | 166 | 822.8833 | 1643.7520 | 1643.7402 | 7.18 | T | 1 | 18 | 0.014 | EAFHKFQEDR +dK_MMCCH-2 (K) | 161 |
| 4 | 184 | 192 | 726.8424 | 1451.6701 | 1451.6649 | 3.58 | T | 1 | 20 | 0.009 | CPRPDAKDR +dK_MMCCH-2 (K) | 190 |
| 5 | 553 | 560 | 663.8682 | 1325.7219 | 1325.7166 | 4.00 | T | 2 | 20 | 0.011 | VKFDKVPR +dK_MMCCH-2 (K) | 554 |
| 6 | 566 | 570 | 485.2449 | 968.4752 | 968.4749 | 0.31 | T | 1 | 16 | 0.025 | KNVDR +dK_MMCCH-2 (K) | 566 |
| 7 | 581 | 590 | 733.9201 | 1465.8256 | 1465.8214 | 2.93 | T | 2 | 24 | 0.0038 | KALALLKEDK +dK_MMCCH-2 (K) | 581 |
| 8 | 588 | 606 | 781.0359 | 2340.0858 | 2340.0845 | 0.60 | T | 1 | 26 | 0.0025 | EDKSAGGFQQLGAFHGEPK +dK_MMCCH-2 (K) | 590 |
| 9 | 645 | 681 | 1158.5337 | 4630.1057 | 4630.1063 | -0.15 | T | 1 | 25 | 0.0032 | HGYDGALPYWDWTSPLNHLPELADHEKYVDPEDGVEK +dK_MMCCH-2 (K) | 671 |
| 10 | 682 | 699 | 620.2917 | 2477.1377 | 2477.1434 | -2.34 | T | 1 | 18 | 0.015 | HNPWFDGHIDTVDKTTTR +dK_MMCCH-2 (K) | 695 |
| 11 | 700 | 719 | 887.7701 | 2660.2886 | 2660.2945 | -2.22 | T | 1 | 48 | 1.60E-05 | SVQNKLFEQPEFGHYTSIAK +dK_MMCCH-2 (K) | 704 |
| 12 | 778 | 789 | 958.0007 | 1913.9869 | 1913.9862 | 0.37 | T | 1 | 22 | 0.0059 | IWAIWQALQKYR +Deamidated (NQ); dK_MMCCH-2 (K) | 787 |
| 13 | 790 | 804 | 1003.4695 | 2004.9244 | 2004.9220 | 1.20 | T | 0 | 56 | 2.60E-06 | GKPYNVANCAVTSMR +dK_MMCCH-2 (K) | 791 |
| 14 | 858 | 863 | 520.2990 | 1038.5835 | 1038.5783 | 4.91 | T | 1 | 16 | 0.024 | KLEAIK +dK_MMCCH-2 (K) | 858 |
| 15 | 953 | 965 | 635.3314 | 1902.9723 | 1902.9662 | 3.15 | T | 1 | 54 | 3.60E-06 | DLFKQPSVIHEPR +dK_MMCCH-2 (K) | 956 |
| 16 | 986 | 1000 | 1027.0338 | 2052.0531 | 2052.0561 | -1.46 | T | 1 | 51 | 8.60E-06 | KNIENLSLGELESLR +dK_MMCCH-2 (K) | 986 |
| 17 | 1048 | 1060 | 913.5288 | 1825.0431 | 1825.0423 | 0.44 | T | 1 | 37 | 0.00021 | LYVVVVENALLKK +dK_MMCCH-2 (K) | 1060 |
| 18 | 1092 | 1114 | 791.8768 | 3163.4780 | 3163.4682 | 3.07 | T | 1 | 38 | 0.00017 | QHHYETNPFHHGKITHENEITTR +dK_MMCCH-2 (K) | 1104 |
| 19 | 1199 | 1203 | 353.1911 | 1056.5514 | 1056.5538 | -2.37 | T | 1 | 17 | 0.019 | KRPYR +dK_MMCCH-2 (K) | 1199 |
| 20 | 1331 | 1338 | 620.3545 | 1238.6944 | 1238.6944 | 0.00 | T | 1 | 24 | 0.0039 | LDITKALK +dK_MMCCH-2 (K) | 1335 |
| 21 | 1395 | 1402 | 649.8365 | 1297.6585 | 1297.6588 | -0.15 | T | 1 | 21 | 0.0078 | KDITQLDK +dK_MMCCH-2 (K) | 1395 |
| 22 | 1473 | 1489 | 778.4027 | 2332.1863 | 2332.1827 | 1.54 | T | 1 | 47 | 2.00E-05 | KHGAVVGLPYWDWTLPR +dK_MMCCH-2 (K) | 1473 |
| 23 | 1721 | 1746 | 1087.4764 | 3259.4075 | 3259.3937 | 4.23 | T | 1 | 13 | 0.047 | TAGDCEDAGYFTVLGGEKEMPWAFDR +dK_MMCCH-2 (K) | 1738 |
| 24 | 1747 | 1758 | 613.9770 | 1838.9091 | 1838.9012 | 4.35 | T | 1 | 21 | 0.0079 | LYKYDITETLDK +dK_MMCCH-2 (K) | 1749 |

Figure 23A. KLH 1 (continued)

| | | | | | | | | | | | | |
|---|---|---|---|---|---|---|---|---|---|---|---|---|
| 25 | 1923 | 1932 | 756.3808 | 1510.7470 | 1510.7425 | 3.04 | T | 1 | 27 | 0.0019 | NKVMPNPFAR +dK_MMCCH-2 (K) | 1924 |
| 26 | 2100 | 2109 | 492.2468 | 1964.9580 | 1964.9601 | -1.07 | T | 2 | 19 | 0.014 | EIKDKQHHVR +2 dK_MMCCH-2 (K) | 2102, 2104 |
| 27 | 2121 | 2149 | 1186.8922 | 3557.6548 | 3557.6378 | 4.78 | T | 1 | 46 | 2.80E-05 | TSADVQFQICKTSEDCHHGGQIFVLGGTK +dK_MMCCH-2 (K) | 2131 |
| 28 | 2359 | 2367 | 736.3499 | 1470.6853 | 1470.6813 | 2.72 | T | 1 | 35 | 0.00031 | DKLFNDPER +dK_MMCCH-2 (K) | 2360 |
| 29 | 2507 | 2514 | 682.3525 | 1362.6905 | 1362.6853 | 3.82 | T | 1 | 27 | 0.0019 | KLEHELEK +dK_MMCCH-2 (K) | 2507 |
| 30 | 2515 | 2520 | 571.7622 | 1141.5099 | 1141.5074 | 2.19 | T | 1 | 18 | 0.017 | QKEEDR +dK_MMCCH-2 (K) | 2516 |
| 31 | 2595 | 2609 | 983.5306 | 1965.0467 | 1965.0493 | -1.27 | T | 1 | 21 | 0.0081 | IIDTSGKQLPSDLIK +dK_MMCCH-2 (K) | 2601 |
| 32 | 2621 | 2636 | 802.7113 | 2405.1121 | 2405.1083 | 1.54 | T | 1 | 55 | 3.00E-06 | HHEKHHEDHHEDILVR +dK_MMCCH-2 (K) | 2624 |
| 33 | 2637 | 2651 | 1069.5327 | 2137.0509 | 2137.0374 | 6.27 | T | 1 | 71 | 8.70E-08 | KNIHSLSHHEAEELR +dK_MMCCH-2 (K) | 2637 |
| 34 | 2761 | 2814 | 1324.8373 | 6619.1500 | 6619.1192 | 4.65 | T | 1 | 13 | 0.046 | DVNEAIFQQTKFGEFSSIFYLALQALEEDNYCDFEVQYEILHNEVHALIGGAEK +dK_MMCCH-2 (K) | 2771 |
| 35 | 2851 | 2883 | 1009.2160 | 4032.8349 | 4032.8346 | 0.07 | T | 0 | 30 | 0.001 | VKPAHAGSCAGDIMHVPLHPFNYESVNNDDFTR +dK_MMCCH-2 (K) | 2852 |
| 36 | 2897 | 2919 | 1066.5197 | 3196.5371 | 3196.5287 | 2.63 | T | 1 | 59 | 1.30E-06 | FNYKYDNLNLHGHNIEELEEVLR +dK_MMCCH-2 (K) | 2900 |
| 37 | 2999 | 3021 | 1106.2400 | 3315.6981 | 3315.6923 | 1.75 | T | 2 | 21 | 0.0071 | FDLKKYDHTELDASVLPAPIIVR +2 dK_MMCCH-2 (K) | 3002, 3003 |
| 38 | 3003 | 3021 | 825.7711 | 2474.2914 | 2474.2879 | 1.37 | T | 1 | 27 | 0.0022 | KYDHTELDASVLPAPIIVR +dK_MMCCH-2 (K) | 3003 |
| 39 | 123 | 151 | 616.6606 | 3693.9202 | 3693.9049 | 4.14 | G | 2 | 30 | 0.00099 | KGFTDPPVKHHQSANLLVRKNINDLTREE +dK_MMCCH-2 (K) | 123 |
| 40 | 123 | 151 | 616.6599 | 3693.9154 | 3693.9049 | 2.84 | G | 2 | 23 | 0.005 | KGFTDPPVKHHQSANLLVRKNINDLTREE +dK_MMCCH-2 (K) | 131 |
| 41 | 158 | 176 | 846.3828 | 2536.1266 | 2536.1329 | -2.48 | G | 3 | 24 | 0.0038 | AFHKFQEDRSVDGYQATAE +dK_MMCCH-2 (K) | 161 |
| 42 | 435 | 442 | 676.3245 | 1350.6344 | 1350.6312 | 2.37 | G | 1 | 18 | 0.015 | NIEKMIHE +dK_MMCCH-2 (K) | 438 |
| 43 | 526 | 544 | 777.7501 | 2330.2285 | 2330.2304 | -0.82 | G | 1 | 24 | 0.0044 | VDGTKLASSLIPHASVIRE +dK_MMCCH-2 (K) | 530 |
| 44 | 589 | 612 | 1102.8262 | 3305.4567 | 3305.4621 | -1.63 | G | 1 | 34 | 0.00039 | DKSAGGFQQLGAFHGEPKWCPSPE +2 dK_MMCCH-2 (K) | 590, 606 |
| 45 | 589 | 612 | 990.1141 | 2967.3206 | 2967.3320 | -3.84 | G | 1 | 23 | 0.0055 | DKSAGGFQQLGAFHGEPKWCPSPE +dK_MMCCH-2 (K) | 606 |
| 46 | 666 | 676 | 827.3729 | 1652.7313 | 1652.7392 | -4.78 | G | 2 | 14 | 0.038 | LADHEKYVDPE +dK_MMCCH-2 (K) | 671 |
| 47 | 825 | 841 | 820.3713 | 2458.0922 | 2458.0940 | -0.73 | G | 1 | 25 | 0.003 | HSVPFNVFDYKTNFNYE +dK_MMCCH-2 (K) | 835 |
| 48 | 847 | 860 | 643.6777 | 1928.0112 | 1928.0077 | 1.82 | G | 0 | 24 | 0.0043 | FNGLSISQLNKKLE +dK_MMCCH-2 (K) | 858 |
| 49 | 942 | 970 | 727.3804 | 3631.8658 | 3631.8973 | -8.70 | G | 2 | 18 | 0.016 | VFDLKPASLGKDLFKQPSVIHEPRIGHHE +dK_MMCCH-2 (K) | 956 |
| 50 | 1055 | 1076 | 810.1655 | 3236.6328 | 3236.6402 | -2.32 | G | 1 | 30 | 0.00092 | NALLKKGSSVAVPYWDWTKRIE +2 dK_MMCCH-2 (K) | 1059, 1060 |

Figure 23A. KLH 1 (continued)

| | | | | | | | | | | | | |
|---|---|---|---|---|---|---|---|---|---|---|---|---|
| 51 | 1055 | 1076 | 725.6326 | 2898.5012 | 2898.5102 | -3.11 | G | 1 | 37 | 0.0002 | NALLKKGSSVAVPYWDWTKRIE +dK_MMCCH-2 (K) | 1060 |
| 52 | 1097 | 1110 | 999.9615 | 1997.9085 | 1997.9054 | 1.60 | G | 1 | 22 | 0.0062 | TNPFHHGKITHENE +dK_MMCCH-2 (K) | 1104 |
| 53 | 1376 | 1396 | 864.3896 | 2590.1471 | 2590.1428 | 1.66 | G | 3 | 22 | 0.0067 | AGTDSAHTDDGHTEPVMIRKD +dK_MMCCH-2 (K) | 1395 |
| 54 | 1376 | 1413 | 915.2663 | 4571.2951 | 4571.2636 | 6.89 | G | 5 | 21 | 0.0079 | AGTDSAHTDDGHTEPVMIRKDITQLDKRQQLSLVKALE +Deamidated (NQ); dK_MMCCH-2 (K); Oxidation (M) | 1402 |
| 55 | 1397 | 1413 | 774.4324 | 2320.2753 | 2320.2824 | -3.06 | G | 1 | 22 | 0.006 | ITQLDKRQQLSLVKALE +dK_MMCCH-2 (K) | 1410 |
| 56 | 1506 | 1522 | 753.3741 | 2257.1004 | 2257.1089 | -3.77 | G | 2 | 21 | 0.0076 | TGRDIPNPFIGSKIEFE +dK_MMCCH-2 (K) | 1518 |
| 57 | 1755 | 1765 | 570.6044 | 1708.7915 | 1708.7913 | 0.12 | G | 2 | 27 | 0.0019 | TLDKMNLRHDE +dK_MMCCH-2 (K) | 1758 |
| 58 | 2089 | 2100 | 854.4117 | 1706.8088 | 1706.8008 | 4.75 | G | 2 | 41 | 7.40E-05 | IGGMNLHEIEKE +dK_MMCCH-2 (K) | 2099 |
| 59 | 2647 | 2661 | 715.6783 | 2144.0130 | 2144.0095 | 1.63 | G | 4 | 24 | 0.0043 | AEELRDALYKLQNDE +dK_MMCCH-2 (K) | 2656 |
| 60 | 3090 | 3097 | 632.8027 | 1263.5908 | 1263.5879 | 2.30 | G | 0 | 14 | 0.039 | LGKMYSVE +dK_MMCCH-2 (K) | 3092 |
| 61 | 59 | 68 | 721.3470 | 1440.6794 | 1440.6781 | 0.90 | C | 1 | 33 | 0.00045 | VLGGPSEMKW +dK_MMCCH-2 (K) | 67 |
| 62 | 160 | 171 | 909.9108 | 1817.8070 | 1817.8043 | 1.49 | C | 1 | 19 | 0.014 | HKFQEDRSVDGY +dK_MMCCH-2 (K) | 161 |
| 63 | 584 | 595 | 787.4013 | 1572.7879 | 1572.7858 | 1.40 | C | 2 | 36 | 0.00027 | ALLKEDKSAGGF +dK_MMCCH-2 (K) | 590 |
| 64 | 715 | 723 | 655.8733 | 1309.7320 | 1309.7316 | 0.38 | C | 1 | 15 | 0.032 | TSIAKQVLL +dK_MMCCH-2 (K) | 719 |
| 65 | 783 | 788 | 544.7770 | 1087.5394 | 1087.5372 | 2.02 | C | 1 | 18 | 0.017 | QALQKY +dK_MMCCH-2 (K) | 787 |
| 66 | 860 | 869 | 789.8865 | 1577.7584 | 1577.7548 | 2.28 | C | 1 | 13 | 0.045 | EAIKSQDRFF +dK_MMCCH-2 (K) | 863 |
| 67 | 944 | 955 | 821.4309 | 1640.8473 | 1640.8484 | -0.67 | C | 3 | 15 | 0.035 | DLKPASLGKDLF +dK_MMCCH-2 (K) | 946 |
| 68 | 1013 | 1018 | 516.7577 | 1031.5009 | 1031.4998 | 1.16 | C | 0 | 22 | 0.0065 | ESIAKF +dK_MMCCH-2 (K) | 1017 |
| 69 | 1330 | 1337 | 620.3552 | 1238.6958 | 1238.6944 | 1.05 | C | 1 | 21 | 0.0071 | KLDITKAL +dK_MMCCH-2 (K) | 1335 |
| 70 | 1515 | 1521 | 566.2921 | 1130.5697 | 1130.5682 | 1.33 | C | 0 | 17 | 0.022 | IGSKIEF +dK_MMCCH-2 (K) | 1518 |
| 71 | 1649 | 1664 | 752.3290 | 2253.9651 | 2253.9637 | 0.62 | C | 2 | 15 | 0.034 | NLNDHTHDFSKPEDTF +dK_MMCCH-2 (K) | 1659 |
| 72 | 1818 | 1827 | 753.3682 | 1504.7218 | 1504.7232 | -0.93 | C | 1 | 21 | 0.0077 | SERDIGSLKY +dK_MMCCH-2 (K) | 1826 |
| 73 | 2076 | 2082 | 612.3054 | 1222.5962 | 1222.5944 | 1.47 | C | 3 | 19 | 0.014 | KYELLGY +dK_MMCCH-2 (K) | 2076 |
| 74 | 2160 | 2184 | 1085.5089 | 3253.5049 | 3253.5179 | -4.00 | C | 3 | 32 | 0.00064 | KYDITHALHDAHITPEDVFHPSEPF +dK_MMCCH-2 (K) | 2160 |
| 75 | 2476 | 2488 | 923.9213 | 1845.8280 | 1845.8244 | 1.95 | C | 1 | 26 | 0.0025 | THSNAKPTDVFEY +dK_MMCCH-2 (K) | 2481 |
| 76 | 2656 | 2672 | 747.3419 | 2239.0039 | 2239.0004 | 1.56 | C | 2 | 30 | 0.001 | KLQNDESHGGYEHIAGF +dK_MMCCH-2 (K) | 2656 |
| 77 | 2738 | 2749 | 856.3748 | 1710.7350 | 1710.7348 | 0.12 | C | 2 | 21 | 0.0074 | ADSGNNNPFFKY +dK_MMCCH-2 (K) | 2748 |

Figure 23A. KLH 1 (continued)

| | | | | | | | | | | | | |
|---|---|---|---|---|---|---|---|---|---|---|---|---|
| 78 | 2946 | 2960 | 965.4052 | 1928.7957 | 1928.7986 | -1.50 | C | 1 | 21 | 0.0072 | IKSGTDSDDEYAGSF +dK_MMCCH-2 (K) | 2947 |
| 79 | 2964 | 2971 | 607.2726 | 1212.5306 | 1212.5307 | -0.08 | C | 0 | 17 | 0.02 | GGAKEMPW +dK_MMCCH-2 (K) | 2967 |
| 80 | 1 | 9 | 733.3323 | 1464.6500 | 1464.6516 | -1.09 | Th | 2 | 19 | 0.014 | MTPEELKTY +dK_MMCCH-2 (K); Oxidation (M) | 7 |
| 81 | 38 | 56 | 861.6808 | 2582.0205 | 2582.0108 | 3.76 | Th | 2 | 17 | 0.019 | VCIPDDNDRNDDHCEKAGD +dK_MMCCH-2 (K) | 53 |
| 82 | 59 | 69 | 785.3759 | 1568.7371 | 1568.7367 | 0.25 | Th | 3 | 52 | 6.90E-06 | VLGGPSEMKWQ +dK_MMCCH-2 (K) | 67 |
| 83 | 78 | 84 | 569.2864 | 1136.5583 | 1136.5536 | 4.14 | Th | 1 | 34 | 0.00041 | LSDTVHK +dK_MMCCH-2 (K) | 84 |
| 84 | 117 | 124 | 620.8127 | 1239.6109 | 1239.6070 | 3.15 | Th | 1 | 24 | 0.0044 | VVHHPEKG +dK_MMCCH-2 (K) | 123 |
| 85 | 431 | 439 | 715.8506 | 1429.6866 | 1429.6833 | 2.38 | Th | 3 | 16 | 0.027 | ISLENIEKM +dK_MMCCH-2 (K); Oxidation (M) | 438 |
| 86 | 521 | 530 | 707.8433 | 1413.6720 | 1413.6698 | 1.56 | Th | 2 | 69 | 1.10E-07 | VDITEVDGTK +dK_MMCCH-2 (K) | 530 |
| 87 | 585 | 591 | 585.8104 | 1169.6062 | 1169.6002 | 5.13 | Th | 1 | 35 | 0.00033 | LLKEDKS +dK_MMCCH-2 (K) | 590 |
| 88 | 601 | 612 | 904.8962 | 1807.7779 | 1807.7698 | 4.48 | Th | 1 | 18 | 0.018 | FHGEPKWCPSPE +dK_MMCCH-2 (K) | 606 |
| 89 | 666 | 672 | 607.2816 | 1212.5486 | 1212.5485 | 0.08 | Th | 2 | 23 | 0.0045 | LADHEKY +dK_MMCCH-2 (K) | 671 |
| 90 | 821 | 828 | 617.8116 | 1233.6086 | 1233.6064 | 1.78 | Th | 1 | 23 | 0.005 | VTKEHSVP +dK_MMCCH-2 (K) | 823 |
| 91 | 831 | 837 | 612.7867 | 1223.5588 | 1223.5533 | 4.50 | Th | 2 | 22 | 0.007 | VFDYKTN +dK_MMCCH-2 (K) | 835 |
| 92 | 1100 | 1104 | 482.2287 | 962.4428 | 962.4433 | -0.52 | Th | 0 | 20 | 0.0093 | FHHGK +dK_MMCCH-2 (K) | 1104 |
| 93 | 1111 | 1120 | 742.3818 | 1482.7491 | 1482.7388 | 6.95 | Th | 1 | 22 | 0.0066 | ITTRDPKDSL +dK_MMCCH-2 (K) | 1117 |
| 94 | 1156 | 1164 | 630.8400 | 1259.6654 | 1259.6584 | 5.56 | Th | 2 | 14 | 0.038 | LLGGKGKYS +dK_MMCCH-2 (K) | 1162 |
| 95 | 1219 | 1232 | 653.9541 | 1958.8405 | 1958.8316 | 4.54 | Th | 1 | 30 | 0.0011 | FDKSDNNDEATKTH +dK_MMCCH-2 (K) | 1221 |
| 96 | 1219 | 1232 | 980.4238 | 1958.8330 | 1958.8316 | 0.71 | Th | 1 | 37 | 0.0002 | FDKSDNNDEATKTH +dK_MMCCH-2 (K) | 1230 |
| 97 | 1580 | 1587 | 572.7732 | 1143.5319 | 1143.5271 | 4.28 | Th | 1 | 16 | 0.023 | VGGKEPYG +dK_MMCCH-2 (K) | 1583 |
| 98 | 1657 | 1668 | 921.9167 | 1841.8189 | 1841.8182 | 0.38 | Th | 2 | 49 | 1.30E-05 | FSKPEDTFDYQK +dK_MMCCH-2 (K) | 1659 |
| 99 | 1657 | 1668 | 921.9177 | 1841.8208 | 1841.8182 | 1.41 | Th | 2 | 56 | 2.20E-06 | FSKPEDTFDYQK +dK_MMCCH-2 (K) | 1668 |
| 100 | 1733 | 1741 | 664.3229 | 1326.6313 | 1326.6312 | 0.08 | Th | 2 | 17 | 0.02 | VLGGEKEMP +dK_MMCCH-1 (K); Oxidation (M) | 1738 |
| 101 | 1756 | 1760 | 479.7244 | 957.4342 | 957.4300 | 4.49 | Th | 1 | 16 | 0.024 | LDKMN +dK_MMCCH-2 (K) | 1758 |
| 102 | 1894 | 1904 | 850.4153 | 1698.8161 | 1698.8116 | 2.65 | Th | 5 | 42 | 5.60E-05 | VAVPYWDWTKP +dK_MMCCH-2 (K) | 1903 |
| 103 | 2064 | 2068 | 499.7591 | 997.5036 | 997.5055 | -2.01 | Th | 0 | 18 | 0.014 | FTKKH +dK_MMCCH-2 (K) | 2067 |
| 104 | 2094 | 2100 | 618.3030 | 1234.5914 | 1234.5903 | 0.89 | Th | 1 | 27 | 0.0022 | LHEIEKE +dK_MMCCH-2 (K) | 2099 |

Figure 23A. KLH 1 (continued)

| | | | | | | | | | | | | |
|---|---|---|---|---|---|---|---|---|---|---|---|---|
| 105 | 2129 | 2141 | 933.8804 | 1865.7462 | 1865.7495 | -1.77 | Th | 0 | 25 | 0.0035 | ICKTSEDCHHGGQ +dK_MMCCH-2 (K) | 2131 |
| 106 | 2238 | 2248 | 778.8785 | 1555.7425 | 1555.7440 | -0.90 | Th | 3 | 44 | 4.10E-05 | LTTAEVDNLKD +dK_MMCCH-2 (K) | 2247 |
| 107 | 2475 | 2487 | 915.9249 | 1829.8353 | 1829.8295 | 3.22 | Th | 3 | 17 | 0.018 | FTHSNAKPTDVFE +dK_MMCCH-2 (K) | 2481 |
| 108 | 2512 | 2521 | 807.3982 | 1612.7819 | 1612.7766 | 3.29 | Th | 0 | 35 | 0.00033 | LEKQKEEDRT +dK_MMCCH-2 (K) | 2514 |
| 109 | 2508 | 2521 | 708.0113 | 2121.0120 | 2121.0048 | 3.44 | Th | 1 | 14 | 0.042 | LEHELEKQKEEDRT +dK_MMCCH-2 (K) | 2516 |
| 110 | 2595 | 2606 | 806.4034 | 1610.7923 | 1610.7862 | 3.79 | Th | 2 | 35 | 0.00033 | IIDTSGKQLPSD +dK_MMCCH-2 (K) | 2601 |
| 111 | 2607 | 2612 | 520.7794 | 1039.5443 | 1039.5446 | -0.29 | Th | 2 | 22 | 0.0058 | LIKMPT +dK_MMCCH-2 (K) | 2609 |
| 112 | 2766 | 2771 | 551.7853 | 1101.5561 | 1101.5529 | 3.00 | Th | 1 | 24 | 0.0043 | IFQQTK +dK_MMCCH-2 (K) | 2771 |
| 113 | 2808 | 2816 | 638.3184 | 1274.6223 | 1274.6217 | 0.47 | Th | 3 | 27 | 0.0018 | LIGGAEKYS +dK_MMCCH-2 (K) | 2814 |
| 114 | 2946 | 2960 | 965.4048 | 1928.7950 | 1928.7986 | -1.87 | Th | 3 | 30 | 0.001 | IKSGTDSDDEYAGSF +dK_MMCCH-2 (K) | 2947 |
| 115 | 2990 | 2996 | 583.2842 | 1164.5538 | 1164.5485 | 4.55 | Th | 1 | 38 | 0.00016 | LTDDHVK +dK_MMCCH-2 (K) | 2996 |
| 116 | 3076 | 3084 | 724.3623 | 1446.7100 | 1446.7040 | 4.22 | Th | 2 | 13 | 0.046 | FKCKVPPFS +dK_MMCCH-2 (K) | 3079 |

**Figure 23B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1043.0241 | 2084.0335 | 2084.0347 | -0.58 | T | 1 | 44 | 3.80E-05 | KNVDSLSSDEVLALEK +dK_MMCCH-2 (K) | 26 |
| 2 | 147 | 155 | 694.3694 | 1386.7242 | 1386.7217 | 1.80 | T | 1 | 22 | 0.006 | ADITFLNKK +dK_MMCCH-2 (K) | 155 |
| 3 | 164 | 175 | 596.3101 | 1785.9085 | 1785.8985 | 5.66 | T | 1 | 44 | 4.40E-05 | LFEKVQPGHHTR +dK_MMCCH-2 (K) | 167 |
| 4 | 249 | 271 | 987.4429 | 2959.3068 | 2959.3051 | 0.57 | T | 1 | 33 | 0.00051 | GKDPNSADCAHNLIHTPMEPFDR +dK_MMCCH-2 (K) | 250 |
| 5 | 281 | 293 | 610.9435 | 1829.8088 | 1829.8043 | 2.46 | T | 0 | 56 | 2.70E-06 | EHAKPADSFDYGR +dK_MMCCH-2 (K) | 284 |
| 6 | 419 | 446 | 846.4591 | 3381.8073 | 3381.8132 | -1.74 | T | 1 | 24 | 0.0038 | QPTLVHRPAKGHFDKPPVPVAQANLAVR +Deamidated (NQ); dK_MMCCH-2 (K) | 428 |
| 7 | 429 | 446 | 752.0663 | 2253.1770 | 2253.1729 | 1.86 | T | 0 | 52 | 7.00E-06 | GHFDKPPVPVAQANLAVR +dK_MMCCH-2 (K) | 433 |
| 8 | 467 | 488 | 708.5934 | 2830.3444 | 2830.3385 | 2.08 | T | 1 | 65 | 2.90E-07 | FQNDKSVDGYQATVEFHALPAR +dK_MMCCH-2 (K) | 471 |
| 9 | 489 | 497 | 726.8424 | 1451.6701 | 1451.6649 | 3.58 | T | 1 | 20 | 0.009 | CPRPDAKDR +dK_MMCCH-2 (K) | 495 |
| 10 | 936 | 950 | 1080.0117 | 2158.0089 | 2158.0095 | -0.28 | T | 1 | 46 | 2.60E-05 | KHGFTGGLPYWDWTR +dK_MMCCH-2 (K) | 936 |
| 11 | 1082 | 1096 | 996.4610 | 1990.9074 | 1990.9063 | 0.60 | T | 0 | 85 | 3.00E-09 | GKPYNTANCAIASMR +dK_MMCCH-2 (K) | 1083 |
| 12 | 1097 | 1115 | 827.0761 | 2478.2065 | 2478.2101 | -1.45 | T | 0 | 56 | 2.60E-06 | KPLQPFGLDSVINPDDETR +dK_MMCCH-2 (K) | 1097 |
| 13 | 1123 | 1149 | 1190.5605 | 3568.6598 | 3568.6497 | 2.86 | T | 1 | 63 | 5.20E-07 | VFDYKNNFDYEYESLAFNGLSIAQLDR +dK_MMCCH-2 (K) | 1127 |
| 14 | 1355 | 1385 | 1271.2697 | 3810.7871 | 3810.7876 | -0.10 | T | 1 | 27 | 0.0019 | GSAVAVPYWDWTEKADSLPSLINDATYFNSR +dK_MMCCH-2 (K) | 1368 |
| 15 | 1409 | 1450 | 1371.8883 | 5483.5241 | 5483.5213 | 0.51 | T | 1 | 54 | 4.10E-06 | DPQPELWDNKDFYENVMLALEQDNFCDFEIQLELIHNALHSR +dK_MMCCH-2 (K) | 1418 |
| 16 | 1455 | 1480 | 1117.8800 | 3350.6182 | 3350.6070 | 3.31 | T | 1 | 42 | 6.00E-05 | AKYSLSSLDYTAFDPVFFLHHANVDR +dK_MMCCH-2 (K) | 1456 |
| 17 | 1493 | 1507 | 721.6616 | 2161.9629 | 2161.9594 | 1.57 | T | 1 | 62 | 6.50E-07 | KKPYNEADCAVNEMR +dK_MMCCH-2 (K) | 1494 |
| 18 | 1542 | 1564 | 1070.1942 | 3207.5608 | 3207.5448 | 4.99 | T | 1 | 33 | 0.00048 | YQYDNLQFNHFSIQKLDQTIQAR +dK_MMCCH-2 (K) | 1556 |
| 19 | 1691 | 1704 | 999.5225 | 1997.0305 | 1997.0292 | 0.65 | T | 1 | 23 | 0.0055 | EVSSLTTLEKHFLR +dK_MMCCH-2 (K) | 1700 |
| 20 | 1757 | 1768 | 612.9788 | 1835.9146 | 1835.9128 | 1.03 | T | 1 | 16 | 0.025 | LYTVQFEDSLKR +dK_MMCCH-2 (K) | 1767 |
| 21 | 1804 | 1825 | 945.4641 | 2833.3705 | 2833.3633 | 2.54 | T | 1 | 39 | 0.00014 | TFPNPFLKANIEFEGEGVTTER +dK_MMCCH-2 (K) | 1811 |
| 22 | 1949 | 1963 | 1119.4961 | 2236.9776 | 2236.9735 | 1.83 | T | 0 | 45 | 3.30E-05 | TQEFSKPEDTFDYHR +dK_MMCCH-2 (K) | 1954 |
| 23 | 2228 | 2239 | 870.4186 | 1738.8227 | 1738.8236 | -0.52 | T | 1 | 16 | 0.026 | GYIKSEDAYTVR +dK_MMCCH-2 (K) | 2231 |
| 24 | 2527 | 2541 | 1007.5152 | 2013.0158 | 2013.0088 | 3.48 | T | 1 | 79 | 1.40E-08 | KDVTSLTASEIENLR +dK_MMCCH-2 (K) | 2527 |

Figure 23B. KLH 2 (continued)

| | | | | | | | | | | | | |
|---|---|---|---|---|---|---|---|---|---|---|---|---|
| 25 | 2728 | 2739 | 958.0007 | 1913.9869 | 1913.9862 | 0.37 | T | 1 | 22 | 0.0059 | IWAIWQALQKYR +Deamidated (NQ); dK_MMCCH-2 (K) | 2737 |
| 26 | 2755 | 2764 | 764.3814 | 1526.7483 | 1526.7439 | 2.88 | T | 0 | 33 | 0.00051 | QPLKPFSESR +Deamidated (NQ); dK_MMCCH-2 (K) | 2758 |
| 27 | 2866 | 2873 | 659.3627 | 1316.7108 | 1316.7050 | 4.40 | T | 1 | 21 | 0.0089 | YDITKVLK +dK_MMCCH-2 (K) | 2870 |
| 28 | 3148 | 3155 | 629.3356 | 1256.6567 | 1256.6587 | -1.59 | T | 1 | 19 | 0.013 | KKPYNAAK +dK_MMCCH-2 (K) | 3149 |
| 29 | 167 | 178 | 590.9629 | 1769.8668 | 1769.8705 | -2.09 | G | 0 | 38 | 0.00014 | KVQPGHHTRLME +dK_MMCCH-2 (K) | 167 |
| 30 | 433 | 456 | 975.8492 | 2924.5259 | 2924.5429 | -5.81 | G | 2 | 16 | 0.025 | KPPVPVAQANLAVRKNINDLTAEE +dK_MMCCH-2 (K) | 433 |
| 31 | 466 | 481 | 732.3397 | 2193.9972 | 2194.0001 | -1.32 | G | 2 | 25 | 0.0032 | RFQNDKSVDGYQATVE +dK_MMCCH-2 (K) | 471 |
| 32 | 1117 | 1135 | 1404.1260 | 2806.2374 | 2806.2373 | 0.04 | G | 3 | 16 | 0.024 | HSVPFRVFDYKNNFDYEYE +dK_MMCCH-2 (K) | 1127 |
| 33 | 1215 | 1235 | 721.6066 | 2882.3974 | 2882.3949 | 0.87 | G | 3 | 25 | 0.0031 | ITQQLHDLDLHVGDNFFLKYE +dK_MMCCH-2 (K) | 1233 |
| 34 | 1292 | 1303 | 879.4659 | 1756.9173 | 1756.9182 | -0.46 | G | 0 | 42 | 5.80E-05 | SIRSAFLQIQKE +dK_MMCCH-2 (K) | 1302 |
| 35 | 1308 | 1320 | 904.9471 | 1807.8797 | 1807.8749 | 2.66 | G | 0 | 21 | 0.0082 | NIAKFHGKPGLCE +dK_MMCCH-2 (K) | 1311 |
| 36 | 1308 | 1320 | 603.6324 | 1807.8755 | 1807.8749 | 0.33 | G | 0 | 26 | 0.0026 | NIAKFHGKPGLCE +dK_MMCCH-2 (K) | 1315 |
| 37 | 1952 | 1967 | 792.6802 | 2375.0187 | 2375.0205 | -0.76 | G | 3 | 34 | 0.00037 | FSKPEDTFDYHRFGYE +dK_MMCCH-2 (K) | 1954 |
| 38 | 1973 | 1990 | 811.7253 | 2432.1540 | 2432.1505 | 1.48 | G | 0 | 14 | 0.041 | FVGMSVSSLHNYIKQQQE +dK_MMCCH-2 (K) | 1986 |
| 39 | 2471 | 2484 | 990.9680 | 1979.9215 | 1979.9227 | -0.61 | G | 3 | 22 | 0.0069 | DVFDTDAPFFIKVE +dK_MMCCH-2 (K) | 2482 |
| 40 | 3386 | 3405 | 658.3167 | 2629.2377 | 2629.2271 | 4.03 | G | 2 | 31 | 0.00087 | LDHAYSLRDGHYYIAGPTKD +dK_MMCCH-2 (K) | 3404 |
| 41 | 153 | 165 | 629.9987 | 1886.9742 | 1886.9673 | 3.66 | C | 1 | 15 | 0.031 | NKKTSRAVDDRLF +dK_MMCCH-2 (K) | 154 |
| 42 | 277 | 296 | 883.7534 | 2648.2384 | 2648.2329 | 2.08 | C | 4 | 41 | 8.10E-05 | DLTREHAKPADSFDYGRLGY +dK_MMCCH-2 (K) | 284 |
| 43 | 364 | 373 | 735.3599 | 1468.7052 | 1468.7094 | -2.93 | C | 2 | 29 | 0.0012 | LLGGPTEMKW +dK_MMCCH-2 (K) | 372 |
| 44 | 432 | 443 | 793.9158 | 1585.8171 | 1585.8174 | -0.19 | C | 0 | 16 | 0.027 | DKPPVPVAQANL +dK_MMCCH-2 (K) | 433 |
| 45 | 629 | 635 | 539.2513 | 1076.4881 | 1076.4848 | 3.07 | C | 0 | 16 | 0.024 | VGGSEKY +dK_MMCCH-2 (K) | 634 |
| 46 | 1075 | 1080 | 544.7770 | 1087.5394 | 1087.5372 | 2.02 | C | 1 | 18 | 0.017 | QALQKY +dK_MMCCH-2 (K) | 1079 |
| 47 | 1212 | 1230 | 875.0869 | 2622.2389 | 2622.2424 | -1.33 | C | 4 | 17 | 0.02 | KYEITQQLHDLDLHVGDNF +dK_MMCCH-2 (K) | 1212 |
| 48 | 1298 | 1312 | 824.0828 | 2469.2266 | 2469.2324 | -2.31 | C | 2 | 19 | 0.012 | LQIQKEGIYENIAKF +2 dK_MMCCH-2 (K) | 1302, 1311 |
| 49 | 1298 | 1312 | 711.3739 | 2131.0999 | 2131.1023 | -1.17 | C | 2 | 36 | 0.00023 | LQIQKEGIYENIAKF +dK_MMCCH-2 (K) | 1311 |
| 50 | 1526 | 1536 | 851.3828 | 1700.7511 | 1700.7505 | 0.35 | C | 1 | 19 | 0.014 | KHNLPQDSFDY +dK_MMCCH-2 (K) | 1526 |
| 51 | 1589 | 1606 | 784.3549 | 2350.0428 | 2350.0392 | 1.53 | C | 0 | 23 | 0.0054 | ICVEQGGEQNCKTKAGSF +dK_MMCCH-2 (K) | 1602 |

**Figure 23B. KLH 2 (continued)**

| | | | | | | | | | | | | |
|---|---|---|---|---|---|---|---|---|---|---|---|---|
| 52 | 1696 | 1702 | 607.3007 | 1212.5868 | 1212.5849 | 1.57 | C | 1 | 23 | 0.0045 | TTLEKHF +dK_MMCCH-2 (K) | 1700 |
| 53 | 1708 | 1719 | 839.8433 | 1677.6720 | 1677.6651 | 4.11 | C | 0 | 28 | 0.0015 | KNMQADDSPDGY +dK_MMCCH-2 (K) | 1708 |
| 54 | 1801 | 1809 | 706.8373 | 1411.6601 | 1411.6595 | 0.50 | C | 0 | 19 | 0.012 | SHKTFPNPF +dK_MMCCH-2 (K) | 1803 |
| 55 | 1985 | 1995 | 850.4278 | 1698.8410 | 1698.8399 | 0.65 | C | 0 | 32 | 0.00062 | IKQQQEADRVF +dK_MMCCH-2 (K) | 1986 |
| 56 | 2001 | 2011 | 726.8445 | 1451.6744 | 1451.6755 | -0.76 | C | 1 | 22 | 0.0059 | KGFGQSASVSF +dK_MMCCH-2 (K) | 2001 |
| 57 | 2424 | 2438 | 1032.4364 | 2062.8582 | 2062.8621 | -1.84 | C | 0 | 20 | 0.011 | ICKTADDCHASGMIF +dK_MMCCH-2 (K) | 2426 |
| 58 | 2455 | 2462 | 645.3273 | 1288.6401 | 1288.6373 | 2.17 | C | 1 | 19 | 0.013 | KYDITQAL +dK_MMCCH-2 (K) | 2455 |
| 59 | 2463 | 2479 | 752.3498 | 2254.0277 | 2254.0253 | 1.11 | C | 1 | 18 | 0.016 | KAQSIHPEDVFDTDAPF +dK_MMCCH-2 (K) | 2463 |
| 60 | 2506 | 2532 | 761.6044 | 3042.3884 | 3042.3738 | 4.80 | C | 0 | 19 | 0.012 | SAGEGHTDDHGSDHIAGSGVRKDVTSL +dK_MMCCH-2 (K) | 2527 |
| 61 | 2754 | 2760 | 598.3299 | 1194.6452 | 1194.6471 | -1.51 | C | 1 | 18 | 0.017 | KQPLKPF +dK_MMCCH-2 (K) | 2754 |
| 62 | 2754 | 2760 | 598.3325 | 1194.6504 | 1194.6471 | 2.76 | C | 1 | 23 | 0.0046 | KQPLKPF +dK_MMCCH-2 (K) | 2758 |
| 63 | 2865 | 2872 | 673.3627 | 1344.7109 | 1344.7112 | -0.15 | C | 1 | 15 | 0.032 | RYDITKVL +dK_MMCCH-2 (K) | 2870 |
| 64 | 3235 | 3242 | 617.3137 | 1232.6129 | 1232.6111 | 1.46 | C | 0 | 22 | 0.0057 | TSANVKIY +dK_MMCCH-2 (K) | 3240 |
| 65 | 3399 | 3406 | 576.8025 | 1151.5904 | 1151.5896 | 0.69 | C | 0 | 24 | 0.0036 | IAGPTKDL +dK_MMCCH-2 (K) | 3404 |
| 66 | 181 | 191 | 853.3754 | 1704.7363 | 1704.7375 | -0.70 | Th | 3 | 58 | 1.50E-06 | LDALEQDEFCK +dK_MMCCH-2 (K) | 191 |
| 67 | 247 | 260 | 631.6194 | 1891.8363 | 1891.8305 | 3.07 | Th | 2 | 18 | 0.016 | LRGKDPNSADCAHN +dK_MMCCH-2 (K) | 250 |
| 68 | 283 | 293 | 782.8565 | 1563.6985 | 1563.7028 | -2.75 | Th | 3 | 31 | 0.00076 | AKPADSFDYGR +dK_MMCCH-2 (K) | 284 |
| 69 | 365 | 374 | 715.3283 | 1428.6421 | 1428.6418 | 0.21 | Th | 2 | 41 | 7.20E-05 | LGGPTEMKWG +dK_MMCCH-2 (K); Oxidation (M) | 372 |
| 70 | 431 | 437 | 569.2861 | 1136.5576 | 1136.5576 | 0.00 | Th | 1 | 13 | 0.047 | FDKPPVP +dK_MMCCH-2 (K) | 433 |
| 71 | 467 | 472 | 538.7416 | 1075.4687 | 1075.4644 | 4.00 | Th | 0 | 13 | 0.045 | FQNDKS +dK_MMCCH-2 (K) | 471 |
| 72 | 629 | 636 | 582.7663 | 1163.5180 | 1163.5169 | 1.03 | Th | 1 | 24 | 0.0037 | VGGSEKYS +dK_MMCCH-2 (K) | 634 |
| 73 | 893 | 904 | 904.8962 | 1807.7779 | 1807.7698 | 4.48 | Th | 1 | 18 | 0.018 | FHGEPKWCPSPE +dK_MMCCH-2 (K) | 898 |
| 74 | 1414 | 1419 | 564.7561 | 1127.4976 | 1127.4957 | 1.69 | Th | 1 | 21 | 0.0075 | LWDNKD +dK_MMCCH-2 (K) | 1418 |
| 75 | 1525 | 1539 | 738.3547 | 2212.0424 | 2212.0371 | 2.40 | Th | 3 | 43 | 5.10E-05 | LKHNLPQDSFDYQNR +dK_MMCCH-2 (K) | 1526 |
| 76 | 1589 | 1605 | 735.3312 | 2202.9719 | 2202.9708 | 0.50 | Th | 2 | 29 | 0.0012 | ICVEQGGEQNCKTKAGS +dK_MMCCH-2 (K) | 1600 |
| 77 | 1589 | 1605 | 735.3312 | 2202.9717 | 2202.9708 | 0.41 | Th | 2 | 34 | 0.0004 | ICVEQGGEQNCKTKAGS +dK_MMCCH-2 (K) | 1602 |
| 78 | 1800 | 1809 | 780.3710 | 1558.7274 | 1558.7279 | -0.32 | Th | 2 | 28 | 0.0017 | FSHKTFPNPF +dK_MMCCH-2 (K) | 1803 |

**Figure 23B. KLH 2 (continued)**

| | | | | | | | | | | | | |
|---|---|---|---|---|---|---|---|---|---|---|---|---|
| 79 | 1952 | 1963 | 627.2840 | 1878.8302 | 1878.8247 | 2.93 | Th | 2 | 51 | 7.90E-06 | FSKPEDTFDYHR +dK_MMCCH-2 (K) | 1954 |
| 80 | 1985 | 1994 | 776.8959 | 1551.7772 | 1551.7715 | 3.67 | Th | 2 | 23 | 0.0045 | IKQQQEADRV +dK_MMCCH-2 (K) | 1986 |
| 81 | 2193 | 2199 | 667.3011 | 1332.5876 | 1332.5849 | 2.03 | Th | 2 | 27 | 0.0019 | YWDWTKP +dK_MMCCH-2 (K) | 2198 |
| 82 | 2200 | 2206 | 562.3088 | 1122.6031 | 1122.5995 | 3.30 | Th | 2 | 22 | 0.0061 | ISKLPDL +dK_MMCCH-2 (K) | 2202 |
| 83 | 2225 | 2229 | 462.2199 | 922.4251 | 922.4259 | -0.76 | Th | 2 | 17 | 0.021 | FAKGY +dK_MMCCH-2 (K) | 2227 |
| 84 | 2359 | 2363 | 509.2324 | 1016.4503 | 1016.4460 | 4.33 | Th | 1 | 15 | 0.034 | FTKMH +dK_MMCCH-2 (K); Oxidation (M) | 2361 |
| 85 | 2941 | 2950 | 720.8391 | 1439.6635 | 1439.6602 | 2.29 | Th | 3 | 45 | 2.90E-05 | LDEANDLKNA +dK_MMCCH-2 (K) | 2948 |
| 86 | 3009 | 3015 | 573.2759 | 1144.5372 | 1144.5335 | 3.23 | Th | 0 | 15 | 0.033 | LKEHGSH +dK_MMCCH-2 (K) | 3010 |
| 87 | 3020 | 3026 | 662.2905 | 1322.5664 | 1322.5642 | 1.66 | Th | 2 | 20 | 0.0092 | YWDWTKS +dK_MMCCH-2 (K) | 3025 |
| 88 | 3399 | 3405 | 520.2601 | 1038.5057 | 1038.5056 | 0.10 | Th | 1 | 20 | 0.011 | IAGPTKD +dK_MMCCH-2 (K) | 3404 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the lysine conjugation site for KLH1 or KLH2.

**Figure 24. Identification details of MMCCH-conjugated peptides for sample 3 (1st LC-MS/MS)**

**Figure 24A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 124 | 141 | 589.3095 | 2353.2089 | 2353.2001 | 3.74 | T | 1 | 37 | 0.00018 | GFTDPPVKHHQSANLLVR +dK_MMCCH-2 (K) | 131 |
| 2 | 142 | 149 | 656.3412 | 1310.6678 | 1310.6653 | 1.98 | T | 1 | 35 | 0.00029 | KNINDLTR +dK_MMCCH-2 (K) | 142 |
| 3 | 157 | 166 | 822.8796 | 1643.7446 | 1643.7402 | 2.68 | T | 1 | 18 | 0.015 | EAFHKFQEDR +dK_MMCCH-2 (K) | 161 |
| 4 | 184 | 192 | 726.8427 | 1451.6707 | 1451.6649 | 4.00 | T | 1 | 22 | 0.0063 | CPRPDAKDR +dK_MMCCH-2 (K) | 190 |
| 5 | 377 | 402 | 833.4029 | 3329.5825 | 3329.5784 | 1.23 | T | 0 | 28 | 0.0016 | AHCAISLEHMHLKPFAFSSPLNNNEK +dK_MMCCH-2 (K) | 389 |
| 6 | 460 | 477 | 781.3949 | 2341.1629 | 2341.1624 | 0.21 | T | 1 | 22 | 0.0068 | TSANVDIFIKTTDSVQHK +dK_MMCCH-2 (K) | 469 |
| 7 | 470 | 488 | 748.0465 | 2241.1177 | 2241.1100 | 3.44 | T | 1 | 43 | 5.20E-05 | TTDSVQHKAGTFAVLGGSK +dK_MMCCH-2 (K) | 477 |
| 8 | 553 | 560 | 663.8680 | 1325.7214 | 1325.7166 | 3.62 | T | 2 | 21 | 0.0073 | VKFDKVPR +dK_MMCCH-2 (K) | 554 |
| 9 | 566 | 570 | 485.2456 | 968.4767 | 968.4749 | 1.86 | T | 1 | 17 | 0.018 | KNVDR +dK_MMCCH-2 (K) | 566 |
| 10 | 581 | 587 | 547.8373 | 1093.6601 | 1093.6569 | 2.93 | T | 1 | 22 | 0.006 | KALALLK +dK_MMCCH-2 (K) | 581 |
| 11 | 588 | 606 | 781.0351 | 2340.0835 | 2340.0845 | -0.43 | T | 1 | 30 | 0.001 | EDKSAGGFQQLGAFHGEPK +dK_MMCCH-2 (K) | 590 |
| 12 | 591 | 615 | 753.6043 | 3010.3881 | 3010.3742 | 4.62 | T | 1 | 19 | 0.012 | SAGGFQQLGAFHGEPKWCPSPEASK +dK_MMCCH-2 (K) | 606 |
| 13 | 645 | 681 | 1158.5398 | 4630.1301 | 4630.1063 | 5.12 | T | 1 | 27 | 0.0018 | HGYDGALPYWDWTSPLNHLPELADHEKYVDPEDGVEK +dK_MMCCH-2 (K) | 671 |
| 14 | 682 | 699 | 620.2947 | 2477.1496 | 2477.1434 | 2.50 | T | 1 | 21 | 0.0082 | HNPWFDGHIDTVDKTTTR +dK_MMCCH-2 (K) | 695 |
| 15 | 700 | 719 | 887.7702 | 2660.2888 | 2660.2945 | -2.14 | T | 1 | 39 | 0.00013 | SVQNKLFEQPEFGHYTSIAK +dK_MMCCH-2 (K) | 704 |
| 16 | 790 | 804 | 669.3174 | 2004.9305 | 2004.9220 | 4.24 | T | 0 | 38 | 0.00017 | GKPYNVANCAVTSMR +dK_MMCCH-2 (K) | 791 |
| 17 | 858 | 863 | 520.2993 | 1038.5840 | 1038.5783 | 5.39 | T | 1 | 23 | 0.0051 | KLEAIK +dK_MMCCH-2 (K) | 858 |
| 18 | 953 | 965 | 635.3319 | 1902.9737 | 1902.9662 | 3.94 | T | 1 | 53 | 4.80E-06 | DLFKQPSVIHEPR +dK_MMCCH-2 (K) | 956 |
| 19 | 986 | 1000 | 1027.0366 | 2052.0587 | 2052.0561 | 1.27 | T | 1 | 75 | 2.90E-08 | KNIENLSLGELESLR +dK_MMCCH-2 (K) | 986 |
| 20 | 1048 | 1060 | 609.3551 | 1825.0435 | 1825.0423 | 0.66 | T | 1 | 44 | 3.80E-05 | LYVVVVENALLKK +dK_MMCCH-2 (K) | 1060 |
| 21 | 1092 | 1114 | 791.8734 | 3163.4645 | 3163.4682 | -1.17 | T | 1 | 28 | 0.0018 | QHHYETNPFHHGKITHENEITTR +dK_MMCCH-2 (K) | 1104 |
| 22 | 1199 | 1203 | 353.1914 | 1056.5522 | 1056.5538 | -1.51 | T | 1 | 17 | 0.018 | KRPYR +dK_MMCCH-2 (K) | 1199 |
| 23 | 1331 | 1338 | 620.3551 | 1238.6956 | 1238.6944 | 0.97 | T | 1 | 17 | 0.018 | LDITKALK +dK_MMCCH-2 (K) | 1335 |
| 24 | 1395 | 1402 | 649.8379 | 1297.6612 | 1297.6588 | 1.85 | T | 1 | 26 | 0.0022 | KDITQLDK +dK_MMCCH-2 (K) | 1395 |

Figure 24A. KLH 1 (continued)

| | | | | | | | | | | | | |
|---|---|---|---|---|---|---|---|---|---|---|---|---|
| 25 | 1395 | 1403 | 485.5953 | 1453.7639 | 1453.7599 | 2.75 | T | 2 | 16 | 0.025 | KDITQLDKR +dK_MMCCH-2 (K) | 1402 |
| 26 | 1417 | 1445 | 844.1611 | 3372.6154 | 3372.6020 | 4.00 | T | 1 | 19 | 0.012 | ADHSSDGFQAIASFHALPPLCPSPAASKR +dK_MMCCH-2 (K) | 1444 |
| 27 | 1473 | 1489 | 778.4021 | 2332.1845 | 2332.1827 | 0.77 | T | 1 | 47 | 1.90E-05 | KHGAVVGLPYWDWTLPR +dK_MMCCH-2 (K) | 1473 |
| 28 | 1721 | 1746 | 1092.8043 | 3275.3911 | 3275.3886 | 0.76 | T | 1 | 14 | 0.044 | TAGDCEDAGYFTVLGGEKEMPWAFDR +dK_MMCCH-2 (K); Oxidation (M) | 1738 |
| 29 | 1890 | 1922 | 879.8327 | 4394.1271 | 4394.1048 | 5.07 | T | 1 | 16 | 0.024 | HGSSVAVPYWDWTKPIHNIPHLFTDKEYYDVWR +dK_MMCCH-2 (K) | 1915 |
| 30 | 1923 | 1932 | 756.3803 | 1510.7461 | 1510.7425 | 2.38 | T | 1 | 26 | 0.0028 | NKVMPNPFAR +dK_MMCCH-2 (K) | 1924 |
| 31 | 2067 | 2076 | 746.8841 | 1491.7536 | 1491.7544 | -0.54 | T | 1 | 28 | 0.0014 | KHAVPNDVFK +dK_MMCCH-2 (K) | 2067 |
| 32 | 2121 | 2149 | 890.4184 | 3557.6444 | 3557.6378 | 1.86 | T | 1 | 29 | 0.0013 | TSADVQFQICKTSEDCHHGGQIFVLGGTK +dK_MMCCH-2 (K) | 2131 |
| 33 | 2359 | 2367 | 736.8409 | 1471.6672 | 1471.6653 | 1.29 | T | 1 | 19 | 0.012 | DKLFNDPER +Deamidated (NQ); dK_MMCCH-2 (K) | 2360 |
| 34 | 2461 | 2490 | 642.8212 | 3850.8834 | 3850.8638 | 5.09 | T | 0 | 17 | 0.02 | RPLRPFSDPINHNAFTHSNAKPTDVFEYSR +dK_MMCCH-2 (K) | 2481 |
| 35 | 2507 | 2514 | 682.3522 | 1362.6898 | 1362.6853 | 3.30 | T | 1 | 40 | 9.90E-05 | KLEHELEK +dK_MMCCH-2 (K) | 2507 |
| 36 | 2515 | 2520 | 571.7623 | 1141.5100 | 1141.5074 | 2.28 | T | 1 | 19 | 0.013 | QKEEDR +dK_MMCCH-2 (K) | 2516 |
| 37 | 2595 | 2609 | 983.5305 | 1965.0465 | 1965.0493 | -1.42 | T | 1 | 20 | 0.01 | IIDTSGKQLPSDLIK +dK_MMCCH-2 (K) | 2601 |
| 38 | 2621 | 2636 | 802.7087 | 2405.1044 | 2405.1083 | -1.62 | T | 1 | 48 | 1.50E-05 | HHEKHHEDHHEDILVR +dK_MMCCH-2 (K) | 2624 |
| 39 | 2637 | 2651 | 1069.5308 | 2137.0470 | 2137.0374 | 4.45 | T | 1 | 61 | 8.10E-07 | KNIHSLSHHEAEELR +dK_MMCCH-2 (K) | 2637 |
| 40 | 2652 | 2682 | 975.1997 | 3896.7695 | 3896.7563 | 3.39 | T | 1 | 19 | 0.012 | DALYKLQNDESHGGYEHIAGFHGYPNLCPEK +dK_MMCCH-2 (K) | 2656 |
| 41 | 2657 | 2686 | 934.9150 | 3735.6311 | 3735.6358 | -1.28 | T | 1 | 22 | 0.0068 | LQNDESHGGYEHIAGFHGYPNLCPEKGDEK +dK_MMCCH-2 (K) | 2682 |
| 42 | 2657 | 2686 | 1246.2205 | 3735.6396 | 3735.6358 | 0.99 | T | 1 | 24 | 0.0036 | LQNDESHGGYEHIAGFHGYPNLCPEKGDEK +dK_MMCCH-2 (K) | 2686 |
| 43 | 2714 | 2748 | 1087.7703 | 4347.0519 | 4347.0524 | -0.12 | T | 1 | 32 | 0.00056 | KHGSHLGIPYWDWTQTISSLPTFFADSGNNNPFFK +dK_MMCCH-2 (K) | 2714 |
| 44 | 2851 | 2883 | 1009.2175 | 4032.8410 | 4032.8346 | 1.59 | T | 0 | 60 | 1.10E-06 | VKPAHAGSCAGDIMHVPLHPFNYESVNNDDFTR +dK_MMCCH-2 (K) | 2852 |
| 45 | 2897 | 2919 | 800.1419 | 3196.5385 | 3196.5287 | 3.07 | T | 1 | 66 | 2.30E-07 | FNYKYDNLNLHGHNIEELEEVLR +dK_MMCCH-2 (K) | 2900 |
| 46 | 2938 | 2947 | 730.4152 | 1458.8158 | 1458.8156 | 0.07 | T | 1 | 35 | 0.00028 | TTAVVKVYIK +dK_MMCCH-2 (K) | 2943 |
| 47 | 3003 | 3021 | 825.7698 | 2474.2877 | 2474.2879 | -0.08 | T | 1 | 62 | 7.00E-07 | KYDHTELDASVLPAPIIVR +dK_MMCCH-2 (K) | 3003 |
| 48 | 3022 | 3044 | 966.5270 | 2896.5593 | 2896.5521 | 2.49 | T | 1 | 15 | 0.03 | RPNNAVFDIIEIPIGKDVNLPPK +dK_MMCCH-2 (K) | 3037 |
| 49 | 123 | 151 | 616.6582 | 3693.9058 | 3693.9049 | 0.24 | G | 2 | 27 | 0.0018 | KGFTDPPVKHHQSANLLVRKNINDLTREE +dK_MMCCH-2 (K) | 123 |
| 50 | 123 | 151 | 739.7917 | 3693.9221 | 3693.9049 | 4.63 | G | 2 | 22 | 0.006 | KGFTDPPVKHHQSANLLVRKNINDLTREE +dK_MMCCH-2 (K) | 131 |
| 51 | 158 | 176 | 846.3843 | 2536.1310 | 2536.1329 | -0.75 | G | 3 | 20 | 0.011 | AFHKFQEDRSVDGYQATAE +dK_MMCCH-2 (K) | 161 |

Figure 24A. KLH 1 (continued)

| | | | | | | | | | | | | |
|---|---|---|---|---|---|---|---|---|---|---|---|---|
| 52 | 526 | 544 | 777.7485 | 2330.2236 | 2330.2304 | -2.92 | G | 1 | 22 | 0.0068 | VDGTKLASSLIPHASVIRE +dK_MMCCH-2 (K) | 530 |
| 53 | 589 | 612 | 1102.8267 | 3305.4582 | 3305.4621 | -1.18 | G | 1 | 15 | 0.029 | DKSAGGFQQLGAFHGEPKWCPSPE +2 dK_MMCCH-2 (K) | 590, 606 |
| 54 | 825 | 841 | 820.3707 | 2458.0902 | 2458.0940 | -1.55 | G | 1 | 24 | 0.004 | HSVPFNVFDYKTNFNYE +dK_MMCCH-2 (K) | 835 |
| 55 | 847 | 860 | 644.0053 | 1928.9941 | 1928.9917 | 1.24 | G | 0 | 17 | 0.019 | FNGLSISQLNKKLE +Deamidated (NQ); dK_MMCCH-2 (K) | 858 |
| 56 | 942 | 972 | 764.6007 | 3817.9672 | 3817.9614 | 1.52 | G | 3 | 17 | 0.021 | VFDLKPASLGKDLFKQPSVIHEPRIGHHEGE +dK_MMCCH-2 (K) | 946 |
| 57 | 942 | 970 | 908.9828 | 3631.9022 | 3631.8973 | 1.35 | G | 2 | 25 | 0.003 | VFDLKPASLGKDLFKQPSVIHEPRIGHHE +dK_MMCCH-2 (K) | 956 |
| 58 | 1397 | 1413 | 774.4363 | 2320.2872 | 2320.2824 | 2.07 | G | 1 | 16 | 0.024 | ITQLDKRQQLSLVKALE +dK_MMCCH-2 (K) | 1410 |
| 59 | 1495 | 1522 | 866.6916 | 3462.7373 | 3462.7381 | -0.26 | G | 3 | 16 | 0.023 | LLTVSTIHDPETGRDIPNPFIGSKIEFE +dK_MMCCH-2 (K) | 1518 |
| 60 | 1755 | 1765 | 570.6053 | 1708.7940 | 1708.7913 | 1.64 | G | 2 | 18 | 0.016 | TLDKMNLRHDE +dK_MMCCH-2 (K) | 1758 |
| 61 | 2089 | 2100 | 854.4076 | 1706.8006 | 1706.8008 | -0.06 | G | 2 | 21 | 0.0077 | IGGMNLHEIEKE +dK_MMCCH-2 (K) | 2099 |
| 62 | 2647 | 2661 | 715.6776 | 2144.0108 | 2144.0095 | 0.61 | G | 4 | 29 | 0.0013 | AEELRDALYKLQNDE +dK_MMCCH-2 (K) | 2656 |
| 63 | 3090 | 3097 | 632.8022 | 1263.5898 | 1263.5879 | 1.50 | G | 0 | 14 | 0.04 | LGKMYSVE +dK_MMCCH-2 (K) | 3092 |
| 64 | 59 | 68 | 729.3448 | 1456.6750 | 1456.6731 | 1.37 | C | 1 | 19 | 0.012 | VLGGPSEMKW +dK_MMCCH-2 (K); Oxidation (M) | 67 |
| 65 | 94 | 100 | 573.2989 | 1144.5833 | 1144.5838 | -0.44 | C | 1 | 23 | 0.0048 | TVKAELF +dK_MMCCH-2 (K) | 96 |
| 66 | 140 | 159 | 692.6202 | 2766.4519 | 2766.4486 | 1.16 | C | 3 | 28 | 0.0018 | VRKNINDLTREEVLNLREAF +dK_MMCCH-2 (K) | 142 |
| 67 | 160 | 171 | 606.9435 | 1817.8086 | 1817.8043 | 2.37 | C | 1 | 19 | 0.012 | HKFQEDRSVDGY +dK_MMCCH-2 (K) | 161 |
| 68 | 429 | 450 | 990.8075 | 2969.4007 | 2969.3899 | 3.64 | C | 1 | 36 | 0.00024 | GGISLENIEKMIHENQQEDRIY +dK_MMCCH-2 (K); Oxidation (M) | 438 |
| 69 | 584 | 595 | 787.4017 | 1572.7888 | 1572.7858 | 1.91 | C | 2 | 42 | 6.40E-05 | ALLKEDKSAGGF +dK_MMCCH-2 (K) | 590 |
| 70 | 715 | 723 | 655.8735 | 1309.7325 | 1309.7316 | 0.76 | C | 1 | 26 | 0.0023 | TSIAKQVLL +dK_MMCCH-2 (K) | 719 |
| 71 | 783 | 788 | 544.7770 | 1087.5395 | 1087.5372 | 2.11 | C | 1 | 26 | 0.0024 | QALQKY +dK_MMCCH-2 (K) | 787 |
| 72 | 944 | 955 | 821.4324 | 1640.8503 | 1640.8484 | 1.16 | C | 3 | 25 | 0.0034 | DLKPASLGKDLF +dK_MMCCH-2 (K) | 946 |
| 73 | 1013 | 1018 | 516.7581 | 1031.5017 | 1031.4998 | 1.84 | C | 0 | 13 | 0.049 | ESIAKF +dK_MMCCH-2 (K) | 1017 |
| 74 | 1330 | 1337 | 620.3546 | 1238.6947 | 1238.6944 | 0.16 | C | 1 | 21 | 0.0089 | KLDITKAL +dK_MMCCH-2 (K) | 1335 |
| 75 | 1580 | 1586 | 544.2614 | 1086.5083 | 1086.5056 | 2.49 | C | 0 | 13 | 0.049 | VGGKEPY +dK_MMCCH-2 (K) | 1583 |
| 76 | 1649 | 1664 | 1127.9900 | 2253.9654 | 2253.9637 | 0.75 | C | 2 | 18 | 0.014 | NLNDHTHDFSKPEDTF +dK_MMCCH-2 (K) | 1659 |
| 77 | 1649 | 1669 | 979.4319 | 2935.2738 | 2935.2759 | -0.72 | C | 4 | 24 | 0.0036 | NLNDHTHDFSKPEDTFDYQKF +dK_MMCCH-2 (K) | 1668 |
| 78 | 1732 | 1742 | 792.8744 | 1583.7342 | 1583.7364 | -1.39 | C | 1 | 17 | 0.02 | TVLGGEKEMPW +dK_MMCCH-2 (K) | 1738 |

**Figure 24A. KLH 1 (continued)**

| | | | | | | | | | | | | |
|---|---|---|---|---|---|---|---|---|---|---|---|---|
| 79 | 1818 | 1827 | 753.3699 | 1504.7253 | 1504.7232 | 1.40 | C | 1 | 18 | 0.018 | SERDIGSLKY +dK_MMCCH-2 (K) | 1826 |
| 80 | 2076 | 2082 | 612.3045 | 1222.5944 | 1222.5944 | 0.08 | C | 3 | 14 | 0.037 | KYELLGY +dK_MMCCH-2 (K) | 2076 |
| 81 | 2160 | 2184 | 1085.5143 | 3253.5210 | 3253.5179 | 0.95 | C | 3 | 21 | 0.0089 | KYDITHALHDAHITPEDVFHPSEPF +dK_MMCCH-2 (K) | 2160 |
| 82 | 2476 | 2488 | 923.9219 | 1845.8293 | 1845.8244 | 2.71 | C | 1 | 25 | 0.0029 | THSNAKPTDVFEY +dK_MMCCH-2 (K) | 2481 |
| 83 | 2656 | 2672 | 747.3428 | 2239.0067 | 2239.0004 | 2.81 | C | 2 | 30 | 0.00098 | KLQNDESHGGYEHIAGF +dK_MMCCH-2 (K) | 2656 |
| 84 | 2738 | 2749 | 856.3768 | 1710.7390 | 1710.7348 | 2.46 | C | 2 | 24 | 0.004 | ADSGNNNPFFKY +dK_MMCCH-2 (K) | 2748 |
| 85 | 2946 | 2960 | 965.4055 | 1928.7964 | 1928.7986 | -1.14 | C | 1 | 40 | 9.10E-05 | IKSGTDSDDEYAGSF +dK_MMCCH-2 (K) | 2947 |
| 86 | 2961 | 2971 | 769.8918 | 1537.7691 | 1537.7673 | 1.24 | C | 1 | 21 | 0.0078 | VILGGAKEMPW +dK_MMCCH-2 (K) | 2967 |
| 87 | 1 | 9 | 733.3348 | 1464.6550 | 1464.6516 | 2.32 | Th | 2 | 17 | 0.021 | MTPEELKTY +dK_MMCCH-2 (K); Oxidation (M) | 7 |
| 88 | 38 | 56 | 861.6805 | 2582.0198 | 2582.0108 | 3.49 | Th | 2 | 15 | 0.033 | VCIPDDNDRNDDHCEKAGD +dK_MMCCH-2 (K) | 53 |
| 89 | 59 | 69 | 785.3774 | 1568.7402 | 1568.7367 | 2.23 | Th | 3 | 61 | 7.10E-07 | VLGGPSEMKWQ +dK_MMCCH-2 (K) | 67 |
| 90 | 78 | 84 | 569.2872 | 1136.5599 | 1136.5536 | 5.54 | Th | 1 | 49 | 1.10E-05 | LSDTVHK +dK_MMCCH-2 (K) | 84 |
| 91 | 116 | 124 | 670.3482 | 1338.6819 | 1338.6755 | 4.78 | Th | 2 | 27 | 0.002 | VVVHHPEKG +dK_MMCCH-2 (K) | 123 |
| 92 | 125 | 135 | 815.8878 | 1629.7611 | 1629.7610 | 0.06 | Th | 1 | 14 | 0.044 | FTDPPVKHHQS +dK_MMCCH-2 (K) | 131 |
| 93 | 431 | 439 | 707.8528 | 1413.6911 | 1413.6883 | 1.98 | Th | 3 | 39 | 0.00013 | ISLENIEKM +dK_MMCCH-2 (K) | 438 |
| 94 | 521 | 530 | 707.8450 | 1413.6754 | 1413.6698 | 3.96 | Th | 2 | 74 | 4.30E-08 | VDITEVDGTK +dK_MMCCH-2 (K) | 530 |
| 95 | 585 | 591 | 585.8101 | 1169.6057 | 1169.6002 | 4.70 | Th | 1 | 35 | 0.00034 | LLKEDKS +dK_MMCCH-2 (K) | 590 |
| 96 | 601 | 612 | 904.8956 | 1807.7767 | 1807.7698 | 3.82 | Th | 1 | 25 | 0.0035 | FHGEPKWCPSPE +dK_MMCCH-2 (K) | 606 |
| 97 | 666 | 672 | 607.2832 | 1212.5518 | 1212.5485 | 2.80 | Th | 2 | 23 | 0.0046 | LADHEKY +dK_MMCCH-2 (K) | 671 |
| 98 | 821 | 828 | 617.8120 | 1233.6095 | 1233.6064 | 2.51 | Th | 1 | 19 | 0.012 | VTKEHSVP +dK_MMCCH-2 (K) | 823 |
| 99 | 831 | 837 | 612.7877 | 1223.5608 | 1223.5533 | 6.13 | Th | 2 | 25 | 0.003 | VFDYKTN +dK_MMCCH-2 (K) | 835 |
| 100 | 1064 | 1074 | 586.9629 | 1757.8668 | 1757.8600 | 3.93 | Th | 5 | 15 | 0.032 | VAVPYWDWTKR +dK_MMCCH-2 (K) | 1073 |
| 101 | 1156 | 1164 | 630.8397 | 1259.6648 | 1259.6584 | 5.08 | Th | 2 | 22 | 0.0058 | LLGGKGKYS +dK_MMCCH-2 (K) | 1162 |
| 102 | 1219 | 1232 | 980.4239 | 1958.8332 | 1958.8316 | 0.82 | Th | 1 | 53 | 4.70E-06 | FDKSDNNDEATKTH +dK_MMCCH-2 (K) | 1230 |
| 103 | 1579 | 1587 | 665.8146 | 1329.6146 | 1329.6064 | 6.17 | Th | 2 | 19 | 0.012 | WVGGKEPYG +dK_MMCCH-2 (K) | 1583 |
| 104 | 1657 | 1668 | 921.9210 | 1841.8275 | 1841.8182 | 5.05 | Th | 2 | 42 | 6.10E-05 | FSKPEDTFDYQK +dK_MMCCH-2 (K) | 1659 |
| 105 | 1657 | 1668 | 921.9207 | 1841.8269 | 1841.8182 | 4.72 | Th | 2 | 50 | 9.90E-06 | FSKPEDTFDYQK +dK_MMCCH-2 (K) | 1668 |

Figure 24A. KLH 1 (continued)

| | | | | | | | | | | | | |
|---|---|---|---|---|---|---|---|---|---|---|---|---|
| 106 | 1733 | 1741 | 664.3254 | 1326.6362 | 1326.6312 | 3.77 | Th | 2 | 17 | 0.022 | VLGGEKEMP +dK_MMCCH-1 (K); Oxidation (M) | 1738 |
| 107 | 1756 | 1760 | 479.7249 | 957.4353 | 957.4300 | 5.64 | Th | 1 | 13 | 0.049 | LDKMN +dK_MMCCH-2 (K) | 1758 |
| 108 | 1894 | 1904 | 850.4138 | 1698.8131 | 1698.8116 | 0.88 | Th | 5 | 29 | 0.0013 | VAVPYWDWTKP +dK_MMCCH-2 (K) | 1903 |
| 109 | 2094 | 2100 | 618.3031 | 1234.5916 | 1234.5903 | 1.05 | Th | 1 | 26 | 0.0024 | LHEIEKE +dK_MMCCH-2 (K) | 2099 |
| 110 | 2129 | 2141 | 933.8805 | 1865.7465 | 1865.7495 | -1.61 | Th | 0 | 27 | 0.0019 | ICKTSEDCHHGGQ +dK_MMCCH-2 (K) | 2131 |
| 111 | 2238 | 2249 | 814.3991 | 1626.7837 | 1626.7811 | 1.60 | Th | 4 | 49 | 1.30E-05 | LTTAEVDNLKDA +dK_MMCCH-2 (K) | 2247 |
| 112 | 2475 | 2484 | 728.3341 | 1454.6535 | 1454.6500 | 2.41 | Th | 1 | 27 | 0.002 | FTHSNAKPTD +dK_MMCCH-2 (K) | 2481 |
| 113 | 2512 | 2521 | 538.6010 | 1612.7810 | 1612.7766 | 2.73 | Th | 0 | 33 | 0.00055 | LEKQKEEDRT +dK_MMCCH-2 (K) | 2514 |
| 114 | 2595 | 2607 | 862.9463 | 1723.8780 | 1723.8702 | 4.52 | Th | 3 | 30 | 0.0011 | IIDTSGKQLPSDL +dK_MMCCH-2 (K) | 2601 |
| 115 | 2607 | 2612 | 520.7814 | 1039.5483 | 1039.5446 | 3.56 | Th | 2 | 23 | 0.0056 | LIKMPT +dK_MMCCH-2 (K) | 2609 |
| 116 | 2766 | 2771 | 551.7883 | 1101.5621 | 1101.5529 | 8.35 | Th | 1 | 24 | 0.0041 | IFQQTK +dK_MMCCH-2 (K) | 2771 |
| 117 | 2808 | 2816 | 638.3212 | 1274.6279 | 1274.6217 | 4.94 | Th | 3 | 27 | 0.0019 | LIGGAEKYS +dK_MMCCH-2 (K) | 2814 |
| 118 | 2946 | 2960 | 965.4112 | 1928.8078 | 1928.7986 | 4.77 | Th | 3 | 54 | 3.80E-06 | IKSGTDSDDEYAGSF +dK_MMCCH-2 (K) | 2947 |
| 119 | 2961 | 2970 | 691.8644 | 1381.7142 | 1381.7098 | 3.18 | Th | 4 | 19 | 0.011 | VILGGAKEMP +dK_MMCCH-1 (K); Oxidation (M) | 2967 |
| 120 | 2990 | 2996 | 583.2855 | 1164.5565 | 1164.5485 | 6.87 | Th | 1 | 38 | 0.00015 | LTDDHVK +dK_MMCCH-2 (K) | 2996 |

Figure 24B. KLH 2

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1043.0231 | 2084.0316 | 2084.0347 | -1.49 | T | 1 | 50 | 1.10E-05 | KNVDSLSSDEVLALEK +dK_MMCCH-2 (K) | 26 |
| 2 | 147 | 155 | 694.3697 | 1386.7249 | 1386.7217 | 2.31 | T | 1 | 47 | 2.20E-05 | ADITFLNKK +dK_MMCCH-2 (K) | 155 |
| 3 | 164 | 175 | 596.3073 | 1785.9001 | 1785.8985 | 0.90 | T | 1 | 36 | 0.00025 | LFEKVQPGHHTR +dK_MMCCH-2 (K) | 167 |
| 4 | 249 | 271 | 740.8343 | 2959.3081 | 2959.3051 | 0.98 | T | 1 | 25 | 0.0035 | GKDPNSADCAHNLIHTPMEPFDR +dK_MMCCH-2 (K) | 250 |
| 5 | 281 | 293 | 610.9426 | 1829.8061 | 1829.8043 | 0.98 | T | 0 | 50 | 1.00E-05 | EHAKPADSFDYGR +dK_MMCCH-2 (K) | 284 |
| 6 | 359 | 377 | 844.0674 | 2529.1803 | 2529.1861 | -2.29 | T | 1 | 17 | 0.02 | AGDFFLLGGPTEMKWGFYR +dK_MMCCH-2 (K) | 372 |
| 7 | 419 | 446 | 677.3699 | 3381.8133 | 3381.8132 | 0.03 | T | 1 | 56 | 2.50E-06 | QPTLVHRPAKGHFDKPPVPVAQANLAVR +Deamidated (NQ); dK_MMCCH-2 (K) | 428 |
| 8 | 429 | 446 | 752.0684 | 2253.1833 | 2253.1729 | 4.62 | T | 0 | 56 | 2.50E-06 | GHFDKPPVPVAQANLAVR +dK_MMCCH-2 (K) | 433 |
| 9 | 467 | 488 | 944.4542 | 2830.3408 | 2830.3385 | 0.85 | T | 1 | 66 | 2.80E-07 | FQNDKSVDGYQATVEFHALPAR +dK_MMCCH-2 (K) | 471 |
| 10 | 489 | 497 | 726.8427 | 1451.6707 | 1451.6649 | 4.00 | T | 1 | 22 | 0.0063 | CPRPDAKDR +dK_MMCCH-2 (K) | 495 |
| 11 | 936 | 950 | 720.3431 | 2158.0074 | 2158.0095 | -0.97 | T | 1 | 28 | 0.0016 | KHGFTGGLPYWDWTR +dK_MMCCH-2 (K) | 936 |
| 12 | 1082 | 1096 | 996.4633 | 1990.9120 | 1990.9063 | 2.86 | T | 0 | 22 | 0.006 | GKPYNTANCAIASMR +dK_MMCCH-2 (K) | 1083 |
| 13 | 1097 | 1115 | 827.0760 | 2478.2061 | 2478.2101 | -1.57 | T | 0 | 78 | 1.60E-08 | KPLQPFGLDSVINPDDETR +dK_MMCCH-2 (K) | 1097 |
| 14 | 1123 | 1149 | 1190.5605 | 3568.6598 | 3568.6497 | 2.86 | T | 1 | 75 | 3.00E-08 | VFDYKNNFDYEYESLAFNGLSIAQLDR +dK_MMCCH-2 (K) | 1127 |
| 15 | 1295 | 1311 | 764.0679 | 2289.1818 | 2289.1715 | 4.50 | T | 1 | 17 | 0.018 | SAFLQIQKEGIYENIAK +dK_MMCCH-2 (K) | 1302 |
| 16 | 1355 | 1385 | 1271.2678 | 3810.7816 | 3810.7876 | -1.55 | T | 1 | 52 | 6.40E-06 | GSAVAVPYWDWTEKADSLPSLINDATYFNSR +dK_MMCCH-2 (K) | 1368 |
| 17 | 1409 | 1450 | 1097.7150 | 5483.5388 | 5483.5213 | 3.19 | T | 1 | 18 | 0.016 | DPQPELWDNKDFYENVMLALEQDNFCDFEIQLELIHNALHSR +dK_MMCCH-2 (K) | 1418 |
| 18 | 1455 | 1480 | 838.6584 | 3350.6045 | 3350.6070 | -0.78 | T | 1 | 56 | 2.70E-06 | AKYSLSSLDYTAFDPVFFLHHANVDR +dK_MMCCH-2 (K) | 1456 |
| 19 | 1493 | 1507 | 721.6590 | 2161.9552 | 2161.9594 | -1.99 | T | 1 | 50 | 1.00E-05 | KKPYNEADCAVNEMR +dK_MMCCH-2 (K) | 1494 |
| 20 | 1508 | 1539 | 1032.2455 | 4124.9528 | 4124.9360 | 4.07 | T | 1 | 28 | 0.0014 | KPLQPFNNPELNSDSMTLKHNLPQDSFDYQNR +dK_MMCCH-2 (K) | 1526 |
| 21 | 1542 | 1564 | 1070.1910 | 3207.5513 | 3207.5448 | 2.03 | T | 1 | 47 | 1.80E-05 | YQYDNLQFNHFSIQKLDQTIQAR +dK_MMCCH-2 (K) | 1556 |
| 22 | 1757 | 1768 | 612.9789 | 1835.9148 | 1835.9128 | 1.09 | T | 1 | 22 | 0.0057 | LYTVQFEDSLKR +dK_MMCCH-2 (K) | 1767 |
| 23 | 1949 | 1963 | 560.2522 | 2236.9797 | 2236.9735 | 2.77 | T | 0 | 57 | 2.00E-06 | TQEFSKPEDTFDYHR +dK_MMCCH-2 (K) | 1954 |
| 24 | 2045 | 2052 | 660.3506 | 1318.6867 | 1318.6843 | 1.90 | T | 1 | 15 | 0.035 | YDITKTLK +dK_MMCCH-2 (K) | 2052 |

**Figure 24B. KLH 2 (continued)**

| | | | | | | | | | | | | |
|---|---|---|---|---|---|---|---|---|---|---|---|---|
| 25 | 2058 | 2069 | 909.4624 | 1816.9102 | 1816.9070 | 1.82 | T | 1 | 24 | 0.0043 | YDDTFTIKVHIK +dK_MMCCH-2 (K) | 2065 |
| 26 | 2228 | 2239 | 870.4213 | 1738.8280 | 1738.8236 | 2.53 | T | 1 | 19 | 0.012 | GYIKSEDAYTVR +dK_MMCCH-2 (K) | 2231 |
| 27 | 2527 | 2541 | 1007.5135 | 2013.0124 | 2013.0088 | 1.79 | T | 1 | 50 | 9.00E-06 | KDVTSLTASEIENLR +dK_MMCCH-2 (K) | 2527 |
| 28 | 2755 | 2764 | 764.3831 | 1526.7517 | 1526.7439 | 5.11 | T | 0 | 19 | 0.013 | QPLKPFSESR +Deamidated (NQ); dK_MMCCH-2 (K) | 2758 |
| 29 | 2866 | 2873 | 659.3611 | 1316.7076 | 1316.7050 | 1.97 | T | 1 | 13 | 0.05 | YDITKVLK +dK_MMCCH-2 (K) | 2870 |
| 30 | 3148 | 3155 | 629.3355 | 1256.6565 | 1256.6587 | -1.75 | T | 1 | 18 | 0.016 | KKPYNAAK +dK_MMCCH-2 (K) | 3149 |
| 31 | 433 | 456 | 975.8590 | 2924.5552 | 2924.5429 | 4.21 | G | 2 | 27 | 0.0019 | KPPVPVAQANLAVRKNINDLTAEE +dK_MMCCH-2 (K) | 433 |
| 32 | 466 | 481 | 1098.4963 | 2194.9781 | 2194.9841 | -2.73 | G | 2 | 31 | 0.00082 | RFQNDKSVDGYQATVE +Deamidated (NQ); dK_MMCCH-2 (K) | 471 |
| 33 | 1215 | 1235 | 721.6063 | 2882.3959 | 2882.3949 | 0.35 | G | 3 | 28 | 0.0016 | ITQQLHDLDLHVGDNFFLKYE +dK_MMCCH-2 (K) | 1233 |
| 34 | 1292 | 1303 | 879.4652 | 1756.9159 | 1756.9182 | -1.31 | G | 0 | 42 | 6.80E-05 | SIRSAFLQIQKE +dK_MMCCH-2 (K) | 1302 |
| 35 | 1308 | 1320 | 603.6326 | 1807.8761 | 1807.8749 | 0.61 | G | 0 | 20 | 0.01 | NIAKFHGKPGLCE +dK_MMCCH-2 (K) | 1315 |
| 36 | 1403 | 1422 | 921.4208 | 2761.2407 | 2761.2330 | 2.79 | G | 3 | 15 | 0.03 | NAVTSRDPQPELWDNKDFYE +dK_MMCCH-2 (K) | 1418 |
| 37 | 1952 | 1967 | 792.6837 | 2375.0293 | 2375.0205 | 3.71 | G | 3 | 19 | 0.014 | FSKPEDTFDYHRFGYE +dK_MMCCH-2 (K) | 1954 |
| 38 | 2477 | 2484 | 644.8373 | 1287.6600 | 1287.6573 | 2.10 | G | 0 | 22 | 0.0068 | APFFIKVE +dK_MMCCH-2 (K) | 2482 |
| 39 | 3386 | 3405 | 877.4165 | 2629.2277 | 2629.2271 | 0.23 | G | 2 | 21 | 0.0073 | LDHAYSLRDGHYYIAGPTKD +dK_MMCCH-2 (K) | 3404 |
| 40 | 38 | 55 | 1181.0392 | 2360.0638 | 2360.0478 | 6.78 | C | 3 | 14 | 0.036 | ALEKALDDLQQDDSNQGY +dK_MMCCH-2 (K) | 41 |
| 41 | 277 | 296 | 883.7539 | 2648.2399 | 2648.2329 | 2.64 | C | 4 | 39 | 0.00014 | DLTREHAKPADSFDYGRLGY +dK_MMCCH-2 (K) | 284 |
| 42 | 364 | 373 | 735.3606 | 1468.7066 | 1468.7094 | -1.91 | C | 2 | 22 | 0.0067 | LLGGPTEMKW +dK_MMCCH-2 (K) | 372 |
| 43 | 432 | 443 | 793.9159 | 1585.8173 | 1585.8174 | -0.06 | C | 0 | 26 | 0.0028 | DKPPVPVAQANL +dK_MMCCH-2 (K) | 433 |
| 44 | 629 | 635 | 539.2532 | 1076.4919 | 1076.4848 | 6.60 | C | 0 | 20 | 0.0095 | VGGSEKY +dK_MMCCH-2 (K) | 634 |
| 45 | 1007 | 1015 | 669.8709 | 1337.7271 | 1337.7265 | 0.52 | C | 1 | 16 | 0.025 | TDIAKQVLL +dK_MMCCH-2 (K) | 1011 |
| 46 | 1075 | 1080 | 544.7770 | 1087.5395 | 1087.5372 | 2.11 | C | 1 | 26 | 0.0024 | QALQKY +dK_MMCCH-2 (K) | 1079 |
| 47 | 1125 | 1134 | 854.8459 | 1707.6772 | 1707.6763 | 0.59 | C | 3 | 33 | 0.00052 | DYKNNFDYEY +dK_MMCCH-2 (K) | 1127 |
| 48 | 1162 | 1177 | 690.0399 | 2067.0979 | 2067.0863 | 5.61 | C | 4 | 18 | 0.015 | AGFLLHEIGQSALVKF +dK_MMCCH-2 (K) | 1176 |
| 49 | 1298 | 1306 | 715.3727 | 1428.7309 | 1428.7323 | -0.91 | C | 1 | 20 | 0.01 | LQIQKEGIY +dK_MMCCH-2 (K) | 1302 |
| 50 | 1298 | 1312 | 824.0880 | 2469.2422 | 2469.2324 | 3.97 | C | 2 | 13 | 0.047 | LQIQKEGIYENIAKF +2 dK_MMCCH-2 (K) | 1302, 1311 |
| 51 | 1416 | 1426 | 863.3786 | 1724.7426 | 1724.7426 | 0.06 | C | 2 | 27 | 0.0022 | DNKDFYENVML +dK_MMCCH-2 (K) | 1418 |

**Figure 24B. KLH 2 (continued)**

| | | | | | | | | | | | | |
|---|---|---|---|---|---|---|---|---|---|---|---|---|
| 52 | 1526 | 1540 | 749.6839 | 2246.0299 | 2246.0215 | 3.74 | C | 2 | 47 | 1.80E-05 | KHNLPQDSFDYQNRF +dK_MMCCH-2 (K) | 1526 |
| 53 | 1589 | 1606 | 784.3566 | 2350.0479 | 2350.0392 | 3.70 | C | 0 | 31 | 0.00076 | ICVEQGGEQNCKTKAGSF +dK_MMCCH-2 (K) | 1600 |
| 54 | 1589 | 1606 | 784.3541 | 2350.0404 | 2350.0392 | 0.51 | C | 0 | 30 | 0.001 | ICVEQGGEQNCKTKAGSF +dK_MMCCH-2 (K) | 1602 |
| 55 | 1696 | 1702 | 607.2995 | 1212.5844 | 1212.5849 | -0.41 | C | 1 | 27 | 0.002 | TTLEKHF +dK_MMCCH-2 (K) | 1700 |
| 56 | 1708 | 1719 | 839.8421 | 1677.6696 | 1677.6651 | 2.74 | C | 0 | 38 | 0.00017 | KNMQADDSPDGY +dK_MMCCH-2 (K) | 1708 |
| 57 | 1801 | 1809 | 706.8386 | 1411.6627 | 1411.6595 | 2.27 | C | 0 | 24 | 0.0044 | SHKTFPNPF +dK_MMCCH-2 (K) | 1803 |
| 58 | 1985 | 1995 | 850.4311 | 1698.8476 | 1698.8399 | 4.53 | C | 0 | 39 | 0.00012 | IKQQQEADRVF +dK_MMCCH-2 (K) | 1986 |
| 59 | 2001 | 2011 | 726.8469 | 1451.6792 | 1451.6755 | 2.48 | C | 1 | 27 | 0.0021 | KGFGQSASVSF +dK_MMCCH-2 (K) | 2001 |
| 60 | 2194 | 2207 | 1042.5349 | 2083.0553 | 2083.0489 | 3.07 | C | 3 | 15 | 0.035 | WDWTKPISKLPDLF +dK_MMCCH-2 (K) | 2202 |
| 61 | 2230 | 2236 | 582.2687 | 1162.5228 | 1162.5216 | 1.03 | C | 0 | 14 | 0.042 | IKSEDAY +dK_MMCCH-2 (K) | 2231 |
| 62 | 2463 | 2479 | 1128.0164 | 2254.0182 | 2254.0253 | -3.15 | C | 1 | 18 | 0.015 | KAQSIHPEDVFDTDAPF +dK_MMCCH-2 (K) | 2463 |
| 63 | 2506 | 2532 | 761.6029 | 3042.3825 | 3042.3738 | 2.89 | C | 0 | 17 | 0.021 | SAGEGHTDDHGSDHIAGSGVRKDVTSL +dK_MMCCH-2 (K) | 2527 |
| 64 | 2754 | 2760 | 598.3301 | 1194.6457 | 1194.6471 | -1.17 | C | 1 | 15 | 0.029 | KQPLKPF +dK_MMCCH-2 (K) | 2754 |
| 65 | 2754 | 2760 | 598.3317 | 1194.6489 | 1194.6471 | 1.51 | C | 1 | 23 | 0.005 | KQPLKPF +dK_MMCCH-2 (K) | 2758 |
| 66 | 2865 | 2872 | 673.3643 | 1344.7141 | 1344.7112 | 2.16 | C | 1 | 13 | 0.046 | RYDITKVL +dK_MMCCH-2 (K) | 2870 |
| 67 | 3235 | 3242 | 617.3138 | 1232.6131 | 1232.6111 | 1.62 | C | 0 | 20 | 0.0099 | TSANVKIY +dK_MMCCH-2 (K) | 3240 |
| 68 | 3399 | 3406 | 576.8038 | 1151.5930 | 1151.5896 | 2.87 | C | 0 | 20 | 0.0093 | IAGPTKDL +dK_MMCCH-2 (K) | 3404 |
| 69 | 69 | 75 | 604.2924 | 1206.5703 | 1206.5703 | 0.00 | Th | 0 | 22 | 0.0069 | VDKHEKN +dK_MMCCH-2 (K) | 74 |
| 70 | 165 | 175 | 558.6142 | 1672.8208 | 1672.8144 | 3.83 | Th | 1 | 23 | 0.0053 | FEKVQPGHHTR +dK_MMCCH-2 (K) | 167 |
| 71 | 180 | 191 | 909.9193 | 1817.8241 | 1817.8216 | 1.38 | Th | 4 | 57 | 2.20E-06 | ILDALEQDEFCK +dK_MMCCH-2 (K) | 191 |
| 72 | 247 | 260 | 631.6202 | 1891.8389 | 1891.8305 | 4.44 | Th | 2 | 15 | 0.032 | LRGKDPNSADCAHN +dK_MMCCH-2 (K) | 250 |
| 73 | 283 | 293 | 782.8608 | 1563.7070 | 1563.7028 | 2.69 | Th | 3 | 39 | 0.00014 | AKPADSFDYGR +dK_MMCCH-2 (K) | 284 |
| 74 | 365 | 372 | 585.7805 | 1169.5465 | 1169.5461 | 0.34 | Th | 1 | 43 | 5.50E-05 | LGGPTEMK +dK_MMCCH-2 (K) | 372 |
| 75 | 431 | 437 | 569.2866 | 1136.5586 | 1136.5576 | 0.79 | Th | 1 | 13 | 0.046 | FDKPPVP +dK_MMCCH-2 (K) | 433 |
| 76 | 467 | 472 | 538.7430 | 1075.4714 | 1075.4644 | 6.51 | Th | 0 | 14 | 0.045 | FQNDKS +dK_MMCCH-2 (K) | 471 |
| 77 | 554 | 565 | 811.8927 | 1621.7708 | 1621.7658 | 3.14 | Th | 1 | 15 | 0.029 | LDPVTGETKNNP +dK_MMCCH-2 (K) | 562 |
| 78 | 629 | 636 | 582.7668 | 1163.5191 | 1163.5169 | 1.98 | Th | 1 | 22 | 0.006 | VGGSEKYS +dK_MMCCH-2 (K) | 634 |

**Figure 24B. KLH 2 (continued)**

| | | | | | | | | | | | | |
|---|---|---|---|---|---|---|---|---|---|---|---|---|
| 79 | 893 | 904 | 904.8956 | 1807.7767 | 1807.7698 | 3.82 | Th | 1 | 25 | 0.0035 | FHGEPKWCPSPE +dK_MMCCH-2 (K) | 898 |
| 80 | 1362 | 1374 | 968.4326 | 1934.8507 | 1934.8397 | 5.69 | Th | 4 | 16 | 0.026 | YWDWTEKADSLPS +dK_MMCCH-2 (K) | 1368 |
| 81 | 1414 | 1419 | 564.7549 | 1127.4952 | 1127.4957 | -0.44 | Th | 1 | 15 | 0.029 | LWDNKD +dK_MMCCH-2 (K) | 1418 |
| 82 | 1525 | 1539 | 738.3549 | 2212.0429 | 2212.0371 | 2.62 | Th | 3 | 48 | 1.50E-05 | LKHNLPQDSFDYQNR +dK_MMCCH-2 (K) | 1526 |
| 83 | 1589 | 1605 | 735.3308 | 2202.9706 | 2202.9708 | -0.09 | Th | 2 | 18 | 0.017 | ICVEQGGEQNCKTKAGS +dK_MMCCH-2 (K) | 1600 |
| 84 | 1589 | 1605 | 735.3312 | 2202.9717 | 2202.9708 | 0.41 | Th | 2 | 39 | 0.00012 | ICVEQGGEQNCKTKAGS +dK_MMCCH-2 (K) | 1602 |
| 85 | 1800 | 1809 | 780.3749 | 1558.7353 | 1558.7279 | 4.75 | Th | 2 | 18 | 0.016 | FSHKTFPNPF +dK_MMCCH-2 (K) | 1803 |
| 86 | 1952 | 1963 | 940.4234 | 1878.8322 | 1878.8247 | 4.05 | Th | 2 | 28 | 0.0015 | FSKPEDTFDYHR +dK_MMCCH-2 (K) | 1954 |
| 87 | 1985 | 1994 | 776.8957 | 1551.7768 | 1551.7715 | 3.42 | Th | 2 | 22 | 0.0062 | IKQQQEADRV +dK_MMCCH-2 (K) | 1986 |
| 88 | 2193 | 2199 | 667.3019 | 1332.5892 | 1332.5849 | 3.23 | Th | 2 | 27 | 0.0021 | YWDWTKP +dK_MMCCH-2 (K) | 2198 |
| 89 | 2200 | 2206 | 562.3099 | 1122.6052 | 1122.5995 | 5.08 | Th | 2 | 24 | 0.004 | ISKLPDL +dK_MMCCH-2 (K) | 2202 |
| 90 | 2225 | 2229 | 462.2227 | 922.4309 | 922.4259 | 5.42 | Th | 2 | 17 | 0.022 | FAKGY +dK_MMCCH-2 (K) | 2227 |
| 91 | 2359 | 2363 | 509.2313 | 1016.4481 | 1016.4460 | 2.07 | Th | 1 | 14 | 0.04 | FTKMH +dK_MMCCH-2 (K); Oxidation (M) | 2361 |
| 92 | 2941 | 2950 | 720.8409 | 1439.6672 | 1439.6602 | 4.86 | Th | 3 | 42 | 5.90E-05 | LDEANDLKNA +dK_MMCCH-2 (K) | 2948 |
| 93 | 3009 | 3015 | 573.2752 | 1144.5359 | 1144.5335 | 2.01 | Th | 0 | 15 | 0.031 | LKEHGSH +dK_MMCCH-2 (K) | 3010 |
| 94 | 3020 | 3026 | 662.2903 | 1322.5661 | 1322.5642 | 1.51 | Th | 2 | 28 | 0.0017 | YWDWTKS +dK_MMCCH-2 (K) | 3025 |
| 95 | 3399 | 3405 | 520.2604 | 1038.5062 | 1038.5056 | 0.58 | Th | 1 | 27 | 0.002 | IAGPTKD +dK_MMCCH-2 (K) | 3404 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the lysine conjugation site for KLH1 or KLH2.

**Figure 25. Identification details of MMCCH-conjugated peptides for sample 4 (1st LC-MS/MS)**

**Figure 25A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 124 | 141 | 785.4103 | 2353.2092 | 2353.2001 | 3.87 | T | 1 | 19 | 0.011 | GFTDPPVKHHQSANLLVR +dK_MMCCH-2 (K) | 131 |
| 2 | 142 | 149 | 656.3425 | 1310.6705 | 1310.6653 | 4.04 | T | 1 | 35 | 0.00034 | KNINDLTR +dK_MMCCH-2 (K) | 142 |
| 3 | 157 | 166 | 822.8790 | 1643.7435 | 1643.7402 | 2.01 | T | 1 | 18 | 0.016 | EAFHKFQEDR +dK_MMCCH-2 (K) | 161 |
| 4 | 184 | 192 | 726.8442 | 1451.6738 | 1451.6649 | 6.13 | T | 1 | 22 | 0.0061 | CPRPDAKDR +dK_MMCCH-2 (K) | 190 |
| 5 | 470 | 488 | 748.0502 | 2241.1289 | 2241.1100 | 8.43 | T | 1 | 24 | 0.0039 | TTDSVQHKAGTFAVLGGSK +dK_MMCCH-2 (K) | 477 |
| 6 | 553 | 560 | 663.8695 | 1325.7245 | 1325.7166 | 5.96 | T | 2 | 22 | 0.0058 | VKFDKVPR +dK_MMCCH-2 (K) | 554 |
| 7 | 553 | 560 | 555.6243 | 1663.8510 | 1663.8466 | 2.64 | T | 2 | 15 | 0.029 | VKFDKVPR +2 dK_MMCCH-2 (K) | 554, 557 |
| 8 | 566 | 570 | 485.2455 | 968.4765 | 968.4749 | 1.65 | T | 1 | 17 | 0.022 | KNVDR +dK_MMCCH-2 (K) | 566 |
| 9 | 581 | 590 | 733.9194 | 1465.8242 | 1465.8214 | 1.91 | T | 2 | 40 | 0.00011 | KALALLKEDK +dK_MMCCH-2 (K) | 581 |
| 10 | 588 | 606 | 781.0386 | 2340.0941 | 2340.0845 | 4.10 | T | 1 | 21 | 0.008 | EDKSAGGFQQLGAFHGEPK +dK_MMCCH-2 (K) | 590 |
| 11 | 591 | 615 | 1004.4697 | 3010.3872 | 3010.3742 | 4.32 | T | 1 | 18 | 0.015 | SAGGFQQLGAFHGEPKWCPSPEASK +dK_MMCCH-2 (K) | 606 |
| 12 | 645 | 681 | 1158.5359 | 4630.1145 | 4630.1063 | 1.75 | T | 1 | 21 | 0.0072 | HGYDGALPYWDWTSPLNHLPELADHEKYVDPEDGVEK +dK_MMCCH-2 (K) | 671 |
| 13 | 682 | 699 | 620.2941 | 2477.1474 | 2477.1434 | 1.61 | T | 1 | 23 | 0.0045 | HNPWFDGHIDTVDKTTTR +dK_MMCCH-2 (K) | 695 |
| 14 | 700 | 719 | 887.7753 | 2660.3040 | 2660.2945 | 3.57 | T | 1 | 32 | 0.00064 | SVQNKLFEQPEFGHYTSIAK +dK_MMCCH-2 (K) | 704 |
| 15 | 790 | 804 | 1003.4777 | 2004.9409 | 2004.9220 | 9.43 | T | 0 | 63 | 5.00E-07 | GKPYNVANCAVTSMR +dK_MMCCH-2 (K) | 791 |
| 16 | 953 | 965 | 635.3326 | 1902.9761 | 1902.9662 | 5.20 | T | 1 | 46 | 2.40E-05 | DLFKQPSVIHEPR +dK_MMCCH-2 (K) | 956 |
| 17 | 986 | 1000 | 685.0287 | 2052.0642 | 2052.0561 | 4.00 | T | 1 | 68 | 1.80E-07 | KNIENLSLGELESLR +dK_MMCCH-2 (K) | 986 |
| 18 | 1048 | 1060 | 913.5324 | 1825.0503 | 1825.0423 | 4.38 | T | 1 | 19 | 0.013 | LYVVVVENALLKK +dK_MMCCH-2 (K) | 1060 |
| 19 | 1092 | 1114 | 633.7048 | 3163.4875 | 3163.4682 | 6.07 | T | 1 | 40 | 0.0001 | QHHYETNPFHHGKITHENEITTR +dK_MMCCH-2 (K) | 1104 |
| 20 | 1199 | 1203 | 353.1928 | 1056.5566 | 1056.5538 | 2.65 | T | 1 | 17 | 0.019 | KRPYR +dK_MMCCH-2 (K) | 1199 |
| 21 | 1395 | 1403 | 485.5976 | 1453.7709 | 1453.7599 | 7.57 | T | 2 | 29 | 0.0014 | KDITQLDKR +dK_MMCCH-2 (K) | 1395 |
| 22 | 1395 | 1403 | 598.3081 | 1791.9025 | 1791.8899 | 7.03 | T | 2 | 15 | 0.03 | KDITQLDKR +2 dK_MMCCH-2 (K) | 1395, 1402 |
| 23 | 1473 | 1489 | 778.4055 | 2332.1946 | 2332.1827 | 5.10 | T | 1 | 46 | 2.80E-05 | KHGAVVGLPYWDWTLPR +dK_MMCCH-2 (K) | 1473 |
| 24 | 1721 | 1746 | 1087.4788 | 3259.4145 | 3259.3937 | 6.38 | T | 1 | 15 | 0.031 | TAGDCEDAGYFTVLGGEKEMPWAFDR +dK_MMCCH-2 (K) | 1738 |

**Figure 25A. KLH 1 (continued)**

| | | | | | | | | | | | | |
|---|---|---|---|---|---|---|---|---|---|---|---|---|
| 25 | 1747 | 1758 | 613.9782 | 1838.9128 | 1838.9012 | 6.31 | T | 1 | 27 | 0.0019 | LYKYDITETLDK +dK_MMCCH-2 (K) | 1749 |
| 26 | 1890 | 1922 | 1099.5339 | 4394.1067 | 4394.1048 | 0.43 | T | 1 | 13 | 0.048 | HGSSVAVPYWDWTKPIHNIPHLFTDKEYYDVWR +dK_MMCCH-2 (K) | 1915 |
| 27 | 1923 | 1932 | 756.3811 | 1510.7476 | 1510.7425 | 3.44 | T | 1 | 21 | 0.0077 | NKVMPNPFAR +dK_MMCCH-2 (K) | 1924 |
| 28 | 2100 | 2109 | 492.2501 | 1964.9713 | 1964.9601 | 5.70 | T | 2 | 21 | 0.0075 | EIKDKQHHVR +2 dK_MMCCH-2 (K) | 2102, 2104 |
| 29 | 2121 | 2149 | 890.4190 | 3557.6469 | 3557.6378 | 2.56 | T | 1 | 29 | 0.0012 | TSADVQFQICKTSEDCHHGGQIFVLGGTK +dK_MMCCH-2 (K) | 2131 |
| 30 | 2359 | 2367 | 736.3516 | 1470.6887 | 1470.6813 | 5.03 | T | 1 | 35 | 0.00032 | DKLFNDPER +dK_MMCCH-2 (K) | 2360 |
| 31 | 2507 | 2514 | 682.3550 | 1362.6955 | 1362.6853 | 7.49 | T | 1 | 22 | 0.0065 | KLEHELEK +dK_MMCCH-2 (K) | 2507 |
| 32 | 2515 | 2520 | 571.7631 | 1141.5117 | 1141.5074 | 3.77 | T | 1 | 19 | 0.013 | QKEEDR +dK_MMCCH-2 (K) | 2516 |
| 33 | 2621 | 2636 | 802.7125 | 2405.1157 | 2405.1083 | 3.08 | T | 1 | 47 | 2.10E-05 | HHEKHHEDHHEDILVR +dK_MMCCH-2 (K) | 2624 |
| 34 | 2637 | 2651 | 713.3571 | 2137.0495 | 2137.0374 | 5.66 | T | 1 | 69 | 1.40E-07 | KNIHSLSHHEAEELR +dK_MMCCH-2 (K) | 2637 |
| 35 | 2657 | 2686 | 1246.2233 | 3735.6480 | 3735.6358 | 3.24 | T | 1 | 17 | 0.019 | LQNDESHGGYEHIAGFHGYPNLCPEKGDEK +dK_MMCCH-2 (K) | 2686 |
| 36 | 2851 | 2883 | 807.5757 | 4032.8423 | 4032.8346 | 1.91 | T | 0 | 45 | 3.50E-05 | VKPAHAGSCAGDIMHVPLHPFNYESVNNDDFTR +dK_MMCCH-2 (K) | 2852 |
| 37 | 2897 | 2919 | 800.1410 | 3196.5351 | 3196.5287 | 2.00 | T | 1 | 53 | 5.10E-06 | FNYKYDNLNLHGHNIEELEEVLR +dK_MMCCH-2 (K) | 2900 |
| 38 | 3003 | 3021 | 825.7765 | 2474.3076 | 2474.2879 | 7.96 | T | 1 | 41 | 7.90E-05 | KYDHTELDASVLPAPIIVR +dK_MMCCH-2 (K) | 3003 |
| 39 | 123 | 151 | 807.4167 | 4032.0474 | 4032.0350 | 3.08 | G | 2 | 24 | 0.0036 | KGFTDPPVKHHQSANLLVRKNINDLTREE +2 dK_MMCCH-2 (K) | 123, 131 |
| 40 | 123 | 151 | 739.7923 | 3693.9251 | 3693.9049 | 5.47 | G | 2 | 33 | 0.00049 | KGFTDPPVKHHQSANLLVRKNINDLTREE +dK_MMCCH-2 (K) | 131 |
| 41 | 158 | 176 | 846.7141 | 2537.1203 | 2537.1169 | 1.34 | G | 3 | 23 | 0.0052 | AFHKFQEDRSVDGYQATAE +Deamidated (NQ); dK_MMCCH-2 (K) | 161 |
| 42 | 435 | 442 | 676.3256 | 1350.6366 | 1350.6312 | 4.00 | G | 1 | 14 | 0.038 | NIEKMIHE +dK_MMCCH-2 (K) | 438 |
| 43 | 526 | 544 | 777.7495 | 2330.2265 | 2330.2304 | -1.67 | G | 1 | 29 | 0.0013 | VDGTKLASSLIPHASVIRE +dK_MMCCH-2 (K) | 530 |
| 44 | 589 | 612 | 1102.8308 | 3305.4706 | 3305.4621 | 2.60 | G | 1 | 14 | 0.037 | DKSAGGFQQLGAFHGEPKWCPSPE +2 dK_MMCCH-2 (K) | 590, 606 |
| 45 | 589 | 612 | 990.1188 | 2967.3345 | 2967.3320 | 0.84 | G | 1 | 28 | 0.0015 | DKSAGGFQQLGAFHGEPKWCPSPE +dK_MMCCH-2 (K) | 606 |
| 46 | 825 | 841 | 820.3754 | 2458.1045 | 2458.0940 | 4.27 | G | 1 | 25 | 0.003 | HSVPFNVFDYKTNFNYE +dK_MMCCH-2 (K) | 835 |
| 47 | 847 | 860 | 965.0179 | 1928.0212 | 1928.0077 | 7.00 | G | 0 | 17 | 0.018 | FNGLSISQLNKKLE +dK_MMCCH-2 (K) | 858 |
| 48 | 942 | 970 | 908.9860 | 3631.9147 | 3631.8973 | 4.79 | G | 2 | 20 | 0.01 | VFDLKPASLGKDLFKQPSVIHEPRIGHHE +dK_MMCCH-2 (K) | 956 |
| 49 | 1055 | 1076 | 810.1685 | 3236.6450 | 3236.6402 | 1.45 | G | 1 | 14 | 0.041 | NALLKKGSSVAVPYWDWTKRIE +2 dK_MMCCH-2 (K) | 1059, 1060 |
| 50 | 1055 | 1076 | 725.6360 | 2898.5149 | 2898.5102 | 1.62 | G | 1 | 18 | 0.018 | NALLKKGSSVAVPYWDWTKRIE +dK_MMCCH-2 (K) | 1073 |
| 51 | 1097 | 1110 | 999.9629 | 1997.9112 | 1997.9054 | 2.90 | G | 1 | 20 | 0.011 | TNPFHHGKITHENE +dK_MMCCH-2 (K) | 1104 |

**Figure 25A. KLH 1 (continued)**

| | | | | | | | | | | | | |
|---|---|---|---|---|---|---|---|---|---|---|---|---|
| 52 | 1376 | 1396 | 864.3887 | 2590.1444 | 2590.1428 | 0.58 | G | 3 | 27 | 0.0021 | AGTDSAHTDDGHTEPVMIRKD +dK_MMCCH-2 (K) | 1395 |
| 53 | 1397 | 1413 | 774.4371 | 2320.2894 | 2320.2824 | 3.02 | G | 1 | 15 | 0.035 | ITQLDKRQQLSLVKALE +dK_MMCCH-2 (K) | 1402 |
| 54 | 1397 | 1413 | 774.4396 | 2320.2969 | 2320.2824 | 6.25 | G | 1 | 21 | 0.0089 | ITQLDKRQQLSLVKALE +dK_MMCCH-2 (K) | 1410 |
| 55 | 1506 | 1522 | 753.3802 | 2257.1187 | 2257.1089 | 4.34 | G | 2 | 27 | 0.0022 | TGRDIPNPFIGSKIEFE +dK_MMCCH-2 (K) | 1518 |
| 56 | 1755 | 1765 | 570.6056 | 1708.7949 | 1708.7913 | 2.17 | G | 2 | 31 | 0.00077 | TLDKMNLRHDE +dK_MMCCH-2 (K) | 1758 |
| 57 | 2059 | 2078 | 777.1311 | 3104.4953 | 3104.4888 | 2.09 | G | 1 | 18 | 0.018 | INHNQFTKKHAVPNDVFKYE +2 dK_MMCCH-2 (K) | 2067, 2076 |
| 58 | 2089 | 2100 | 854.4106 | 1706.8066 | 1706.8008 | 3.46 | G | 2 | 29 | 0.0012 | IGGMNLHEIEKE +dK_MMCCH-2 (K) | 2099 |
| 59 | 2647 | 2661 | 715.6794 | 2144.0163 | 2144.0095 | 3.17 | G | 4 | 29 | 0.0012 | AEELRDALYKLQNDE +dK_MMCCH-2 (K) | 2656 |
| 60 | 2765 | 2774 | 753.8675 | 1505.7204 | 1505.7225 | -1.33 | G | 0 | 14 | 0.041 | AIFQQTKFGE +dK_MMCCH-2 (K) | 2771 |
| 61 | 3090 | 3097 | 632.8040 | 1263.5935 | 1263.5879 | 4.43 | G | 0 | 16 | 0.027 | LGKMYSVE +dK_MMCCH-2 (K) | 3092 |
| 62 | 94 | 100 | 573.2996 | 1144.5847 | 1144.5838 | 0.79 | C | 1 | 16 | 0.027 | TVKAELF +dK_MMCCH-2 (K) | 96 |
| 63 | 468 | 481 | 935.9766 | 1869.9387 | 1869.9295 | 4.92 | C | 0 | 22 | 0.0061 | IKTTDSVQHKAGTF +dK_MMCCH-2 (K) | 469 |
| 64 | 584 | 595 | 787.3998 | 1572.7851 | 1572.7858 | -0.38 | C | 2 | 32 | 0.00063 | ALLKEDKSAGGF +dK_MMCCH-2 (K) | 590 |
| 65 | 715 | 723 | 655.8738 | 1309.7331 | 1309.7316 | 1.22 | C | 1 | 15 | 0.029 | TSIAKQVLL +dK_MMCCH-2 (K) | 719 |
| 66 | 783 | 788 | 544.7775 | 1087.5404 | 1087.5372 | 2.94 | C | 1 | 34 | 0.0004 | QALQKY +dK_MMCCH-2 (K) | 787 |
| 67 | 830 | 838 | 743.3415 | 1484.6684 | 1484.6646 | 2.56 | C | 2 | 16 | 0.025 | NVFDYKTNF +dK_MMCCH-2 (K) | 835 |
| 68 | 860 | 869 | 789.8851 | 1577.7556 | 1577.7548 | 0.51 | C | 1 | 13 | 0.047 | EAIKSQDRFF +dK_MMCCH-2 (K) | 863 |
| 69 | 956 | 974 | 638.3217 | 2549.2578 | 2549.2485 | 3.65 | C | 0 | 15 | 0.029 | KQPSVIHEPRIGHHEGEVY +dK_MMCCH-2 (K) | 956 |
| 70 | 1330 | 1337 | 620.3554 | 1238.6963 | 1238.6944 | 1.45 | C | 1 | 26 | 0.0028 | KLDITKAL +dK_MMCCH-2 (K) | 1335 |
| 71 | 1580 | 1586 | 544.2607 | 1086.5069 | 1086.5056 | 1.20 | C | 0 | 14 | 0.038 | VGGKEPY +dK_MMCCH-2 (K) | 1583 |
| 72 | 1649 | 1664 | 752.3309 | 2253.9708 | 2253.9637 | 3.15 | C | 2 | 17 | 0.02 | NLNDHTHDFSKPEDTF +dK_MMCCH-2 (K) | 1659 |
| 73 | 1818 | 1827 | 753.3695 | 1504.7245 | 1504.7232 | 0.86 | C | 1 | 25 | 0.0031 | SERDIGSLKY +dK_MMCCH-2 (K) | 1826 |
| 74 | 2076 | 2082 | 612.3039 | 1222.5932 | 1222.5944 | -0.90 | C | 3 | 19 | 0.013 | KYELLGY +dK_MMCCH-2 (K) | 2076 |
| 75 | 2476 | 2488 | 923.9219 | 1845.8293 | 1845.8244 | 2.71 | C | 1 | 20 | 0.0097 | THSNAKPTDVFEY +dK_MMCCH-2 (K) | 2481 |
| 76 | 2656 | 2666 | 793.3512 | 1584.6878 | 1584.6879 | 0.00 | C | 1 | 22 | 0.0057 | KLQNDESHGGY +dK_MMCCH-2 (K) | 2656 |
| 77 | 2713 | 2723 | 787.9122 | 1573.8099 | 1573.8075 | 1.52 | C | 1 | 14 | 0.042 | KKHGSHLGIPY +dK_MMCCH-2 (K) | 2714 |
| 78 | 2809 | 2815 | 538.2603 | 1074.5059 | 1074.5056 | 0.37 | C | 0 | 19 | 0.014 | IGGAEKY +dK_MMCCH-2 (K) | 2814 |

Figure 25A. KLH 1 (continued)

| | | | | | | | | | | | | |
|---|---|---|---|---|---|---|---|---|---|---|---|---|
| 79 | 2934 | 2945 | 816.4465 | 1630.8785 | 1630.8753 | 1.96 | C | 0 | 17 | 0.02 | SGIRTTAVVKVY +dK_MMCCH-2 (K) | 2943 |
| 80 | 2946 | 2960 | 965.4076 | 1928.8007 | 1928.7986 | 1.09 | C | 1 | 26 | 0.0027 | IKSGTDSDDEYAGSF +dK_MMCCH-2 (K) | 2947 |
| 81 | 2964 | 2971 | 607.2730 | 1212.5315 | 1212.5307 | 0.58 | C | 0 | 20 | 0.0092 | GGAKEMPW +dK_MMCCH-2 (K) | 2967 |
| 82 | 1 | 9 | 725.3406 | 1448.6667 | 1448.6567 | 6.90 | Th | 2 | 23 | 0.0054 | MTPEELKTY +dK_MMCCH-2 (K) | 7 |
| 83 | 38 | 56 | 861.6813 | 2582.0222 | 2582.0108 | 4.42 | Th | 2 | 13 | 0.047 | VCIPDDNDRNDDHCEKAGD +dK_MMCCH-2 (K) | 53 |
| 84 | 59 | 69 | 785.3785 | 1568.7424 | 1568.7367 | 3.63 | Th | 3 | 66 | 2.60E-07 | VLGGPSEMKWQ +dK_MMCCH-2 (K) | 67 |
| 85 | 78 | 84 | 569.2871 | 1136.5597 | 1136.5536 | 5.37 | Th | 1 | 49 | 1.20E-05 | LSDTVHK +dK_MMCCH-2 (K) | 84 |
| 86 | 116 | 124 | 447.2341 | 1338.6804 | 1338.6755 | 3.66 | Th | 2 | 31 | 0.00079 | VVVHHPEKG +dK_MMCCH-2 (K) | 123 |
| 87 | 125 | 135 | 815.8884 | 1629.7623 | 1629.7610 | 0.80 | Th | 1 | 16 | 0.023 | FTDPPVKHHQS +dK_MMCCH-2 (K) | 131 |
| 88 | 431 | 439 | 707.8542 | 1413.6938 | 1413.6883 | 3.89 | Th | 3 | 36 | 0.00025 | ISLENIEKM +dK_MMCCH-2 (K) | 438 |
| 89 | 519 | 530 | 807.9007 | 1613.7868 | 1613.7859 | 0.62 | Th | 3 | 60 | 9.80E-07 | VTVDITEVDGTK +dK_MMCCH-2 (K) | 530 |
| 90 | 585 | 591 | 585.8112 | 1169.6079 | 1169.6002 | 6.58 | Th | 1 | 26 | 0.0023 | LLKEDKS +dK_MMCCH-2 (K) | 590 |
| 91 | 601 | 612 | 904.8990 | 1807.7834 | 1807.7698 | 7.52 | Th | 1 | 25 | 0.0032 | FHGEPKWCPSPE +dK_MMCCH-2 (K) | 606 |
| 92 | 666 | 672 | 607.2826 | 1212.5507 | 1212.5485 | 1.90 | Th | 2 | 24 | 0.0039 | LADHEKY +dK_MMCCH-2 (K) | 671 |
| 93 | 829 | 837 | 743.3404 | 1484.6662 | 1484.6646 | 1.08 | Th | 3 | 21 | 0.0087 | FNVFDYKTN +dK_MMCCH-2 (K) | 835 |
| 94 | 1066 | 1074 | 530.2600 | 1587.7580 | 1587.7544 | 2.27 | Th | 3 | 14 | 0.043 | VPYWDWTKR +dK_MMCCH-2 (K) | 1073 |
| 95 | 1111 | 1120 | 742.3792 | 1482.7439 | 1482.7388 | 3.37 | Th | 1 | 16 | 0.025 | ITTRDPKDSL +dK_MMCCH-2 (K) | 1117 |
| 96 | 1219 | 1232 | 766.6617 | 2296.9634 | 2296.9617 | 0.74 | Th | 1 | 17 | 0.018 | FDKSDNNDEATKTH +2 dK_MMCCH-2 (K) | 1221, 1230 |
| 97 | 1219 | 1232 | 980.4233 | 1958.8321 | 1958.8316 | 0.26 | Th | 1 | 38 | 0.00014 | FDKSDNNDEATKTH +dK_MMCCH-2 (K) | 1230 |
| 98 | 1657 | 1668 | 921.9182 | 1841.8219 | 1841.8182 | 2.01 | Th | 2 | 56 | 2.60E-06 | FSKPEDTFDYQK +dK_MMCCH-2 (K) | 1659 |
| 99 | 1657 | 1668 | 921.9215 | 1841.8283 | 1841.8182 | 5.48 | Th | 2 | 57 | 2.20E-06 | FSKPEDTFDYQK +dK_MMCCH-2 (K) | 1668 |
| 100 | 1733 | 1741 | 664.3221 | 1326.6297 | 1326.6312 | -1.13 | Th | 2 | 18 | 0.016 | VLGGEKEMP +dK_MMCCH-1 (K); Oxidation (M) | 1738 |
| 101 | 1894 | 1904 | 850.4122 | 1698.8098 | 1698.8116 | -1.06 | Th | 5 | 37 | 0.00022 | VAVPYWDWTKP +dK_MMCCH-2 (K) | 1903 |
| 102 | 2064 | 2068 | 499.7597 | 997.5048 | 997.5055 | -0.70 | Th | 0 | 21 | 0.008 | FTKKH +dK_MMCCH-2 (K) | 2067 |
| 103 | 2094 | 2100 | 618.3042 | 1234.5938 | 1234.5903 | 2.83 | Th | 1 | 27 | 0.0022 | LHEIEKE +dK_MMCCH-2 (K) | 2099 |
| 104 | 2238 | 2248 | 778.8801 | 1555.7457 | 1555.7440 | 1.09 | Th | 3 | 48 | 1.70E-05 | LTTAEVDNLKD +dK_MMCCH-2 (K) | 2247 |
| 105 | 2475 | 2490 | 746.3550 | 2236.0431 | 2236.0259 | 7.69 | Th | 4 | 22 | 0.006 | FTHSNAKPTDVFEYSR +dK_MMCCH-2 (K) | 2481 |

**Figure 25A. KLH 1 (continued)**

| | | | | | | | | | | | | |
|---|---|---|---|---|---|---|---|---|---|---|---|---|
| 106 | 2512 | 2521 | 807.3973 | 1612.7801 | 1612.7766 | 2.17 | Th | 0 | 29 | 0.0013 | LEKQKEEDRT +dK_MMCCH-2 (K) | 2514 |
| 107 | 2508 | 2521 | 708.0122 | 2121.0148 | 2121.0048 | 4.71 | Th | 1 | 17 | 0.019 | LEHELEKQKEEDRT +dK_MMCCH-2 (K) | 2516 |
| 108 | 2595 | 2606 | 806.4047 | 1610.7949 | 1610.7862 | 5.40 | Th | 2 | 28 | 0.0016 | IIDTSGKQLPSD +dK_MMCCH-2 (K) | 2601 |
| 109 | 2607 | 2612 | 520.7805 | 1039.5465 | 1039.5446 | 1.83 | Th | 2 | 19 | 0.013 | LIKMPT +dK_MMCCH-2 (K) | 2609 |
| 110 | 2766 | 2771 | 551.7868 | 1101.5590 | 1101.5529 | 5.63 | Th | 1 | 29 | 0.0012 | IFQQTK +dK_MMCCH-2 (K) | 2771 |
| 111 | 2808 | 2816 | 638.3200 | 1274.6255 | 1274.6217 | 2.98 | Th | 3 | 27 | 0.0018 | LIGGAEKYS +dK_MMCCH-2 (K) | 2814 |
| 112 | 2946 | 2960 | 965.4102 | 1928.8059 | 1928.7986 | 3.78 | Th | 3 | 30 | 0.0011 | IKSGTDSDDEYAGSF +dK_MMCCH-2 (K) | 2947 |
| 113 | 2961 | 2970 | 691.8640 | 1381.7135 | 1381.7098 | 2.68 | Th | 4 | 18 | 0.016 | VILGGAKEMP +dK_MMCCH-1 (K); Oxidation (M) | 2967 |
| 114 | 2990 | 2996 | 583.2845 | 1164.5544 | 1164.5485 | 5.07 | Th | 1 | 38 | 0.00017 | LTDDHVK +dK_MMCCH-2 (K) | 2996 |

**Figure 25B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1043.0275 | 2084.0404 | 2084.0347 | 2.74 | T | 1 | 49 | 1.20E-05 | KNVDSLSSDEVLALEK +dK_MMCCH-2 (K) | 26 |
| 2 | 147 | 155 | 694.3721 | 1386.7297 | 1386.7217 | 5.77 | T | 1 | 35 | 0.00029 | ADITFLNKK +dK_MMCCH-2 (K) | 155 |
| 3 | 164 | 175 | 596.3124 | 1785.9155 | 1785.8985 | 9.52 | T | 1 | 42 | 6.60E-05 | LFEKVQPGHHTR +dK_MMCCH-2 (K) | 167 |
| 4 | 249 | 271 | 987.4443 | 2959.3110 | 2959.3051 | 1.99 | T | 1 | 36 | 0.00024 | GKDPNSADCAHNLIHTPMEPFDR +dK_MMCCH-2 (K) | 250 |
| 5 | 281 | 293 | 610.9466 | 1829.8179 | 1829.8043 | 7.49 | T | 0 | 54 | 4.30E-06 | EHAKPADSFDYGR +dK_MMCCH-2 (K) | 284 |
| 6 | 419 | 446 | 677.3707 | 3381.8173 | 3381.8132 | 1.21 | T | 1 | 27 | 0.0022 | QPTLVHRPAKGHFDKPPVPVAQANLAVR +Deamidated (NQ); dK_MMCCH-2 (K) | 428 |
| 7 | 429 | 446 | 752.0682 | 2253.1829 | 2253.1729 | 4.44 | T | 0 | 52 | 5.90E-06 | GHFDKPPVPVAQANLAVR +dK_MMCCH-2 (K) | 433 |
| 8 | 467 | 488 | 708.5943 | 2830.3481 | 2830.3385 | 3.39 | T | 1 | 69 | 1.30E-07 | FQNDKSVDGYQATVEFHALPAR +dK_MMCCH-2 (K) | 471 |
| 9 | 489 | 497 | 726.8442 | 1451.6738 | 1451.6649 | 6.13 | T | 1 | 22 | 0.0061 | CPRPDAKDR +dK_MMCCH-2 (K) | 495 |
| 10 | 936 | 950 | 720.3452 | 2158.0138 | 2158.0095 | 1.99 | T | 1 | 30 | 0.00097 | KHGFTGGLPYWDWTR +dK_MMCCH-2 (K) | 936 |
| 11 | 1082 | 1096 | 996.4687 | 1990.9228 | 1990.9063 | 8.29 | T | 0 | 46 | 2.70E-05 | GKPYNTANCAIASMR +dK_MMCCH-2 (K) | 1083 |
| 12 | 1097 | 1115 | 827.0787 | 2478.2142 | 2478.2101 | 1.65 | T | 0 | 66 | 2.30E-07 | KPLQPFGLDSVINPDDETR +dK_MMCCH-2 (K) | 1097 |
| 13 | 1123 | 1149 | 1190.5612 | 3568.6617 | 3568.6497 | 3.36 | T | 1 | 79 | 1.20E-08 | VFDYKNNFDYEYESLAFNGLSIAQLDR +dK_MMCCH-2 (K) | 1127 |
| 14 | 1355 | 1385 | 1271.2694 | 3810.7864 | 3810.7876 | -0.31 | T | 1 | 38 | 0.00015 | GSAVAVPYWDWTEKADSLPSLINDATYFNSR +dK_MMCCH-2 (K) | 1368 |
| 15 | 1409 | 1450 | 1097.7141 | 5483.5342 | 5483.5213 | 2.35 | T | 1 | 40 | 9.60E-05 | DPQPELWDNKDFYENVMLALEQDNFCDFEIQLELIHNALHSR +dK_MMCCH-2 (K) | 1418 |
| 16 | 1455 | 1480 | 1117.8821 | 3350.6244 | 3350.6070 | 5.19 | T | 1 | 34 | 0.00044 | AKYSLSSLDYTAFDPVFFLHHANVDR +dK_MMCCH-2 (K) | 1456 |
| 17 | 1493 | 1507 | 721.6577 | 2161.9513 | 2161.9594 | -3.75 | T | 1 | 66 | 2.40E-07 | KKPYNEADCAVNEMR +dK_MMCCH-2 (K) | 1494 |
| 18 | 1542 | 1564 | 802.8964 | 3207.5566 | 3207.5448 | 3.68 | T | 1 | 19 | 0.013 | YQYDNLQFNHFSIQKLDQTIQAR +dK_MMCCH-2 (K) | 1556 |
| 19 | 1622 | 1633 | 591.9822 | 1772.9247 | 1772.9171 | 4.29 | T | 1 | 30 | 0.00093 | LYKFDITSALHK +dK_MMCCH-2 (K) | 1624 |
| 20 | 1690 | 1704 | 532.2896 | 2125.1293 | 2125.1241 | 2.45 | T | 2 | 23 | 0.0046 | KEVSSLTTLEKHFLR +dK_MMCCH-2 (K) | 1700 |
| 21 | 1757 | 1768 | 612.9798 | 1835.9176 | 1835.9128 | 2.61 | T | 1 | 16 | 0.027 | LYTVQFEDSLKR +dK_MMCCH-2 (K) | 1767 |
| 22 | 1949 | 1963 | 560.2533 | 2236.9841 | 2236.9735 | 4.74 | T | 0 | 48 | 1.60E-05 | TQEFSKPEDTFDYHR +dK_MMCCH-2 (K) | 1954 |
| 23 | 2218 | 2231 | 937.4839 | 1872.9532 | 1872.9444 | 4.70 | T | 1 | 23 | 0.0049 | DAVVNNPFAKGYIK +dK_MMCCH-2 (K) | 2231 |

**Figure 25B. KLH 2 (continued)**

| | | | | | | | | | | | | |
|---|---|---|---|---|---|---|---|---|---|---|---|---|
| 24 | 2400 | 2404 | 479.2450 | 956.4754 | 956.4749 | 0.52 | T | 1 | 16 | 0.025 | KSQTR +dK_MMCCH-2 (K) | 2400 |
| 25 | 2453 | 2463 | 839.9330 | 1677.8515 | 1677.8436 | 4.71 | T | 1 | 16 | 0.026 | NFKYDITQALK +dK_MMCCH-2 (K) | 2455 |
| 26 | 2527 | 2541 | 1007.5168 | 2013.0190 | 2013.0088 | 5.07 | T | 1 | 18 | 0.017 | KDVTSLTASEIENLR +dK_MMCCH-2 (K) | 2527 |
| 27 | 2755 | 2764 | 764.3846 | 1526.7546 | 1526.7439 | 7.01 | T | 0 | 37 | 0.00019 | QPLKPFSESR +Deamidated (NQ); dK_MMCCH-2 (K) | 2758 |
| 28 | 2810 | 2814 | 455.8921 | 1364.6544 | 1364.6468 | 5.50 | T | 2 | 15 | 0.031 | KKEER +2 dK_MMCCH-2 (K) | 2810, 2811 |
| 29 | 2866 | 2873 | 659.3627 | 1316.7108 | 1316.7050 | 4.40 | T | 1 | 16 | 0.023 | YDITKVLK +dK_MMCCH-2 (K) | 2870 |
| 30 | 3148 | 3155 | 629.3372 | 1256.6599 | 1256.6587 | 0.95 | T | 1 | 20 | 0.0093 | KKPYNAAK +dK_MMCCH-2 (K) | 3149 |
| 31 | 167 | 178 | 590.9643 | 1769.8710 | 1769.8705 | 0.28 | G | 0 | 22 | 0.0059 | KVQPGHHTRLME +dK_MMCCH-2 (K) | 167 |
| 32 | 311 | 322 | 567.6295 | 1699.8666 | 1699.8603 | 3.65 | G | 1 | 17 | 0.022 | LNVYLGERAAKE +dK_MMCCH-2 (K) | 321 |
| 33 | 433 | 456 | 975.8561 | 2924.5464 | 2924.5429 | 1.20 | G | 2 | 26 | 0.0023 | KPPVPVAQANLAVRKNINDLTAEE +dK_MMCCH-2 (K) | 433 |
| 34 | 466 | 481 | 1098.4999 | 2194.9852 | 2194.9841 | 0.50 | G | 2 | 32 | 0.00061 | RFQNDKSVDGYQATVE +Deamidated (NQ); dK_MMCCH-2 (K) | 471 |
| 35 | 1117 | 1133 | 1258.0826 | 2514.1507 | 2514.1314 | 7.68 | G | 2 | 17 | 0.021 | HSVPFRVFDYKNNFDYE +dK_MMCCH-2 (K) | 1127 |
| 36 | 1215 | 1235 | 961.8072 | 2882.3997 | 2882.3949 | 1.67 | G | 3 | 40 | 0.0001 | ITQQLHDLDLHVGDNFFLKYE +dK_MMCCH-2 (K) | 1233 |
| 37 | 1292 | 1303 | 879.4671 | 1756.9196 | 1756.9182 | 0.85 | G | 0 | 49 | 1.20E-05 | SIRSAFLQIQKE +dK_MMCCH-2 (K) | 1302 |
| 38 | 1308 | 1320 | 904.9448 | 1807.8751 | 1807.8749 | 0.06 | G | 0 | 28 | 0.0015 | NIAKFHGKPGLCE +dK_MMCCH-2 (K) | 1311 |
| 39 | 1308 | 1320 | 904.9462 | 1807.8778 | 1807.8749 | 1.55 | G | 0 | 35 | 0.00033 | NIAKFHGKPGLCE +dK_MMCCH-2 (K) | 1315 |
| 40 | 1403 | 1422 | 921.4190 | 2761.2350 | 2761.2330 | 0.72 | G | 3 | 23 | 0.0052 | NAVTSRDPQPELWDNKDFYE +dK_MMCCH-2 (K) | 1418 |
| 41 | 1952 | 1967 | 792.6818 | 2375.0236 | 2375.0205 | 1.31 | G | 3 | 31 | 0.00086 | FSKPEDTFDYHRFGYE +dK_MMCCH-2 (K) | 1954 |
| 42 | 1973 | 1990 | 817.0616 | 2448.1629 | 2448.1454 | 7.15 | G | 0 | 22 | 0.0063 | FVGMSVSSLHNYIKQQQE +dK_MMCCH-2 (K); Oxidation (M) | 1986 |
| 43 | 3386 | 3405 | 658.3166 | 2629.2375 | 2629.2271 | 3.96 | G | 2 | 24 | 0.0038 | LDHAYSLRDGHYYIAGPTKD +dK_MMCCH-2 (K) | 3404 |
| 44 | 277 | 296 | 883.7536 | 2648.2390 | 2648.2329 | 2.27 | C | 4 | 41 | 7.80E-05 | DLTREHAKPADSFDYGRLGY +dK_MMCCH-2 (K) | 284 |
| 45 | 629 | 635 | 539.2515 | 1076.4885 | 1076.4848 | 3.44 | C | 0 | 25 | 0.0034 | VGGSEKY +dK_MMCCH-2 (K) | 634 |
| 46 | 1075 | 1080 | 544.7775 | 1087.5404 | 1087.5372 | 2.94 | C | 1 | 34 | 0.0004 | QALQKY +dK_MMCCH-2 (K) | 1079 |
| 47 | 1212 | 1230 | 875.0881 | 2622.2424 | 2622.2424 | -0.04 | C | 4 | 20 | 0.01 | KYEITQQLHDLDLHVGDNF +dK_MMCCH-2 (K) | 1212 |
| 48 | 1298 | 1306 | 715.3740 | 1428.7334 | 1428.7323 | 0.77 | C | 1 | 18 | 0.016 | LQIQKEGIY +dK_MMCCH-2 (K) | 1302 |
| 49 | 1416 | 1421 | 570.2391 | 1138.4637 | 1138.4641 | -0.35 | C | 1 | 16 | 0.022 | DNKDFY +dK_MMCCH-2 (K) | 1418 |
| 50 | 1526 | 1540 | 749.6825 | 2246.0257 | 2246.0215 | 1.87 | C | 2 | 32 | 0.00061 | KHNLPQDSFDYQNRF +dK_MMCCH-2 (K) | 1526 |

**Figure 25B. KLH 2 (continued)**

| | | | | | | | | | | | | |
|---|---|---|---|---|---|---|---|---|---|---|---|---|
| 51 | 1589 | 1606 | 784.3539 | 2350.0400 | 2350.0392 | 0.34 | C | 0 | 19 | 0.011 | ICVEQGGEQNCKTKAGSF +dK_MMCCH-2 (K) | 1602 |
| 52 | 1696 | 1702 | 607.3019 | 1212.5893 | 1212.5849 | 3.63 | C | 1 | 17 | 0.02 | TTLEKHF +dK_MMCCH-2 (K) | 1700 |
| 53 | 1801 | 1809 | 706.8394 | 1411.6643 | 1411.6595 | 3.40 | C | 0 | 14 | 0.043 | SHKTFPNPF +dK_MMCCH-2 (K) | 1803 |
| 54 | 1985 | 1995 | 850.4289 | 1698.8432 | 1698.8399 | 1.94 | C | 0 | 26 | 0.0023 | IKQQQEADRVF +dK_MMCCH-2 (K) | 1986 |
| 55 | 2455 | 2462 | 645.3275 | 1288.6405 | 1288.6373 | 2.48 | C | 1 | 22 | 0.0065 | KYDITQAL +dK_MMCCH-2 (K) | 2455 |
| 56 | 2463 | 2479 | 1128.0192 | 2254.0238 | 2254.0253 | -0.67 | C | 1 | 16 | 0.027 | KAQSIHPEDVFDTDAPF +dK_MMCCH-2 (K) | 2463 |
| 57 | 2865 | 2872 | 673.3657 | 1344.7168 | 1344.7112 | 4.16 | C | 1 | 22 | 0.0067 | RYDITKVL +dK_MMCCH-2 (K) | 2870 |
| 58 | 3235 | 3242 | 617.3148 | 1232.6150 | 1232.6111 | 3.16 | C | 0 | 27 | 0.002 | TSANVKIY +dK_MMCCH-2 (K) | 3240 |
| 59 | 3399 | 3406 | 576.8029 | 1151.5912 | 1151.5896 | 1.30 | C | 0 | 39 | 0.00012 | IAGPTKDL +dK_MMCCH-2 (K) | 3404 |
| 60 | 165 | 175 | 558.6147 | 1672.8224 | 1672.8144 | 4.78 | Th | 1 | 22 | 0.0058 | FEKVQPGHHTR +dK_MMCCH-2 (K) | 167 |
| 61 | 180 | 191 | 909.9202 | 1817.8259 | 1817.8216 | 2.37 | Th | 4 | 65 | 3.30E-07 | ILDALEQDEFCK +dK_MMCCH-2 (K) | 191 |
| 62 | 247 | 260 | 631.6201 | 1891.8385 | 1891.8305 | 4.23 | Th | 2 | 13 | 0.046 | LRGKDPNSADCAHN +dK_MMCCH-2 (K) | 250 |
| 63 | 283 | 293 | 782.8600 | 1563.7054 | 1563.7028 | 1.73 | Th | 3 | 34 | 0.00039 | AKPADSFDYGR +dK_MMCCH-2 (K) | 284 |
| 64 | 364 | 372 | 642.3248 | 1282.6350 | 1282.6301 | 3.82 | Th | 2 | 41 | 7.30E-05 | LLGGPTEMK +dK_MMCCH-2 (K) | 372 |
| 65 | 431 | 437 | 569.2872 | 1136.5599 | 1136.5576 | 2.02 | Th | 1 | 13 | 0.045 | FDKPPVP +dK_MMCCH-2 (K) | 433 |
| 66 | 629 | 636 | 582.7666 | 1163.5186 | 1163.5169 | 1.55 | Th | 1 | 32 | 0.00069 | VGGSEKYS +dK_MMCCH-2 (K) | 634 |
| 67 | 893 | 904 | 904.8990 | 1807.7834 | 1807.7698 | 7.52 | Th | 1 | 25 | 0.0032 | FHGEPKWCPSPE +dK_MMCCH-2 (K) | 898 |
| 68 | 1123 | 1129 | 619.2855 | 1236.5564 | 1236.5485 | 6.39 | Th | 2 | 14 | 0.042 | VFDYKNN +dK_MMCCH-2 (K) | 1127 |
| 69 | 1414 | 1419 | 564.7574 | 1127.5003 | 1127.4957 | 4.08 | Th | 1 | 14 | 0.039 | LWDNKD +dK_MMCCH-2 (K) | 1418 |
| 70 | 1525 | 1539 | 738.3558 | 2212.0457 | 2212.0371 | 3.84 | Th | 3 | 48 | 1.50E-05 | LKHNLPQDSFDYQNR +dK_MMCCH-2 (K) | 1526 |
| 71 | 1589 | 1605 | 735.3320 | 2202.9741 | 2202.9708 | 1.50 | Th | 2 | 22 | 0.0068 | ICVEQGGEQNCKTKAGS +dK_MMCCH-2 (K) | 1600 |
| 72 | 1589 | 1605 | 735.3343 | 2202.9810 | 2202.9708 | 4.68 | Th | 2 | 38 | 0.00016 | ICVEQGGEQNCKTKAGS +dK_MMCCH-2 (K) | 1602 |
| 73 | 1800 | 1809 | 780.3723 | 1558.7301 | 1558.7279 | 1.41 | Th | 2 | 26 | 0.0025 | FSHKTFPNPF +dK_MMCCH-2 (K) | 1803 |
| 74 | 1952 | 1963 | 627.2842 | 1878.8309 | 1878.8247 | 3.30 | Th | 2 | 47 | 1.90E-05 | FSKPEDTFDYHR +dK_MMCCH-2 (K) | 1954 |
| 75 | 1985 | 1994 | 518.2667 | 1551.7783 | 1551.7715 | 4.38 | Th | 2 | 25 | 0.0034 | IKQQQEADRV +dK_MMCCH-2 (K) | 1986 |
| 76 | 2193 | 2199 | 667.3005 | 1332.5864 | 1332.5849 | 1.13 | Th | 2 | 22 | 0.0057 | YWDWTKP +dK_MMCCH-2 (K) | 2198 |
| 77 | 2200 | 2206 | 562.3085 | 1122.6025 | 1122.5995 | 2.67 | Th | 2 | 22 | 0.0057 | ISKLPDL +dK_MMCCH-2 (K) | 2202 |

**Figure 25B. KLH 2 (continued)**

| | | | | | | | | | | | | |
|---|---|---|---|---|---|---|---|---|---|---|---|---|
| 78 | 2225 | 2229 | 462.2214 | 922.4282 | 922.4259 | 2.49 | Th | 2 | 14 | 0.036 | FAKGY +dK_MMCCH-2 (K) | 2227 |
| 79 | 2359 | 2363 | 509.2317 | 1016.4489 | 1016.4460 | 2.95 | Th | 1 | 17 | 0.019 | FTKMH +dK_MMCCH-2 (K); Oxidation (M) | 2361 |
| 80 | 2941 | 2950 | 720.8408 | 1439.6670 | 1439.6602 | 4.72 | Th | 3 | 45 | 2.80E-05 | LDEANDLKNA +dK_MMCCH-2 (K) | 2948 |
| 81 | 3009 | 3015 | 573.2736 | 1144.5327 | 1144.5335 | -0.70 | Th | 0 | 14 | 0.041 | LKEHGSH +dK_MMCCH-2 (K) | 3010 |
| 82 | 3399 | 3405 | 520.2607 | 1038.5069 | 1038.5056 | 1.25 | Th | 1 | 25 | 0.0029 | IAGPTKD +dK_MMCCH-2 (K) | 3404 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the lysine conjugation site for KLH1 or KLH2.

**Figure 26. Identification details of MMCCH-conjugated peptides for sample 1 (2nd LC-MS/MS)**

**Figure 26A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 124 | 141 | 785.4114 | 2353.2125 | 2353.2001 | 5.27 | T | 1 | 14 | 0.037 | GFTDPPVKHHQSANLLVR +dK_MMCCH-2 (K) | 131 |
| 2 | 142 | 149 | 656.3405 | 1310.6665 | 1310.6653 | 0.92 | T | 1 | 25 | 0.003 | KNINDLTR +dK_MMCCH-2 (K) | 142 |
| 3 | 157 | 166 | 548.9218 | 1643.7436 | 1643.7402 | 2.07 | T | 1 | 20 | 0.011 | EAFHKFQEDR +dK_MMCCH-2 (K) | 161 |
| 4 | 460 | 477 | 781.3956 | 2341.1649 | 2341.1624 | 1.07 | T | 1 | 15 | 0.033 | TSANVDIFIKTTDSVQHK +dK_MMCCH-2 (K) | 469 |
| 5 | 470 | 488 | 748.0444 | 2241.1115 | 2241.1100 | 0.67 | T | 1 | 25 | 0.0034 | TTDSVQHKAGTFAVLGGSK +dK_MMCCH-2 (K) | 477 |
| 6 | 553 | 560 | 663.8681 | 1325.7216 | 1325.7166 | 3.85 | T | 2 | 24 | 0.0041 | VKFDKVPR +dK_MMCCH-2 (K) | 554 |
| 7 | 566 | 570 | 485.2447 | 968.4748 | 968.4749 | -0.10 | T | 1 | 19 | 0.014 | KNVDR +dK_MMCCH-2 (K) | 566 |
| 8 | 581 | 587 | 547.8373 | 1093.6600 | 1093.6569 | 2.83 | T | 1 | 20 | 0.01 | KALALLK +dK_MMCCH-2 (K) | 581 |
| 9 | 588 | 606 | 781.0350 | 2340.0831 | 2340.0845 | -0.60 | T | 1 | 29 | 0.0013 | EDKSAGGFQQLGAFHGEPK +dK_MMCCH-2 (K) | 590 |
| 10 | 645 | 681 | 1158.5393 | 4630.1281 | 4630.1063 | 4.71 | T | 1 | 18 | 0.014 | HGYDGALPYWDWTSPLNHLPELADHEKYVDPEDGVEK +dK_MMCCH-2 (K) | 671 |
| 11 | 682 | 699 | 1239.5804 | 2477.1463 | 2477.1434 | 1.17 | T | 1 | 16 | 0.025 | HNPWFDGHIDTVDKTTTR +dK_MMCCH-2 (K) | 695 |
| 12 | 700 | 719 | 887.7732 | 2660.2977 | 2660.2945 | 1.24 | T | 1 | 44 | 3.70E-05 | SVQNKLFEQPEFGHYTSIAK +dK_MMCCH-2 (K) | 704 |
| 13 | 778 | 789 | 958.0048 | 1913.9950 | 1913.9862 | 4.60 | T | 1 | 31 | 0.00074 | IWAIWQALQKYR +Deamidated (NQ); dK_MMCCH-2 (K) | 787 |
| 14 | 790 | 804 | 669.3164 | 2004.9274 | 2004.9220 | 2.69 | T | 0 | 37 | 0.00019 | GKPYNVANCAVTSMR +dK_MMCCH-2 (K) | 791 |
| 15 | 858 | 863 | 520.2980 | 1038.5814 | 1038.5783 | 2.98 | T | 1 | 17 | 0.019 | KLEAIK +dK_MMCCH-2 (K) | 858 |
| 16 | 953 | 965 | 635.3307 | 1902.9704 | 1902.9662 | 2.21 | T | 1 | 48 | 1.70E-05 | DLFKQPSVIHEPR +dK_MMCCH-2 (K) | 956 |
| 17 | 986 | 1000 | 685.0263 | 2052.0571 | 2052.0561 | 0.49 | T | 1 | 55 | 3.10E-06 | KNIENLSLGELESLR +dK_MMCCH-2 (K) | 986 |
| 18 | 1092 | 1114 | 791.8780 | 3163.4829 | 3163.4682 | 4.62 | T | 1 | 38 | 0.00015 | QHHYETNPFHHGKITHENEITTR +dK_MMCCH-2 (K) | 1104 |
| 19 | 1118 | 1162 | 1414.4275 | 5653.6810 | 5653.6850 | -0.71 | T | 1 | 28 | 0.0015 | DSLFHSDYFYEQVLYALEQDNFCDFEIQLEILHNALHSLLGGKGK +dK_MMCCH-2 (K) | 1162 |
| 20 | 1199 | 1203 | 353.1912 | 1056.5517 | 1056.5538 | -1.99 | T | 1 | 17 | 0.019 | KRPYR +dK_MMCCH-2 (K) | 1199 |
| 21 | 1395 | 1403 | 485.5965 | 1453.7677 | 1453.7599 | 5.37 | T | 2 | 33 | 0.00054 | KDITQLDKR +dK_MMCCH-2 (K) | 1395 |
| 22 | 1473 | 1489 | 778.4053 | 2332.1942 | 2332.1827 | 4.93 | T | 1 | 45 | 3.10E-05 | KHGAVVGLPYWDWTLPR +dK_MMCCH-2 (K) | 1473 |
| 23 | 1923 | 1932 | 756.3799 | 1510.7452 | 1510.7425 | 1.79 | T | 1 | 30 | 0.00099 | NKVMPNPFAR +dK_MMCCH-2 (K) | 1924 |

**Figure 26A. KLH 1 (continued)**

| | | | | | | | | | | | | |
|---|---|---|---|---|---|---|---|---|---|---|---|---|
| 24 | 2067 | 2076 | 746.8890 | 1491.7635 | 1491.7544 | 6.10 | T | 1 | 19 | 0.011 | KHAVPNDVFK +dK_MMCCH-2 (K) | 2067 |
| 25 | 2068 | 2083 | 753.7155 | 2258.1247 | 2258.1194 | 2.35 | T | 1 | 13 | 0.047 | HAVPNDVFKYELLGYR +dK_MMCCH-2 (K) | 2076 |
| 26 | 2121 | 2149 | 890.4212 | 3557.6557 | 3557.6378 | 5.03 | T | 1 | 31 | 0.00073 | TSADVQFQICKTSEDCHHGGQIFVLGGTK +dK_MMCCH-2 (K) | 2131 |
| 27 | 2233 | 2251 | 834.0938 | 2499.2596 | 2499.2349 | 9.88 | T | 2 | 16 | 0.025 | KEINTLTTAEVDNLKDAMR +dK_MMCCH-2 (K) | 2247 |
| 28 | 2299 | 2314 | 715.0192 | 2142.0357 | 2142.0238 | 5.56 | T | 1 | 14 | 0.037 | LYTKQMEDALTAHGAR +dK_MMCCH-2 (K) | 2302 |
| 29 | 2359 | 2367 | 736.3490 | 1470.6834 | 1470.6813 | 1.43 | T | 1 | 34 | 0.00038 | DKLFNDPER +dK_MMCCH-2 (K) | 2360 |
| 30 | 2461 | 2490 | 642.8188 | 3850.8691 | 3850.8638 | 1.38 | T | 0 | 15 | 0.029 | RPLRPFSDPINHNAFTHSNAKPTDVFEYSR +dK_MMCCH-2 (K) | 2481 |
| 31 | 2507 | 2514 | 682.3505 | 1362.6865 | 1362.6853 | 0.88 | T | 1 | 33 | 0.00053 | KLEHELEK +dK_MMCCH-2 (K) | 2507 |
| 32 | 2515 | 2520 | 571.7624 | 1141.5103 | 1141.5074 | 2.63 | T | 1 | 22 | 0.0058 | QKEEDR +dK_MMCCH-2 (K) | 2516 |
| 33 | 2621 | 2636 | 802.7112 | 2405.1119 | 2405.1083 | 1.50 | T | 1 | 47 | 2.00E-05 | HHEKHHEDHHEDILVR +dK_MMCCH-2 (K) | 2624 |
| 34 | 2637 | 2651 | 1069.5295 | 2137.0445 | 2137.0374 | 3.32 | T | 1 | 59 | 1.40E-06 | KNIHSLSHHEAEELR +dK_MMCCH-2 (K) | 2637 |
| 35 | 2657 | 2686 | 934.9169 | 3735.6384 | 3735.6358 | 0.67 | T | 1 | 18 | 0.014 | LQNDESHGGYEHIAGFHGYPNLCPEKGDEK +dK_MMCCH-2 (K) | 2686 |
| 36 | 2761 | 2814 | 1328.4335 | 6637.1313 | 6637.0821 | 7.41 | T | 1 | 17 | 0.022 | DVNEAIFQQTKFGEFSSIFYLALQALEEDNYCDFEVQYEILHNEVHALIGGAEK +4 Deamidated (NQ); dK_MMCCH-1 (K) | 2771 |
| 37 | 2851 | 2883 | 807.5799 | 4032.8631 | 4032.8346 | 7.07 | T | 0 | 36 | 0.00023 | VKPAHAGSCAGDIMHVPLHPFNYESVNNDDFTR +dK_MMCCH-2 (K) | 2852 |
| 38 | 2897 | 2919 | 1066.5194 | 3196.5364 | 3196.5287 | 2.41 | T | 1 | 68 | 1.50E-07 | FNYKYDNLNLHGHNIEELEEVLR +dK_MMCCH-2 (K) | 2900 |
| 39 | 3003 | 3021 | 825.7721 | 2474.2944 | 2474.2879 | 2.63 | T | 1 | 39 | 0.00014 | KYDHTELDASVLPAPIIVR +dK_MMCCH-2 (K) | 3003 |
| 40 | 123 | 151 | 616.6594 | 3693.9130 | 3693.9049 | 2.19 | G | 2 | 19 | 0.013 | KGFTDPPVKHHQSANLLVRKNINDLTREE +dK_MMCCH-2 (K) | 123 |
| 41 | 123 | 151 | 739.7917 | 3693.9221 | 3693.9049 | 4.63 | G | 2 | 28 | 0.0015 | KGFTDPPVKHHQSANLLVRKNINDLTREE +dK_MMCCH-2 (K) | 131 |
| 42 | 158 | 176 | 846.3880 | 2536.1422 | 2536.1329 | 3.67 | G | 3 | 27 | 0.002 | AFHKFQEDRSVDGYQATAE +dK_MMCCH-2 (K) | 161 |
| 43 | 526 | 544 | 777.7497 | 2330.2272 | 2330.2304 | -1.37 | G | 1 | 22 | 0.0064 | VDGTKLASSLIPHASVIRE +dK_MMCCH-2 (K) | 530 |
| 44 | 589 | 612 | 990.1168 | 2967.3286 | 2967.3320 | -1.15 | G | 1 | 28 | 0.0015 | DKSAGGFQQLGAFHGEPKWCPSPE +dK_MMCCH-2 (K) | 606 |
| 45 | 825 | 841 | 820.7065 | 2459.0978 | 2459.0780 | 8.05 | G | 1 | 25 | 0.003 | HSVPFNVFDYKTNFNYE +Deamidated (NQ); dK_MMCCH-2 (K) | 835 |
| 46 | 942 | 972 | 764.6027 | 3817.9772 | 3817.9614 | 4.14 | G | 3 | 14 | 0.043 | VFDLKPASLGKDLFKQPSVIHEPRIGHHEGE +dK_MMCCH-2 (K) | 946 |
| 47 | 942 | 970 | 727.3896 | 3631.9116 | 3631.8973 | 3.91 | G | 2 | 16 | 0.024 | VFDLKPASLGKDLFKQPSVIHEPRIGHHE +dK_MMCCH-2 (K) | 956 |
| 48 | 1055 | 1076 | 810.1689 | 3236.6464 | 3236.6402 | 1.92 | G | 1 | 30 | 0.00099 | NALLKKGSSVAVPYWDWTKRIE +2 dK_MMCCH-2 (K) | 1059, 1060 |
| 49 | 1055 | 1076 | 725.6339 | 2898.5063 | 2898.5102 | -1.35 | G | 1 | 41 | 7.10E-05 | NALLKKGSSVAVPYWDWTKRIE +dK_MMCCH-2 (K) | 1060 |

**Figure 26A. KLH 1 (continued)**

| | | | | | | | | | | | | |
|---|---|---|---|---|---|---|---|---|---|---|---|---|
| 50 | 1055 | 1076 | 725.6342 | 2898.5076 | 2898.5102 | -0.90 | G | 1 | 21 | 0.0085 | NALLKKGSSVAVPYWDWTKRIE +dK_MMCCH-2 (K) | 1073 |
| 51 | 1097 | 1110 | 999.9632 | 1997.9118 | 1997.9054 | 3.25 | G | 1 | 18 | 0.016 | TNPFHHGKITHENE +dK_MMCCH-2 (K) | 1104 |
| 52 | 1376 | 1396 | 648.5449 | 2590.1506 | 2590.1428 | 3.01 | G | 3 | 29 | 0.0012 | AGTDSAHTDDGHTEPVMIRKD +dK_MMCCH-2 (K) | 1395 |
| 53 | 1397 | 1413 | 774.4335 | 2320.2786 | 2320.2824 | -1.64 | G | 1 | 25 | 0.0034 | ITQLDKRQQLSLVKALE +dK_MMCCH-2 (K) | 1410 |
| 54 | 1506 | 1522 | 753.3776 | 2257.1110 | 2257.1089 | 0.93 | G | 2 | 26 | 0.0028 | TGRDIPNPFIGSKIEFE +dK_MMCCH-2 (K) | 1518 |
| 55 | 1755 | 1765 | 570.6055 | 1708.7946 | 1708.7913 | 1.93 | G | 2 | 25 | 0.0031 | TLDKMNLRHDE +dK_MMCCH-2 (K) | 1758 |
| 56 | 2089 | 2100 | 854.4103 | 1706.8061 | 1706.8008 | 3.16 | G | 2 | 30 | 0.00094 | IGGMNLHEIEKE +dK_MMCCH-2 (K) | 2099 |
| 57 | 2647 | 2661 | 715.6783 | 2144.0130 | 2144.0095 | 1.63 | G | 4 | 18 | 0.016 | AEELRDALYKLQNDE +dK_MMCCH-2 (K) | 2656 |
| 58 | 3090 | 3097 | 632.8034 | 1263.5923 | 1263.5879 | 3.40 | G | 0 | 16 | 0.026 | LGKMYSVE +dK_MMCCH-2 (K) | 3092 |
| 59 | 59 | 68 | 721.3486 | 1440.6827 | 1440.6781 | 3.19 | C | 1 | 21 | 0.0084 | VLGGPSEMKW +dK_MMCCH-2 (K) | 67 |
| 60 | 94 | 100 | 573.2995 | 1144.5844 | 1144.5838 | 0.52 | C | 1 | 17 | 0.02 | TVKAELF +dK_MMCCH-2 (K) | 96 |
| 61 | 429 | 450 | 990.8052 | 2969.3937 | 2969.3899 | 1.31 | C | 1 | 14 | 0.036 | GGISLENIEKMIHENQQEDRIY +dK_MMCCH-2 (K); Oxidation (M) | 438 |
| 62 | 584 | 595 | 787.4013 | 1572.7879 | 1572.7858 | 1.40 | C | 2 | 46 | 2.60E-05 | ALLKEDKSAGGF +dK_MMCCH-2 (K) | 590 |
| 63 | 715 | 723 | 655.8741 | 1309.7336 | 1309.7316 | 1.60 | C | 1 | 15 | 0.032 | TSIAKQVLL +dK_MMCCH-2 (K) | 719 |
| 64 | 783 | 788 | 544.7768 | 1087.5390 | 1087.5372 | 1.66 | C | 1 | 23 | 0.0054 | QALQKY +dK_MMCCH-2 (K) | 787 |
| 65 | 830 | 838 | 743.3419 | 1484.6692 | 1484.6646 | 3.10 | C | 2 | 22 | 0.006 | NVFDYKTNF +dK_MMCCH-2 (K) | 835 |
| 66 | 944 | 955 | 821.4310 | 1640.8475 | 1640.8484 | -0.55 | C | 3 | 18 | 0.016 | DLKPASLGKDLF +dK_MMCCH-2 (K) | 946 |
| 67 | 1330 | 1337 | 620.3562 | 1238.6978 | 1238.6944 | 2.74 | C | 1 | 22 | 0.0064 | KLDITKAL +dK_MMCCH-2 (K) | 1335 |
| 68 | 1580 | 1586 | 544.2618 | 1086.5091 | 1086.5056 | 3.22 | C | 0 | 23 | 0.0055 | VGGKEPY +dK_MMCCH-2 (K) | 1583 |
| 69 | 1818 | 1827 | 753.3697 | 1504.7249 | 1504.7232 | 1.20 | C | 1 | 18 | 0.015 | SERDIGSLKY +dK_MMCCH-2 (K) | 1826 |
| 70 | 2076 | 2082 | 612.3051 | 1222.5955 | 1222.5944 | 0.98 | C | 3 | 15 | 0.031 | KYELLGY +dK_MMCCH-2 (K) | 2076 |
| 71 | 2160 | 2184 | 814.3868 | 3253.5180 | 3253.5179 | 0.03 | C | 3 | 14 | 0.036 | KYDITHALHDAHITPEDVFHPSEPF +dK_MMCCH-2 (K) | 2160 |
| 72 | 2476 | 2488 | 923.9227 | 1845.8309 | 1845.8244 | 3.58 | C | 1 | 28 | 0.0017 | THSNAKPTDVFEY +dK_MMCCH-2 (K) | 2481 |
| 73 | 2656 | 2672 | 747.3438 | 2239.0094 | 2239.0004 | 4.02 | C | 2 | 26 | 0.0026 | KLQNDESHGGYEHIAGF +dK_MMCCH-2 (K) | 2656 |
| 74 | 2738 | 2749 | 856.3752 | 1710.7358 | 1710.7348 | 0.58 | C | 2 | 15 | 0.033 | ADSGNNNPFFKY +dK_MMCCH-2 (K) | 2748 |
| 75 | 2946 | 2960 | 965.4056 | 1928.7966 | 1928.7986 | -1.04 | C | 1 | 24 | 0.0044 | IKSGTDSDDEYAGSF +dK_MMCCH-2 (K) | 2947 |
| 76 | 2961 | 2971 | 769.8948 | 1537.7751 | 1537.7673 | 5.07 | C | 1 | 16 | 0.024 | VILGGAKEMPW +dK_MMCCH-2 (K) | 2967 |

Figure 26A. KLH 1 (continued)

| | | | | | | | | | | | | |
|---|---|---|---|---|---|---|---|---|---|---|---|---|
| 77 | 1 | 8 | 643.8057 | 1285.5968 | 1285.5934 | 2.64 | Th | 1 | 26 | 0.0026 | MTPEELKT +dK_MMCCH-2 (K) | 7 |
| 78 | 38 | 56 | 861.6823 | 2582.0251 | 2582.0108 | 5.54 | Th | 2 | 17 | 0.022 | VCIPDDNDRNDDHCEKAGD +dK_MMCCH-2 (K) | 53 |
| 79 | 59 | 69 | 785.3758 | 1568.7370 | 1568.7367 | 0.19 | Th | 3 | 59 | 1.40E-06 | VLGGPSEMKWQ +dK_MMCCH-2 (K) | 67 |
| 80 | 78 | 84 | 569.2868 | 1136.5590 | 1136.5536 | 4.75 | Th | 1 | 42 | 6.90E-05 | LSDTVHK +dK_MMCCH-2 (K) | 84 |
| 81 | 117 | 124 | 620.8154 | 1239.6162 | 1239.6070 | 7.34 | Th | 1 | 28 | 0.0016 | VVHHPEKG +dK_MMCCH-2 (K) | 123 |
| 82 | 269 | 282 | 660.0024 | 1976.9855 | 1976.9778 | 3.89 | Th | 4 | 27 | 0.0019 | VIAFEENAPHTKRQ +dK_MMCCH-2 (K) | 280 |
| 83 | 431 | 439 | 707.8527 | 1413.6908 | 1413.6883 | 1.70 | Th | 3 | 42 | 6.60E-05 | ISLENIEKM +dK_MMCCH-2 (K) | 438 |
| 84 | 521 | 530 | 707.8467 | 1413.6788 | 1413.6698 | 6.37 | Th | 2 | 74 | 4.30E-08 | VDITEVDGTK +dK_MMCCH-2 (K) | 530 |
| 85 | 585 | 591 | 585.8110 | 1169.6074 | 1169.6002 | 6.16 | Th | 1 | 35 | 0.00033 | LLKEDKS +dK_MMCCH-2 (K) | 590 |
| 86 | 601 | 612 | 904.8955 | 1807.7765 | 1807.7698 | 3.65 | Th | 1 | 21 | 0.0086 | FHGEPKWCPSPE +dK_MMCCH-2 (K) | 606 |
| 87 | 666 | 672 | 607.2842 | 1212.5539 | 1212.5485 | 4.45 | Th | 2 | 23 | 0.0046 | LADHEKY +dK_MMCCH-2 (K) | 671 |
| 88 | 831 | 837 | 612.7877 | 1223.5608 | 1223.5533 | 6.13 | Th | 2 | 17 | 0.019 | VFDYKTN +dK_MMCCH-2 (K) | 835 |
| 89 | 954 | 959 | 529.7660 | 1057.5175 | 1057.5154 | 1.99 | Th | 1 | 16 | 0.027 | LFKQPS +Deamidated (NQ); dK_MMCCH-2 (K) | 956 |
| 90 | 1100 | 1104 | 482.2290 | 962.4434 | 962.4433 | 0.10 | Th | 0 | 16 | 0.023 | FHHGK +dK_MMCCH-2 (K) | 1104 |
| 91 | 1111 | 1120 | 742.3796 | 1482.7446 | 1482.7388 | 3.91 | Th | 1 | 18 | 0.017 | ITTRDPKDSL +dK_MMCCH-2 (K) | 1117 |
| 92 | 1156 | 1164 | 630.8406 | 1259.6666 | 1259.6584 | 6.51 | Th | 2 | 17 | 0.019 | LLGGKGKYS +dK_MMCCH-2 (K) | 1162 |
| 93 | 1219 | 1232 | 980.4258 | 1958.8370 | 1958.8316 | 2.76 | Th | 1 | 58 | 1.60E-06 | FDKSDNNDEATKTH +dK_MMCCH-2 (K) | 1230 |
| 94 | 1580 | 1587 | 572.7747 | 1143.5348 | 1143.5271 | 6.73 | Th | 1 | 14 | 0.043 | VGGKEPYG +dK_MMCCH-2 (K) | 1583 |
| 95 | 1657 | 1668 | 921.9188 | 1841.8231 | 1841.8182 | 2.66 | Th | 2 | 34 | 0.00041 | FSKPEDTFDYQK +dK_MMCCH-2 (K) | 1659 |
| 96 | 1657 | 1668 | 921.9185 | 1841.8225 | 1841.8182 | 2.33 | Th | 2 | 39 | 0.00012 | FSKPEDTFDYQK +dK_MMCCH-2 (K) | 1668 |
| 97 | 1733 | 1741 | 664.3225 | 1326.6305 | 1326.6312 | -0.53 | Th | 2 | 17 | 0.021 | VLGGEKEMP +dK_MMCCH-1 (K); Oxidation (M) | 1738 |
| 98 | 1898 | 1904 | 667.3018 | 1332.5891 | 1332.5849 | 3.15 | Th | 2 | 27 | 0.0021 | YWDWTKP +dK_MMCCH-2 (K) | 1903 |
| 99 | 2064 | 2068 | 499.7606 | 997.5067 | 997.5055 | 1.20 | Th | 0 | 22 | 0.007 | FTKKH +dK_MMCCH-2 (K) | 2067 |
| 100 | 2094 | 2100 | 618.3083 | 1234.6020 | 1234.5903 | 9.48 | Th | 1 | 26 | 0.0023 | LHEIEKE +dK_MMCCH-2 (K) | 2099 |
| 101 | 2129 | 2141 | 622.9256 | 1865.7550 | 1865.7495 | 2.95 | Th | 0 | 24 | 0.0041 | ICKTSEDCHHGGQ +dK_MMCCH-2 (K) | 2131 |
| 102 | 2238 | 2248 | 778.8793 | 1555.7440 | 1555.7440 | 0.00 | Th | 3 | 42 | 6.60E-05 | LTTAEVDNLKD +dK_MMCCH-2 (K) | 2247 |
| 103 | 2480 | 2487 | 622.7983 | 1243.5820 | 1243.5795 | 2.01 | Th | 2 | 20 | 0.0094 | AKPTDVFE +dK_MMCCH-2 (K) | 2481 |

Figure 26A. KLH 1 (continued)

| | | | | | | | | | | | | |
|---|---|---|---|---|---|---|---|---|---|---|---|---|
| 104 | 2512 | 2521 | 807.3970 | 1612.7794 | 1612.7766 | 1.67 | Th | 0 | 32 | 0.00061 | LEKQKEEDRT +dK_MMCCH-2 (K) | 2514 |
| 105 | 2595 | 2606 | 806.4030 | 1610.7915 | 1610.7862 | 3.29 | Th | 2 | 34 | 0.00039 | IIDTSGKQLPSD +dK_MMCCH-2 (K) | 2601 |
| 106 | 2607 | 2612 | 520.7797 | 1039.5449 | 1039.5446 | 0.29 | Th | 2 | 19 | 0.014 | LIKMPT +dK_MMCCH-2 (K) | 2609 |
| 107 | 2766 | 2771 | 551.7854 | 1101.5562 | 1101.5529 | 3.09 | Th | 1 | 25 | 0.0029 | IFQQTK +dK_MMCCH-2 (K) | 2771 |
| 108 | 2808 | 2816 | 638.3187 | 1274.6229 | 1274.6217 | 1.02 | Th | 3 | 27 | 0.002 | LIGGAEKYS +dK_MMCCH-2 (K) | 2814 |
| 109 | 2946 | 2960 | 965.4091 | 1928.8037 | 1928.7986 | 2.64 | Th | 3 | 40 | 9.50E-05 | IKSGTDSDDEYAGSF +dK_MMCCH-2 (K) | 2947 |
| 110 | 2990 | 2996 | 583.2850 | 1164.5555 | 1164.5485 | 6.01 | Th | 1 | 38 | 0.00018 | LTDDHVK +dK_MMCCH-2 (K) | 2996 |
| 111 | 3076 | 3084 | 724.3611 | 1446.7077 | 1446.7040 | 2.63 | Th | 2 | 15 | 0.03 | FKCKVPPFS +dK_MMCCH-2 (K) | 3079 |

Figure 26B. KLH 2

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1043.0270 | 2084.0394 | 2084.0347 | 2.26 | T | 1 | 49 | 1.30E-05 | KNVDSLSSDEVLALEK +dK_MMCCH-2 (K) | 26 |
| 2 | 147 | 155 | 694.3710 | 1386.7275 | 1386.7217 | 4.18 | T | 1 | 19 | 0.014 | ADITFLNKK +dK_MMCCH-2 (K) | 155 |
| 3 | 164 | 175 | 596.3106 | 1785.9100 | 1785.8985 | 6.44 | T | 1 | 43 | 5.00E-05 | LFEKVQPGHHTR +dK_MMCCH-2 (K) | 167 |
| 4 | 249 | 271 | 987.4406 | 2959.3000 | 2959.3051 | -1.72 | T | 1 | 30 | 0.001 | GKDPNSADCAHNLIHTPMEPFDR +dK_MMCCH-2 (K) | 250 |
| 5 | 281 | 293 | 610.9454 | 1829.8143 | 1829.8043 | 5.47 | T | 0 | 54 | 3.80E-06 | EHAKPADSFDYGR +dK_MMCCH-2 (K) | 284 |
| 6 | 419 | 446 | 846.4622 | 3381.8195 | 3381.8132 | 1.86 | T | 1 | 27 | 0.0021 | QPTLVHRPAKGHFDKPPVPVAQANLAVR +Deamidated (NQ); dK_MMCCH-2 (K) | 428 |
| 7 | 429 | 446 | 752.0668 | 2253.1785 | 2253.1729 | 2.49 | T | 0 | 41 | 7.30E-05 | GHFDKPPVPVAQANLAVR +dK_MMCCH-2 (K) | 433 |
| 8 | 462 | 466 | 486.7367 | 971.4589 | 971.4568 | 2.16 | T | 1 | 13 | 0.046 | KAMER +dK_MMCCH-2 (K) | 462 |
| 9 | 467 | 488 | 708.5958 | 2830.3539 | 2830.3385 | 5.48 | T | 1 | 69 | 1.10E-07 | FQNDKSVDGYQATVEFHALPAR +dK_MMCCH-2 (K) | 471 |
| 10 | 936 | 950 | 720.3436 | 2158.0089 | 2158.0095 | -0.28 | T | 1 | 34 | 0.00037 | KHGFTGGLPYWDWTR +dK_MMCCH-2 (K) | 936 |
| 11 | 995 | 1052 | 1170.7141 | 7018.2410 | 7018.2306 | 1.48 | T | 1 | 13 | 0.047 | DDLYQSPGFGHYTDIAKQVLLAFEQDDFCDFEVQFEIAHNFIHALVGGNEPYSMSSLR +dK_MMCCH-2 (K) | 1011 |
| 12 | 1082 | 1096 | 996.4616 | 1990.9087 | 1990.9063 | 1.21 | T | 0 | 76 | 2.80E-08 | GKPYNTANCAIASMR +dK_MMCCH-2 (K) | 1083 |
| 13 | 1097 | 1115 | 827.0759 | 2478.2060 | 2478.2101 | -1.65 | T | 0 | 68 | 1.50E-07 | KPLQPFGLDSVINPDDETR +dK_MMCCH-2 (K) | 1097 |
| 14 | 1123 | 1149 | 1190.5593 | 3568.6562 | 3568.6497 | 1.82 | T | 1 | 69 | 1.30E-07 | VFDYKNNFDYEYESLAFNGLSIAQLDR +dK_MMCCH-2 (K) | 1127 |
| 15 | 1355 | 1385 | 1271.2737 | 3810.7992 | 3810.7876 | 3.04 | T | 1 | 49 | 1.30E-05 | GSAVAVPYWDWTEKADSLPSLINDATYFNSR +dK_MMCCH-2 (K) | 1368 |
| 16 | 1409 | 1450 | 1371.8909 | 5483.5344 | 5483.5213 | 2.39 | T | 1 | 50 | 1.00E-05 | DPQPELWDNKDFYENVMLALEQDNFCDFEIQLELIHNALHSR +dK_MMCCH-2 (K) | 1418 |
| 17 | 1455 | 1480 | 1117.8749 | 3350.6028 | 3350.6070 | -1.25 | T | 1 | 69 | 1.40E-07 | AKYSLSSLDYTAFDPVFFLHHANVDR +dK_MMCCH-2 (K) | 1456 |
| 18 | 1493 | 1507 | 721.6661 | 2161.9766 | 2161.9594 | 7.91 | T | 1 | 37 | 0.00021 | KKPYNEADCAVNEMR +dK_MMCCH-2 (K) | 1493 |
| 19 | 1493 | 1507 | 721.6617 | 2161.9634 | 2161.9594 | 1.85 | T | 1 | 49 | 1.30E-05 | KKPYNEADCAVNEMR +dK_MMCCH-2 (K) | 1494 |
| 20 | 1508 | 1526 | 837.7523 | 2510.2351 | 2510.2185 | 6.61 | T | 0 | 20 | 0.011 | KPLQPFNNPELNSDSMTLK +dK_MMCCH-2 (K) | 1508 |
| 21 | 1508 | 1539 | 825.9982 | 4124.9545 | 4124.9360 | 4.48 | T | 1 | 24 | 0.0041 | KPLQPFNNPELNSDSMTLKHNLPQDSFDYQNR +dK_MMCCH-2 (K) | 1526 |
| 22 | 1542 | 1564 | 1070.1957 | 3207.5652 | 3207.5448 | 6.39 | T | 1 | 23 | 0.0045 | YQYDNLQFNHFSIQKLDQTIQAR +dK_MMCCH-2 (K) | 1556 |
| 23 | 1690 | 1704 | 532.2902 | 2125.1318 | 2125.1241 | 3.58 | T | 2 | 17 | 0.022 | KEVSSLTTLEKHFLR +dK_MMCCH-2 (K) | 1700 |

**Figure 26B. KLH 2 (continued)**

| | | | | | | | | | | | | |
|---|---|---|---|---|---|---|---|---|---|---|---|---|
| 24 | 1757 | 1768 | 612.9789 | 1835.9150 | 1835.9128 | 1.20 | T | 1 | 14 | 0.041 | LYTVQFEDSLKR +dK_MMCCH-2 (K) | 1767 |
| 25 | 1949 | 1963 | 746.6681 | 2236.9824 | 2236.9735 | 3.98 | T | 0 | 39 | 0.00013 | TQEFSKPEDTFDYHR +dK_MMCCH-2 (K) | 1954 |
| 26 | 2045 | 2052 | 660.3516 | 1318.6887 | 1318.6843 | 3.34 | T | 1 | 21 | 0.0084 | YDITKTLK +dK_MMCCH-2 (K) | 2049 |
| 27 | 2116 | 2125 | 715.3641 | 1428.7136 | 1428.7105 | 2.17 | T | 1 | 21 | 0.0083 | DLASLKSAMR +dK_MMCCH-2 (K) | 2121 |
| 28 | 2218 | 2231 | 937.4800 | 1872.9454 | 1872.9444 | 0.53 | T | 1 | 19 | 0.014 | DAVVNNPFAKGYIK +dK_MMCCH-2 (K) | 2227 |
| 29 | 2228 | 2239 | 870.4218 | 1738.8291 | 1738.8236 | 3.16 | T | 1 | 20 | 0.011 | GYIKSEDAYTVR +dK_MMCCH-2 (K) | 2231 |
| 30 | 2527 | 2541 | 1007.5172 | 2013.0197 | 2013.0088 | 5.41 | T | 1 | 26 | 0.0027 | KDVTSLTASEIENLR +dK_MMCCH-2 (K) | 2527 |
| 31 | 2728 | 2739 | 958.0048 | 1913.9950 | 1913.9862 | 4.60 | T | 1 | 31 | 0.00074 | IWAIWQALQKYR +Deamidated (NQ); dK_MMCCH-2 (K) | 2737 |
| 32 | 2755 | 2764 | 764.3823 | 1526.7500 | 1526.7439 | 4.00 | T | 0 | 33 | 0.00045 | QPLKPFSESR +Deamidated (NQ); dK_MMCCH-2 (K) | 2758 |
| 33 | 2866 | 2873 | 659.3626 | 1316.7107 | 1316.7050 | 4.33 | T | 1 | 15 | 0.029 | YDITKVLK +dK_MMCCH-2 (K) | 2870 |
| 34 | 3148 | 3155 | 629.3355 | 1256.6565 | 1256.6587 | -1.75 | T | 1 | 19 | 0.013 | KKPYNAAK +dK_MMCCH-2 (K) | 3149 |
| 35 | 167 | 178 | 590.9645 | 1769.8716 | 1769.8705 | 0.62 | G | 0 | 36 | 0.00027 | KVQPGHHTRLME +dK_MMCCH-2 (K) | 167 |
| 36 | 433 | 456 | 975.8562 | 2924.5468 | 2924.5429 | 1.30 | G | 2 | 21 | 0.0088 | KPPVPVAQANLAVRKNINDLTAEE +dK_MMCCH-2 (K) | 433 |
| 37 | 466 | 481 | 1098.4998 | 2194.9850 | 2194.9841 | 0.41 | G | 2 | 35 | 0.00032 | RFQNDKSVDGYQATVE +Deamidated (NQ); dK_MMCCH-2 (K) | 471 |
| 38 | 1215 | 1235 | 721.6072 | 2882.3999 | 2882.3949 | 1.70 | G | 3 | 28 | 0.0017 | ITQQLHDLDLHVGDNFFLKYE +dK_MMCCH-2 (K) | 1233 |
| 39 | 1292 | 1303 | 879.4667 | 1756.9189 | 1756.9182 | 0.46 | G | 0 | 49 | 1.20E-05 | SIRSAFLQIQKE +dK_MMCCH-2 (K) | 1302 |
| 40 | 1308 | 1320 | 904.9512 | 1807.8878 | 1807.8749 | 7.08 | G | 0 | 17 | 0.018 | NIAKFHGKPGLCE +dK_MMCCH-2 (K) | 1311 |
| 41 | 1308 | 1320 | 904.9452 | 1807.8758 | 1807.8749 | 0.50 | G | 0 | 27 | 0.0022 | NIAKFHGKPGLCE +dK_MMCCH-2 (K) | 1315 |
| 42 | 1354 | 1378 | 1043.5070 | 3127.4991 | 3127.4961 | 0.96 | G | 3 | 17 | 0.019 | RGSAVAVPYWDWTEKADSLPSLIND +dK_MMCCH-2 (K) | 1368 |
| 43 | 1403 | 1422 | 921.4236 | 2761.2491 | 2761.2330 | 5.83 | G | 3 | 20 | 0.0092 | NAVTSRDPQPELWDNKDFYE +dK_MMCCH-2 (K) | 1418 |
| 44 | 1952 | 1967 | 792.6805 | 2375.0196 | 2375.0205 | -0.38 | G | 3 | 37 | 0.00021 | FSKPEDTFDYHRFGYE +dK_MMCCH-2 (K) | 1954 |
| 45 | 1973 | 1990 | 817.0591 | 2448.1556 | 2448.1454 | 4.17 | G | 0 | 19 | 0.012 | FVGMSVSSLHNYIKQQQE +dK_MMCCH-2 (K); Oxidation (M) | 1986 |
| 46 | 2477 | 2484 | 644.8365 | 1287.6585 | 1287.6573 | 0.93 | G | 0 | 16 | 0.027 | APFFIKVE +dK_MMCCH-2 (K) | 2482 |
| 47 | 3386 | 3405 | 658.3146 | 2629.2292 | 2629.2271 | 0.80 | G | 2 | 25 | 0.0032 | LDHAYSLRDGHYYIAGPTKD +dK_MMCCH-2 (K) | 3404 |
| 48 | 152 | 165 | 667.6934 | 2000.0584 | 2000.0513 | 3.55 | C | 2 | 13 | 0.047 | LNKKTSRAVDDRLF +dK_MMCCH-2 (K) | 155 |
| 49 | 166 | 176 | 547.2858 | 1638.8355 | 1638.8300 | 3.30 | C | 0 | 24 | 0.0036 | EKVQPGHHTRL +dK_MMCCH-2 (K) | 167 |
| 50 | 277 | 296 | 883.7568 | 2648.2487 | 2648.2329 | 5.97 | C | 4 | 39 | 0.00014 | DLTREHAKPADSFDYGRLGY +dK_MMCCH-2 (K) | 284 |

Figure 26B. KLH 2 (continued)

| | | | | | | | | | | | | |
|---|---|---|---|---|---|---|---|---|---|---|---|---|
| 51 | 364 | 373 | 743.3605 | 1484.7064 | 1484.7044 | 1.35 | C | 2 | 19 | 0.012 | LLGGPTEMKW +dK_MMCCH-2 (K); Oxidation (M) | 372 |
| 52 | 423 | 443 | 872.8013 | 2615.3820 | 2615.3795 | 0.96 | C | 1 | 13 | 0.05 | VHRPAKGHFDKPPVPVAQANL +dK_MMCCH-2 (K) | 428 |
| 53 | 629 | 635 | 539.2519 | 1076.4892 | 1076.4848 | 4.09 | C | 0 | 19 | 0.014 | VGGSEKY +dK_MMCCH-2 (K) | 634 |
| 54 | 1007 | 1015 | 669.8724 | 1337.7302 | 1337.7265 | 2.77 | C | 1 | 30 | 0.0011 | TDIAKQVLL +dK_MMCCH-2 (K) | 1011 |
| 55 | 1075 | 1080 | 544.7768 | 1087.5390 | 1087.5372 | 1.66 | C | 1 | 23 | 0.0054 | QALQKY +dK_MMCCH-2 (K) | 1079 |
| 56 | 1298 | 1306 | 715.3770 | 1428.7395 | 1428.7323 | 5.04 | C | 1 | 14 | 0.041 | LQIQKEGIY +dK_MMCCH-2 (K) | 1302 |
| 57 | 1526 | 1540 | 749.6843 | 2246.0312 | 2246.0215 | 4.32 | C | 2 | 36 | 0.00028 | KHNLPQDSFDYQNRF +dK_MMCCH-2 (K) | 1526 |
| 58 | 1589 | 1606 | 784.3560 | 2350.0461 | 2350.0392 | 2.94 | C | 0 | 24 | 0.0045 | ICVEQGGEQNCKTKAGSF +dK_MMCCH-2 (K) | 1602 |
| 59 | 1696 | 1702 | 607.3021 | 1212.5896 | 1212.5849 | 3.88 | C | 1 | 16 | 0.027 | TTLEKHF +dK_MMCCH-2 (K) | 1700 |
| 60 | 1708 | 1719 | 839.8457 | 1677.6768 | 1677.6651 | 7.03 | C | 0 | 16 | 0.023 | KNMQADDSPDGY +dK_MMCCH-2 (K) | 1708 |
| 61 | 1801 | 1809 | 706.8377 | 1411.6607 | 1411.6595 | 0.92 | C | 0 | 15 | 0.035 | SHKTFPNPF +dK_MMCCH-2 (K) | 1803 |
| 62 | 1985 | 1995 | 850.4290 | 1698.8435 | 1698.8399 | 2.12 | C | 0 | 42 | 6.20E-05 | IKQQQEADRVF +dK_MMCCH-2 (K) | 1986 |
| 63 | 1999 | 2003 | 458.2544 | 914.4942 | 914.4936 | 0.77 | C | 2 | 23 | 0.0046 | LLKGF +dK_MMCCH-2 (K) | 2001 |
| 64 | 2455 | 2462 | 645.3270 | 1288.6395 | 1288.6373 | 1.71 | C | 1 | 18 | 0.017 | KYDITQAL +dK_MMCCH-2 (K) | 2455 |
| 65 | 2463 | 2479 | 1128.0206 | 2254.0267 | 2254.0253 | 0.67 | C | 1 | 13 | 0.048 | KAQSIHPEDVFDTDAPF +dK_MMCCH-2 (K) | 2463 |
| 66 | 2754 | 2760 | 598.3320 | 1194.6494 | 1194.6471 | 1.93 | C | 1 | 22 | 0.0058 | KQPLKPF +dK_MMCCH-2 (K) | 2754 |
| 67 | 2754 | 2760 | 598.3337 | 1194.6529 | 1194.6471 | 4.85 | C | 1 | 24 | 0.0041 | KQPLKPF +dK_MMCCH-2 (K) | 2758 |
| 68 | 3235 | 3242 | 617.3142 | 1232.6139 | 1232.6111 | 2.27 | C | 0 | 27 | 0.002 | TSANVKIY +dK_MMCCH-2 (K) | 3240 |
| 69 | 165 | 175 | 558.6147 | 1672.8224 | 1672.8144 | 4.78 | Th | 1 | 16 | 0.025 | FEKVQPGHHTR +dK_MMCCH-2 (K) | 167 |
| 70 | 184 | 191 | 703.8036 | 1405.5927 | 1405.5894 | 2.42 | Th | 1 | 33 | 0.00052 | LEQDEFCK +dK_MMCCH-2 (K) | 191 |
| 71 | 247 | 260 | 631.6209 | 1891.8407 | 1891.8305 | 5.39 | Th | 2 | 17 | 0.021 | LRGKDPNSADCAHN +dK_MMCCH-2 (K) | 250 |
| 72 | 283 | 293 | 782.8585 | 1563.7025 | 1563.7028 | -0.19 | Th | 3 | 47 | 2.00E-05 | AKPADSFDYGR +dK_MMCCH-2 (K) | 284 |
| 73 | 365 | 374 | 707.3312 | 1412.6478 | 1412.6469 | 0.71 | Th | 2 | 42 | 6.90E-05 | LGGPTEMKWG +dK_MMCCH-2 (K) | 372 |
| 74 | 467 | 472 | 538.7419 | 1075.4693 | 1075.4644 | 4.56 | Th | 0 | 13 | 0.048 | FQNDKS +dK_MMCCH-2 (K) | 471 |
| 75 | 554 | 565 | 811.8957 | 1621.7768 | 1621.7658 | 6.84 | Th | 1 | 18 | 0.016 | LDPVTGETKNNP +dK_MMCCH-2 (K) | 562 |
| 76 | 629 | 636 | 582.7667 | 1163.5189 | 1163.5169 | 1.72 | Th | 1 | 16 | 0.024 | VGGSEKYS +dK_MMCCH-2 (K) | 634 |
| 77 | 893 | 904 | 904.8955 | 1807.7765 | 1807.7698 | 3.65 | Th | 1 | 21 | 0.0086 | FHGEPKWCPSPE +dK_MMCCH-2 (K) | 898 |

**Figure 26B. KLH 2 (continued)**

| | | | | | | | | | | | | |
|---|---|---|---|---|---|---|---|---|---|---|---|---|
| 78 | 1300 | 1308 | 716.3455 | 1430.6765 | 1430.6751 | 0.91 | Th | 2 | 23 | 0.0048 | IQKEGIYEN +dK_MMCCH-2 (K) | 1302 |
| 79 | 1414 | 1419 | 564.7546 | 1127.4947 | 1127.4957 | -0.89 | Th | 1 | 14 | 0.04 | LWDNKD +dK_MMCCH-2 (K) | 1418 |
| 80 | 1525 | 1539 | 738.3547 | 2212.0422 | 2212.0371 | 2.31 | Th | 3 | 39 | 0.00012 | LKHNLPQDSFDYQNR +dK_MMCCH-2 (K) | 1526 |
| 81 | 1589 | 1605 | 735.3315 | 2202.9726 | 2202.9708 | 0.82 | Th | 2 | 18 | 0.017 | ICVEQGGEQNCKTKAGS +dK_MMCCH-2 (K) | 1600 |
| 82 | 1589 | 1605 | 735.3328 | 2202.9766 | 2202.9708 | 2.68 | Th | 2 | 21 | 0.0076 | ICVEQGGEQNCKTKAGS +dK_MMCCH-2 (K) | 1602 |
| 83 | 1800 | 1809 | 780.3729 | 1558.7313 | 1558.7279 | 2.18 | Th | 2 | 14 | 0.043 | FSHKTFPNPF +dK_MMCCH-2 (K) | 1803 |
| 84 | 1952 | 1963 | 627.2849 | 1878.8327 | 1878.8247 | 4.26 | Th | 2 | 48 | 1.70E-05 | FSKPEDTFDYHR +dK_MMCCH-2 (K) | 1954 |
| 85 | 1985 | 1994 | 776.8962 | 1551.7778 | 1551.7715 | 4.06 | Th | 2 | 28 | 0.0015 | IKQQQEADRV +dK_MMCCH-2 (K) | 1986 |
| 86 | 2193 | 2199 | 667.3018 | 1332.5891 | 1332.5849 | 3.15 | Th | 2 | 27 | 0.0021 | YWDWTKP +dK_MMCCH-2 (K) | 2198 |
| 87 | 2200 | 2206 | 562.3079 | 1122.6012 | 1122.5995 | 1.51 | Th | 2 | 19 | 0.013 | ISKLPDL +dK_MMCCH-2 (K) | 2202 |
| 88 | 2225 | 2229 | 462.2217 | 922.4289 | 922.4259 | 3.36 | Th | 2 | 22 | 0.0056 | FAKGY +dK_MMCCH-2 (K) | 2227 |
| 89 | 2230 | 2237 | 632.7946 | 1263.5747 | 1263.5693 | 4.27 | Th | 2 | 18 | 0.016 | IKSEDAYT +dK_MMCCH-2 (K) | 2231 |
| 90 | 2804 | 2815 | 937.4586 | 1872.9026 | 1872.9073 | -2.51 | Th | 2 | 15 | 0.034 | AMLQERKKEERT +Deamidated (NQ); dK_MMCCH-2 (K); Oxidation (M) | 2811 |
| 91 | 2941 | 2950 | 720.8405 | 1439.6663 | 1439.6602 | 4.24 | Th | 3 | 49 | 1.30E-05 | LDEANDLKNA +dK_MMCCH-2 (K) | 2948 |
| 92 | 3009 | 3015 | 573.2770 | 1144.5395 | 1144.5335 | 5.24 | Th | 0 | 16 | 0.023 | LKEHGSH +dK_MMCCH-2 (K) | 3010 |
| 93 | 3399 | 3405 | 520.2615 | 1038.5085 | 1038.5056 | 2.79 | Th | 1 | 28 | 0.0015 | IAGPTKD +dK_MMCCH-2 (K) | 3404 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the lysine conjugation site for KLH1 or KLH2.

**Figure 27. Identification details of MMCCH-conjugated peptides for sample 2 (2nd LC-MS/MS)**

**Figure 27A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 124 | 141 | 785.4103 | 2353.2092 | 2353.2001 | 3.87 | T | 1 | 17 | 0.02 | GFTDPPVKHHQSANLLVR +dK_MMCCH-2 (K) | 131 |
| 2 | 142 | 149 | 656.3403 | 1310.6661 | 1310.6653 | 0.69 | T | 1 | 29 | 0.0013 | KNINDLTR +dK_MMCCH-2 (K) | 142 |
| 3 | 157 | 166 | 823.3675 | 1644.7204 | 1644.7242 | -2.31 | T | 1 | 24 | 0.0038 | EAFHKFQEDR +Deamidated (NQ); dK_MMCCH-2 (K) | 161 |
| 4 | 184 | 192 | 726.8430 | 1451.6715 | 1451.6649 | 4.48 | T | 1 | 26 | 0.0026 | CPRPDAKDR +dK_MMCCH-2 (K) | 190 |
| 5 | 553 | 560 | 663.8685 | 1325.7224 | 1325.7166 | 4.37 | T | 2 | 26 | 0.0026 | VKFDKVPR +dK_MMCCH-2 (K) | 554 |
| 6 | 566 | 570 | 485.2447 | 968.4748 | 968.4749 | -0.21 | T | 1 | 19 | 0.014 | KNVDR +dK_MMCCH-2 (K) | 566 |
| 7 | 581 | 587 | 547.8374 | 1093.6602 | 1093.6569 | 3.02 | T | 1 | 18 | 0.018 | KALALLK +dK_MMCCH-2 (K) | 581 |
| 8 | 588 | 606 | 781.0366 | 2340.0879 | 2340.0845 | 1.45 | T | 1 | 14 | 0.036 | EDKSAGGFQQLGAFHGEPK +dK_MMCCH-2 (K) | 590 |
| 9 | 645 | 681 | 1158.5381 | 4630.1233 | 4630.1063 | 3.65 | T | 1 | 18 | 0.015 | HGYDGALPYWDWTSPLNHLPELADHEKYVDPEDGVEK +dK_MMCCH-2 (K) | 671 |
| 10 | 682 | 699 | 620.2961 | 2477.1555 | 2477.1434 | 4.84 | T | 1 | 21 | 0.0075 | HNPWFDGHIDTVDKTTTR +dK_MMCCH-2 (K) | 695 |
| 11 | 700 | 719 | 887.7724 | 2660.2954 | 2660.2945 | 0.34 | T | 1 | 20 | 0.01 | SVQNKLFEQPEFGHYTSIAK +dK_MMCCH-2 (K) | 704 |
| 12 | 778 | 789 | 957.9997 | 1913.9848 | 1913.9862 | -0.68 | T | 1 | 39 | 0.00012 | IWAIWQALQKYR +Deamidated (NQ); dK_MMCCH-2 (K) | 787 |
| 13 | 790 | 804 | 1003.4713 | 2004.9281 | 2004.9220 | 3.04 | T | 0 | 48 | 1.60E-05 | GKPYNVANCAVTSMR +dK_MMCCH-2 (K) | 791 |
| 14 | 858 | 863 | 520.2989 | 1038.5832 | 1038.5783 | 4.72 | T | 1 | 17 | 0.022 | KLEAIK +dK_MMCCH-2 (K) | 858 |
| 15 | 916 | 924 | 740.8735 | 1479.7324 | 1479.7319 | 0.27 | T | 1 | 16 | 0.025 | VFKYDITEK +dK_MMCCH-2 (K) | 918 |
| 16 | 953 | 965 | 635.3304 | 1902.9693 | 1902.9662 | 1.63 | T | 1 | 43 | 5.20E-05 | DLFKQPSVIHEPR +dK_MMCCH-2 (K) | 956 |
| 17 | 986 | 1000 | 1027.0363 | 2052.0579 | 2052.0561 | 0.93 | T | 1 | 66 | 2.50E-07 | KNIENLSLGELESLR +dK_MMCCH-2 (K) | 986 |
| 18 | 1092 | 1114 | 791.8748 | 3163.4699 | 3163.4682 | 0.54 | T | 1 | 41 | 8.30E-05 | QHHYETNPFHHGKITHENEITTR +dK_MMCCH-2 (K) | 1104 |
| 19 | 1118 | 1162 | 1414.4266 | 5653.6775 | 5653.6850 | -1.34 | T | 1 | 23 | 0.0049 | DSLFHSDYFYEQVLYALEQDNFCDFEIQLEILHNALHSLLGGKGK +dK_MMCCH-2 (K) | 1162 |
| 20 | 1199 | 1203 | 353.1902 | 1056.5487 | 1056.5538 | -4.83 | T | 1 | 17 | 0.02 | KRPYR +dK_MMCCH-2 (K) | 1199 |
| 21 | 1395 | 1403 | 727.8865 | 1453.7585 | 1453.7599 | -0.96 | T | 2 | 30 | 0.00092 | KDITQLDKR +dK_MMCCH-2 (K) | 1395 |
| 22 | 1395 | 1403 | 598.3055 | 1791.8946 | 1791.8899 | 2.62 | T | 2 | 15 | 0.031 | KDITQLDKR +2 dK_MMCCH-2 (K) | 1395, 1402 |
| 23 | 1417 | 1445 | 1125.2129 | 3372.6168 | 3372.6020 | 4.42 | T | 1 | 16 | 0.024 | ADHSSDGFQAIASFHALPPLCPSPAASKR +dK_MMCCH-2 (K) | 1444 |

**Figure 27A. KLH 1 (continued)**

| | | | | | | | | | | | | |
|---|---|---|---|---|---|---|---|---|---|---|---|---|
| 24 | 1473 | 1489 | 778.4003 | 2332.1790 | 2332.1827 | -1.59 | T | 1 | 46 | 2.60E-05 | KHGAVVGLPYWDWTLPR +dK_MMCCH-2 (K) | 1473 |
| 25 | 1747 | 1758 | 613.9767 | 1838.9082 | 1838.9012 | 3.81 | T | 1 | 21 | 0.0084 | LYKYDITETLDK +dK_MMCCH-2 (K) | 1749 |
| 26 | 1923 | 1932 | 756.3794 | 1510.7442 | 1510.7425 | 1.19 | T | 1 | 28 | 0.0016 | NKVMPNPFAR +dK_MMCCH-2 (K) | 1924 |
| 27 | 2100 | 2109 | 492.2465 | 1964.9570 | 1964.9601 | -1.58 | T | 2 | 20 | 0.0094 | EIKDKQHHVR +2 dK_MMCCH-2 (K) | 2102, 2104 |
| 28 | 2121 | 2149 | 890.4189 | 3557.6464 | 3557.6378 | 2.42 | T | 1 | 40 | 9.10E-05 | TSADVQFQICKTSEDCHHGGQIFVLGGTK +dK_MMCCH-2 (K) | 2131 |
| 29 | 2299 | 2314 | 715.0167 | 2142.0283 | 2142.0238 | 2.15 | T | 1 | 32 | 0.00069 | LYTKQMEDALTAHGAR +dK_MMCCH-2 (K) | 2302 |
| 30 | 2359 | 2367 | 736.3491 | 1470.6836 | 1470.6813 | 1.56 | T | 1 | 35 | 0.00031 | DKLFNDPER +dK_MMCCH-2 (K) | 2360 |
| 31 | 2507 | 2514 | 682.3539 | 1362.6932 | 1362.6853 | 5.80 | T | 1 | 40 | 0.0001 | KLEHELEK +dK_MMCCH-2 (K) | 2507 |
| 32 | 2515 | 2520 | 571.7606 | 1141.5066 | 1141.5074 | -0.70 | T | 1 | 21 | 0.0086 | QKEEDR +dK_MMCCH-2 (K) | 2516 |
| 33 | 2595 | 2609 | 983.5358 | 1965.0571 | 1965.0493 | 4.02 | T | 1 | 15 | 0.035 | IIDTSGKQLPSDLIK +dK_MMCCH-2 (K) | 2601 |
| 34 | 2621 | 2636 | 802.7097 | 2405.1073 | 2405.1083 | -0.42 | T | 1 | 51 | 8.60E-06 | HHEKHHEDHHEDILVR +dK_MMCCH-2 (K) | 2624 |
| 35 | 2637 | 2651 | 1069.5260 | 2137.0374 | 2137.0374 | 0.00 | T | 1 | 74 | 4.00E-08 | KNIHSLSHHEAEELR +dK_MMCCH-2 (K) | 2637 |
| 36 | 2761 | 2814 | 1324.8440 | 6619.1836 | 6619.1192 | 9.73 | T | 1 | 16 | 0.023 | DVNEAIFQQTKFGEFSSIFYLALQALEEDNYCDFEVQYEILHNEVHALIGGAEK +dK_MMCCH-2 (K) | 2771 |
| 37 | 2851 | 2883 | 1009.2193 | 4032.8481 | 4032.8346 | 3.35 | T | 0 | 51 | 7.20E-06 | VKPAHAGSCAGDIMHVPLHPFNYESVNNDDFTR +dK_MMCCH-2 (K) | 2852 |
| 38 | 2897 | 2919 | 1066.5216 | 3196.5430 | 3196.5287 | 4.47 | T | 1 | 75 | 3.50E-08 | FNYKYDNLNLHGHNIEELEEVLR +dK_MMCCH-2 (K) | 2900 |
| 39 | 2938 | 2947 | 730.4158 | 1458.8170 | 1458.8156 | 0.96 | T | 1 | 17 | 0.02 | TTAVVKVYIK +dK_MMCCH-2 (K) | 2943 |
| 40 | 3003 | 3021 | 825.7742 | 2474.3007 | 2474.2879 | 5.17 | T | 1 | 39 | 0.00013 | KYDHTELDASVLPAPIIVR +dK_MMCCH-2 (K) | 3003 |
| 41 | 123 | 151 | 616.6595 | 3693.9132 | 3693.9049 | 2.25 | G | 2 | 21 | 0.0073 | KGFTDPPVKHHQSANLLVRKNINDLTREE +dK_MMCCH-2 (K) | 123 |
| 42 | 123 | 151 | 673.0134 | 4032.0366 | 4032.0350 | 0.40 | G | 2 | 16 | 0.023 | KGFTDPPVKHHQSANLLVRKNINDLTREE +2 dK_MMCCH-2 (K) | 123, 142 |
| 43 | 123 | 151 | 739.7915 | 3693.9211 | 3693.9049 | 4.39 | G | 2 | 22 | 0.0063 | KGFTDPPVKHHQSANLLVRKNINDLTREE +dK_MMCCH-2 (K) | 131 |
| 44 | 158 | 176 | 846.3875 | 2536.1407 | 2536.1329 | 3.08 | G | 3 | 19 | 0.011 | AFHKFQEDRSVDGYQATAE +dK_MMCCH-2 (K) | 161 |
| 45 | 435 | 442 | 676.3240 | 1350.6335 | 1350.6312 | 1.78 | G | 1 | 26 | 0.0026 | NIEKMIHE +dK_MMCCH-2 (K) | 438 |
| 46 | 526 | 544 | 777.7499 | 2330.2280 | 2330.2304 | -1.03 | G | 1 | 27 | 0.0021 | VDGTKLASSLIPHASVIRE +dK_MMCCH-2 (K) | 530 |
| 47 | 589 | 612 | 990.1188 | 2967.3345 | 2967.3320 | 0.84 | G | 1 | 16 | 0.024 | DKSAGGFQQLGAFHGEPKWCPSPE +dK_MMCCH-2 (K) | 606 |
| 48 | 825 | 841 | 820.3725 | 2458.0957 | 2458.0940 | 0.69 | G | 1 | 38 | 0.00015 | HSVPFNVFDYKTNFNYE +dK_MMCCH-2 (K) | 835 |
| 49 | 847 | 860 | 965.0152 | 1928.0158 | 1928.0077 | 4.20 | G | 0 | 23 | 0.005 | FNGLSISQLNKKLE +dK_MMCCH-2 (K) | 858 |

**Figure 27A. KLH 1 (continued)**

| | | | | | | | | | | | | |
|---|---|---|---|---|---|---|---|---|---|---|---|---|
| 50 | 1055 | 1076 | 810.1688 | 3236.6462 | 3236.6402 | 1.82 | G | 1 | 24 | 0.0041 | NALLKKGSSVAVPYWDWTKRIE +2 dK_MMCCH-2 (K) | 1059, 1060 |
| 51 | 1055 | 1076 | 725.6330 | 2898.5029 | 2898.5102 | -2.52 | G | 1 | 37 | 0.00021 | NALLKKGSSVAVPYWDWTKRIE +dK_MMCCH-2 (K) | 1060 |
| 52 | 1097 | 1110 | 999.9601 | 1997.9057 | 1997.9054 | 0.15 | G | 1 | 23 | 0.0052 | TNPFHHGKITHENE +dK_MMCCH-2 (K) | 1104 |
| 53 | 1376 | 1396 | 864.3901 | 2590.1484 | 2590.1428 | 2.16 | G | 3 | 20 | 0.011 | AGTDSAHTDDGHTEPVMIRKD +dK_MMCCH-2 (K) | 1395 |
| 54 | 1397 | 1413 | 665.6099 | 2658.4103 | 2658.4125 | -0.79 | G | 1 | 13 | 0.046 | ITQLDKRQQLSLVKALE +2 dK_MMCCH-2 (K) | 1402, 1410 |
| 55 | 1397 | 1413 | 774.4366 | 2320.2879 | 2320.2824 | 2.37 | G | 1 | 22 | 0.0064 | ITQLDKRQQLSLVKALE +dK_MMCCH-2 (K) | 1410 |
| 56 | 1495 | 1522 | 866.6912 | 3462.7358 | 3462.7381 | -0.66 | G | 3 | 21 | 0.0086 | LLTVSTIHDPETGRDIPNPFIGSKIEFE +dK_MMCCH-2 (K) | 1518 |
| 57 | 1755 | 1765 | 570.6042 | 1708.7909 | 1708.7913 | -0.18 | G | 2 | 27 | 0.002 | TLDKMNLRHDE +dK_MMCCH-2 (K) | 1758 |
| 58 | 2089 | 2100 | 854.4125 | 1706.8104 | 1706.8008 | 5.68 | G | 2 | 20 | 0.011 | IGGMNLHEIEKE +dK_MMCCH-2 (K) | 2099 |
| 59 | 2647 | 2661 | 715.6751 | 2144.0035 | 2144.0095 | -2.80 | G | 4 | 35 | 0.00035 | AEELRDALYKLQNDE +dK_MMCCH-2 (K) | 2656 |
| 60 | 3090 | 3097 | 632.8023 | 1263.5901 | 1263.5879 | 1.66 | G | 0 | 15 | 0.029 | LGKMYSVE +dK_MMCCH-2 (K) | 3092 |
| 61 | 59 | 68 | 729.3450 | 1456.6755 | 1456.6731 | 1.65 | C | 1 | 17 | 0.022 | VLGGPSEMKW +dK_MMCCH-2 (K); Oxidation (M) | 67 |
| 62 | 94 | 100 | 573.2991 | 1144.5836 | 1144.5838 | -0.17 | C | 1 | 20 | 0.011 | TVKAELF +dK_MMCCH-2 (K) | 96 |
| 63 | 160 | 171 | 606.9445 | 1817.8116 | 1817.8043 | 4.02 | C | 1 | 17 | 0.021 | HKFQEDRSVDGY +dK_MMCCH-2 (K) | 161 |
| 64 | 587 | 595 | 638.7999 | 1275.5852 | 1275.5805 | 3.68 | C | 0 | 31 | 0.00081 | KEDKSAGGF +dK_MMCCH-2 (K) | 590 |
| 65 | 715 | 723 | 655.8767 | 1309.7387 | 1309.7316 | 5.50 | C | 1 | 20 | 0.0094 | TSIAKQVLL +dK_MMCCH-2 (K) | 719 |
| 66 | 783 | 788 | 544.7763 | 1087.5381 | 1087.5372 | 0.83 | C | 1 | 22 | 0.0067 | QALQKY +dK_MMCCH-2 (K) | 787 |
| 67 | 918 | 928 | 856.9330 | 1711.8515 | 1711.8491 | 1.40 | C | 2 | 14 | 0.038 | KYDITEKLHDL +dK_MMCCH-2 (K) | 924 |
| 68 | 1330 | 1337 | 620.3548 | 1238.6950 | 1238.6944 | 0.48 | C | 1 | 24 | 0.004 | KLDITKAL +dK_MMCCH-2 (K) | 1335 |
| 69 | 1431 | 1446 | 696.3564 | 2086.0473 | 2086.0492 | -0.91 | C | 1 | 13 | 0.045 | HALPPLCPSPAASKRF +dK_MMCCH-2 (K) | 1444 |
| 70 | 1580 | 1586 | 544.2603 | 1086.5061 | 1086.5056 | 0.46 | C | 0 | 13 | 0.046 | VGGKEPY +dK_MMCCH-2 (K) | 1583 |
| 71 | 1649 | 1664 | 752.3298 | 2253.9675 | 2253.9637 | 1.69 | C | 2 | 14 | 0.044 | NLNDHTHDFSKPEDTF +dK_MMCCH-2 (K) | 1659 |
| 72 | 1818 | 1827 | 753.3687 | 1504.7227 | 1504.7232 | -0.27 | C | 1 | 22 | 0.0061 | SERDIGSLKY +dK_MMCCH-2 (K) | 1826 |
| 73 | 2476 | 2488 | 923.9230 | 1845.8315 | 1845.8244 | 3.90 | C | 1 | 22 | 0.0061 | THSNAKPTDVFEY +dK_MMCCH-2 (K) | 2481 |
| 74 | 2656 | 2666 | 793.3514 | 1584.6882 | 1584.6879 | 0.25 | C | 1 | 32 | 0.00057 | KLQNDESHGGY +dK_MMCCH-2 (K) | 2656 |
| 75 | 2738 | 2749 | 856.3745 | 1710.7343 | 1710.7348 | -0.29 | C | 2 | 19 | 0.013 | ADSGNNNPFFKY +dK_MMCCH-2 (K) | 2748 |
| 76 | 2946 | 2960 | 965.4053 | 1928.7960 | 1928.7986 | -1.35 | C | 1 | 23 | 0.0053 | IKSGTDSDDEYAGSF +dK_MMCCH-2 (K) | 2947 |

Figure 27A. KLH 1 (continued)

| | | | | | | | | | | | | |
|---|---|---|---|---|---|---|---|---|---|---|---|---|
| 77 | 2961 | 2971 | 769.8909 | 1537.7673 | 1537.7673 | 0.00 | C | 1 | 19 | 0.013 | VILGGAKEMPW +dK_MMCCH-2 (K) | 2967 |
| 78 | 1 | 9 | 725.3376 | 1448.6606 | 1448.6567 | 2.69 | Th | 2 | 25 | 0.0032 | MTPEELKTY +dK_MMCCH-2 (K) | 7 |
| 79 | 38 | 56 | 861.6801 | 2582.0183 | 2582.0108 | 2.90 | Th | 2 | 15 | 0.03 | VCIPDDNDRNDDHCEKAGD +dK_MMCCH-2 (K) | 53 |
| 80 | 59 | 69 | 785.3757 | 1568.7368 | 1568.7367 | 0.06 | Th | 3 | 58 | 1.70E-06 | VLGGPSEMKWQ +dK_MMCCH-2 (K) | 67 |
| 81 | 78 | 84 | 569.2871 | 1136.5595 | 1136.5536 | 5.19 | Th | 1 | 50 | 9.20E-06 | LSDTVHK +dK_MMCCH-2 (K) | 84 |
| 82 | 118 | 124 | 571.2781 | 1140.5417 | 1140.5386 | 2.72 | Th | 0 | 23 | 0.0046 | VHHPEKG +dK_MMCCH-2 (K) | 123 |
| 83 | 431 | 439 | 707.8519 | 1413.6893 | 1413.6883 | 0.71 | Th | 3 | 24 | 0.004 | ISLENIEKM +dK_MMCCH-2 (K) | 438 |
| 84 | 521 | 531 | 764.3844 | 1526.7542 | 1526.7538 | 0.26 | Th | 3 | 44 | 3.60E-05 | VDITEVDGTKL +dK_MMCCH-2 (K) | 530 |
| 85 | 585 | 591 | 585.8102 | 1169.6058 | 1169.6002 | 4.79 | Th | 1 | 35 | 0.00031 | LLKEDKS +dK_MMCCH-2 (K) | 590 |
| 86 | 601 | 612 | 904.8973 | 1807.7801 | 1807.7698 | 5.70 | Th | 1 | 18 | 0.018 | FHGEPKWCPSPE +dK_MMCCH-2 (K) | 606 |
| 87 | 666 | 672 | 607.2836 | 1212.5526 | 1212.5485 | 3.38 | Th | 2 | 23 | 0.0045 | LADHEKY +dK_MMCCH-2 (K) | 671 |
| 88 | 673 | 685 | 930.4165 | 1858.8184 | 1858.8196 | -0.65 | Th | 2 | 14 | 0.044 | VDPEDGVEKHNPW +dK_MMCCH-2 (K) | 681 |
| 89 | 831 | 837 | 612.7872 | 1223.5598 | 1223.5533 | 5.31 | Th | 2 | 26 | 0.0023 | VFDYKTN +dK_MMCCH-2 (K) | 835 |
| 90 | 1156 | 1164 | 630.8394 | 1259.6642 | 1259.6584 | 4.60 | Th | 2 | 15 | 0.028 | LLGGKGKYS +dK_MMCCH-2 (K) | 1162 |
| 91 | 1219 | 1232 | 766.6633 | 2296.9682 | 2296.9617 | 2.83 | Th | 1 | 17 | 0.02 | FDKSDNNDEATKTH +2 dK_MMCCH-2 (K) | 1221, 1230 |
| 92 | 1219 | 1232 | 653.9531 | 1958.8376 | 1958.8316 | 3.01 | Th | 1 | 26 | 0.0024 | FDKSDNNDEATKTH +dK_MMCCH-2 (K) | 1230 |
| 93 | 1580 | 1587 | 572.7737 | 1143.5329 | 1143.5271 | 5.16 | Th | 1 | 18 | 0.016 | VGGKEPYG +dK_MMCCH-2 (K) | 1583 |
| 94 | 1657 | 1668 | 921.9181 | 1841.8216 | 1841.8182 | 1.85 | Th | 2 | 34 | 0.00042 | FSKPEDTFDYQK +dK_MMCCH-2 (K) | 1659 |
| 95 | 1657 | 1668 | 921.9170 | 1841.8194 | 1841.8182 | 0.65 | Th | 2 | 45 | 3.20E-05 | FSKPEDTFDYQK +dK_MMCCH-2 (K) | 1668 |
| 96 | 1733 | 1741 | 664.3219 | 1326.6292 | 1326.6312 | -1.43 | Th | 2 | 17 | 0.019 | VLGGEKEMP +dK_MMCCH-1 (K); Oxidation (M) | 1738 |
| 97 | 1894 | 1904 | 850.4162 | 1698.8178 | 1698.8116 | 3.65 | Th | 5 | 36 | 0.00024 | VAVPYWDWTKP +dK_MMCCH-2 (K) | 1903 |
| 98 | 2064 | 2068 | 499.7590 | 997.5035 | 997.5055 | -2.01 | Th | 0 | 20 | 0.0095 | FTKKH +dK_MMCCH-2 (K) | 2067 |
| 99 | 2094 | 2100 | 618.3051 | 1234.5957 | 1234.5903 | 4.29 | Th | 1 | 29 | 0.0013 | LHEIEKE +dK_MMCCH-2 (K) | 2099 |
| 100 | 2129 | 2141 | 622.9244 | 1865.7515 | 1865.7495 | 1.07 | Th | 0 | 26 | 0.0026 | ICKTSEDCHHGGQ +dK_MMCCH-2 (K) | 2131 |
| 101 | 2238 | 2249 | 814.3990 | 1626.7835 | 1626.7811 | 1.54 | Th | 4 | 45 | 3.40E-05 | LTTAEVDNLKDA +dK_MMCCH-2 (K) | 2247 |
| 102 | 2512 | 2521 | 807.3978 | 1612.7810 | 1612.7766 | 2.67 | Th | 0 | 25 | 0.0035 | LEKQKEEDRT +dK_MMCCH-2 (K) | 2514 |
| 103 | 2595 | 2606 | 806.4025 | 1610.7904 | 1610.7862 | 2.61 | Th | 2 | 31 | 0.00079 | IIDTSGKQLPSD +dK_MMCCH-2 (K) | 2601 |

**Figure 27A. KLH 1 (continued)**

| | | | | | | | | | | | | |
|---|---|---|---|---|---|---|---|---|---|---|---|---|
| 104 | 2607 | 2612 | 520.7792 | 1039.5439 | 1039.5446 | -0.67 | Th | 2 | 26 | 0.0027 | LIKMPT +dK_MMCCH-2 (K) | 2609 |
| 105 | 2766 | 2771 | 551.7890 | 1101.5634 | 1101.5529 | 9.62 | Th | 1 | 29 | 0.0013 | IFQQTK +dK_MMCCH-2 (K) | 2771 |
| 106 | 2808 | 2816 | 638.3207 | 1274.6268 | 1274.6217 | 4.08 | Th | 3 | 27 | 0.0018 | LIGGAEKYS +dK_MMCCH-2 (K) | 2814 |
| 107 | 2946 | 2960 | 965.4059 | 1928.7972 | 1928.7986 | -0.73 | Th | 3 | 37 | 0.0002 | IKSGTDSDDEYAGSF +dK_MMCCH-2 (K) | 2947 |
| 108 | 2990 | 2996 | 583.2842 | 1164.5538 | 1164.5485 | 4.55 | Th | 1 | 38 | 0.00017 | LTDDHVK +dK_MMCCH-2 (K) | 2996 |

Figure 27B. KLH 2

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1043.0210 | 2084.0274 | 2084.0347 | -3.50 | T | 1 | 32 | 0.00066 | KNVDSLSSDEVLALEK +dK_MMCCH-2 (K) | 26 |
| 2 | 147 | 155 | 694.3692 | 1386.7238 | 1386.7217 | 1.51 | T | 1 | 15 | 0.031 | ADITFLNKK +dK_MMCCH-2 (K) | 155 |
| 3 | 164 | 175 | 596.3080 | 1785.9023 | 1785.8985 | 2.13 | T | 1 | 33 | 0.00048 | LFEKVQPGHHTR +dK_MMCCH-2 (K) | 167 |
| 4 | 249 | 271 | 987.4402 | 2959.2989 | 2959.3051 | -2.10 | T | 1 | 39 | 0.00012 | GKDPNSADCAHNLIHTPMEPFDR +dK_MMCCH-2 (K) | 250 |
| 5 | 281 | 293 | 610.9460 | 1829.8163 | 1829.8043 | 6.56 | T | 0 | 55 | 3.40E-06 | EHAKPADSFDYGR +dK_MMCCH-2 (K) | 284 |
| 6 | 359 | 377 | 849.4008 | 2545.1806 | 2545.1810 | -0.16 | T | 1 | 14 | 0.041 | AGDFFLLGGPTEMKWGFYR +dK_MMCCH-2 (K); Oxidation (M) | 372 |
| 7 | 429 | 446 | 752.0674 | 2253.1805 | 2253.1729 | 3.37 | T | 0 | 52 | 6.60E-06 | GHFDKPPVPVAQANLAVR +dK_MMCCH-2 (K) | 433 |
| 8 | 467 | 488 | 708.5944 | 2830.3483 | 2830.3385 | 3.50 | T | 1 | 65 | 2.90E-07 | FQNDKSVDGYQATVEFHALPAR +dK_MMCCH-2 (K) | 471 |
| 9 | 489 | 497 | 726.8430 | 1451.6715 | 1451.6649 | 4.48 | T | 1 | 26 | 0.0026 | CPRPDAKDR +dK_MMCCH-2 (K) | 495 |
| 10 | 936 | 950 | 1080.0154 | 2158.0162 | 2158.0095 | 3.10 | T | 1 | 43 | 5.40E-05 | KHGFTGGLPYWDWTR +dK_MMCCH-2 (K) | 936 |
| 11 | 1082 | 1096 | 996.4625 | 1990.9104 | 1990.9063 | 2.06 | T | 0 | 38 | 0.00014 | GKPYNTANCAIASMR +dK_MMCCH-2 (K) | 1083 |
| 12 | 1097 | 1115 | 827.0796 | 2478.2169 | 2478.2101 | 2.78 | T | 0 | 65 | 3.10E-07 | KPLQPFGLDSVINPDDETR +dK_MMCCH-2 (K) | 1097 |
| 13 | 1123 | 1149 | 1190.5587 | 3568.6543 | 3568.6497 | 1.32 | T | 1 | 70 | 1.10E-07 | VFDYKNNFDYEYESLAFNGLSIAQLDR +dK_MMCCH-2 (K) | 1127 |
| 14 | 1355 | 1385 | 1271.2697 | 3810.7871 | 3810.7876 | -0.10 | T | 1 | 27 | 0.0018 | GSAVAVPYWDWTEKADSLPSLINDATYFNSR +dK_MMCCH-2 (K) | 1368 |
| 15 | 1409 | 1450 | 1097.7139 | 5483.5330 | 5483.5213 | 2.13 | T | 1 | 37 | 0.00018 | DPQPELWDNKDFYENVMLALEQDNFCDFEIQLELIHNALHSR +dK_MMCCH-2 (K) | 1418 |
| 16 | 1455 | 1480 | 838.6586 | 3350.6054 | 3350.6070 | -0.48 | T | 1 | 54 | 3.80E-06 | AKYSLSSLDYTAFDPVFFLHHANVDR +dK_MMCCH-2 (K) | 1456 |
| 17 | 1493 | 1507 | 721.6621 | 2161.9645 | 2161.9594 | 2.36 | T | 1 | 48 | 1.60E-05 | KKPYNEADCAVNEMR +dK_MMCCH-2 (K) | 1494 |
| 18 | 1542 | 1564 | 1070.1956 | 3207.5649 | 3207.5448 | 6.27 | T | 1 | 22 | 0.0058 | YQYDNLQFNHFSIQKLDQTIQAR +dK_MMCCH-2 (K) | 1556 |
| 19 | 1690 | 1704 | 709.3851 | 2125.1334 | 2125.1241 | 4.33 | T | 2 | 16 | 0.026 | KEVSSLTTLEKHFLR +dK_MMCCH-2 (K) | 1700 |
| 20 | 1757 | 1768 | 612.9798 | 1835.9176 | 1835.9128 | 2.61 | T | 1 | 14 | 0.043 | LYTVQFEDSLKR +dK_MMCCH-2 (K) | 1767 |
| 21 | 1804 | 1825 | 945.4659 | 2833.3760 | 2833.3633 | 4.48 | T | 1 | 14 | 0.044 | TFPNPFLKANIEFEGEGVTTER +dK_MMCCH-2 (K) | 1811 |
| 22 | 1949 | 1963 | 560.2521 | 2236.9795 | 2236.9735 | 2.64 | T | 0 | 55 | 2.90E-06 | TQEFSKPEDTFDYHR +dK_MMCCH-2 (K) | 1954 |
| 23 | 2045 | 2052 | 660.3509 | 1318.6872 | 1318.6843 | 2.27 | T | 1 | 15 | 0.034 | YDITKTLK +dK_MMCCH-2 (K) | 2049 |
| 24 | 2228 | 2239 | 870.4226 | 1738.8307 | 1738.8236 | 4.08 | T | 1 | 14 | 0.038 | GYIKSEDAYTVR +dK_MMCCH-2 (K) | 2231 |
| 25 | 2453 | 2463 | 839.9299 | 1677.8452 | 1677.8436 | 0.95 | T | 1 | 13 | 0.048 | NFKYDITQALK +dK_MMCCH-2 (K) | 2455 |

Figure 27B. KLH 2 (continued)

| | | | | | | | | | | | | |
|---|---|---|---|---|---|---|---|---|---|---|---|---|
| 26 | 2527 | 2541 | 1007.5138 | 2013.0130 | 2013.0088 | 2.09 | T | 1 | 66 | 2.80E-07 | KDVTSLTASEIENLR +dK_MMCCH-2 (K) | 2527 |
| 27 | 2728 | 2739 | 957.9997 | 1913.9848 | 1913.9862 | -0.68 | T | 1 | 39 | 0.00012 | IWAIWQALQKYR +Deamidated (NQ); dK_MMCCH-2 (K) | 2737 |
| 28 | 2755 | 2764 | 764.3805 | 1526.7464 | 1526.7439 | 1.64 | T | 0 | 33 | 0.00054 | QPLKPFSESR +Deamidated (NQ); dK_MMCCH-2 (K) | 2758 |
| 29 | 2866 | 2873 | 659.3614 | 1316.7083 | 1316.7050 | 2.51 | T | 1 | 18 | 0.016 | YDITKVLK +dK_MMCCH-2 (K) | 2870 |
| 30 | 3148 | 3155 | 629.3343 | 1256.6541 | 1256.6587 | -3.58 | T | 1 | 16 | 0.028 | KKPYNAAK +dK_MMCCH-2 (K) | 3149 |
| 31 | 167 | 178 | 590.9642 | 1769.8707 | 1769.8705 | 0.11 | G | 0 | 27 | 0.0018 | KVQPGHHTRLME +dK_MMCCH-2 (K) | 167 |
| 32 | 466 | 481 | 1098.4980 | 2194.9815 | 2194.9841 | -1.18 | G | 2 | 24 | 0.0036 | RFQNDKSVDGYQATVE +Deamidated (NQ); dK_MMCCH-2 (K) | 471 |
| 33 | 1215 | 1235 | 721.6050 | 2882.3908 | 2882.3949 | -1.42 | G | 3 | 36 | 0.00026 | ITQQLHDLDLHVGDNFFLKYE +dK_MMCCH-2 (K) | 1233 |
| 34 | 1292 | 1303 | 879.4659 | 1756.9172 | 1756.9182 | -0.57 | G | 0 | 36 | 0.00028 | SIRSAFLQIQKE +dK_MMCCH-2 (K) | 1302 |
| 35 | 1308 | 1320 | 904.9471 | 1807.8796 | 1807.8749 | 2.60 | G | 0 | 29 | 0.0014 | NIAKFHGKPGLCE +dK_MMCCH-2 (K) | 1315 |
| 36 | 1403 | 1422 | 921.4155 | 2761.2248 | 2761.2330 | -2.97 | G | 3 | 23 | 0.0054 | NAVTSRDPQPELWDNKDFYE +dK_MMCCH-2 (K) | 1418 |
| 37 | 1952 | 1967 | 792.6802 | 2375.0189 | 2375.0205 | -0.67 | G | 3 | 30 | 0.00092 | FSKPEDTFDYHRFGYE +dK_MMCCH-2 (K) | 1954 |
| 38 | 1973 | 1990 | 811.7284 | 2432.1635 | 2432.1505 | 5.39 | G | 0 | 21 | 0.008 | FVGMSVSSLHNYIKQQQE +dK_MMCCH-2 (K) | 1986 |
| 39 | 2477 | 2484 | 644.8361 | 1287.6576 | 1287.6573 | 0.16 | G | 0 | 25 | 0.003 | APFFIKVE +dK_MMCCH-2 (K) | 2482 |
| 40 | 3007 | 3011 | 477.7571 | 953.4996 | 953.5004 | -0.84 | G | 0 | 13 | 0.045 | RALKE +dK_MMCCH-2 (K) | 3010 |
| 41 | 3386 | 3405 | 658.3147 | 2629.2297 | 2629.2271 | 0.99 | G | 2 | 30 | 0.00091 | LDHAYSLRDGHYYIAGPTKD +dK_MMCCH-2 (K) | 3404 |
| 42 | 277 | 296 | 883.7569 | 2648.2489 | 2648.2329 | 6.00 | C | 4 | 32 | 0.00056 | DLTREHAKPADSFDYGRLGY +dK_MMCCH-2 (K) | 284 |
| 43 | 364 | 373 | 743.3618 | 1484.7091 | 1484.7044 | 3.17 | C | 2 | 13 | 0.047 | LLGGPTEMKW +dK_MMCCH-2 (K); Oxidation (M) | 372 |
| 44 | 432 | 443 | 793.9156 | 1585.8166 | 1585.8174 | -0.50 | C | 0 | 18 | 0.018 | DKPPVPVAQANL +dK_MMCCH-2 (K) | 433 |
| 45 | 629 | 635 | 539.2512 | 1076.4878 | 1076.4848 | 2.69 | C | 0 | 13 | 0.048 | VGGSEKY +dK_MMCCH-2 (K) | 634 |
| 46 | 1075 | 1080 | 544.7763 | 1087.5381 | 1087.5372 | 0.83 | C | 1 | 22 | 0.0067 | QALQKY +dK_MMCCH-2 (K) | 1079 |
| 47 | 1212 | 1230 | 875.0863 | 2622.2371 | 2622.2424 | -2.06 | C | 4 | 14 | 0.04 | KYEITQQLHDLDLHVGDNF +dK_MMCCH-2 (K) | 1212 |
| 48 | 1416 | 1421 | 570.2397 | 1138.4648 | 1138.4641 | 0.61 | C | 1 | 17 | 0.018 | DNKDFY +dK_MMCCH-2 (K) | 1418 |
| 49 | 1589 | 1606 | 784.3536 | 2350.0391 | 2350.0392 | -0.04 | C | 0 | 20 | 0.01 | ICVEQGGEQNCKTKAGSF +dK_MMCCH-2 (K) | 1602 |
| 50 | 1696 | 1702 | 607.3007 | 1212.5869 | 1212.5849 | 1.65 | C | 1 | 21 | 0.0086 | TTLEKHF +dK_MMCCH-2 (K) | 1700 |
| 51 | 1708 | 1719 | 839.8414 | 1677.6683 | 1677.6651 | 1.91 | C | 0 | 22 | 0.007 | KNMQADDSPDGY +dK_MMCCH-2 (K) | 1708 |
| 52 | 1782 | 1796 | 1014.5062 | 2026.9978 | 2026.9921 | 2.76 | C | 1 | 25 | 0.0029 | KPQSALPDLVTQETY +dK_MMCCH-2 (K) | 1782 |

Figure 27B. KLH 2 (continued)

| | | | | | | | | | | | | |
|---|---|---|---|---|---|---|---|---|---|---|---|---|
| 53 | 1801 | 1809 | 706.8369 | 1411.6591 | 1411.6595 | -0.21 | C | 0 | 28 | 0.0017 | SHKTFPNPF +dK_MMCCH-2 (K) | 1803 |
| 54 | 1982 | 1995 | 705.3458 | 2113.0155 | 2113.0051 | 4.92 | C | 1 | 25 | 0.0031 | HNYIKQQQEADRVF +dK_MMCCH-2 (K) | 1986 |
| 55 | 1999 | 2003 | 458.2542 | 914.4939 | 914.4936 | 0.44 | C | 2 | 26 | 0.0027 | LLKGF +dK_MMCCH-2 (K) | 2001 |
| 56 | 2455 | 2462 | 645.3264 | 1288.6381 | 1288.6373 | 0.62 | C | 1 | 18 | 0.017 | KYDITQAL +dK_MMCCH-2 (K) | 2455 |
| 57 | 2463 | 2479 | 1128.0186 | 2254.0225 | 2254.0253 | -1.20 | C | 1 | 20 | 0.011 | KAQSIHPEDVFDTDAPF +dK_MMCCH-2 (K) | 2463 |
| 58 | 2754 | 2760 | 598.3323 | 1194.6501 | 1194.6471 | 2.51 | C | 1 | 23 | 0.0045 | KQPLKPF +dK_MMCCH-2 (K) | 2758 |
| 59 | 2865 | 2872 | 673.3627 | 1344.7108 | 1344.7112 | -0.30 | C | 1 | 18 | 0.014 | RYDITKVL +dK_MMCCH-2 (K) | 2870 |
| 60 | 3235 | 3242 | 617.3138 | 1232.6130 | 1232.6111 | 1.54 | C | 0 | 19 | 0.012 | TSANVKIY +dK_MMCCH-2 (K) | 3240 |
| 61 | 3399 | 3406 | 576.8050 | 1151.5954 | 1151.5896 | 5.04 | C | 0 | 17 | 0.022 | IAGPTKDL +dK_MMCCH-2 (K) | 3404 |
| 62 | 184 | 191 | 703.8036 | 1405.5926 | 1405.5894 | 2.28 | Th | 1 | 41 | 8.60E-05 | LEQDEFCK +dK_MMCCH-2 (K) | 191 |
| 63 | 247 | 260 | 631.6191 | 1891.8356 | 1891.8305 | 2.70 | Th | 2 | 15 | 0.033 | LRGKDPNSADCAHN +dK_MMCCH-2 (K) | 250 |
| 64 | 283 | 293 | 782.8574 | 1563.7002 | 1563.7028 | -1.66 | Th | 3 | 32 | 0.00071 | AKPADSFDYGR +dK_MMCCH-2 (K) | 284 |
| 65 | 365 | 372 | 585.7805 | 1169.5464 | 1169.5461 | 0.26 | Th | 1 | 40 | 9.60E-05 | LGGPTEMK +dK_MMCCH-2 (K) | 372 |
| 66 | 467 | 472 | 538.7411 | 1075.4677 | 1075.4644 | 3.07 | Th | 0 | 13 | 0.049 | FQNDKS +dK_MMCCH-2 (K) | 471 |
| 67 | 629 | 636 | 582.7668 | 1163.5191 | 1163.5169 | 1.98 | Th | 1 | 21 | 0.0074 | VGGSEKYS +dK_MMCCH-2 (K) | 634 |
| 68 | 893 | 904 | 904.8973 | 1807.7801 | 1807.7698 | 5.70 | Th | 1 | 18 | 0.018 | FHGEPKWCPSPE +dK_MMCCH-2 (K) | 898 |
| 69 | 1300 | 1308 | 716.3438 | 1430.6731 | 1430.6751 | -1.47 | Th | 2 | 14 | 0.044 | IQKEGIYEN +dK_MMCCH-2 (K) | 1302 |
| 70 | 1414 | 1419 | 564.7560 | 1127.4975 | 1127.4957 | 1.60 | Th | 1 | 21 | 0.0077 | LWDNKD +dK_MMCCH-2 (K) | 1418 |
| 71 | 1525 | 1539 | 738.3560 | 2212.0461 | 2212.0371 | 4.02 | Th | 3 | 40 | 9.70E-05 | LKHNLPQDSFDYQNR +dK_MMCCH-2 (K) | 1526 |
| 72 | 1589 | 1605 | 735.3304 | 2202.9695 | 2202.9708 | -0.59 | Th | 2 | 15 | 0.03 | ICVEQGGEQNCKTKAGS +dK_MMCCH-2 (K) | 1600 |
| 73 | 1589 | 1605 | 735.3321 | 2202.9744 | 2202.9708 | 1.68 | Th | 2 | 38 | 0.00016 | ICVEQGGEQNCKTKAGS +dK_MMCCH-2 (K) | 1602 |
| 74 | 1800 | 1809 | 780.3717 | 1558.7288 | 1558.7279 | 0.64 | Th | 2 | 16 | 0.026 | FSHKTFPNPF +dK_MMCCH-2 (K) | 1803 |
| 75 | 1952 | 1963 | 627.2838 | 1878.8294 | 1878.8247 | 2.50 | Th | 2 | 52 | 6.20E-06 | FSKPEDTFDYHR +dK_MMCCH-2 (K) | 1954 |
| 76 | 1985 | 1994 | 776.8953 | 1551.7760 | 1551.7715 | 2.90 | Th | 2 | 24 | 0.0038 | IKQQQEADRV +dK_MMCCH-2 (K) | 1986 |
| 77 | 2193 | 2199 | 667.3006 | 1332.5866 | 1332.5849 | 1.28 | Th | 2 | 27 | 0.002 | YWDWTKP +dK_MMCCH-2 (K) | 2198 |
| 78 | 2200 | 2206 | 562.3071 | 1122.5997 | 1122.5995 | 0.18 | Th | 2 | 19 | 0.014 | ISKLPDL +dK_MMCCH-2 (K) | 2202 |
| 79 | 2225 | 2229 | 462.2212 | 922.4278 | 922.4259 | 2.06 | Th | 2 | 22 | 0.0067 | FAKGY +dK_MMCCH-2 (K) | 2227 |

Figure 27B. KLH 2 (continued)

| | | | | | | | | | | | | |
|---|---|---|---|---|---|---|---|---|---|---|---|---|
| 80 | 2230 | 2237 | 632.7943 | 1263.5739 | 1263.5693 | 3.72 | Th | 2 | 18 | 0.016 | IKSEDAYT +dK_MMCCH-2 (K) | 2231 |
| 81 | 2359 | 2363 | 509.2319 | 1016.4492 | 1016.4460 | 3.25 | Th | 1 | 21 | 0.0082 | FTKMH +dK_MMCCH-2 (K); Oxidation (M) | 2361 |
| 82 | 2941 | 2950 | 720.8395 | 1439.6645 | 1439.6602 | 2.99 | Th | 3 | 46 | 2.80E-05 | LDEANDLKNA +dK_MMCCH-2 (K) | 2948 |
| 83 | 3009 | 3015 | 573.2736 | 1144.5326 | 1144.5335 | -0.87 | Th | 0 | 17 | 0.02 | LKEHGSH +dK_MMCCH-2 (K) | 3010 |
| 84 | 3399 | 3405 | 520.2613 | 1038.5080 | 1038.5056 | 2.31 | Th | 1 | 27 | 0.0022 | IAGPTKD +dK_MMCCH-2 (K) | 3404 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the lysine conjugation site for KLH1 or KLH2.

## Figure 28. Identification details of MMCCH-conjugated peptides for sample 3 (2nd LC-MS/MS)

### Figure 28A. KLH 1

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 124 | 141 | 589.3078 | 2353.2020 | 2353.2001 | 0.81 | T | 1 | 27 | 0.0018 | GFTDPPVKHHQSANLLVR +dK_MMCCH-2 (K) | 131 |
| 2 | 142 | 149 | 656.3417 | 1310.6689 | 1310.6653 | 2.82 | T | 1 | 26 | 0.0026 | KNINDLTR +dK_MMCCH-2 (K) | 142 |
| 3 | 157 | 166 | 822.8806 | 1643.7467 | 1643.7402 | 3.95 | T | 1 | 18 | 0.015 | EAFHKFQEDR +dK_MMCCH-2 (K) | 161 |
| 4 | 252 | 281 | 735.7524 | 3673.7255 | 3673.7008 | 6.72 | T | 1 | 20 | 0.011 | TYVDSHGASHTNPFHSSVIAFEENAPHTKR +dK_MMCCH-2 (K) | 280 |
| 5 | 460 | 477 | 781.3948 | 2341.1625 | 2341.1624 | 0.04 | T | 1 | 29 | 0.0012 | TSANVDIFIKTTDSVQHK +dK_MMCCH-2 (K) | 469 |
| 6 | 470 | 488 | 748.0457 | 2241.1153 | 2241.1100 | 2.36 | T | 1 | 41 | 7.70E-05 | TTDSVQHKAGTFAVLGGSK +dK_MMCCH-2 (K) | 477 |
| 7 | 553 | 560 | 663.8673 | 1325.7201 | 1325.7166 | 2.64 | T | 2 | 21 | 0.0076 | VKFDKVPR +dK_MMCCH-2 (K) | 554 |
| 8 | 553 | 560 | 442.9153 | 1325.7240 | 1325.7166 | 5.58 | T | 2 | 15 | 0.033 | VKFDKVPR +dK_MMCCH-2 (K) | 557 |
| 9 | 566 | 570 | 485.2460 | 968.4774 | 968.4749 | 2.48 | T | 1 | 15 | 0.029 | KNVDR +dK_MMCCH-2 (K) | 566 |
| 10 | 581 | 587 | 547.8371 | 1093.6596 | 1093.6569 | 2.47 | T | 1 | 29 | 0.0014 | KALALLK +dK_MMCCH-2 (K) | 581 |
| 11 | 588 | 606 | 781.0346 | 2340.0820 | 2340.0845 | -1.07 | T | 1 | 29 | 0.0014 | EDKSAGGFQQLGAFHGEPK +dK_MMCCH-2 (K) | 590 |
| 12 | 591 | 615 | 1004.4717 | 3010.3934 | 3010.3742 | 6.38 | T | 1 | 18 | 0.017 | SAGGFQQLGAFHGEPKWCPSPEASK +dK_MMCCH-2 (K) | 606 |
| 13 | 645 | 681 | 1544.3801 | 4630.1186 | 4630.1063 | 2.63 | T | 1 | 27 | 0.0021 | HGYDGALPYWDWTSPLNHLPELADHEKYVDPEDGVEK +dK_MMCCH-2 (K) | 671 |
| 14 | 682 | 699 | 620.2958 | 2477.1540 | 2477.1434 | 4.28 | T | 1 | 26 | 0.0027 | HNPWFDGHIDTVDKTTTR +dK_MMCCH-2 (K) | 695 |
| 15 | 700 | 719 | 887.7726 | 2660.2959 | 2660.2945 | 0.53 | T | 1 | 51 | 8.60E-06 | SVQNKLFEQPEFGHYTSIAK +dK_MMCCH-2 (K) | 704 |
| 16 | 790 | 804 | 669.3168 | 2004.9287 | 2004.9220 | 3.34 | T | 0 | 35 | 0.0003 | GKPYNVANCAVTSMR +dK_MMCCH-2 (K) | 791 |
| 17 | 805 | 835 | 777.1814 | 3880.8706 | 3880.8771 | -1.67 | T | 1 | 14 | 0.044 | EPLQPFGLSANINTDHVTKEHSVPFNVFDYK +dK_MMCCH-2 (K) | 823 |
| 18 | 858 | 863 | 520.2996 | 1038.5846 | 1038.5783 | 5.97 | T | 1 | 13 | 0.046 | KLEAIK +dK_MMCCH-2 (K) | 858 |
| 19 | 879 | 884 | 500.2816 | 998.5487 | 998.5470 | 1.60 | T | 1 | 14 | 0.042 | KSSLVK +dK_MMCCH-2 (K) | 879 |
| 20 | 953 | 965 | 635.3298 | 1902.9677 | 1902.9662 | 0.79 | T | 1 | 42 | 6.50E-05 | DLFKQPSVIHEPR +dK_MMCCH-2 (K) | 956 |
| 21 | 986 | 1000 | 1027.0387 | 2052.0628 | 2052.0561 | 3.31 | T | 1 | 77 | 2.20E-08 | KNIENLSLGELESLR +dK_MMCCH-2 (K) | 986 |
| 22 | 1048 | 1060 | 609.3545 | 1825.0416 | 1825.0423 | -0.38 | T | 1 | 36 | 0.00025 | LYVVVVENALLKK +dK_MMCCH-2 (K) | 1060 |
| 23 | 1092 | 1114 | 791.8781 | 3163.4831 | 3163.4682 | 4.71 | T | 1 | 34 | 0.00041 | QHHYETNPFHHGKITHENEITTR +dK_MMCCH-2 (K) | 1104 |
| 24 | 1199 | 1203 | 353.1910 | 1056.5512 | 1056.5538 | -2.46 | T | 1 | 17 | 0.019 | KRPYR +dK_MMCCH-2 (K) | 1199 |

Figure 28A. KLH 1 (continued)

| | | | | | | | | | | | | |
|---|---|---|---|---|---|---|---|---|---|---|---|---|
| 25 | 1331 | 1338 | 620.3541 | 1238.6937 | 1238.6944 | -0.57 | T | 1 | 18 | 0.014 | LDITKALK +dK_MMCCH-2 (K) | 1335 |
| 26 | 1395 | 1403 | 598.3055 | 1791.8948 | 1791.8899 | 2.73 | T | 2 | 17 | 0.021 | KDITQLDKR +2 dK_MMCCH-2 (K) | 1395, 1402 |
| 27 | 1395 | 1403 | 485.5956 | 1453.7648 | 1453.7599 | 3.37 | T | 2 | 17 | 0.019 | KDITQLDKR +dK_MMCCH-2 (K) | 1402 |
| 28 | 1417 | 1445 | 1125.2156 | 3372.6249 | 3372.6020 | 6.82 | T | 1 | 30 | 0.00092 | ADHSSDGFQAIASFHALPPLCPSPAASKR +dK_MMCCH-2 (K) | 1444 |
| 29 | 1473 | 1489 | 778.4014 | 2332.1823 | 2332.1827 | -0.17 | T | 1 | 42 | 6.00E-05 | KHGAVVGLPYWDWTLPR +dK_MMCCH-2 (K) | 1473 |
| 30 | 1721 | 1746 | 1087.4750 | 3259.4031 | 3259.3937 | 2.88 | T | 1 | 34 | 0.00042 | TAGDCEDAGYFTVLGGEKEMPWAFDR +dK_MMCCH-2 (K) | 1738 |
| 31 | 1890 | 1922 | 879.8322 | 4394.1247 | 4394.1048 | 4.53 | T | 1 | 14 | 0.043 | HGSSVAVPYWDWTKPIHNIPHLFTDKEYYDVWR +dK_MMCCH-2 (K) | 1915 |
| 32 | 1923 | 1932 | 756.3802 | 1510.7458 | 1510.7425 | 2.25 | T | 1 | 25 | 0.0032 | NKVMPNPFAR +dK_MMCCH-2 (K) | 1924 |
| 33 | 2067 | 2076 | 746.8856 | 1491.7567 | 1491.7544 | 1.54 | T | 1 | 25 | 0.0032 | KHAVPNDVFK +dK_MMCCH-2 (K) | 2067 |
| 34 | 2068 | 2083 | 753.7166 | 2258.1280 | 2258.1194 | 3.81 | T | 1 | 14 | 0.041 | HAVPNDVFKYELLGYR +dK_MMCCH-2 (K) | 2076 |
| 35 | 2100 | 2109 | 492.2479 | 1964.9626 | 1964.9601 | 1.27 | T | 2 | 22 | 0.0068 | EIKDKQHHVR +2 dK_MMCCH-2 (K) | 2102, 2104 |
| 36 | 2121 | 2149 | 890.4148 | 3557.6301 | 3557.6378 | -2.19 | T | 1 | 34 | 0.00036 | TSADVQFQICKTSEDCHHGGQIFVLGGTK +dK_MMCCH-2 (K) | 2131 |
| 37 | 2359 | 2367 | 736.3485 | 1470.6825 | 1470.6813 | 0.82 | T | 1 | 32 | 0.00068 | DKLFNDPER +dK_MMCCH-2 (K) | 2360 |
| 38 | 2461 | 2490 | 642.8189 | 3850.8694 | 3850.8638 | 1.45 | T | 0 | 16 | 0.024 | RPLRPFSDPINHNAFTHSNAKPTDVFEYSR +dK_MMCCH-2 (K) | 2481 |
| 39 | 2507 | 2514 | 682.3526 | 1362.6906 | 1362.6853 | 3.89 | T | 1 | 33 | 0.00049 | KLEHELEK +dK_MMCCH-2 (K) | 2507 |
| 40 | 2508 | 2516 | 746.3801 | 1490.7457 | 1490.7439 | 1.21 | T | 1 | 21 | 0.0082 | LEHELEKQK +dK_MMCCH-2 (K) | 2516 |
| 41 | 2595 | 2609 | 983.5291 | 1965.0437 | 1965.0493 | -2.85 | T | 1 | 18 | 0.018 | IIDTSGKQLPSDLIK +dK_MMCCH-2 (K) | 2601 |
| 42 | 2621 | 2636 | 802.7109 | 2405.1110 | 2405.1083 | 1.12 | T | 1 | 48 | 1.80E-05 | HHEKHHEDHHEDILVR +dK_MMCCH-2 (K) | 2624 |
| 43 | 2637 | 2651 | 713.3541 | 2137.0406 | 2137.0374 | 1.45 | T | 1 | 61 | 7.40E-07 | KNIHSLSHHEAEELR +dK_MMCCH-2 (K) | 2637 |
| 44 | 2657 | 2686 | 1246.2177 | 3735.6311 | 3735.6358 | -1.26 | T | 1 | 16 | 0.028 | LQNDESHGGYEHIAGFHGYPNLCPEKGDEK +dK_MMCCH-2 (K) | 2686 |
| 45 | 2714 | 2748 | 1087.7693 | 4347.0481 | 4347.0524 | -1.01 | T | 1 | 47 | 1.80E-05 | KHGSHLGIPYWDWTQTISSLPTFFADSGNNNPFFK +dK_MMCCH-2 (K) | 2714 |
| 46 | 2851 | 2883 | 1009.2180 | 4032.8430 | 4032.8346 | 2.08 | T | 0 | 74 | 4.20E-08 | VKPAHAGSCAGDIMHVPLHPFNYESVNNDDFTR +dK_MMCCH-2 (K) | 2852 |
| 47 | 2897 | 2919 | 1066.5209 | 3196.5408 | 3196.5287 | 3.79 | T | 1 | 50 | 1.00E-05 | FNYKYDNLNLHGHNIEELEEVLR +dK_MMCCH-2 (K) | 2900 |
| 48 | 2938 | 2947 | 730.4150 | 1458.8154 | 1458.8156 | -0.14 | T | 1 | 22 | 0.0064 | TTAVVKVYIK +dK_MMCCH-2 (K) | 2943 |
| 49 | 3003 | 3021 | 825.7742 | 2474.3007 | 2474.2879 | 5.17 | T | 1 | 54 | 3.90E-06 | KYDHTELDASVLPAPIIVR +dK_MMCCH-2 (K) | 3003 |
| 50 | 3022 | 3044 | 966.5219 | 2896.5439 | 2896.5521 | -2.83 | T | 1 | 16 | 0.027 | RPNNAVFDIIEIPIGKDVNLPPK +dK_MMCCH-2 (K) | 3037 |
| 51 | 123 | 151 | 616.6577 | 3693.9026 | 3693.9049 | -0.62 | G | 2 | 16 | 0.027 | KGFTDPPVKHHQSANLLVRKNINDLTREE +dK_MMCCH-2 (K) | 123 |

Figure 28A. KLH 1 (continued)

| | | | | | | | | | | | | |
|---|---|---|---|---|---|---|---|---|---|---|---|---|
| 52 | 123 | 151 | 739.7898 | 3693.9126 | 3693.9049 | 2.06 | G | 2 | 20 | 0.01 | KGFTDPPVKHHQSANLLVRKNINDLTREE +dK_MMCCH-2 (K) | 131 |
| 53 | 158 | 176 | 846.3861 | 2536.1365 | 2536.1329 | 1.42 | G | 3 | 23 | 0.0055 | AFHKFQEDRSVDGYQATAE +dK_MMCCH-2 (K) | 161 |
| 54 | 526 | 544 | 777.7485 | 2330.2238 | 2330.2304 | -2.83 | G | 1 | 15 | 0.032 | VDGTKLASSLIPHASVIRE +dK_MMCCH-2 (K) | 530 |
| 55 | 589 | 612 | 990.1155 | 2967.3248 | 2967.3320 | -2.43 | G | 1 | 15 | 0.03 | DKSAGGFQQLGAFHGEPKWCPSPE +dK_MMCCH-2 (K) | 606 |
| 56 | 825 | 841 | 820.3744 | 2458.1013 | 2458.0940 | 2.97 | G | 1 | 22 | 0.0059 | HSVPFNVFDYKTNFNYE +dK_MMCCH-2 (K) | 835 |
| 57 | 942 | 972 | 955.4959 | 3817.9545 | 3817.9614 | -1.81 | G | 3 | 20 | 0.011 | VFDLKPASLGKDLFKQPSVIHEPRIGHHEGE +dK_MMCCH-2 (K) | 946 |
| 58 | 942 | 970 | 727.3887 | 3631.9073 | 3631.8973 | 2.73 | G | 2 | 19 | 0.014 | VFDLKPASLGKDLFKQPSVIHEPRIGHHE +dK_MMCCH-2 (K) | 956 |
| 59 | 1055 | 1076 | 967.1804 | 2898.5194 | 2898.5102 | 3.17 | G | 1 | 24 | 0.0039 | NALLKKGSSVAVPYWDWTKRIE +dK_MMCCH-2 (K) | 1060 |
| 60 | 1397 | 1413 | 774.4367 | 2320.2883 | 2320.2824 | 2.54 | G | 1 | 21 | 0.0074 | ITQLDKRQQLSLVKALE +dK_MMCCH-2 (K) | 1410 |
| 61 | 1506 | 1522 | 753.3771 | 2257.1094 | 2257.1089 | 0.22 | G | 2 | 20 | 0.009 | TGRDIPNPFIGSKIEFE +dK_MMCCH-2 (K) | 1518 |
| 62 | 1755 | 1765 | 570.6043 | 1708.7911 | 1708.7913 | -0.12 | G | 2 | 37 | 0.00022 | TLDKMNLRHDE +dK_MMCCH-2 (K) | 1758 |
| 63 | 2647 | 2661 | 715.6772 | 2144.0097 | 2144.0095 | 0.09 | G | 4 | 29 | 0.0012 | AEELRDALYKLQNDE +dK_MMCCH-2 (K) | 2656 |
| 64 | 59 | 68 | 729.3455 | 1456.6764 | 1456.6731 | 2.27 | C | 1 | 25 | 0.003 | VLGGPSEMKW +dK_MMCCH-2 (K); Oxidation (M) | 67 |
| 65 | 94 | 100 | 573.2996 | 1144.5846 | 1144.5838 | 0.61 | C | 1 | 23 | 0.0048 | TVKAELF +dK_MMCCH-2 (K) | 96 |
| 66 | 160 | 171 | 606.9457 | 1817.8152 | 1817.8043 | 6.00 | C | 1 | 15 | 0.035 | HKFQEDRSVDGY +dK_MMCCH-2 (K) | 161 |
| 67 | 429 | 450 | 990.8041 | 2969.3906 | 2969.3899 | 0.24 | C | 1 | 41 | 7.40E-05 | GGISLENIEKMIHENQQEDRIY +dK_MMCCH-2 (K); Oxidation (M) | 438 |
| 68 | 584 | 595 | 787.4019 | 1572.7892 | 1572.7858 | 2.16 | C | 2 | 37 | 0.0002 | ALLKEDKSAGGF +dK_MMCCH-2 (K) | 590 |
| 69 | 715 | 723 | 655.8740 | 1309.7334 | 1309.7316 | 1.37 | C | 1 | 20 | 0.0092 | TSIAKQVLL +dK_MMCCH-2 (K) | 719 |
| 70 | 783 | 788 | 544.7759 | 1087.5373 | 1087.5372 | 0.09 | C | 1 | 30 | 0.0011 | QALQKY +dK_MMCCH-2 (K) | 787 |
| 71 | 830 | 838 | 743.3391 | 1484.6635 | 1484.6646 | -0.74 | C | 2 | 27 | 0.002 | NVFDYKTNF +dK_MMCCH-2 (K) | 835 |
| 72 | 918 | 928 | 856.9315 | 1711.8484 | 1711.8491 | -0.41 | C | 2 | 42 | 7.10E-05 | KYDITEKLHDL +dK_MMCCH-2 (K) | 924 |
| 73 | 944 | 955 | 821.4331 | 1640.8517 | 1640.8484 | 2.01 | C | 3 | 24 | 0.0036 | DLKPASLGKDLF +dK_MMCCH-2 (K) | 946 |
| 74 | 1013 | 1018 | 516.7581 | 1031.5017 | 1031.4998 | 1.84 | C | 0 | 14 | 0.038 | ESIAKF +dK_MMCCH-2 (K) | 1017 |
| 75 | 1330 | 1337 | 620.3556 | 1238.6966 | 1238.6944 | 1.78 | C | 1 | 21 | 0.0073 | KLDITKAL +dK_MMCCH-2 (K) | 1335 |
| 76 | 1649 | 1664 | 1127.9868 | 2253.9591 | 2253.9637 | -2.04 | C | 2 | 24 | 0.0037 | NLNDHTHDFSKPEDTF +dK_MMCCH-2 (K) | 1659 |
| 77 | 1649 | 1669 | 979.4308 | 2935.2705 | 2935.2759 | -1.84 | C | 4 | 15 | 0.029 | NLNDHTHDFSKPEDTFDYQKF +dK_MMCCH-2 (K) | 1668 |
| 78 | 1732 | 1742 | 792.8743 | 1583.7340 | 1583.7364 | -1.52 | C | 1 | 14 | 0.039 | TVLGGEKEMPW +dK_MMCCH-2 (K) | 1738 |

**Figure 28A. KLH 1 (continued)**

| | | | | | | | | | | | | |
|---|---|---|---|---|---|---|---|---|---|---|---|---|
| 79 | 1818 | 1827 | 753.3699 | 1504.7253 | 1504.7232 | 1.40 | C | 1 | 22 | 0.0067 | SERDIGSLKY +dK_MMCCH-2 (K) | 1826 |
| 80 | 1913 | 1921 | 778.8339 | 1555.6532 | 1555.6541 | -0.58 | C | 2 | 13 | 0.048 | TDKEYYDVW +dK_MMCCH-2 (K) | 1915 |
| 81 | 2476 | 2488 | 923.9222 | 1845.8299 | 1845.8244 | 3.03 | C | 1 | 25 | 0.0032 | THSNAKPTDVFEY +dK_MMCCH-2 (K) | 2481 |
| 82 | 2656 | 2666 | 793.3528 | 1584.6910 | 1584.6879 | 2.02 | C | 1 | 23 | 0.0052 | KLQNDESHGGY +dK_MMCCH-2 (K) | 2656 |
| 83 | 2713 | 2723 | 787.9119 | 1573.8093 | 1573.8075 | 1.14 | C | 1 | 17 | 0.021 | KKHGSHLGIPY +dK_MMCCH-2 (K) | 2714 |
| 84 | 2809 | 2815 | 538.2600 | 1074.5055 | 1074.5056 | -0.09 | C | 0 | 31 | 0.00079 | IGGAEKY +dK_MMCCH-2 (K) | 2814 |
| 85 | 2946 | 2960 | 965.4038 | 1928.7931 | 1928.7986 | -2.85 | C | 1 | 27 | 0.0021 | IKSGTDSDDEYAGSF +dK_MMCCH-2 (K) | 2947 |
| 86 | 2961 | 2971 | 769.8921 | 1537.7696 | 1537.7673 | 1.50 | C | 1 | 13 | 0.048 | VILGGAKEMPW +dK_MMCCH-2 (K) | 2967 |
| 87 | 1 | 9 | 733.3350 | 1464.6554 | 1464.6516 | 2.53 | Th | 2 | 25 | 0.003 | MTPEELKTY +dK_MMCCH-2 (K); Oxidation (M) | 7 |
| 88 | 38 | 56 | 861.6832 | 2582.0279 | 2582.0108 | 6.58 | Th | 2 | 25 | 0.0029 | VCIPDDNDRNDDHCEKAGD +dK_MMCCH-2 (K) | 53 |
| 89 | 59 | 69 | 785.3786 | 1568.7426 | 1568.7367 | 3.76 | Th | 3 | 54 | 4.20E-06 | VLGGPSEMKWQ +dK_MMCCH-2 (K) | 67 |
| 90 | 78 | 84 | 569.2875 | 1136.5604 | 1136.5536 | 5.98 | Th | 1 | 45 | 3.20E-05 | LSDTVHK +dK_MMCCH-2 (K) | 84 |
| 91 | 118 | 124 | 571.2790 | 1140.5435 | 1140.5386 | 4.30 | Th | 0 | 24 | 0.0042 | VHHPEKG +dK_MMCCH-2 (K) | 123 |
| 92 | 125 | 135 | 815.8901 | 1629.7656 | 1629.7610 | 2.82 | Th | 1 | 17 | 0.02 | FTDPPVKHHQS +dK_MMCCH-2 (K) | 131 |
| 93 | 272 | 282 | 848.3909 | 1694.7673 | 1694.7722 | -2.89 | Th | 1 | 22 | 0.0065 | FEENAPHTKRQ +Deamidated (NQ); dK_MMCCH-2 (K) | 280 |
| 94 | 431 | 439 | 707.8525 | 1413.6904 | 1413.6883 | 1.49 | Th | 3 | 40 | 0.00011 | ISLENIEKM +dK_MMCCH-2 (K) | 438 |
| 95 | 521 | 530 | 707.8452 | 1413.6759 | 1413.6698 | 4.32 | Th | 2 | 69 | 1.20E-07 | VDITEVDGTK +dK_MMCCH-2 (K) | 530 |
| 96 | 585 | 591 | 585.8106 | 1169.6065 | 1169.6002 | 5.47 | Th | 1 | 35 | 0.00032 | LLKEDKS +dK_MMCCH-2 (K) | 590 |
| 97 | 601 | 612 | 904.8967 | 1807.7789 | 1807.7698 | 5.03 | Th | 1 | 22 | 0.0064 | FHGEPKWCPSPE +dK_MMCCH-2 (K) | 606 |
| 98 | 666 | 672 | 607.2830 | 1212.5514 | 1212.5485 | 2.39 | Th | 2 | 23 | 0.0047 | LADHEKY +dK_MMCCH-2 (K) | 671 |
| 99 | 821 | 828 | 617.8124 | 1233.6102 | 1233.6064 | 3.08 | Th | 1 | 22 | 0.0063 | VTKEHSVP +dK_MMCCH-2 (K) | 823 |
| 100 | 832 | 837 | 563.2518 | 1124.4891 | 1124.4848 | 3.82 | Th | 1 | 29 | 0.0014 | FDYKTN +dK_MMCCH-2 (K) | 835 |
| 101 | 954 | 959 | 529.7658 | 1057.5169 | 1057.5154 | 1.42 | Th | 1 | 14 | 0.042 | LFKQPS +Deamidated (NQ); dK_MMCCH-2 (K) | 956 |
| 102 | 1111 | 1120 | 742.3787 | 1482.7429 | 1482.7388 | 2.77 | Th | 1 | 18 | 0.017 | ITTRDPKDSL +dK_MMCCH-2 (K) | 1117 |
| 103 | 1156 | 1164 | 630.8383 | 1259.6621 | 1259.6584 | 2.94 | Th | 2 | 19 | 0.013 | LLGGKGKYS +dK_MMCCH-2 (K) | 1162 |
| 104 | 1219 | 1232 | 653.9537 | 1958.8392 | 1958.8316 | 3.88 | Th | 1 | 25 | 0.0032 | FDKSDNNDEATKTH +dK_MMCCH-2 (K) | 1230 |
| 105 | 1579 | 1587 | 665.8126 | 1329.6106 | 1329.6064 | 3.16 | Th | 2 | 20 | 0.011 | WVGGKEPYG +dK_MMCCH-2 (K) | 1583 |

Figure 28A. KLH 1 (continued)

| | | | | | | | | | | | | |
|---|---|---|---|---|---|---|---|---|---|---|---|---|
| 106 | 1657 | 1668 | 921.9197 | 1841.8249 | 1841.8182 | 3.64 | Th | 2 | 32 | 0.00058 | FSKPEDTFDYQK +dK_MMCCH-2 (K) | 1659 |
| 107 | 1657 | 1668 | 921.9213 | 1841.8280 | 1841.8182 | 5.32 | Th | 2 | 44 | 4.20E-05 | FSKPEDTFDYQK +dK_MMCCH-2 (K) | 1668 |
| 108 | 1733 | 1741 | 664.3256 | 1326.6367 | 1326.6312 | 4.15 | Th | 2 | 14 | 0.04 | VLGGEKEMP +dK_MMCCH-1 (K); Oxidation (M) | 1738 |
| 109 | 1894 | 1904 | 850.4187 | 1698.8228 | 1698.8116 | 6.59 | Th | 5 | 34 | 0.00044 | VAVPYWDWTKP +dK_MMCCH-2 (K) | 1903 |
| 110 | 2064 | 2068 | 499.7599 | 997.5052 | 997.5055 | -0.40 | Th | 0 | 19 | 0.014 | FTKKH +dK_MMCCH-2 (K) | 2067 |
| 111 | 2094 | 2100 | 618.3057 | 1234.5968 | 1234.5903 | 5.18 | Th | 1 | 26 | 0.0023 | LHEIEKE +dK_MMCCH-2 (K) | 2099 |
| 112 | 2129 | 2141 | 622.9242 | 1865.7507 | 1865.7495 | 0.64 | Th | 0 | 26 | 0.0026 | ICKTSEDCHHGGQ +dK_MMCCH-2 (K) | 2131 |
| 113 | 2238 | 2249 | 814.4017 | 1626.7888 | 1626.7811 | 4.73 | Th | 4 | 38 | 0.00015 | LTTAEVDNLKDA +dK_MMCCH-2 (K) | 2247 |
| 114 | 2480 | 2487 | 622.7999 | 1243.5852 | 1243.5795 | 4.58 | Th | 2 | 17 | 0.019 | AKPTDVFE +dK_MMCCH-2 (K) | 2481 |
| 115 | 2512 | 2521 | 538.6006 | 1612.7799 | 1612.7766 | 2.05 | Th | 0 | 41 | 8.60E-05 | LEKQKEEDRT +dK_MMCCH-2 (K) | 2514 |
| 116 | 2595 | 2606 | 806.4045 | 1610.7945 | 1610.7862 | 5.15 | Th | 2 | 26 | 0.0026 | IIDTSGKQLPSD +dK_MMCCH-2 (K) | 2601 |
| 117 | 2607 | 2612 | 520.7819 | 1039.5493 | 1039.5446 | 4.52 | Th | 2 | 25 | 0.0035 | LIKMPT +dK_MMCCH-2 (K) | 2609 |
| 118 | 2766 | 2771 | 551.7871 | 1101.5597 | 1101.5529 | 6.17 | Th | 1 | 25 | 0.0029 | IFQQTK +dK_MMCCH-2 (K) | 2771 |
| 119 | 2809 | 2816 | 581.7781 | 1161.5416 | 1161.5376 | 3.44 | Th | 2 | 13 | 0.045 | IGGAEKYS +dK_MMCCH-2 (K) | 2814 |
| 120 | 2946 | 2960 | 965.4075 | 1928.8004 | 1928.7986 | 0.93 | Th | 3 | 68 | 1.40E-07 | IKSGTDSDDEYAGSF +dK_MMCCH-2 (K) | 2947 |
| 121 | 2990 | 2996 | 583.2851 | 1164.5556 | 1164.5485 | 6.10 | Th | 1 | 33 | 0.00047 | LTDDHVK +dK_MMCCH-2 (K) | 2996 |

Figure 28B. KLH 2

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1043.0237 | 2084.0328 | 2084.0347 | -0.91 | T | 1 | 54 | 4.20E-06 | KNVDSLSSDEVLALEK +dK_MMCCH-2 (K) | 26 |
| 2 | 147 | 155 | 694.3700 | 1386.7254 | 1386.7217 | 2.67 | T | 1 | 22 | 0.007 | ADITFLNKK +dK_MMCCH-2 (K) | 155 |
| 3 | 164 | 175 | 596.3071 | 1785.8996 | 1785.8985 | 0.62 | T | 1 | 36 | 0.00024 | LFEKVQPGHHTR +dK_MMCCH-2 (K) | 167 |
| 4 | 249 | 271 | 987.4432 | 2959.3077 | 2959.3051 | 0.88 | T | 1 | 28 | 0.0018 | GKDPNSADCAHNLIHTPMEPFDR +dK_MMCCH-2 (K) | 250 |
| 5 | 281 | 293 | 610.9432 | 1829.8079 | 1829.8043 | 1.97 | T | 0 | 55 | 3.30E-06 | EHAKPADSFDYGR +dK_MMCCH-2 (K) | 284 |
| 6 | 359 | 377 | 849.3962 | 2545.1669 | 2545.1810 | -5.58 | T | 1 | 14 | 0.041 | AGDFFLLGGPTEMKWGFYR +dK_MMCCH-2 (K); Oxidation (M) | 372 |
| 7 | 419 | 446 | 677.3699 | 3381.8130 | 3381.8132 | -0.06 | T | 1 | 45 | 3.20E-05 | QPTLVHRPAKGHFDKPPVPVAQANLAVR +Deamidated (NQ); dK_MMCCH-2 (K) | 428 |
| 8 | 429 | 446 | 752.0659 | 2253.1759 | 2253.1729 | 1.38 | T | 0 | 50 | 1.10E-05 | GHFDKPPVPVAQANLAVR +dK_MMCCH-2 (K) | 433 |
| 9 | 467 | 488 | 944.4543 | 2830.3410 | 2830.3385 | 0.88 | T | 1 | 70 | 9.50E-08 | FQNDKSVDGYQATVEFHALPAR +dK_MMCCH-2 (K) | 471 |
| 10 | 908 | 924 | 620.2817 | 2477.0979 | 2477.1166 | -7.59 | T | 1 | 18 | 0.017 | KFACCVHGMAVFPHWHR +dK_MMCCH-2 (K) | 908 |
| 11 | 936 | 950 | 1080.0133 | 2158.0121 | 2158.0095 | 1.20 | T | 1 | 40 | 0.0001 | KHGFTGGLPYWDWTR +dK_MMCCH-2 (K) | 936 |
| 12 | 1082 | 1096 | 996.4623 | 1990.9100 | 1990.9063 | 1.86 | T | 0 | 14 | 0.041 | GKPYNTANCAIASMR +dK_MMCCH-2 (K) | 1083 |
| 13 | 1097 | 1115 | 827.0796 | 2478.2169 | 2478.2101 | 2.78 | T | 0 | 81 | 8.40E-09 | KPLQPFGLDSVINPDDETR +dK_MMCCH-2 (K) | 1097 |
| 14 | 1123 | 1149 | 1190.5598 | 3568.6576 | 3568.6497 | 2.21 | T | 1 | 67 | 1.80E-07 | VFDYKNNFDYEYESLAFNGLSIAQLDR +dK_MMCCH-2 (K) | 1127 |
| 15 | 1295 | 1311 | 764.0689 | 2289.1849 | 2289.1715 | 5.85 | T | 1 | 17 | 0.022 | SAFLQIQKEGIYENIAK +dK_MMCCH-2 (K) | 1302 |
| 16 | 1355 | 1385 | 953.7057 | 3810.7937 | 3810.7876 | 1.60 | T | 1 | 61 | 8.90E-07 | GSAVAVPYWDWTEKADSLPSLINDATYFNSR +dK_MMCCH-2 (K) | 1368 |
| 17 | 1409 | 1450 | 1371.8844 | 5483.5085 | 5483.5213 | -2.33 | T | 1 | 33 | 0.00047 | DPQPELWDNKDFYENVMLALEQDNFCDFEIQLELIHNALHSR +dK_MMCCH-2 (K) | 1418 |
| 18 | 1455 | 1480 | 1117.8724 | 3350.5955 | 3350.6070 | -3.43 | T | 1 | 58 | 1.50E-06 | AKYSLSSLDYTAFDPVFFLHHANVDR +dK_MMCCH-2 (K) | 1456 |
| 19 | 1493 | 1507 | 721.6637 | 2161.9693 | 2161.9594 | 4.53 | T | 1 | 58 | 1.50E-06 | KKPYNEADCAVNEMR +dK_MMCCH-2 (K) | 1494 |
| 20 | 1508 | 1539 | 825.9995 | 4124.9609 | 4124.9360 | 6.04 | T | 1 | 25 | 0.0033 | KPLQPFNNPELNSDSMTLKHNLPQDSFDYQNR +dK_MMCCH-2 (K) | 1526 |
| 21 | 1542 | 1564 | 1070.1924 | 3207.5553 | 3207.5448 | 3.30 | T | 1 | 18 | 0.018 | YQYDNLQFNHFSIQKLDQTIQAR +dK_MMCCH-2 (K) | 1556 |
| 22 | 1625 | 1646 | 906.8055 | 2717.3946 | 2717.3887 | 2.17 | T | 1 | 22 | 0.0062 | FDITSALHKLGVPLDGHGFDIK +dK_MMCCH-2 (K) | 1633 |
| 23 | 1757 | 1768 | 612.9796 | 1835.9168 | 1835.9128 | 2.23 | T | 1 | 18 | 0.015 | LYTVQFEDSLKR +dK_MMCCH-2 (K) | 1767 |
| 24 | 1949 | 1963 | 560.2521 | 2236.9792 | 2236.9735 | 2.55 | T | 0 | 52 | 6.90E-06 | TQEFSKPEDTFDYHR +dK_MMCCH-2 (K) | 1954 |

Figure 28B. KLH 2 (continued)

| | | | | | | | | | | | | |
|---|---|---|---|---|---|---|---|---|---|---|---|---|
| 25 | 2045 | 2052 | 660.3497 | 1318.6848 | 1318.6843 | 0.38 | T | 1 | 21 | 0.0073 | YDITKTLK +dK_MMCCH-2 (K) | 2049 |
| 26 | 2058 | 2069 | 909.4633 | 1816.9121 | 1816.9070 | 2.81 | T | 1 | 14 | 0.036 | YDDTFTIKVHIK +dK_MMCCH-2 (K) | 2065 |
| 27 | 2228 | 2239 | 870.4176 | 1738.8206 | 1738.8236 | -1.73 | T | 1 | 14 | 0.036 | GYIKSEDAYTVR +dK_MMCCH-2 (K) | 2231 |
| 28 | 2527 | 2541 | 1007.5128 | 2013.0110 | 2013.0088 | 1.04 | T | 1 | 56 | 2.30E-06 | KDVTSLTASEIENLR +dK_MMCCH-2 (K) | 2527 |
| 29 | 2755 | 2764 | 764.3820 | 1526.7494 | 1526.7439 | 3.60 | T | 0 | 19 | 0.012 | QPLKPFSESR +Deamidated (NQ); dK_MMCCH-2 (K) | 2758 |
| 30 | 2866 | 2873 | 659.3597 | 1316.7048 | 1316.7050 | -0.15 | T | 1 | 13 | 0.046 | YDITKVLK +dK_MMCCH-2 (K) | 2870 |
| 31 | 3148 | 3155 | 629.3353 | 1256.6561 | 1256.6587 | -2.07 | T | 1 | 17 | 0.02 | KKPYNAAK +dK_MMCCH-2 (K) | 3149 |
| 32 | 466 | 481 | 1098.4951 | 2194.9757 | 2194.9841 | -3.83 | G | 2 | 25 | 0.0035 | RFQNDKSVDGYQATVE +Deamidated (NQ); dK_MMCCH-2 (K) | 471 |
| 33 | 1215 | 1235 | 962.1380 | 2883.3922 | 2883.3789 | 4.58 | G | 3 | 15 | 0.033 | ITQQLHDLDLHVGDNFFLKYE +Deamidated (NQ); dK_MMCCH-2 (K) | 1233 |
| 34 | 2477 | 2484 | 644.8370 | 1287.6594 | 1287.6573 | 1.63 | G | 0 | 15 | 0.031 | APFFIKVE +dK_MMCCH-2 (K) | 2482 |
| 35 | 1292 | 1303 | 586.6456 | 1756.9151 | 1756.9182 | -1.76 | G | 0 | 21 | 0.008 | SIRSAFLQIQKE +dK_MMCCH-2 (K) | 1302 |
| 36 | 1292 | 1303 | 879.4651 | 1756.9156 | 1756.9182 | -1.42 | G | 0 | 39 | 0.00011 | SIRSAFLQIQKE +dK_MMCCH-2 (K) | 1302 |
| 37 | 1292 | 1303 | 879.4658 | 1756.9170 | 1756.9182 | -0.68 | G | 0 | 39 | 0.00012 | SIRSAFLQIQKE +dK_MMCCH-2 (K) | 1302 |
| 38 | 1292 | 1303 | 879.4702 | 1756.9259 | 1756.9182 | 4.38 | G | 0 | 26 | 0.0026 | SIRSAFLQIQKE +dK_MMCCH-2 (K) | 1302 |
| 39 | 1308 | 1320 | 603.6327 | 1807.8762 | 1807.8749 | 0.72 | G | 0 | 16 | 0.023 | NIAKFHGKPGLCE +dK_MMCCH-2 (K) | 1315 |
| 40 | 1290 | 1303 | 667.3554 | 1999.0444 | 1999.0448 | -0.20 | G | 1 | 17 | 0.02 | IESIRSAFLQIQKE +dK_MMCCH-2 (K) | 1302 |
| 41 | 1290 | 1303 | 1000.5361 | 1999.0576 | 1999.0448 | 6.40 | G | 1 | 45 | 3.00E-05 | IESIRSAFLQIQKE +dK_MMCCH-2 (K) | 1302 |
| 42 | 466 | 481 | 732.3397 | 2193.9973 | 2194.0001 | -1.28 | G | 2 | 15 | 0.032 | RFQNDKSVDGYQATVE +dK_MMCCH-2 (K) | 471 |
| 43 | 466 | 481 | 1098.0066 | 2193.9986 | 2194.0001 | -0.68 | G | 2 | 16 | 0.026 | RFQNDKSVDGYQATVE +dK_MMCCH-2 (K) | 471 |
| 44 | 466 | 481 | 732.3409 | 2194.0008 | 2194.0001 | 0.32 | G | 2 | 16 | 0.026 | RFQNDKSVDGYQATVE +dK_MMCCH-2 (K) | 471 |
| 45 | 1952 | 1967 | 594.7626 | 2375.0212 | 2375.0205 | 0.29 | G | 3 | 18 | 0.017 | FSKPEDTFDYHRFGYE +dK_MMCCH-2 (K) | 1954 |
| 46 | 1952 | 1967 | 792.6822 | 2375.0247 | 2375.0205 | 1.77 | G | 3 | 21 | 0.0089 | FSKPEDTFDYHRFGYE +dK_MMCCH-2 (K) | 1954 |
| 47 | 3386 | 3405 | 877.4151 | 2629.2235 | 2629.2271 | -1.37 | G | 2 | 15 | 0.031 | LDHAYSLRDGHYYIAGPTKD +dK_MMCCH-2 (K) | 3404 |
| 48 | 3386 | 3405 | 658.3136 | 2629.2253 | 2629.2271 | -0.68 | G | 2 | 13 | 0.045 | LDHAYSLRDGHYYIAGPTKD +dK_MMCCH-2 (K) | 3404 |
| 49 | 1403 | 1422 | 921.4198 | 2761.2376 | 2761.2330 | 1.67 | G | 3 | 14 | 0.038 | NAVTSRDPQPELWDNKDFYE +dK_MMCCH-2 (K) | 1418 |
| 50 | 1215 | 1235 | 961.8065 | 2882.3976 | 2882.3949 | 0.90 | G | 3 | 32 | 0.00065 | ITQQLHDLDLHVGDNFFLKYE +dK_MMCCH-2 (K) | 1233 |
| 51 | 1215 | 1235 | 721.6078 | 2882.4021 | 2882.3949 | 2.46 | G | 3 | 18 | 0.018 | ITQQLHDLDLHVGDNFFLKYE +dK_MMCCH-2 (K) | 1233 |

**Figure 28B. KLH 2 (continued)**

| | | | | | | | | | | | | |
|---|---|---|---|---|---|---|---|---|---|---|---|---|
| 52 | 1973 | 1990 | 817.0586 | 2448.1541 | 2448.1454 | 3.59 | G | 0 | 16 | 0.028 | FVGMSVSSLHNYIKQQQE +dK_MMCCH-2 (K); Oxidation (M) | 1986 |
| 53 | 277 | 296 | 883.7546 | 2648.2419 | 2648.2329 | 3.40 | C | 4 | 38 | 0.00017 | DLTREHAKPADSFDYGRLGY +dK_MMCCH-2 (K) | 284 |
| 54 | 315 | 325 | 808.4200 | 1614.8254 | 1614.8188 | 4.09 | C | 1 | 13 | 0.049 | LGERAAKERTF +dK_MMCCH-2 (K) | 321 |
| 55 | 629 | 635 | 539.2531 | 1076.4917 | 1076.4848 | 6.32 | C | 0 | 19 | 0.014 | VGGSEKY +dK_MMCCH-2 (K) | 634 |
| 56 | 1075 | 1080 | 544.7759 | 1087.5373 | 1087.5372 | 0.09 | C | 1 | 30 | 0.0011 | QALQKY +dK_MMCCH-2 (K) | 1079 |
| 57 | 1125 | 1134 | 854.8457 | 1707.6768 | 1707.6763 | 0.35 | C | 3 | 20 | 0.011 | DYKNNFDYEY +dK_MMCCH-2 (K) | 1127 |
| 58 | 1165 | 1177 | 896.9915 | 1791.9685 | 1791.9593 | 5.13 | C | 3 | 15 | 0.031 | LLHEIGQSALVKF +dK_MMCCH-2 (K) | 1176 |
| 59 | 1526 | 1540 | 749.6849 | 2246.0330 | 2246.0215 | 5.12 | C | 2 | 38 | 0.00015 | KHNLPQDSFDYQNRF +dK_MMCCH-2 (K) | 1526 |
| 60 | 1696 | 1702 | 607.3009 | 1212.5872 | 1212.5849 | 1.90 | C | 1 | 17 | 0.022 | TTLEKHF +dK_MMCCH-2 (K) | 1700 |
| 61 | 1708 | 1719 | 839.8434 | 1677.6723 | 1677.6651 | 4.35 | C | 0 | 28 | 0.0015 | KNMQADDSPDGY +dK_MMCCH-2 (K) | 1708 |
| 62 | 1801 | 1809 | 706.8389 | 1411.6632 | 1411.6595 | 2.62 | C | 0 | 16 | 0.026 | SHKTFPNPF +dK_MMCCH-2 (K) | 1803 |
| 63 | 1985 | 1995 | 850.4283 | 1698.8421 | 1698.8399 | 1.30 | C | 0 | 39 | 0.00014 | IKQQQEADRVF +dK_MMCCH-2 (K) | 1986 |
| 64 | 2001 | 2011 | 726.8464 | 1451.6783 | 1451.6755 | 1.93 | C | 1 | 28 | 0.0014 | KGFGQSASVSF +dK_MMCCH-2 (K) | 2001 |
| 65 | 2463 | 2479 | 1128.0177 | 2254.0208 | 2254.0253 | -1.95 | C | 1 | 23 | 0.0055 | KAQSIHPEDVFDTDAPF +dK_MMCCH-2 (K) | 2463 |
| 66 | 2754 | 2760 | 598.3302 | 1194.6458 | 1194.6471 | -1.00 | C | 1 | 25 | 0.0033 | KQPLKPF +dK_MMCCH-2 (K) | 2754 |
| 67 | 2754 | 2760 | 598.3341 | 1194.6537 | 1194.6471 | 5.52 | C | 1 | 14 | 0.041 | KQPLKPF +dK_MMCCH-2 (K) | 2758 |
| 68 | 2865 | 2872 | 673.3649 | 1344.7152 | 1344.7112 | 2.97 | C | 1 | 19 | 0.012 | RYDITKVL +dK_MMCCH-2 (K) | 2870 |
| 69 | 3235 | 3242 | 617.3129 | 1232.6112 | 1232.6111 | 0.08 | C | 0 | 34 | 0.00039 | TSANVKIY +dK_MMCCH-2 (K) | 3240 |
| 70 | 3399 | 3406 | 576.8040 | 1151.5934 | 1151.5896 | 3.21 | C | 0 | 23 | 0.005 | IAGPTKDL +dK_MMCCH-2 (K) | 3404 |
| 71 | 184 | 191 | 703.8063 | 1405.5980 | 1405.5894 | 6.12 | Th | 1 | 29 | 0.0013 | LEQDEFCK +dK_MMCCH-2 (K) | 191 |
| 72 | 247 | 257 | 785.8562 | 1569.6978 | 1569.6916 | 4.01 | Th | 1 | 20 | 0.0099 | LRGKDPNSADC +dK_MMCCH-2 (K) | 250 |
| 73 | 283 | 293 | 782.8611 | 1563.7077 | 1563.7028 | 3.20 | Th | 3 | 34 | 0.00038 | AKPADSFDYGR +dK_MMCCH-2 (K) | 284 |
| 74 | 365 | 372 | 585.7803 | 1169.5461 | 1169.5461 | 0.00 | Th | 1 | 31 | 0.00085 | LGGPTEMK +dK_MMCCH-2 (K) | 372 |
| 75 | 431 | 437 | 569.2877 | 1136.5609 | 1136.5576 | 2.90 | Th | 1 | 15 | 0.034 | FDKPPVP +dK_MMCCH-2 (K) | 433 |
| 76 | 629 | 636 | 582.7676 | 1163.5206 | 1163.5169 | 3.18 | Th | 1 | 22 | 0.0064 | VGGSEKYS +dK_MMCCH-2 (K) | 634 |
| 77 | 893 | 904 | 904.8967 | 1807.7789 | 1807.7698 | 5.03 | Th | 1 | 22 | 0.0064 | FHGEPKWCPSPE +dK_MMCCH-2 (K) | 898 |
| 78 | 1124 | 1129 | 569.7510 | 1137.4875 | 1137.4801 | 6.51 | Th | 1 | 13 | 0.049 | FDYKNN +dK_MMCCH-2 (K) | 1127 |

Figure 28B. KLH 2 (continued)

| | | | | | | | | | | | | |
|---|---|---|---|---|---|---|---|---|---|---|---|---|
| 79 | 1525 | 1539 | 738.3566 | 2212.0481 | 2212.0371 | 4.93 | Th | 3 | 49 | 1.30E-05 | LKHNLPQDSFDYQNR +dK_MMCCH-2 (K) | 1526 |
| 80 | 1589 | 1605 | 735.3315 | 2202.9728 | 2202.9708 | 0.91 | Th | 2 | 18 | 0.015 | ICVEQGGEQNCKTKAGS +dK_MMCCH-2 (K) | 1600 |
| 81 | 1589 | 1605 | 735.3331 | 2202.9776 | 2202.9708 | 3.09 | Th | 2 | 34 | 0.00042 | ICVEQGGEQNCKTKAGS +dK_MMCCH-2 (K) | 1602 |
| 82 | 1800 | 1809 | 780.3743 | 1558.7340 | 1558.7279 | 3.91 | Th | 2 | 24 | 0.004 | FSHKTFPNPF +dK_MMCCH-2 (K) | 1803 |
| 83 | 1952 | 1963 | 627.2844 | 1878.8314 | 1878.8247 | 3.57 | Th | 2 | 47 | 2.00E-05 | FSKPEDTFDYHR +dK_MMCCH-2 (K) | 1954 |
| 84 | 1985 | 1994 | 776.8963 | 1551.7780 | 1551.7715 | 4.19 | Th | 2 | 20 | 0.0095 | IKQQQEADRV +dK_MMCCH-2 (K) | 1986 |
| 85 | 2193 | 2199 | 667.3038 | 1332.5931 | 1332.5849 | 6.15 | Th | 2 | 30 | 0.0011 | YWDWTKP +dK_MMCCH-2 (K) | 2198 |
| 86 | 2200 | 2206 | 562.3095 | 1122.6045 | 1122.5995 | 4.45 | Th | 2 | 21 | 0.0077 | ISKLPDL +dK_MMCCH-2 (K) | 2202 |
| 87 | 2225 | 2229 | 462.2224 | 922.4301 | 922.4259 | 4.66 | Th | 2 | 24 | 0.0039 | FAKGY +dK_MMCCH-2 (K) | 2227 |
| 88 | 2941 | 2950 | 720.8415 | 1439.6684 | 1439.6602 | 5.70 | Th | 3 | 46 | 2.60E-05 | LDEANDLKNA +dK_MMCCH-2 (K) | 2948 |
| 89 | 3009 | 3015 | 573.2734 | 1144.5322 | 1144.5335 | -1.14 | Th | 0 | 16 | 0.025 | LKEHGSH +dK_MMCCH-2 (K) | 3010 |
| 90 | 3020 | 3026 | 662.2908 | 1322.5671 | 1322.5642 | 2.19 | Th | 2 | 19 | 0.011 | YWDWTKS +dK_MMCCH-2 (K) | 3025 |
| 91 | 3399 | 3405 | 520.2607 | 1038.5069 | 1038.5056 | 1.25 | Th | 1 | 27 | 0.002 | IAGPTKD +dK_MMCCH-2 (K) | 3404 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the lysine conjugation site for KLH1 or KLH2.

**Figure 29. Identification details of MMCCH-conjugated peptides for sample 4 (2nd LC-MS/MS)**

**Figure 29A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 124 | 141 | 785.4094 | 2353.2064 | 2353.2001 | 2.68 | T | 1 | 15 | 0.034 | GFTDPPVKHHQSANLLVR +dK_MMCCH-2 (K) | 131 |
| 2 | 142 | 149 | 656.3414 | 1310.6682 | 1310.6653 | 2.21 | T | 1 | 29 | 0.0012 | KNINDLTR +dK_MMCCH-2 (K) | 142 |
| 3 | 157 | 166 | 548.9227 | 1643.7464 | 1643.7402 | 3.71 | T | 1 | 21 | 0.008 | EAFHKFQEDR +dK_MMCCH-2 (K) | 161 |
| 4 | 184 | 192 | 484.8979 | 1451.6719 | 1451.6649 | 4.82 | T | 1 | 14 | 0.039 | CPRPDAKDR +dK_MMCCH-2 (K) | 190 |
| 5 | 553 | 560 | 663.8693 | 1325.7240 | 1325.7166 | 5.58 | T | 2 | 22 | 0.0068 | VKFDKVPR +dK_MMCCH-2 (K) | 554 |
| 6 | 566 | 570 | 485.2459 | 968.4772 | 968.4749 | 2.37 | T | 1 | 19 | 0.014 | KNVDR +dK_MMCCH-2 (K) | 566 |
| 7 | 581 | 590 | 733.9196 | 1465.8246 | 1465.8214 | 2.18 | T | 2 | 30 | 0.0011 | KALALLKEDK +dK_MMCCH-2 (K) | 581 |
| 8 | 588 | 606 | 781.0378 | 2340.0917 | 2340.0845 | 3.08 | T | 1 | 15 | 0.031 | EDKSAGGFQQLGAFHGEPK +dK_MMCCH-2 (K) | 590 |
| 9 | 591 | 615 | 1004.4722 | 3010.3949 | 3010.3742 | 6.88 | T | 1 | 16 | 0.027 | SAGGFQQLGAFHGEPKWCPSPEASK +dK_MMCCH-2 (K) | 606 |
| 10 | 682 | 699 | 620.2965 | 2477.1567 | 2477.1434 | 5.37 | T | 1 | 15 | 0.03 | HNPWFDGHIDTVDKTTTR +dK_MMCCH-2 (K) | 695 |
| 11 | 700 | 719 | 887.7751 | 2660.3034 | 2660.2945 | 3.38 | T | 1 | 20 | 0.0097 | SVQNKLFEQPEFGHYTSIAK +dK_MMCCH-2 (K) | 704 |
| 12 | 778 | 789 | 958.0043 | 1913.9941 | 1913.9862 | 4.13 | T | 1 | 15 | 0.03 | IWAIWQALQKYR +Deamidated (NQ); dK_MMCCH-2 (K) | 787 |
| 13 | 790 | 804 | 1003.4770 | 2004.9394 | 2004.9220 | 8.73 | T | 0 | 81 | 7.20E-09 | GKPYNVANCAVTSMR +dK_MMCCH-2 (K) | 791 |
| 14 | 858 | 863 | 520.3001 | 1038.5857 | 1038.5783 | 7.03 | T | 1 | 15 | 0.028 | KLEAIK +dK_MMCCH-2 (K) | 858 |
| 15 | 859 | 867 | 699.3604 | 1396.7061 | 1396.7020 | 2.94 | T | 1 | 15 | 0.034 | LEAIKSQDR +dK_MMCCH-2 (K) | 863 |
| 16 | 879 | 884 | 500.2828 | 998.5511 | 998.5470 | 4.11 | T | 1 | 14 | 0.04 | KSSLVK +dK_MMCCH-2 (K) | 879 |
| 17 | 953 | 965 | 635.3318 | 1902.9735 | 1902.9662 | 3.84 | T | 1 | 48 | 1.50E-05 | DLFKQPSVIHEPR +dK_MMCCH-2 (K) | 956 |
| 18 | 986 | 1000 | 685.0270 | 2052.0591 | 2052.0561 | 1.46 | T | 1 | 23 | 0.0047 | KNIENLSLGELESLR +dK_MMCCH-2 (K) | 986 |
| 19 | 1048 | 1060 | 913.5313 | 1825.0481 | 1825.0423 | 3.18 | T | 1 | 35 | 0.0003 | LYVVVVENALLKK +dK_MMCCH-2 (K) | 1060 |
| 20 | 1092 | 1114 | 791.8763 | 3163.4760 | 3163.4682 | 2.47 | T | 1 | 42 | 6.40E-05 | QHHYETNPFHHGKITHENEITTR +dK_MMCCH-2 (K) | 1104 |
| 21 | 1199 | 1203 | 353.1925 | 1056.5556 | 1056.5538 | 1.61 | T | 1 | 17 | 0.018 | KRPYR +dK_MMCCH-2 (K) | 1199 |
| 22 | 1395 | 1403 | 485.5970 | 1453.7692 | 1453.7599 | 6.40 | T | 2 | 31 | 0.00082 | KDITQLDKR +dK_MMCCH-2 (K) | 1395 |
| 23 | 1473 | 1489 | 778.4048 | 2332.1925 | 2332.1827 | 4.20 | T | 1 | 38 | 0.00015 | KHGAVVGLPYWDWTLPR +dK_MMCCH-2 (K) | 1473 |
| 24 | 1519 | 1530 | 599.6281 | 1795.8623 | 1795.8563 | 3.34 | T | 1 | 20 | 0.0096 | IEFEGENVHTKR +dK_MMCCH-2 (K) | 1529 |

**Figure 29A. KLH 1 (continued)**

| | | | | | | | | | | | | |
|---|---|---|---|---|---|---|---|---|---|---|---|---|
| 25 | 1747 | 1758 | 613.9781 | 1838.9124 | 1838.9012 | 6.14 | T | 1 | 18 | 0.014 | LYKYDITETLDK +dK_MMCCH-2 (K) | 1749 |
| 26 | 1923 | 1932 | 756.3818 | 1510.7491 | 1510.7425 | 4.43 | T | 1 | 22 | 0.0064 | NKVMPNPFAR +dK_MMCCH-2 (K) | 1924 |
| 27 | 2100 | 2109 | 492.2497 | 1964.9698 | 1964.9601 | 4.94 | T | 2 | 16 | 0.027 | EIKDKQHHVR +2 dK_MMCCH-2 (K) | 2102, 2104 |
| 28 | 2121 | 2149 | 890.4196 | 3557.6494 | 3557.6378 | 3.26 | T | 1 | 23 | 0.0049 | TSADVQFQICKTSEDCHHGGQIFVLGGTK +dK_MMCCH-2 (K) | 2131 |
| 29 | 2299 | 2314 | 715.0188 | 2142.0346 | 2142.0238 | 5.04 | T | 1 | 37 | 0.00019 | LYTKQMEDALTAHGAR +dK_MMCCH-2 (K) | 2302 |
| 30 | 2359 | 2367 | 736.3524 | 1470.6903 | 1470.6813 | 6.12 | T | 1 | 32 | 0.00064 | DKLFNDPER +dK_MMCCH-2 (K) | 2360 |
| 31 | 2461 | 2490 | 642.8185 | 3850.8672 | 3850.8638 | 0.88 | T | 0 | 18 | 0.015 | RPLRPFSDPINHNAFTHSNAKPTDVFEYSR +dK_MMCCH-2 (K) | 2481 |
| 32 | 2507 | 2514 | 682.3542 | 1362.6938 | 1362.6853 | 6.24 | T | 1 | 33 | 0.00051 | KLEHELEK +dK_MMCCH-2 (K) | 2507 |
| 33 | 2515 | 2520 | 571.7616 | 1141.5086 | 1141.5074 | 1.14 | T | 1 | 17 | 0.018 | QKEEDR +dK_MMCCH-2 (K) | 2516 |
| 34 | 2621 | 2636 | 802.7122 | 2405.1147 | 2405.1083 | 2.62 | T | 1 | 51 | 8.10E-06 | HHEKHHEDHHEDILVR +dK_MMCCH-2 (K) | 2624 |
| 35 | 2637 | 2651 | 713.3541 | 2137.0404 | 2137.0374 | 1.36 | T | 1 | 59 | 1.20E-06 | KNIHSLSHHEAEELR +dK_MMCCH-2 (K) | 2637 |
| 36 | 2851 | 2883 | 807.5740 | 4032.8335 | 4032.8346 | -0.27 | T | 0 | 42 | 6.80E-05 | VKPAHAGSCAGDIMHVPLHPFNYESVNNDDFTR +dK_MMCCH-2 (K) | 2852 |
| 37 | 2897 | 2919 | 800.1400 | 3196.5307 | 3196.5287 | 0.63 | T | 1 | 45 | 3.50E-05 | FNYKYDNLNLHGHNIEELEEVLR +dK_MMCCH-2 (K) | 2900 |
| 38 | 3003 | 3021 | 825.7752 | 2474.3036 | 2474.2879 | 6.35 | T | 1 | 17 | 0.018 | KYDHTELDASVLPAPIIVR +dK_MMCCH-2 (K) | 3003 |
| 39 | 123 | 151 | 673.0145 | 4032.0434 | 4032.0350 | 2.08 | G | 2 | 18 | 0.017 | KGFTDPPVKHHQSANLLVRKNINDLTREE +2 dK_MMCCH-2 (K) | 123, 131 |
| 40 | 123 | 151 | 739.7914 | 3693.9208 | 3693.9049 | 4.30 | G | 2 | 32 | 0.00068 | KGFTDPPVKHHQSANLLVRKNINDLTREE +dK_MMCCH-2 (K) | 131 |
| 41 | 158 | 169 | 606.2845 | 1815.8318 | 1815.8250 | 3.74 | G | 2 | 27 | 0.0019 | AFHKFQEDRSVD +dK_MMCCH-2 (K) | 161 |
| 42 | 435 | 442 | 676.3252 | 1350.6358 | 1350.6312 | 3.48 | G | 1 | 21 | 0.008 | NIEKMIHE +dK_MMCCH-2 (K) | 438 |
| 43 | 526 | 544 | 777.7501 | 2330.2285 | 2330.2304 | -0.82 | G | 1 | 15 | 0.029 | VDGTKLASSLIPHASVIRE +dK_MMCCH-2 (K) | 530 |
| 44 | 589 | 612 | 990.1202 | 2967.3389 | 2967.3320 | 2.33 | G | 1 | 31 | 0.0008 | DKSAGGFQQLGAFHGEPKWCPSPE +dK_MMCCH-2 (K) | 606 |
| 45 | 825 | 841 | 820.3759 | 2458.1057 | 2458.0940 | 4.76 | G | 1 | 25 | 0.0031 | HSVPFNVFDYKTNFNYE +dK_MMCCH-2 (K) | 835 |
| 46 | 847 | 860 | 965.0205 | 1928.0265 | 1928.0077 | 9.75 | G | 0 | 19 | 0.013 | FNGLSISQLNKKLE +dK_MMCCH-2 (K) | 858 |
| 47 | 942 | 970 | 908.9841 | 3631.9074 | 3631.8973 | 2.78 | G | 2 | 15 | 0.035 | VFDLKPASLGKDLFKQPSVIHEPRIGHHE +dK_MMCCH-2 (K) | 956 |
| 48 | 1097 | 1110 | 999.9612 | 1997.9078 | 1997.9054 | 1.20 | G | 1 | 23 | 0.0049 | TNPFHHGKITHENE +dK_MMCCH-2 (K) | 1104 |
| 49 | 1376 | 1396 | 648.5447 | 2590.1496 | 2590.1428 | 2.63 | G | 3 | 22 | 0.0066 | AGTDSAHTDDGHTEPVMIRKD +dK_MMCCH-2 (K) | 1395 |
| 50 | 1397 | 1413 | 774.4365 | 2320.2876 | 2320.2824 | 2.20 | G | 1 | 14 | 0.044 | ITQLDKRQQLSLVKALE +dK_MMCCH-2 (K) | 1402 |
| 51 | 1397 | 1413 | 774.4338 | 2320.2797 | 2320.2824 | -1.16 | G | 1 | 29 | 0.0011 | ITQLDKRQQLSLVKALE +dK_MMCCH-2 (K) | 1410 |

**Figure 29A. KLH 1 (continued)**

| | | | | | | | | | | | | |
|---|---|---|---|---|---|---|---|---|---|---|---|---|
| 52 | 1495 | 1522 | 1155.5880 | 3463.7422 | 3463.7221 | 5.80 | G | 3 | 20 | 0.0096 | LLTVSTIHDPETGRDIPNPFIGSKIEFE +Deamidated (NQ); dK_MMCCH-2 (K) | 1518 |
| 53 | 1755 | 1765 | 570.6062 | 1708.7968 | 1708.7913 | 3.22 | G | 2 | 37 | 0.0002 | TLDKMNLRHDE +dK_MMCCH-2 (K) | 1758 |
| 54 | 2089 | 2100 | 854.4081 | 1706.8016 | 1706.8008 | 0.53 | G | 2 | 41 | 8.90E-05 | IGGMNLHEIEKE +dK_MMCCH-2 (K) | 2099 |
| 55 | 2624 | 2646 | 637.9164 | 3184.5458 | 3184.5373 | 2.67 | G | 4 | 14 | 0.036 | KHHEDHHEDILVRKNIHSLSHHE +dK_MMCCH-2 (K) | 2624 |
| 56 | 2647 | 2661 | 715.6800 | 2144.0181 | 2144.0095 | 4.01 | G | 4 | 22 | 0.0066 | AEELRDALYKLQNDE +dK_MMCCH-2 (K) | 2656 |
| 57 | 3090 | 3097 | 632.8043 | 1263.5940 | 1263.5879 | 4.75 | G | 0 | 16 | 0.027 | LGKMYSVE +dK_MMCCH-2 (K) | 3092 |
| 58 | 59 | 68 | 729.3447 | 1456.6749 | 1456.6731 | 1.24 | C | 1 | 30 | 0.00091 | VLGGPSEMKW +dK_MMCCH-2 (K); Oxidation (M) | 67 |
| 59 | 160 | 177 | 828.0412 | 2481.1018 | 2481.0907 | 4.47 | C | 2 | 14 | 0.043 | HKFQEDRSVDGYQATAEY +dK_MMCCH-2 (K) | 161 |
| 60 | 587 | 595 | 638.8013 | 1275.5880 | 1275.5805 | 5.88 | C | 0 | 33 | 0.00052 | KEDKSAGGF +dK_MMCCH-2 (K) | 590 |
| 61 | 715 | 723 | 655.8733 | 1309.7320 | 1309.7316 | 0.38 | C | 1 | 23 | 0.0056 | TSIAKQVLL +dK_MMCCH-2 (K) | 719 |
| 62 | 783 | 788 | 544.7773 | 1087.5400 | 1087.5372 | 2.57 | C | 1 | 30 | 0.0011 | QALQKY +dK_MMCCH-2 (K) | 787 |
| 63 | 830 | 838 | 743.3416 | 1484.6687 | 1484.6646 | 2.76 | C | 2 | 13 | 0.047 | NVFDYKTNF +dK_MMCCH-2 (K) | 835 |
| 64 | 860 | 868 | 716.3497 | 1430.6848 | 1430.6864 | -1.12 | C | 0 | 14 | 0.038 | EAIKSQDRF +dK_MMCCH-2 (K) | 863 |
| 65 | 944 | 955 | 821.4307 | 1640.8469 | 1640.8484 | -0.91 | C | 3 | 20 | 0.011 | DLKPASLGKDLF +dK_MMCCH-2 (K) | 946 |
| 66 | 1330 | 1337 | 620.3547 | 1238.6949 | 1238.6944 | 0.40 | C | 1 | 22 | 0.007 | KLDITKAL +dK_MMCCH-2 (K) | 1335 |
| 67 | 1431 | 1446 | 696.3586 | 2086.0541 | 2086.0492 | 2.35 | C | 1 | 16 | 0.026 | HALPPLCPSPAASKRF +dK_MMCCH-2 (K) | 1444 |
| 68 | 1580 | 1586 | 544.2616 | 1086.5086 | 1086.5056 | 2.85 | C | 0 | 17 | 0.02 | VGGKEPY +dK_MMCCH-2 (K) | 1583 |
| 69 | 1818 | 1827 | 753.3703 | 1504.7260 | 1504.7232 | 1.93 | C | 1 | 22 | 0.0062 | SERDIGSLKY +dK_MMCCH-2 (K) | 1826 |
| 70 | 2076 | 2082 | 612.3033 | 1222.5920 | 1222.5944 | -1.96 | C | 3 | 14 | 0.037 | KYELLGY +dK_MMCCH-2 (K) | 2076 |
| 71 | 2476 | 2488 | 923.9218 | 1845.8291 | 1845.8244 | 2.55 | C | 1 | 17 | 0.02 | THSNAKPTDVFEY +dK_MMCCH-2 (K) | 2481 |
| 72 | 2656 | 2666 | 793.3524 | 1584.6902 | 1584.6879 | 1.45 | C | 1 | 24 | 0.0045 | KLQNDESHGGY +dK_MMCCH-2 (K) | 2656 |
| 73 | 2964 | 2971 | 607.2731 | 1212.5317 | 1212.5307 | 0.82 | C | 0 | 13 | 0.047 | GGAKEMPW +dK_MMCCH-2 (K) | 2967 |
| 74 | 1 | 9 | 725.3336 | 1448.6526 | 1448.6567 | -2.90 | Th | 2 | 28 | 0.0015 | MTPEELKTY +dK_MMCCH-2 (K) | 7 |
| 75 | 38 | 56 | 861.6798 | 2582.0174 | 2582.0108 | 2.56 | Th | 2 | 19 | 0.013 | VCIPDDNDRNDDHCEKAGD +dK_MMCCH-2 (K) | 53 |
| 76 | 59 | 69 | 785.3771 | 1568.7397 | 1568.7367 | 1.91 | Th | 3 | 54 | 3.80E-06 | VLGGPSEMKWQ +dK_MMCCH-2 (K) | 67 |
| 77 | 78 | 84 | 569.2875 | 1136.5604 | 1136.5536 | 5.98 | Th | 1 | 52 | 6.40E-06 | LSDTVHK +dK_MMCCH-2 (K) | 84 |
| 78 | 118 | 124 | 571.2795 | 1140.5444 | 1140.5386 | 5.09 | Th | 0 | 24 | 0.0042 | VHHPEKG +dK_MMCCH-2 (K) | 123 |

**Figure 29A. KLH 1 (continued)**

| | | | | | | | | | | | | |
|---|---|---|---|---|---|---|---|---|---|---|---|---|
| 79 | 431 | 439 | 707.8488 | 1413.6831 | 1413.6883 | -3.75 | Th | 3 | 19 | 0.012 | ISLENIEKM +dK_MMCCH-2 (K) | 438 |
| 80 | 521 | 530 | 707.8455 | 1413.6765 | 1413.6698 | 4.74 | Th | 2 | 57 | 2.00E-06 | VDITEVDGTK +dK_MMCCH-2 (K) | 530 |
| 81 | 585 | 591 | 585.8103 | 1169.6060 | 1169.6002 | 5.04 | Th | 1 | 25 | 0.0029 | LLKEDKS +dK_MMCCH-2 (K) | 590 |
| 82 | 601 | 612 | 904.8980 | 1807.7813 | 1807.7698 | 6.36 | Th | 1 | 21 | 0.0084 | FHGEPKWCPSPE +dK_MMCCH-2 (K) | 606 |
| 83 | 666 | 672 | 607.2825 | 1212.5505 | 1212.5485 | 1.65 | Th | 2 | 25 | 0.0034 | LADHEKY +dK_MMCCH-2 (K) | 671 |
| 84 | 831 | 837 | 612.7867 | 1223.5589 | 1223.5533 | 4.66 | Th | 2 | 28 | 0.0017 | VFDYKTN +dK_MMCCH-2 (K) | 835 |
| 85 | 1219 | 1232 | 766.6623 | 2296.9650 | 2296.9617 | 1.48 | Th | 1 | 15 | 0.034 | FDKSDNNDEATKTH +2 dK_MMCCH-2 (K) | 1221, 1230 |
| 86 | 1580 | 1587 | 572.7716 | 1143.5287 | 1143.5271 | 1.40 | Th | 1 | 17 | 0.019 | VGGKEPYG +dK_MMCCH-2 (K) | 1583 |
| 87 | 1657 | 1668 | 921.9177 | 1841.8208 | 1841.8182 | 1.41 | Th | 2 | 35 | 0.00035 | FSKPEDTFDYQK +dK_MMCCH-2 (K) | 1659 |
| 88 | 1657 | 1668 | 921.9194 | 1841.8242 | 1841.8182 | 3.26 | Th | 2 | 30 | 0.00099 | FSKPEDTFDYQK +dK_MMCCH-2 (K) | 1668 |
| 89 | 1733 | 1741 | 664.3220 | 1326.6294 | 1326.6312 | -1.36 | Th | 2 | 18 | 0.015 | VLGGEKEMP +dK_MMCCH-1 (K); Oxidation (M) | 1738 |
| 90 | 1894 | 1904 | 850.4149 | 1698.8152 | 1698.8116 | 2.12 | Th | 5 | 39 | 0.00012 | VAVPYWDWTKP +dK_MMCCH-2 (K) | 1903 |
| 91 | 2064 | 2068 | 499.7595 | 997.5045 | 997.5055 | -1.00 | Th | 0 | 20 | 0.011 | FTKKH +dK_MMCCH-2 (K) | 2067 |
| 92 | 2094 | 2100 | 618.3040 | 1234.5934 | 1234.5903 | 2.43 | Th | 1 | 20 | 0.0093 | LHEIEKE +dK_MMCCH-2 (K) | 2099 |
| 93 | 2129 | 2141 | 622.9255 | 1865.7546 | 1865.7495 | 2.73 | Th | 0 | 19 | 0.014 | ICKTSEDCHHGGQ +dK_MMCCH-2 (K) | 2131 |
| 94 | 2238 | 2248 | 778.8811 | 1555.7476 | 1555.7440 | 2.38 | Th | 3 | 23 | 0.0051 | LTTAEVDNLKD +dK_MMCCH-2 (K) | 2247 |
| 95 | 2480 | 2490 | 825.8984 | 1649.7822 | 1649.7759 | 3.82 | Th | 3 | 26 | 0.0024 | AKPTDVFEYSR +dK_MMCCH-2 (K) | 2481 |
| 96 | 2512 | 2521 | 538.6002 | 1612.7787 | 1612.7766 | 1.24 | Th | 0 | 39 | 0.00013 | LEKQKEEDRT +dK_MMCCH-2 (K) | 2514 |
| 97 | 2512 | 2521 | 807.3967 | 1612.7788 | 1612.7766 | 1.30 | Th | 0 | 20 | 0.0096 | LEKQKEEDRT +dK_MMCCH-2 (K) | 2516 |
| 98 | 2595 | 2606 | 806.4067 | 1610.7988 | 1610.7862 | 7.82 | Th | 2 | 38 | 0.00017 | IIDTSGKQLPSD +dK_MMCCH-2 (K) | 2601 |
| 99 | 2607 | 2612 | 520.7801 | 1039.5456 | 1039.5446 | 0.96 | Th | 2 | 23 | 0.0052 | LIKMPT +dK_MMCCH-2 (K) | 2609 |
| 100 | 2766 | 2771 | 551.7874 | 1101.5603 | 1101.5529 | 6.72 | Th | 1 | 36 | 0.00025 | IFQQTK +dK_MMCCH-2 (K) | 2771 |
| 101 | 2808 | 2816 | 638.3197 | 1274.6248 | 1274.6217 | 2.51 | Th | 3 | 28 | 0.0017 | LIGGAEKYS +dK_MMCCH-2 (K) | 2814 |
| 102 | 2946 | 2960 | 965.4082 | 1928.8018 | 1928.7986 | 1.66 | Th | 3 | 34 | 0.00039 | IKSGTDSDDEYAGSF +dK_MMCCH-2 (K) | 2947 |
| 103 | 2990 | 2996 | 583.2842 | 1164.5539 | 1164.5485 | 4.64 | Th | 1 | 38 | 0.00014 | LTDDHVK +dK_MMCCH-2 (K) | 2996 |

Figure 29B. KLH 2

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 26 | 41 | 1043.0233 | 2084.0321 | 2084.0347 | -1.25 | T | 1 | 56 | 2.30E-06 | KNVDSLSSDEVLALEK +dK_MMCCH-2 (K) | 26 |
| 2 | 147 | 155 | 694.3710 | 1386.7274 | 1386.7217 | 4.11 | T | 1 | 32 | 0.00062 | ADITFLNKK +dK_MMCCH-2 (K) | 155 |
| 3 | 164 | 175 | 596.3118 | 1785.9135 | 1785.8985 | 8.40 | T | 1 | 41 | 8.70E-05 | LFEKVQPGHHTR +dK_MMCCH-2 (K) | 167 |
| 4 | 249 | 271 | 987.4451 | 2959.3136 | 2959.3051 | 2.84 | T | 1 | 25 | 0.003 | GKDPNSADCAHNLIHTPMEPFDR +dK_MMCCH-2 (K) | 250 |
| 5 | 281 | 293 | 610.9456 | 1829.8150 | 1829.8043 | 5.90 | T | 0 | 54 | 3.60E-06 | EHAKPADSFDYGR +dK_MMCCH-2 (K) | 284 |
| 6 | 419 | 446 | 846.4617 | 3381.8176 | 3381.8132 | 1.30 | T | 1 | 26 | 0.0025 | QPTLVHRPAKGHFDKPPVPVAQANLAVR +Deamidated (NQ); dK_MMCCH-2 (K) | 428 |
| 7 | 429 | 446 | 752.0691 | 2253.1854 | 2253.1729 | 5.59 | T | 0 | 50 | 9.70E-06 | GHFDKPPVPVAQANLAVR +dK_MMCCH-2 (K) | 433 |
| 8 | 467 | 488 | 944.4572 | 2830.3498 | 2830.3385 | 3.99 | T | 1 | 65 | 2.90E-07 | FQNDKSVDGYQATVEFHALPAR +dK_MMCCH-2 (K) | 471 |
| 9 | 489 | 497 | 484.8979 | 1451.6719 | 1451.6649 | 4.82 | T | 1 | 14 | 0.039 | CPRPDAKDR +dK_MMCCH-2 (K) | 495 |
| 10 | 936 | 950 | 720.3440 | 2158.0101 | 2158.0095 | 0.32 | T | 1 | 32 | 0.0007 | KHGFTGGLPYWDWTR +dK_MMCCH-2 (K) | 936 |
| 11 | 1082 | 1096 | 996.4645 | 1990.9144 | 1990.9063 | 4.07 | T | 0 | 77 | 1.80E-08 | GKPYNTANCAIASMR +dK_MMCCH-2 (K) | 1083 |
| 12 | 1097 | 1115 | 827.0785 | 2478.2138 | 2478.2101 | 1.49 | T | 0 | 66 | 2.50E-07 | KPLQPFGLDSVINPDDETR +dK_MMCCH-2 (K) | 1097 |
| 13 | 1123 | 1149 | 1190.5604 | 3568.6594 | 3568.6497 | 2.75 | T | 1 | 68 | 1.70E-07 | VFDYKNNFDYEYESLAFNGLSIAQLDR +dK_MMCCH-2 (K) | 1127 |
| 14 | 1295 | 1311 | 764.0680 | 2289.1821 | 2289.1715 | 4.67 | T | 1 | 13 | 0.05 | SAFLQIQKEGIYENIAK +dK_MMCCH-2 (K) | 1302 |
| 15 | 1355 | 1385 | 1271.2700 | 3810.7882 | 3810.7876 | 0.18 | T | 1 | 27 | 0.0022 | GSAVAVPYWDWTEKADSLPSLINDATYFNSR +dK_MMCCH-2 (K) | 1368 |
| 16 | 1409 | 1450 | 1371.8911 | 5483.5353 | 5483.5213 | 2.55 | T | 1 | 17 | 0.022 | DPQPELWDNKDFYENVMLALEQDNFCDFEIQLELIHNALHSR +dK_MMCCH-2 (K) | 1418 |
| 17 | 1455 | 1480 | 1117.8834 | 3350.6284 | 3350.6070 | 6.39 | T | 1 | 40 | 0.00011 | AKYSLSSLDYTAFDPVFFLHHANVDR +dK_MMCCH-2 (K) | 1456 |
| 18 | 1493 | 1507 | 721.6597 | 2161.9574 | 2161.9594 | -0.97 | T | 1 | 55 | 3.20E-06 | KKPYNEADCAVNEMR +dK_MMCCH-2 (K) | 1494 |
| 19 | 1542 | 1564 | 802.8948 | 3207.5503 | 3207.5448 | 1.71 | T | 1 | 30 | 0.00094 | YQYDNLQFNHFSIQKLDQTIQAR +dK_MMCCH-2 (K) | 1556 |
| 20 | 1622 | 1633 | 591.9822 | 1772.9247 | 1772.9171 | 4.29 | T | 1 | 23 | 0.0047 | LYKFDITSALHK +dK_MMCCH-2 (K) | 1624 |
| 21 | 1690 | 1704 | 532.2889 | 2125.1267 | 2125.1241 | 1.18 | T | 2 | 21 | 0.0085 | KEVSSLTTLEKHFLR +dK_MMCCH-2 (K) | 1700 |
| 22 | 1949 | 1963 | 560.2532 | 2236.9836 | 2236.9735 | 4.47 | T | 0 | 46 | 2.30E-05 | TQEFSKPEDTFDYHR +dK_MMCCH-2 (K) | 1954 |
| 23 | 2400 | 2404 | 479.2446 | 956.4746 | 956.4749 | -0.42 | T | 1 | 14 | 0.043 | KSQTR +dK_MMCCH-2 (K) | 2400 |
| 24 | 2527 | 2541 | 1007.5180 | 2013.0215 | 2013.0088 | 6.26 | T | 1 | 69 | 1.40E-07 | KDVTSLTASEIENLR +dK_MMCCH-2 (K) | 2527 |

**Figure 29B. KLH 2 (continued)**

| | | | | | | | | | | | | |
|---|---|---|---|---|---|---|---|---|---|---|---|---|
| 25 | 2728 | 2739 | 958.0043 | 1913.9941 | 1913.9862 | 4.13 | T | 1 | 15 | 0.03 | IWAIWQALQKYR +Deamidated (NQ); dK_MMCCH-2 (K) | 2737 |
| 26 | 2755 | 2764 | 764.3857 | 1526.7569 | 1526.7439 | 8.51 | T | 0 | 34 | 0.00043 | QPLKPFSESR +Deamidated (NQ); dK_MMCCH-2 (K) | 2758 |
| 27 | 41 | 49 | 692.3309 | 1382.6472 | 1382.6388 | 6.08 | G | 2 | 20 | 0.01 | KALDDLQQD +dK_MMCCH-2 (K) | 41 |
| 28 | 167 | 178 | 590.9647 | 1769.8723 | 1769.8705 | 1.02 | G | 0 | 24 | 0.0036 | KVQPGHHTRLME +dK_MMCCH-2 (K) | 167 |
| 29 | 318 | 322 | 456.7346 | 911.4547 | 911.4535 | 1.43 | G | 0 | 18 | 0.018 | RAAKE +dK_MMCCH-2 (K) | 321 |
| 30 | 433 | 456 | 975.8574 | 2924.5504 | 2924.5429 | 2.56 | G | 2 | 21 | 0.0074 | KPPVPVAQANLAVRKNINDLTAEE +dK_MMCCH-2 (K) | 433 |
| 31 | 466 | 481 | 1098.5017 | 2194.9889 | 2194.9841 | 2.19 | G | 2 | 36 | 0.00023 | RFQNDKSVDGYQATVE +Deamidated (NQ); dK_MMCCH-2 (K) | 471 |
| 32 | 1117 | 1135 | 1404.1268 | 2806.2391 | 2806.2373 | 0.64 | G | 3 | 17 | 0.021 | HSVPFRVFDYKNNFDYEYE +dK_MMCCH-2 (K) | 1127 |
| 33 | 1215 | 1235 | 961.8077 | 2882.4014 | 2882.3949 | 2.26 | G | 3 | 40 | 9.80E-05 | ITQQLHDLDLHVGDNFFLKYE +dK_MMCCH-2 (K) | 1233 |
| 34 | 1292 | 1303 | 879.4660 | 1756.9174 | 1756.9182 | -0.40 | G | 0 | 44 | 4.30E-05 | SIRSAFLQIQKE +dK_MMCCH-2 (K) | 1302 |
| 35 | 1308 | 1320 | 904.9468 | 1807.8791 | 1807.8749 | 2.32 | G | 0 | 22 | 0.0066 | NIAKFHGKPGLCE +dK_MMCCH-2 (K) | 1311 |
| 36 | 1308 | 1320 | 904.9480 | 1807.8814 | 1807.8749 | 3.60 | G | 0 | 34 | 0.00036 | NIAKFHGKPGLCE +dK_MMCCH-2 (K) | 1315 |
| 37 | 1354 | 1378 | 1043.5093 | 3127.5060 | 3127.4961 | 3.17 | G | 3 | 16 | 0.022 | RGSAVAVPYWDWTEKADSLPSLIND +dK_MMCCH-2 (K) | 1368 |
| 38 | 1403 | 1422 | 921.4252 | 2761.2537 | 2761.2330 | 7.50 | G | 3 | 17 | 0.018 | NAVTSRDPQPELWDNKDFYE +dK_MMCCH-2 (K) | 1418 |
| 39 | 1952 | 1967 | 792.6834 | 2375.0284 | 2375.0205 | 3.33 | G | 3 | 34 | 0.00036 | FSKPEDTFDYHRFGYE +dK_MMCCH-2 (K) | 1954 |
| 40 | 1973 | 1990 | 811.7261 | 2432.1566 | 2432.1505 | 2.51 | G | 0 | 24 | 0.0037 | FVGMSVSSLHNYIKQQQE +dK_MMCCH-2 (K) | 1986 |
| 41 | 2475 | 2484 | 752.8744 | 1503.7342 | 1503.7320 | 1.53 | G | 1 | 19 | 0.013 | TDAPFFIKVE +dK_MMCCH-2 (K) | 2482 |
| 42 | 3386 | 3405 | 658.3157 | 2629.2338 | 2629.2271 | 2.55 | G | 2 | 22 | 0.007 | LDHAYSLRDGHYYIAGPTKD +dK_MMCCH-2 (K) | 3404 |
| 43 | 277 | 296 | 883.7536 | 2648.2390 | 2648.2329 | 2.27 | C | 4 | 35 | 0.00034 | DLTREHAKPADSFDYGRLGY +dK_MMCCH-2 (K) | 284 |
| 44 | 629 | 635 | 539.2529 | 1076.4913 | 1076.4848 | 6.04 | C | 0 | 21 | 0.0076 | VGGSEKY +dK_MMCCH-2 (K) | 634 |
| 45 | 1007 | 1015 | 669.8713 | 1337.7280 | 1337.7265 | 1.12 | C | 1 | 27 | 0.0021 | TDIAKQVLL +dK_MMCCH-2 (K) | 1011 |
| 46 | 1075 | 1080 | 544.7773 | 1087.5400 | 1087.5372 | 2.57 | C | 1 | 30 | 0.0011 | QALQKY +dK_MMCCH-2 (K) | 1079 |
| 47 | 1298 | 1306 | 715.3745 | 1428.7343 | 1428.7323 | 1.47 | C | 1 | 14 | 0.045 | LQIQKEGIY +dK_MMCCH-2 (K) | 1302 |
| 48 | 1589 | 1606 | 784.3547 | 2350.0422 | 2350.0392 | 1.28 | C | 0 | 22 | 0.0068 | ICVEQGGEQNCKTKAGSF +dK_MMCCH-2 (K) | 1602 |
| 49 | 1696 | 1702 | 607.3019 | 1212.5892 | 1212.5849 | 3.55 | C | 1 | 23 | 0.0054 | TTLEKHF +dK_MMCCH-2 (K) | 1700 |
| 50 | 1782 | 1796 | 1014.5057 | 2026.9968 | 2026.9921 | 2.32 | C | 1 | 14 | 0.041 | KPQSALPDLVTQETY +dK_MMCCH-2 (K) | 1782 |
| 51 | 1801 | 1809 | 706.8389 | 1411.6632 | 1411.6595 | 2.62 | C | 0 | 15 | 0.033 | SHKTFPNPF +dK_MMCCH-2 (K) | 1803 |

Figure 29B. KLH 2 (continued)

| | | | | | | | | | | | | |
|---|---|---|---|---|---|---|---|---|---|---|---|---|
| 52 | 1985 | 1995 | 850.4291 | 1698.8436 | 1698.8399 | 2.18 | C | 0 | 31 | 0.00089 | IKQQQEADRVF +dK_MMCCH-2 (K) | 1986 |
| 53 | 2001 | 2011 | 726.8444 | 1451.6743 | 1451.6755 | -0.83 | C | 1 | 34 | 0.00042 | KGFGQSASVSF +dK_MMCCH-2 (K) | 2001 |
| 54 | 2455 | 2462 | 645.3267 | 1288.6388 | 1288.6373 | 1.16 | C | 1 | 21 | 0.0078 | KYDITQAL +dK_MMCCH-2 (K) | 2455 |
| 55 | 2463 | 2479 | 752.3476 | 2254.0210 | 2254.0253 | -1.91 | C | 1 | 14 | 0.037 | KAQSIHPEDVFDTDAPF +dK_MMCCH-2 (K) | 2463 |
| 56 | 2754 | 2760 | 598.3323 | 1194.6501 | 1194.6471 | 2.51 | C | 1 | 14 | 0.04 | KQPLKPF +dK_MMCCH-2 (K) | 2758 |
| 57 | 2865 | 2872 | 673.3655 | 1344.7164 | 1344.7112 | 3.94 | C | 1 | 14 | 0.041 | RYDITKVL +dK_MMCCH-2 (K) | 2870 |
| 58 | 3235 | 3242 | 617.3137 | 1232.6129 | 1232.6111 | 1.46 | C | 0 | 29 | 0.0014 | TSANVKIY +dK_MMCCH-2 (K) | 3240 |
| 59 | 3399 | 3406 | 576.8031 | 1151.5916 | 1151.5896 | 1.74 | C | 0 | 19 | 0.014 | IAGPTKDL +dK_MMCCH-2 (K) | 3404 |
| 60 | 69 | 75 | 604.2934 | 1206.5722 | 1206.5703 | 1.66 | Th | 0 | 17 | 0.022 | VDKHEKN +dK_MMCCH-2 (K) | 74 |
| 61 | 165 | 175 | 558.6143 | 1672.8211 | 1672.8144 | 4.01 | Th | 1 | 13 | 0.048 | FEKVQPGHHTR +dK_MMCCH-2 (K) | 167 |
| 62 | 181 | 191 | 853.3782 | 1704.7419 | 1704.7375 | 2.58 | Th | 3 | 58 | 1.60E-06 | LDALEQDEFCK +dK_MMCCH-2 (K) | 191 |
| 63 | 247 | 257 | 785.8571 | 1569.6996 | 1569.6916 | 5.10 | Th | 1 | 13 | 0.046 | LRGKDPNSADC +dK_MMCCH-2 (K) | 250 |
| 64 | 278 | 293 | 734.3531 | 2200.0374 | 2200.0371 | 0.14 | Th | 4 | 26 | 0.0027 | LTREHAKPADSFDYGR +dK_MMCCH-2 (K) | 284 |
| 65 | 365 | 372 | 585.7806 | 1169.5466 | 1169.5461 | 0.43 | Th | 1 | 50 | 9.10E-06 | LGGPTEMK +dK_MMCCH-2 (K) | 372 |
| 66 | 431 | 437 | 569.2895 | 1136.5644 | 1136.5576 | 5.98 | Th | 1 | 16 | 0.025 | FDKPPVP +dK_MMCCH-2 (K) | 433 |
| 67 | 629 | 636 | 582.7663 | 1163.5180 | 1163.5169 | 1.03 | Th | 1 | 31 | 0.00077 | VGGSEKYS +dK_MMCCH-2 (K) | 634 |
| 68 | 893 | 904 | 904.8980 | 1807.7813 | 1807.7698 | 6.36 | Th | 1 | 21 | 0.0084 | FHGEPKWCPSPE +dK_MMCCH-2 (K) | 898 |
| 69 | 1123 | 1129 | 619.2853 | 1236.5560 | 1236.5485 | 6.07 | Th | 2 | 18 | 0.014 | VFDYKNN +dK_MMCCH-2 (K) | 1127 |
| 70 | 1414 | 1419 | 564.7562 | 1127.4979 | 1127.4957 | 1.95 | Th | 1 | 17 | 0.021 | LWDNKD +dK_MMCCH-2 (K) | 1418 |
| 71 | 1525 | 1539 | 738.3549 | 2212.0428 | 2212.0371 | 2.53 | Th | 3 | 36 | 0.00024 | LKHNLPQDSFDYQNR +dK_MMCCH-2 (K) | 1526 |
| 72 | 1589 | 1605 | 735.3313 | 2202.9721 | 2202.9708 | 0.59 | Th | 2 | 15 | 0.028 | ICVEQGGEQNCKTKAGS +dK_MMCCH-2 (K) | 1600 |
| 73 | 1589 | 1605 | 735.3325 | 2202.9757 | 2202.9708 | 2.27 | Th | 2 | 39 | 0.00013 | ICVEQGGEQNCKTKAGS +dK_MMCCH-2 (K) | 1602 |
| 74 | 1800 | 1809 | 780.3741 | 1558.7337 | 1558.7279 | 3.79 | Th | 2 | 18 | 0.016 | FSHKTFPNPF +dK_MMCCH-2 (K) | 1803 |
| 75 | 1952 | 1963 | 627.2840 | 1878.8302 | 1878.8247 | 2.93 | Th | 2 | 44 | 3.90E-05 | FSKPEDTFDYHR +dK_MMCCH-2 (K) | 1954 |
| 76 | 1985 | 1994 | 776.8959 | 1551.7772 | 1551.7715 | 3.67 | Th | 2 | 22 | 0.0064 | IKQQQEADRV +dK_MMCCH-2 (K) | 1986 |
| 77 | 2193 | 2199 | 667.3012 | 1332.5877 | 1332.5849 | 2.10 | Th | 2 | 25 | 0.0034 | YWDWTKP +dK_MMCCH-2 (K) | 2198 |
| 78 | 2200 | 2206 | 562.3086 | 1122.6026 | 1122.5995 | 2.85 | Th | 2 | 22 | 0.0062 | ISKLPDL +dK_MMCCH-2 (K) | 2202 |

**Figure 29B. KLH 2 (continued)**

| | | | | | | | | | | | | |
|---|---|---|---|---|---|---|---|---|---|---|---|---|
| 79 | 2225 | 2229 | 462.2211 | 922.4277 | 922.4259 | 1.95 | Th | 2 | 23 | 0.0053 | FAKGY +dK_MMCCH-2 (K) | 2227 |
| 80 | 2230 | 2237 | 632.7936 | 1263.5726 | 1263.5693 | 2.61 | Th | 2 | 14 | 0.045 | IKSEDAYT +dK_MMCCH-2 (K) | 2231 |
| 81 | 2359 | 2363 | 509.2314 | 1016.4482 | 1016.4460 | 2.16 | Th | 1 | 26 | 0.0026 | FTKMH +dK_MMCCH-2 (K); Oxidation (M) | 2361 |
| 82 | 2941 | 2950 | 720.8408 | 1439.6670 | 1439.6602 | 4.72 | Th | 3 | 45 | 3.20E-05 | LDEANDLKNA +dK_MMCCH-2 (K) | 2948 |
| 83 | 3009 | 3015 | 573.2726 | 1144.5307 | 1144.5335 | -2.45 | Th | 0 | 14 | 0.043 | LKEHGSH +dK_MMCCH-2 (K) | 3010 |
| 84 | 3399 | 3405 | 520.2607 | 1038.5069 | 1038.5056 | 1.25 | Th | 1 | 25 | 0.0029 | IAGPTKD +dK_MMCCH-2 (K) | 3404 |

"Start" and "End" indicates the number of residue of KLH1 or KLH2. "Observed" column implies the observed m/z value in the survey scan while "Mr(expt)", "Mr(calc)", "ppm" indicate the experimental molecular weight(MW), calculated MW, and the difference between observed MW and theoretical MW respectively. In the "Enzyme" column, T, C, G and Th stand for trypsin, chymotrypsin, Glu-C and thermolysin respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the lysine conjugation site for KLH1 or KLH2.

Figure 30

Fig. 30(A) KLH 1 (1st LC-MS/MS)

| # | K site | sample | | | | # | K site | sample | | | | # | K site | sample | | | | # | K site | sample | | | |
|---|---|---|---|---|---|---|---|---|---|---|---|---|---|---|---|---|---|---|---|---|---|---|---|
| | | 1 | 2 | 3 | 4 | | | 1 | 2 | 3 | 4 | | | 1 | 2 | 3 | 4 | | | 1 | 2 | 3 | 4 |
| 1 | K 7 | | | | | 51 | K 879 | | | | | 101 | K 2033 | | | | | 151 | K 3044 | | | | |
| 2 | K 26 | | | | | 52 | K 884 | | | | | 102 | K 2066 | | | | | 152 | K 3048 | | | | |
| 3 | K 53 | | | | | 53 | K 918 | | | | | 103 | K 2067 | | | | | 153 | K 3052 | | | | |
| 4 | K 67 | | | | | 54 | K 924 | | | | | 104 | K 2076 | | | | | 154 | K 3077 | | | | |
| 5 | K 84 | | | | | 55 | K 929 | | | | | 105 | K 2099 | | | | | 155 | K 3079 | | | | |
| 6 | K 88 | | | | | 56 | K 946 | | | | | 106 | K 2102 | | | | | 156 | K 3092 | | | | |
| 7 | K 96 | | | | | 57 | K 952 | | | | | 107 | K 2104 | | | | | | total | 14 | 7 | 4 | 8 |
| 8 | K 123 | | | | | 58 | K 956 | O | O | | O | 108 | K 2131 | | | | | | | | | | |
| 9 | K 131 | | | | | 59 | K 986 | O | | | | 109 | K 2149 | | | | | | | | | | |
| 10 | K 142 | O | | | | 60 | K 1017 | | | | | 110 | K 2160 | | | | | | | | | | |
| 11 | K 161 | O | | | | 61 | K 1059 | | | | | 111 | K 2187 | | | | | | | | | | |
| 12 | K 190 | | | | | 62 | K 1060 | | | | | 112 | K 2233 | | | | | | | | | | |
| 13 | K 251 | | | | | 63 | K 1073 | | | | | 113 | K 2247 | | O | O | O | | | | | | |
| 14 | K 280 | | | | | 64 | K 1104 | O | O | O | O | 114 | K 2281 | | | | | | | | | | |
| 15 | K 289 | | | | | 65 | K 1117 | | | | | 115 | K 2302 | | | | | | | | | | |
| 16 | K 373 | | | | | 66 | K 1160 | | | | | 116 | K 2360 | O | | | O | | | | | | |
| 17 | K 389 | | | | | 67 | K 1162 | | | | | 117 | K 2460 | | | | | | | | | | |
| 18 | K 402 | | | O | O | 68 | K 1199 | | | | | 118 | K 2481 | | | | | | | | | | |
| 19 | K 411 | | | | | 69 | K 1221 | | | | | 119 | K 2506 | | | | | | | | | | |
| 20 | K 438 | | | | | 70 | K 1230 | | | | | 120 | K 2507 | | | | | | | | | | |
| 21 | K 469 | | | | | 71 | K 1270 | | | | | 121 | K 2514 | | | | | | | | | | |
| 22 | K 477 | | | | | 72 | K 1303 | | | | | 122 | K 2516 | | | | | | | | | | |
| 23 | K 488 | | | | | 73 | K 1330 | | | | | 123 | K 2531 | | | | | | | | | | |
| 24 | K 491 | | | | | 74 | K 1335 | | | | | 124 | K 2532 | | | | | | | | | | |
| 25 | K 499 | | | | | 75 | K 1338 | | | | | 125 | K 2579 | | | | | | | | | | |
| 26 | K 507 | | | | O | 76 | K 1339 | | | | | 126 | K 2601 | | | | | | | | | | |
| 27 | K 530 | | | | | 77 | K 1395 | | | | | 127 | K 2609 | | | | | | | | | | |
| 28 | K 549 | | | | | 78 | K 1402 | | | | | 128 | K 2620 | | | | | | | | | | |
| 29 | K 554 | | | | | 79 | K 1410 | | | | | 129 | K 2624 | | | | | | | | | | |
| 30 | K 557 | | | | | 80 | K 1416 | | | | | 130 | K 2637 | | | | | | | | | | |
| 31 | K 566 | | | | | 81 | K 1444 | | | | | 131 | K 2656 | | | | | | | | | | |
| 32 | K 581 | | | | | 82 | K 1473 | O | | | | 132 | K 2682 | | | | | | | | | | |
| 33 | K 587 | | | | | 83 | K 1518 | | | | | 133 | K 2686 | | | | | | | | | | |
| 34 | K 590 | O | O | | | 84 | K 1529 | | | | | 134 | K 2713 | | | | | | | | | | |
| 35 | K 606 | | | | | 85 | K 1543 | | | | | 135 | K 2714 | | | | | | | | | | |
| 36 | K 615 | | | | | 86 | K 1583 | | | | | 136 | K 2748 | | | | | | | | | | |
| 37 | K 616 | O | | | | 87 | K 1636 | | | | | 137 | K 2771 | | | | | | | | | | |
| 38 | K 671 | | | | | 88 | K 1640 | | | | | 138 | K 2814 | | | | | | | | | | |
| 39 | K 681 | | | | | 89 | K 1659 | | | | | 139 | K 2838 | | | | | | | | | | |
| 40 | K 695 | | | | | 90 | K 1668 | | | | | 140 | K 2848 | | | | | | | | | | |
| 41 | K 704 | | | | | 91 | K 1738 | | | | | 141 | K 2852 | O | O | O | O | | | | | | |
| 42 | K 719 | O | O | | | 92 | K 1749 | | | | | 142 | K 2900 | O | | | | | | | | | |
| 43 | K 787 | | | | | 93 | K 1758 | | | | | 143 | K 2924 | | | | | | | | | | |
| 44 | K 791 | | | | | 94 | K 1809 | | | | | 144 | K 2943 | | | | | | | | | | |
| 45 | K 823 | | | | | 95 | K 1826 | | | | | 145 | K 2947 | | | | | | | | | | |
| 46 | K 835 | | | | | 96 | K 1853 | | | | | 146 | K 2967 | | | | | | | | | | |
| 47 | K 857 | | | | | 97 | K 1903 | | | | | 147 | K 2996 | | | | | | | | | | |
| 48 | K 858 | | | | | 98 | K 1915 | | | | | 148 | K 3002 | | | | | | | | | | |
| 49 | K 863 | | | | | 99 | K 1924 | O | O | | O | 149 | K 3003 | O | | | | | | | | | |
| 50 | K 878 | | | | | 100 | K 2022 | | | | | 150 | K 3037 | | | | | | | | | | |

Figure 30 (continued)

Fig. 30(B) KLH 2 (1st LC-MS/MS)

| # | K site | sample | | | | # | K site | sample | | | | # | K site | sample | | | |
|---|---|---|---|---|---|---|---|---|---|---|---|---|---|---|---|---|---|
| | | 1 | 2 | 3 | 4 | | | 1 | 2 | 3 | 4 | | | 1 | 2 | 3 | 4 |
| 1 | K 26 | O | O | | | 51 | K 1127 | O | O | | | 101 | K 2198 | | | | |
| 2 | K 41 | | | | | 52 | K 1155 | | | | | 102 | K 2202 | | | | |
| 3 | K 71 | | | | | 53 | K 1176 | | | | | 103 | K 2227 | | | | |
| 4 | K 74 | | | | | 54 | K 1181 | | | | | 104 | K 2231 | | | | |
| 5 | K 104 | | | | | 55 | K 1212 | | | | | 105 | K 2317 | | | | |
| 6 | K 105 | | | | | 56 | K 1233 | | | | | 106 | K 2327 | | | | |
| 7 | K 138 | | | | | 57 | K 1280 | | | | | 107 | K 2328 | | | | |
| 8 | K 154 | | | | | 58 | K 1302 | O | | | | 108 | K 3330 | | | | |
| 9 | K 155 | | | | | 59 | K 1311 | | | | | 109 | K 2335 | | | | |
| 10 | K 167 | O | O | | O | 60 | K 1315 | | | | | 110 | K 2345 | | | | |
| 11 | K 191 | | | | | 61 | K 1368 | O | | O | | 111 | K 2361 | | | | |
| 12 | K 250 | O | O | | O | 62 | K 1418 | | | | | 112 | K 2400 | | | | |
| 13 | K 284 | O | O | | O | 63 | K 1456 | O | O | | | 113 | K 2426 | | | | |
| 14 | K 321 | | | | | 64 | K 1493 | | | | | 114 | K 2444 | | | O | |
| 15 | K 358 | | | | | 65 | K 1494 | | | | | 115 | K 2455 | | | | |
| 16 | K 372 | | | | | 66 | K 1508 | O | | | | 116 | K 2463 | | O | O | O |
| 17 | K 401 | | | | | 67 | K 1526 | O | O | O | O | 117 | K 2482 | | | | |
| 18 | K 428 | | | | | 68 | K 1556 | | | | | 118 | K 2490 | | | | |
| 19 | K 433 | O | | | O | 69 | K 1565 | | | | | 119 | K 2527 | | | | |
| 20 | K 447 | | | | | 70 | K 1600 | | | | | 120 | K 2596 | | | | |
| 21 | K 462 | | | | | 71 | K 1602 | O | | | | 121 | K 2737 | | | | |
| 22 | K 471 | O | O | | O | 72 | K 1624 | | | | | 122 | K 2754 | | | | |
| 23 | K 495 | | | | | 73 | K 1633 | | | | | 123 | K 2758 | | | | |
| 24 | K 562 | | | | | 74 | K 1646 | | | | | 124 | K 2810 | | | | |
| 25 | K 585 | | | | | 75 | K 1675 | | | | | 125 | K 2811 | | | | |
| 26 | K 589 | | | | | 76 | K 1690 | O | | | | 126 | K 2870 | | | | |
| 27 | K 634 | | | | | 77 | K 1700 | | | | | 127 | K 2873 | | | | |
| 28 | K 672 | | | | | 78 | K 1705 | | | | | 128 | K 2888 | | | | |
| 29 | K 675 | | | | | 79 | K 1708 | | | | | 129 | K 2903 | | | | |
| 30 | K 688 | | | | | 80 | K 1767 | | | | | 130 | K 2934 | | | | |
| 31 | K 701 | | | | | 81 | K 1782 | | | | | 131 | K 2948 | | | | |
| 32 | K 741 | | | | | 82 | K 1803 | | | | | 132 | K 2953 | | | | |
| 33 | K 774 | | | | | 83 | K 1811 | | | | | 133 | K 3010 | | | | |
| 34 | K 776 | | | | | 84 | K 1835 | | | | | 134 | K 3025 | | | | |
| 35 | K 787 | | | | | 85 | K 1879 | | | | | 135 | K 3045 | | | | |
| 36 | K 790 | | | | | 86 | K 1921 | | | | | 136 | K 3050 | | | | |
| 37 | K 798 | | | | | 87 | K 1931 | | | | | 137 | K 3145 | | | | |
| 38 | K 806 | | | | | 88 | K 1935 | | | | | 138 | K 3148 | | | | |
| 39 | K 848 | | | | | 89 | K 1947 | | | | | 139 | K 3149 | | | | |
| 40 | K 855 | | | | | 90 | K 1954 | O | O | O | O | 140 | K 3155 | | | | |
| 41 | K 858 | | | | | 91 | K 1986 | O | O | | O | 141 | K 3184 | | | | |
| 42 | K 882 | | | | | 92 | K 2001 | | | | | 142 | K 3193 | | | | |
| 43 | K 898 | | | | | 93 | K 2044 | | | | | 143 | K 3240 | | | | |
| 44 | K 907 | | | | | 94 | K 2049 | | | | | 144 | K 3263 | | | | |
| 45 | K 908 | | | | | 95 | K 2052 | | | | | 145 | K 3274 | | | | |
| 46 | K 936 | | | | | 96 | K 2055 | | | | | 146 | K 3321 | | | | |
| 47 | K 1011 | | | | | 97 | K 2065 | | | | | 147 | K 3336 | | | | |
| 48 | K 1079 | | | | | 98 | K 2069 | | | | | 148 | K 3340 | | | | |
| 49 | K 1083 | | | | | 99 | K 2092 | | | | | 149 | K 3344 | | | | |
| 50 | K 1097 | O | O | O | O | 100 | K 2121 | | | | | 150 | K 3404 | | | | |
| | | | | | | | | | | | | | total | 17 | 12 | 6 | 10 |

Figure 30 (continued)

Fig. 30(C) KLH 1 ($2^{nd}$ LC-MS/MS)

| # | K site | sample | | | | # | K site | sample | | | | # | K site | sample | | | | # | K site | sample | | | |
|---|---|---|---|---|---|---|---|---|---|---|---|---|---|---|---|---|---|---|---|---|---|---|---|
| | | 1 | 2 | 3 | 4 | | | 1 | 2 | 3 | 4 | | | 1 | 2 | 3 | 4 | | | 1 | 2 | 3 | 4 |
| 1 | K 7 | | | | | 51 | K 879 | | | | | 101 | K 2033 | | | | | 151 | K 3044 | | | | |
| 2 | K 26 | | | | | 52 | K 884 | | | | | 102 | K 2066 | | | | | 152 | K 3048 | | | | |
| 3 | K 53 | | | | | 53 | K 918 | | | | | 103 | K 2067 | | | | | 153 | K 3052 | | | | |
| 4 | K 67 | | | | | 54 | K 924 | | | | | 104 | K 2076 | | | | | 154 | K 3077 | | | | |
| 5 | K 84 | | | | | 55 | K 929 | | | | | 105 | K 2099 | | | | | 155 | K 3079 | | | | |
| 6 | K 88 | | | | | 56 | K 946 | | | | | 106 | K 2102 | | | | | 156 | K 3092 | | | | |
| 7 | K 96 | | | | | 57 | K 952 | | | | | 107 | K 2104 | | | | | | total | 13 | 7 | 6 | 9 |
| 8 | K 123 | | | | | 58 | K 956 | O | O | | O | 108 | K 2131 | | | | | | | | | | |
| 9 | K 131 | | | | | 59 | K 986 | | | | | 109 | K 2149 | | | | | | | | | | |
| 10 | K 142 | | | | | 60 | K 1017 | | | | | 110 | K 2160 | | | | | | | | | | |
| 11 | K 161 | O | | O | | 61 | K 1059 | | | | | 111 | K 2187 | | | | | | | | | | |
| 12 | K 190 | | | | | 62 | K 1060 | | | | | 112 | K 2233 | | | | | | | | | | |
| 13 | K 251 | | | | | 63 | K 1073 | | | | | 113 | K 2247 | O | O | O | O | | | | | | |
| 14 | K 280 | | | | | 64 | K 1104 | O | O | O | O | 114 | K 2281 | | | | | | | | | | |
| 15 | K 289 | | | | | 65 | K 1117 | | | | | 115 | K 2302 | | | | | | | | | | |
| 16 | K 373 | | | | | 66 | K 1160 | | | | | 116 | K 2360 | O | O | | | | | | | | |
| 17 | K 389 | | | | | 67 | K 1162 | | | | | 117 | K 2460 | | | | | | | | | | |
| 18 | K 402 | | | O | | 68 | K 1199 | | | | | 118 | K 2481 | | | | | | | | | | |
| 19 | K 411 | | | | | 69 | K 1221 | | | | | 119 | K 2506 | | | | | | | | | | |
| 20 | K 438 | | | | | 70 | K 1230 | | | | | 120 | K 2507 | | | | | | | | | | |
| 21 | K 469 | | | | | 71 | K 1270 | | | | | 121 | K 2514 | | | | | | | | | | |
| 22 | K 477 | | | | | 72 | K 1303 | | | | | 122 | K 2516 | | | | | | | | | | |
| 23 | K 488 | | | | | 73 | K 1330 | | | | | 123 | K 2531 | | | | | | | | | | |
| 24 | K 491 | | | | | 74 | K 1335 | | | | | 124 | K 2532 | | | | | | | | | | |
| 25 | K 499 | | | | | 75 | K 1338 | | | | | 125 | K 2579 | | | | | | | | | | |
| 26 | K 507 | | | | | 76 | K 1339 | | | | | 126 | K 2601 | | | | | | | | | | |
| 27 | K 530 | | | | | 77 | K 1395 | | | | | 127 | K 2609 | | | | | | | | | | |
| 28 | K 549 | | | | | 78 | K 1402 | | | | | 128 | K 2620 | | | | | | | | | | |
| 29 | K 554 | | | | | 79 | K 1410 | | | | | 129 | K 2624 | | | | | | | | | | |
| 30 | K 557 | | | | | 80 | K 1416 | | | | | 130 | K 2637 | | | | | | | | | | |
| 31 | K 566 | | | | | 81 | K 1444 | O | O | | | 131 | K 2656 | | | | | | | | | | |
| 32 | K 581 | | | | | 82 | K 1473 | O | | | | 132 | K 2682 | | | | | | | | | | |
| 33 | K 587 | | | | | 83 | K 1518 | | | | | 133 | K 2686 | | | | | | | | | | |
| 34 | K 590 | O | | | | 84 | K 1529 | | | | | 134 | K 2713 | | | | | | | | | | |
| 35 | K 606 | | | | O | 85 | K 1543 | | | | | 135 | K 2714 | | | | | | | | | | |
| 36 | K 615 | | | | | 86 | K 1583 | | | | O | 136 | K 2748 | | | | | | | | | | |
| 37 | K 616 | | | | | 87 | K 1636 | | | | | 137 | K 2771 | | | | | | | | | | |
| 38 | K 671 | | | | | 88 | K 1640 | | | | | 138 | K 2814 | | | | | | | | | | |
| 39 | K 681 | | | | | 89 | K 1659 | | O | | O | 139 | K 2838 | | | | | | | | | | |
| 40 | K 695 | | | | | 90 | K 1668 | | | | | 140 | K 2848 | | | | | | | | | | |
| 41 | K 704 | | | | | 91 | K 1738 | | | | | 141 | K 2852 | O | O | O | O | | | | | | |
| 42 | K 719 | O | | O | | 92 | K 1749 | | | | | 142 | K 2900 | O | | | | | | | | | |
| 43 | K 787 | | | | O | 93 | K 1758 | | | | | 143 | K 2924 | | | | | | | | | | |
| 44 | K 791 | | | | | 94 | K 1809 | | | | | 144 | K 2943 | | | | | | | | | | |
| 45 | K 823 | | | | | 95 | K 1826 | | | | | 145 | K 2947 | | | | | | | | | | |
| 46 | K 835 | | | | | 96 | K 1853 | | | | | 146 | K 2967 | | | | | | | | | | |
| 47 | K 857 | | | | | 97 | K 1903 | | | | | 147 | K 2996 | | | | | | | | | | |
| 48 | K 858 | | | | | 98 | K 1915 | | | | | 148 | K 3002 | | | | | | | | | | |
| 49 | K 863 | | | | | 99 | K 1924 | O | | | O | 149 | K 3003 | O | | | | | | | | | |
| 50 | K 878 | | | | | 100 | K 2022 | | | | | 150 | K 3037 | | | | | | | | | | |

Figure 30 (continued)

Fig. 30(D) KLH 2 (2nd LC-MS/MS)

| # | K site | sample | | | | # | K site | sample | | | | # | K site | sample | | | |
|---|---|---|---|---|---|---|---|---|---|---|---|---|---|---|---|---|---|
| | | 1 | 2 | 3 | 4 | | | 1 | 2 | 3 | 4 | | | 1 | 2 | 3 | 4 |
| 1 | K 26 | O | O | | | 51 | K 1127 | | | | | 101 | K 2198 | | | | |
| 2 | K 41 | | | | | 52 | K 1155 | | | | | 102 | K 2202 | | | | |
| 3 | K 71 | | | | | 53 | K 1176 | | | | | 103 | K 2227 | | | | |
| 4 | K 74 | | | | | 54 | K 1181 | | | | | 104 | K 2231 | | | | |
| 5 | K 104 | | | | | 55 | K 1212 | | | | | 105 | K 2317 | | | | |
| 6 | K 105 | | | | | 56 | K 1233 | | | O | O | 106 | K 2327 | | | | |
| 7 | K 138 | | | | | 57 | K 1280 | | | | | 107 | K 2328 | | | | |
| 8 | K 154 | | | | | 58 | K 1302 | O | O | | | 108 | K 3330 | | | | |
| 9 | K 155 | | | | | 59 | K 1311 | | | | | 109 | K 2335 | | | | |
| 10 | K 167 | O | O | | O | 60 | K 1315 | | | | | 110 | K 2345 | | | | |
| 11 | K 191 | | | | | 61 | K 1368 | | O | O | | 111 | K 2361 | | | | |
| 12 | K 250 | | O | | | 62 | K 1418 | | | | | 112 | K 2400 | | | | |
| 13 | K 284 | O | O | | O | 63 | K 1456 | O | | | | 113 | K 2426 | | | | |
| 14 | K 321 | | | | | 64 | K 1493 | | | | | 114 | K 2444 | | | | |
| 15 | K 358 | | | | | 65 | K 1494 | | | | | 115 | K 2455 | | | | |
| 16 | K 372 | | | | | 66 | K 1508 | | | | | 116 | K 2463 | O | O | | O |
| 17 | K 401 | | | | | 67 | K 1526 | O | O | O | | 117 | K 2482 | | | | |
| 18 | K 428 | | | | | 68 | K 1556 | O | | | | 118 | K 2490 | | | | |
| 19 | K 433 | O | O | O | | 69 | K 1565 | | | | | 119 | K 2527 | O | O | | |
| 20 | K 447 | | | | | 70 | K 1600 | | | | | 120 | K 2596 | | | | |
| 21 | K 462 | | | | | 71 | K 1602 | | | | | 121 | K 2737 | | | | O |
| 22 | K 471 | O | O | | O | 72 | K 1624 | | | | | 122 | K 2754 | | | | |
| 23 | K 495 | | | | | 73 | K 1633 | | | | | 123 | K 2758 | | | | |
| 24 | K 562 | | | | | 74 | K 1646 | | | | | 124 | K 2810 | | | | |
| 25 | K 585 | | | | | 75 | K 1675 | | | | | 125 | K 2811 | | | | |
| 26 | K 589 | | | | | 76 | K 1690 | | | | | 126 | K 2870 | | | | |
| 27 | K 634 | | | | | 77 | K 1700 | | | | | 127 | K 2873 | | | | |
| 28 | K 672 | | | | | 78 | K 1705 | | | | | 128 | K 2888 | | | | |
| 29 | K 675 | | | | | 79 | K 1708 | | | | | 129 | K 2903 | | | | |
| 30 | K 688 | | | | | 80 | K 1767 | | | | | 130 | K 2934 | | | | |
| 31 | K 701 | | | | | 81 | K 1782 | | | | | 131 | K 2948 | | | | |
| 32 | K 741 | | | | | 82 | K 1803 | | | | | 132 | K 2953 | | | | |
| 33 | K 774 | | | | | 83 | K 1811 | | | | | 133 | K 3010 | | | | |
| 34 | K 776 | | | | | 84 | K 1835 | | | | | 134 | K 3025 | O | | | |
| 35 | K 787 | | | | | 85 | K 1879 | | | | | 135 | K 3045 | | | | |
| 36 | K 790 | | | | | 86 | K 1921 | | | | | 136 | K 3050 | | | | |
| 37 | K 798 | | | | | 87 | K 1931 | | | | | 137 | K 3145 | | | | |
| 38 | K 806 | | | | | 88 | K 1935 | | | | | 138 | K 3148 | | | | |
| 39 | K 848 | | | | | 89 | K 1947 | | | | | 139 | K 3149 | | | | |
| 40 | K 855 | | | | | 90 | K 1954 | O | O | | O | 140 | K 3155 | | | | |
| 41 | K 858 | | | | | 91 | K 1986 | | O | | O | 141 | K 3184 | | | | |
| 42 | K 882 | | | | | 92 | K 2001 | | | | | 142 | K 3193 | | | | |
| 43 | K 898 | | | | O | 93 | K 2044 | | | | | 143 | K 3240 | | | | |
| 44 | K 907 | | | | | 94 | K 2049 | | | | | 144 | K 3263 | | | | |
| 45 | K 908 | | | | | 95 | K 2052 | | | | | 145 | K 3274 | | | | |
| 46 | K 936 | O | | | | 96 | K 2055 | | | | | 146 | K 3321 | | | | |
| 47 | K 1011 | | | | | 97 | K 2065 | | | | | 147 | K 3336 | | | | |
| 48 | K 1079 | | | | | 98 | K 2069 | | | | | 148 | K 3340 | | | | |
| 49 | K 1083 | | | | | 99 | K 2092 | | | | | 149 | K 3344 | | | | |
| 50 | K 1097 | O | O | O | O | 100 | K 2121 | | | | | 150 | K 3404 | | | | |
| | | | | | | | | | | | | | total | 15 | 14 | 5 | 10 |

Figure 31

Fig. 31(A) KLH 1 (1st LC-MS/MS)

| # | K site | sample | | | | # | K site | sample | | | | # | K site | sample | | | | # | K site | sample | | | |
|---|---|---|---|---|---|---|---|---|---|---|---|---|---|---|---|---|---|---|---|---|---|---|---|
| | | 1 | 2 | 3 | 4 | | | 1 | 2 | 3 | 4 | | | 1 | 2 | 3 | 4 | | | 1 | 2 | 3 | 4 |
| 1 | K 7 | O | O | O | O | 51 | K 879 | | | | | 101 | K 2033 | | | | | 151 | K 3044 | | | | |
| 2 | K 26 | | | | | 52 | K 884 | | | | | 102 | K 2066 | | | | | 152 | K 3048 | | | | |
| 3 | K 53 | O | O | O | O | 53 | K 918 | | | | | 103 | K 2067 | O | O | O | O | 153 | K 3052 | | | | |
| 4 | K 67 | O | O | O | O | 54 | K 924 | | | | | 104 | K 2076 | O | O | O | O | 154 | K 3077 | | | | |
| 5 | K 84 | O | O | O | O | 55 | K 929 | | | | | 105 | K 2099 | O | O | O | O | 155 | K 3079 | | O | | |
| 6 | K 88 | | | | | 56 | K 946 | O | O | O | | 106 | K 2102 | | O | | O | 156 | K 3092 | O | O | O | O |
| 7 | K 96 | O | | O | O | 57 | K 952 | | | | | 107 | K 2104 | | O | | O | | total | 86 | 83 | 86 | 84 |
| 8 | K 123 | O | O | O | O | 58 | K 956 | O | O | O | O | 108 | K 2131 | O | O | O | O | | | | | | |
| 9 | K 131 | O | O | O | O | 59 | K 986 | O | O | O | O | 109 | K 2149 | | | | | | | | | | |
| 10 | K 142 | | O | O | O | 60 | K 1017 | O | O | O | | 110 | K 2160 | O | O | O | | | | | | | |
| 11 | K 161 | O | O | O | O | 61 | K 1059 | O | O | | O | 111 | K 2187 | | | | | | | | | | |
| 12 | K 190 | | O | O | O | 62 | K 1060 | O | O | O | O | 112 | K 2233 | | | | | | | | | | |
| 13 | K 251 | | | | | 63 | K 1073 | O | | O | O | 113 | K 2247 | O | O | O | O | | | | | | |
| 14 | K 280 | | | | | 64 | K 1104 | O | O | O | O | 114 | K 2281 | | | | | | | | | | |
| 15 | K 289 | | | | | 65 | K 1117 | | O | | O | 115 | K 2302 | O | | | | | | | | | |
| 16 | K 373 | | | | | 66 | K 1160 | | | | | 116 | K 2360 | O | O | O | O | | | | | | |
| 17 | K 389 | O | | O | | 67 | K 1162 | O | O | O | | 117 | K 2460 | | | | | | | | | | |
| 18 | K 402 | | | | | 68 | K 1199 | O | O | O | O | 118 | K 2481 | O | O | O | O | | | | | | |
| 19 | K 411 | | | | | 69 | K 1221 | | O | | O | 119 | K 2506 | | | | | | | | | | |
| 20 | K 438 | O | O | O | O | 70 | K 1230 | O | O | O | O | 120 | K 2507 | O | O | O | O | | | | | | |
| 21 | K 469 | O | | O | O | 71 | K 1270 | | | | | 121 | K 2514 | O | O | O | O | | | | | | |
| 22 | K 477 | O | | O | O | 72 | K 1303 | | | | | 122 | K 2516 | O | O | O | O | | | | | | |
| 23 | K 488 | | | | | 73 | K 1330 | | | | | 123 | K 2531 | | | | | | | | | | |
| 24 | K 491 | O | | | | 74 | K 1335 | O | O | O | O | 124 | K 2532 | | | | | | | | | | |
| 25 | K 499 | | | | | 75 | K 1338 | | | | | 125 | K 2579 | | | | | | | | | | |
| 26 | K 507 | | | | | 76 | K 1339 | | | | | 126 | K 2601 | O | O | O | O | | | | | | |
| 27 | K 530 | O | O | O | O | 77 | K 1395 | O | O | O | O | 127 | K 2609 | O | O | O | O | | | | | | |
| 28 | K 549 | | | | | 78 | K 1402 | O | O | O | O | 128 | K 2620 | | | | | | | | | | |
| 29 | K 554 | O | O | O | O | 79 | K 1410 | O | O | O | O | 129 | K 2624 | O | O | O | O | | | | | | |
| 30 | K 557 | | | | O | 80 | K 1416 | | | | | 130 | K 2637 | O | O | O | O | | | | | | |
| 31 | K 566 | O | O | O | O | 81 | K 1444 | O | | O | | 131 | K 2656 | O | O | O | O | | | | | | |
| 32 | K 581 | O | O | O | O | 82 | K 1473 | O | O | O | O | 132 | K 2682 | | | O | | | | | | | |
| 33 | K 587 | | | | | 83 | K 1518 | O | O | O | O | 133 | K 2686 | | | O | O | | | | | | |
| 34 | K 590 | O | O | O | O | 84 | K 1529 | O | | | | 134 | K 2713 | | | | | | | | | | |
| 35 | K 606 | O | O | O | O | 85 | K 1543 | | | | | 135 | K 2714 | | | O | O | | | | | | |
| 36 | K 615 | | | | | 86 | K 1583 | O | O | O | O | 136 | K 2748 | O | O | O | | | | | | | |
| 37 | K 616 | | | | | 87 | K 1636 | | | | | 137 | K 2771 | O | O | O | O | | | | | | |
| 38 | K 671 | O | O | O | O | 88 | K 1640 | | | | | 138 | K 2814 | O | O | O | O | | | | | | |
| 39 | K 681 | O | | | | 89 | K 1659 | O | O | O | O | 139 | K 2838 | | | | | | | | | | |
| 40 | K 695 | O | O | O | O | 90 | K 1668 | O | O | O | O | 140 | K 2848 | | | | | | | | | | |
| 41 | K 704 | O | O | O | O | 91 | K 1738 | O | O | O | O | 141 | K 2852 | O | O | O | O | | | | | | |
| 42 | K 719 | O | O | O | O | 92 | K 1749 | O | O | | O | 142 | K 2900 | O | O | O | O | | | | | | |
| 43 | K 787 | O | O | O | O | 93 | K 1758 | O | O | O | O | 143 | K 2924 | | | | | | | | | | |
| 44 | K 791 | O | O | O | O | 94 | K 1809 | | | | | 144 | K 2943 | O | | O | O | | | | | | |
| 45 | K 823 | O | O | O | | 95 | K 1826 | O | O | O | O | 145 | K 2947 | O | O | O | O | | | | | | |
| 46 | K 835 | O | O | O | O | 96 | K 1853 | | | | | 146 | K 2967 | O | O | O | O | | | | | | |
| 47 | K 857 | | | | | 97 | K 1903 | O | O | O | O | 147 | K 2996 | O | O | O | O | | | | | | |
| 48 | K 858 | O | O | O | O | 98 | K 1915 | | | O | O | 148 | K 3002 | | O | | | | | | | | |
| 49 | K 863 | | O | | O | 99 | K 1924 | O | O | O | O | 149 | K 3003 | O | O | O | O | | | | | | |
| 50 | K 878 | | | | | 100 | K 2022 | | | | | 150 | K 3037 | O | | O | | | | | | | |

Figure 31 (continued)

Fig. 31(B) KLH 2 (1st LC-MS/MS)

| # | K site | sample | | | | # | K site | sample | | | | # | K site | sample | | | |
|---|---|---|---|---|---|---|---|---|---|---|---|---|---|---|---|---|---|
| | | 1 | 2 | 3 | 4 | | | 1 | 2 | 3 | 4 | | | 1 | 2 | 3 | 4 |
| 1 | K 26 | O | O | O | O | 51 | K 1127 | O | O | O | O | 101 | K 2198 | O | O | O | O |
| 2 | K 41 | O | | O | | 52 | K 1155 | | | | | 102 | K 2202 | O | O | O | O |
| 3 | K 71 | | | | | 53 | K 1176 | | | O | | 103 | K 2227 | O | O | O | O |
| 4 | K 74 | O | | O | | 54 | K 1181 | | | | | 104 | K 2231 | O | O | O | O |
| 5 | K 104 | | | | | 55 | K 1212 | | O | | O | 105 | K 2317 | | | | |
| 6 | K 105 | | | | | 56 | K 1233 | O | O | O | O | 106 | K 2327 | | | | |
| 7 | K 138 | | | | | 57 | K 1280 | | | | | 107 | K 2328 | | | | |
| 8 | K 154 | O | O | | | 58 | K 1302 | O | O | O | O | 108 | K 3330 | | | | |
| 9 | K 155 | O | O | O | O | 59 | K 1311 | O | O | O | O | 109 | K 2335 | | | | |
| 10 | K 167 | O | O | O | O | 60 | K 1315 | O | O | O | O | 110 | K 2345 | | | | |
| 11 | K 191 | O | O | O | O | 61 | K 1368 | O | O | O | O | 111 | K 2361 | O | O | O | O |
| 12 | K 250 | O | O | O | O | 62 | K 1418 | O | O | O | O | 112 | K 2400 | | | | O |
| 13 | K 284 | O | O | O | O | 63 | K 1456 | O | O | O | O | 113 | K 2426 | | O | | |
| 14 | K 321 | O | | | O | 64 | K 1493 | O | | | | 114 | K 2444 | | | | |
| 15 | K 358 | | | | | 65 | K 1494 | O | O | O | O | 115 | K 2455 | O | O | | O |
| 16 | K 372 | O | O | O | O | 66 | K 1508 | O | | | | 116 | K 2463 | O | O | O | O |
| 17 | K 401 | | | | | 67 | K 1526 | O | O | O | O | 117 | K 2482 | O | O | O | |
| 18 | K 428 | O | O | O | O | 68 | K 1556 | O | O | O | O | 118 | K 2490 | | | | |
| 19 | K 433 | O | O | O | O | 69 | K 1565 | | | | | 119 | K 2527 | O | O | O | O |
| 20 | K 447 | | | | | 70 | K 1600 | O | O | O | O | 120 | K 2596 | | | | |
| 21 | K 462 | | | | | 71 | K 1602 | O | O | O | O | 121 | K 2737 | O | O | | |
| 22 | K 471 | O | O | O | O | 72 | K 1624 | O | | | O | 122 | K 2754 | O | O | O | |
| 23 | K 495 | | O | O | O | 73 | K 1633 | | | | | 123 | K 2758 | O | O | O | O |
| 24 | K 562 | | | O | | 74 | K 1646 | O | | | | 124 | K 2810 | | | | O |
| 25 | K 585 | | | | | 75 | K 1675 | | | | | 125 | K 2811 | | | | O |
| 26 | K 589 | | | | | 76 | K 1690 | O | | | | 126 | K 2870 | O | O | O | O |
| 27 | K 634 | O | O | O | O | 77 | K 1700 | O | O | O | O | 127 | K 2873 | | | | |
| 28 | K 672 | | | | | 78 | K 1705 | | | | | 128 | K 2888 | | | | |
| 29 | K 675 | | | | | 79 | K 1708 | O | O | O | | 129 | K 2903 | | | | |
| 30 | K 688 | | | | | 80 | K 1767 | O | O | O | O | 130 | K 2934 | | | | |
| 31 | K 701 | | | | | 81 | K 1782 | O | | | | 131 | K 2948 | O | O | O | O |
| 32 | K 741 | | | | | 82 | K 1803 | O | O | O | O | 132 | K 2953 | | | | |
| 33 | K 774 | | | | | 83 | K 1811 | O | O | | | 133 | K 3010 | O | O | O | O |
| 34 | K 776 | | | | | 84 | K 1835 | | | | | 134 | K 3025 | | O | O | |
| 35 | K 787 | | | | | 85 | K 1879 | | | | | 135 | K 3045 | | | | |
| 36 | K 790 | O | | | | 86 | K 1921 | | | | | 136 | K 3050 | | | | |
| 37 | K 798 | | | | | 87 | K 1931 | | | | | 137 | K 3145 | | | | |
| 38 | K 806 | | | | | 88 | K 1935 | | | | | 138 | K 3148 | | | | |
| 39 | K 848 | | | | | 89 | K 1947 | | | | | 139 | K 3149 | O | O | O | O |
| 40 | K 855 | | | | | 90 | K 1954 | O | O | O | O | 140 | K 3155 | | | | |
| 41 | K 858 | | | | | 91 | K 1986 | O | O | O | O | 141 | K 3184 | | | | |
| 42 | K 882 | | | | | 92 | K 2001 | O | O | O | | 142 | K 3193 | | | | |
| 43 | K 898 | O | O | O | O | 93 | K 2044 | | | | | 143 | K 3240 | O | O | O | O |
| 44 | K 907 | | | | | 94 | K 2049 | O | | | | 144 | K 3263 | | | | |
| 45 | K 908 | | | | | 95 | K 2052 | | | O | | 145 | K 3274 | | | | |
| 46 | K 936 | O | O | O | O | 96 | K 2055 | | | | | 146 | K 3321 | | | | |
| 47 | K 1011 | O | | O | | 97 | K 2065 | | | O | | 147 | K 3336 | | | | |
| 48 | K 1079 | O | O | O | O | 98 | K 2069 | | | | | 148 | K 3340 | | | | |
| 49 | K 1083 | O | O | O | O | 99 | K 2092 | | | | | 149 | K 3344 | | | | |
| 50 | K 1097 | O | O | O | O | 100 | K 2121 | O | | | | 150 | K 3404 | O | O | O | O |
| | | | | | | | | | | | | | total | 69 | 60 | 61 | 56 |

Figure 31 (continued)

Fig. 31(C) KLH 1 ($2^{nd}$ LC-MS/MS)

| # | K site | sample | | | |
|---|---|---|---|---|---|
| | | 1 | 2 | 3 | 4 |
| 1 | K 7 | O | O | O | O |
| 2 | K 26 | | | | |
| 3 | K 53 | O | O | O | O |
| 4 | K 67 | O | O | O | O |
| 5 | K 84 | O | O | O | O |
| 6 | K 88 | | | | |
| 7 | K 96 | O | O | O | |
| 8 | K 123 | O | O | O | O |
| 9 | K 131 | O | O | O | O |
| 10 | K 142 | O | O | O | O |
| 11 | K 161 | O | O | O | O |
| 12 | K 190 | | O | | O |
| 13 | K 251 | | | | |
| 14 | K 280 | O | | O | |
| 15 | K 289 | | | | |
| 16 | K 373 | | | | |
| 17 | K 389 | | | | |
| 18 | K 402 | | | | |
| 19 | K 411 | | | | |
| 20 | K 438 | O | O | O | O |
| 21 | K 469 | O | | O | |
| 22 | K 477 | O | | O | |
| 23 | K 488 | | | | |
| 24 | K 491 | | | | |
| 25 | K 499 | | | | |
| 26 | K 507 | | | | |
| 27 | K 530 | O | O | O | O |
| 28 | K 549 | | | | |
| 29 | K 554 | O | O | O | O |
| 30 | K 557 | | | O | |
| 31 | K 566 | O | O | O | O |
| 32 | K 581 | O | O | O | O |
| 33 | K 587 | | | | |
| 34 | K 590 | O | O | O | O |
| 35 | K 606 | O | O | O | O |
| 36 | K 615 | | | | |
| 37 | K 616 | | | | |
| 38 | K 671 | O | O | O | O |
| 39 | K 681 | | O | | |
| 40 | K 695 | O | O | O | O |
| 41 | K 704 | O | O | O | O |
| 42 | K 719 | O | O | O | O |
| 43 | K 787 | O | O | O | O |
| 44 | K 791 | O | O | O | O |
| 45 | K 823 | | | O | |
| 46 | K 835 | O | O | O | O |
| 47 | K 857 | | | | |
| 48 | K 858 | O | O | O | O |
| 49 | K 863 | | | | O |
| 50 | K 878 | | | | |

| # | K site | sample | | | |
|---|---|---|---|---|---|
| | | 1 | 2 | 3 | 4 |
| 51 | K 879 | | | O | O |
| 52 | K 884 | | | | |
| 53 | K 918 | | O | | |
| 54 | K 924 | | O | O | |
| 55 | K 929 | | | | |
| 56 | K 946 | O | | O | O |
| 57 | K 952 | | | | |
| 58 | K 956 | O | O | O | O |
| 59 | K 986 | O | O | O | O |
| 60 | K 1017 | | | O | |
| 61 | K 1059 | O | O | | |
| 62 | K 1060 | O | O | O | O |
| 63 | K 1073 | O | | | |
| 64 | K 1104 | O | O | O | O |
| 65 | K 1117 | O | | O | |
| 66 | K 1160 | | | | |
| 67 | K 1162 | O | O | O | |
| 68 | K 1199 | O | O | O | O |
| 69 | K 1221 | | O | | O |
| 70 | K 1230 | O | O | O | O |
| 71 | K 1270 | | | | |
| 72 | K 1303 | | | | |
| 73 | K 1330 | | | | |
| 74 | K 1335 | O | O | O | O |
| 75 | K 1338 | | | | |
| 76 | K 1339 | | | | |
| 77 | K 1395 | O | O | O | O |
| 78 | K 1402 | | O | O | O |
| 79 | K 1410 | O | O | O | O |
| 80 | K 1416 | | | | |
| 81 | K 1444 | | O | O | O |
| 82 | K 1473 | O | O | O | O |
| 83 | K 1518 | O | O | O | O |
| 84 | K 1529 | | | | O |
| 85 | K 1543 | | | | |
| 86 | K 1583 | O | O | O | O |
| 87 | K 1636 | | | | |
| 88 | K 1640 | | | | |
| 89 | K 1659 | O | O | O | O |
| 90 | K 1668 | O | O | O | O |
| 91 | K 1738 | O | O | O | O |
| 92 | K 1749 | | O | | O |
| 93 | K 1758 | O | O | O | O |
| 94 | K 1809 | | | | |
| 95 | K 1826 | O | O | O | O |
| 96 | K 1853 | | | | |
| 97 | K 1903 | O | O | O | O |
| 98 | K 1915 | | | O | |
| 99 | K 1924 | O | O | O | O |
| 100 | K 2022 | | | | |

| # | K site | sample | | | |
|---|---|---|---|---|---|
| | | 1 | 2 | 3 | 4 |
| 101 | K 2033 | | | | |
| 102 | K 2066 | | | | |
| 103 | K 2067 | O | O | O | O |
| 104 | K 2076 | O | | O | O |
| 105 | K 2099 | O | O | O | O |
| 106 | K 2102 | | O | O | O |
| 107 | K 2104 | | O | O | O |
| 108 | K 2131 | O | O | O | O |
| 109 | K 2149 | | | | |
| 110 | K 2160 | O | | | |
| 111 | K 2187 | | | | |
| 112 | K 2233 | | | | |
| 113 | K 2247 | O | O | O | O |
| 114 | K 2281 | | | | |
| 115 | K 2302 | O | O | | O |
| 116 | K 2360 | O | O | O | O |
| 117 | K 2460 | | | | |
| 118 | K 2481 | O | O | O | O |
| 119 | K 2506 | | | | |
| 120 | K 2507 | O | O | O | O |
| 121 | K 2514 | O | O | O | O |
| 122 | K 2516 | O | O | O | O |
| 123 | K 2531 | | | | |
| 124 | K 2532 | | | | |
| 125 | K 2579 | | | | |
| 126 | K 2601 | O | O | O | O |
| 127 | K 2609 | O | O | O | O |
| 128 | K 2620 | | | | |
| 129 | K 2624 | O | O | O | O |
| 130 | K 2637 | O | O | O | O |
| 131 | K 2656 | O | O | O | O |
| 132 | K 2682 | | | | |
| 133 | K 2686 | O | | O | |
| 134 | K 2713 | | | | |
| 135 | K 2714 | | | O | |
| 136 | K 2748 | O | O | | |
| 137 | K 2771 | O | O | O | O |
| 138 | K 2814 | O | O | O | O |
| 139 | K 2838 | | | | |
| 140 | K 2848 | | | | |
| 141 | K 2852 | O | O | O | O |
| 142 | K 2900 | O | O | O | O |
| 143 | K 2924 | | | | |
| 144 | K 2943 | | O | O | |
| 145 | K 2947 | O | O | O | O |
| 146 | K 2967 | O | O | O | O |
| 147 | K 2996 | O | O | O | O |
| 148 | K 3002 | | | | |
| 149 | K 3003 | O | O | O | O |
| 150 | K 3037 | | | O | |

| # | K site | sample | | | |
|---|---|---|---|---|---|
| | | 1 | 2 | 3 | 4 |
| 151 | K 3044 | | | | |
| 152 | K 3048 | | | | |
| 153 | K 3052 | | | | |
| 154 | K 3077 | | | | |
| 155 | K 3079 | O | | | |
| 156 | K 3092 | O | O | | O |
| | total | 80 | 81 | 86 | 78 |

Figure 31 (continued)

Fig. 31(D) KLH 2 ($2^{nd}$ LC-MS/MS)

| # | K site | sample | | | | # | K site | sample | | | | # | K site | sample | | | |
|---|---|---|---|---|---|---|---|---|---|---|---|---|---|---|---|---|---|
| | | 1 | 2 | 3 | 4 | | | 1 | 2 | 3 | 4 | | | 1 | 2 | 3 | 4 |
| 1 | K 26 | O | O | O | O | 51 | K 1127 | O | O | O | O | 101 | K 2198 | O | O | O | O |
| 2 | K 41 | | | | O | 52 | K 1155 | | | | | 102 | K 2202 | O | O | O | O |
| 3 | K 71 | | | | | 53 | K 1176 | | | O | | 103 | K 2227 | O | O | O | O |
| 4 | K 74 | | | | O | 54 | K 1181 | | | | | 104 | K 2231 | O | O | O | O |
| 5 | K 104 | | | | | 55 | K 1212 | | O | | | 105 | K 2317 | | | | |
| 6 | K 105 | | | | | 56 | K 1233 | O | O | O | O | 106 | K 2327 | | | | |
| 7 | K 138 | | | | | 57 | K 1280 | | | | | 107 | K 2328 | | | | |
| 8 | K 154 | | | | | 58 | K 1302 | O | O | O | O | 108 | K 3330 | | | | |
| 9 | K 155 | O | O | O | O | 59 | K 1311 | O | | | O | 109 | K 2335 | | | | |
| 10 | K 167 | O | O | O | O | 60 | K 1315 | O | O | O | O | 110 | K 2345 | | | | |
| 11 | K 191 | O | O | O | O | 61 | K 1368 | O | O | O | O | 111 | K 2361 | | O | | O |
| 12 | K 250 | O | O | O | O | 62 | K 1418 | O | O | O | O | 112 | K 2400 | | | | O |
| 13 | K 284 | O | O | O | O | 63 | K 1456 | O | O | O | O | 113 | K 2426 | | | | |
| 14 | K 321 | | | O | O | 64 | K 1493 | O | | | | 114 | K 2444 | | | | |
| 15 | K 358 | | | | | 65 | K 1494 | O | O | O | O | 115 | K 2455 | O | O | | O |
| 16 | K 372 | O | O | O | O | 66 | K 1508 | O | | | | 116 | K 2463 | O | O | O | O |
| 17 | K 401 | | | | | 67 | K 1526 | O | O | O | O | 117 | K 2482 | O | O | O | O |
| 18 | K 428 | O | | O | O | 68 | K 1556 | O | O | O | O | 118 | K 2490 | | | | |
| 19 | K 433 | O | O | O | O | 69 | K 1565 | | | | | 119 | K 2527 | O | O | O | O |
| 20 | K 447 | | | | | 70 | K 1600 | O | O | O | O | 120 | K 2596 | | | | |
| 21 | K 462 | O | | | | 71 | K 1602 | O | O | O | O | 121 | K 2737 | O | O | | O |
| 22 | K 471 | O | O | O | O | 72 | K 1624 | | | | O | 122 | K 2754 | O | | O | |
| 23 | K 495 | | O | | O | 73 | K 1633 | | | O | | 123 | K 2758 | O | O | O | O |
| 24 | K 562 | O | | | | 74 | K 1646 | | | | | 124 | K 2810 | | | | |
| 25 | K 585 | | | | | 75 | K 1675 | | | | | 125 | K 2811 | O | | | |
| 26 | K 589 | | | | | 76 | K 1690 | | | | | 126 | K 2870 | O | O | O | O |
| 27 | K 634 | O | O | O | O | 77 | K 1700 | O | O | O | O | 127 | K 2873 | | | | |
| 28 | K 672 | | | | | 78 | K 1705 | | | | | 128 | K 2888 | | | | |
| 29 | K 675 | | | | | 79 | K 1708 | O | O | O | | 129 | K 2903 | | | | |
| 30 | K 688 | | | | | 80 | K 1767 | O | O | O | | 130 | K 2934 | | | | |
| 31 | K 701 | | | | | 81 | K 1782 | | O | | O | 131 | K 2948 | O | O | O | O |
| 32 | K 741 | | | | | 82 | K 1803 | O | O | O | O | 132 | K 2953 | | | | |
| 33 | K 774 | | | | | 83 | K 1811 | | O | | | 133 | K 3010 | O | O | O | O |
| 34 | K 776 | | | | | 84 | K 1835 | | | | | 134 | K 3025 | | | O | |
| 35 | K 787 | | | | | 85 | K 1879 | | | | | 135 | K 3045 | | | | |
| 36 | K 790 | | | | | 86 | K 1921 | | | | | 136 | K 3050 | | | | |
| 37 | K 798 | | | | | 87 | K 1931 | | | | | 137 | K 3145 | | | | |
| 38 | K 806 | | | | | 88 | K 1935 | | | | | 138 | K 3148 | | | | |
| 39 | K 848 | | | | | 89 | K 1947 | | | | | 139 | K 3149 | O | O | O | |
| 40 | K 855 | | | | | 90 | K 1954 | O | O | O | O | 140 | K 3155 | | | | |
| 41 | K 858 | | | | | 91 | K 1986 | O | O | O | O | 141 | K 3184 | | | | |
| 42 | K 882 | | | | | 92 | K 2001 | O | O | O | O | 142 | K 3193 | | | | |
| 43 | K 898 | O | O | O | O | 93 | K 2044 | | | | | 143 | K 3240 | O | O | O | O |
| 44 | K 907 | | | | | 94 | K 2049 | O | O | O | | 144 | K 3263 | | | | |
| 45 | K 908 | | | O | | 95 | K 2052 | | | | | 145 | K 3274 | | | | |
| 46 | K 936 | O | O | O | O | 96 | K 2055 | | | | | 146 | K 3321 | | | | |
| 47 | K 1011 | O | | | O | 97 | K 2065 | | | O | | 147 | K 3336 | | | | |
| 48 | K 1079 | O | O | O | O | 98 | K 2069 | | | | | 148 | K 3340 | | | | |
| 49 | K 1083 | O | O | O | O | 99 | K 2092 | | | | | 149 | K 3344 | | | | |
| 50 | K 1097 | O | O | O | O | 100 | K 2121 | O | | | | 150 | K 3404 | O | O | O | O |
| | | | | | | | | | | | | | total | 61 | 56 | 57 | 58 |

## Figure 32

### Figure 32A. The summary of Globo-H conjugation analysis in the first LC-MS/MS run.

| Sample | 1 | | | 2 | | | 3 | | | 4 | | |
|---|---|---|---|---|---|---|---|---|---|---|---|---|
| KLH1/KLH2 | KLH1 | KLH2 | total | KLH1 | KLH2 | total | KLH1 | KLH2 | total | KLH1 | KLH2 | total |
| Total K | 156 | 150 | 306 | 156 | 150 | 306 | 156 | 150 | 306 | 156 | 150 | 306 |
| Globo H-conjugated K | 14 | 17 | 31 | 7 | 12 | 19 | 4 | 6 | 10 | 8 | 10 | 18 |
| MMCCH-conjugated K | 86 | 69 | 155 | 83 | 60 | 143 | 86 | 61 | 147 | 84 | 56 | 140 |

### Figure 32B. The summary of Globo-H conjugation analysis in the second LC-MS/MS run.

| Sample | 1 | | | 2 | | | 3 | | | 4 | | |
|---|---|---|---|---|---|---|---|---|---|---|---|---|
| KLH1/KLH2 | KLH1 | KLH2 | total | KLH1 | KLH2 | total | KLH1 | KLH2 | total | KLH1 | KLH2 | total |
| Total K | 156 | 150 | 306 | 156 | 150 | 306 | 156 | 150 | 306 | 156 | 150 | 306 |
| Globo H-conjugated K | 13 | 15 | 28 | 7 | 14 | 21 | 6 | 5 | 11 | 9 | 10 | 19 |
| MMCCH-conjugated K | 80 | 61 | 141 | 81 | 56 | 137 | 86 | 57 | 143 | 78 | 58 | 136 |

The "Total K" indicates the numbers of lysine residues from KLH1 and KLH2 sequences. The "Globo H-conjugated K" indicates the numbers of identified Globo H conjugated lysines. The "MMCCH-conjugated K" indicates the numbers of identified MMCCH conjugated lysines.

Figure 33

Globo H and MMCCH derivative analysis on HNPQR residues

| Sample | LC-MS/MS |
|---|---|
| 1 | Figure 34 |
| 2 | Figure 35 |
| 3 | Figure 36 |
| 4 | Figure 37 |

**Figure 34. Identification details of Globo H and MMCCH-conjugated peptides on HNPQR residues for sample 1**

**Figure 34A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 1036 | 1047 | 633.3067 | 1896.8983 | 1896.9028 | -2.43 | Th | 3 | 42 | 7.20E-05 | VHGMPTFPHWHR +GMd_NL997 (HKNPQR) | H1037 |
| 2 | 193 | 208 | 802.6955 | 2405.0647 | 2405.0479 | 6.99 | T | 0 | 18 | 0.017 | YACCVHGMPIFPHWHR +Md_deamidated (HKNPQR) | H198 |
| 3 | 157 | 166 | 548.9234 | 1643.7484 | 1643.7402 | 4.99 | T | 1 | 19 | 0.012 | EAFHKFQEDR +Md_deamidated (HKNPQR) | H160 |
| 4 | 1923 | 1932 | 756.3790 | 1510.7434 | 1510.7425 | 0.66 | T | 1 | 18 | 0.015 | NKVMPNPFAR +Md_deamidated (HKNPQR) | N1923 |
| 5 | 1097 | 1110 | 999.9608 | 1997.9070 | 1997.9054 | 0.85 | G | 1 | 16 | 0.027 | TNPFHHGKITHENE +Md_deamidated (HKNPQR) | H1101 |
| 6 | 686 | 692 | 578.7572 | 1155.4998 | 1155.5019 | -1.82 | Th | 1 | 13 | 0.049 | FDGHIDT +OBI822_Md (HKNPQR) | H689 |

**Figure 34B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 2541 | 2556 | 856.3899 | 2566.1479 | 2566.1403 | 2.96 | C | 1 | 32 | 0.00058 | RHALQSVMDDDGPNGF +Oxidation (M); 2 GMd_NL997 (HKNPQR) | P2553, N2554 |
| 2 | 3243 | 3256 | 657.3114 | 1968.9124 | 1968.9000 | 6.30 | C | 1 | 16 | 0.023 | LVREGHDDENVGSF +GMd_NL997 (HKNPQR) | N3252 |
| 3 | 1330 | 1341 | 633.3067 | 1896.8983 | 1896.9028 | -2.43 | Th | 3 | 42 | 7.20E-05 | VHGMPTFPHWHR +GMd_NL997 (HKNPQR) | H1331 |
| 4 | 281 | 293 | 915.9143 | 1829.8140 | 1829.8043 | 5.36 | T | 0 | 16 | 0.026 | EHAKPADSFDYGR +Md_deamidated (HKNPQR) | H282 |
| 5 | 496 | 513 | 880.3888 | 2638.1446 | 2638.1239 | 7.85 | T | 1 | 33 | 0.00051 | DRFACCVHGMATFPHWHR +Oxidation (M); Md_deamidated (HKNPQR) | H503 |
| 6 | 2755 | 2764 | 763.8912 | 1525.7678 | 1525.7599 | 5.24 | T | 0 | 33 | 0.00054 | QPLKPFSESR +Md_deamidated (HKNPQR) | P2756 |
| 7 | 466 | 481 | 1098.0108 | 2194.0070 | 2194.0001 | 3.19 | G | 2 | 18 | 0.036 | RFQNDKSVDGYQATVE +Md_deamidated (HKNPQR) | Q477 |
| 8 | 277 | 289 | 608.9613 | 1823.8621 | 1823.8512 | 5.92 | C | 1 | 15 | 0.033 | DLTREHAKPADSF +Md_deamidated (HKNPQR) | P285 |
| 9 | 1383 | 1394 | 705.3279 | 2112.9619 | 2112.9510 | 5.16 | C | 1 | 20 | 0.023 | NSRSQTFDPNPF +2 OBI822_Md (HKNPQR) | P1391, N1392 |
| 10 | 1383 | 1394 | 705.3276 | 2112.9610 | 2112.9510 | 4.73 | C | 1 | 19 | 0.028 | NSRSQTFDPNPF +2 OBI822_Md (HKNPQR) | N1392, P1393 |
| 11 | 278 | 288 | 781.8885 | 1561.7624 | 1561.7559 | 4.23 | Th | 2 | 13 | 0.092 | LTREHAKPADS +Md_deamidated (HKNPQR) | P285 |
| 12 | 913 | 924 | 609.3028 | 1824.8866 | 1824.8817 | 2.69 | Th | 5 | 39 | 0.00038 | VHGMAVFPHWHR +OBI822_Md (HKNPQR) | H914 |

"Start" and "End" indicate the number of residue of KLH1 or KLH2. "Observed" column implies the observed m/z value in the survey scan while "Mr(expt)", "Mr(calc)", "ppm" indicate the experimental molecular weight (MW), calculated MW, and the difference between observed MW and theoretical MW, respectively. In the "Enzyme" column, T, Th, G and C indicate trypsin, thermolysin, endoproteinase Glu-C and chymotrypsin, respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the HNPQR conjugation site for KLH1 or KLH2 sequence.

**Figure 35. Identification details of Globo H and MMCCH-conjugated peptides on HNPQR residues for sample 2**

**Figure 35A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 1187 | 1196 | 862.9495 | 1723.8844 | 1723.8868 | -1.39 | T | 0 | 14 | 0.037 | IWAIWQDLQR +GMd_NL997 (HKNPQR) | R1196 |
| 2 | 1036 | 1047 | 633.3056 | 1896.8950 | 1896.9028 | -4.16 | Th | 3 | 43 | 6.60E-05 | VHGMPTFPHWHR +GMd_NL997 (HKNPQR) | H1037 |
| 3 | 157 | 166 | 548.9238 | 1643.7496 | 1643.7402 | 5.72 | T | 1 | 23 | 0.0053 | EAFHKFQEDR +Md_deamidated (HKNPQR) | H160 |
| 4 | 193 | 208 | 802.6948 | 2405.0626 | 2405.0479 | 6.11 | T | 0 | 24 | 0.0039 | YACCVHGMPIFPHWHR +Md_deamidated (HKNPQR) | H198 |
| 5 | 360 | 370 | 870.9418 | 1739.8690 | 1739.8527 | 9.37 | T | 0 | 17 | 0.02 | LWAVWQALQMR +Deamidated (NQ); Md_deamidated (HKNPQR) | R370 |
| 6 | 1092 | 1114 | 1055.4976 | 3163.4710 | 3163.4682 | 0.85 | T | 1 | 21 | 0.0092 | QHHYETNPFHHGKITHENEITTR +Md_deamidated (HKNPQR) | H1101 |
| 7 | 1092 | 1114 | 1055.4982 | 3163.4728 | 3163.4682 | 1.42 | T | 1 | 26 | 0.003 | QHHYETNPFHHGKITHENEITTR +Md_deamidated (HKNPQR) | H1102 |
| 8 | 1923 | 1932 | 756.3809 | 1510.7472 | 1510.7425 | 3.18 | T | 1 | 25 | 0.0033 | NKVMPNPFAR +Md_deamidated (HKNPQR) | N1923 |
| 9 | 2515 | 2520 | 571.7600 | 1141.5054 | 1141.5074 | -1.66 | T | 1 | 15 | 0.03 | QKEEDR +Md_deamidated (HKNPQR) | Q2515 |
| 10 | 2647 | 2661 | 715.6796 | 2144.0170 | 2144.0095 | 3.45 | G | 4 | 25 | 0.0029 | AEELRDALYKLQNDE +Md_deamidated (HKNPQR) | N2659 |
| 11 | 1733 | 1741 | 664.3222 | 1326.6298 | 1326.6312 | -0.98 | Th | 2 | 25 | 0.0029 | VLGGEKEMP +Oxidation (M); OBI822_Md (HKNPQR) | P1741 |

**Figure 35B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 1481 | 1490 | 862.9495 | 1723.8844 | 1723.8868 | -1.39 | T | 0 | 14 | 0.037 | IWAIWQDLQR +GMd_NL997 (HKNPQR) | R1490 |
| 2 | 1948 | 1963 | 950.4320 | 2848.2742 | 2848.2949 | -7.27 | T | 1 | 23 | 0.0046 | RTQEFSKPEDTFDYHR +Deamidated (NQ); 2 GMd_NL997 (HKNPQR) | R1948 |
| 3 | 3243 | 3256 | 657.3103 | 1968.9091 | 1968.9000 | 4.62 | C | 1 | 18 | 0.015 | LVREGHDDENVGSF +GMd_NL997 (HKNPQR) | N3252 |
| 4 | 1330 | 1341 | 633.3056 | 1896.8950 | 1896.9028 | -4.16 | Th | 3 | 43 | 6.60E-05 | VHGMPTFPHWHR +GMd_NL997 (HKNPQR) | H1331 |
| 5 | 3243 | 3256 | 657.3101 | 1968.9085 | 1968.9000 | 4.32 | Th | 3 | 18 | 0.028 | LVREGHDDENVGSF +GMd_NL997 (HKNPQR) | N3252 |
| 6 | 159 | 175 | 781.7303 | 2342.1691 | 2342.1590 | 4.31 | T | 2 | 19 | 0.012 | AVDDRLFEKVQPGHHTR +Md_deamidated (HKNPQR) | R163 |
| 7 | 281 | 293 | 915.9102 | 1829.8058 | 1829.8043 | 0.87 | T | 0 | 52 | 6.10E-06 | EHAKPADSFDYGR +Md_deamidated (HKNPQR) | H282 |
| 8 | 496 | 513 | 880.3886 | 2638.1440 | 2638.1239 | 7.62 | T | 1 | 35 | 0.00033 | DRFACCVHGMATFPHWHR +Oxidation (M); Md_deamidated (HKNPQR) | H503 |
| 9 | 2755 | 2764 | 763.8908 | 1525.7670 | 1525.7599 | 4.72 | T | 0 | 41 | 8.00E-05 | QPLKPFSESR +Md_deamidated (HKNPQR) | P2756 |
| 10 | 466 | 481 | 1098.0066 | 2193.9986 | 2194.0001 | -0.64 | G | 2 | 17 | 0.049 | RFQNDKSVDGYQATVE +Md_deamidated (HKNPQR) | Q477 |
| 11 | 913 | 924 | 609.3012 | 1824.8818 | 1824.8817 | 0.05 | Th | 5 | 20 | 0.031 | VHGMAVFPHWHR +OBI822_Md (HKNPQR) | H914 |

"Start" and "End" indicate the number of residue of KLH1 or KLH2. "Observed" column implies the observed m/z value in the survey scan while "Mr(expt)", "Mr(calc)", "ppm" indicate the experimental molecular weight (MW), calculated MW, and the difference between observed MW and theoretical MW, respectively. In the "Enzyme" column, T, Th, G and C indicate trypsin, thermolysin, endoproteinase Glu-C and chymotrypsin, respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the HNPQR conjugation site for KLH1 or KLH2 sequence.

**Figure 36. Identification details of Globo H and MMCCH-conjugated peptides on HNPQR residues for sample 3**

**Figure 36A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 1036 | 1047 | 633.3054 | 1896.8944 | 1896.9028 | -4.48 | Th | 3 | 43 | 6.80E-05 | VHGMPTFPHWHR +GMd_NL997 (HKNPQR) | H1037 |
| 2 | 2754 | 2761 | 678.8257 | 1355.6368 | 1355.6503 | -9.96 | Th | 1 | 22 | 0.0058 | INQDTVRD +GMd_NL997 (HKNPQR) | R2760 |
| 3 | 157 | 166 | 548.9228 | 1643.7466 | 1643.7402 | 3.89 | T | 1 | 23 | 0.0047 | EAFHKFQEDR +Md_deamidated (HKNPQR) | H160 |
| 4 | 193 | 208 | 802.6941 | 2405.0605 | 2405.0479 | 5.24 | T | 0 | 16 | 0.025 | YACCVHGMPIFPHWHR +Md_deamidated (HKNPQR) | H198 |
| 5 | 2515 | 2520 | 571.7623 | 1141.5100 | 1141.5074 | 2.37 | T | 1 | 23 | 0.0045 | QKEEDR +Md_deamidated (HKNPQR) | Q2515 |
| 6 | 2625 | 2636 | 743.0091 | 2226.0055 | 2226.0099 | -1.98 | T | 0 | 28 | 0.0017 | HHEDHHEDILVR +OBI822_Md (HKNPQR); Md_deamidated (HKNPQR) | H2625, H2626 |
| 7 | 2647 | 2661 | 715.6772 | 2144.0098 | 2144.0095 | 0.09 | G | 4 | 32 | 0.00069 | AEELRDALYKLQNDE +Md_deamidated (HKNPQR) | Q2658 |
| 8 | 160 | 171 | 606.9428 | 1817.8066 | 1817.8043 | 1.27 | C | 1 | 20 | 0.0095 | HKFQEDRSVDGY +Md_deamidated (HKNPQR) | H160 |
| 9 | 944 | 955 | 821.4324 | 1640.8502 | 1640.8484 | 1.16 | C | 3 | 35 | 0.00035 | DLKPASLGKDLF +Md_deamidated (HKNPQR) | P947 |
| 10 | 1649 | 1669 | 979.4319 | 2935.2739 | 2935.2759 | -0.72 | C | 4 | 31 | 0.0025 | NLNDHTHDFSKPEDTFDYQKF +Md_deamidated (HKNPQR) | Q1667 |
| 11 | 2160 | 2184 | 1085.5138 | 3253.5196 | 3253.5179 | 0.52 | C | 3 | 21 | 0.01 | KYDITHALHDAHITPEDVFHPSEPF +Md_deamidated (HKNPQR) | H2165 |
| 12 | 686 | 692 | 578.7544 | 1155.4942 | 1155.5019 | -6.66 | Th | 1 | 14 | 0.04 | FDGHIDT +OBI822_Md (HKNPQR) | H689 |
| 13 | 1733 | 1741 | 664.3258 | 1326.6370 | 1326.6312 | 4.45 | Th | 2 | 24 | 0.0036 | VLGGEKEMP +Oxidation (M); OBI822_Md (HKNPQR) | P1741 |

**Figure 36B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 3243 | 3256 | 657.3107 | 1968.9103 | 1968.9000 | 5.23 | C | 1 | 17 | 0.021 | LVREGHDDENVGSF +GMd_NL997 (HKNPQR) | N3252 |
| 2 | 1330 | 1341 | 633.3054 | 1896.8944 | 1896.9028 | -4.48 | Th | 3 | 43 | 6.80E-05 | VHGMPTFPHWHR +GMd_NL997 (HKNPQR) | H1331 |
| 3 | 3243 | 3256 | 657.3104 | 1968.9094 | 1968.9000 | 4.77 | Th | 3 | 25 | 0.0055 | LVREGHDDENVGSF +GMd_NL997 (HKNPQR) | N3252 |
| 4 | 159 | 175 | 781.7293 | 2342.1661 | 2342.1590 | 3.03 | T | 2 | 20 | 0.0096 | AVDDRLFEKVQPGHHTR +Md_deamidated (HKNPQR) | R163 |
| 5 | 281 | 293 | 915.9102 | 1829.8058 | 1829.8043 | 0.87 | T | 0 | 44 | 4.50E-05 | EHAKPADSFDYGR +Md_deamidated (HKNPQR) | H282 |
| 6 | 3275 | 3295 | 987.4552 | 2959.3438 | 2959.3494 | -1.89 | T | 1 | 16 | 0.026 | YDITEVANRLNMHHDDTFNFR +OBI822_Md (HKNPQR) | N3285 |
| 7 | 277 | 291 | 701.6560 | 2101.9462 | 2101.9415 | 2.24 | C | 2 | 15 | 0.048 | DLTREHAKPADSFDY +Md_deamidated (HKNPQR) | P285 |
| 8 | 1383 | 1394 | 705.3264 | 2112.9574 | 2112.9510 | 3.03 | C | 1 | 25 | 0.0079 | NSRSQTFDPNPF +2 OBI822_Md (HKNPQR) | N1392, P1393 |
| 9 | 913 | 924 | 609.3025 | 1824.8857 | 1824.8817 | 2.19 | Th | 5 | 23 | 0.014 | VHGMAVFPHWHR +OBI822_Md (HKNPQR) | H914 |

"Start" and "End" indicate the number of residue of KLH1 or KLH2. "Observed" column implies the observed m/z value in the survey scan while "Mr(expt)", "Mr(calc)", "ppm" indicate the experimental molecular weight (MW), calculated MW, and the difference between observed MW and theoretical MW, respectively. In the "Enzyme" column, T, Th, G and C indicate trypsin, thermolysin, endoproteinase Glu-C and chymotrypsin, respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the HNPQR conjugation site for KLH1 or KLH2 sequence.

**Figure 37. Identification details of Globo H and MMCCH-conjugated peptides on HNPQR residues for sample 4**

**Figure 37A. KLH 1**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 1933 | 1944 | 597.6233 | 1789.8481 | 1789.8458 | 1.29 | T | 0 | 14 | 0.042 | GYVPSHDTYTVR +GMd_NL997 (HKNPQR) | H1938 |
| 2 | 1036 | 1047 | 633.3073 | 1896.9001 | 1896.9028 | -1.48 | Th | 3 | 39 | 0.00014 | VHGMPTFPHWHR +GMd_NL997 (HKNPQR) | H1037 |
| 3 | 2754 | 2761 | 678.8262 | 1355.6378 | 1355.6503 | -9.22 | Th | 1 | 32 | 0.00059 | INQDTVRD +GMd_NL997 (HKNPQR) | R2760 |
| 4 | 157 | 166 | 548.9249 | 1643.7529 | 1643.7402 | 7.73 | T | 1 | 20 | 0.0093 | EAFHKFQEDR +Md_deamidated (HKNPQR) | H160 |
| 6 | 2515 | 2520 | 571.7632 | 1141.5118 | 1141.5074 | 3.94 | T | 1 | 24 | 0.0043 | QKEEDR +Md_deamidated (HKNPQR) | Q2515 |
| 7 | 2647 | 2661 | 715.6789 | 2144.0149 | 2144.0095 | 2.47 | G | 4 | 30 | 0.00097 | AEELRDALYKLQNDE +Md_deamidated (HKNPQR) | N2659 |
| 8 | 1733 | 1741 | 664.3221 | 1326.6296 | 1326.6312 | -1.13 | Th | 2 | 26 | 0.0024 | VLGGEKEMP +Oxidation (M); OBI822_Md (HKNPQR) | P1741 |
| 9 | 1930 | 1934 | 483.2334 | 964.4522 | 964.4589 | -6.84 | Th | 2 | 18 | 0.015 | FARGY +OBI822_Md (HKNPQR) | R1932 |

**Figure 37B. KLH 2**

| # | Start | End | Observed | Mr(expt) | Mr(calc) | ppm | Enzyme | MC | Score | E value | Peptide | Mod_site |
|---|---|---|---|---|---|---|---|---|---|---|---|---|
| 1 | 1948 | 1963 | 950.4304 | 2848.2694 | 2848.2949 | -8.95 | T | 1 | 26 | 0.0023 | RTQEFSKPEDTFDYHR +Deamidated (NQ); 2 GMd_NL997 (HKNPQR) | R1948 |
| 2 | 3243 | 3256 | 657.3107 | 1968.9103 | 1968.9000 | 5.23 | C | 1 | 19 | 0.011 | LVREGHDDENVGSF +GMd_NL997 (HKNPQR) | N3252 |
| 3 | 1330 | 1341 | 633.3073 | 1896.9001 | 1896.9028 | -1.48 | Th | 3 | 39 | 0.00014 | VHGMPTFPHWHR +GMd_NL997 (HKNPQR) | H1331 |
| 4 | 3243 | 3256 | 657.3099 | 1968.9079 | 1968.9000 | 4.01 | Th | 3 | 15 | 0.051 | LVREGHDDENVGSF +GMd_NL997 (HKNPQR) | N3252 |
| 5 | 281 | 293 | 915.9126 | 1829.8106 | 1829.8043 | 3.50 | T | 0 | 48 | 1.70E-05 | EHAKPADSFDYGR +Md_deamidated (HKNPQR) | H282 |
| 6 | 1097 | 1115 | 827.0797 | 2478.2173 | 2478.2101 | 2.91 | T | 0 | 59 | 2.60E-06 | KPLQPFGLDSVINPDDETR +Md_deamidated (HKNPQR) | Q1100 |
| 7 | 2755 | 2764 | 764.3863 | 1526.7580 | 1526.7439 | 9.30 | T | 0 | 21 | 0.0075 | QPLKPFSESR +Deamidated (NQ); Md_deamidated (HKNPQR) | P2756 |
| 8 | 1383 | 1394 | 705.3266 | 2112.9580 | 2112.9510 | 3.31 | C | 1 | 20 | 0.024 | NSRSQTFDPNPF +2 OBI822_Md (HKNPQR) | P1391, N1392 |
| 9 | 2360 | 2370 | 806.9017 | 1611.7888 | 1611.7789 | 6.14 | C | 1 | 16 | 0.026 | TKMHAVPNTLF +Oxidation (M); Md_deamidated (HKNPQR) | H2363 |
| 10 | 913 | 924 | 609.3015 | 1824.8827 | 1824.8817 | 0.55 | Th | 5 | 20 | 0.031 | VHGMAVFPHWHR +OBI822_Md (HKNPQR) | H914 |

"Start" and "End" indicate the number of residue of KLH1 or KLH2. "Observed" column implies the observed m/z value in the survey scan while "Mr(expt)", "Mr(calc)", "ppm" indicate the experimental molecular weight (MW), calculated MW, and the difference between observed MW and theoretical MW, respectively. In the "Enzyme" column, T, Th, G and C indicate trypsin, thermolysin, endoproteinase Glu-C and chymotrypsin, respectively. MC stands for missed cleavage. Scores are the peptide ion score directly reported from Mascot database search engine. E value denotes the expectation value for each identified peptide. The peptide sequences as well as its modifications are listed in "Peptide" column. "Mod_site" indicates the HNPQR conjugation site for KLH1 or KLH2 sequence.

**Figure 38**
**Summary of Globo H and MMCCH derivative analysis on HNPQR residues**

| Sample | 1 | | | 2 | | | 3 | | | 4 | | |
|---|---|---|---|---|---|---|---|---|---|---|---|---|
| KLH1/KLH2 | KLH1 | KLH2 | Total | KLH1 | KLH2 | Total | KLH1 | KLH2 | Total | KLH1 | KLH2 | Total |
| Globo H-conjugated | 1 | 4 | 5 | 2 | 4 | 6 | 2 | 2 | 4 | 3 | 3 | 6 |
| MMCCH- conjugated | 5 | 9 | 14 | 9 | 6 | 15 | 11 | 7 | 18 | 5 | 7 | 12 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100136042 A, Wong
- US 485546
- US 6544952 B
- US 5500362 A
- US 5821337 A
- US 5057540 A
- US 6524584 B
- US 8268969 B
- US 5814344 A
- US 5100669 A
- US 4849222 A
- WO 9511010 A
- WO 9307861 A
- US 4522811 A
- US 62050567 B


### Non-patent literature cited in the description


- **BREMER E G et al.** *J Biol Chem,* 1984, vol. 259, 14773-14777
- **RAGUPATHI G et al.** *Angew Chem Int Ed,* 1997, vol. 36, 125-128
- **GILEWSKI T et al.** *Proc Natl Acad Sci USA,* 2001, vol. 98, 3270-3275
- **HUANG C- Y et al.** *Proc Natl Acad Sci USA,* 2006, vol. 103, 15-20
- **WANG C-C et al.** *Proc Natl Acad Sci USA,* 2008, vol. 105 (33), 11661-11666
- **CHANG, Y-J et al.** *Proc Natl Acad Sci USA,* 2007, vol. 104 (25), 10299-10304
- **SLOVIN et al.** *Proc Natl Acad Sci USA,* 1999, vol. 96, 5710-5
- **GILEWSKI et al.** *Proc Natl Acad Sci USA,* 2001, vol. 98, 3270-5
- **SAMBROOK ; FRITSCH ; MANIATIS.** Molecular Cloning A Laboratory Manual. Cold Spring Harbor Laboratory Press, 1989
- DNA Cloning. 1985, vol. I, II
- **R. I. FRESHNEY.** Culture Of Animal Cells. Alan R. Liss, Inc, 1987
- Immobilized Cells And Enzymes. IRL Press, 1986
- **B. PERBAL.** *A Practical Guide To Molecular Cloning,* 1984
- Methods In Enzymology. Academic Press, Inc,
- Gene Transfer Vectors For Mammalian Cells. Cold Spring Harbor Laboratory, 1987
- Methods In Enzymology. vol. 154, 155
- Immunochemical Methods In Cell And Molecular Biology. Academic Press, 1987
- **HARLOW ; LANES.** Antibodies: A Laboratory Manual. Cold Spring Harbor Laboratory Press, 1988
- Handbook Of Experimental Immunology. 1986, vol. I-IV
- **GOEBEL, W. F. ; AVERY, O. T.** *J. Exp. Med.,* 1929, vol. 50, 521
- **AVERY, O. T. ; GOEBEL, W. F.** *J. Exp. Med.,* 1929, vol. 50, 533
- **ALLEN, P. Z. ; GOLDSTEIN, I. J.** *Biochemistry,* 1967, vol. 6, 3029
- **RUDE, E. ; DELIUS, M. M.** *Carbohydr. Res.,* 1968, vol. 8, 219
- **HIMMELSPACH, K. et al.** *Eur. J. Immunol.,* 1971, vol. 1, 106
- **FIELDER, R. J. et al.** *J. Immunol.,* 1970, vol. 105, 265
- **DENNIS, J.** *Oxford Glycosystems Glyconews Second,* 1992
- **LLOYD, K. O.** Specific Immunotherapy of Cancer with Vaccines. New York Academy of Sciences, 1993, 50-58
- **TOYOKUNI, T. et al.** *J. Am. Chem. Soc.,* 1994, vol. 116, 395
- **SLOVIN S F et al.** *Proc Natl Acad Sci USA,* 1997, vol. 96, 5710-5715
- **ZHU J. et al.** *J. Am. Chem. Soc.,* 2009, vol. 131 (26), 9298-9303
- **HUANG et al.** *Proc. Natl. Acad. Sci. USA,* 2006, vol. 103, 15-20
- **WANG et al.** *Proc Natl Acad Sci USA.,* 19 August 2008, vol. 105 (33), 11661-11666
- **RAVETCH ; KINET.** *Annu. Rev. Immunol.,* 1991, vol. 9, 457-92
- **CLYNES et al.** *Proc. Natl. Acad. Sci. (USA),* 1998, vol. 95, 652-656
- **GAZZANO-SANTARO et al.** *J. Immunol. Methods,* 1996, vol. 202, 163

• Cancer Principles and Practice of Oncology. Lippincott Williams & Wilkins Publishers, 15 February 2001

• *Micron,* 1999, vol. 30, 597-623